(19) Europäisches Patentamt
European Patent Office
Office européen des brevets

(11) EP 4 471 033 A1

(12) EUROPEAN PATENT APPLICATION
published in accordance with Art. 153(4) EPC

(43) Date of publication:
**04.12.2024 Bulletin 2024/49**

(21) Application number: **23746466.4**

(22) Date of filing: **29.01.2023**

(51) International Patent Classification (IPC):
***C07D 487/04*** (2006.01) ***C07D 471/04*** (2006.01)
***C07D 471/10*** (2006.01) ***C07D 471/14*** (2006.01)
***C07D 471/20*** (2006.01) ***C07D 487/10*** (2006.01)
***C07D 498/04*** (2006.01) ***C07D 498/14*** (2006.01)
***A61P 35/00*** (2006.01) ***A61K 31/407*** (2006.01)
***A61K 31/435*** (2006.01) ***A61K 31/437*** (2006.01)
***A61K 31/438*** (2006.01)

(52) Cooperative Patent Classification (CPC):
**A61K 31/407; A61K 31/435; A61K 31/437; A61K 31/438; A61P 35/00; C07D 471/04; C07D 471/10; C07D 471/14; C07D 471/20; C07D 487/04; C07D 487/10; C07D 498/04; C07D 498/14**

(86) International application number:
**PCT/CN2023/073745**

(87) International publication number:
**WO 2023/143589 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.01.2022 CN 202210109488**
**04.01.2023 CN 202310005966**

(71) Applicant: **Gan & Lee Pharmaceuticals Co., Ltd.**
**Beijing 101109 (CN)**

(72) Inventors:
- **XU, Fuming**
  **Beijing 101109 (CN)**
- **CHEN, Wenmin**
  **Beijing 101109 (CN)**
- **ZHOU, Guan**
  **Beijing 101109 (CN)**
- **YUAN, Hang**
  **Beijing 101109 (CN)**
- **LIU, Xiaobing**
  **Beijing 101109 (CN)**
- **WANG, Kanghua**
  **Beijing 101109 (CN)**
- **LI, Xiaoguang**
  **Beijing 101109 (CN)**

(74) Representative: **Kraus & Lederer PartGmbB**
**Thomas-Wimmer-Ring 15**
**80539 München (DE)**

# (54) CEREBLON E3 UBIQUITIN LIGASE INHIBITOR

(57) The present invention relates to a compound represented by formula (I), or an isomer, isotope derivative, polymorph, prodrug thereof, or a pharmaceutically acceptable salt or solvate thereof: wherein R1, R2, R3a, R3b, R3c, R3d, W1, W2, G, Z and n are as described. The compound of formula I is a human cereblon (CRBN) E3 ubiquitin ligase inhibitor, which can be used for preparing PROTAC molecules. The CRBN E3 ubiquitin ligase inhibitor and PROTAC molecule can be used to treat cancer and other diseases.

EP 4 471 033 A1

## Description

### Technical Field

The present invention relates to a cereblon E3 ubiquitin ligase inhibitor, a Proteolysis Targeting Chimeras (PROTAC) compound comprising the inhibitor, and a use thereof in medicine.

### Background

Cereblon (CRBN) is a brain-associated protein with ionic protease activity, which can interact with DNA damage binding protein-1 (DDB1), cadherin 4 (Cullin4, Cul4A or Cul4B), and the regulatory factor Cullins 1 (RoC1) to form a functional E3 ubiquitin ligase complex (CRBN-CRL4), which binds to the substrate and undergoes proteolysis through the ubiquitin-proteasome pathway. Immunomodulators (IMiDs), such as thalidomide, lenalidomide, and pomalidomide, etc., bind to CRBN to recruit new substrates that bind them to CRBN-CRL4, resulting in its ubiquitination and increased proteasome-dependent degradation to treat cancer and other diseases.

On this basis, CRBN ligands have been widely used in protein degradation, and a series of CRBN ligand-based protein degradation targeting chimeras (PROTACs) molecules have been developed. Due to the influence of the CRBN ligand itself on the target, it may additionally degrade the domain protein. So the design and synthesis of new CRBN ligands are of great significance for the further development of PROTACs molecules.

The present invention will disclose a series of novel CRBN ligands and can further synthesize corresponding PROTACs molecules.

### SUMMARY

The first aspect of the present invention provides a compound represented by formula I, or an isomer, an isotopic derivative, a polymorph, a prodrug thereof, or a pharmaceutically acceptable salt or a solvate thereof:

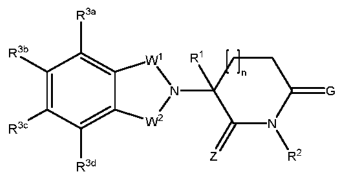

I,

wherein:

$W^1$ and $W^2$ are identical or different, and are each independently $CR^aRb$, C(=O), $NR^a$ or $SO_2$, and at least one of $W^1$ and $W^2$ is C(=O);

G and Z are identical or different, and are each independently selected from O, S, and Se;

$R^{3b}$ and $R^{3c}$ together with the carbon atoms to which they are attached form

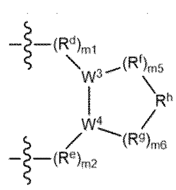

or

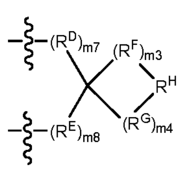

,

and $R^{3a}$ and $R^{3d}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino,

alkylacyl, alkyloxyacyl, alkylaminoacyl, cycloalkyl, heterocyclyl, aryl and heteroaryl, wherein the alkyl, heteroalkyl, alkenyl, alkynyl, alkoxy, cycloalkyl, heterocyclyl, aryl and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkyloxyacyl, alkylaminoacyl, aryl and heteroaryl;

or $R^{3c}$ and $R^{3d}$ together with the carbon atoms to which they are attached form

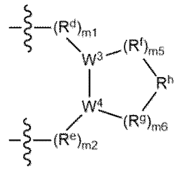

or

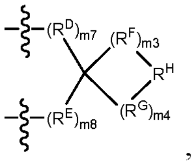

,

and $R^{3a}$ and $R^{3b}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkyloxyacyl, alkylaminoacyl, cycloalkyl, heterocyclyl, aryl and heteroaryl, wherein the alkyl, heteroalkyl, alkenyl, alkynyl, alkoxy, cycloalkyl, heterocyclyl, aryl and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkanoyl, alkyloxyacyl, alkylaminoacyl, aryl and heteroaryl;

or $R^{3a}$ and $R^{3b}$ together with the carbon atoms to which they are attached

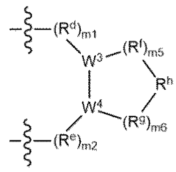

or

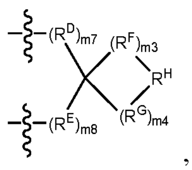

,

and $R^{3c}$ and $R^{3d}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkyloxyacyl, alkylaminoacyl, cycloalkyl, heterocyclyl, aryl and heteroaryl, wherein the alkyl, heteroalkyl, alkenyl, alkynyl, alkoxy, cycloalkyl, heterocyclyl, aryl and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkanoyl, alkyloxyacyl, alkylaminoacyl, aryl and heteroaryl;

$R^d$, $R^e$, $R^f$, $R^g$, $R^D$, $R^E$, $R^F$, and $R^G$ at each occurrence are each independently $C(R^m)_2$, $NR^m$, C(=O), O, or S;

$W^3$ and $W^4$ at each occurrence are each independently $CR^m$ or N;

$R^h$ and $R^H$ at each occurrence are each independently $NR^{1h}$, C(=O), $SO_2$, or $CR^{2h}R^{3h}$, when $R^D$, $R^E$, $R^F$, and $R^G$ are all $C(R^m)_2$, $R^H$ is C(=O), or $CR^{2h}R^{3h}$;

m1 and m2 at each occurrence are independently an integer of 0, 1, 2, 3, 4, 5, or 6, and $m1+m2 \leq 6$;
m3 at each occurrence is independently an integer of 0, 1, 2, 3, 4, 5, 6, or 7, and m4 is an integer of 1, 2, 3, 4, 5, 6, 7, or 8, and $m3+ m4 \leq 8$;
m5 and m6 at each occurrence are each independently an integer of 0, 1, 2, 3, 4, 5, 6, or 7, and $m5+ m6 \leq 7$;
m7 and m8 at each occurrence are each independently an integer of 0, 1, 2, 3, 4, 5, 6, or 7, and $m7+ m8 \leq 7$;
$R^m$ at each occurrence is independently selected from H, deuterium atom, halogen, alkyl, deuteroalkyl, heteroalkyl, alkenyl, alkynyl, alkoxy, haloalkyl, haloalkoxy, hydroxyl, hydroxyalkyl, nitro, cyano, amino, alkylamino, alkylacyl, alkyloxyacyl, alkylaminoacyl, cycloalkyl, heterocyclyl, aryl and heteroaryl, wherein the alkyl , heteroalkyl, alkenyl, alkynyl, alkoxy, cycloalkyl, heterocyclyl, aryl and heteroaryl are each independently and optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkyloxyacyl, alkylaminoacyl, aryl and heteroaryl;

$R^{1h}$, $R^{2h}$, and $R^{3h}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkyloxyacyl, alkylaminoacyl, cycloalkyl, heterocyclyl, aryl, and heteroaryl, wherein the alkyl, heteroalkyl, alkoxy, cycloalkyl, heterocyclyl, aryl, and heteroaryl are each independently substituted with one or more substituents selected from halogen, alkyl, heteroalkyl, alkenyl, alkynyl, alkoxy, haloalkyl, hydroxyl, hydroxyalkyl, cyano, amino, nitro, cycloalkyl, heterocyclyl, alkylamino, alkylacyl, alkyloxyacyl, alkylaminoacyl, aryl and heteroaryl;
$R^1$ is selected from H, halogen, deuterium atom, $C_1$-$C_6$ alkyl, $C_1$-$C_6$ alkoxy, hydroxyl, cycloalkyl, $C_1$-$C_6$ haloalkyl, and hydroxyalkyl, preferably, $R^1$ is selected from H, F, Cl, Br, I, $C_1$-$C_3$ alkyl, $C_1$-$C_3$ alkoxy, $C_3$-$C_6$ cycloalkyl, $C_1$-$C_3$ haloalkyl, hydroxyl, and $C_1$-$C_6$ hydroxyalkyl;
$R^2$, $R^a$, and $R^b$ are each independently selected from H, deuterium atom, F, Cl, Br, I, $C_1$-$C_6$ alkyl, hydroxyl, $C_1$-$C_6$ haloalkyl, $C_1$-$C_6$ hydroxyalkyl and $C_1$-$C_6$ alkoxy; and
n is 0, 1, 2 or 3.

In one embodiment, $W^1$ and $W^2$ are identical or different, and are each independently $CH_2$ or C(=O), and at least one of $W^1$ and $W^2$ is C(=O);
In another embodiment, G and Z are O.
In another embodiment, $R^{3b}$ and $R^{3c}$ together with the carbon atoms to which they are attached form

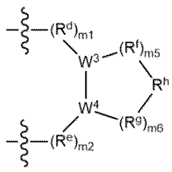
or
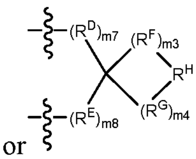
,

and $R^{3a}$ and $R^{3d}$ are each independently selected from H, deuterium atom, halogen, $C_1$-$C_6$ alkyl, heteroalkyl, $C_2$-$C_6$ alkenyl, $C_2$-$C_6$ alkynyl, $C_1$ -$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, aryl and heteroaryl are each independently substituted with one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, aryl and heteroaryl , preferably $R^{3a}$ and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$-$C_6$ alkyl, $C_1$- $C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano , amino , nitro , $C_3$-$C_8$ cycloalkyl, $C_4$-$C_{10}$ heterocyclyl, $C_6$-$C_{10}$ aryl and $C_5$-$C_{10}$ heteroaryl, preferably $R^{3a}$ and $R_{3d}$ are each independently selected from H, deuterium atom, halogen, $C_1$-$C_3$ alkyl, and $C_1$-$C_3$ alkoxy; In another embodiment, $R^{3c}$ and $R^{3d}$ together with the carbon atoms to which they are attached

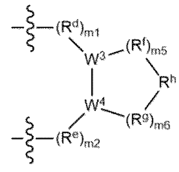

or

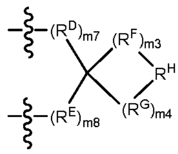

,

and $R^{3a}$ and $R^{3b}$ are each independently selected from H, deuterium atom, halogen, $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl , $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$ -$C_6$ alkoxy, $C_3$-$C_8$ cycloalkyl, $C_4$-$C_{10}$ heterocyclyl, $C_6$ -$C_{10}$ aryl, $C_5$-$C_{10}$ heteroaryl are each independently optionally substituted with one or more substituents selected from the groups of halogen, $C_1$ -$C_6$ alkyl, $C_1$ - $C_6$ heteroalkyl, $C_1$ -$C_6$ alkoxy, $C_1$ -$C_6$ haloalkyl, hydroxyl, $C_1$ -$C_6$ hydroxyalkyl, cyano , amino , nitro , $C_3$-$C_8$ cycloalkyl , $C_4$-$C_{10}$ heterocyclyl, $C_6$ -$C_{10}$ aryl and $C_5$ -$C_{10}$ heteroaryl, preferably $R^{3a}$ and $R^{3b}$ are each independently selected from H, deuterium atom, F, Cl, Br, I, $C_1$ -$C_6$ alkyl, $C_1$ -$C_6$ heteroalkyl, $C_2$ -$C_6$ alkenyl, $C_2$ -$C_6$ alkynyl, $C_1$ -$C_6$ alkoxy, hydroxyl, nitro, cyano, amino, $C_3$ -$C_8$ cycloalkyl, $C_4$ -$C_{10}$ heterocyclyl, $C_6$ - $C_{10}$ aryl and $C_5$ -$C_{10}$ heteroaryl, wherein the $C_1$ -$C_6$ alkyl, $C_1$ -$C_6$ heteroalkyl, $C_2$ -$C_6$ alkenyl, $C_2$ -$C_6$ alkynyl, $C_1$ -$C_6$ alkoxy, $C_3$ -$C_8$ cycloalkyl, $C_4$ -$C_{10}$ heterocyclyl, $C_6$ -$C_{10}$ aryl and $C_5$ -$C_{10}$ heteroaryl are each independently optionally substituted with one or more substituents selected from F, Cl, Br, I, $C_1$-$C_6$ alkyl, $C_1$ -$C_6$ heteroalkyl, $C_1$-$C_6$ alkoxy, $C_1$ -$C_6$ haloalkyl, hydroxyl, $C_1$-$C_6$ hydroxyalkyl, cyano, amino, nitro, $C_3$-$C_8$ cycloalkyl, $C_4$ -$C_{10}$ heterocyclyl, $C_6$-$C_{10}$ aryl, and $C_5$ -$C_{10}$ heteroaryl, preferably $R^{3a}$ and $R^{3b}$ are each independently selected from H, deuterium atom, halogen, $C_1$-$C_3$ alkyl, and $C_1$-$C_3$ alkoxy; In another embodiment, $R^{3a}$ and $R^{3b}$ together with the carbon atoms to which they are attached form

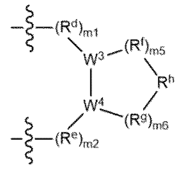

or

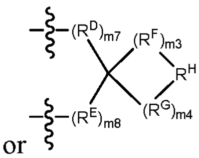

,

and $R^{3c}$ and $R^{3d}$ are each independently selected from H, deuterium, halogen, $C_1$ -$C_6$ alkyl, heteroalkyl, $C_2$ -$C_6$ alkenyl, $C_2$ -$C_6$ alkynyl, $C_1$ -$C_6$ alkoxy, $C_1$ -$C_6$ haloalkoxy, $C_1$ -$C_6$ haloalkoxy, hydroxyl, $C_1$ -$C_6$ hydroxyalkyl, nitro, cyano, amino, $C_3$ -$C_8$ cycloalkyl, $C_4$ -$C_{10}$ heterocyclyl, $C_6$ -$C_{10}$ aryl, and $C_5$-$C_{10}$ heteroaryl, wherein the $C_1$-$C_6$ alkyl, $C_1$ -$C_6$ heteroalkyl, $C_2$ -$C_6$ alkenyl, $C_2$ -$C_6$ alkynyl, $C_1$ -$C_6$ alkoxy, $C_3$ -$C_8$ cycloalkyl, $C_4$ -$C_{10}$ heterocyclyl, $C_6$ -$C_{10}$ aryl, $C_5$ -$C_{10}$ heteroaryl are each independently optionally substituted with one or more substituents selected from the group of halogen, $C_1$-$C_6$ alkyl, $C_1$ -$C_6$ heteroalkyl, $C_1$ -$C_6$ alkoxy, $C_1$ -$C_6$ haloalkyl, hydroxyl, $C_1$ -$C_6$ hydroxyalkyl, cyano, amino, nitro, $C_3$-$C_8$ cycloalkyl, $C_4$-$C_{10}$ heterocyclyl, $C_6$ -$C_{10}$ aryl and $C_5$ -$C_{10}$ heteroaryl, preferably $R^{3c}$ and $R^{3d}$ each independently selected from H, deuterium atom, F, Cl, Br, I, $C_1$ -$C_6$ alkyl, $C_1$ -$C_6$ heteroalkyl, $C_2$ -$C_6$ alkenyl, $C_2$ -$C_6$ alkynyl, $C_1$ -$C_6$ alkoxy, hydroxyl, nitro, cyano, amino, $C_3$ -$C_8$ cycloalkyl, $C_4$ -$C_{10}$ heterocyclyl, $C_6$ - $C_{10}$ aryl and $C_5$ -$C_{10}$ heteroaryl, wherein the $C_1$ -$C_6$ alkyl, $C_1$ -$C_6$ heteroalkyl, $C_2$ -$C_6$ alkenyl, $C_2$ -$C_6$ alkynyl, $C_1$ -$C_6$ alkoxy, $C_3$ -$C_8$ cycloalkyl, $C_4$ -$C_{10}$ heterocyclyl, $C_6$ -$C_{10}$ aryl and $C_5$ -$C_{10}$ heteroaryl are each independently optionally substituted with one or more substituents selected from F, Cl, Br, I, $C_1$ -$C_6$ alkyl, $C_1$ -$C_6$ heteroalkyl, $C_1$ -$C_6$ alkoxy, $C_1$ -$C_6$ haloalkyl, hydroxyl, $C_1$-$C_6$ hydroxyalkyl, cyano, amino, nitro, $C_3$ -$C_8$ cycloalkyl, $C_4$ -$C_{10}$ heterocyclyl, $C_6$ -$C_{10}$ aryl, and $C_5$ -$C_{10}$ heteroaryl, preferably $R^{3c}$ and $R^{3d}$ each independently selected from H, deuterium atom, halogen, $C_1$ -$C_3$ alkyl, and $C_1$ -$C_3$ alkoxy.

In another embodiment, at each occurrence, $R^d$ , $R^e$, $R^f$, $R^g$, $R^D$, $R^E$, $R^F$ , and $R^G$ at each occurrence are independently $C(R^m)_2$ or O.

In another embodiment, $W^3$ and $W^4$ are CH.

In another embodiment, $R^{1h}$, $R^{2h}$ , and $R^{3h}$ are each independently selected from H, deuterium atom, halogen, $C_1$ -$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, $C_1$ -$C_6$ haloalkyl, $C_1$ -$C_6$ haloalkoxy, hydroxyl, $C_1$ -$C_6$ hydroxyalkyl, nitro, cyano, amino, $C_3$ -$C_8$ cycloalkyl, $C_4$ -$C_{10}$ heterocyclyl, $C_6$-$C_{10}$ aryl, and $C_5$-$C_{10}$ heteroaryl, wherein the $C_1$-$C_6$ alkyl, $C_1$ -$C_6$ heteroalkyl, $C_2$ -$C_6$ alkenyl, $C_2$ -$C_6$ alkynyl, $C_1$ -$C_6$ alkoxy, $C_3$ -$C_8$ cycloalkyl, $C_4$ -$C_{10}$ heterocyclyl, $C_6$ -$C_{10}$ aryl, and $C_5$ -$C_{10}$ heteroaryl are independently and optionally substituted with one or more substituents selected from the group consisting of halogen, $C_1$ -$C_6$ alkyl, $C_1$ -$C_6$ heteroalkyl, $C_1$ -$C_6$ alkoxy, $C_1$ -$C_6$ haloalkyl, hydroxyl, $C_1$ -$C_6$ hydroxyalkyl, cyano, amino, nitro, $C_3$-$C_8$ cycloalkyl, $C_4$-$C_{10}$ heterocyclyl, $C_6$ -$C_{10}$ aryl, and $C_5$ -$C_{10}$ heteroaryl.

In another embodiment, m1 and m2 at each occurrence are each independently an integer of 0, 1, 2, or 3, and $m1+m2 \leq 3$; preferably m1+m2=1 or m1+m2=2.

In another embodiment, m3 at each occurrence is independently an integer of 0, 1, 2, 3, or 4, and m4 is an integer

of 1, 2, 3, 4, or 5, and m3+m4≤5; preferably m3+m4=2 , m3+m4=3 or m3+m4=4.

In another embodiment, m5 and m6 at each occurrence are each independently an integer of 0, 1, 2, 3 or 4, and m5+m6≤4, preferably m5+m6=2 or m5+m6=3;.

In another embodiment, m7 and m8 at each occurrence are independently an integer of 0, 1, 2, 3 or 4, and m7+m8≤4, preferably m7+m8=2 or m7+m8=3.

In another embodiment, at each occurrence is independently selected from H, deuterium atom, halogen, $C_1$ -$C_6$ alkyl, $C_1$ -$C_6$ heteroalkyl, $C_2$ -$C_6$ alkenyl, $C_2$ -$C_6$ alkynyl, $C_1$ -$C_6$ alkoxy, $C_1$ -$C_6$ haloalkyl, $C_1$ -$C_6$ haloalkoxy, hydroxyl, $C_1$ -$C_6$ hydroxyalkyl, nitro, cyano, amino, $C_3$ -$C_8$ cycloalkyl, $C_4$ -$C_{10}$ heterocyclyl, aryl and heteroaryl, wherein the $C_1$ -$C_6$ alkyl, $C_1$ -$C_6$ heteroalkyl, $C_2$-$C_6$ alkenyl, $C_2$ -$C_6$ alkynyl, $C_1$ -$C_6$ alkoxy, $C_3$ -$C_8$ cycloalkyl, $C_4$ -$C_{10}$ heterocyclyl, $C_6$ -$C_{10}$ aryl, and $C_5$ -$C_{10}$ heteroaryl are each independently optionally substituted with one or more substituents selected from the group consisting of halogen, $C_1$ -$C_6$ alkyl, $C_1$ -C heteroalkyl, $C_1$ -$C_6$ alkoxy, $C_1$ -$C_6$ haloalkyl, hydroxyl, $C_1$ -$C_6$ hydroxyalkyl, cyano, amino, nitro, $C_3$ -$C_8$ cycloalkyl, $C_4$ -$C_{10}$ heterocyclyl, $C_6$-$C_{10}$ aryl and $C_5$-$C_{10}$ heteroaryl.

In another embodiment, $R^1$ is selected from H, halogen, $C_1$ -$C_3$ alkyl, and hydroxyl; preferably, $R^1$ is selected from H, F, Cl, Br, I, $C_1$ -$C_3$ alkyl, and hydroxyl.

In another embodiment, $R^2$ is selected from H, and $C_1$ -$C_3$ alkyl.

In another embodiment, n is 0 or 1.

In another embodiment, the compound is selected from the following structures:

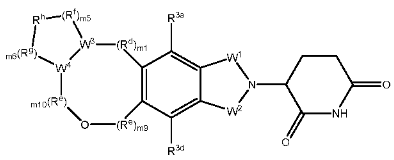

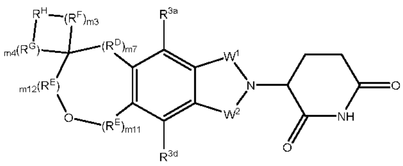

,

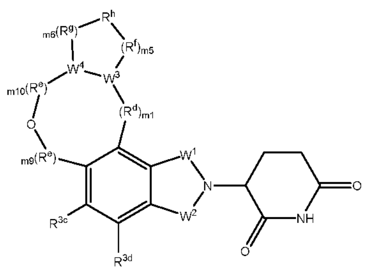

,

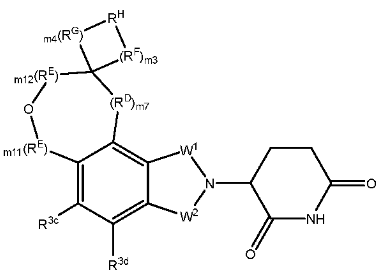

,

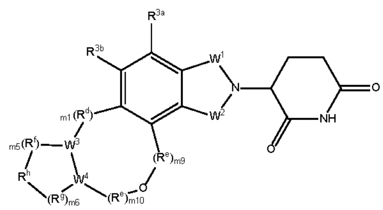

,

and

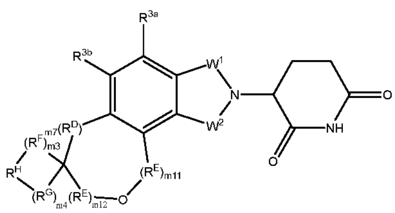

$W^1$, $W^2$, $W^3$, $W^4$, $R^{3a}$, $R^{3b}$ , $R^{3c}$, $R^{3d}$ , $R^d$, $R^e$, $R^f$, $R^h$, $R^g$, $R^D$, $R^E$, $R^F$ $R^H$ , $R^G$ m3, m4, m5 and m6 are as defined in claim 1, 2 or 3, preferably, $R^{3a}$ , $R^{3b}$ , $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$ -$C_{10}$ heterocyclyl, $C_6$ -$C_{10}$ aryl and $C_5$ -$C_{10}$ heteroaryl are each independently substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; and

m1, m9 and m10 at each occurrence are each independently an integer of 0, 1, 2, 3, 4, or 5, and m1+m9+m10≤5; preferably, m1, m9 and m10 at each occurrence are each independently an integer of 0, 1, or 2, and m1+m9+m10≤2, preferably m1+m9+m10=1 or m1+m9+m10=0; and

m7, m11 and m12 at each occurrence are each independently an integer of 0, 1, 2, 3, 4, 5, or 6, and m7+m11+m12≤6; preferably, m7, m11 and m12 at each occurrence are each independently an integer of 0, 1, 2, or 3, and

m7+m11+m12≤3, preferably m7+m11+m12=2 or m7+m11+m12=1.

In another embodiment, the compound is selected from the following structures:

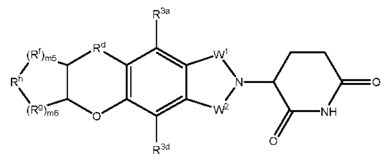 , 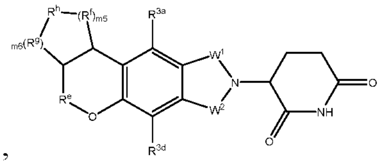 , 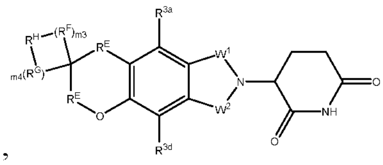 ,

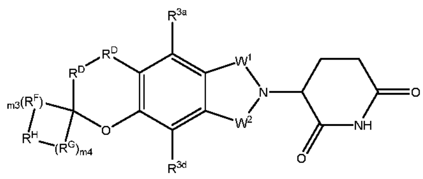 , 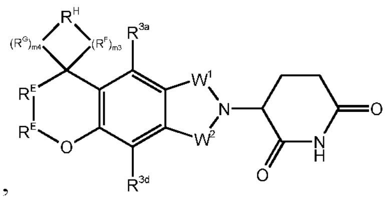 , 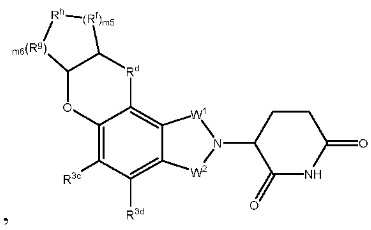 ,

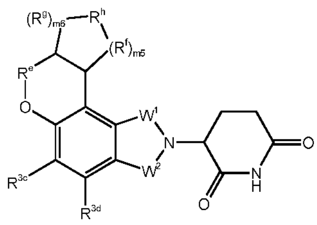 , 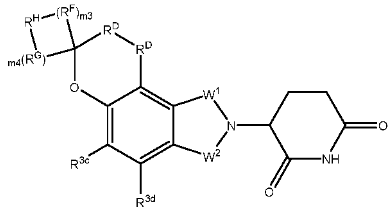 , 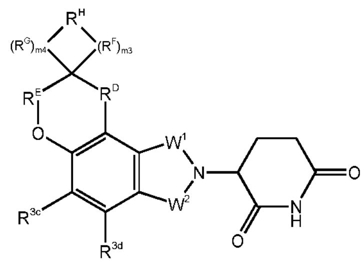 ,

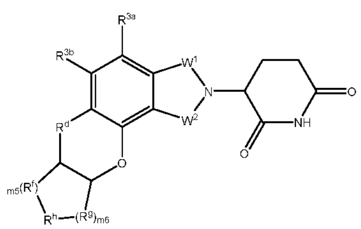 , 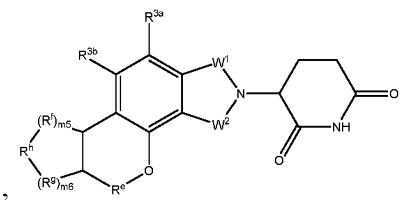 , 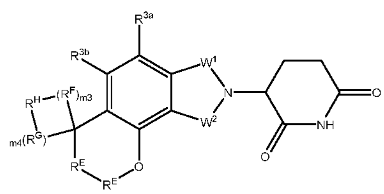 ,

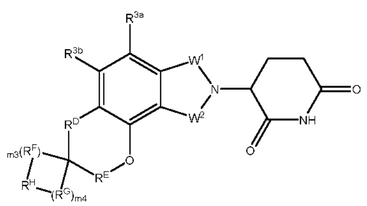 , 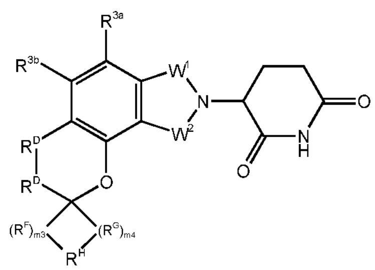 , 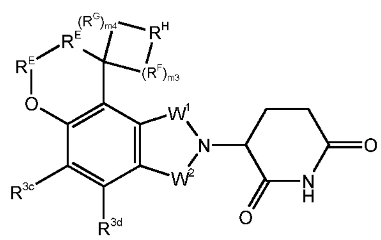 ,

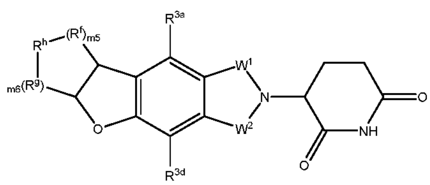 , 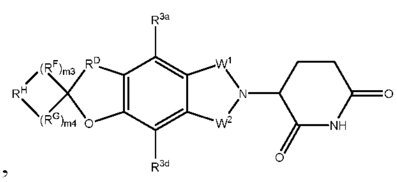 , 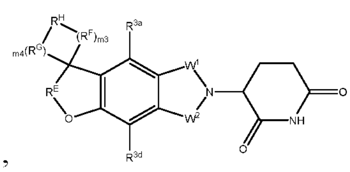 ,

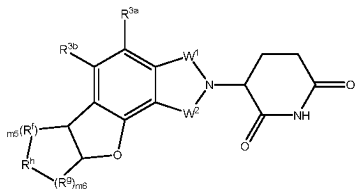 , 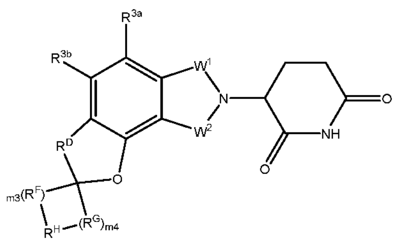 , 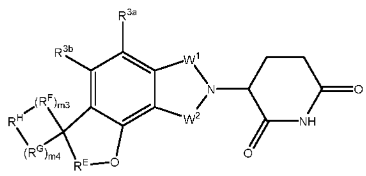 ,

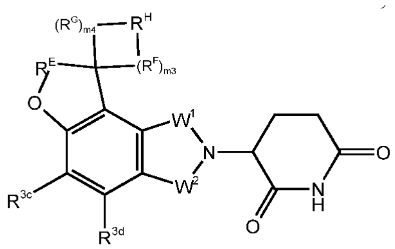 , 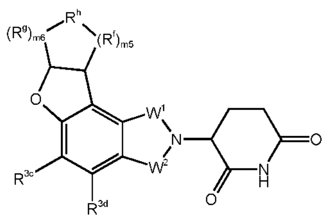 and 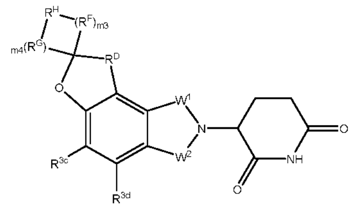 ,

wherein:

$W^1$, $W^2$, $R^{3a}$, $R^{3b}$, $R^{3c}$, $R^{3d}$, $R^d$, $R^e$, $R^f$, $R^h$, $R^g$, $R^D$, $R^E$, $R^F$, $R^H$, $R^G$, m3, m4, m5, and m6 are as defined hereinbefore.

In another embodiment, the compound is selected from the following structures:

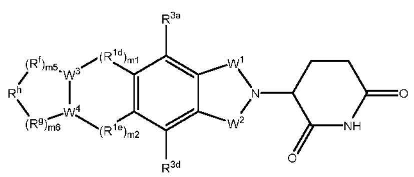 , 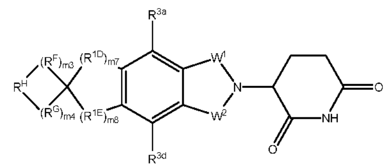 ,

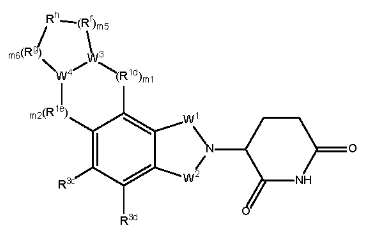 , 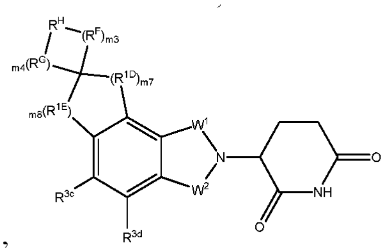 , 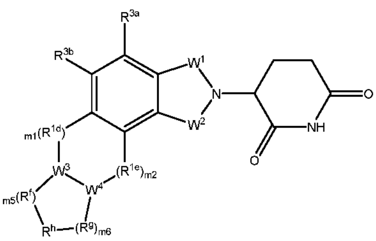 ,

and

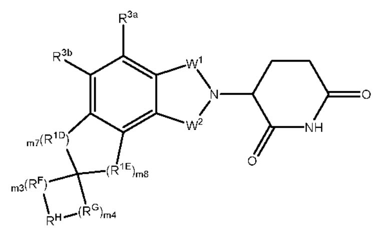

wherein:

$W^1$, $W^2$, $W^3$, $W^4$, $R^{3a}$, $R^{3b}$, $R^{3c}$, $R^{3d}$, $R^f$, $R^h$, $R^g$, $R^F$, $R^G$ m1, m2, m3, m4, m5, m6, m7, and m8 are as defined in claim 1, 2, or 3, and preferably, $R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl, and $C_5$-$C_{10}$ heteroaryl;

$R^{1d}$ , $R^{1e}$, $R^{1D}$ and $R^{1E}$ at each occurrence are independently $C(R^m)_2$;
$R^H$ is $NR^{1h}$, C(=O), or $CR^{2h}R^{3h}$, and when $R^F$ and $R^G$ both are $C(R^m)_2$, $R^H$ is C(=O), or $CR^{2h}R^{3h}$; and $R^{1h}$ , $R^{2h}$ , $R^{3h}$ , and are as defined previously.

In another embodiment, the compound is selected from the following structures:

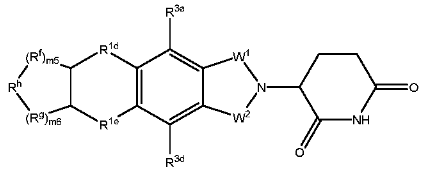
,
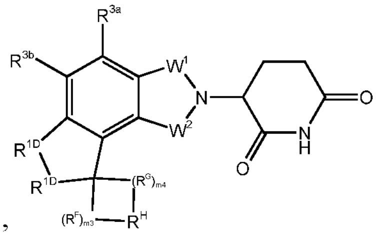
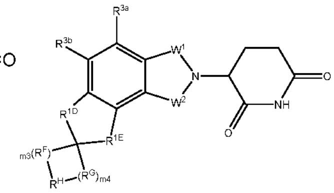

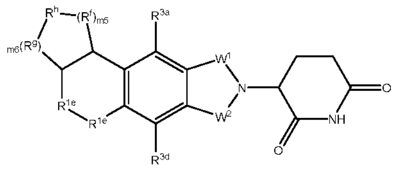
,
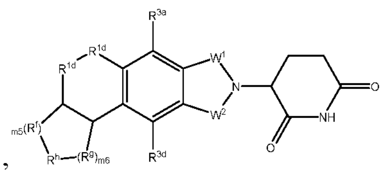
,
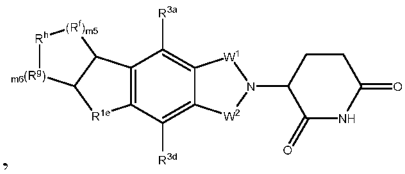
,

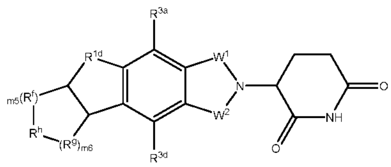
,
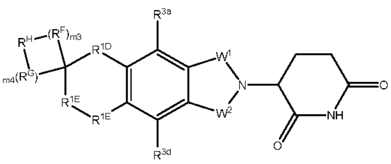
,
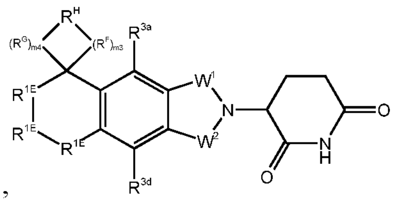
,

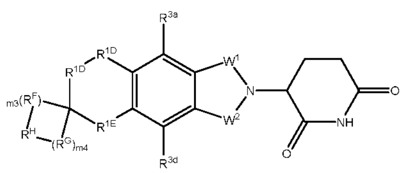
,
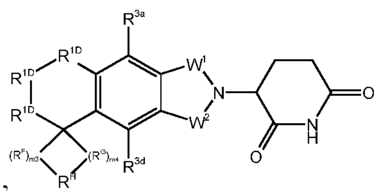
, ,
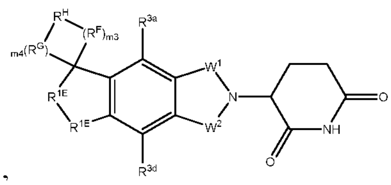

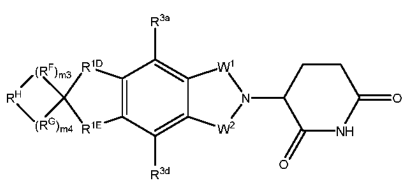
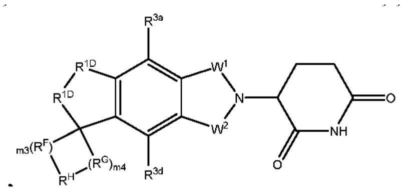

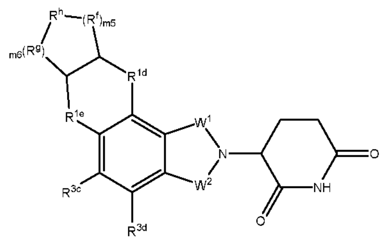
, ,
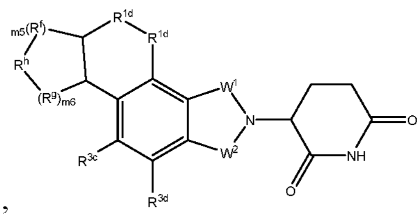
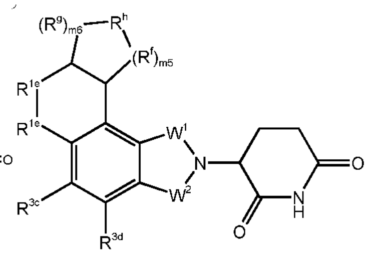
,

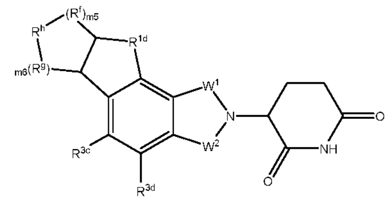 , 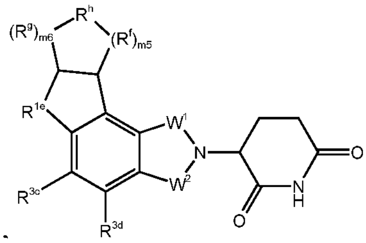 , 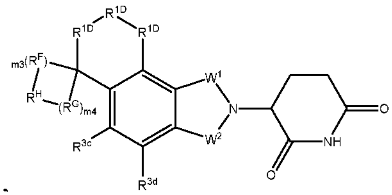 ,

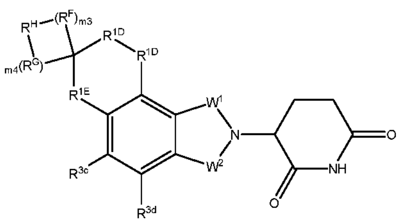 , 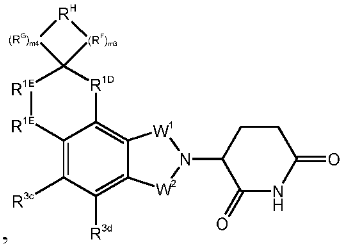 , 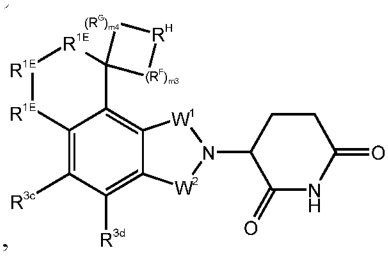 ,

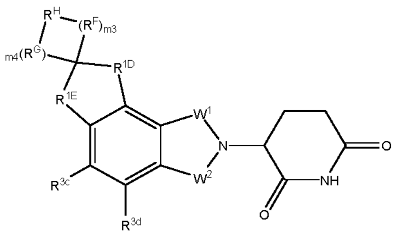 , 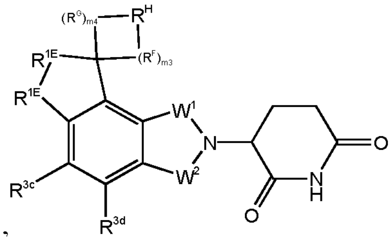 ,

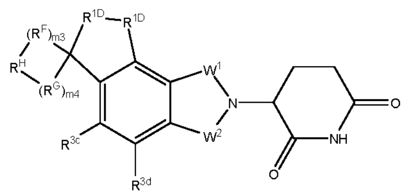 , 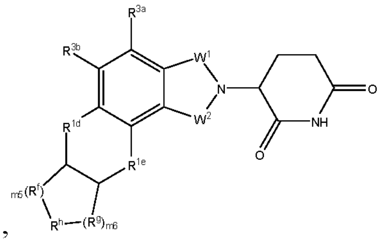 ,

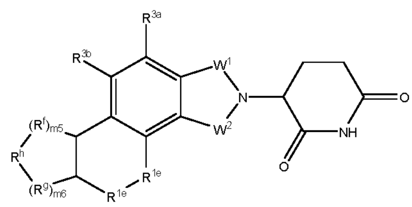 , 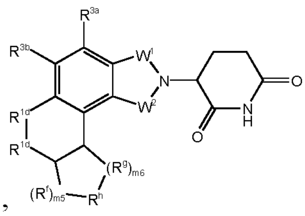 , 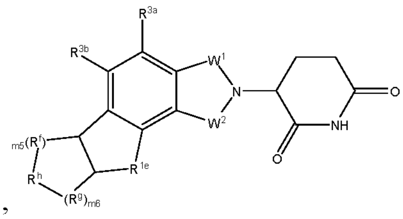 ,

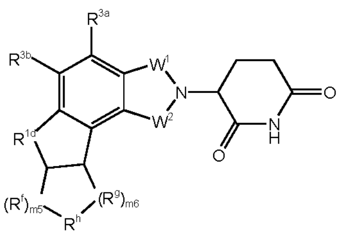 , 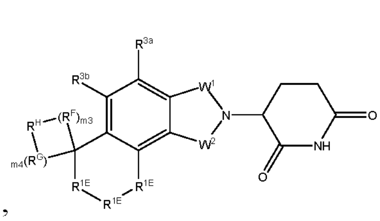 , 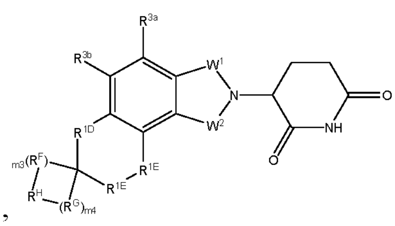 ,

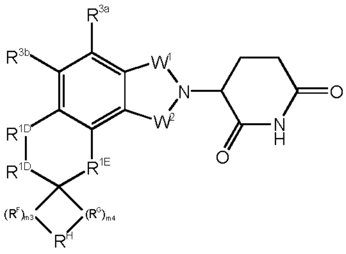,
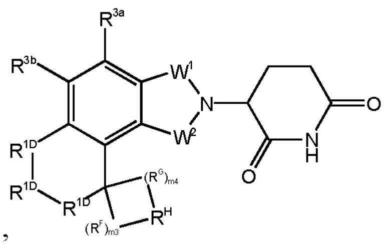,
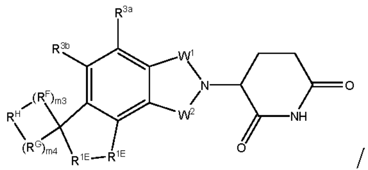/

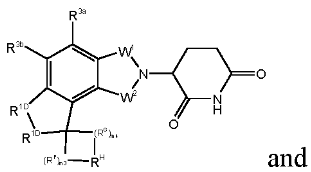 and 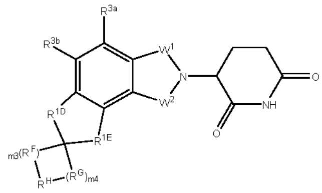;

wherein $W^1$, $W^2$, $R^{3a}$, $R^{3b}$, $R^{3c}$, $R^{3d}$, $R^f$, $R^h$, $R^g$, $R^F$, $R^G$, m3, m4, m5, and m6 are as defined in claims 1, 2, or 3; and $R^{1d}$, $R^{1e}$, $R^{1D}$, and $R^{1E}$ are each independently C(R )$^m{}_2$ at each occurrence; $R^H$ is $NR^{1h}$, C(=O), or $CR^{2h}R^{3h}$, and $R^H$ is C(=O), or $CR^{2h}R^{3h}$ when both $R^F$ and $R^G$ are C(R )$^m{}_2$ ; and $R^{1h}$, $R^{2h}$, $R^{3h}$, and $R^m$ are defined as claims 1, 2 or 3;

In another embodiment, the compound is selected from the following structures:

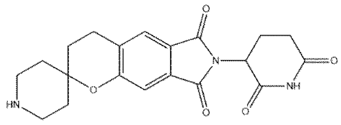,
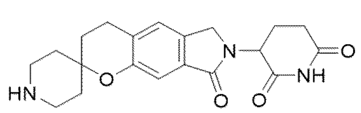,
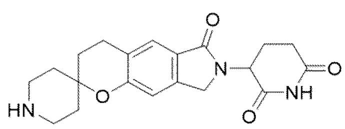,
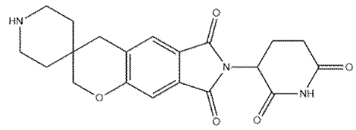,
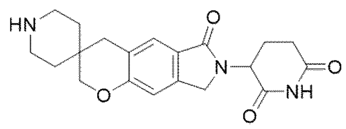,
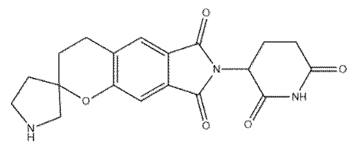,
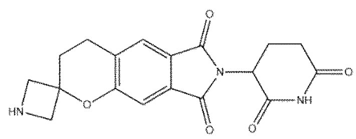,
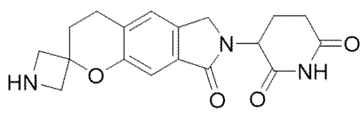,
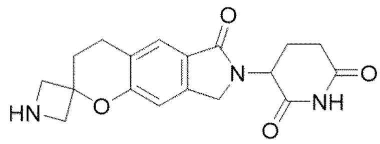,
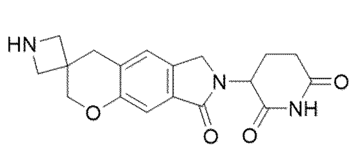,
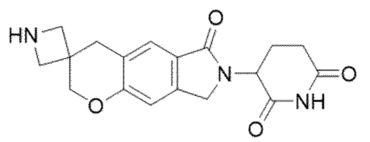,
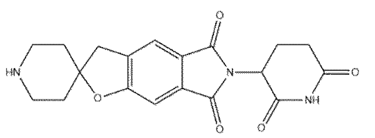,
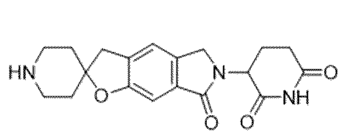,
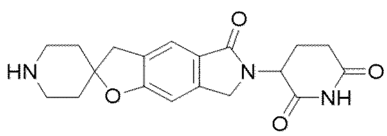,
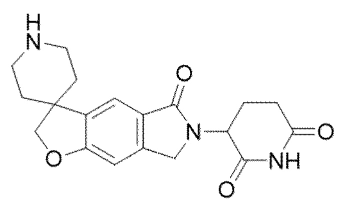,

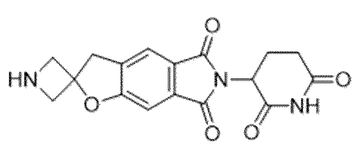,
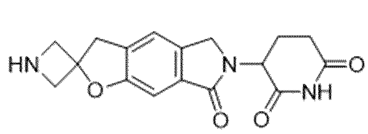,
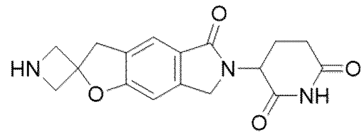,

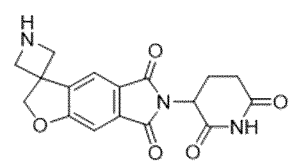,
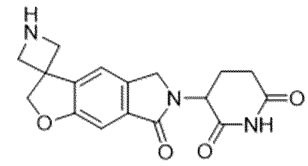,
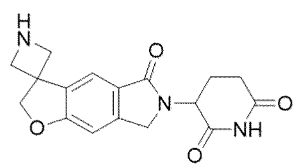,

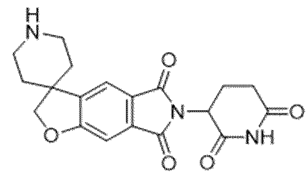,
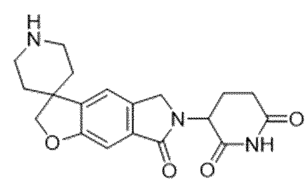,
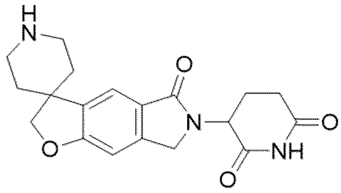,

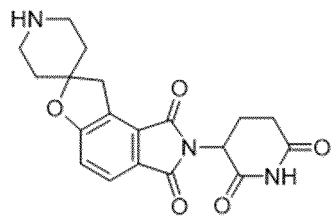,
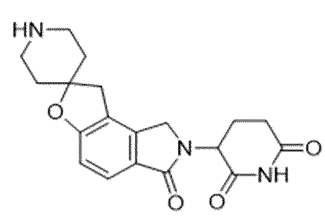,
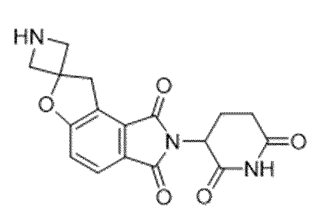,

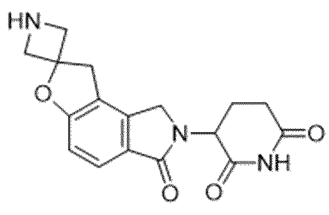,
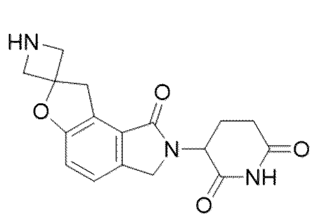,
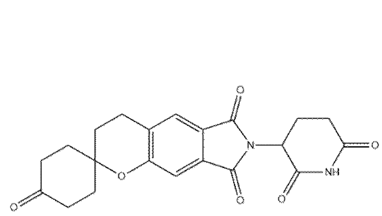,

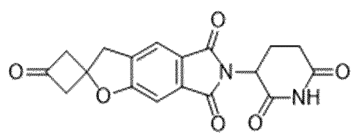,
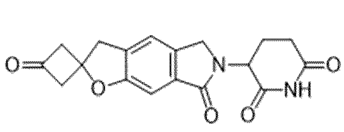,
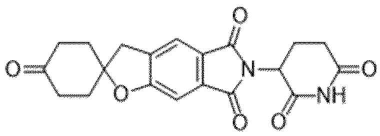,

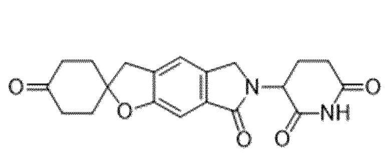,
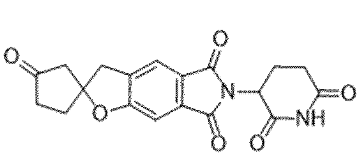,
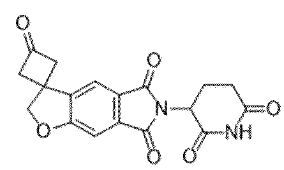,

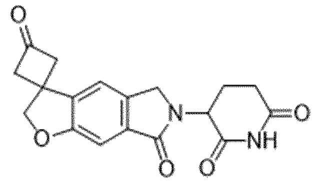 , 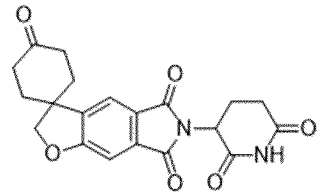 , 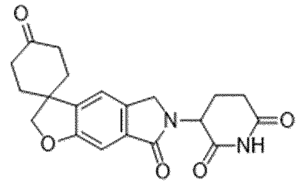 ,

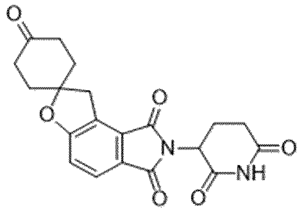 , 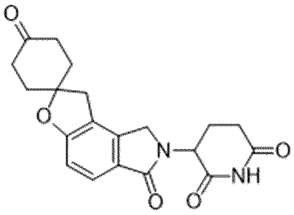 , 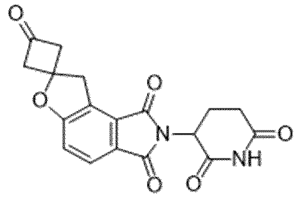 ,

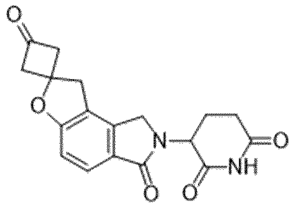 , 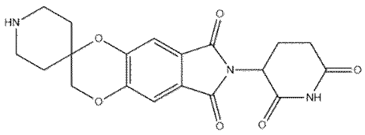 , 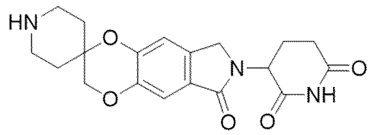 ,

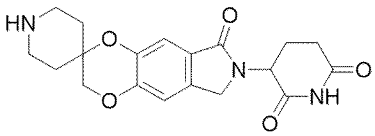 , 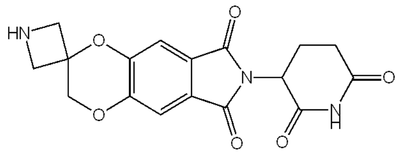 , 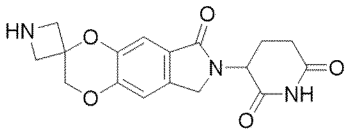 ,

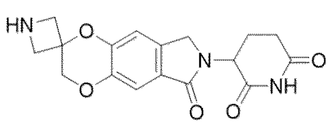 , 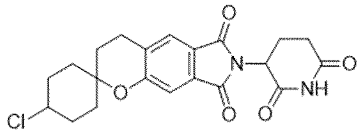 , 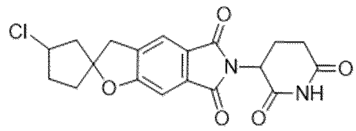 ,

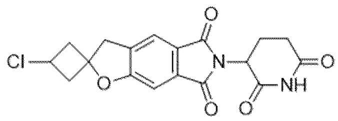 , 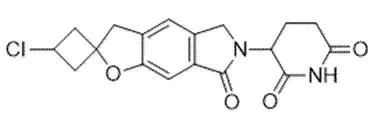 , 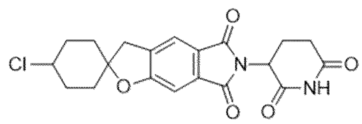 ,

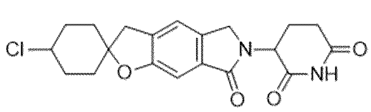 , 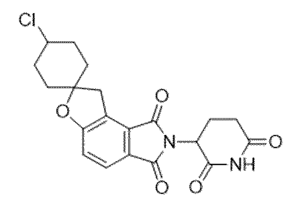 , 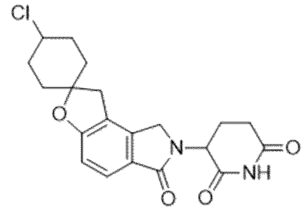 ,

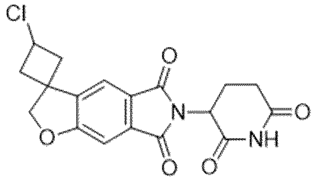,
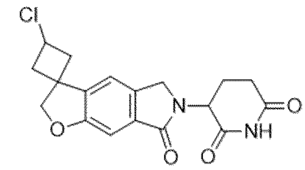,
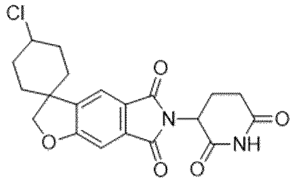,

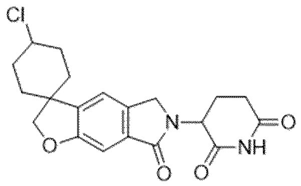,
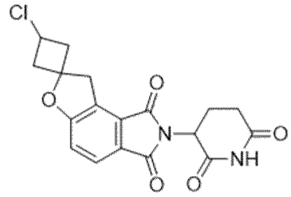,
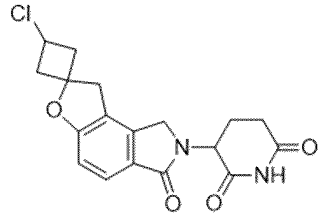,

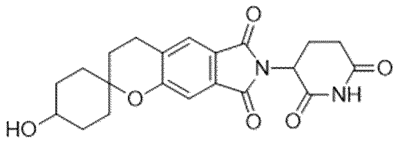,
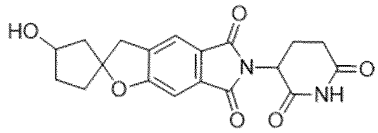,
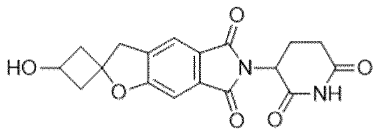,

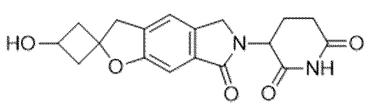,
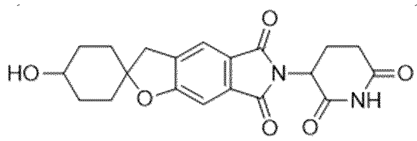,
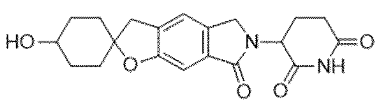,

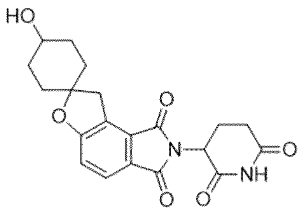,
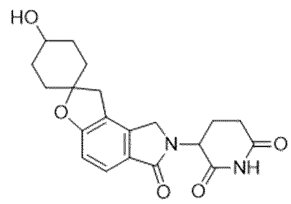,
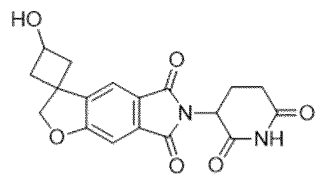,

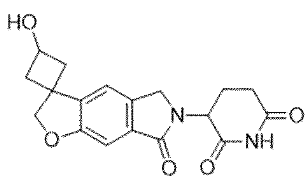,
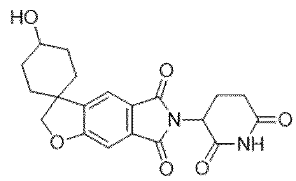,
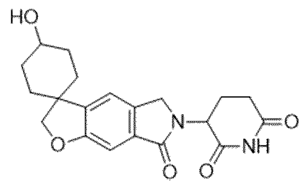,

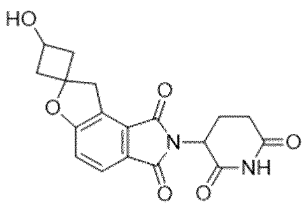,
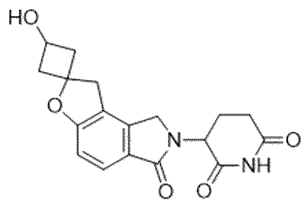,
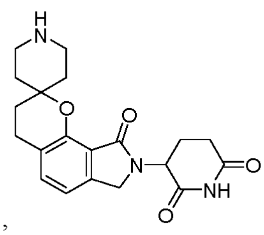,
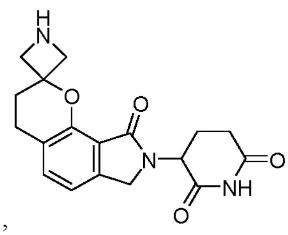,

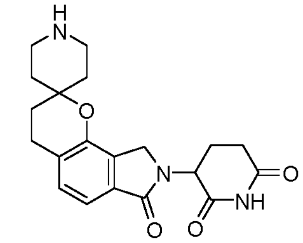 , 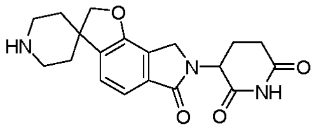 , 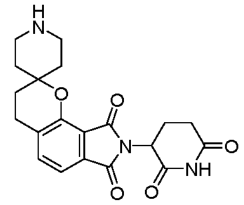 , 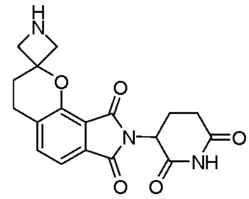 ,

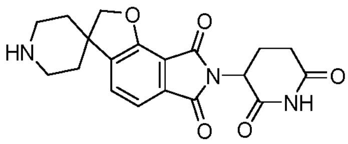 , 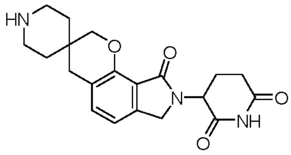 , 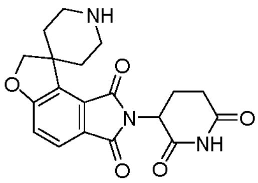 , 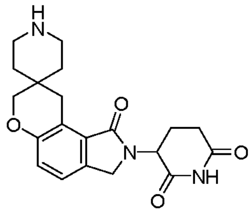 ,

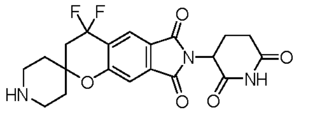 , 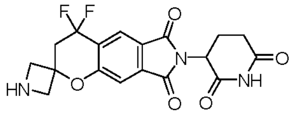 , 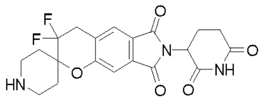 ,

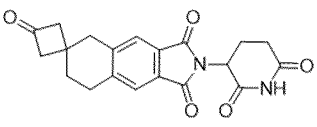 , 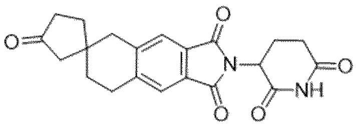 , 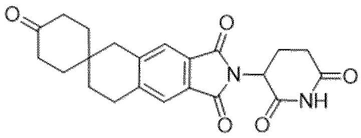 ,

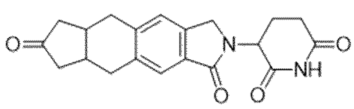 , 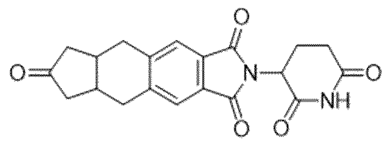 , 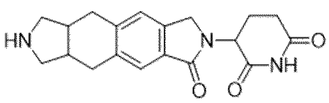 ,

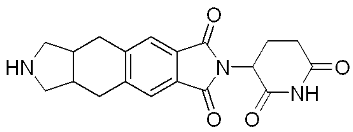 , 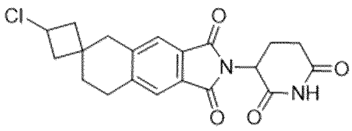 , 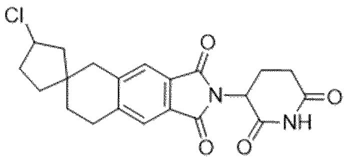 ,

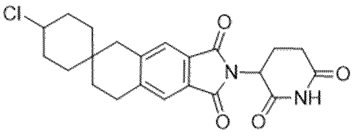 , 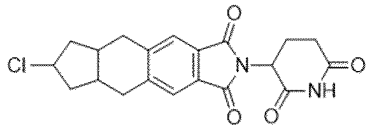 , 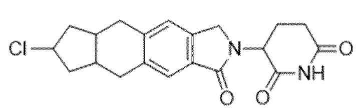 ,

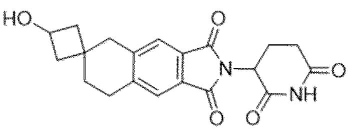 , 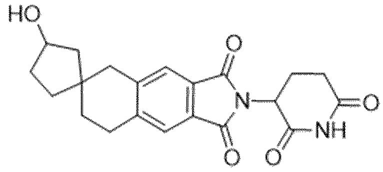 , 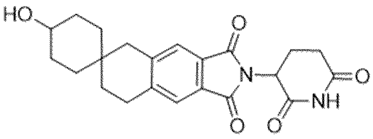 ,

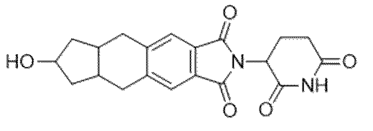 , 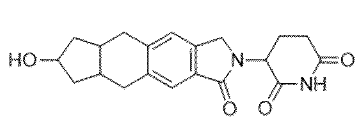 , 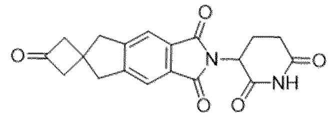 ,

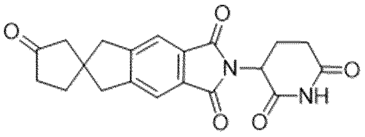 , 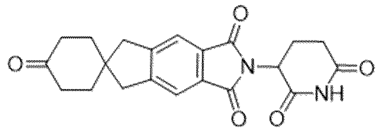 , 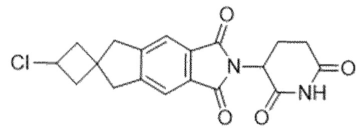 ,

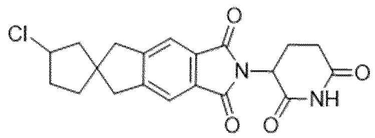 , 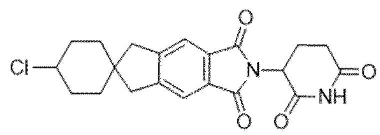 , 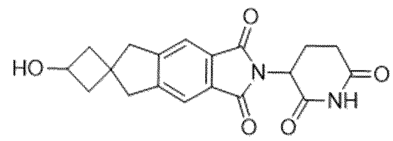 ,

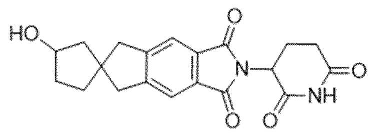 ,和 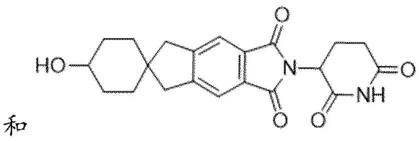 。

A second aspect of the present invention discloses a compound, wherein the compound has the following chemical structure:

CLM-L-PTM,

or an isomer, an isotopic derivative, a polymorph, a prodrug, or a pharmaceutically acceptable salt or a solvate thereof, wherein:

PTM is a target protein binding moiety;

L is a bond or chemical linker moiety covalently connecting the CLM and the PTM, and

CLM is a cereblon E3 ubiquitin ligase binding moiety selected from the following structures:

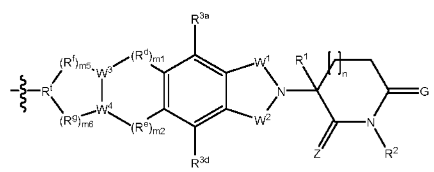 , 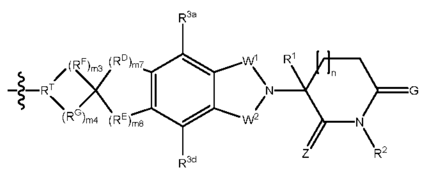 ,

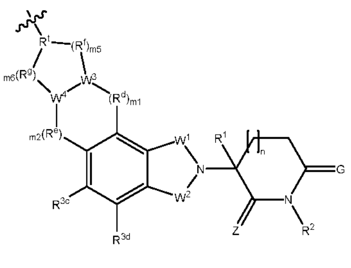 , 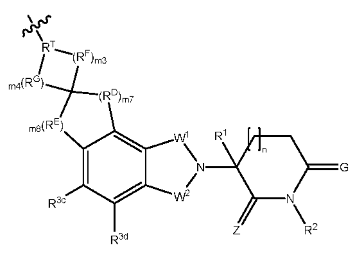 , 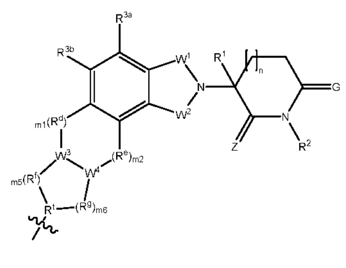 ,

and

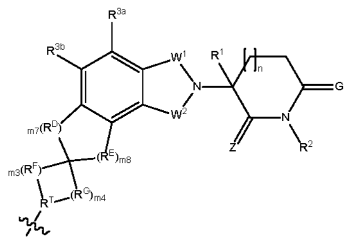

wherein:

$W^1$ and $W^2$ are identical or different, and are each independently $CR^aR^b$ or C(=O), and at least one of $W^1$ and $W^2$ is C(=O);
G and Z are identical or different, and are each independently selected from O, S, and Se;
$R^{3a}$ , $R^{3b}$ , $R^{3c}$ , and $R^{3d}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkyloxyacyl, alkylaminoacyl, cycloalkyl, heterocyclyl, aryl and heteroaryl, wherein the alkyl, heteroalkyl, alkenyl, alkynyl, alkoxy, cycloalkyl, heterocyclyl, aryl and heteroaryl are each independently optionally substituted with one or more substituents selected from the group consisting of halogen, alkyl, heteroalkyl, alkoxyl, haloalkyl, hydroxyl, hydroxyalkyl, cyano, amino, nitro, cycloalkyl, heterocyclyl, alkenyl, alkynyl, alkylamino, alkylacyl, alkyloxyacyl, alkylaminoacyl, aryl and heteroaryl;
$R^d$ , $R^e$, $R^f$, $R^g$ , $R^D$, $R^E$, $R^F$ , and $R^G$ at each occurrence are each independently $C(R^m)_2$, $NR^m$, C(=O), O, or S;
$W^3$ and $W^4$ at each occurrence are each independently $CR^m$ or N;
$R^t$ and $R^T$ at each occurrence are each independently N or $CR^{2h}$, and when $R^D$, $R^E$, $R^F$, and $R^G$ are all $C(R^m)_2$, $R^T$ is $CR^{2h}$;
m1 and m2 at each occurrence are each independently an integer of 0, 1, 2, 3, 4, 5, or 6, and m1+m2≤6;
m3 at each occurrence is each independently an integer of 0, 1, 2, 3, 4, 5, 6, or 7, and m4 is an integer of 1, 2, 3, 4, 5, 6, 7, or 8, and m3+m4≤8;
m5 and m6 at each occurrence are each independently an integer of 0, 1, 2, 3, 4, 5, 6, or 7, and m5+m6≤7;
m7 and m8 at each occurrence are each independently an integer of 0, 1, 2, 3, 4, 5, 6, or 7, and m7+m8≤7;
at each occurrence is each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkyloxyacyl, alkylaminoacyl, cycloalkyl, heterocyclyl, aryl and heteroaryl, wherein the alkyl , heteroalkyl, alkenyl, alkynyl, alkoxy, cycloalkyl, heterocyclyl, aryl and heteroaryl are each independently optionally substituted with one or more substituents selected from the group consisting of halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkyloxyacyl, alkylaminoacyl, aryl and heteroaryl;
$R^{2h}$ is selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano , amino, alkylamino, alkylacyl, alkyloxyacyl, alkylaminoacyl, cycloalkyl, heterocyclyl, aryl, heteroaryl, alkenyl and alkynyl, wherein the alkyl, heteroalkyl, alkoxy, cycloalkyl, heterocyclyl, aryl, and heteroaryl are each independently optionally substituted with one or more substituents selected from the group consisting of halogen, alkyl, heteroalkyl, alkenyl, alkynyl, alkoxy, haloalkyl, hydroxyl, hydroxyalkyl, cyano, amino, nitro, cycloalkyl, heterocyclyl, alkylamino, alkylacyl, alkyloxyacyl, alkylaminoacyl, aryl, and heteroaryl;
$R^1$ is selected from H, halogen, deuterium atom, $C_1$ -$C_6$ alkyl, $C_1$ -$C_6$ alkoxy, hydroxyl, cycloalkyl, $C_1$-$C_6$ haloalkyl, and hydroxyalkyl;
$R^2$, $R^a$, and $R^b$ are each independently selected from H, $C_1$ -$C_3$ alkyl, $C_3$ -$C_6$ cycloalkyl, and $C_1$ -$C_6$ alkoxy; and
n is 0, 1, 2 or 3.

In one embodiment, $W^1$ and $W^2$ are identical or different, and are each independently $CH_2$ or C(=O), and at least one of $W^1$ and $W^2$ is C(=O).

In another embodiment, $R^{3a}$, $R^{3b}$, $R^{3c}$, and $R^{3d}$ are each independently selected from H, deuterium atom, halogen, $C_1$ -$C_6$ alkyl, heteroalkyl, $C_2$ -$C_6$ alkenyl, $C_2$ -$C_6$ alkynyl, $C_1$ -$C_6$ alkoxy, $C_1$ -$C_6$ haloalkyl, C -$C_6$ haloalkoxy, hydroxyl, $C_1$ -$C_6$ hydroxyalkyl, nitro, cyano, amino, $C_3$ -$C_8$ cycloalkyl, $C_4$ -$C_{10}$ heterocyclyl, $C_6$ -$C_{10}$ aryl, and $C_5$ -$C_{10}$ heteroaryl, wherein the $C_1$-$C_6$ alkyl, $C_1$-$C_6$ heteroalkyl, $C_2$ -$C_6$ alkenyl, $C_2$ -$C_6$ alkynyl, $C_1$ -$C_6$ alkoxy, $C_3$ -$C_8$ cycloalkyl, $C_4$ -$C_{10}$ heterocyclyl, $C_6$ -$C_{10}$ aryl and $C_5$ -$C_{10}$ heteroaryl are each independently optionally substituted with one or more substituents selected from the group consisting of halogen, $C_1$ -$C_6$ alkyl, $C_1$ -$C_6$ heteroalkyl, $C_1$ -$C_6$ alkoxy, $C_1$ -$C_6$ haloalkyl, hydroxyl, $C_1$ -$C_6$ hydroxyalkyl, cyano, amino, nitro, $C_3$ -$C_8$ cycloalkyl, $C_4$-$C_{10}$ heterocyclyl, $C_6$-$C_{10}$ aryl and

$C_5$-$C_{10}$ heteroaryl, preferably $R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkyl, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently substituted with one or more of the substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl ; preferably $R^{3a}$ and $R^{3b}$ are each independently selected from H, deuterium atom, halogen, $C_1$-$C_3$ alkyl, and $C_1$-$C_3$ alkoxy, preferably $R^{3c}$ and $R^{3d}$ are each independently selected from H, deuterium atom, F, Cl, Br, I, $C_1$-$C_3$ alkyl, and $C_1$-$C_3$ alkoxy.

In another embodiment, at each occurrence, $R^d$, $R^e$, $R^f$ and $R^g$ at each occurrence are each independently $C(R^m)_2$ or O.

In another embodiment, at each occurrence, $R^D$ , $R^E$ , $R^F$ and $R^G$ at each occurrence are each independently $C(R^m)_2$ or O.

In another embodiment, $W^3$ and $W^4$ are CH.

In another embodiment, $R^{2h}$ is selected from H, deuterium atom, halogen, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl , $C_4$-$C_{10}$ heterocyclyl, $C_6$-$C_{10}$ aryl and $C_5$-$C_{10}$ heteroaryl are each independently optionally substituted with one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; preferably $R^{2h}$ is selected from H, deuterium atom, F, Cl, Br, I, $C_1$-$C_6$ alkyl, $C_1$-$C_6$ deuterated alkyl, $C_1$-$C_6$ heteroalkyl, $C_2$- $C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$- $C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$- $C_{10}$ heterocyclyl, $C_6$-$C_{10}$ aryl and $C_5$-$C_{10}$ heteroaryl are each independently substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; preferably $R^{2h}$ is selected from H, deuterium atom, halogen, $C_1$-$C_3$ alkyl, and $C_1$-$C_3$ alkoxy; preferably $R^{2h}$ is selected from H, deuterium atom, F, Cl, Br, I, $C_1$-$C_3$ alkyl, $C_1$-$C_3$ haloalkyl, $C_1$-$C_3$ alkoxy, and $C_1$-$C_3$ haloalkoxy.

In another embodiment, m1 and m2 at each occurrence are each independently an integer of 0, 1, 2, or 3, and $m1+m2\leq3$, preferably m1+m2=1 or m1+m2=2.

In another embodiment, m3 at each occurrence is independently an integer of 0, 1, 2, 3, or 4, and m4 is an integer of 1, 2, 3, 4, or 5, and $m3+m4\leq5$, preferably m3+m4=2, m3+m4=3 or m3+m4=4.

In another embodiment, at each occurrence, m5 and m6 at each occurrence are each independently an integer of 0, 1, 2, 3 or 4, and $m5+m6\leq4$, preferably m5+m6=2 or m5+m6=3.

In another embodiment, m7 and m8 at each occurrence are each independently an integer of 0, 1, 2, 3 or 4, and $m7+m8\leq4$, preferably m7+m8=2, or m7+m8=3.

In another embodiment, at each occurrence is each independently selected from H, deuterium atom, halogen, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; preferably, at each occurrence is independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl , $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; preferably at each occurrence is independently selected from H, deuterium atom, halogen, $C_1$-$C_3$ alkyl, and $C_1$-$C_3$ alkoxy.

In another embodiment, $R^1$ is selected from H, halogen, $C_1$-$C_3$ alkyl, and hydroxyl; preferably, $R^1$ is selected from H, F, Cl, Br, I, $C_1$-$C_3$ alkyl, and hydroxyl.

In another embodiment, $R^2$ is selected from H, and $C_1$-$C_3$ alkyl.

In another embodiment, n is 0 or 1.

In another embodiment, said CLM is selected from the following structures:

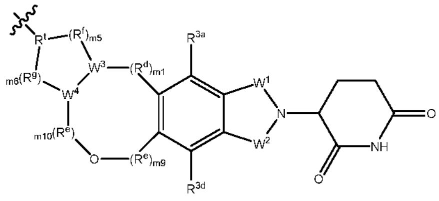
,
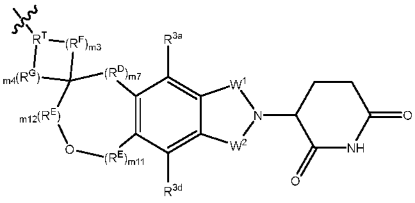
,

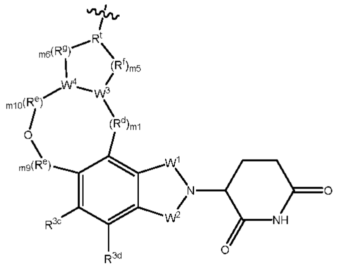
,
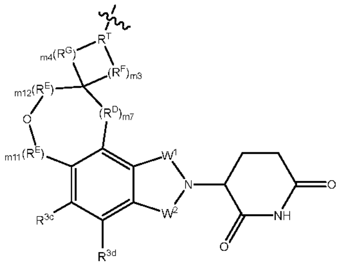
,
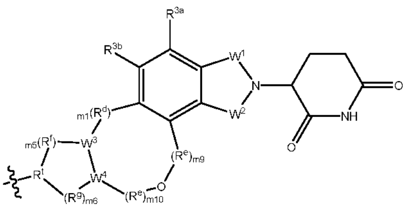
,

and

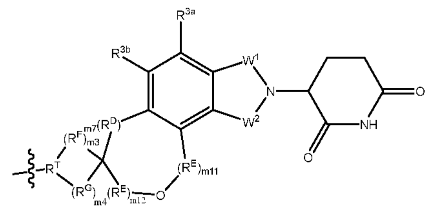

wherein:

$W^1$, $W^2$, $W^3$, $W^4$, $R^{3a}$, $R^{3b}$, $R^{3c}$, $R^{3d}$, $R^d$, $R^e$, $R^f$, $R^t$, $R^g$, $R^D$, $R^E$, $R^F$, $R^T$, $R^G$, m3, m4, m5 and m6 are as defined in claim 9, 10 or 11, preferably, $R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl;

m1, m9 and m10 at each occurrence are each independently an integer of 0, 1, 2, 3, 4, or 5, and $m1+m9+m10 \leq 5$; preferably, m1, m9 and m10 at each occurrence are each independently an integer of 0, 1, or 2, and $m1+m9+m10 \leq 2$, preferably $m1+m9+m10=1$ or $m1+m9+m10=0$; and

m7, m11 and m12 at each occurrence are each independently an integer of 0, 1, 2, 3, 4, 5, or 6, and $m7+m11+m12 \leq 6$; preferably, m7, m11 and m12 at each occurrence are each independently an integer of 0, 1, 2, or 3, and $m7+m11+m12 \leq 3$, preferably $m7+m11+m12=2$ or $m7+m11+m12=1$.

In another embodiment, the CLM is selected from the following structures:

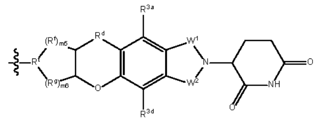
,
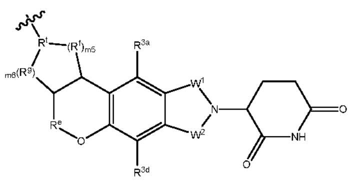
,
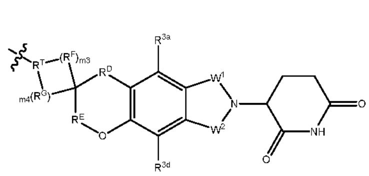
,

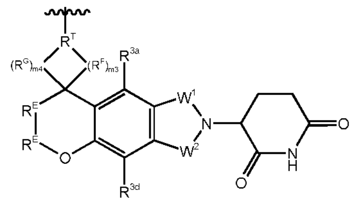,
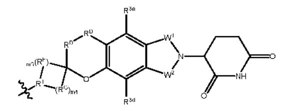,
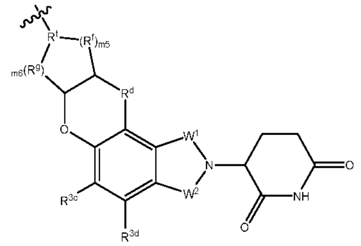,

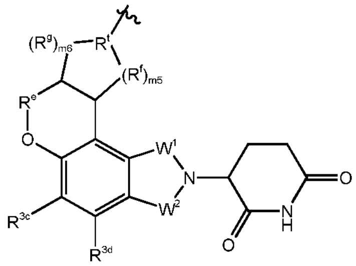,
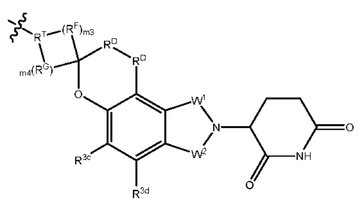,
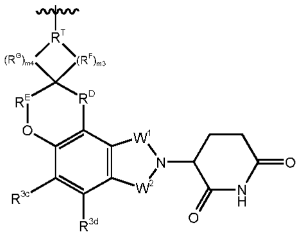, ,

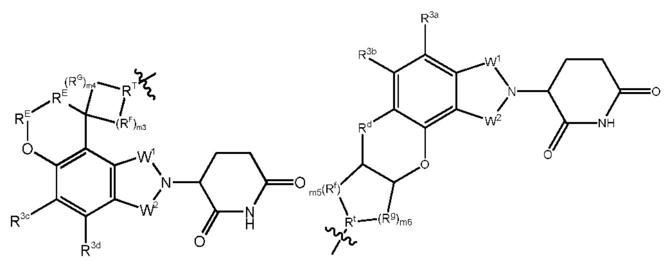,
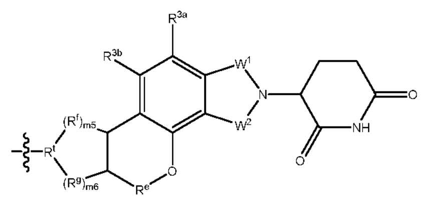,

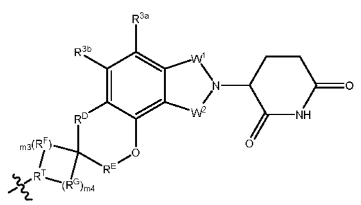,
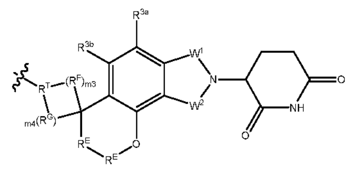,
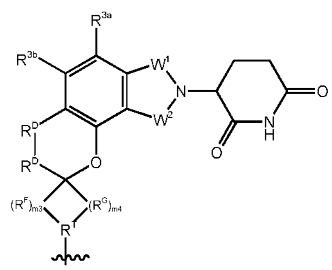,

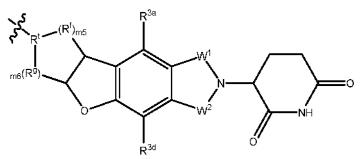,
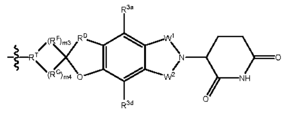,
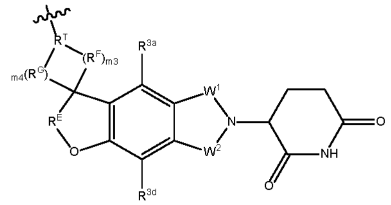,

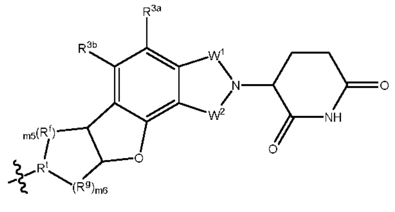,
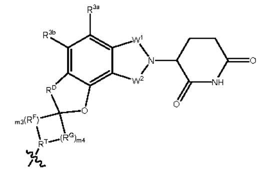,
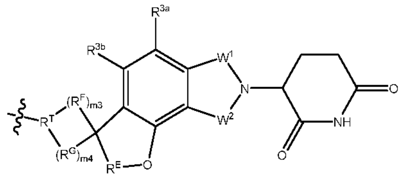,

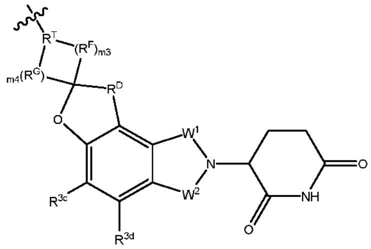,
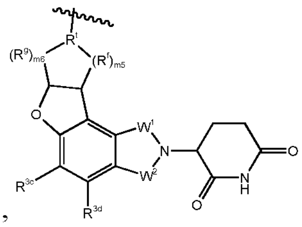, and
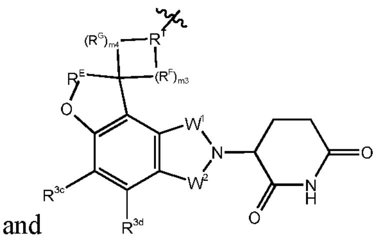

wherein:

$W^1$, $W^2$, $R^{3a}$, $R^{3b}$, $R^{3c}$, $R^{3d}$, $R^d$, $R^e$, $R^f$, $R^t$, $R^g$, $R^D$, $R^E$, $R^F$, $R^T$, $R^G$, m3, m4, m5, and m6 are as defined previously.

In another embodiment, the CLM is selected from the following structures:

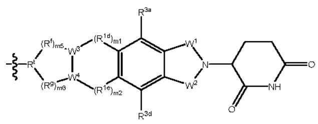,
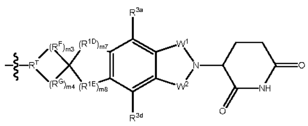,
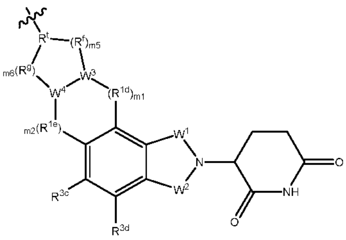,
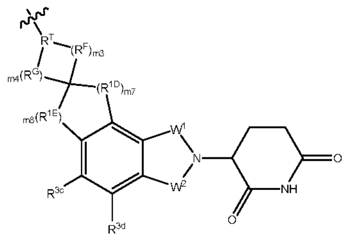,
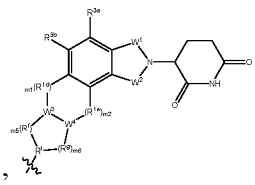, and
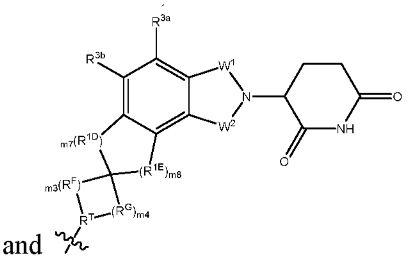

wherein:

$W^1$, $W^2$, $W^3$, $W^4$, $R^{3a}$, $R^{3b}$, $R^{3c}$, $R^{3d}$, $R^f$, $R^t$, $R^g$, $R^F$, $R^G$, m1, m2, m3, m4, m5, m6, m7, and m8 are as defined in claim 9, 10 or 11, preferably, $R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl;

$R^{1d}$, $R^{1e}$, $R^{1D}$ and $R^{1E}$ at each occurrence are each independently $C(R^m)_2$;

$R^T$ is N, or $CR^{2h}$, when both $R^F$ and $R^G$ are $C(R^m)_2$, $R^T$ is $CR^{2h}$;

$R^{2h}$, and $R^m$ are as defined previously.

In another embodiment, the CLM is selected from the following structures:

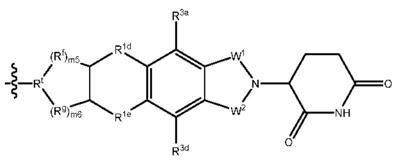,
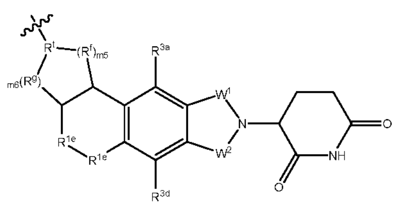,

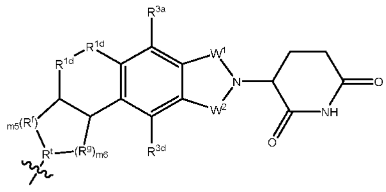,
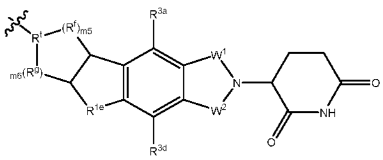,
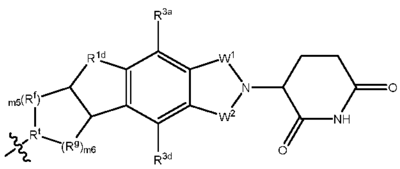,

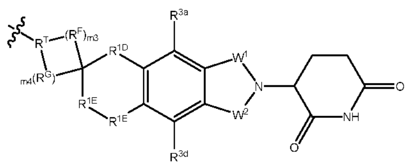,
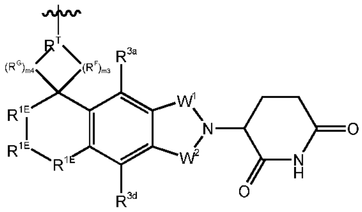,
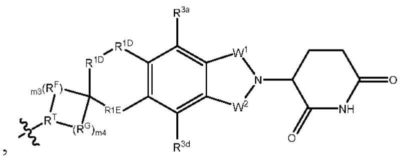,

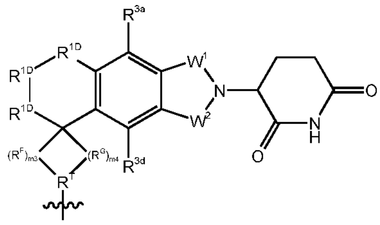,
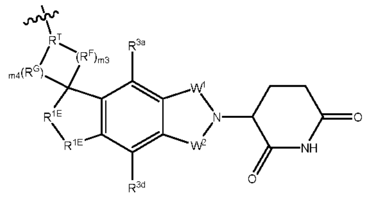,
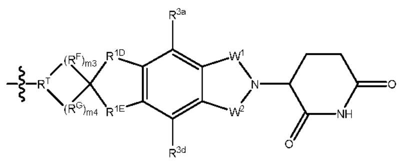,

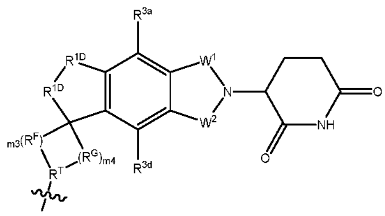,
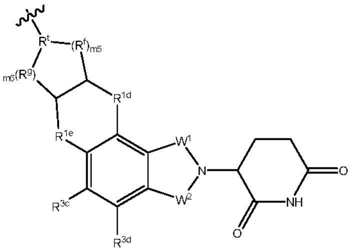,
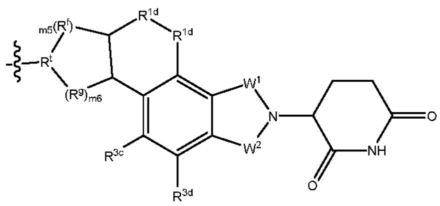,

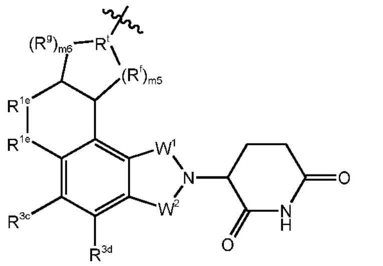,
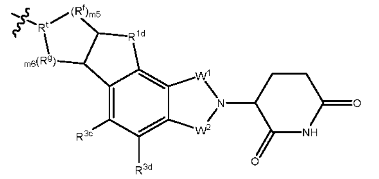,
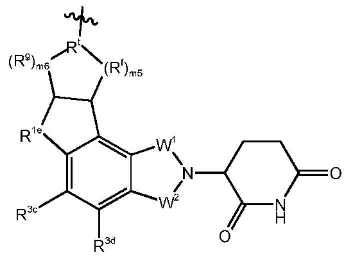,

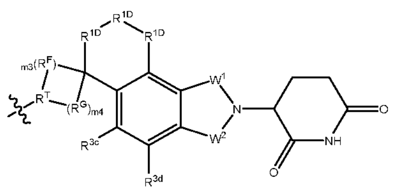,
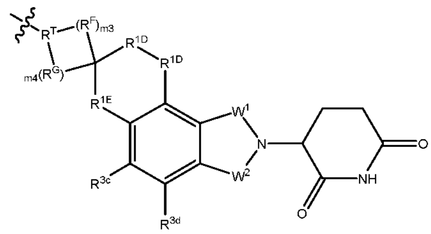,
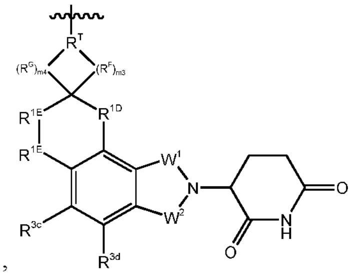,

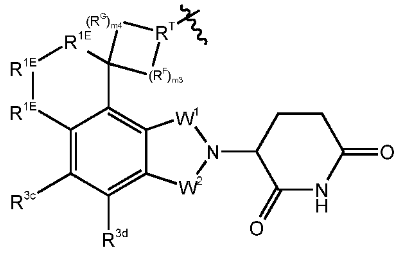 , 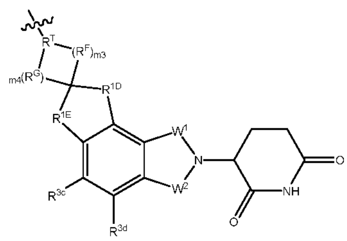 , 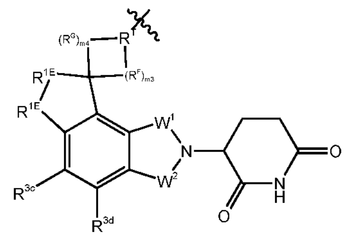 ,

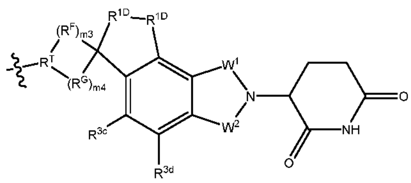 , 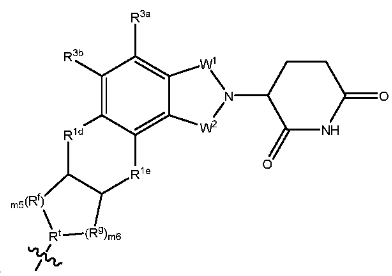 , 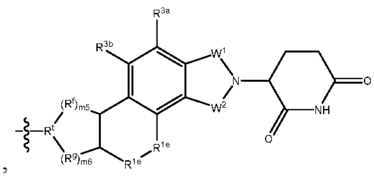 ,

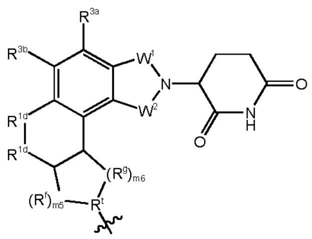 , 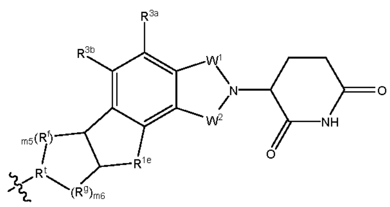 , 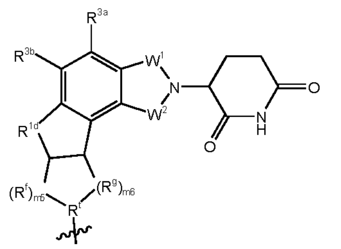 ,

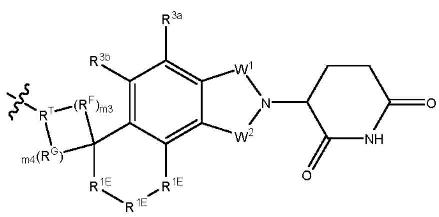 , 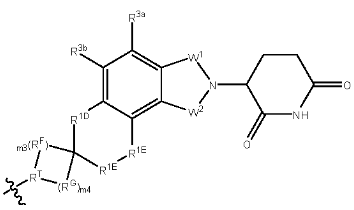 , 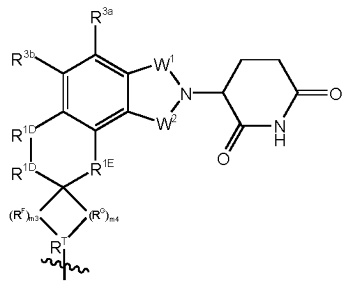 ,

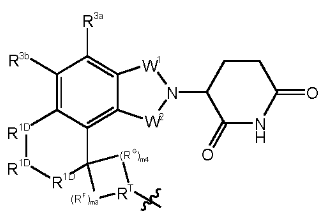 , 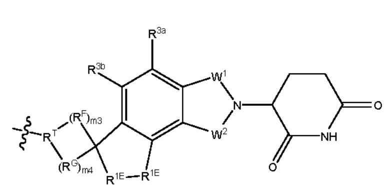 , 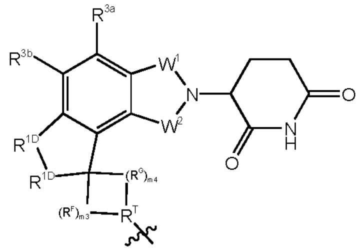

and

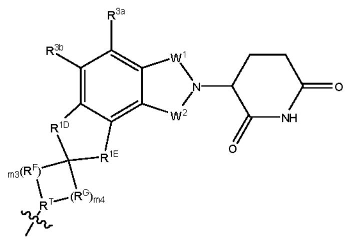
,

wherein $W^1$, $W^2$, $R^{3a}$, $R^{3b}$, $R^{3c}$, $R^{3d}$, $R^f$, $R^t$, $R^g$, $R^F$, $R^T$, $R^G$, $R^{1d}$, $R^{1e}$, $R^{1D}$, $R^{1E}$, m3, m4, m5, and m6 are as defined previously.

In another embodiment, the CLM is selected from the following structures:

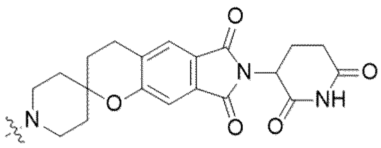
,
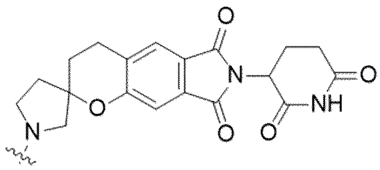
,
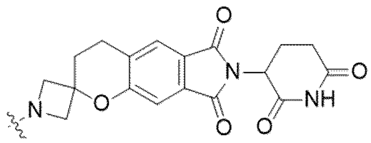
,
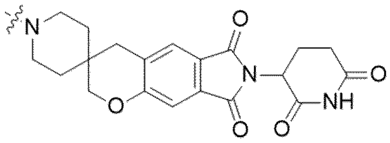
,
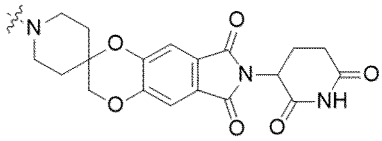
,
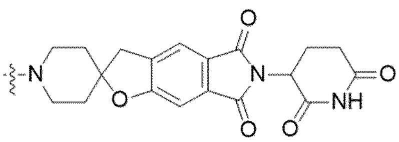
,
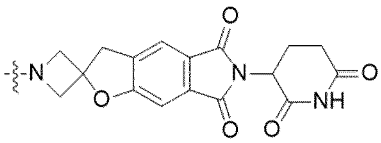
,
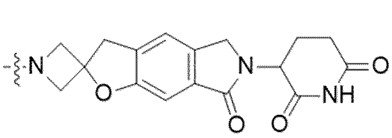
,
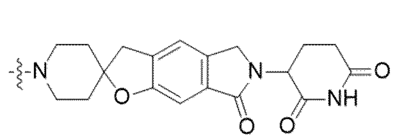
,
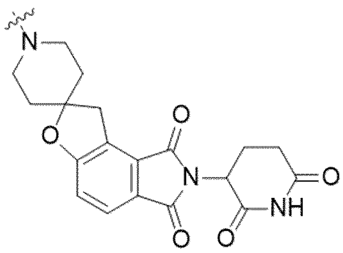
,
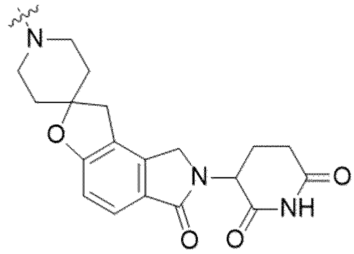
,
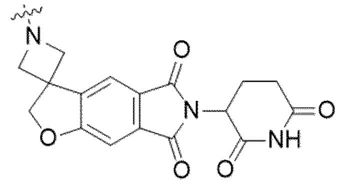
,
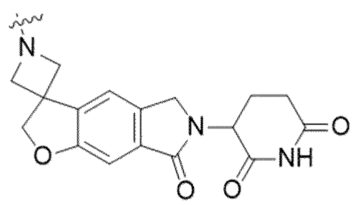
,
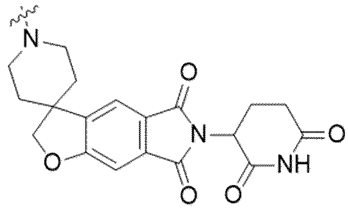
,
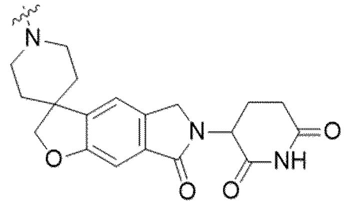
,

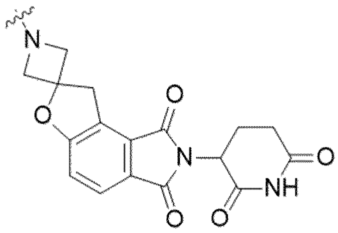 , 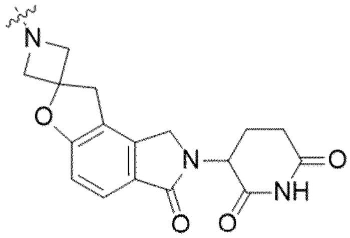 , 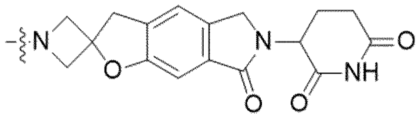 ,

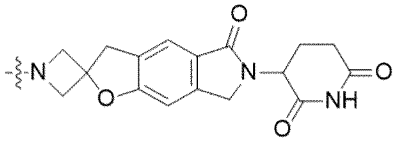 , 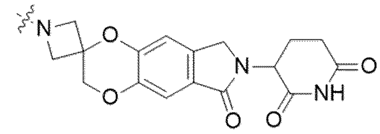 , 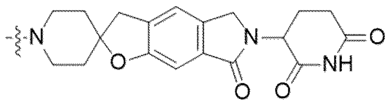 ,

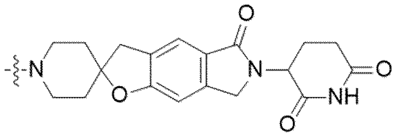 , 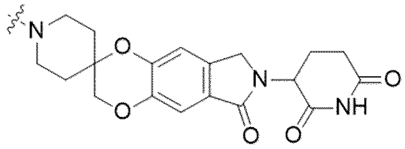 , 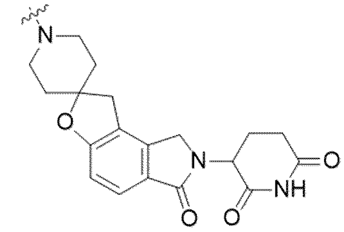 ,

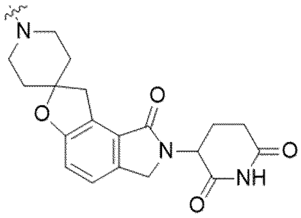 , 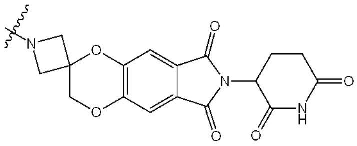 , 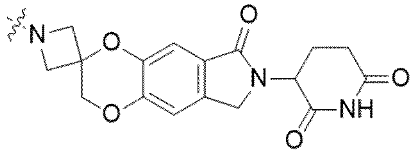 ,

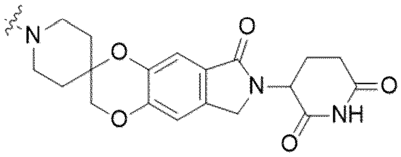 , 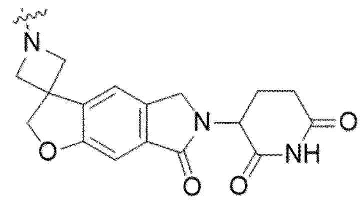 , 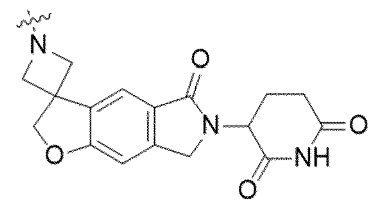 ,

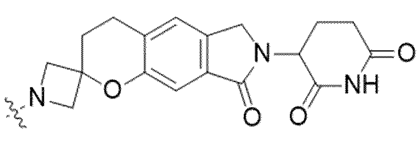 , 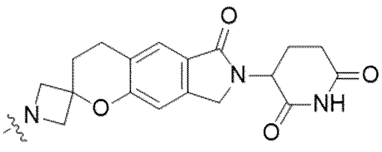 , 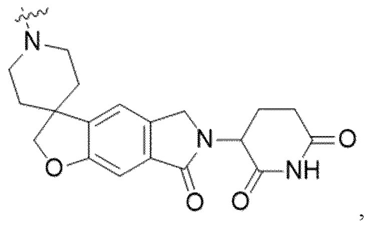 ,

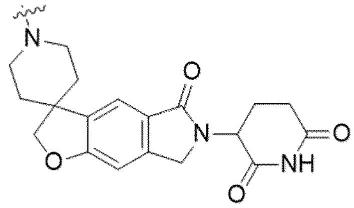 , 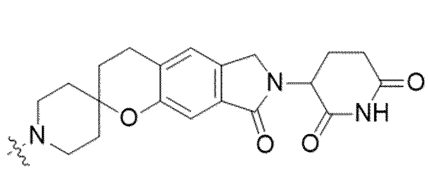 , 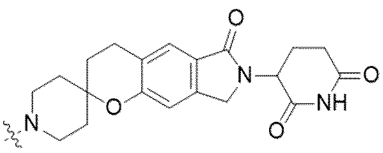 ,

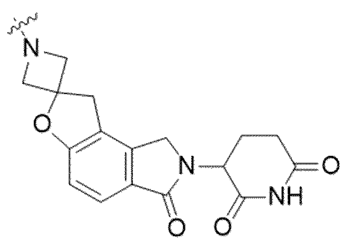 , 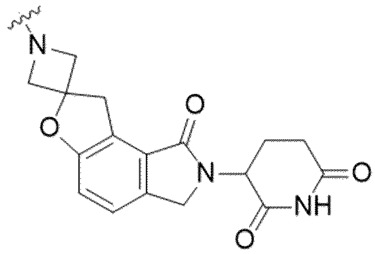 , 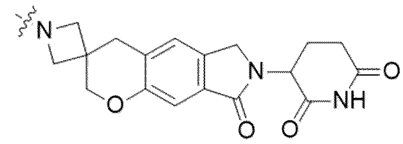 ,

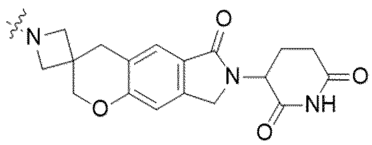 , 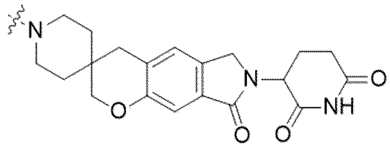 , 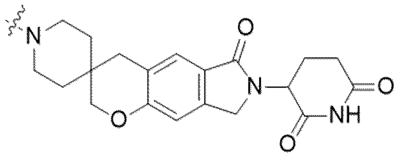 ,

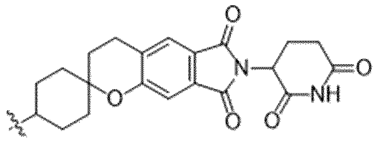 , 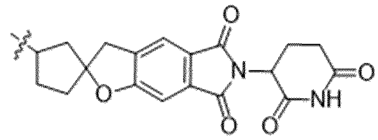 , 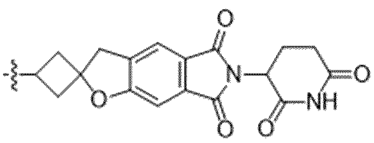 ,

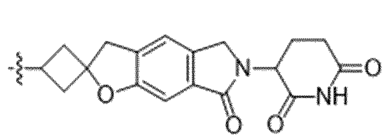 , 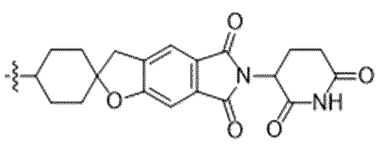 , 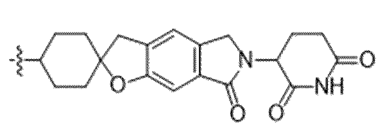 ,

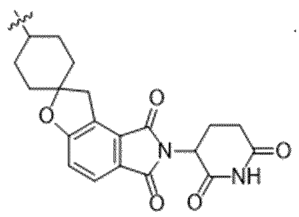 , 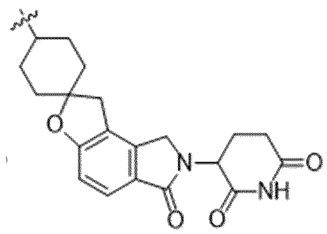 , 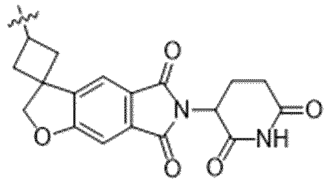 ,

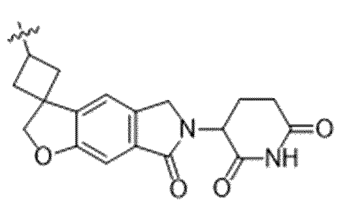 , 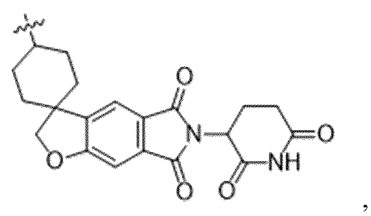 , 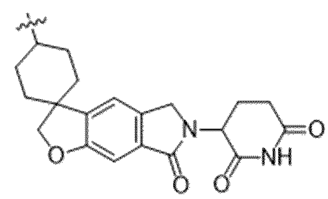 ,

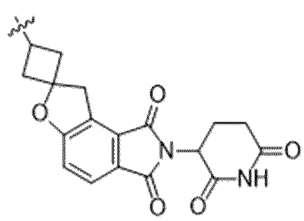 , 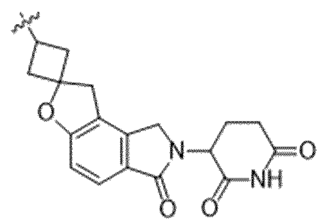 , 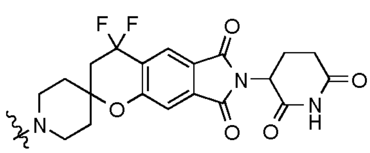 ,

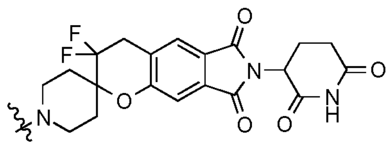, 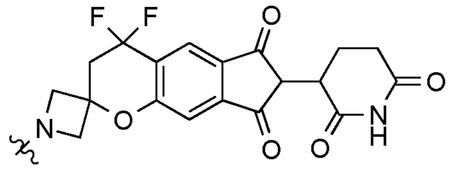, 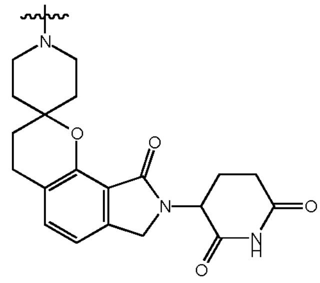,

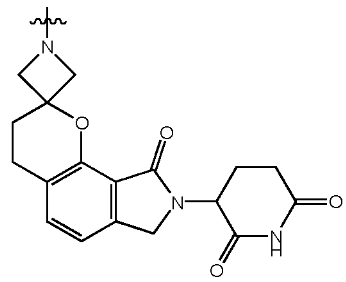, 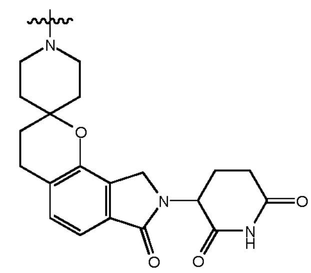, 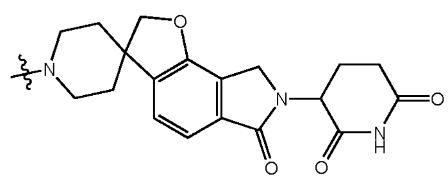,

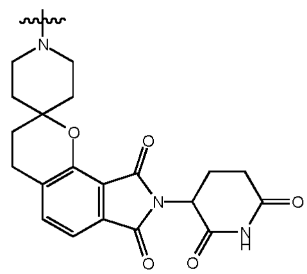, 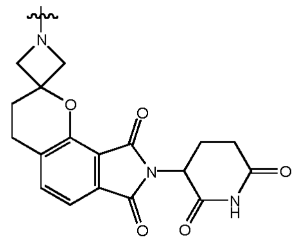, 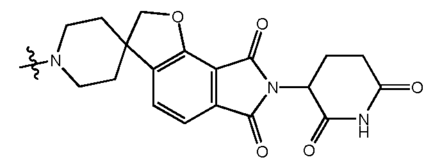,

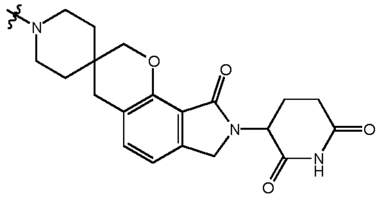, 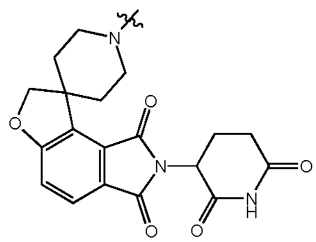, 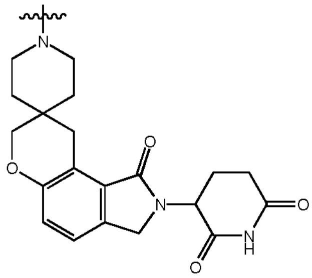,

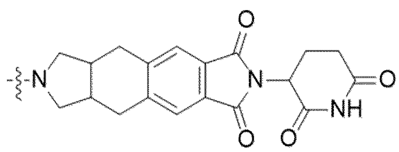, 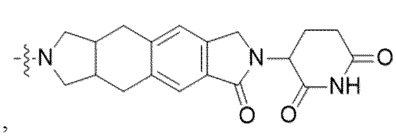, 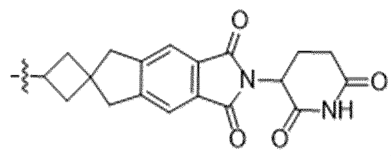,

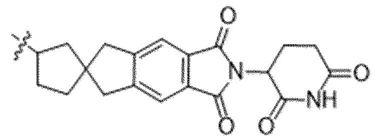, 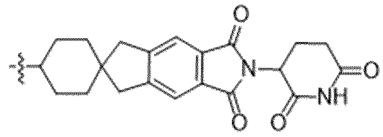, 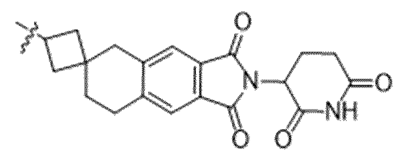,

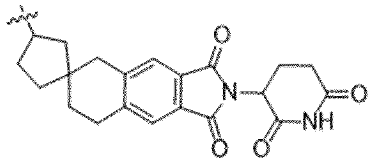,
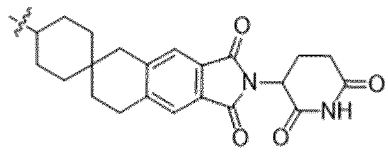,
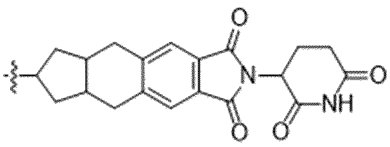,

and

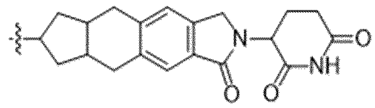;

In another embodiment, L is a bond or -(B )$^L_q$ -:

a bond or -($B^L$)$_q$-:

$B^L$ at each occurrence is identical or different, and is each independently selected from: $CR^{L1}R^{L2}$, O, S, SO, $SO_2$, $NR^{L3}$, $SO_2NR^{L3}$, $SONR^{L3}$, $CONR^{L3}$, $NR^{L3}CONR^{L4}$, $NR^{L3}SO_2NR^{L4}$, CO, $CR^{L1}$=$CR^{L2}$, C≡C, $SiR^{L1}R^{L2}$, P(O)$R^{L1}$, P(O)$OR^{L1}$, $NR^{L3}$C(=NCN)$NR^{L4}$, $NR^{L3}$C(=NCN), $NR^{L3}$C(=$CNO_2$)$NR^{L4}$, cycloalkylene, heterocyclylene, arylene, or heteroarylene, wherein the cycloalkylene, heterocyclylene, arylene, and heteroarylene are optionally substituted with 0-6 $R^{L1}$ and/or $R^{L2}$;

$R^{L1}$ , $R^{L2}$ , $R^{L3}$ , and $R^{L4}$ at each occurrence are independently selected from H, halogen, $C_{1-8}$ alkyl, O-$C_{1-8}$ alkyl, S-$C_{1-8}$ alkyl, NH-$C_{1-8}$ alkyl, N($C_{1-8}$ alkyl)$_2$ , $C_{3-11}$ cycloalkyl, aryl, heteroaryl, $C_{3-11}$ heterocyclyl, O-$C_{3-8}$ cycloalkyl, O-$C_{3-11}$ heterocyclyl, O-aryl, O-heteroaryl, S-$C_{3-8}$ cycloalkyl, NH-$C_{3-8}$ cycloalkyl, N ($C_{3-8}$ cycloalkyl)$_2$ , N($C_{3-8}$ cycloalkyl)($C_{1-8}$ alkyl), NH-$C_{3-8}$ heterocyclyl, N ($C_{3-8}$ heterocyclyl)$_2$ , N($C_{3-8}$ heterocyclyl)($C_{1-8}$ alkyl), NH-aryl, N(aryl)($C_{1-8}$ alkyl), NH-heteroaryl, N(heteroaryl)($C_{1-8}$ alkyl), OH, $NH_2$, SH, $SO_2$P(O)(O- $C_{1-8}$ alkyl)($C_{1-8}$ alkyl), P(O)(O-$C_{1-8}$ alkyl)$_2$, C≡C-$C_{1-8}$ alkyl, C≡CH, CH=CH-($C_{1-8}$ alkyl), C($C_{1-8}$ alkyl)=CH-($C_{1-8}$ alkyl), C($C_{1-8}$ alkyl)=C($C_{1-8}$ alkyl)$_2$, Si(OH)$_3$, Si($C_{1-8}$alkyl)$_3$ , Si(OH)($C_{1-8}$ alkyl)$_2$ , CO-$C_{1-8}$alkyl, $CO_2H$, CN, $CF_3$ , $CHF_2$, $CH_2F$, $NO_2$, $SF_5$, $SO_2$NH-$C_{1-8}$ alkyl, $SO_2$N($C_{1-8}$ alkyl)$_2$, SONH-$C_{1-8}$ alkyl, SON($C_{1-8}$ alkyl)$_2$ , CONH-$C_{1-8}$ alkyl, CON($C_{1-8}$ alkyl)$_2$ , N($C_{1-8}$ alkyl)CONH($C_{1-8}$ alkyl), N($C_{1-8}$ alkyl)CON($C_{1-8}$ alkyl)$_2$ , NHCONH($C_{1-8}$ alkyl), NHCON($C_{1-8}$ alkyl)$_2$ , $NHCONH_2$, N($C_{1-8}$ alkyl) SOzNH($C_{1-8}$ alkyl), N($C_{1-8}$ alkyl)$SO_2$N($C_{1-8}$ alkyl)$_2$ , $NHSO_2$NH($C_{1-8}$ alkyl), $NHSO_2$N($C_{1-8}$ alkyl)$_2$ , and $NHSO_2NH_2$, optionally, the $C_{1-8}$ alkyl, $C_{3-11}$ cycloalkyl, $C_{3-11}$ heterocyclyl, $C_{6-10}$ aryl, and $C_{5-10}$ heteroaryl are each independently substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, halocycloalkyl, haloheterocyclyl, alkylamino, aryl, heteroaryl, haloaryl, and haloheteroaryl; and

q is an integer greater than or equal to 1; preferably, q is 1, 2, 3, 4, 5, 6, 7, 8, 9, 10, 11, 12, 13, 14, 15, 16, 17, 18, 19 or 20.

In another embodiment, $B^L$ is selected from one or more of the following structures: -O-, -S-, -SO-,-$SO_2$-, -$CH_2$ -, -CO-, -NH-,

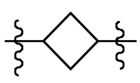, 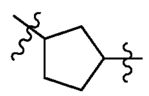, 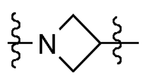, 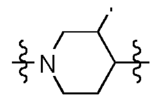, 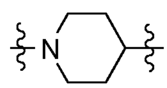,

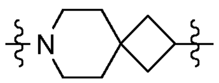, 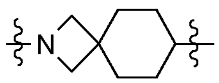, 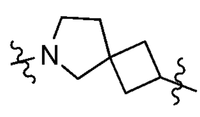, 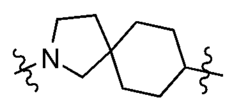, 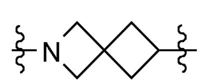,

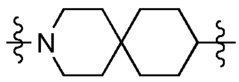, 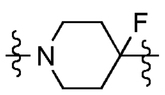, 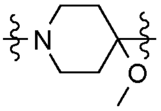, 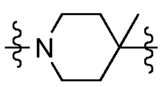, 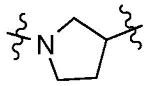, 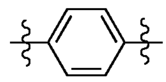,

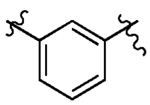 , 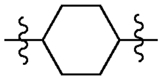 , 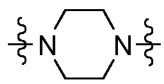 , 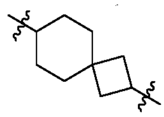 , 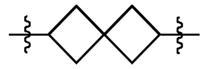 ,

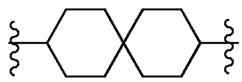 , 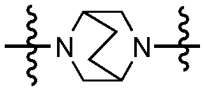 , 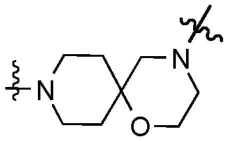 , 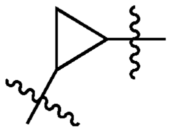

and

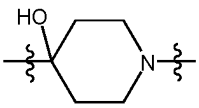

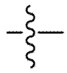

is the point of attachment.

In another embodiment, L is selected from the following structures:

covalent bond, $-(CH_2)_j-$, $-(CH_2)_p-NH-(CH_2)_s-$, $-(CH_2)_y-NH-(CH_2)_j-NH-(CH_2)_s-$, $-(CH_2)_p-CO-(CH_2)_s-$, $-(CH_2)_p-O-(CH_2)_s-$, $-(CH_2)_y-CO-(CH_2)_j-CO-(CH_2)_s-$, $-(CH_2)_y-O-(CH_2)_j-O-(CH_2)_s-$, $-(CH_2)_p-O-(CH_2)_j-CO-(CH_2)_s-$, $-(CH_2)_y-CO-(CH_2)_j-CO-(CH_2)_s-$, $-(CH_2)_p-NH-(CH_2)y-O-(CH_2)_j-CO-(CH_2)_s-$, $-(CH_2)_y-CO-(CH_2)_j-O-(CH_2)_s-NH-(CH_2)p-$,

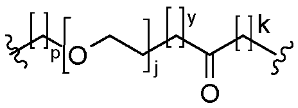 , 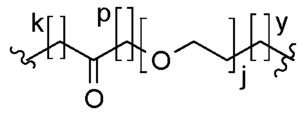 ,

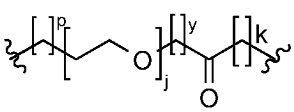 , 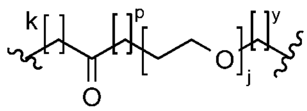 , 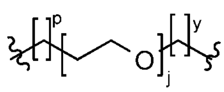 , 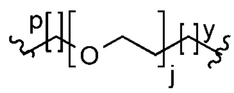 ,

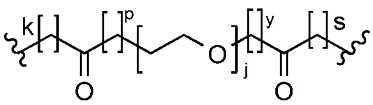 , 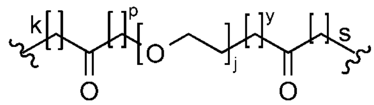 , 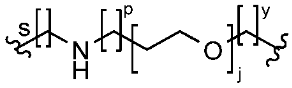 ,

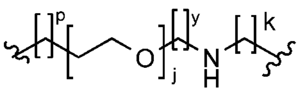 , 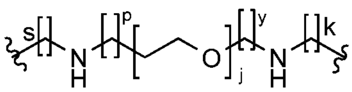 , 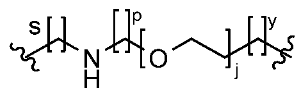 ,

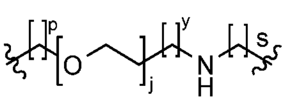 , 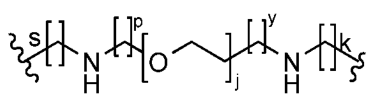 , 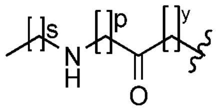 , 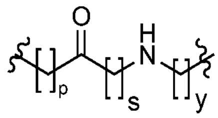 ,

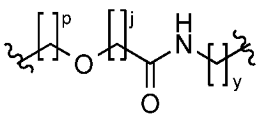 , 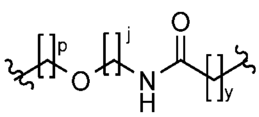 , 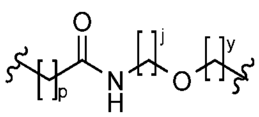 , 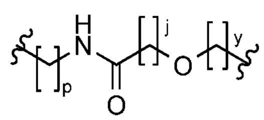 ,

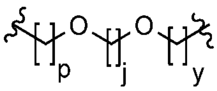,
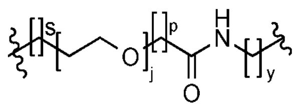,
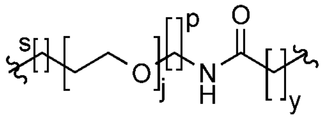,

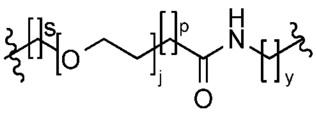,
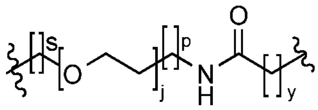,
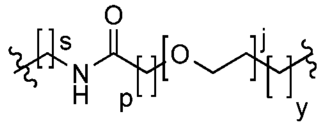,

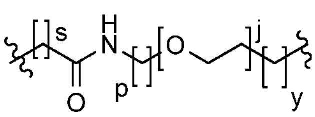,
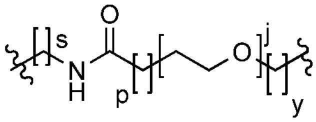,
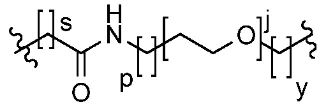,

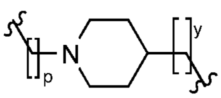,
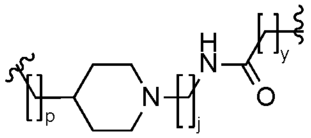,
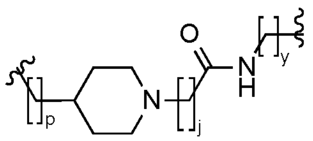,

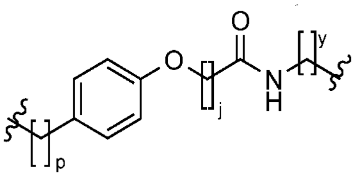,
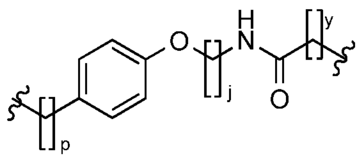,
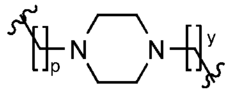,

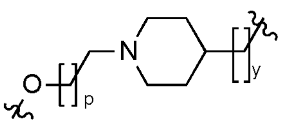,
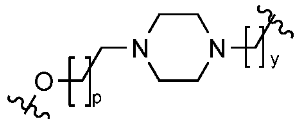,
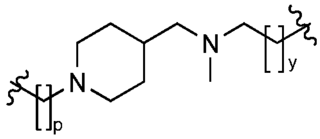,

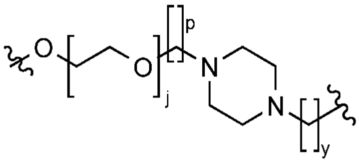,
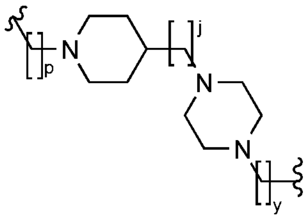,
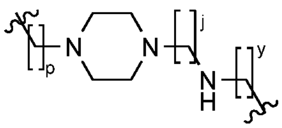,

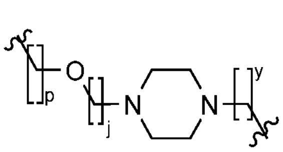,
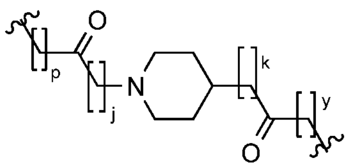,
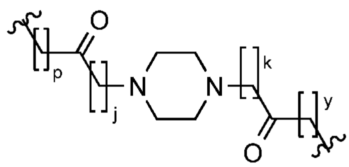,

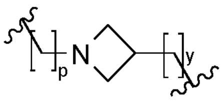,
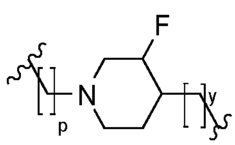,
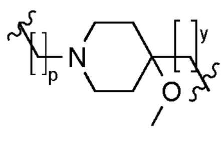,
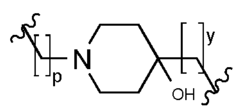,

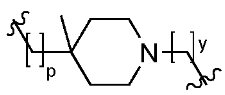 , 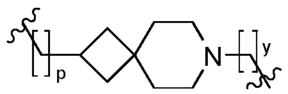 , 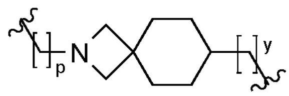 , 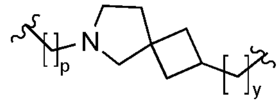 ,

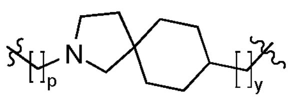 , 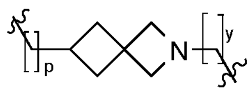 , 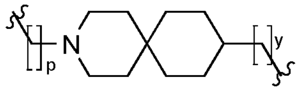 , 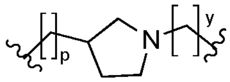 ,

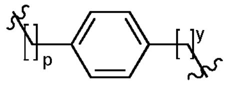 , 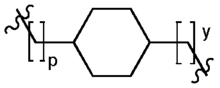 , 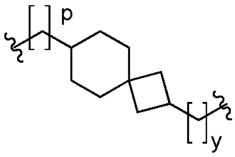 , 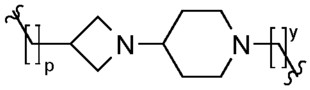 ,

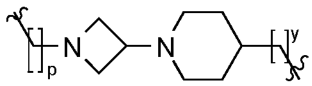 ,, 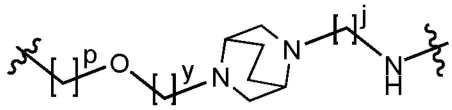 ,

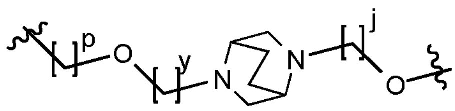 , 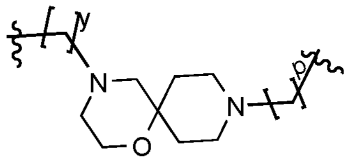 , 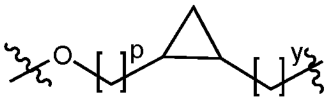 ,

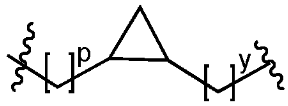 , 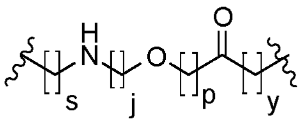 , 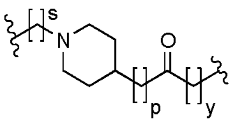 , 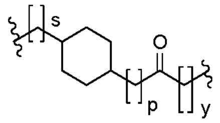 ,

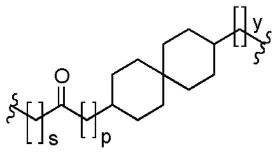 , 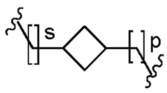 , 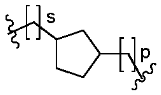 , 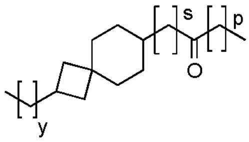 , 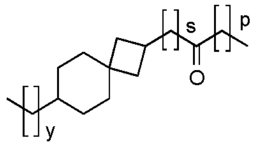 ,

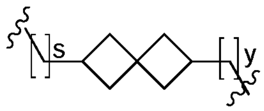 ,and 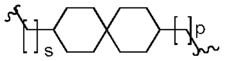 ;

where j is 1, 2, 3, 4, 5, 6, 7, 8, 9 or 10;

k, s, p and y are 0, 1, 2, 3, 4, 5, 6, 7, 8, 9 or 10;

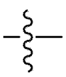

is the point of attachment with CLM or PTM;

Preferably L is selected from covalent bonds, $-CH_2-$,$-(CH_2)_2-$,$-(CH_2)_3-$,$-(CH_2)_4-$,$-(CH_2)_5-$,$-(CH_2)_6-$,$-(CH_2)_7-$, $-(CH_2)_8-$,$-NH-CH_2-$,$-NH-(CH_2)_2-$,$-NH-(CH_2)_3-$,$-NH-(CH_2)_4-$,$-NH-(CH_2)_5-$,$-NH-(CH_2)_6-$,$-NH-(CH_2)_7-$,$-NH-(CH_2)_8-$,$-CO-NH-CH_2-$,$-CO-NH-(CH_2)_2-$,$-CO-NH-(CH_2)_3-$,$-CO-NH-(CH_2)_4-$,$-CO-NH-(CH_2)_5-$,$-CO-NH-(CH_2)_6-$,$-CO-NH-(CH_2)_7-$,-CO-

NH-(CH$_2$)$_8$-,-CH$_2$-NH-,-(CH$_2$)$_2$-NH-,-(CH$_2$)$_3$-NH-,-(CH$_2$)$_4$-NH-,-(CH$_2$)$_5$-NH-,-(CH$_2$)$_6$-NH-,-(CH$_2$)$_7$-NH-,-(CH$_2$)$_8$-NH-,-NH-CH$_2$-NH-,-NH-(CH$_2$)$_2$-NH-,-NH-(CH$_2$)$_3$-NH-,-NH-(CH$_2$)$_4$-NH-,-NH-(CH$_2$)$_5$-NH-,-NH-(CH$_2$)$_6$-NH-,-NH-(CH$_2$)$_7$-NH-,-NH-(CH$_2$)$_8$-NH-,-(CH$_2$-CH$_2$-O)-CH$_2$-CH$_2$-,-(CH$_2$-CH$_2$-O)$_2$-CH$_2$-CH$_2$-,-(CH$_2$-CH$_2$-O)$_3$-CH$_2$-CH$_2$-,-NH-(CH$_2$-CH$_2$-O)-CH$_2$-CH$_2$-,-NH-(CH$_2$-CH$_2$-O)$_2$-CH$_2$-CH$_2$-,-NH-(CH$_2$-CH$_2$-O)$_3$-CH$_2$-CH$_2$-,-CO-NH-(CH$_2$-CH$_2$-O)-CH$_2$-CH$_2$-,-CO-NH-(CH$_2$-CH$_2$-O)$_2$-CH$_2$-CH$_2$-,-CO-NH-(CH$_2$-CH$_2$-O)$_3$-CH$_2$-CH$_2$-,-(CH$_2$-CH$_2$-O)-CH$_2$-CH$_2$-NH-,-(CH$_2$-CH$_2$-O)$_2$-CH$_2$-CH$_2$-NH-,-(CH$_2$-CH$_2$-O)$_3$-CH$_2$-CH$_2$-NH-,-NH-(CH$_2$-CH$_2$-O)-CH$_2$-CH$_2$-NH-,-NH-(CH$_2$-CH$_2$-O)$_2$-CH$_2$-CH$_2$-NH-,-NH-(CH$_2$-CH$_2$-O)$_3$-CH$_2$-CH$_2$-NH-,-CO-NH-(CH$_2$-CH$_2$-O)-CH$_2$-CH$_2$-NH-,-CO-NH-(CH$_2$-CH$_2$-O)$_2$-CH$_2$-CH$_2$-NH-,-CO-NH-(CH$_2$-CH$_2$-O)$_3$-CH$_2$-CH$_2$-NH-,-CH$_2$-CH$_2$-(O-CH$_2$-CH$_2$)-,-CH$_2$-CH$_2$-(O-CH$_2$-CH$_2$)$_2$-,-CH$_2$-CH$_2$-(O-CH$_2$-CH$_2$)$_3$-,-NH-CH$_2$-CH$_2$-(O-CH$_2$-CH$_2$)-,-NH-CH$_2$-CH$_2$-(O-CH$_2$-CH$_2$)$_2$-,-NH-CH$_2$-CH$_2$-(O-CH$_2$-CH$_2$)$_3$-,-CO-NH-CH$_2$-CH$_2$-(O-CH$_2$-CH$_2$)-,-CO-NH-CH$_2$-CH$_2$-(O-CH$_2$-CH$_2$)$_2$-,-CO-NH-CH$_2$-CH$_2$-(O-CH$_2$-CH$_2$)$_3$-,-CH$_2$-CH$_2$-(O-CH$_2$-CH$_2$)-NH-,-CH$_2$-CH$_2$-(O-CH$_2$-CH$_2$)$_2$-NH-,-CH$_2$-CH$_2$-(O-CH$_2$-CH$_2$)$_3$-NH-,-NH-CH$_2$-CH$_2$-(O-CH$_2$-CH$_2$)-NH-,-NH-CH$_2$-CH$_2$-(O-CH$_2$-CH$_2$)$_2$-NH-,-NH-CH$_2$-CH$_2$-(O-CH$_2$-CH$_2$)$_3$-NH-,-NH-CH$_2$-CH$_2$-O-CH$_2$-CH$_2$-CO-,-CO-CH$_2$-CH$_2$-O-CH$_2$-CH$_2$-NH-,-NH-(CH$_2$)$_4$-CO-,-NH-(CH$_2$)$_5$-CO-,-NH-(CH$_2$)$_6$-CO-,-CO-(CH$_2$)$_4$-NH-,-CO-(CH$_2$)$_5$-NH-,-CO-(CH$_2$)$_6$-NH-,-NH-(CH$_2$-CH$_2$-O)-(CH$_2$)$_3$-,-NH-(CH$_2$-CH$_2$-O)-(CH$_2$)$_4$-,-NH-(CH$_2$-CH$_2$-O)-(CH$_2$)$_s$-,-NH-(CH$_2$-CH$_2$-O)-(CH$_2$)$_6$-,-(CH$_2$)$_3$-(O-CH$_2$-CH$_2$)-NH-,-(CH$_2$)$_4$-(O-CH$_2$-CH$_2$)-NH-,-(CH$_2$)$_5$-(O-CH$_2$-CH$_2$)-NH-,-(CH$_2$)$_6$-(O-CH$_2$-CH$_2$)-NH-,-CH$_2$-CH$_2$-O-(CH$_2$)$_2$-CO-,-CH$_2$-CH$_2$-O-(CH$_2$)$_3$-CO-,-CH$_2$-CH$_2$-O-(CH$_2$)$_4$-CO-,-CO-(CH$_2$)$_2$-O-CH$_2$-CH$_2$-,-CO-(CH$_2$)$_3$-O-CH$_2$-CH$_2$-,-CO-(CH$_2$)$_4$-O-CH$_2$-CH$_2$-,-CO-(CH$_2$)$_2$-,-CO-(CH$_2$)$_3$-,-CO-(CH$_2$)$_4$-,-CO-(CH$_2$)$_5$-,-CO-(CH$_2$)$_6$-,-(CH$_2$)$_2$-CO-,-(CH$_2$)$_3$-CO-,-(CH$_2$)$_4$-CO-,-(CH$_2$)$_5$-CO-,-(CH$_2$)$_6$-CO-,-CO-(CH$_2$)$_2$-CO-,-CO-(CH$_2$)$_3$-CO-,-CO-(CH$_2$)$_4$-CO-,-CO-(CH$_2$)$_5$-CO-,-CO-(CH$_2$)$_6$-CO-,-CH$_2$-CO-CH$_2$-,-CH$_2$-CO-(CH$_2$)$_2$-,-CH$_2$-CO-(CH$_2$)$_3$-,-CH$_2$-CO-(CH$_2$)$_4$-,-(CH$_2$)$_2$-CO-CH$_2$-,-(CH$_2$)$_2$-CO-(CH$_2$)$_2$-,-(CH$_2$)$_2$-CO-(CH$_2$)$_3$-,-(CH$_2$)$_2$-CO-(CH$_2$)$_4$-,-(CH$_2$)$_3$-CO-CH$_2$-,-(CH$_2$)$_3$-CO-(CH$_2$)$_2$-,-(CH$_2$)$_3$-CO-(CH$_2$)$_3$-,-(CH$_2$)$_3$-CO-(CH$_2$)$_4$-,-(CH$_2$)$_4$-CO-CH$_2$-,-(CH$_2$)$_4$-CO-(CH$_2$)$_2$-,-(CH$_2$)$_4$-CO-(CH$_2$)$_3$-,-(CH$_2$)$_4$-CO-(CH$_2$)$_4$-,-CH$_2$-O-CH$_2$-,-CH$_2$-O-(CH$_2$)$_2$-,-CH$_2$-O-(CH$_2$)$_3$-,-CH$_2$-O-(CH$_2$)$_4$-,-(CH$_2$)$_2$-O-CH$_2$-,-(CH$_2$)$_2$-O-(CH$_2$)$_2$-,-(CH$_2$)$_2$-O-(CH$_2$)$_3$-,-(CH$_2$)$_2$-O-(CH$_2$)$_4$-,-(CH$_2$)$_3$-O-CH$_2$-,-(CH$_2$)$_3$-O-(CH$_2$)$_2$-,-(CH$_2$)$_3$-O-(CH$_2$)$_3$-,-(CH$_2$)$_3$-O-(CH$_2$)$_4$-,-(CH$_2$)$_4$-O-CH$_2$-,-(CH$_2$)$_4$-O-(CH$_2$)$_2$-,-(CH$_2$)$_4$-O-(CH$_2$)$_3$-,-(CH$_2$)$_4$-O-(CH$_2$)$_4$-,

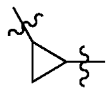, 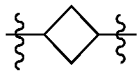, 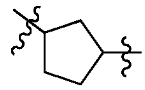,

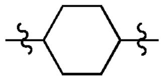, 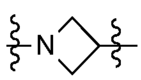, 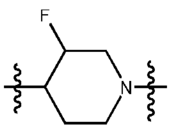, 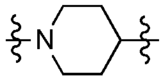, 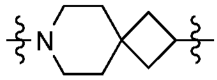, 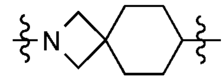,

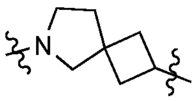, 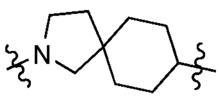, 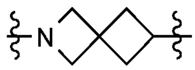, 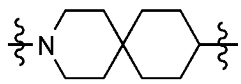, 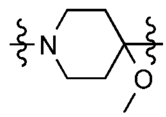,

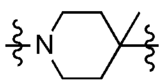, 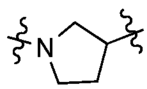, 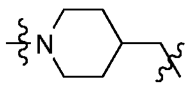, 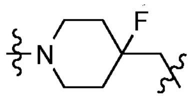, 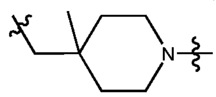, 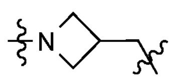,

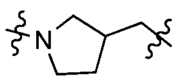, 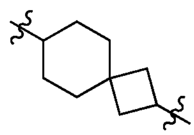, 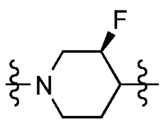, 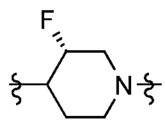, 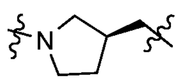, 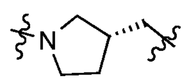,

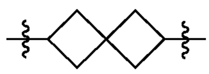, 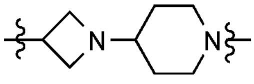, 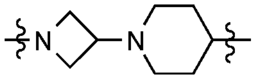, 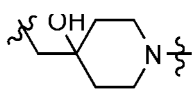, 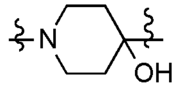,

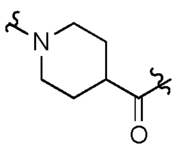 , 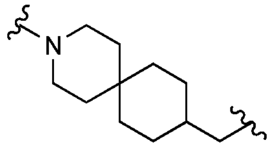 , 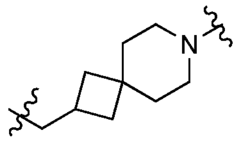 , 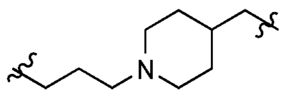 ,

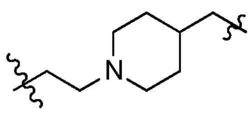 , 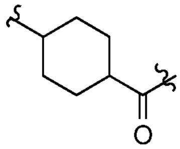 , 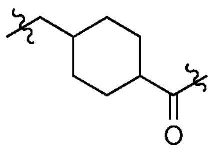 , 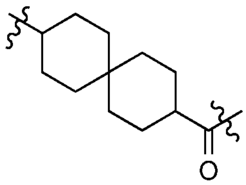 , 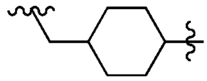 ,

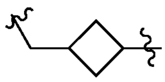 , 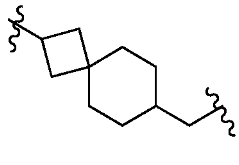 , 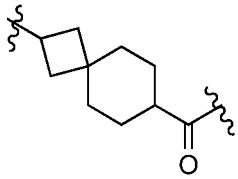 , 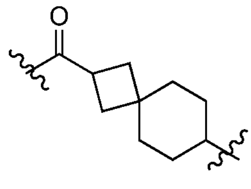 ,

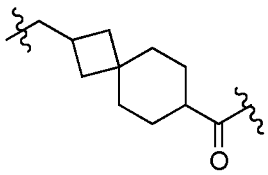 , 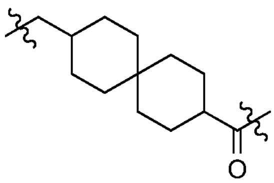 , 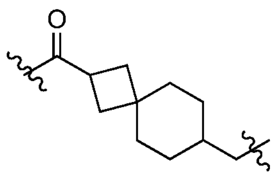 , 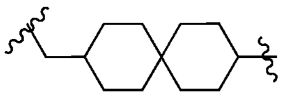 ,

and

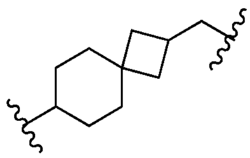 ;

In another embodiment, the PTM is a moiety that binds to a target protein or polypeptide, wherein the target protein is selected from the following proteins: structural proteins, receptors, enzymes, cell surface proteins; proteins pertinent to the integrated function of a cell, including proteins involved in catalytic activity, aromatase activity, motor activity, helicase activity, metabolic process, antioxidant activity, proteolysis, biosynthesis; proteins with kinase activity, oxidoreductase activity, transferase activity, hydrolase activity, lyase activity, isomerase activity, ligase activity, enzyme regulator activity, signal transducer activity, structural molecule activity, binding activity, receptor activity, cell motility, membrane fusion, cell communication, regulation of biological process, development, cell differentiation, response to stimulus; behavioral proteins; cell adhesion proteins; proteins involved in cell death; and proteins involved in transportation, including proteins of protein transporter activity, nuclear transporter activity, ion transporter activity, channel transporter activity, carrier activity, permease activity, secretion activity, electron transporter activity, pathogenesis, chaperone regulator activity, nucleic acid binding activity, transcription regulator activity, extracellular tissues and biogenesis activity, and translation regulator activity.

In another embodiment, PTM is a moiety binding to a target protein or polypeptide, wherein the target protein is selected from the group consisting of B7.1 and B7, TNFR2, NADPH oxidase, BclIBax, and other conjugates in the apotosis pathway, C5a receptor, HMG-CoA reductase, PDE V phosphodiesterase type, PDEIV phosphodiesterase type 4, PDEI I, PDEI II, PDE III, squalene epoxidation enzyme, CXCR1, CXCR2, nitric oxide (NO) synthase, cyclo-oxygenase 1, cyclo-oxygenase 2, 5HT receptors, dopamine receptors, G proteins (ie Gq), histamine receptors, 5-lipoxygenase, tryptase serine protease, thymidylate synthase, purine nucleoside phosphorylase, GAPDH trypanosomal, glycogen phosphorylase, carbonic anhydrase, chemokine receptor, JAW STAT, RXR and its analog, HIV1 protease, HIV1 integrase, influenza neuraminidase, hepatitis B reverse transcriptase, sodium channel, multi drug resistant bacteria, protein P-glycoprotein, tyrosine kinases, CD23, CD124, tyrosine kinase p561ck , CD4, CD5, IL-2 receptor, IL-1 receptor, TNF-αR, ICAM1, $Ca^{2+}$

channels, VCAM, VLA-4 integrin, selectins, CD40/CD40L, inosine monophosphate dehydrogenase, p38 MAP kinase, RaslRaflMEWERK pathway, interleukin-1 converting enzyme, caspase, HCV, NS3 protease, HCV NS3 RNA helicase, glycinamide ribonucleotide formyl transferase, rhinovirus, 3C protease, herpes simplex virus-I (HSV-I), protease, cytomegalovirus (CMV) protease, poly(ADP-ribose) polymerase, cyclindependent kinase 4/6, vascular endothelial growth factor, oxytocin receptor, microsomal transfer protein inhibitor, bile acid transport inhibitor, 5α reductase inhibitors, angiotensin 11, glycine receptor, noradrenaline reuptake receptor, endothelin receptor, neuropeptide Y and receptor, adenosine receptors, adenosine kinase and AMP deaminase, purinergic receptors (P2Y1, P2Y2, P2Y4, P2Y6, P2X1-7), farnesyltransferases, geranylgeranyl transferase, TrkA a receptor for NGF, β-amyloid, tyrosine kinase Flk-II KDR, vitronectin receptor, integrin receptor, Her-21 neu, telomerase inhibition, cytosolic phospholipase A2, EGF receptor tyrosine kinase, ecdysone 20-monooxygenase, ion channel of GABA-gated chloride channel, acetylcholinesterase, voltage-sensitive sodium channel protein, calcium release channel and chloride ion channel, acetyl-CoA carboxylase, adenylosuccinate synthetase, protoporphyrinogen oxidase, enolpyruvylshikimate phosphate synthase, MYC protein, androgen receptor, estrogen receptor, aurora kinase A, Bcr-Abl protein, epidermal growth factor receptor, anaplastic lymphoma kinase, Bruton's tyrosine kinase, bromodomain protein 4.

In another embodiment, the PTM is an Hsp90 inhibitor, a kinase inhibitor, a phosphatase inhibitor, a MDM2 inhibitor, a compound that binds to a protein comprising the human BET bromodomain, a HDAC inhibitor, a human lysine methyltransferase inhibitor, a compound that binds to RAF receptor, a compound that binds to FKBP, an angiogenesis inhibitor, an immunosuppressive compound, an compound that binds to aryl hydrocarbon receptor, a compound that binds to androgen receptor, a compound that binds to estrogen receptors, a compound that binds to thyroid hormone receptor, a compound that binds to HIV protease, a compound that binds to HIV integrase, a compound that binds to HCV protease, a compound that binds to acyl protein thioesterase 1 and/or 2, a compound that binds to c-MYC protein, a compound that binds to laser kinase A, a compound that binds to Bcr-Abl protein, a compound that binds to bromodomain protein 4 (BRD4), a compound that binds to anaplastic lymphoma kinase (ALK), a compound that binds to Bruton's tyrosine kinase (BTK), a compound that binds to cyclin-dependent kinase 4/6 (CDK4/6), or a compound that binds to epidermal growth factor receptor.

In another embodiment, the PTM is a moiety binding to the androgen receptor.

In another embodiment, the PTM is selected from the following structures:

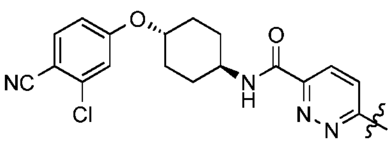
or
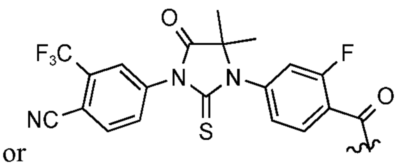

In another embodiment, the compound is selected from the following structures:

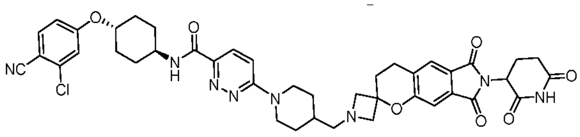
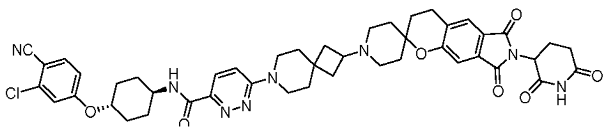
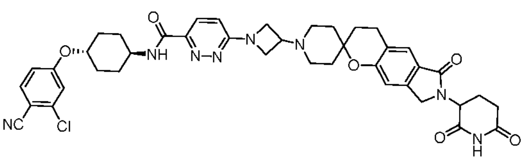
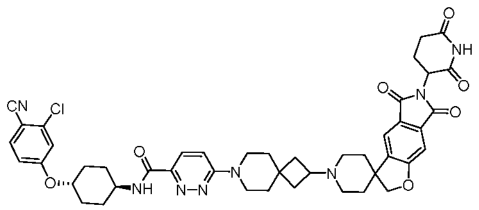
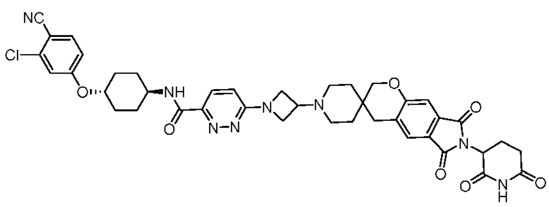
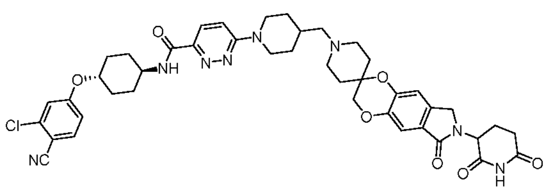

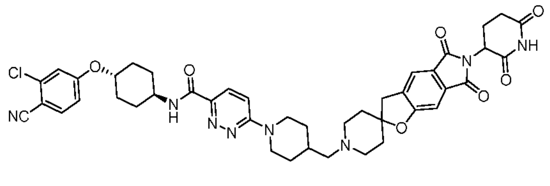

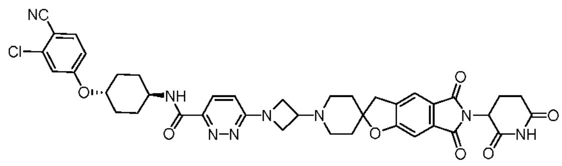

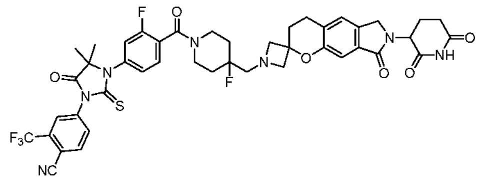

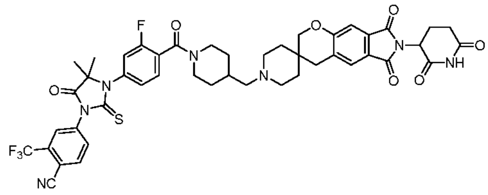

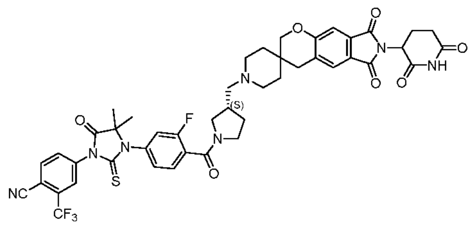

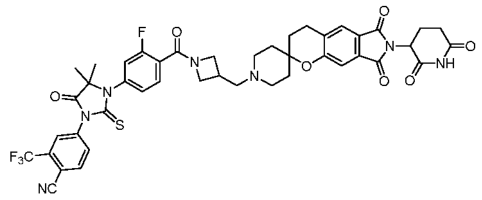

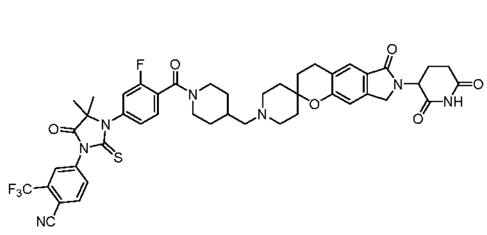

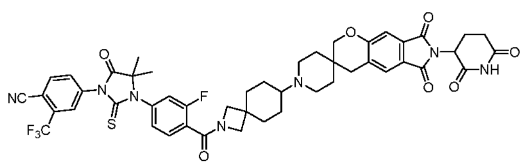

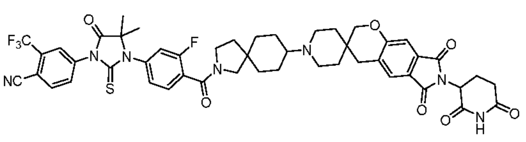

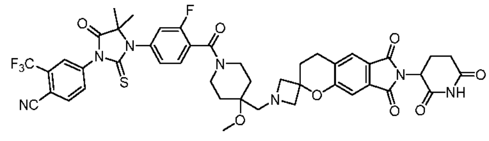

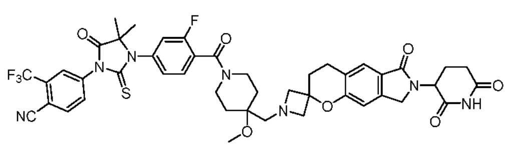

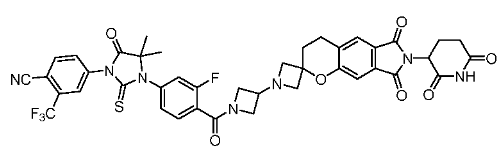

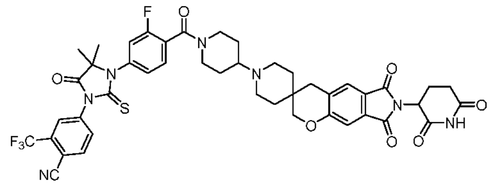

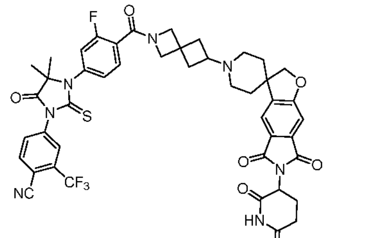

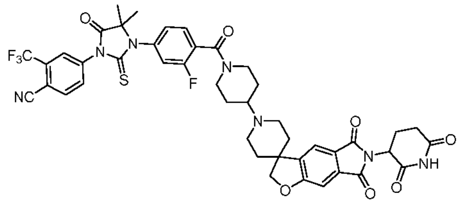

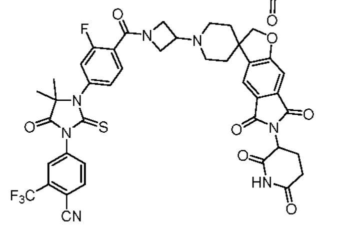

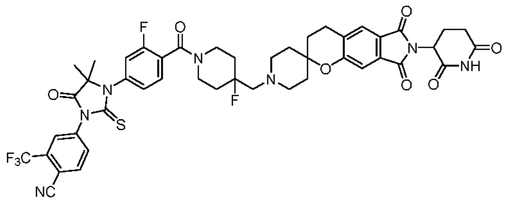

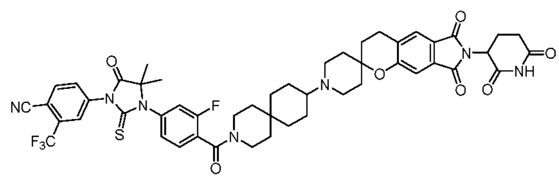

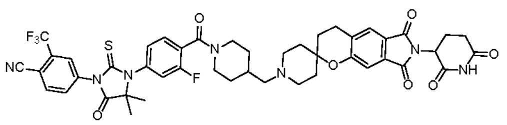

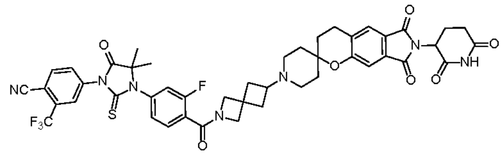

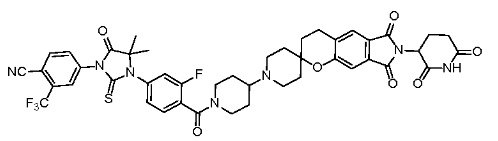

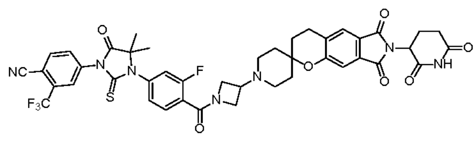

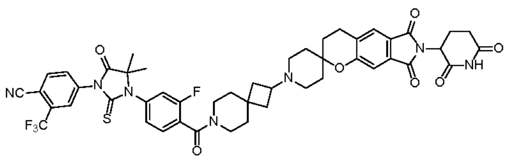

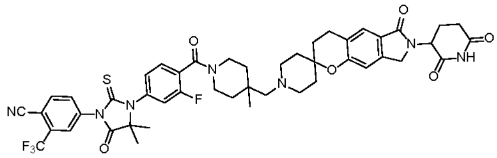

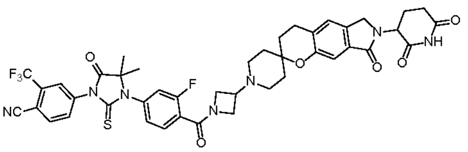

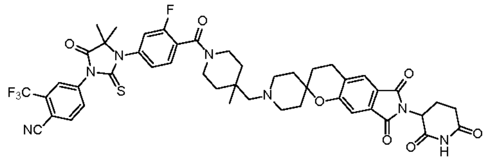

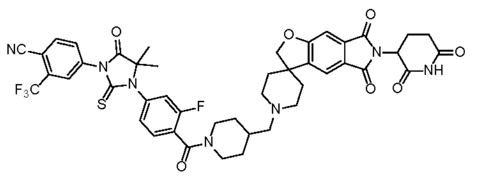

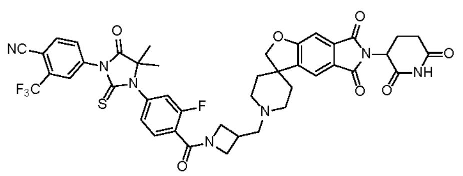

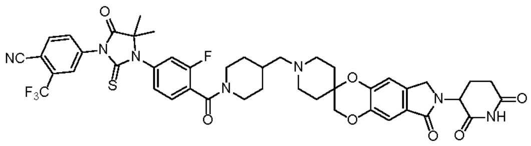

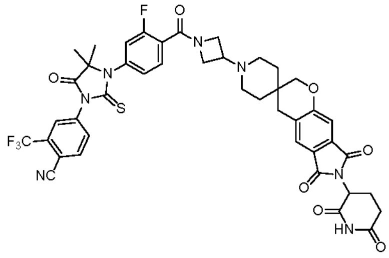

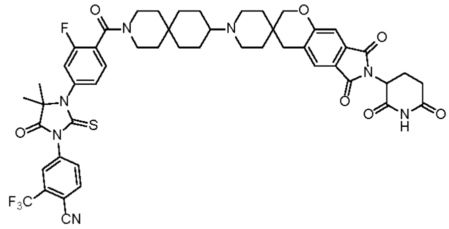

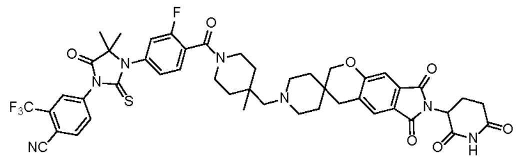

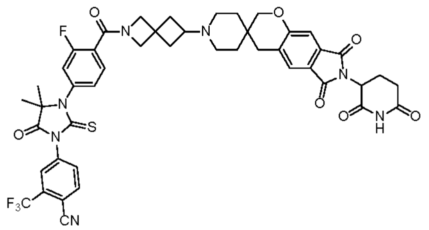

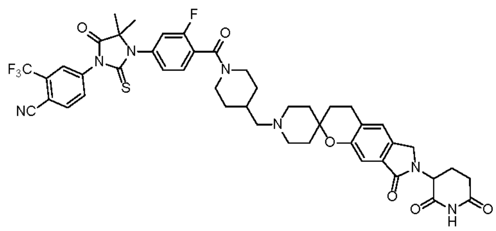

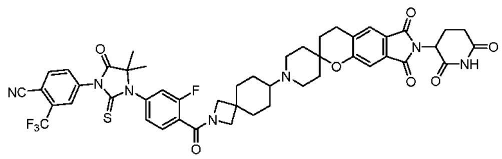

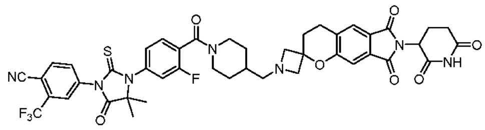

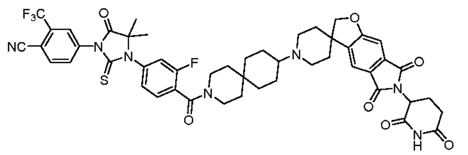

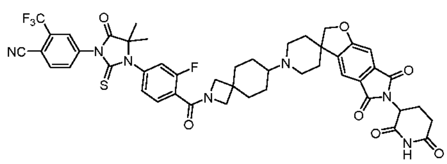

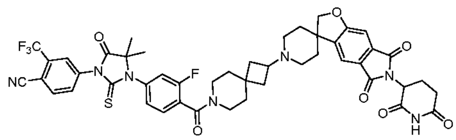

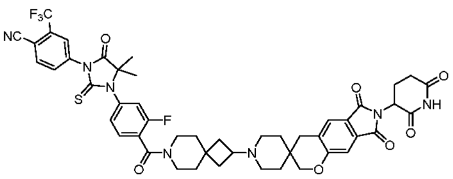

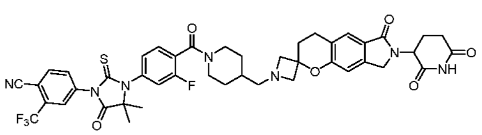

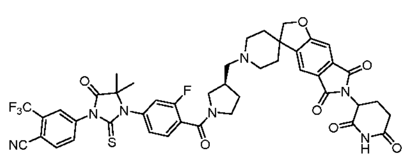

In another embodiment, the PTM is a moiety binding to aurora kinase A (AURORA-A).

In another embodiment, the PTM has the following structure:

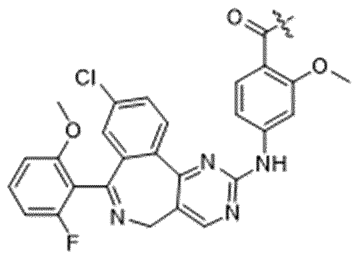

In another embodiment, the compound has the following structures:

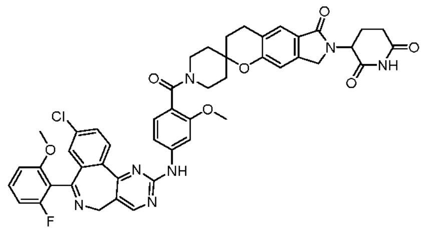

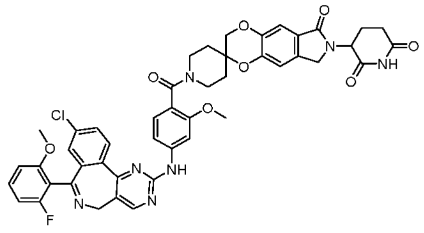

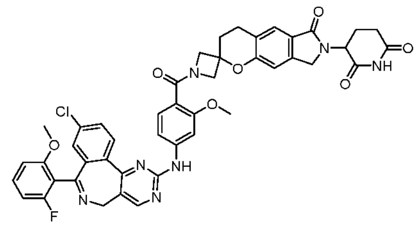

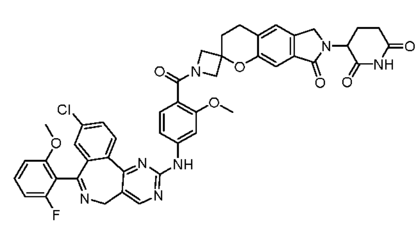

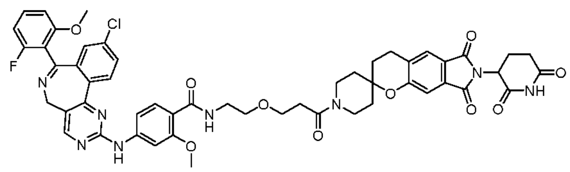

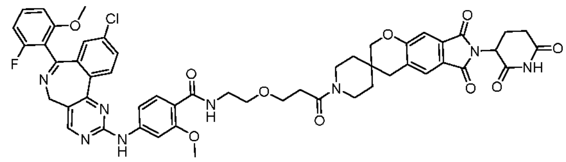

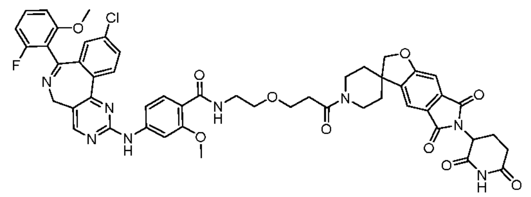

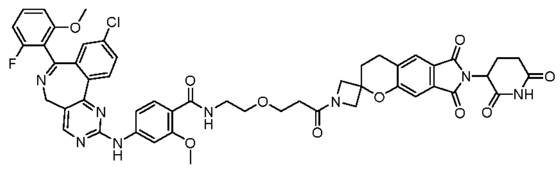

In another embodiment, the PTM is a moiety binding to Bcr-Abl protein.

In another embodiment, the PTM has the following structure:

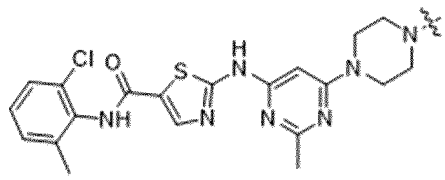

In another embodiment, the compound has the following structures:

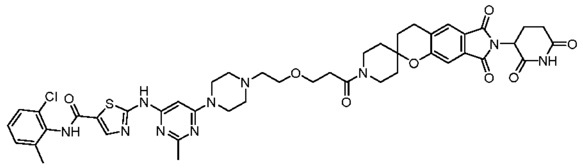
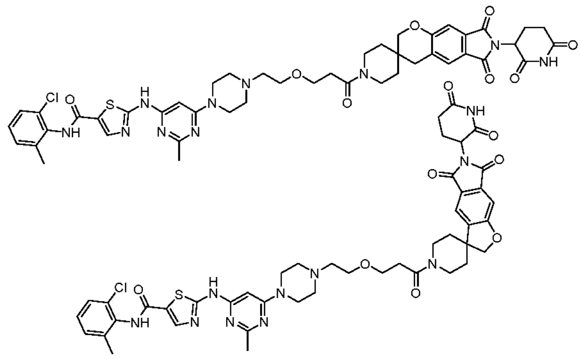
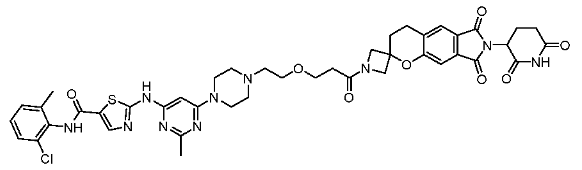
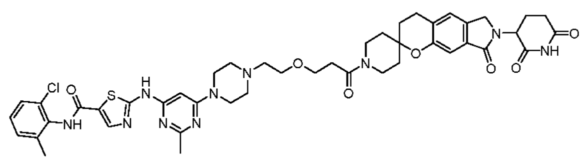
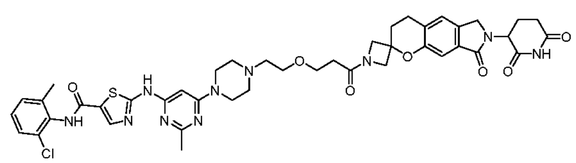
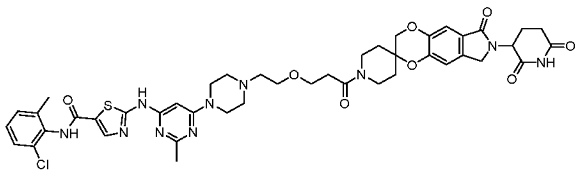
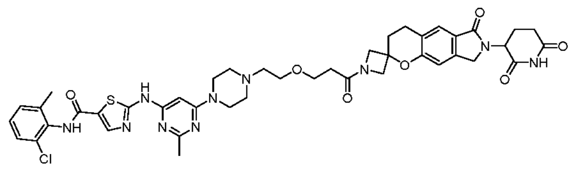

In another embodiment, the PTM is a moiety that binds to epidermal growth factor receptor (EGFR).

In another embodiment, the PTM has the following structure:

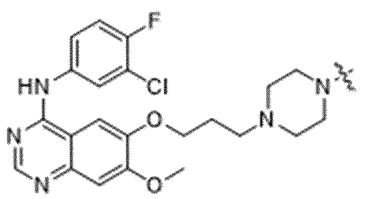

In another embodiment, the compound has the following structures:

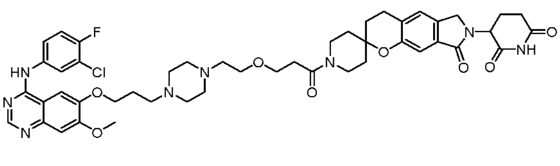
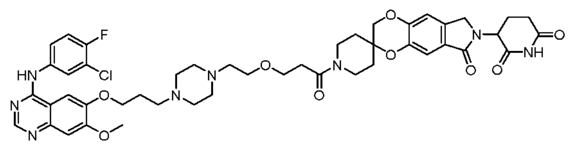
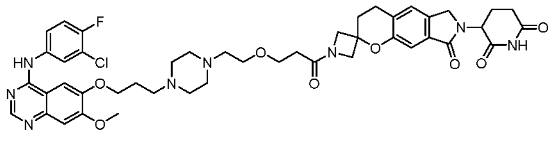
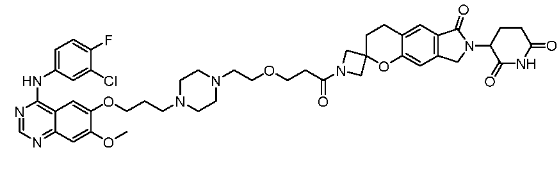

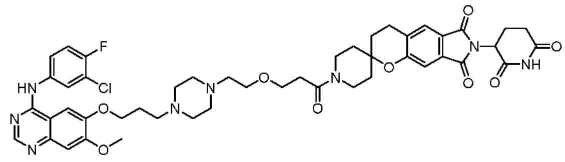

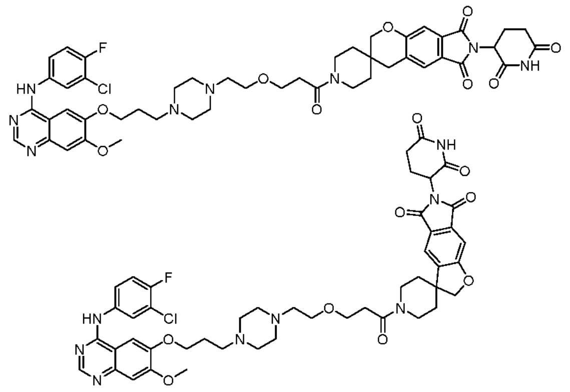

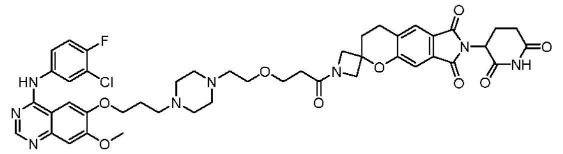

In another embodiment, the PTM is a moiety binding to anaplastic lymphoma kinase (ALK).

In another embodiment, the PTM has the following structure:

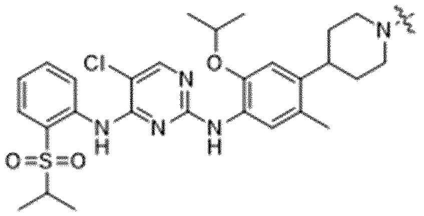

In another embodiment, the compound has the following structures:

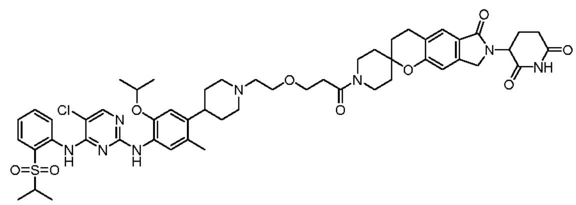

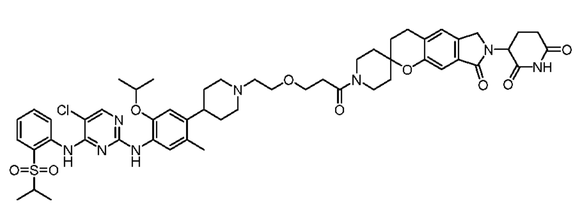

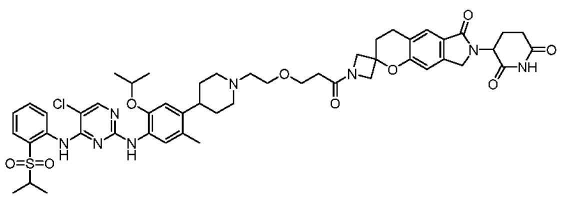

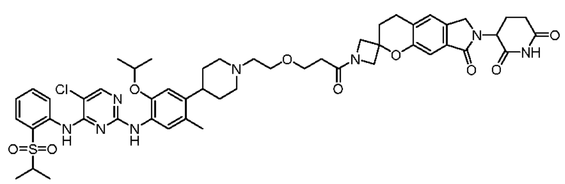

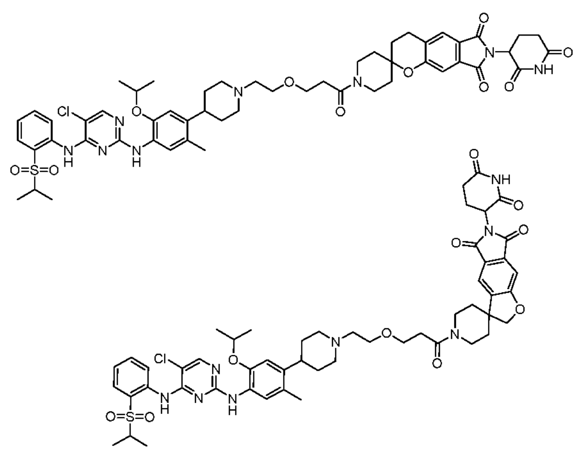

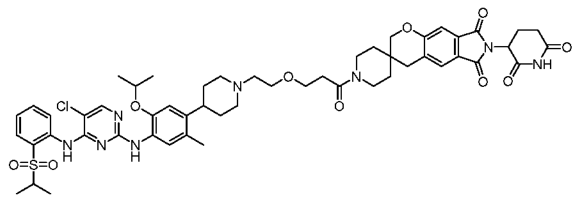

In another embodiment, the PTM is a moiety that binds to Bruton's tyrosine kinase (BTK).

In another embodiment, the PTM has the following structure:

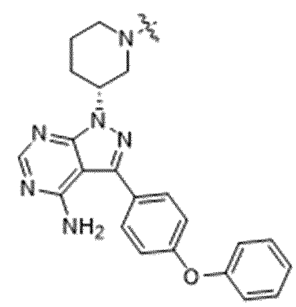

In another embodiment, the compound has the following structures:

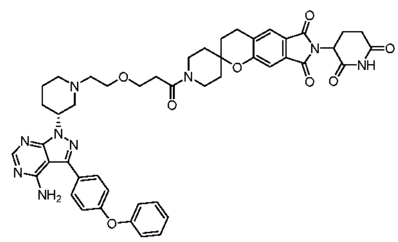

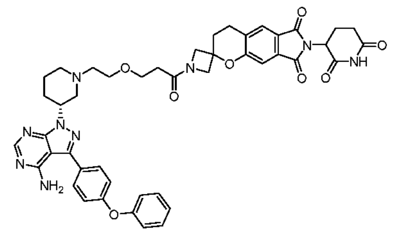

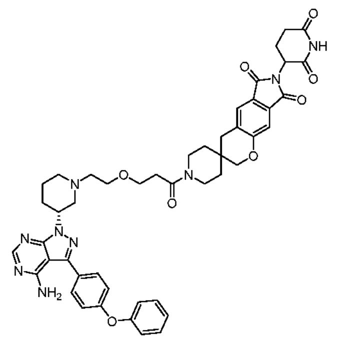

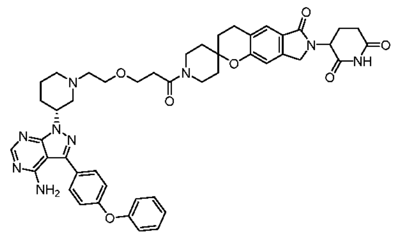

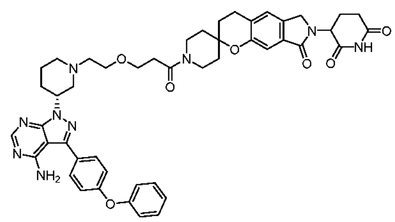

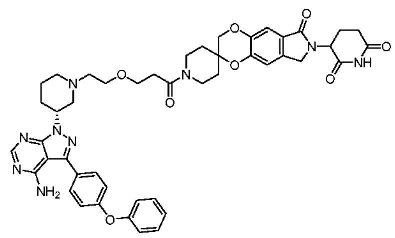

In another embodiment, the PTM is a moiety binding to cyclin-dependent kinase 4/6 (CDK4/6).

In another embodiment, the PTM has the following structures:

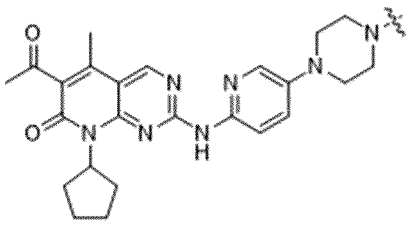

or

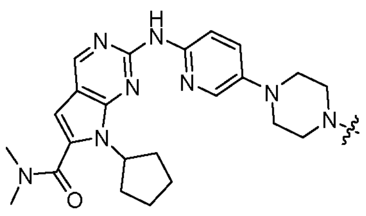

In another embodiment, the compound has the following structures:

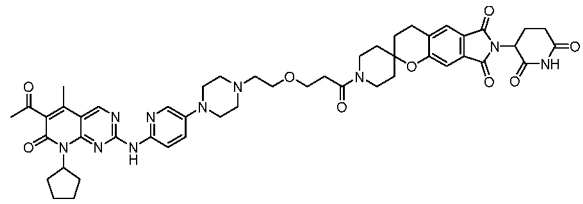

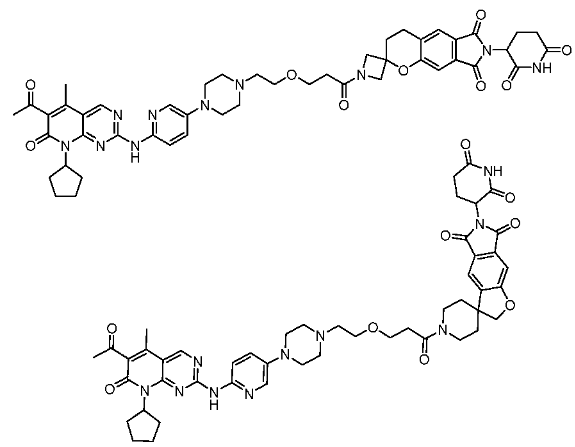

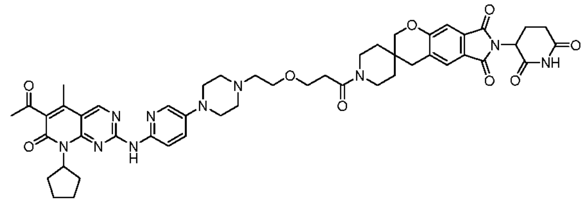

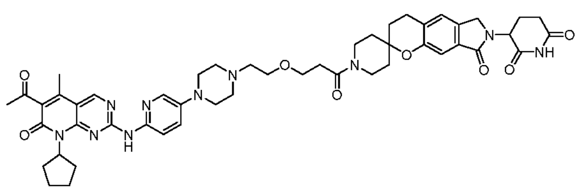

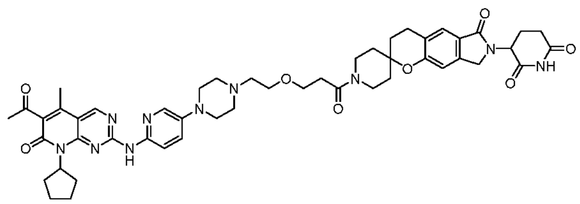

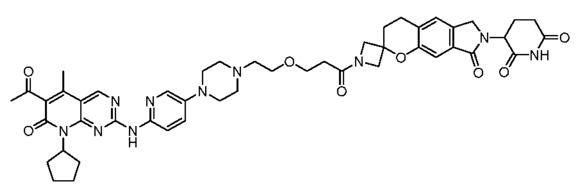

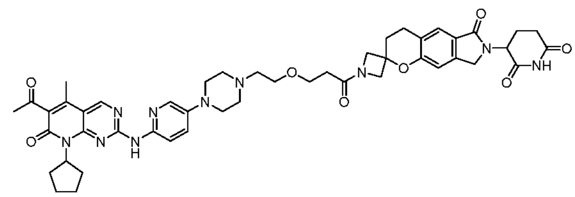

In another embodiment, the PTM is a moiety binding to bromodomain protein 4 (BRD4).

In another embodiment, the PTM has the following structure:

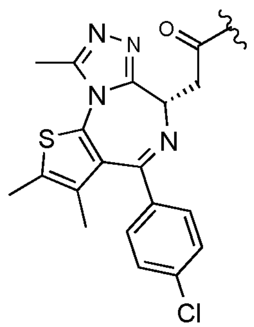

In another embodiment, the compound has the following structures:

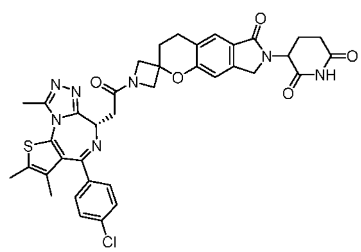

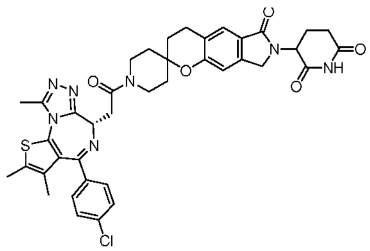

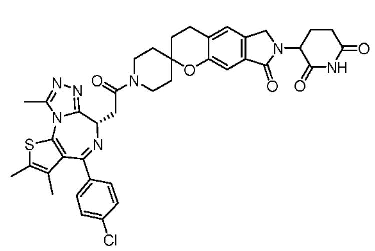

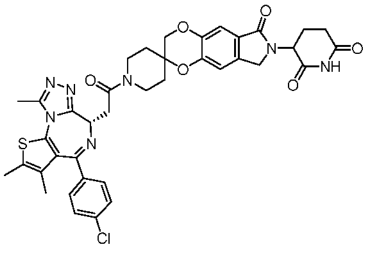

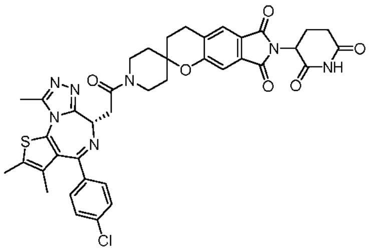

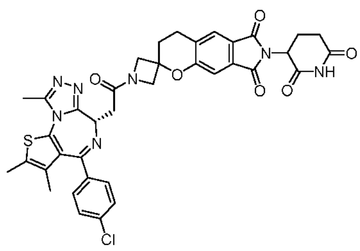

In another embodiment, the PTM is a moiety binding to estrogen receptor (ER).

In another embodiment, the PTM has the following structure:

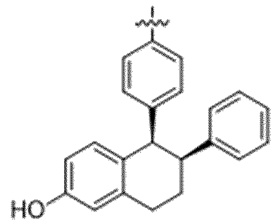

In another embodiment, the compound has the following structures:

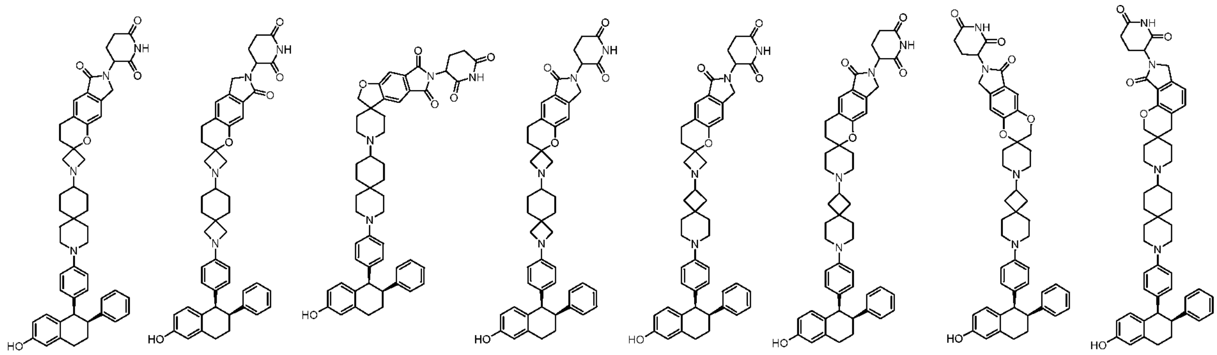

A third aspect of the present invention provides a pharmaceutical composition, the pharmaceutical composition comprising a compound provided in the first or second aspect of the present invention.

In one embodiment, use of a compound described in the first or second aspect of the present invention, or a pharmaceutical composition described in the third aspect of the present invention, in the preparation of a medicament for the treatment or prevention of diseases treated by degrading target protein bound to a ligand of the target protein, the target protein is preferably cyclin-dependent kinase 4/6, androgen receptor, estrogen receptor, aurora kinase A, Bcr-Abl protein, epidermal growth factor receptor, anaplastic lymphoma kinase, Bruton's tyrosine kinase, or bromodomain protein 4.

In another embodiment, the use of a compound described in the first or second aspect of the present invention, or a pharmaceutical composition described in the third aspect of the present invention, in the preparation of a medicament for the treatment or prevention of diseases treated by binding to cereblon in vivo.

In another embodiment, the use of a compound described in the first or second aspect of the present invention, or a pharmaceutical composition described in the third aspect of the present invention, in the treatment or prevention of a disease associated with the accumulation and/or aggregation of a target protein, the target protein is preferably cyclin-dependent kinase 4/6, androgen receptor, estrogen receptor, aurora kinase A, Bcr-Abl protein, epidermal growth factor receptor , mesenchymal lymphoma kinase, Bruton's tyrosine kinase, or bromodomain protein 4.

In another embodiment, wherein the disease is tumor or cancer, preferably the tumor or cancer is breast cancer, ductal carcinoma of the breast, prostate cancer, condylomatous lymphoma, chronic myeloid leukemia, acute myeloid leukemia, myelomonocytic leukemia, non-small cell lung cancer, lung adenocarcinoma, and/or cervical cancer.

In another embodiment, a compound as described in the first or second aspect of the present invention, or a pharmaceutical composition as described in the third aspect of the present invention, for use in the treatment or prevention of disease treated by degradation of a target protein bound to the target protein ligand, the target protein preferably is cyclin-dependent kinase 4/6, androgen receptor, estrogen receptor, aurora kinase A, Bcr-Abl protein, epidermal growth factor receptor, mesenchymal lymphoma kinase, Bruton's tyrosine kinase, or bromodomain protein 4.

In another embodiment, a compound as described in the first or second aspect of the present invention, or a pharmaceutical composition as described in the third aspect of the present invention, for use in the treatment or prevention of disease that is treated by binding to a cereblon protein in vivo.

In another embodiment, a compound as described in the first or second aspect of the present invention, or a pharmaceutical composition as described in the third aspect of the present invention, for use in the treatment or prevention of a disease associated with the accumulation and/or aggregation of a target protein, the target protein is preferably cyclin-dependent kinase 4/6, androgen receptor, estrogen receptor, aurora kinase A, Bcr-Abl protein, epidermal growth factor receptor, mesenchymal lymphoma kinase, Bruton's tyrosine kinase, or bromodomain protein 4.

In another embodiment, the disease is a tumor or cancer, preferably the tumor or cancer is breast cancer, ductal carcinoma of the breast, prostate cancer, condylomatous lymphoma, chronic myeloid leukemia, acute myeloid leukemia, myelomonocytic leukemia, non-small cell lung cancer, lung adenocarcinoma, and/or cervical cancer.

In another embodiment, a method for treating or preventing a disease treated by degrading a target protein bound to a target protein ligand, comprising administering to a subject in need thereof a therapeutically effective amount of a compound described in the first or second aspect of the present invention, or a pharmaceutical composition described in the third aspect of the present invention, the target protein preferably is cyclin-dependent kinase 4/6, androgen receptor, estrogen receptor, aurora kinase A, Bcr-Abl protein, epidermal growth factor receptor, mesenchymal lymphoma kinase, Bruton's tyrosine kinase, or bromodomain protein 4.

In another embodiment, a method for treating or preventing a disease treated by binding to a cereblon protein, comprising administering a therapeutically effective amount of the compound described in the first or second aspect of the present invention, or a pharmaceutical composition described in the third aspect of the present invention.

In another embodiment, a method for treating or preventing a disease associated with the accumulation and/or aggregation of a target protein, comprising administering to a subject in need thereof a therapeutically effective amount of the compound described in the first or second aspect of the present invention, or a pharmaceutical composition described in the third aspect of the present invention, the target protein is preferably cyclin-dependent kinase 4/6, androgen receptor, estrogen receptor, aurora kinase A, Bcr-Abl protein, epidermal growth factor receptor, anaplastic lymphoma kinase, Bruton's tyrosine kinase, or bromodomain protein 4.

In another embodiment, the disease is a tumor or cancer, preferably the tumor or cancer is a breast cancer, breast ductal carcinoma, prostate cancer, mantle cell lymphoma, chronic myelogenous leukemia, acute myeloid leukemia, myelomonocytic leukemia, non-small cell lung cancer, lung adenocarcinoma, and/or cervical cancer.

A fourth aspect of the present invention discloses a method for inducing degradation of a target protein in cells, the method comprising administering an effective amount of the compound as described in the first or second aspect of the present invention, or a pharmaceutical composition as described in the third aspect of the present invention, the target protein is preferably cyclin-dependent kinase 4/6, androgen receptor, estrogen receptor, aurora kinase A, Bcr-Abl protein, epidermal growth factor receptor, anaplastic lymphoma kinase, Bruton's tyrosine Kinase, or bromodomain protein 4.

Specific Embodiments

Definitions

The term "alkyl" as considered herein refers to a saturated aliphatic hydrocarbon group which is a straight or branched chain group containing 1 to 20 carbon atoms, preferably containing 1 to 12 (e.g. 1, 2, 3, 4, 5, 6, 7, 8, 9, 10, 11 and 12) carbon atoms, more preferably an alkyl group containing 1 to 6 carbon atoms. Non-limiting examples include methyl, ethyl, n-propyl, isopropyl, n-butyl, isobutyl, t-butyl, sec-butyl, n-pentyl, 1,1-dimethylpropyl, 1 ,2-dimethylpropyl, 2,2-dimethylpropyl, 1-ethylpropyl, 2-methylbutyl, 3-methylbutyl, n-hexyl, 1-ethyl-2- methylpropyl, 1,1,2-trimethylpropyl, 1,1-dimethylbutyl, 1,2-dimethylbutyl, 2,2-dimethylbutyl, 1,3 -dimethylbutyl, 2-ethylbutyl, 2-methylpentyl, 3-methylpentyl, 4-methylpentyl, 2,3-dimethylbutyl, n-heptyl, 2 -methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2 ,5-dimethylhexyl, 2,2-dimethylhexyl, 3,3-dimethylhexyl, 4,4-dimethylhexyl, 2-ethylhexyl, 3-ethylhexyl, 4-ethylhexylylhexyl, 2-methyl-2-ethylpentyl, 2-methyl-3-ethylpentyl, n-nonyl, 2-methyl-2-ethylhexyl, 2-methyl-3-ethyl 2,2-diethylhexyl, 2,2-diethylpentyl, n-decyl, 3,3-diethylhexyl, 2,2-diethylhexyl, and various branched chain isomers, etc. More preferably are lower alkyl groups containing 1 to 6 carbon atoms, non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethyl dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl groups may be substituted or unsubstituted, and when substituted, the substituents could substitute at any available point of attachment, preferably the substituents are independently and optionally selected from one or more of the groups consisting of H atom, D atom, halogen, alkyl, alkoxy, haloalkyl, hydroxyl, hydroxyalkyl, cyano, amino, nitro, cycloalkyl, heterocyclyl, aryl, heteroaryl.

The term "heteroalkyl" means that one or more $-CH_2-$ in the alkyl group is replaced by a heteroatom selected from NH, O and S or one or more -CH- in the alkyl group is replaced by an N atom; The alkyl group is as defined above; the heteroalkyl group may be substituted or unsubstituted, and when substituted, the substituent could substituteat any available point of attachment, preferably the substituents are independently and optionally selected from one or more of the groups consisting of H atom, D atom, halogen, alkyl, alkoxy, haloalkyl, hydroxyl, hydroxyalkyl, cyano, amino, nitro, cycloalkyl, heterocyclyl, aryl, heteroaryl.

The term "alkoxy" refers to -O-(alkyl) and -O-(unsubstituted cycloalkyl), wherein alkyl or cycloalkyl is as defined herein. Non-limiting examples of alkoxy include: methoxy, ethoxy, propoxy, butoxy, cyclopropoxy, cyclobutoxy, cyclopentyloxy, cyclohexyloxy. Alkoxy may be optionally substituted or unsubstituted, and when substituted, the substituents are preferably one or more of the following groups, independently selected from one or more of the groups consisting of H atom, D atom, halogen, alkyl, alkoxy , Haloalkyl, hydroxyl, hydroxyalkyl, cyano, amino, nitro, cycloalkyl, heterocyclyl, aryl, heteroaryl.

The term "alkenyl" refers to an alkyl compound containing a carbon-carbon double bond in the molecule, wherein the definition of alkyl is as above. Alkenyl groups may be substituted or unsubstituted, and when substituted, the substituents are preferably one or more of the following groups, independently selected from one or more of the groups consisting of hydrogen atom, alkyl, alkoxy, halogen, haloalkyl, hydroxyl , hydroxyalkyl, cyano, amino, nitro, cycloalkyl, heterocyclyl, aryl and heteroaryl.

The term "alkynyl" refers to an alkyl compound containing a carbon-carbon triple bond in the molecule, wherein alkyl is as defined above. The alkynyl group may be substituted or unsubstituted, and when substituted, the substituents are preferably one or more of the following groups, independently selected from one or more of the groups consisting of hydrogen atom, alkyl, alkoxy, halogen, haloalkyl, hydroxyl, hydroxyalkyl, cyano, amino, nitro, cycloalkyl, heterocyclyl, aryl and heteroaryl.

The term "cycloalkyl" refers to a saturated or partially unsaturated monocyclic cyclic hydrocarbon substituent, the cycloalkyl ring contains 3 to 20 carbon atoms, preferably 3 to 12 carbon atoms, more preferably 3 to 8 (e.g. 3, 4, 5, 6, 7 and 8) carbon atoms, more preferably comprising 4 to 7 carbon atoms. Non-limiting examples of monocyclic cycloalkyls include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.

Cycloalkyl groups may be substituted or unsubstituted, and when substituted, the substituents could substitute at any available point of attachment, the substituents are preferably independently and optionally selected from one or more of the group consisting of hydrogen atom, halogen, alkyl, alkoxy, haloalkyl, hydroxyl, hydroxyalkyl, cyano, amino, nitro, cycloalkyl, heterocyclyl, aryl, heteroaryl.

The term "heterocyclyl" refers to a saturated or partially unsaturated monocyclic cyclic hydrocarbon substituent comprising 3 to 20 ring atoms, one or more of which are heteroatoms selected from nitrogen, oxygen or S(O)m (where m is an integer from 0 to 2), excluding ring portions of -O-O-, -O-S- or -S-S-, the remaining ring atoms being carbon. Preferably comprising 3 to 12 (e.g. 3, 4, 5, 6, 7, 8, 9, 10, 11 and 12) ring atoms, of which 1 to 4 (e.g. 1, 2, 3 and 4) are hetero atoms; more preferably containing 3 to 8 ring atoms, of which 1-3 are heteroatoms; more preferably containing 3 to 6 ring atoms, of which 1-3 are heteroatoms; most preferably containing 5 or 6 ring atoms, of which 1-3 are heteroatoms. Non-limiting examples of monocyclic heterocyclyl groups include pyrrolidinyl, tetrahydropyranyl, 1,2.3.6-tetrahydropyridyl, piperidinyl, piperazinyl, morpholinyl, thiomorpholinyl, homopiperazinyl, etc.

The heterocyclyl group may be substituted or unsubstituted, and when substituted, the substituents may be substituted at any available attachment point, the substituents are preferably independently and optionally selected from one or more of the group consisting of hydrogen atoms, halogens, alkyls, alkoxy, haloalkyl, hydroxyl, hydroxyalkyl, cyano, amino, nitro, cycloalkyl, heterocyclyl, aryl, heteroaryl. The term "aryl" refers to a 6 to 14 membered all-carbon monocyclic or fused polycyclic (a fused polycyclic is a ring sharing adjacent pairs of carbon atoms) group having a conjugated $\pi$-electron system, preferably 6 to 10 membered , such as phenyl and naphthyl. The aryl ring includes an aryl ring as described above fused to a heteroaryl, heterocyclyl or cycloalkyl ring, wherein the ring bonded to the parent structure is an aryl ring. Aryl groups may be substituted or unsubstituted, and when substituted, substituents may be substituted at any available point of attachment, the substituents are preferably independently and optionally selected from one or more of the group consisting of hydrogen atoms, halogen, alkyl, alkane oxy, haloalkyl, hydroxyl, hydroxyalkyl, cyano, amino, nitro, cycloalkyl, heterocyclyl, aryl, heteroaryl.

The term "heteroaryl" refers to a heteroaromatic system comprising 1 to 4 (e.g. 1, 2, 3 and 4) heteroatoms, 5 to 14 ring atoms, wherein the heteroatoms are selected from oxygen, sulfur and nitrogen. Heteroaryl is preferably 5 to 10 membered (e.g. 5, 6, 7, 8, 9 or 10 membered), more preferably 5 or 6 membered, e.g. furyl, thienyl, pyridyl, pyrrolyl, N-alkyl Pyrrolyl, pyrimidinyl, pyrazinyl, pyridazinyl, imidazolyl, pyrazolyl, triazolyl, tetrazolyl, etc. The heteroaryl ring includes a heteroaryl as described above fused to an aryl, heterocyclyl or cycloalkyl ring, wherein the ring attached to the parent structure is a heteroaryl ring. Heteroaryl groups may be substituted or unsubstituted, and when substituted, the substituents could substituteat any available point of attachment, preferably independently and optionally selected from one or more of the groups consisting of hydrogen atom, halogens, alkyl, alkoxy, haloalkyl, hydroxyl, hydroxyalkyl, cyano,

amino, nitro, cycloalkyl, heterocyclyl, aryl, heteroaryl. The term "haloalkyl" refers to an alkyl group substituted with one or more halogens, wherein alkyl is as defined above.

The term "hydroxyalkyl" refers to an alkyl group substituted with one or more hydroxy groups, wherein alkyl is as defined above.

The term "halogen" refers to fluorine, chlorine, bromine or iodine.

The term "amino" refers to $-NH_2$.

The term "cyano" refers to -CN.

The term "nitro" refers to $-NO_2$.

The term "ubiquitin ligase" refers to a family of proteins that facilitate the transfer of ubiquitin to specific substrate proteins and target substrate proteins for degradation. For example, Cerebellin is an E3 ubiquitin ligase protein that alone or in combination with E2 ubiquitin conjugating enzymes results in the attachment of ubiquitin to lysines on target proteins and subsequent targeting of specific protein substrates for degradation by the proteasome. Thus, E3 ubiquitin ligases alone or in complex with E2 ubiquitin conjugating enzymes is the cause of the transfer of ubiquitin to target proteins. In general, ubiquitin ligases are involved in polyubiquitination so that the second ubiquitin is attached to the first ubiquitin, the third ubiquitin is attached to the second ubiquitin, and so on. Polyubiquitination marks proteins for degradation by the proteasome. However, there are some ubiquitination events that are restricted to monoubiquitination, where only a single ubiquitin is added to a substrate molecule by an ubiquitin ligase. Monoubiquitinated proteins are not targeted to the proteasome for degradation, but may instead be altered in their cellular location or function, for example via binding to other proteins with domains capable of binding ubiquitin. To complicate matters, different lysines on ubiquitin can be targeted by E3 to make chains. The most common lysine is Lys48 on the ubiquitin chain. This is the lysine used to make polyubiquitin, which is recognized by the proteasome.

The term "target protein" refers to proteins and peptides that have any biological function or activity (including structural, regulatory, hormonal, enzymatic, genetic, immune, contractile, storage, transport, and signal transduction). In some embodiments, target proteins include structural proteins, receptors, enzymes, cell surface proteins, proteins associated with the integrated function of the cell, including proteins involved in each of the following: catalytic activity, aromatase activity, motor activity, helical Enzyme activity, metabolic processes (anabolism and catabolism), antioxidant activity, proteolysis, biosynthesis, proteins with kinase activity, oxidoreductase activity, transferase activity, hydrolase activity, lyase activity, isomerase activity , ligase activity, enzyme regulator activity, signal transduction factor activity, structural molecule activity, binding activity (protein, lipid carbohydrate), receptor activity, cell motility, membrane fusion, cell communication, regulation of biological processes, development, cell differentiation, stimulus response, behavioral proteins, cell adhesion proteins, white matter involved in cell death, proteins involved in transport (including protein transport activity, nuclear transport, ion transport activity, channel transport activity, carrier activity), permease activity, secretion activity, electron transport activity, pathogenicity, chaperone regulator activity, nucleic acid binding activity, transcription regulator activity, extracellular conformation and biogenesis activity, translation regulator activity. The proteins include proteins from eukaryotes and prokaryotes, the eukaryotes and prokaryotes including microorganisms, viruses, fungi, and parasites, and numerous others, including humans, microorganisms, viruses, fungi, and parasites as targets for drug therapies. Other animals including domestic animals, microorganisms for determining the targets of antibiotics and other antimicrobials and plants and even viruses and numerous others.

The term "isomer" means that the compounds of the present invention may have asymmetric centers and racemates, racemic mixtures and individual diastereomers, all of which, including stereoisomers, geometric isomers are included in the present invention. In the present invention, when a compound or a salt thereof exists in a stereoisomeric form (for example, it contains one or more asymmetric carbon atoms), individual stereoisomers (enantiomers and diastereoisomers) and mixtures thereof are included within the scope of the present invention. The invention also includes individual isomers of the compounds or salts, as well as mixtures of isomers in which one or more chiral centers are inverted. The scope of the present invention includes mixtures of stereoisomers, as well as purified enantiomers or enantiomerically/diastereomerically enriched mixtures. The present invention includes mixtures of stereoisomers of all enantiomers and diastereoisomers in all possible different combinations. The present invention includes all combinations and subsets of stereoisomers of all specific groups defined above. The present invention also includes geometric isomers of the compounds or salts thereof, including cis-trans isomers.

The term "isotopically derivative" refers to compounds that differ in structure only by the presence of one or more isotopically enriched atoms. For example, having the structure of the present disclosure except replacing hydrogen with "deuterium" or "tritium", or replacing fluorine with $^{18}F$-fluorine label ($^{18}F$ isotope), or replacing carbon with $^{11}C$-, $^{13}C$- or $^{14}C$-enriched carbon ("C-, $^{13}C$-, or $^{14}C$-carbon labels; $^{11}C$-, $^{13}C$-, or $^{14}C$-isotopes) are within the scope of the present disclosure. Such compounds are useful, for example, as analytical tools or probes in biological assays, or as tracers for in vivo diagnostic imaging of disease, or as tracers for pharmacodynamic, pharmacokinetic or receptor studies.

"Optional" or "optionally" means that the subsequently described event or circumstance can but need not occur, and the description includes occasions where the event or circumstance occurs or does not occur. For example, "optionally substituted cyclopropyl" means that cyclopropyl group may but need not be substituted, and the description includes cases

where the cyclopropyl group is substituted and cases where the cyclopropyl group is unsubstituted.

"Substituted" means that one or more hydrogen atoms in a group, preferably up to 5, more preferably 1 to 3 hydrogen atoms are independently substituted by the corresponding number of substituents. It goes without saying that substituents are only in their possible chemical positions and that a person skilled in the art can determine possible or impossible substitutions without undue effort (by experiment or theory).

"Medicinal salts" or "pharmaceutically acceptable salts" refers to salts of the disclosed compounds, which are safe and effective when used in mammals, and have the desired biological activity.

Examples

The following examples are offered by way of illustration and not limitation.

Abbreviations used in this article are as follows:

MeOH is methanol;
$H_2SO_4$ is concentrated sulfuric acid;
NIS is N-iodosuccinimide;
TFA is trifluoroacetic acid;
Pd is palladium;
Sn is tin;
Pyrrolidine is tetrahydropyrrole;
OH is hydroxyl group;
Boc is tert-butoxycarbonyl;
$NaBH_4$ is sodium borohydride;
EtSiH is triethylsilane;
$H_2$ is hydrogen;
LiOH is lithium hydroxide;
NaOAc is sodium acetate;
AcOH is acetic acid;
PE is petroleum ether;
EA is ethyl acetate;
$CDCl_3$ is deuterated chloroform;
$HNO_3$ is nitric acid;
$HBF_4$ is tetrafluoroboric acid;
NaNOz is sodium nitrite;
NBS is N-bromosuccinimide;
KOtBu is potassium tert-butoxide;
NaH is sodium hydride;
$B_2pin_2$ is pinacol borate;
Oxone is potassium peroxymonosulfonate;
Cbz is benzyloxycarbonyl;
$PPh_3$ is triphenylphosphine;
$CBr_4$ is carbon tetrabromide;
Zn is zinc;
$NH_4Cl$ is ammonium chloride;
B is boron;
Br is bromine;
$(TMS)_3SiH$ is tris(trimethylsilyl)silane;
AIBN is azobisisobutyronitrile;
DMF is N,N-dimethylformamide;
$K_2CO_3$ is potassium carbonate;
TBAB is tetrabutylammonium bromide;
Bn is benzyl;
$LiAlH_4$ is lithium aluminum hydride;
$BH_3$ is borane;
THF is tetrahydrofuran;
$H_2O_2$ is hydrogen peroxide;
DMP is a Dess-Martin oxidant;
$PBr_3$ is phosphorus tribromide;

$Pd(OAc)_2$ is palladium acetate;
$BF_3Et_2O$ is boron trifluoride ether;
S is sulfur;
MsCl is methanesulfonyl chloride;
TEA is triethylamine;
DBU is 1,8-diazabicyclo[5.4.0]undec-7-ene;
$NaHCO_3$ is sodium bicarbonate;
Malonic acid is propanedioic acid;
PPA is polyphosphoric acid;
HBr is hydrogen bromide;
$Tf_2O$ is trifluoromethanesulfonic anhydride;
Pd/C is palladium on carbon;
DMSO is dimethylsulfoxide;
UPLC is Ultra High Performance Liquid Chromatography;
$MgCl_2$ is magnesium chloride;
NADPH is pyridine nucleotide triphosphate;
$NaS_2O_3$ is sodium thiosulfate;
$Pd(dppf)Cl_2$ or $PdCl_2(dppf)$ is [1,1'-bis(diphenylphosphino)ferrocene]palladium dichloride;
$Boc_2O$ is di-tert-butyl dicarbonate;
AIBN is azobisisobutyronitrile;
DIPEA or DIEA is N,N-diisopropylethylamine;
TsOH is p-toluenesulfonic acid;
$CCl_4$ is carbon tetrachloride;
CAN is cerium ammonium nitrate;
IBX is 2-iodoxybenzoic acid;
HATU is 2-(7-azabenzotriazole)-N,N,N',N'-tetramethyluronium hexafluorophosphate.

The following examples are directed to intermediate compounds and final products identified in the specification and synthetic schemes. The preparation of the compounds of the invention is described in detail using the following examples, but the chemical reactions described are disclosed in terms of their general applicability to the preparation of the compounds of the invention. Occasionally, the reaction may not be applicable to every compound within the scope of the invention as described. Compounds in which this occurs are readily identified by those skilled in the art. In these cases, the reactions can be carried out successfully by routine modifications known to those skilled in the art. In all preparative methods, all starting materials are known or can be readily prepared using known starting materials.

The starting materials, chemical reagents, and solvents used in this disclosure are all commercially available and purchased from Energy Chemical, Bide Pharmatech, Beijing Inochem, Jiangsu Aikon, Sinopharm Group, Beijing J&K Scientific, and Yunnan Xinlan Jing and other companies.

Compound structures are determined by nuclear magnetic resonance (NMR) and/or mass spectroscopy (MS). The determination of nuclear magnetic resonance (NMR) is to use Bruke AVANCE-400/600 nuclear magnetic instrument, and the deuterated solvent used is deuterated dimethyl sulfoxide (DMSO-$d_6$), deuterated chloroform ($CDCl_3$), deuterated methanol ($CD_3OD$) , the internal standard is tetramethylsilane (TMS). Mass spectrometry (MS) is determined using Waters Acquity UPLC® Plus equipment. Waters 2489 equipment is used for HPLC preparation. Medium pressure flash preparative chromatograph uses COMBIFLASH NEXTGEN 300+ equipment. The thin-layer chromatography silica gel plate uses Silica gel 60 thin-layer chromatography silica gel plate (aluminum plate, containing fluorescence). Silica gel (100-200 mesh, 200-300 mesh) used in silica gel thin-layer chromatography was purchased from Inochem.

The reaction process detection in the embodiment adopts thin-layer chromatography (TLC), and the system of the developing agent used for monitoring the reaction and the eluent used for purifying the compound by column chromatography includes: petroleum ether/ethyl acetate system, dichloromethane /methanol system.

Example 1

Synthesis of compound 1

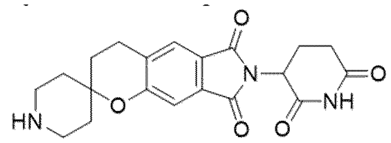

7'-(2,6-dioxopiperidin-3-yl)-3',4'-dihydro-6'H-spiro[piperidin-4,2'-pyrano[2,3- f]isoindole]6',8'(7'H)-dione

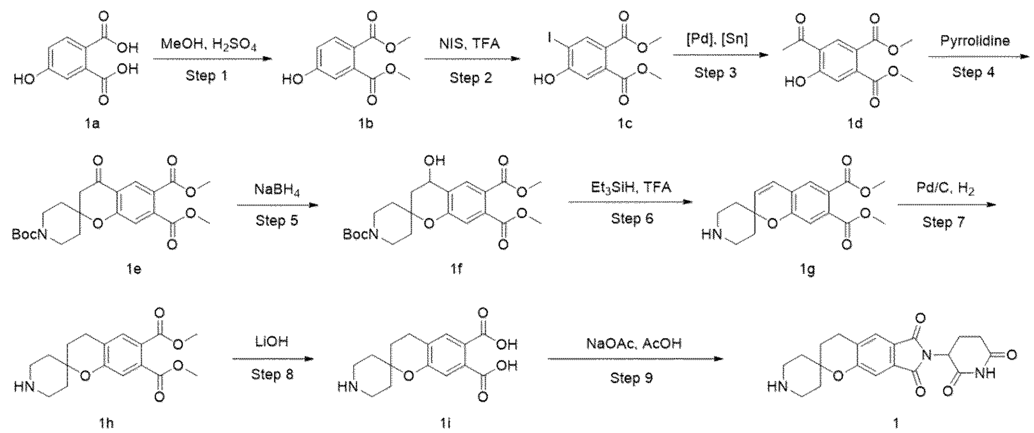


Step 1: Dimethyl 4-hydroxyphthalate (compound 1b)

To a solution of compound 1a (30 g, 164.7 mmol) in methanol (300 mL) was slowly added concentrated sulfuric acid (45 mL). The reaction solution was stirred at 65°C for 5 hours. The resulting mixture was poured into ice water, filtered and washed with water, and dried in vacuo to give white solid compound 1b (30.0 g, 87%).

$^1$H NMR (600 MHz, DMSO-$d_6$) δ 10.66 (s, 1H), 7.70 (d, $J$ = 8.5 Hz, 1H), 6.96 (dd, $J$ = 8.5, 2.5 Hz, 1H), 6.93 (d, $J$ = 2.5 Hz, 1H), 3.79 (s, 3H), 3.76 (s, 3H).
LC-MS(ESI): [M-H]$^+$ = 209.22

Step2: dimethyl 4-hydroxy-5-iodophthalate (compound 1c)

To a solution of compound 1b (20.0 g, 95.2 mmol) in trifluoroacetic acid (60 mL), N-iodosuccinimide (23.6 g, 104.7 mmol) was added slowly. The reaction was stirred overnight at room temperature. The resulting mixture was concentrated under vacuum and purified by C18 reverse phase column to give compound 1c (16.3 g, 51%) as a white solid.

$^1$H NMR (600 MHz, DMSO-$d_6$) δ 11.59 (s, 1H), 8.11 (s, 1H), 7.02 (s, 1H), 3.79 (s, 3H), 3.77 (s, 3H).
LC-MS(ESI): [M-H]$^+$ = 335.06

Step 3: Dimethyl 4-acetyl-5-hydroxyphthalate (Compound 1d)

Under the condition of nitrogen protection, compound 1c (15.0g, 44.6mmol), tributyl (1-ethoxyethylene) tin (32.2g, 89.3mmol) and bis (triphenylphosphine) palladium dichloride ( II) (3.1 g, 4.5 mmol) was dissolved in tetrahydrofuran (75 mL), heated to 65° C. and stirred for 15 h. After the reaction was completed, 1M dilute hydrochloric acid solution was added to the system and stirred for 0.5 h, and then potassium fluoride was added. The mixture was filtered and the filtrate was concentrated to give crude product. And purified by column chromatography (PE:EA=0-40%) to obtain compound 1d (9.8 g, 87%) as a yellow oil.

$^1$H NMR (400 MHz, $CDCl_3$) δ 12.65 (s, 1H), 8.37 (s, 1H), 7.12 (s, 1H), 3.96 (s, 3H), 3.92 (s, 3H), 2.73 (s, 3H).
LC-MS(ESI): [M+H]$^+$ = 253.15

Step 4: 1'-(tert-butyl) 6,7-dimethyl 4-oxospiro[chroman-2,4'-piperidine]-1',6,7-tricarboxylate (compound 1e)

Compound 1d (5.0 g, 19.8 mmol), N-(tert-butoxycarbonyl)-4-piperidone (1.4 g, 19.8 mmol) and tetrahydropyrrole (4.0 g, 19.8 mmol) were dissolved in methanol (50 mL), the mixture was stirred overnight at 70 °C. After spin-drying, column chromatography (PE:EA =0-35%) purified to obtain compound 1e (6.9 g, 80%) as a yellow oil.

$^1$H NMR (600 MHz, DMSO-$d_6$) δ 8.17 (s, 1H), 7.36 (s, 1H), 3.83 (s, 3H), 3.82 (s, 3H), 3.73 (s, 2H), 3.13 (d, *J* = 40.7 Hz, 2H), 2.96 (s, 2H), 1.95 - 1.86 (m, 2H), 1.66 (dq, *J* = 13.2, 5.0 Hz, 2H), 1.40 (s, 9H).
LC-MS(ESI): $[M-H]^+$ = 432.22

Step 5: 1'-(tert-butyl) 6,7-dimethyl 4-hydroxy spiro[chroman-2,4'-piperidine]-1',6,7-tricarboxylate (compound 1f)

To a solution of compound 1e (5.0 g, 11.5 mmol) in methanol (50 mL) was added sodium borohydride (0.7 g, 17.3 mmol) under ice-bath conditions. The reaction was stirred overnight at 70 °C. The resulting mixture was concentrated in vacuo and purified by column chromatography (PE:EA =0-50%) to give compound 1f (3.3 g, 66%) as a white solid.

$^1$H NMR (600 MHz, DMSO-$d_6$) δ 7.92 (s, 1H), 7.02 (s, 1H), 5.73 (d, *J* = 6.2 Hz, 1H), 4.74 (dt, *J* = 9.3, 6.1 Hz, 1H), 3.79 (s, 6H), 3.74 - 3.64 (m, 2H), 3.06 (s, 2H), 2.18 (dd, *J* = 13.6, 6.1 Hz, 1H), 1.78 (ddt, *J* = 23.0, 13.5, 6.3 Hz, 2H), 1.72 - 1.64 (m, 2H), 1.57 (ddd, *J* = 13.7, 11.3, 4.7 Hz, 1H), 1.40 (s, 9H).
LC-MS(ESI): $[M-H]^+$ = 464.39

Step 6: spiro[chromene-2,4'-piperidine]-6,7-dicarboxylic acid dimethyl ester (compound 1g)

Compound 1f (3.0 g, 6.9 mmol) and triethylsilane (3.2 g, 27.6 mmol) were dissolved in trifluoroacetic acid (30 mL), and the mixture was stirred overnight at 80° C. The resulting mixture was concentrated in vacuo and purified by C18 reverse phase column to give Compound 1g (1.8 g, 83%) as a colorless oil.

$^1$H NMR (400 MHz, $CDCl_3$) δ 7.53 (s, 1H), 7.11 (s, 1H), 6.51 (d, *J* = 9.9 Hz, 1H), 5.72 (d, *J* = 9.8 Hz, 1H), 4.66 (s, 2H), 3.93 (s, 3H), 3.89 (s, 3H), 3.42 - 3.31 (m, 4H), 2.23 (d, *J* = 14.6 Hz, 2H), 2.12 - 2.02 (m, 2H).
LC-MS(ESI): $[M+H]^+$ = 318.25

Step 7: spiro[chroman-2,4'-piperidine]-6,7-dicarboxylic acid dimethyl ester (compound 1h)

Compound 1g (1.6g, 5.0mmol) was dissolved in methanol (20mL), and palladium on carbon (0.2g) was added. The reaction was stirred overnight at room temperature under hydrogen. The mixture was filtered and spin-dried to give compound 1h (1.3 g, 81%) as a yellow oil.

$^1$H NMR (400 MHz, $CDCl_3$) δ 7.61 (s, 1H), 7.14 (s, 1H), 3.93 (s, 3H), 3.89 (s, 3H), 3.35 (d, *J* = 6.2 Hz, 4H), 3.30 (s, 2H), 2.87 (t, *J* = 6.8 Hz, 2H), 2.05 - 2.00 (m, 2H), 1.95 (t, *J* = 6.7 Hz, 2H).
LC-MS(ESI): $[M+H]^+$ = 320.28

Step 8: spiro[chroman-2,4'-piperidine]-6,7-dicarboxylic acid (compound 1i)

Compound 1h (1.5 g, 4.7 mmol) was dissolved in a suspension of methanol and water, and lithium hydroxide (1.7 g, 70.5 mmol) was added. The reaction was stirred overnight at room temperature. The reaction mixture was concentrated in vacuo. The crude product was used directly in the next step without further purification.
LC-MS(ESI): $[M+H]^+$ = 292.21

Step 9: 7'-(2,6-dioxopiperidin-3-yl)-3',4'-dihydro-6'H-spiro[piperidine-4,2'-pyrano[ 2,3-*f*]isoindole]6',8'(7'*H*)-dione (compound 1)

Compound 1i (1.3g, 7.7mmol) was dissolved in acetic acid (15mL) solution, 3-aminopiperidine-2,6-dione hydrochloride (1.52g, 9.2mmol) and sodium acetate (2.1g, 15.5 mmol), the reaction was stirred for 4 hours at 110°C. After the reaction was completed, the mixture was purified by reverse phase column to obtain white solid compound 1 (1.6 g, 81%).

$^1$H NMR (400 MHz, DMSO-$d_6$) δ 11.10 (s, 1H), 7.74 (s, 1H), 7.38 (s, 1H), 5.10 (dd, *J* = 12.9, 5.4 Hz, 1H), 3.28 - 3.14 (m, 4H), 2.95 (t, *J* = 6.8 Hz, 2H), 2.91 - 2.83 (m, 1H), 2.65 - 2.53 (m, 2H), 2.07 - 1.99 (m, 1H), 1.93 (q, *J* = 6.2 Hz, 4H), 1.88 -

1.78 (m, 2H).
LC-MS(ESI): $[M+H]^+$ = 384.32.

Example 2

Synthesis of compound 2

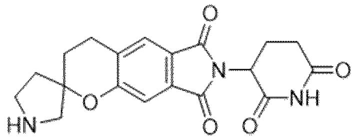

7-(2,6-dioxopiperidin-3-yl)-3,4-dihydro-6H-spiro[pyrano[2,3-f]isoindole-2,3'-pyrrolidine ]-6,8(7H)-dione

Synthesis scheme:

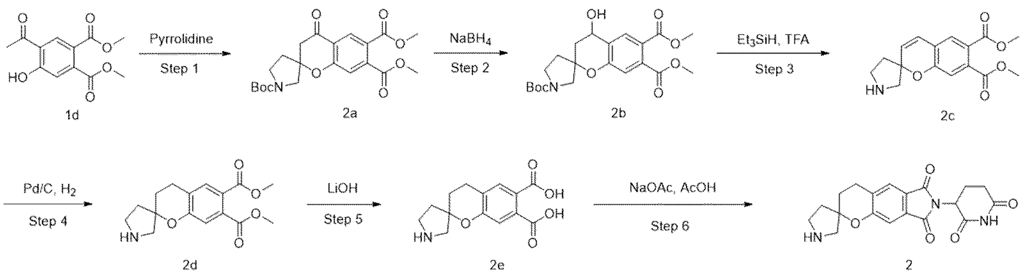


Step 1: 1'-(tert-butyl) 6,7-dimethyl 4-oxospiro[chroman-2,3'-pyrrolidine]-1',6,7-tricarboxylate (compound 2a)

Compound 1d was prepared with reference to Step 1 to Step 3 of Example 1. The obtained compound 1d (5.0 g, 19.8 mmol), 1-tert-butoxycarbonyl-3-pyrrolidone (1.4 g, 19.8 mmol) and tetrahydropyrrole (4.0 g, 19.8 mmol) were dissolved in methanol (50 mL), and the mixture was stirred overnight at 70°C. After spin-drying, purified by column chromatography (PE:EA=0-35%) to obtain compound 2a (5.0 g, 60%) as a yellow oil.

$^1H$ NMR (400 MHz, $CDCl_3$) $\delta$ 8.44 (s, 1H), 7.18 (s, 1H), 3.95 (s, 3H), 3.91 (s, 3H), 3.89 - 3.83 (m, 1H), 3.76 - 3.64 (m, 1H), 3.62 - 3.50 (m, 1H), 3.40 (dd, $J$ = 17.4, 12.4 Hz, 1H), 3.06 - 2.86 (m, 2H), 2.36 - 2.25 (m, 1H), 1.97 (ddd, $J$ = 13.5, 10.4, 9.0 Hz, 1H), 1.47 (d, 9H).
LC-MS(ESI): $[M-H]^+$ = 418.40

Step 2: 1'-(tert-butyl) 6,7-dimethyl 4-hydroxyspiro[chroman-2,3'-pyrrolidine]-1',6,7-tricarboxylate (compound 2b )

Compound 2b was prepared referring to Step 5 of Example 1.
LC-MS(ESI): $[M+Na]^+$ = 444.33

Step 3: spiro[chromene-2,3'-pyrrolidine]-6,7-dicarboxylic acid dimethyl ester (compound 2c)

Compound 2c was prepared referring to Step 6 of Example 1.

$^1H$ NMR (600 MHz, $CDCl_3$) $\delta$ 7.53 (s, 1H), 7.09 (s, 1H), 6.61 (d, $J$ = 9.8 Hz, 1H), 5.79 (d, $J$ = 9.9 Hz, 1H), 4.53 (br s, 2H), 3.91 (s, 3H), 3.89 (s, 3H), 3.70 - 3.55 (m, 3H), 3.27 (d, $J$ = 12.5 Hz, 1H), 2.54 (dd, $J$ = 14.2, 6.4 Hz, 1H), 2.14 (ddd, $J$ = 13.8, 11.0, 6.5 Hz, 1H).
LC-MS(ESI): $[M+H]^+$ = 304.27

Step 4: spiro[chroman-2,3'-pyrrolidine]-6,7-dicarboxylic acid dimethyl ester (compound 2d)

Compound 2d was prepared referring to Step 7 of Example 1.

$^1$H NMR (600 MHz, $CDCl_3$) δ 7.61 (s, 1H), 7.09 (s, 1H), 3.90 (s, 3H), 3.88 (s, 3H), 3.52 (dtd, *J* = 17.8, 11.4, 7.8 Hz, 2H), 3.44 (dd, *J* = 12.7, 1.6 Hz, 1H), 3.25 (d, *J* = 12.6 Hz, 1H), 2.90 (dtd, *J* = 17.3, 10.7, 6.9 Hz, 2H), 2.28 - 2.23 (m, 1H), 2.14 - 2.10 (m, 2H), 2.08 - 2.02 (m, 1H).
LC-MS(ESI): $[M+H]^+$ = 306.22

Step 5: spiro[chroman-2,3'-pyrrolidine]-6,7-dicarboxylic acid (compound 2e)

Compound 2e was prepared referring to Step 8 of Example 1.

LC-MS(ESI): $[M+H]^+$ = 278.22
Step 6: 7-(2,6-dioxopiperidin-3-yl)-3,4-dihydro-6H-spiro[pyrano[2,3-f]isoindole-2,3 '-Pyrrolidine]-6,8(7H)-dione (compound 2)

Compound 2 was prepared referring to Step 9 of Example 1.

$^1$H NMR (400 MHz, DMSO-$d_6$) δ 11.11 (s, 1H), 7.78 (s, 1H), 7.18 (s, 1H), 5.10 (dd, *J* = 12.9, 5.4 Hz, 1H), 3.50 - 3.21 (m, 4H), 3.03 - 2.84 (m, 4H), 2.66 - 2.53 (m, 2H), 2.24 - 2.00 (m, 4H).
LC-MS(ESI): $[M+H]^+$ = 370.34

Example 3

Synthesis of compound 3

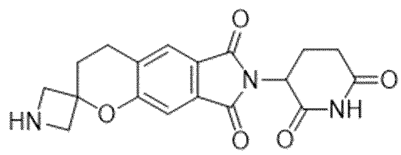

7'-(2,6-dioxopiperidin-3-yl)-3',4'-dihydro-6'H-spiro[azetidine-3,2'-pyrano[2 ,3-f]isoindole]-6',8'(7'H)-dione

Prepare 7'-(2,6-dioxopiperidin-3-yl)-3',4'-dihydro-6'H-spiro[azetidine-3,2'-pyrano[2,3-f]isoindole]-6',8'(7'H)-dione by using the similar step from step 4 to step 9 in example 1 .

$^1$H NMR (600 MHz, DMSO-$d_6$) δ 11.12 (s, 1H), 7.76 (s, 1H), 7.30 (s, 1H), 5.10 (dd, *J* = 12.9, 5.4 Hz, 1H), 4.16 (t, *J* = 8.7 Hz, 4H), 2.97 (t, *J* = 6.5 Hz, 2H), 2.88 (ddd, *J* = 17.0, 13.9, 5.5 Hz, 1H), 2.59 (dt, *J* = 17.1, 3.1 Hz, 1H), 2.54 (dd, *J* = 13.1, 4.5 Hz, 1H), 2.22 (t, *J* = 6.5 Hz, 2H), 2.06 - 2.00 (m, 1H).
LC-MS(ESI): $[M+H]^+$ = 356.26

Example 4

Synthesis of compound 4

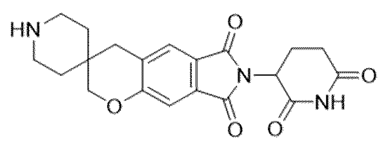

7'-(2,6-dioxopiperidin-3-yl)-2'*H*-spiro[piperidine-4,3'-pyrano[2,3-*f*]isoindole]-6',8'(4'*H*,7'*H*)-dione Synthetic scheme

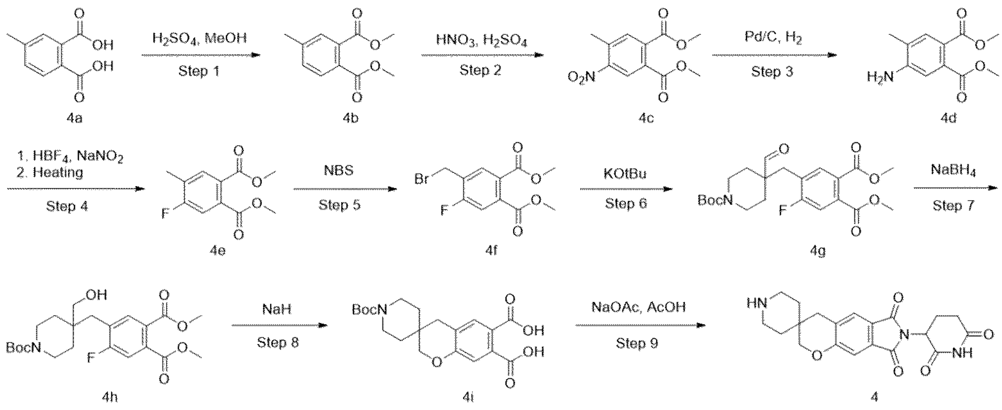


Step 1: dimethyl 4-methylphthalate (compound 4b)

Compound 4a (10 g, 55.56 mmol) was dissolved in 100 mL of methanol, then 15 mL of concentrated sulfuric acid was added, and reacted overnight at room temperature. The reaction solution was poured into ice water, extracted with ethyl acetate and concentrated to obtain compound 4b (10.6 g, 91%) as a colorless oil, which was directly used in the next step without purification.

$^1$H NMR (400 MHz, $CDCl_3$) δ 7.66 (d, *J* = 7.9 Hz, 1H), 7.46 (d, *J* = 1.7 Hz, 1H), 7.32 (dd, *J* = 7.9, 1.8 Hz, 1H), 3.89 (s, 3H), 3.88 (s, 3H), 2.40 (s, 3H).

Step 2: dimethyl 4-methyl-5-nitrophthalate (compound 4c)

Compound 4b (10 g, 48 mmol) was dissolved in 100 mL of concentrated sulfuric acid, then concentrated nitric acid (25 mL, 68%) was slowly added, and reacted overnight at room temperature. After the reaction, the reaction solution was poured into ice water, extracted with ethyl acetate and concentrated. The crude product was purified by column chromatography to obtain compound 4c (5.5 g, 45%) as a white solid.

$^1$H NMR (400 MHz, $CDCl_3$) δ 8.43 (s, 1H), 7.64 (s, 1H), 3.97 (s, 3H), 3.96 (s, 3H), 2.69 (s, 3H).

Step 3: dimethyl 4-amino-5-methylphthalate (compound 4d)

Compound 4c (5.1 g, 20 mmol) was dissolved in 100 mL of methanol, then 0.51 g of palladium on carbon was added, and the reaction system was replaced with nitrogen and hydrogen, and reacted overnight at room temperature. After the reaction was completed, it was filtered with celite, and the filtrate was collected and concentrated to obtain compound 4d (4 g, 91%) as a yellow oil, which was directly used in the next step without purification.

$^1$H NMR (400 MHz, DMSO-$d_6$) δ 7.46 (s, 1H), 6.68 (s, 1H), 5.91 (s, 2H), 3.75 (s, 3H), 3.71 (s, 3H).
LC-MS(ESI): $[M+Na]^+$ = 246.18

Step 4: dimethyl 4-fluoro-5-methylphthalate (compound 4e)

Compound 4d (4 g, 17.92 mmol) was added into 10% fluoboric acid (32 ml), and the solution was suspended. After stirring for 0.5h, cool to 0-5°C, and slowly add 4mL $NaNO_2$ (1.36g, 19.71mmol) aqueous solution for diazotization reaction, and stir in ice bath for 0.5h. The tetrafluoroborate was filtered and the cake was collected and the solid was dried under vacuum. Then, the toluene solution of tetrafluoroborate was placed in an oil bath at 110° C and stirred. After the reaction was completed, it was extracted with ethyl acetate, and the organic phase was washed with water and saturated brine, and dried with anhydrous sodium sulfate. The crude product was concentrated and purified by column chromatography to give compound 4e (2.31 g, 57%) as a light yellow oil.

$^1$H NMR (400 MHz, $CDCl_3$) δ 7.61 (dd, *J* = 7.2, 0.9 Hz, 1H), 7.38 (d, *J* = 9.4 Hz, 1H), 3.92 (s, 3H), 3.91 (s, 3H), 2.35 (d, *J* = 2.0 Hz, 3H).

Step 5: dimethyl 4-(bromomethyl)-5-fluorophthalate (compound 4f)

Compound 4e (2.26g, 10mmol) was dissolved in carbon tetrachloride, then N-bromosuccinimide (2.14g, 12mmol) and azobisisobutyronitrile (0.16g, 1mmol) were added, and heated to reflux overnight. After concentration, purification was performed by reverse column to obtain white solid compound 4f (2.14 g, 70%).

$^1$H NMR (400 MHz, $CDCl_3$) δ 7.87 (d, *J* = 7.1 Hz, 1H), 7.40 (d, *J* = 9.4 Hz, 1H), 4.51 (d, *J* = 1.0 Hz, 2H), 3.94 (s, 3H), 3.93 (s, 3H).

Step 6: 4-((1-(tert-butoxycarbonyl)-4-formylpiperidin-4-yl)methyl)-5-fluorophthalic acid dimethyl ester (compound 4g)

Compound 4f (2.14 g, 7 mmol) was dissolved in anhydrous tetrahydrofuran (30 mL), and potassium tert-butoxide (1.18 g, 10.5 mmol) was slowly added at -30°C. After reacting for 0.5 h, a solution of 1-tert-butoxycarbonylpiperidine-4-carbaldehyde (1.79 g, 8.4 mmol) in tetrahydrofuran (5 mL) was added dropwise at the same temperature. The reaction was moved to room temperature and stirred overnight. The reaction system was quenched with saturated ammonium chloride, and extracted with ethyl acetate. The organic phase was washed with saturated brine, dried over anhydrous sodium sulfate, concentrated and purified by flash silica gel column chromatography to obtain compound 4g (1.68g, 55%) as a colorless oil.

$^1$H NMR (400 MHz, $CDCl_3$) δ 9.58 (d, *J* = 2.3 Hz, 1H), 7.50 (d, *J* = 7.1 Hz, 1H), 7.34 (d, *J* = 9.5 Hz, 1H), 3.96 - 3.84 (m, 8H), 2.91 - 2.74 (m, 4H), 1.96 (d, *J* = 13.0 Hz, 2H), 1.59 - 1.46 (m, 2H), 1.44 (s, 9H).
LC-MS(ESI): $[M\text{-}Boc+H]^+$ = 338.29

Step 7: 4-((1-(tert-butoxycarbonyl)-4-(hydroxymethyl)piperidin-4-yl)methyl)-5-fluorophthalic acid dimethyl ester (compound 4h)

Compound 4g (1.66g, 3.8mmol) was dissolved in methanol (15mL), and sodium borohydride (0.215g, 5.7mmol) was added under ice-cooling conditions, and stirred at room temperature for 3h. The mixture was quenched with saturated ammonium chloride, and extracted with ethyl acetate, the organic phase was washed with saturated brine, dried over anhydrous sodium sulfate, concentrated and purified by column chromatography to obtain colorless oil compound 4h (1.47g, 88% ).

$^1$H NMR (400 MHz, DMSO-*d*$_6$) δ 7.74 (d, *J* = 7.0 Hz, 1H), 7.54 (d, *J* = 9.7 Hz, 1H), 4.81 (t, *J* = 5.0 Hz, 1H), 3.82 (s, 3H), 3.82 (s, 3H), 3.48 - 3.39 (m, 2H), 3.21 (d, *J* = 5.0 Hz, 4H), 2.74 (s, 2H), 1.42 - 1.32 (m, 11H), 1.27 - 1.14 (m, 2H).
LC-MS(ESI): $[M\text{-}Boc+H]^+$ = 340.41

Step 8: 1'-(tert-butoxycarbonyl)spiro[chroman-3,4'-piperidine]-6,7-dicarboxyfic acid (compound 4i)

Compound 4h (1.45g, 3.3mmol) was dissolved in dry N,N-dimethylformamide (6mL), sodium hydride (0.4g, 16.5mmol) was added, and the reaction mixture was carried out at 110°C for 2h. After the reaction was completed, it was quenched with acetic acid and purified by a reverse column to obtain white solid compound 4i (0.65 g, 51%).

$^1$H NMR (400 MHz, DMSO-*d*$_6$) δ 12.93 (s, 2H), 7.50 (s, 1H), 6.92 (s, 1H), 3.99 (s, 2H), 3.53 - 3.42 (m, 2H), 3.33 - 3.23 (m, 2H), 2.75 (s, 2H), 1.40 (s, 9H), 1.39 - 1.28 (m, 4H).
LC-MS(ESI): $[M\text{-}Boc+H]^+$ = 292.23

Step 9: 7'-(2,6-dioxopiperidin-3-yl)-2'H-spiro[piperidine-4,3'-pyrano[2,3-f]isoindole ]-6',8'(4'H,7'H)-dione (Compound 4)

Compound 4i (0.63g, 1.6mmol) was dissolved in acetic acid (4mL), and 3-amino-2,6-piperidinedione hydrochloride (0.33g, 2.0mmol) and sodium acetate (0.39g, 4.8mmol) were added, the reaction mixture was carried out overnight at 110°C. After the reaction was completed, it was purified by reverse column to obtain white solid compound 4 (0.5 g, 82%).

$^1$H NMR (400 MHz, DMSO-*d*$_6$) δ 11.12 (s, 1H), 8.51 (s, 2H), 7.69 (s, 1H), 7.29 (s, 1H), 5.10 (dd, *J* = 12.7, 5.4 Hz, 1H), 4.16 (s, 2H), 3.25 - 3.05 (m, 4H), 2.92 (s, 2H), 2.90 - 2.82 (m, 1H), 2.64 - 2.52 (m, 2H), 2.05 - 1.99 (m, 1H), 1.67 - 1.51 (m, 4H).
LC-MS(ESI): $[M+H]^+$ = 384.36

Example 5

Synthesis of compound 5

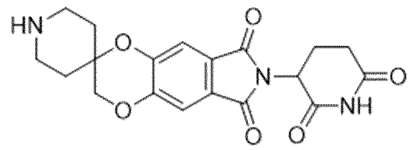

7'-(2,6-dioxopiperidin-3-yl)-7'-hydrogen-3'*H*,6'*H*-spiro[piperidine-4,2'-[1,4]dioxin [2,3-*f*]isoindole]-6',8'-dione

Synthetic scheme

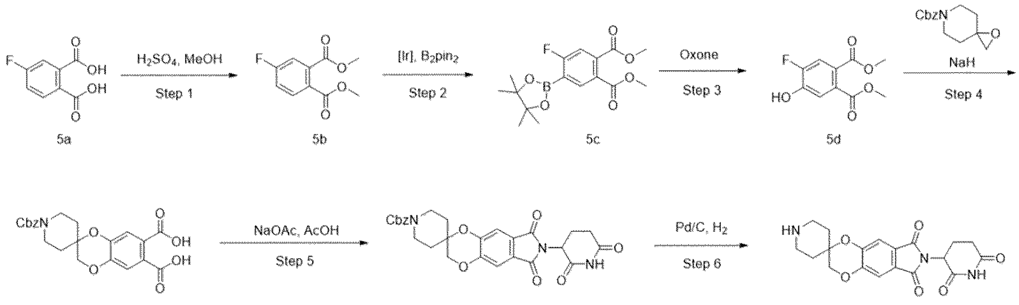


5a 5b 5c 5d 5e 5f 5

Step 1: dimethyl 4-fluorophthalate (compound 5b)

To a solution of compound 5a (5.5 g, 30.0 mmol) in methanol (100 mL) was slowly added concentrated sulfuric acid (15 mL) dropwise. The reaction was stirred overnight at room temperature. The reaction solution was poured into ice water, extracted with ethyl acetate, the organic phases were combined, washed with saturated brine, and dried with anhydrous sodium sulfate. After filtration and concentration under reduced pressure, Compound 5b (5.7 g, 90%) was obtained as a colorless oily liquid, and the crude product was used in the next reaction without further purification.

$^1$H NMR (400 MHz, $CDCl_3$) δ 7.82 (dd, *J* = 8.6, 5.3 Hz, 1H), 7.38 (dd, *J* = 8.6, 2.6 Hz, 1H), 7.82 (ddd, *J* = 8.6, 5.3, 2.6 Hz, 1H), 3.96 (s, 3H), 3.92 (s, 3H).

Step 2: methyl 4-fluoro-5-(4,4,5,5-tetramethyl-1,3,2-dioxaborolan-2-yl)phthalate (compound 5c)

Pinacol diboronate (4.5g, 17.5mmol), 4,4'-di-tert-butyl-2,2'-dipyridine (0.13g, 0.47mmol) and methoxy(cyclooctadiene) Iridium dimer (0.16g, 0.23mmol) were added in the methyl tert-butyl ether solution of 10ml. The methyl tert-butyl ether (10mL) solution of compound 5b (2.5g, 11.8mmol) was added, and replaced with nitrogen. The reaction mixture was stirred at 100 °C for 4 h. After the reaction was completed, it was filtered through diatomaceous earth, and the filtrate was concentrated to obtain crude product (3.2 g), which was used in the next reaction without further purification.

Step 3: dimethyl 4-fluoro-5-hydroxyphthalate (compound 5d)

To a solution of compound 5c (3.38 g, 10 mmol) in acetonitrile (50 mL) was added potassium peroxymonosulfonate (6.15 g), and the reaction was stirred at room temperature overnight. The reaction solution was filtered and the filtrate was concentrated, and the crude product was subjected to silica gel column chromatography to obtain compound 5d (1.6 g, 70%) as a white solid.

$^1$H NMR (400 MHz, $CDCl_3$) δ 8.39 (s, 1H), 7.50 (d, J = 10.8 Hz, 1H), 7.19 (d, J = 8.1 Hz, 1H), 3.88 (s, 3H), 3.86 (s, 3H). LC-MS(ESI): $[M+H]^+$ = 229.18

Step 4: 1'-((benzyloxy)carbonyl)-3*H*-spiro[benzo[*b*][1,4]dioxane-2,4'-piperidine]-6,7-dicarboxylic acid (compound 5e)

Compound 5d (23 mg, 0.1 mmol) was dissolved in 1 ml of ultra-dry N,N-dimethylformamide, followed by the addition of benzyl 1-oxa-6-azaspiro[2.5]octane-6-carboxylate (25mg, 0.1mmol) and sodium hydride (6mg, 0.25mmol), the reaction was heated to 110°C and stirred for 2 days. The reaction solution was prepared by reverse preparation to obtain white solid compound 5e (13 mg, 31%).

$^1$H NMR (600 MHz, $CD_3OD$) δ 7.41 - 7.35 (m, 4H), 7.33 (dd, J = 5.9, 2.9 Hz, 1H), 7.29 (s, 1H), 7.27 (s, 1H), 5.15 (s, 2H), 4.07 (s, 2H), 4.01 (dt, J = 13.6, 4.0 Hz, 2H), 3.42 - 3.32 (m, 2H), 1.82 (d, J = 13.9 Hz, 2H), 1.76 - 1.67 (m, 2H).
LC-MS(ESI): $[M+H]^+$ = 428.36

Step 5: benzyl 7'-(2,6-dioxopiperidin-3-yl)-6',8'-dioxa-7',8'-dihydro-3'*H*,6' *H*-spiro[piperidine-4,2'-[1,4]dioxino[2,3-*f*]isoindole]-1-carboxylate (compound 5f)

Add 3-aminopiperidine-2,6-dione hydrochloride (6 mg, 0.036 mmol) and sodium acetate (7 mg, 0.09 mmol) to a solution of compound 5e (13 mg, 0.03 mmol) in acetic acid (1 mL), and the reaction mixture was heated to 110°C and stirred for 4 hours. After the reaction was completed, the reaction solution was subjected to reverse purification to obtain white solid compound 5f (12 mg, 77%).

$^1$H NMR (600 MHz, CD3OD) δ 7.42 (s, 1H), 7.40 - 7.31 (m, 6H), 5.15 (s, 2H), 5.08 (dd, J = 12.9, 5.5 Hz, 1H), 4.14 (s, 2H), 4.01 (dt, J = 13.8, 4.0 Hz, 2H), 3.43 - 3.32 (m, 2H), 2.91 - 2.83 (m, 1H), 2.79 - 2.69 (m, 2H), 2.14 - 2.09 (m, 1H), 1.83 (d, J = 13.9 Hz, 2H), 1.74 (td, J = 14.4, 12.9, 4.8 Hz, 2H).
LC-MS(ESI): $[M+H]^+$ = 520.29

Step 6: 7'-(2,6-dioxopiperidin-3-yl)-7'-hydrogen-3'*H*,6'*H*-spiro[piperidine-4,2'-[1,4 ]dioxino[2,3-*f*]isoindole]-6',8'-dione (compound 5)

Palladium carbon (5 mg) was added to a methanol solution of compound 5f (11 mg, 0.02 mmol), replaced by hydrogen, and the reaction solution was stirred under hydrogen for 6 hours. The reaction solution was filtered through celite, and the filtrate was concentrated to obtain compound 5 (7 mg, 90%) as a white solid.

$^1$H NMR (400 MHz, DMSO-$d_6$) δ 11.11 (s, 1H), 7.50 (s, 1H), 7.48 (s, 1H), 5.09 (dd, *J* = 12.8, 5.3 Hz, 1H), 4.27 (s, 2H), 3.25 (d, *J* = 12.7 Hz, 2H), 3.18 - 3.11 (m, 2H), 2.89 (ddd, *J* = 16.7, 13.7, 5.3 Hz, 1H), 2.64 - 2.53 (m, 2H), 2.03 (ddd, *J* = 13.3, 5.7, 3.4 Hz, 1H), 1.92 - 1.86 (m, 4H).
LC-MS(ESI): $[M+H]^+$ = 386.32

Example 6

Synthesis of compound 6

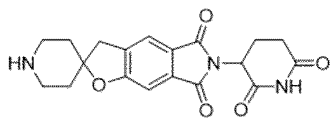

6-(2,6-dioxopiperidin-3-yl)spiro[furo[2,3-f]isoindole-2,4'-piperidine]-5,7(3H,6H)- dione Synthetic scheme

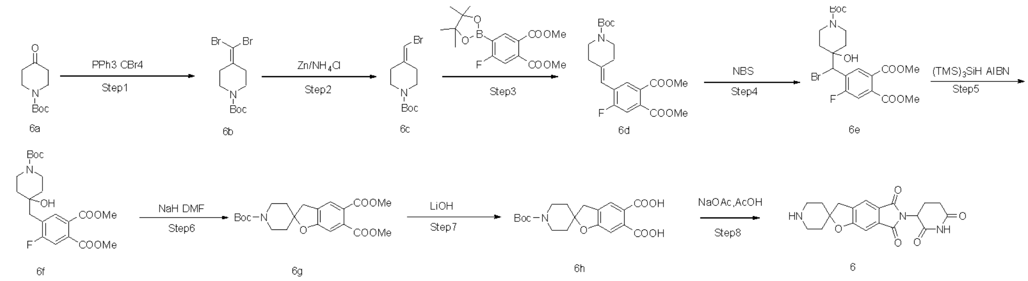


Step 1: tert-butyl 4-(dibromomethyl)piperidine-1-carboxylate (compound 6b)

Under the condition of nitrogen protection, compound 6a (5g, 25.1mmol) and triphenylphosphine (26.3g, 100.4mmol) were dissolved in anhydrous acetonitrile (50mL), and tetrabromo carbonization (16.7g, 50.2mmol) was added to the reaction system in batches at 0°C, after reacting for 30min, the reaction system was raised to room temperature, and reacted overnight. After the reaction was completed, the reaction system was filtered, and the obtained filtrate was concentrated under reduced pressure to obtain crude product, which was purified by column chromatography to obtain a white solid compound 6b (5.5 g, 62%).

$^1$H NMR (600 MHz, DMSO-$d_6$) δ 3.37 (s, 4H), 2.41 (dd, *J* = 7.2, 4.7 Hz, 4H), 1.41 (d, *J* = 0.9 Hz, 9H).

Step 2: tert-butyl 4-(bromomethyl)piperidine-1-carboxylate (compound 6c)

Under the condition of nitrogen protection, compound 6b (5.2g, 18.9mmol) was dissolved in tetrahydrofuran (34mL) and methanol (17mL), ammonium chloride (4.04g, 75.6mmol) was added to the reaction system at 0°C, After reacting at 0°C for 30 min, zinc powder (4.9 g, 75.6 mmol) was added in batches, and reacted overnight at room temperature. After the reaction was completed, the reaction system was filtered, the filter cake was washed with methanol, the obtained filtrate was spin-dried under reduced pressure, and the concentrated crude product was purified by column chromatography to obtain white oily compound 6c (2.6 g, 50%).

$^1$H NMR (600 MHz, DMSO-$d_6$) δ 6.26 (t, *J* = 1.3 Hz, 1H), 3.34 (dt, *J* = 16.4, 6.3 Hz, 4H), 2.32 - 2.27 (m, 2H), 2.23 (ddd, *J* = 7.2, 4.4, 1.2 Hz, 2H), 1.41 (s, 9H).

Step 3: dimethyl 4-(1-(tert-butoxycarbonyl)piperidin-4-yfidene)methyl)-5-fluorophthalate (compound 6d)

Under the condition of nitrogen protection, compound 6c (2g, 7.3mmol), compound 5c (2.64g, 7.8mmol) prepared by step 1 to step 2 of Example 5, palladium acetate (163mg, 0.73mmol), triphenylphosphine (382mg, 1.46mmol), cesium carbonate (7.14g, 21.9mmol) were added to the reaction flask in turn, 1,4-dioxane (20mL) and water (2mL) were added, and after nitrogen replacement, the reaction was carried out at 110°C for 4 hours. After the reaction was completed, the reaction system was cooled to room temperature, and after adding water, it was extracted with ethyl acetate. The organic phase was washed with saturated brine and dried over anhydrous sodium sulfate. The concentrated crude product was purified by column chromatography to obtain light yellow oily compound 6d (1.4 g, 47%).

$^1$H NMR (600 MHz, DMSO-$d_6$) δ 7.65 (d, *J* = 7.0 Hz, 1H), 7.60 (d, *J* = 9.7 Hz, 1H), 6.32 (s, 1H), 3.83 (s, 3H), 3.82 (s, 3H), 3.43 (t, *J* = 5.8 Hz, 2H), 3.35 (s, 1H), 3.33 (s, 1H), 2.37 - 2.32 (m, 2H), 2.25 (t, *J* = 5.9 Hz, 2H), 1.42 (s, 9H).

LC-MS(ESI): $[M-Boc+H]^+$ = 308.28.

Step 4: dimethyl 4-(bromo(1-(tert-butoxycarbonyl)-4-hydroxypiperidin-4-yl)methyl)-5-fluorophthalate (compound 6e)

Under the condition of nitrogen protection, compound 6d (7g, 17.2mmol) was dissolved in tetrahydrofuran (140mL) and water (140mL), N-bromosuccinimide (6.12g, 34.4mmol) was added, and the reaction mixture was carried out overnight at room temperature. After the reaction was completed, excess tetrahydrofuran was removed by concentration under reduced pressure, and after adding water, it was extracted with ethyl acetate. The organic phase was washed with saturated brine and dried over anhydrous sodium sulfate. The concentrated crude product was purified by column chromatography to obtain light yellow oily compound 6e (4.9 g, 56%).

LC-MS(ESI): $[M-Boc+H]^+$ = 404.30/406.30.

Step 5: dimethyl 4-(1-(tert-butoxycarbonyl)-4-hydroxypiperidin-4-yl)methyl)-5-fluorophthalate (compound 6f)

Under the condition of nitrogen protection, compound 6c (10.9g, 21.61mmol) was dissolved in toluene (80ml), and tris(trimethylsilyl)silane (8.06g, 32.42mmol) and azobisisobutyronitrile (357mg, 2.16mmol) were added, the reaction

mixture was carried out overnight at 90°C. After the reaction was completed, excess toluene was removed by concentration under reduced pressure, and the concentrated crude product was purified by column chromatography to obtain light yellow oily compound 6f (1.5 g, 16%).

$^1$H NMR (600 MHz, DMSO-$d_6$) δ 7.77 (d, *J* = 6.9 Hz, 1H), 7.52 (d, *J* = 9.5 Hz, 1H), 4.62 (s, 1H), 3.82 (d, *J* = 2.1 Hz, 6H), 3.66 (s, 2H), 3.34 (s, 4H), 2.79 (s, 2H), 1.38 (s, 9H).

LC-MS(ESI): $[M-Boc+H]^+$ = 326.37.

Step 6: 1'-(tert-butyl)5,6-dimethyl 3H-spiro[benzofuran-2,4'-piperidine]-1',5,6 tricarboxylate (compound 6g)

Under nitrogen protection, compound 6f (1.6g, 3.76mmol) was dissolved in N,N-dimethylformamide (2mL), sodium hydride (300mg, 7.52mmol) was added, and the reaction mixture was carried out overnight at 110°C. After the reaction was completed, water was added, followed by extraction with ethyl acetate. The organic phase was washed with saturated brine, and dried over anhydrous sodium sulfate. The concentrated crude product was purified by reverse purification to obtain 6 g (660 mg, 43%) of a light yellow solid compound.

$^1$H NMR (600 MHz, $CDCl_3$) δ 7.58 (s, 1H), 7.15 (s, 1H), 3.93 (s, 3H), 3.89 (s, 3H), 3.56 (t, J = 5.7 Hz, 2H), 3.42 (t, J = 5.8 Hz, 2H), 2.42 (t, J = 5.9 Hz, 2H), 2.24 (t, J = 5.9 Hz, 2H), 1.61 (s, 2H), 1.50 (s, 9H).

LC-MS(ESI): $[M-Boc+H]^+$ = 306.23.

Step 7: 1'-(tert-butoxycarbonyl)-3H-spiro[benzofuran-2,4'-piperidine]-5,6-dicarboxylic acid (compound 6h)

Under the condition of nitrogen protection, compound 6g (660mg, 1.63mmol) was dissolved in methanol (9ml) and water (1ml), lithium hydroxide (389mg, 16.28mmol) was added, and the reaction mixture was carried out overnight at room temperature. The concentrated crude product was purified by reverse purification to obtain light yellow solid compound 6h (600 mg, 97%).

$^1$H NMR (400 MHz, DMSO-$d_6$) δ 7.39 (s, 1H), 6.37 (s, 1H), 3.17 (s, 2H), 2.29 (d, *J* = 51.9 Hz, 4H), 1.98 (dd, *J* = 13.7, 6.9 Hz, 2H), 1.64 (s, 2H), 1.41 (s, 9H).

LC-MS(ESI): $[M-Boc+H]^+$ = 278.26.

Step 8: 6-(2,6-dioxopiperidin-3-yl)spiro[furo[2,3-f]isoindole-2,4'-piperidine]-5,7(3H ,6H)-dione (Compound 6)

Compound 6h (400mg, 1.06mmol) was dissolved in acetic acid (5mL) solution, 3-aminopiperidine-2,6-dione hydrochloride (169mg, 1.32mmol) and sodium acetate (260mg, 3.18mmol) were added, the reaction was stirred at 110°C for 4 hours. After the reaction was completed, the mixture was purified by reverse phase column to obtain compound 6 (170 mg, 43%) as a white solid.

$^1$H NMR (400 MHz, DMSO-$d_6$) δ 7.46 (s, 1H), 7.11 (s, 1H), 5.04 (dd, J = 12.8, 5.4 Hz, 1H), 2.99 - 2.79 (m, 5H), 2.62 - 2.54 (m, 1H), 2.53 (s, 1H), 2.38 (q, J = 6.3 Hz, 4H), 2.04 - 1.96 (m, 1H), 1.90 (s, 2H).

LC-MS(ESI): $[M+H]^+$ =370.34.

Example 7

Synthesis of compound 7

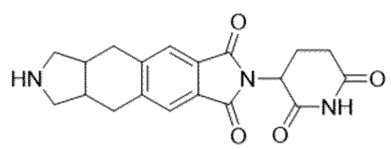

2-(2,6-dioxopiperidin-3-yl)-5a,6,7,8,8a,9-hexahydroisoindolo[5,6-f]isoindole-1,3 (2H,5H)-dionedione

Synthetic scheme

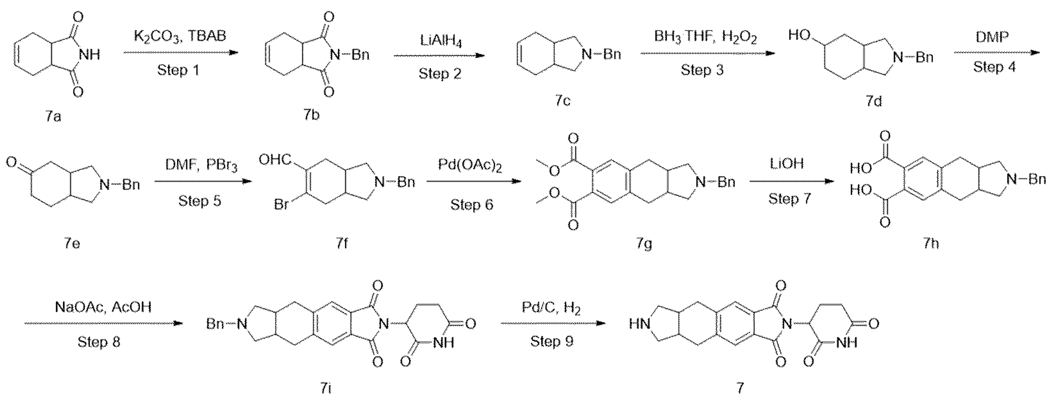


Step 1: 2-benzyl-3a,4,7,7a-tetrahydro-1H-isoindole-1,3(2H)-dione (compound 7b)

Compound 7a (5 g, 33 mmol) was dissolved in anhydrous acetonitrile (50 mL). Tetrabutylammonium bromide (1.23 g, 3.3 mmol) and anhydrous potassium carbonate (14 g, 99 mmol) were added to the solution. Benzyl chloride (5.5 g, 43 mmol) was slowly added to the system and the reaction was carried out overnight at 30°C. The reaction was quenched with water and extracted with ethyl acetate. The organic phase was washed with saturated brine, dried over anhydrous sodium sulfate, and concentrated to obtain compound 7b (7.5 g, 94%) as a white solid.

$^1$H NMR (400 MHz, $CDCl_3$) δ 7.37 - 7.20 (m, 5H), 5.95 - 5.83 (m, 2H), 4.63 (s, 2H), 3.20 - 3.01 (m, 2H), 2.61 (m, 2H), 2.31 - 2.10 (m, 2H).
LC-MS(ESI): $[M+H]^+$ = 242.22

Step 2: 2-benzyl-2,3,3a,4,7,7a-hexahydro-1H-isoindole (compound 7c)

At 0°C, lithium aluminum tetrahydrogen (3.2g, 82mmol) was added to 40mL of anhydrous tetrahydrofuran, then compound 7b (5g, 20.7mmol) was added, and after 5 minutes of ice bathing. the reaction system was moved to a 70°C oil bath, reacted for 3h. After the reaction was completed, water was slowly added dropwise to the reaction solution under ice bath conditions until no bubbles were generated. The filtrate was collected by filtration, extracted with ethyl acetate, and the organic phase was washed with saturated brine and dried over anhydrous sodium sulfate. The compound 7c (3.2 g, 73%) as a yellow oil was obtained by concentration.

$^1$H NMR (400 MHz, $CDCl_3$) δ 7.43 - 7.24 (m, 5H), 5.87 (t, J = 2.7 Hz, 2H), 3.67 (s, 2H), 3.05 - 2.90 (m, 2H), 2.44 (m, 2H), 2.29 - 2.16 (m, 4H), 1.92 (m, 2H).
Step 3: 2-benzyl octahydro-1H-isoindol-5-ol (compound 7d)

Compound 7c (5 g, 23.44 mmol) was dissolved in 20 mL of tetrahydrofuran at 0°C, and 25 mL of 2M borane-tetrahydrofuran complex was added under ice-cooling conditions. After 12 hours of reaction, 13 mL of anhydrous methanol, 10.5mL 3M NaOH solution and 10.5mL hydrogen peroxide were added to the reaction solution. React at 60°C for 6h. After the reaction was completed, it was cooled to room temperature and extracted with ethyl acetate. The organic phase was saturated with saline and dried over anhydrous sodium sulfate. The organic phase was concentrated to obtain a crude product, which was purified by column chromatography to obtain compound 7d (1.2 g, 22.2%).

$^1$H NMR (400 MHz, $CDCl_3$) δ 7.43 - 7.18 (m, 5H), 3.84 (m, 1H), 3.78 (s, 2H), 2.94 (dd, J = 9.8, 6.7 Hz, 1H), 2.82 (dd, J = 9.5, 7.7 Hz, 1H), 2.64 (dd, J = 9.5, 8.4 Hz, 1H), 2.60 - 2.44 (m, 2H), 2.12 (m, 1H), 1.89 - 1.72 (m, 3H), 1.54 (m, 1H), 1.47 - 1.35 (m, 1H), 1.35 - 1.22 (m, 2H).
LC-MS(ESI): $[M+H]^+$ = 232.31

Step 4: 2-benzyl octahydro-5H-isoindol-5-one (compound 7e)

Compound 7d (1.2 g, 12.97 mmol) was dissolved in anhydrous dichloromethane, and Dess Martin oxidant (11 g, 25.95 mmol) was added under ice-cooling conditions, and the reaction was reacted for 12 h. Quench the reaction with saturated sodium bicarbonate and saturated sodium thiosulfate 1:1, collect the filtrate, after filtration, the filtrate was extracted with dichloromethane. Wash with saturated brine, combine the organic phases and dry over anhydrous sodium sulfate. The crude product (1 g) was obtained by concentration and was directly used in the next reaction.

Step 5: 2-benzyl-6-bromo-2,3,3a,4,7,7a-hexahydro-1H-isoindole-5-carbaldehyde (compound 7f)

N,N-Dimethylformamide (1.01 mL, 13.08 mmol) was dissolved in 2 mL of dichloromethane at 0°C. Phosphorus tribromide (1.13 mL, 11.77 mmol) was slowly added dropwise, and stirred at 0° C. for 1 hour. The dichloro solution of compound 7e (1 g, 2.62 mmol) was added dropwise to the above system, and the temperature was raised to room temperature for 10 h. After the reaction, the reaction solution was placed at 0°C and saturated sodium bicarbonate was added until no bubbles were generated. Extract with dichloromethane. The organic phase was washed with saturated brine and dried with anhydrous sodium sulfate. The rude product was obtained by concentration and purified by column chromatography to obtain the target compound 7f (270 mg, 20%).

$^1$H NMR (400 MHz, $CDCl_3$) δ 10.04 (s, 1H), 7.32 (d, J = 5.8 Hz, 5H), 3.60 (s, 2H), 3.21 - 3.10 (m, 2H), 2.85 - 2.72 (m, 3H), 2.50 - 2.43 (m, 1H), 2.33 (dd, J = 9.2, 5.0 Hz, 1H), 2.24 - 2.18 (m, 1H), 1.83 - 1.74 (m, 2H)
LC-MS(ESI): $[M+H]^+$ = 320.16

Step 6: 2-benzyl-2,3,3a,4,9,9a-hexahydro-1H-benzo[f]isoindole-6,7-dicarboxylic acid methyl ester (compound 7g)

Compound 7f (270mg, 0.84mmol), dimethyl itaconate (133.4mg, 0.84mmol), palladium acetate (9.5mg, 0.042mmol), triphenylphosphine (22.11mg, 0.084mmol), sodium acetate (207.5 mg, 2.53mmol) were sequentially added into a pressure vessel, dissolved with tetrahydrofuran, replaced with nitrogen, and heated to 120°C for overnight. After the reaction system was cooled to room temperature, THF was spin-dried, and then the solution was separated and purified by preparative liquid chromatography to obtain compound 7g (71 mg, 22%).
$^1$H NMR (400 MHz, $CDCl_3$) δ 7.52 - 7.39 (m, 7H), 4.38 - 4.14 (m, 3H), 3.91 (d, J = 2.8 Hz, 7H), 3.27 (s, 3H), 2.94 (d, J = 12.0 Hz, 1H), 2.85 - 2.72 (m, 3H), 1.55 (d, J = 6.6 Hz, 1H)
LC-MS(ESI): $[M+H]^+$ = 380.36

Step 7: 2-benzyl-2,3,3a,4,9,9a-hexahydro-1H-benzo[f]isoindole-6,7-dicarboxylic acid (compound 7h)

Compound 7g (71 mg, 0.19 mmol) was dissolved in methanol and water, lithium hydroxide (90 mg, 3.74 mmol) was added, and reacted at room temperature for 20 h. After the reaction, the water and methanol were spin-dried to obtain the crude product (62 mg, 95%). The crude product was used directly in the next step.

LC-MS (ESI): [M+H]+=352.34
Step 8: 7-benzyl-2-(2,6-dioxopiperidin-3-yl)-5a,6,7,8,8a,9-hexahydroisoindolo[5,6- f] Isoindole-1,3(2H,5H)-dione (Compound 7i)

Dissolve compound 7h (62mg, 0.18mmol) in glacial acetic acid, then add sodium acetate (43.2mg, 0.53mmol) and 3-amino-2,6-piperidinedione hydrochloride (44mg, 0.26mmol), and the reaction mixture was reacted in a 110 °C oil bath for overnight. Then methanol and water were spin-dried and the residual product was purified by preparative liquid chromatography to obtain compound 7i (39 mg, 49%).

$^1$H NMR (400 MHz, DMSO-$d_6$) δ 11.14 (s, 1H), 7.98 - 7.72 (m, 2H), 7.64 - 7.41 (m, 5H), 5.14 (dd, J = 12.9, 5.4 Hz, 1H), 4.53 - 4.24 (m, 2H), 4.01 (dd, J = 13.9, 7.6 Hz, 1H), 3.76 (s, 3H), 3.12 (d, J = 9.7 Hz, 1H), 3.06 - 2.91 (m, 2H), 2.92 - 2.76 (m, 3H), 2.67 - 2.54 (m, 2H), 2.04 (dd, J = 12.5, 6.2 Hz, 1H), 1.90 - 1.59 (m, 2H)
LC-MS(ESI): $[M+H]^+$ = 444.41

Step 9: 2-(2,6-dioxopiperidin-3-yl)-5a,6,7,8,8a,9-hexahydroisoindolo[5,6-f]isoindole -1,3(2H,5H)-dione (Compound 7)

Compound 7i (39 mg, 0.088 mmol) was dissolved in 2 mL of methanol, 4 mg of palladium on carbon was added, the reaction was replaced by hydrogen, and reacted overnight at room temperature. Palladium carbon was removed by filtration and methanol was spin-dried, and compound 7 (12 mg, 38%) was obtained by purification by preparative liquid chromatography.

$^1$H NMR (400 MHz, DMSO-$d_6$) δ 11.13 (s, 1H), 9.07 - 8.95 (m, 1H), 7.95 - 7.68 (m, 2H), 5.14 (dd, J = 13.0, 5.3 Hz, 1H), 3.96 - 3.87 (m, 2H), 3.51 (m, 2H), 3.12 (m, 1H), 3.04 - 2.82 (m, 4H), 2.72 - 2.55 (m, 2H), 2.06 (m, 1H), 1.86 (m, 1H), 1.65 - 1.46 (m, 1H)
LC-MS(ESI): $[M+H]^+$ = 354.41

Example 8

Synthesis of Compound 8

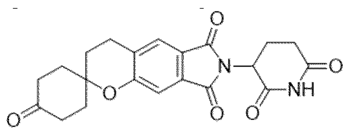

7'-(2,6-dioxopiperidin-3-yl)-3',4'-dihydro-6'H-spiro[cyclohexane-1,2'-pyrano[2,3 -f]isoindole]-4,6',8'(7'H)-trione

Synthetic scheme

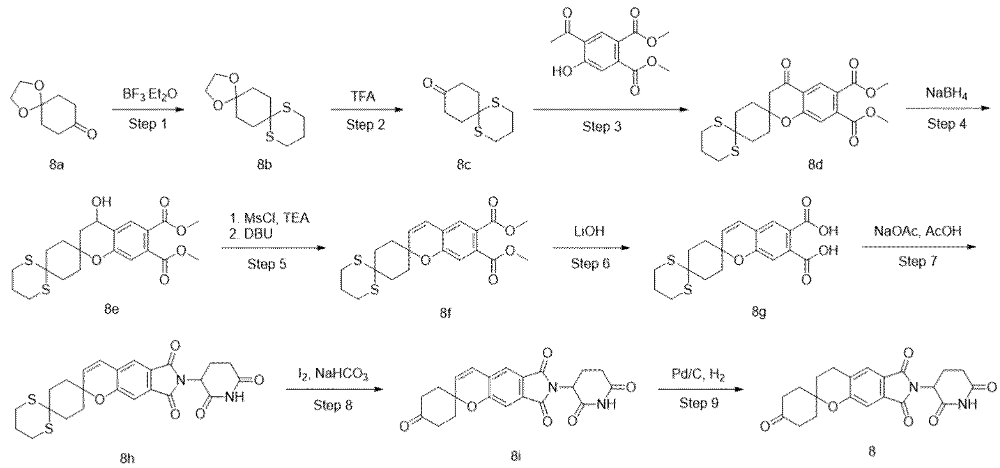

8a 8b 8c 8d 8e 8f 8g 8h 8i 8

Step 1: 1,4-dioxa-9,13-dithiadispiro [4.2.5$^8$.2$^5$]pentadecane (compound 8b)

Compound 8a (10.0 g, 64.0 mmol) was dissolved in 200 mL of dichloromethane, followed by addition of 1,3-propanedithiol (7.0 g, 64.0 mmol). 32 mmol of boron trifluoride ether solution was added dropwise thereto at -18°C. After the addition was complete, the mixture was stirred at -18 °C for 4 h. After the reaction was complete, the solvent was removed under reduced pressure, 500 mL of water was added, and the solid was filtered by suction and purified by C18 column chromatography to obtain compound 8b (2 g, 12.7%).

$^1$H NMR (400 MHz, DMSO-$d_6$) δ 3.85 (s, 4H), 2.84 - 2.78 (m, 4H), 2.06-1.98 (m, 4H), 1.93 - 1.80 (m, 2H), 1.70 - 1.59 (m, 4H)
LC-MS(ESI): $[M+H]^+$ = 247.07

Step 2: 1,5-dithiaspiro[5.5]undecane-9-one (compound 8c)

Compound 8b (2.0 g, 8.0 mmol) was dissolved in 200 mL of dichloromethane, followed by the addition of 100 mL of trifluoroacetic acid at room temperature. After the addition was complete, the mixture was stirred at room temperature for 4 h. After the reaction was complete, sodium bicarbonate (aq) solution was added to the system to adjust the pH to 7. After extraction, the organic phase was dried, concentrated and purified by column chromatography to obtain compound 8c (1.5 g, 91.0%).

$^1$H NMR (400 MHz, DMSO-$d_6$) δ 2.93 - 2.84 (m, 4H), 2.41 - 2.33 (m, 4H), 2.31-2.25 (m, 4H), 1.94 - 1.88 (m, 2H)
LC-MS(ESI): $[M+H]^+$ = 203.05

Step 3: 4-oxodispiro[chroman-2,1'-cyclohexane-4',2"-[1,3] dithiane]-6,7-dicarboxylic acid dimethyl ester ( Compound 8d)

Compound 8c (2.0 g, 9.9 mmol) was dissolved in 200 mL of tetrahydrofuran, then compound 4 (2.0 g, 9.8 mmol), (2.0 g, 28.0 mmol) tetrahydropyrrole were added at room temperature. After the addition was complete, the mixture was stirred at 70 °C for 4 h. After the reaction was completed, the solvent was removed from the system under reduced pressure, and the residue was separated and purified by column chromatography to obtain compound 8d (2.0 g, 46.0%).

$^1$H NMR (400 MHz, DMSO-d6) δ 8.18 (s, 1H), 7.35 (s, 1H), 3.83 (s, 3H), 3.81 (s, 3H), 2.92 (s, 2H), 2.90 - 2.85 (m, 2H), 2.81 - 2.74 (m, 2H), 2.14 - 2.10 (m, 2H), 2.02 - 1.77 (m, 8H).
LC-MS(ESI): $[M+H]^+$ = 437.10

Step 4: 4-hydroxy dispiro [chroman-2,1'-cyclohexane-4',2"-[1,3] dithiane]-6,7-dicarboxylic acid dimethyl ester (compound 8e)

Compound 8d (2.0 g, 4.5 mmol) was dissolved in 50 mL methanol and 50 mL tetrahydrofuran, then sodium borohydride (350.0 mg, 9.0 mmol) was added at room temperature. After the addition was completed, the mixture was stirred at 70 °C for 4 h. After the reaction was completed, the solvent was removed from the system under reduced pressure, and the residue was separated and purified by column chromatography to obtain compound 8e (1.5 g, 75.0%).

$^1$H NMR (600 MHz, DMSO-$d_6$) δ 7.90 (s, 1H), 7.00 (s, 1H), 5.68 (d, *J* = 6.2 Hz, 1H), 4.77-4.71 (m, 1H), 3.79 (s, 6H), 2.96 - 2.72 (m, 4H), 2.16 - 2.01 (m, 4H), 1.93 - 1.79 (m, 5H), 1.77 - 1.70 (m, 3H)
LC-MS(ESI): $[M+H]^+$ = 439.12

Step 5: dispiro[chromene-2,1'-cyclohexane-4',2"-[1,3]dithiane]-6,7-dicarboxylic acid dimethyl ester (compound 8f)

Compound 8e (700 mg, 1.5 mmol) was dissolved in 200 mL of dichloromethane, followed by addition of triethylamine (480.0 mg, 4.5 mmol) and 4-dimethylaminopyridine (100.0 mg, 0.8 mmol) at room temperature. Sulfonyl chloride (720.0 mg, 6.0 mmol) was added dropwise thereto at 0°C. After the addition was completed, the mixture was stirred at 0 °C for 4 h, and the reaction was monitored by TLC. After the reaction was completed, the solvent was removed under reduced pressure. After the addition was completed, the residue was dissolved in 100 mL of toluene, followed by the addition of 1,8-diazabicycloundec-7-ene (1.6 g, 10.0 mmol) at room temperature. The mixture was stirred at 110 °C for 16 h. After the reaction was completed, the solvent was removed from the system under reduced pressure, and the residue was separated and purified by column chromatography to obtain compound 8f (400 mg, 59.0%).

$^1$H NMR (400 MHz, DMSO-$d_6$) δ 7.58 (s, 1H), 7.05 (s, 1H), 6.59 (d, *J* = 9.9 Hz, 1H), 5.95 (d, *J* = 11.4 Hz, 1H), 3.78 (s, 3H), 3.77 (s, 3H), 2.93 - 2.85 (m, 2H), 2.82 - 2.73 (m, 2H), 2.18-2.11 (m, 2H), 2.09 - 1.96 (m, 2H), 1.92 - 1.77 (m, 6H)
LC-MS(ESI): $[M+H]^+$ = 421.11

Step 6: Disspiro[chromene-2,1'-cyclohexane-4',2"-[1,3]dithiane]-6,7-dicarboxylic acid (compound 8g)

Compound 8f (400.0 mg, 0.9 mmol) was dissolved in 50 mL of methanol, 50 mL of tetrahydrofuran, 50 mL of water, and then lithium hydroxide (120.0 mg, 4.5 mmol) was added at room temperature. After the addition was complete, the mixture was stirred at room temperature for 16 h. After the reaction was completed, 1.0M dilute hydrochloric acid solution was added to the system to adjust the pH to 6. After extraction, the organic phase was dried, concentrated and purified by column chromatography to obtain compound 8g (350 mg, 94.0%).

$^1$H NMR (600 MHz, DMSO-$d_6$) δ 12.90 (s, 2H), 7.52 (s, 1H), 6.99 (s, 1H), 6.57 (d, *J* = 9.9 Hz, 1H), 5.90 (d, *J* = 9.9 Hz, 1H), 2.92 - 2.86 (m, 2H), 2.83 - 2.73 (m, 2H), 2.11 (m, 2H), 2.09 - 1.97 (m, 2H), 1.94 - 1.76 (m, 6H)
LC-MS(ESI): $[M+H]^+$ = 393.08

Step 7: 7-(2,6-dioxopiperidin-3-yl)-6H-dispiro[pyrano[2,3-f]isoindole-2,1'-cyclohexane- 4',2"-[1,3]dithiane]-6,8(7H)-dione (compound 8h)

Compound 8g (300.0 mg, 0.7 mmol) was dissolved in 100 mL of acetic acid, followed by addition of 3-aminopiperidine-2,6-dione hydrochloride (170.0 mg, 1.3 mmol), sodium acetate (360.0 mg, 2.1 mmol). After the addition was completed, the mixture was stirred at 110 °C for 4 h. After the reaction was completed, the solvent was removed from the system under reduced pressure, and the residue was separated and purified by column chromatography to obtain

compound 8h (280 mg, 75.0%).

$^1$H NMR (400 MHz, DMSO-$d_6$) δ 11.12 (s, 1H), 7.72 (s, 1H), 7.30 (s, 1H), 6.70 (d, $J$ = 8.0Hz, 1H), 6.03 (d, $J$ = 8.8 Hz, 1H), 5.13-5.07 (m, 1H), 2.94 - 2.84 (m, 3H), 2.84 - 2.76 (m, 2H), 2.64-2.55 (m, 1H), 2.18 - 1.98 (m, 6H), 1.95 - 1.78 (m, 6H)
LC-MS(ESI): $[M+H]^+$ = 485.11

Step 8: 7'-(2,6-oxopiperidin-3-yl)-6'H-spiro[cyclohexane-1,2'-pyrano[2,3-f]isoindole]-4,6',8'(7'H)-trione (compound 8i)

Compound 8h (280.0 mg, 0.5 mmol) was dissolved in 50 mL of acetonitrile, followed by addition of 50 mL of sodium bicarbonate solution, iodine (1.5 g, 5.0 mmol) at room temperature. After the addition was complete, the mixture was stirred at room temperature for 4 h. After the reaction was completed, the solvent was removed under reduced pressure. After extraction, the organic phase was dried, concentrated and purified by column chromatography to obtain compound 8i (100 mg, 44.0%).

$^1$H NMR (600 MHz, DMSO-$d_6$) δ 11.13 (s, 1H), 7.77 (s, 1H), 7.45 (s, 1H), 6.76 (d, $J$ = 10.0 Hz, 1H), 6.09 (d, $J$ = 9.9 Hz, 1H), 5.13-5.09 (m,1H), 2.92-2.84 (m, 1H), 2.78-2.70 (m, 2H), 2.63-2.57 (m, 1H), 2.26-2.15(m, 4H), 2.12 - 2.00 (m, 4H)
LC-MS(ESI): $[M+H]^+$ = 395.12

Step 9: 7'-(2,6-dioxopiperidin-3-yl)-3',4'-dihydro-6'H-spiro[cyclohexane-1,2'-pyrano[2,3-f]isoindole]-4,6',8'(7'H)-trione (compound 8)

Compound 8i (280.0 mg, 0.7 mmol) was dissolved in 50 mL of THF, then Pd/C (150.0 mg) was added at room temperature. After the addition was complete, the mixture was stirred at 50 °C for 4 h under hydrogen atmosphere. After the reaction was completed, the reaction solution was filtered through diatomaceous earth, the solvent was removed under reduced pressure, and compound 8 (195 mg, 70.0%) was obtained after purification by preparative liquid chromatography.

$^1$H NMR (600 MHz, DMSO-$d_6$) δ 11.11 (s, 1H), 7.68 (s, 1H), 7.35 (s, 1H), 5.12-5.06 (m, 1H), 2.99-2.93 (m, 2H), 2.92-2.84 (m, 1H), 2.68 - 2.55 (m, 3H), 2.23-2.15 (m, 2H), 2.13 - 2.00 (m, 4H), 1.99-1.91 (m, 4H)
LC-MS(ESI): $[M+H]^+$ = 397.13

Example 9

Synthesis of compound 9

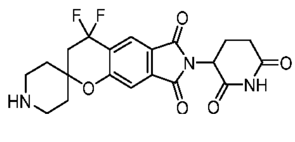

7'-(2,6-dioxopiperidin-3-yl)-4',4'-difluoro-3',4'-dihydro-6'H-spiro[piperidine-4,2' -pyrano[2,3-f]isoindole]-6',8'(7'H)-dione

Synthesis scheme:

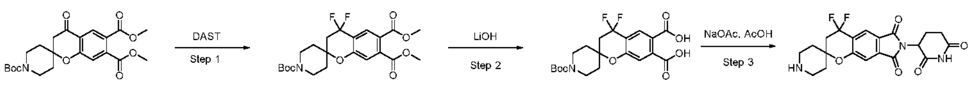


1e 9a 9b 9

Step 1: 1'-(tert-butyl)6,7-dimethyl 4,4-difluorospiro[chroman-2,4'-piperidine]-1',6,7-tricarboxylate (Compound 9a)

Diethylaminosulfur trifluoride (4 mL) was added to compound 1e (2 g, 4.6 mmol), and the reaction solution was stirred at 85° C for 2 hours. The resulting mixture was poured into ice water, extracted three times with ethyl acetate, the organic phase was concentrated in vacuo, and the mixture was purified by reverse column to obtain compound 9a (0.5 g, 24%) as a yellow oily liquid.

$^1H$ NMR (600 MHz, DMSO-$d_6$) δ 8.02 (s, 1H), 7.29 (s, 1H), 3.82 (s, 3H), 3.81 (s, 3H), 3.77 - 3.68 (m, 2H), 3.22 - 3.02 (m, 2H), 2.72 (t, *J* = 14.7 Hz, 2H), 1.85 (d, *J* = 13.9 Hz, 2H), 1.70 (td, *J* = 14.0, 11.6, 4.7 Hz, 2H), 1.40 (s, 9H).

LC-MS(ESI): $[M\text{-}tBu+H]^+$ = 400.31.

Step 2: 1'-(tert-butyl)-4,4-difluorospiro[chroman-2,4'-piperidine]-6,7-dicarboxylic acid (compound 9b)

To a solution of compound 9a (160 mg, 0.4 mmol) in methanol/tetrahydrofuran (2 ml), lithium hydroxide (42 mg, 1.8 mmol) was added slowly. The reaction was stirred overnight at room temperature. The mixture was extracted three times with dichloromethane/methanol (10:1) solvent, and the organic phase was concentrated in vacuo to obtain compound 9b (0.1 g, 67%) as a white solid.

$^1H$ NMR (600 MHz, DMSO-$d_6$) δ 13.27 (br s, 2H), 8.12 (s, 1H), 7.36 (s, 1H), 3.21 - 3.06 (m, 2H), 2.68 (t, *J* = 14.7 Hz, 2H), 2.05 - 1.93 (m, 2H), 1.84 (d, *J* = 13.8 Hz, 2H), 1.68 (td, *J* = 14.0, 12.9, 4.7 Hz, 2H), 1.40 (s, 9H).

LC-MS(ESI): $[M\text{-}tBu+H]^+$ =372.27.

Step 3: 7'-(2,6-dioxopiperidin-3-yl)-4',4'-difluoro-3',4'-dihydro-6'H-spiro[piperidine- 4,2'-pyrano[2,3-f]isoindole]-6',8'(7'H)-dione (compound 9)

To a solution of compound 9b (100mg, 0.23mmol) in acetic acid (1ml) was slowly added 3-aminopiperidine-2,6-dione hydrochloride (77mg, 0.5mmol) and sodium acetate (96mg, 1.2mmol). The reaction was stirred at 110°C for 2 hours. After the reaction was completed, the mixture was purified by reverse phase column to obtain a brown solid compound 9 (50 mg, 51%).

$^1H$ NMR (600 MHz, DMSO-$d_6$) δ 11.15 (s, 1H), 8.10 (s, 1H), 7.68 (s, 1H), 5.16 (dd, *J* = 13.0, 5.4 Hz, 1H), 3.27 - 3.15 (m, 4H), 2.92 - 2.82 (m, 3H), 2.61 (dt, *J* = 17.2, 3.4 Hz, 1H), 2.56 - 2.51 (m, 1H), 2.11 (d, *J* = 14.4 Hz, 2H), 2.05 (ddt, *J* = 12.9, 5.6, 2.5 Hz, 1H), 1.97 - 1.90 (m, 2H).

LC-MS(ESI): $[M+H]^+$ =420.36.

Example 10

Synthesis of Compound 10

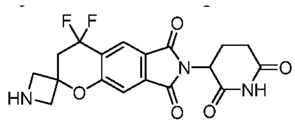

7'-(2,6-dioxopiperidin-3-yl)-4',4'-difluoro-3',4'-dihydro-6'H-spiro[azetidine-3 ,2'-pyrano[2,3-f]isoindole]-6',8'(7'H)-dione

Synthesis scheme:

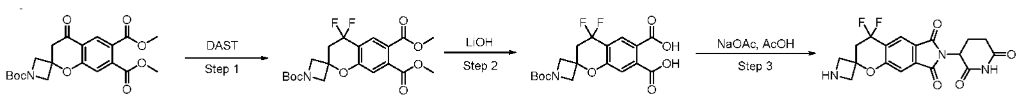


10a 10b 10c 10

Step 1: 1-(tert-butyl) 6',7'-dimethyl 4',4'-difluorospiro[azetidine-3,2'-chromane]-1,6', 7'-tricarboxylate (compound 10b)

10b was prepared by referring to the procedure similar to Step 1 in Example 9.

$^1H$ NMR (400 MHz, DMSO-$d_6$) δ 8.01 (s, 1H), 7.34 (s, 1H), 4.13 (d, *J* = 9.7 Hz, 2H), 3.91 (d, *J* = 9.7 Hz, 2H), 3.83 (s, 3H), 3.82 (s, 3H), 3.04 (t, *J* = 13.4 Hz, 2H), 1.39 (s, 9H).

LC-MS(ESI): $[M\text{-}Boc+H]^+$ = 328.30.

Step 2: 1-(tert-butoxycarbonyl)-4',4'-difluorospiro[azetidine-3,2'-chromane]-6',7'-dicarboxylic acid (compound 10c)

10c was prepared by referring to the similar procedure of step 2 in Example 9.

LC-MS (ESI): [M+H]+=399.28.

Step 3: 7'-(2,6-dioxopiperidin-3-yl)-4',4'-difluoro-3',4'-dihydro-6'H-spiro[azetidine-3,2'-pyrano[2,3-f]isoindole]-6',8'(7'H)-dione (compound 10)

Compound 10 was prepared by referring to the procedure similar to Step 3 in Example 9.

$^1$H NMR (600 MHz, DMSO-$d_6$) δ 11.15 (s, 1H), 8.13 (s, 1H), 7.58 (s, 1H), 5.18 (dd, *J* = 13.0, 5.4 Hz, 1H), 4.33 (d, *J* = 11.7 Hz, 2H), 4.22 (d, *J* = 11.6 Hz, 2H), 3.15 (t, *J* = 13.6 Hz, 2H), 2.94 - 2.87 (m, 1H), 2.65 - 2.59 (m, 1H), 2.54 - 2.51 (m, 1H), 2.10 - 2.05 (m, 1H).

LC-MS(ESI): $[M+H]^+$ = 392.30.

Example 11

Synthesis of Compound 11

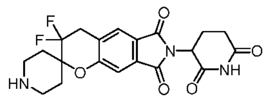

7'-(2,6-dioxopiperidin-3-yl)-3',3'-difluoro-3',4'-dihydro-6'H-spiro[piperidine-4,2' -pyrano[2,3-f]isoindole]-6',8'(7'H)-dione

Synthesis scheme:

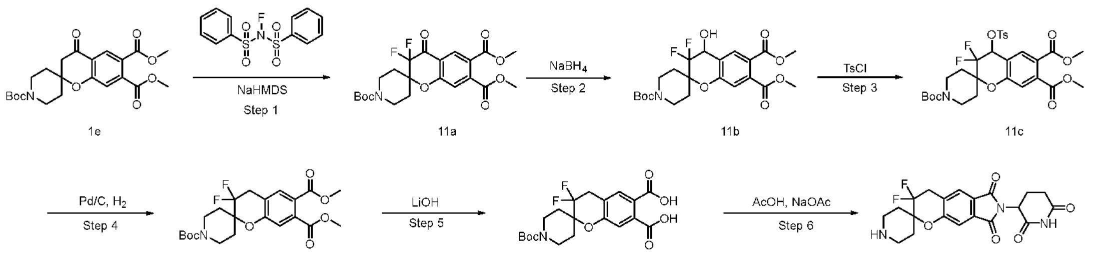


1e 11a 11b 11c 11d 11e 11

Step 1: 1'-(tert-butyl)6,7-dimethyl 3,3-difluoro-4-oxospiro[chromane-2,4'-piperidine]-1',6,7 - tricarboxylate (compound 11a)

Compound 1e (5 g, 11.5 mmol) was dissolved in anhydrous tetrahydrofuran (10 mL). Cool down to - 78°C and add sodium bis(trimethylsilyl)amide (4.23g, 23.07mmol) dropwise, keep this temperature for 2h, then add N-fluorobisbenzenesulfonamide (7.3g, 23.1mmol) dropwise at -78°C. The reaction was stirred overnight at -78°C. The resulting mixture was quenched by pouring into saturated ammonium chloride solution, extracted with ethyl acetate, dried over anhydrous sodium sulfate, filtered and concentrated. The crude product was purified by column chromatography (PE:EA=0-40%) to obtain compound 11a (2.5 g, 46%) as a light yellow solid.

$^1$H NMR (400 MHz, DMSO-$d_6$) δ 8.29 (s, 1H), 7.62 (s, 1H), 3.94 (d, J = 11.6 Hz, 2H), 3.85 (s, 3H), 3.86 (s, 3H), 3.08 (s, 2H), 2.07 (d, J = 13.4 Hz, 2H), 1.74 (td, J = 13.5, 4.7 Hz, 2H), 1.41 (s, 9H).

LC-MS(ESI): $[M+H]^+$ = 470.31.

Step 2: 1'-(tert-butyl)6,7-dimethyl 3,3-difluoro-4-hydroxyspiro[chromane-2,4'-piperidine]-1',6,7-Tricarboxylate (Compound 11b)

Compound 11a (2.5 g, 5.3 mmol) was dissolved in ethanol (5 mL), and sodium borohydride (403 mg, 10.7 mmol) was slowly added at 0°C. The reaction was stirred at room temperature for 1 h. The reaction was quenched with acetone. After concentration in vacuo, the crude product was purified by column chromatography (PE:EA=0-40%) to obtain white solid compound 11b (2.4 g, 95%).

$^1H$ NMR (400 MHz, DMSO-$d_6$) δ 7.90 (s, 1H), 7.23 (s, 1H), 6.68 (d, *J* = 6.1 Hz, 1H), 5.03 (dt, *J* = 16.1, 7.0 Hz, 1H), 3.97 (d, *J* = 14.1 Hz, 1H), 3.91-3.75 (m, 7H), 3.04 (d, *J* = 55.0 Hz, 2H), 1.99 (d, *J* = 13.3 Hz, 1H), 1.84 - 1.74 (m, 2H), 1.70 - 1.56 (m, 1H), 1.41 (s, 9H).

LC-MS(ESI): $[M\text{-}Boc+H]^+$ = 372.34.

Step 3: 1'-(tert-butyl)6,7-dimethyl 3,3-difluoro-4-(tosyloxy)spiro[chromane-2,4'-piperidine]-1 ',6,7-tricarboxylate (compound 11c)

Compound 11b (1.5g, 3.2mmol) was dissolved in dichloromethane (30mL), p-toluenesulfonyl chloride (727mg, 3.8mmol) and triethylamine (642mg, 6.4mmol) were added under ice-cooling conditions. Stir at room temperature for 2h. After concentration in vacuo, the crude product was purified by column chromatography (PE:EA=0-40%) to obtain a white solid compound 11c (1.0 g, 50%).

$^1H$ NMR (600 MHz, DMSO-$d_6$) δ 7.98 (d, *J* = 8.2 Hz, 2H), 7.56 (d, *J* = 8.1 Hz, 2H), 7.41 (s, 1H), 7.35 (s, 1H), 6.25 (dd, *J* = 12.9, 8.1 Hz, 1H), 3.90 (dd, *J* = 21.4, 8.9 Hz, 2H), 3.81 (s, 3H), 3.79 (s, 3H), 3.06 (s, 2H), 2.48 (s, 3H), 1.99 - 1.91 (m, 2H), 1.67 (dtd, *J* = 39.4, 13.3, 4.7 Hz, 2H), 1.41 (s, 9H).

LC-MS(ESI): $[M\text{-}Boc+H]^+$ = 526.34.

Step 4: 1'-(tert-butyl)6,7-dimethyl 3,3-difluorospiro[chromane-2,4'-piperidine]-1',6,7-tricarboxylate (Compound 11d)

Compound 11c (1.0 g, 1.6 mmol) was dissolved in methanol (20 mL), and palladium on carbon (100 mg) was added. Stir under hydrogen atmosphere at room temperature for 12 h. Suction filtration, wash with methanol, and concentration in vacuo to obtain white solid compound 11d (700 mg, 96%).

$^1H$ NMR (600 MHz, DMSO-$d_6$) δ 7.70 (s, 1H), 7.28 (s, 1H), 3.95 - 3.88 (m, 2H), 3.86 - 3.74 (m, 8H), 3.51 (t, *J* = 15.8 Hz, 2H), 1.81 (d, *J* = 13.2 Hz, 2H), 1.69 (td, *J* = 13.6, 4.9 Hz, 2H), 1.42 (s, 9H).

LC-MS(ESI): $[M\text{-}tBu+H]^+$ = 400.44.

Step 5: 1'-(tert-butoxycarbonyl)-3,3-difluorospiro[chromane-2,4'-piperidine]-6,7-dicarboxylic acid (compound 11e)

11e was prepared by referring to the procedure similar to Step 2 in Example 9.

LC-MS (ESI): [M-Boc+H]+=328.32.

Step 6: 7'-(2,6-dioxopiperidin-3-yl)-3',3'-difluoro-3',4'-dihydro-6'H-spiro[piperidine- 4,2'-pyrano[2,3-f]isoindole]-6',8'(7'H)-dione (Compound 11)

Compound 11 was prepared by referring to the procedure similar to Step 3 in Example 9.

$^1H$ NMR (400 MHz, $CD_3OD$) δ 7.78 (s, 1H), 7.55 (s, 1H), 5.13 (dd, *J* = 12.6, 5.4 Hz, 1H), 3.60 (t, *J* = 15.5 Hz, 2H), 3.50 - 3.34 (m, 4H), 2.93 - 2.82 (m, 1H), 2.80 - 2.66 (m, 2H), 2.24 - 2.09 (m, 5H).

LC-MS(ESI): $[M+H]^+$ = 420.41.

Example 12

Synthesis of Compound 12

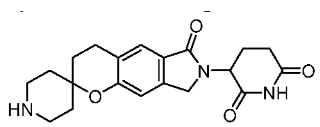

3-(6'-oxo-3',4',6',8'-tetrahydro-7'H-spiro[piperidine-4,2'-pyrano[2,3-f]isoindo Indol]-7'-yl)piperidine-2,6-dione

Synthesis scheme:

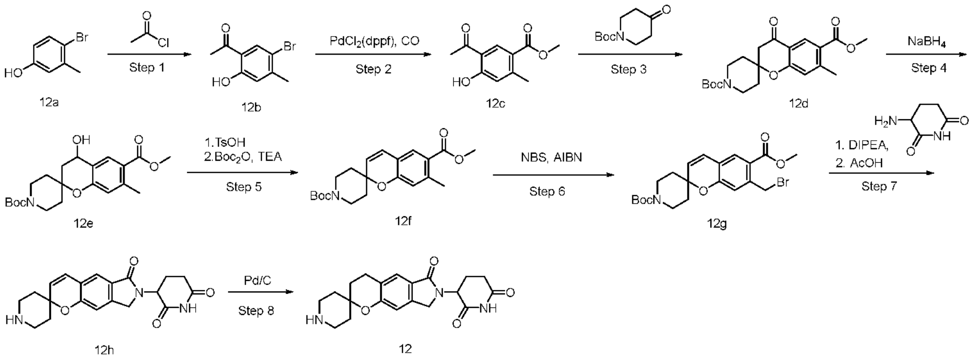


Step 1: 1-(5-bromo-2-hydroxy-4-methylphenyl)ethan-1-one (compound 12b)

A mixture of 4-bromo-3-methylphenol 12a (5.0g, 26.7mmol) and acetyl chloride (10.5g, 134mmol) was stirred at 60°C for 1 hour, then aluminum chloride (5.4g, 40.1 mmo1) was added at room temperature. After stirring at 160° C for 2 hours, the mixture was cooled to room temperature, poured into saturated ammonium chloride solution (50 mL), and extracted with EA (50 mL×3). The combined organic layers were dried over anhydrous sodium sulfate, filtered, and concentrated to afford crude compound 12b as an off-white solid (5.5 g, 90%).

$^1$H NMR (600 MHz, DMSO-$d_6$) δ 11.81 (s, 1H), 8.01 (s, 1H), 7.00 (s, 1H), 2.62 (s, 3H), 2.34 (s, 3H).

Step 2: methyl 5-acetyl-4-hydroxy-2-methylbenzoate (compound 12c)

To a solution of compound 12b (5.5 g, 24.01 mmol) in methanol (50 mL) was added triethylamine (9.72 g, 96.04 mmol) and Pd(dppf)Cl2 (1.76 g, 2.40 mmol). The mixed system was purged with CO three times and then stirred overnight at 60 °C. After the reaction, the system was filtered, the filtrate was concentrated under reduced pressure, and the resulting mixture was purified by column chromatography (PE:EA=6:1) to obtain a white solid 12c (3.26 g, 65%).

$^1$H NMR (600 MHz, DMSO-$d_6$) δ 12.14 (s, 1H), 8.34 (s, 1H), 6.90 (s, 1H), 3.82 (s, 3H), 2.64 (s, 3H), 2.53 (s, 3H).

Step 3: 1'-(tert-butyl) 6-methyl 7-methyl-4-oxospiro[chroman-2,4'-piperidine]-1',6-dicarboxylate (compound 12d)

To a solution of 12c (3.1 g, 14.89 mmol) in ethanol (120 mL) was added N-tert-butoxycarbonyl-3-azetidinone (2.8 g, 16.38 mmol) and tetrahydropyrrole (1.59 g, 22.33 mmol). The reaction was stirred overnight at 80 °C. After completion of the reaction, the resulting mixture was concentrated in vacuo and purified by column chromatography (PE:EA=0-20%) to give yellow solid 12d (1.9 g, 35%).

$^1$H NMR (600 MHz, DMSO-$d_6$) δ 8.25 (s, 1H), 7.08 (s, 1H), 3.82 (s, 3H), 3.72 (s, 2H), 3.14 (s, 2H), 2.89 (s, 2H), 2.55 (s, 3H), 1.91-1.84 (m, 2H), 1.69-1.61 (m, 2H), 1.40 (s, 9H).

LC-MS(ESI): [M-Boc+H]$^+$ =290.27.

Step 4: 1'-(tert-butyl) 6-methyl 4-hydroxyl-7-methylspiro[chroman-2,4'-piperidine]-1',6-dicarboxylate (compound 12e)

To a solution of 12d (1.6 g, 4.43 mmol) in methanol (40 mL) was added sodium borohydride (504 mg, 13.28 mmol). The reaction was stirred at room temperature for 4 hours. After completion of the reaction, the resulting mixture was concentrated in vacuo and purified by column chromatography (PE:EA=0-40%) to give yellow solid 12e (1.5 g, 93.23%).

$^1$H NMR (600 MHz, DMSO-$d_6$) δ 8.01 (s, 1H), 6.73 (s, 1H), 5.52 (d, J = 4.7 Hz, 1H), 4.68 (s, 1H), 3.78 (s, 3H), 3.68 (dd, J = 22.7, 9.0 Hz, 2H), 3.13 (d, J = 77.0 Hz, 2H), 2.46 (s, 3H), 2.13 (dd, J = 13.5, 6.0 Hz, 1H), 1.82-1.62 (m, 4H), 1.59 - 1.50 (m, 1H), 1.41 (s, 9H).

LC-MS(ESI): [M-Boc+H]$^+$ =292.30.

Step 5: 1'-(tert-butyl) 6-methyl 7-methylspiro[chromene-2,4'-piperidine]-1',6-dicarboxylate (compound 12f)

To a solution of 12e (1.5 g, 4.13 mmol) in toluene (40 mL) was added p-toluenesulfonic acid (712 mg, 4.13 mmol). The reaction was stirred overnight at 110°C. After the reaction was complete, the system was concentrated under vacuum to obtain a mixture, which was dissolved in tetrahydrofuran (40 mL), and triethylamine (2.23 g, 22.02 mmol) and di-tert-butyl dicarbonate (2.4 g, 11.01 mmol) were added thereto, and the mixture was stirred overnight at room temperature. After the reaction was completed, the system was concentrated under vacuum, and the resulting mixture was purified by column chromatography (PE:EA=0-30%) to give 12f (1.1 g, 77%) as a yellow solid.

$^{1}$H NMR (600 MHz, DMSO-$d_6$) δ 7.64 (s, 1H), 6.80 (s, 1H), 6.55 (d, *J* = 9.9 Hz, 1H), 5.80 (d, *J* = 9.8 Hz, 1H), 3.78 (s, 3H), 3.70 (d, *J* = 13.0 Hz, 2H), 3.30 - 3.11 (m, 2H), 2.47 (s, 3H), 1.84-1.78 (m, 2H), 1.68-1.60 (m, 2H), 1.41 (s, 9H).
LC-MS(ESI): $[M\text{-}Boc+H]^+$ =274.26.

Step 6: 1'-(tert-butyl)6-methyl 7-(bromomethyl)spiro[chromene-2,4'-piperidine]-1',6-dicarboxylate (compound 12g)

Add N-bromosuccinimide (620 mg, 3.47 mmol) and azobisisobutyronitrile (48 mg, 290 μmol) to a solution of compound 12f (1 g, 2.90 mmol) in carbon tetrachloride (10 mL). The system was replaced with a nitrogen atmosphere, and stirred overnight at 80°C. After the reaction was completed, the system was concentrated under vacuum, and the obtained crude product was directly used in the next step.

Step 7: 3-(6'-oxo-6',8'-dihydro-7'H-spiro[piperidine-4,2'-pyrano[2,3-f]isoindole] -7'-yl) piperidine-2,6-dione (compound 12h)

The mixture obtained in the previous step was dissolved in acetonitrile (40mL), and diisopropylethylamine (1.12g, 8.70mmol) and 3-amino-2,6-piperidinedione (446mg, 3.48mmol) were added thereto, and the mixture was stirrred overnight at 80°C. After concentration, acetic acid (10 mL) was added to continue the reaction for 2 hours. After the reaction was complete, the system was concentrated in vacuo, and the resulting mixture was purified by column chromatography (PE:EA=0-50%) to obtain 12 g (285 mg, 22%) of a black solid.
$^{1}$H NMR (600 MHz, $CD_3OD$) δ 7.52 (s, 1H), 7.13 (s, 1H), 6.69 (d, J = 9.9 Hz, 1H), 5.83 (d, J = 9.9 Hz, 1H), 5.13 (dd, J = 13.3, 5.1 Hz, 1H), 4.45 (dd, J = 35.2, 17.3 Hz, 2H), 3.48-3.40 (m, 2H), 3.38-3.33 (m, 2H), 2.91 (ddd, J = 18.2, 11.8, 5.0 Hz, 2H), 2.82-2.77 (m, 1H), 2.49 (ddd, J = 17.9, 12.7, 3.8 Hz, 1H), 2.29-2.22 (m, 2H), 2.17 (tdd, J = 13.4, 6.7, 4.2 Hz, 1H), 2.03 -1.95 (m, 2H).
LC-MS(ESI): $[M+H]^+$ =368.38.

Step 8: 3-(6'-oxo-3',4',6',8'-tetrahydro-7'H-spiro[piperidine-4,2'-pyrano[2,3- f]isoindole]-7'-yl)piperidine-2,6-dione (compound 12)

Dissolve 12 g of the product from the previous step with methanol (10 mL), add palladium carbon (150 mg) thereto, replace the reaction system with a hydrogen atmosphere, and stir at room temperature for 2 hours. After the reaction is complete, the mixture was filtered, and the filtrate was concentrated under vacuum. Purification by preparative high performance liquid chromatography to obtain compound 12 (120 mg, 35% yield in two steps) as a white solid.
$^{1}$H NMR (600 MHz, DMSO-$d_6$) δ 10.97 (s, 1H), 7.51 (s, 1H), 7.05 (s, 1H), 5.07 (dd, *J* = 13.3, 5.1 Hz, 1H), 4.34 (d, *J* = 16.9 Hz, 1H), 4.22 (d, *J* = 16.9 Hz, 1H), 3.26-3.20 (m, 2H), 3.16-3.08 (m, 2H), 2.94 - 2.86 (m, 3H), 2.63-2.57 (m, 1H), 2.41-2.33 (m, 1H), 2.00 -1.87 (m, 5H), 1.85-1.77 (m, 2H).
LC-MS(ESI): $[M+H]^+$ =370.39.

Example 13

Synthesis of Compound 13

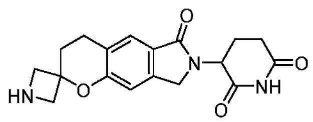

3-(6'-oxo-3',4',6',8'-tetrahydro-7'H-spiro[azetidine-3,2'-pyrano[2,3-f ]isoindole]-7'-yl)piperidine-2,6-dione

Synthesis scheme:

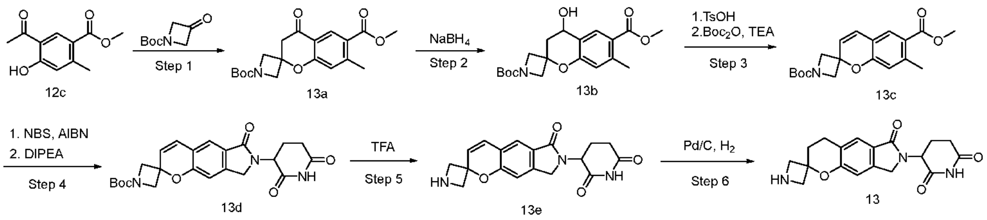


12c 13a 13b 13c 13d 13e 13

Step 1: 1-(tert-butyl) 6'-methyl 7'-methyl-4'-oxospiro[azetidine-3,2'-chroman]-1,6'-di Carboxylate (Compound 13a)

13a was prepared by referring to the procedure similar to Step 3 in Example 12.

$^1$H NMR (600 MHz, DMSO-$d_6$) δ 8.24 (s, 1H), 7.15 (s, 1H), 4.00 (d, *J* = 8.7 Hz, 2H), 3.89 (d, *J* = 8.7 Hz, 2H), 3.82 (s, 3H), 3.20 (s, 2H), 2.56 (s, 3H), 1.38 (s, 9H).

LC-MS(ESI): $[M\text{-}Boc+H]^+$ = 262.25.

Step 2: 1-(tert-butyl) 6'-methyl 4'-hydroxy-7'-methylspiro[azetidine-3,2'-chroman]-1,6'-dicarboxy Ester (compound 13b)

13b was prepared by referring to the procedure similar to Step 4 in Example 12.

$^1$H NMR (600 MHz, $CD_3OD$) δ 8.02 (s, 1H), 6.81 (s, 1H), 4.82 (t, *J* = 5.2 Hz, 1H), 4.28 (d, *J* = 9.6 Hz, 1H), 4.04 (dd, *J* = 19.0, 9.1 Hz, 2H), 3.95 (d, *J* = 9.7 Hz, 1H), 3.87-3.84 (m, 3H), 2.54 (s, 3H), 2.32 (d, *J* = 5.2 Hz, 2H), 1.47 (s, 9H).

LC-MS(ESI): $[M\text{-}Boc+H]^+$ = 264.27.

Step 3: 1-(tert-butyl) 6'-methyl 7'-methylspiro[azetidine-3,2'-chromene]-1,6'-dicarboxylate (compound 13c)

13c was prepared by referring to the similar procedure of Step 5 in Example 12.

$^1$H NMR (600 MHz, DMSO-$d_6$) δ 7.67 (s, 1H), 6.84 (s, 1H), 6.65 (d, *J* = 9.9 Hz, 1H), 6.16 (d, *J* = 9.9 Hz, 1H), 4.04 (s, 4H), 3.78 (s, 3H), 2.47 (s, 3H), 1.40 (s, 9H).

LC-MS(ESI): $[M\text{-}Boc+H]^+$ = 246.23.

Step 4: 7'-(2,6-dioxopiperidin-3-yl)-6'-oxa-7',8'-dihydro-6'H-spiro[azetidine- 3,2'-pyrano[2,3-f]isoindole]-1-carboxylic acid tert-butyl ester (compound 13d)

Add N-bromosuccinimide (620 mg, 3.47 mmol) and azobisisobutyronitrile (48 mg, 290 μmol) to a solution of compound 13c (1 g, 2.90 mmol) in carbon tetrachloride (10 mL).The reaction system was replaced with a nitrogen atmosphere, and stirred overnight at 80°C. The resulting mixture was concentrated under vacuum, dissolved with acetonitrile (40 mL), to which diisopropylethylamine (1.12 g, 8.70 mmol) and 3-amino-2,6-piperidinedione (446 mg, 3.48 mmol) were added, The mixture was stirred overnight at 80 °C. The resulting mixture was concentrated under vacuum and purified by column chromatography (PE:EA=0-50%) toobtain 13d (285 mg, 22%) as a black solid.

$^1$H NMR (600 MHz, $CD_3OD$) δ 7.50 (s, 1H), 7.09 (s, 1H), 6.69 (d, *J* = 9.9 Hz, 1H), 6.17 (d, *J* = 9.8 Hz, 1H), 5.12 (dd, *J* = 13.2, 5.2 Hz, 1H), 4.50-4.40 (m, 2H), 4.24- 4.19 (m, 2H), 4.10 (d, *J* = 8.9 Hz, 2H), 2.94-2.86 (m, 1H), 2.82-2.77 (m, 1H), 2.49 (ddd, *J* = 26.5, 13.2, 4.5 Hz, 1H), 2.19-2.15 (m, 1H), 1.48 (s, 9H).

LC-MS(ESI): $[M+H]^+$ = 440.38.

Step 5: 3-(6'-oxo-6',8'-dihydro-7'H-spiro[azetidine-3,2'-pyrano[2,3-f] isoindole]-7'-yl)piperidine-2,6-dione (compound 13e)

Compound 13d (285 mg, 648.5 μmol) was dissolved in dichloromethane (5 mL) solution, trifluoroacetic acid (636 mg, 6.49 mmol) was added, and the reaction was stirred at room temperature for 2 hours. After the reaction was completed, it was concentrated and used directly in the next step.

LC-MS (ESI): [M+H]+=340.32.

Step 6: 3-(6'-oxo-3',4',6',8'-tetrahydro-7'H-spiro[azetidine-3,2'-pyrano[2 ,3-f]isoindole]-7'-yl)piperidine-2,6-dione (compound 13)

Compound 13 was prepared by referring to the steps similar to Step 8 in Example 12.

$^1$H NMR (400 MHz, $CD_3OD$) δ 7.57 (s, 1H), 7.10 (s, 1H), 5.11 (dd, *J* = 13.3, 5.1 Hz, 1H), 4.50-4.35 (m, 2H), 4.24 (s, 4H), 2.97 (t, *J* = 5.8 Hz, 2H), 2.91- 2.84 (m, 1H), 2.77 (ddd, *J* = 17.6, 4.5, 2.3 Hz, 1H), 2.46 (ddd, *J* = 26.4, 13.2, 4.7 Hz, 1H),

2.28 (t, *J* = 6.1 Hz, 2H), 2.14 (dtd, *J* = 12.7, 5.2, 2.3 Hz, 1H).

LC-MS(ESI): $[M+H]^+$ = 342.32.

Example 14

Synthesis of Compound 14

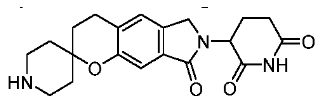

3-(8'-oxo-3',4',6',8'-tetrahydro-7'H-spiro[piperidine-4,2'-pyrano [2,3-f]isoindole]-7'-yl)piperidine-2,6-dione

Synthesis scheme:

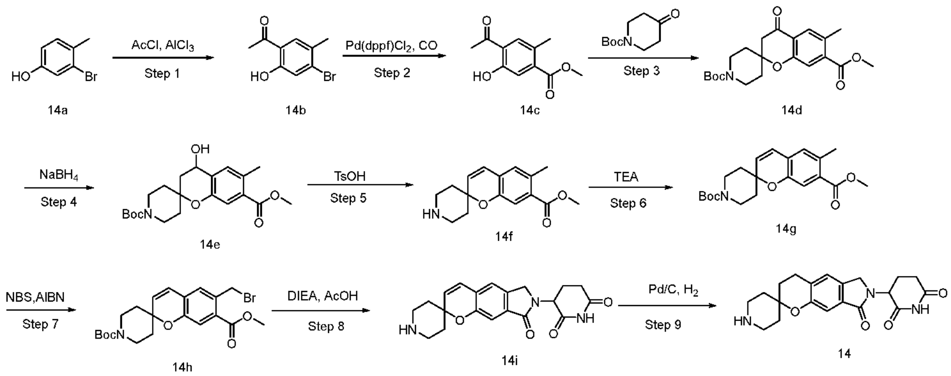


Step 1: 1-(4-bromo-2-hydroxy-5-methylphenyl)ethan-1-one (compound 14b)

14b was prepared by referring to the procedure similar to Step 1 in Example 12. $^1$H NMR (600 MHz, $CDCl_3$) δ 12.10 (s, 1H), 7.57 (s, 1H), 7.25 (s, 1H), 2.63 (s, 3H), 2.39 (s, 3H). Step 2: methyl 4-acetyl-5-hydroxy-2-methylbenzoate (compound 14c)

14c was prepared by referring to the similar procedure of step 2 in Example 12.

$^1$H NMR (600 MHz, $CDCl_3$) δ 11.88 (s, 1H), 7.60 (s, 1H), 7.49 (s, 1H), 3.93 (s, 3H), 2.68 (s, 3H), 2.53 (s, 3H).

Step 3: 1'-(tert-butyl) 7-methyl 6-methyl-4-oxospiro[chroman-2,4'-piperidine]-1',7-dicarboxylate (compound 14d)

14d was prepared by referring to the similar procedure of Step 3 in Example 12.

$^1$H NMR (600 MHz, DMSO-$d_6$) δ 7.64 (s, 1H), 7.45 (s, 1H), 3.85 (s, 3H), 3.71 (s, 2H), 3.12 (d, *J* = 50.1 Hz, 2H), 2.88 (s, 2H), 2.43 (s, 3H), 1.90-1.84 (m, 2H), 1.62 (td, *J* = 12.7, 4.6 Hz, 2H), 1.40 (s, 9H).

LC-MS(ESI): $[M-Boc+H]^+$ = 290.26.

Step 4: 1'-(tert-butyl) 7-methyl 4-hydroxyl-6-methylspiro[chroman-2,4'-piperidine]-1',7-dicarboxylate (compound 14e )

14e was prepared by referring to the similar procedure of Step 4 in Example 12.

$^1$H NMR (600 MHz, $CDCl_3$) δ 7.40 (s, 1H), 7.32 (s, 1H), 4.83 (t, *J* = 7.0 Hz, 1H), 3.90-3.84 (m, 5H), 3.31- 3.05 (m, 2H), 2.93 (s, 1H), 2.49 (s, 3H), 2.11 (dd, *J* = 13.6, 6.1 Hz, 1H), 1.92-1.85 (m, 2H), 1.78-1.72 (m, 1H), 1.63 (td, *J* = 13.3, 4.6 Hz, 1H), 1.52 (ddd, *J* = 24.7, 12.6, 7.9 Hz, 1H), 1.46 (s, 9H).

LC-MS(ESI): $[M-Boc+H]^+$ = 292.27.

Step 5: methyl 6-methylspiro[chroman-2,4'-piperidine]-7-carboxylate (compound 14f)

Compound 14e (15.23 g, 38.91 mmol) and p-toluenesulfonic acid (6.7 g, 38.91 mmol) were dissolved in toluene, and the reaction was refluxed overnight at 110° C. The reaction mixture was concentrated in vacuo to obtain the resulting light yellow oily mixture 14f (8.6 g, 80%). The crude product was used directly in the next step.
LC-MS (ESI): [M-Boc+H]+=274.29.

Step 6: 1'-(tert-butyl) 7-methyl 6-methylspiro[chromene-2,4'-piperidine]-1',7-dicarboxylate (compound 14g)

Compound 14f (8.6g, 31.46mmol), di-tert-butyl dicarbonate (13.73g, 62.93mmol) and triethylamine (9.55g, 94.39mmol) were dissolved in dichloromethane (30mL), and the mixture was stirred at room temperature for 4 hours. The resulting mixture was concentrated under vacuum and purified by column chromatography (PE:EA=0-30%) to obtain 14g (1.8g, 83%) of colorless solid.
$^1$H NMR (600 MHz, $CDCl_3$) δ 7.41 (s, 1H), 6.85 (s, 1H), 6.37 (d, *J* = 9.8 Hz, 1H), 5.65 (d, *J* = 9.6 Hz, 1H), 3.98-3.77 (m, 5H), 3.28 (s, 2H), 2.50 (s, 3H), 1.97 (d, *J* = 13.4 Hz, 2H), 1.59 (td, *J* = 13.4, 4.7 Hz, 2H), 1.47 (s, 9H).
LC-MS(ESI): $[M-Boc+H]^+$ = 274.24.

Step 7: 1'-(tert-butyl) 7-methyl 6-(bromomethyl)spiro[chromene-2,4'-piperidine]-1',7-dicarboxylate (compound 14h)

14h was prepared by referring to the similar procedure of Step 6 in Example 12.

Step 8: 3-(8'-oxo-6',8'-dihydro-7'H-spiro[piperidine-4,2'-pyrano[2,3-f]isoindole] -7'-yl) piperidine-2,6-dione (compound 14i)

14i was prepared by referring to the similar steps of Step 7 in Example 12.
$^1$H NMR (600 MHz, DMSO-$d_6$) δ 11.00 (s, 1H), 7.36 (s, 1H), 7.25 (s, 1H), 6.68 (d, *J* = 9.8 Hz, 1H), 5.99 (d, *J* = 9.8 Hz, 1H), 5.08 (dd, *J* = 13.3, 5.2 Hz, 1H), 4.37 (d, *J* = 16.9 Hz, 1H), 4.25 (d, *J* = 16.9 Hz, 1H), 3.26 - 3.19 (m, 4H), 2.90 (ddd, *J* = 17.3, 13.7, 5.5 Hz, 1H), 2.64-2.57 (m, 1H), 2.39 (qd, *J* = 13.2, 4.5 Hz, 1H), 2.10 - 2.03 (m, 2H), 2.00 (dtd, *J* = 12.8, 5.4, 2.4 Hz, 1H), 1.94 - 1.86 (m, 2H).
LC-MS(ESI): $[M+H]^+$ = 368.37.

Step 9: 3-(8'-oxo-3',4',6',8'-tetrahydro-7'H-spiro[piperidine-4,2'-pyrano[2,3- f]isoindole]-7'-yl)piperidine-2,6-dione (compound 14)

Compound 14 was prepared by referring to the steps similar to Step 8 in Example 12.
$^1$H NMR (600 MHz, DMSO-$d_6$) δ 10.99 (s, 1H), 7.36 (s, 1H), 7.18 (s, 1H), 5.07 (dd, *J* = 13.3, 5.1 Hz, 1H), 4.34 (d, *J* = 16.6 Hz, 1H), 4.21 (d, *J* = 16.5 Hz, 1H), 3.25-3.11 (m, 4H), 2.94-2.87 (m, 3H), 2.63- 2.57 (m, 1H), 2.39 (qd, *J* = 13.2, 4.5 Hz, 1H), 2.01-1.95 (m, 1H), 1.93-1.87 (m, 4H), 1.82-1.74 (m, 2H).
LC-MS(ESI): $[M+H]^+$ = 370.36.

Example 15

Snthesis of Compound 15

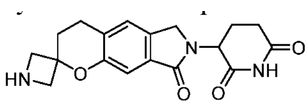

3-(8'-oxo-3',4',6',8'-tetrahydro-7'H-spiro[azetidine-3,2'-pyrano[2,3-f ]isoindole]-7'-yl)piperidine-2,6-dione

Synthesis scheme:

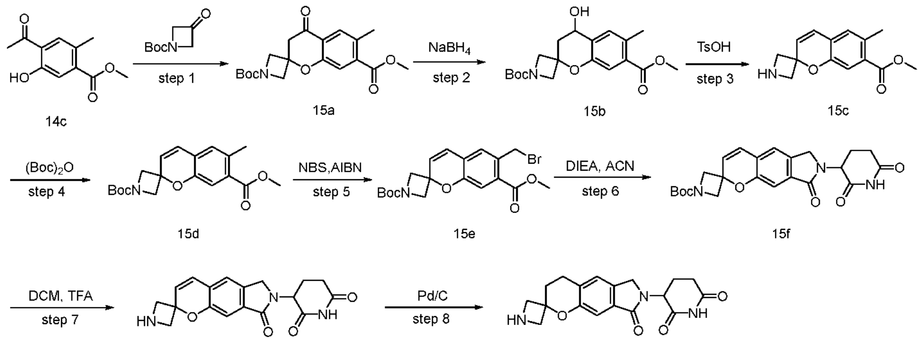


14c 15a 15b 15c 15d 15e 15f 15g 15

Step 1: 1-(tert-butyl) 7'-methyl 6'-methyl-4'-oxaspiro[azetidine-3,2'-chroman]-1,7'-dicarboxylate (Compound 15a)

15a was prepared by referring to the similar steps of Step 3 in Example 12.

$^1$H NMR (600 MHz, $CDCl_3$) δ 7.74 (s, 1H), 7.59 (s, 1H), 4.09 (d, *J* = 9.6 Hz, 2H), 3.97 (d, *J* = 9.5 Hz, 2H), 3.94 (s, 3H), 3.07 (s, 2H), 2.53 (s, 3H), 1.46 (s, 9H).

LC-MS(ESI): $[M\text{-}Boc+H]^+$ =262.28.

Step 2: 1-(tert-butyl) 7'-methyl 4'-hydroxy-6'-methylspiro[azetidine-3,2'-chroman]-1,7'-dicarboxy ester (compound 15b)

15b was prepared by referring to the similar procedure of Step 4 in Example 12.

$^1$H NMR (600 MHz, $CDCl_3$) δ 7.50 (s, 1H), 7.24 (s, 1H), 4.89 (q, *J* = 5.2 Hz, 1H), 4.36 (q, *J* = 7.2 Hz, 1H), 4.27 - 4.23 (m, 1H), 4.10 (d, *J* = 9.3 Hz, 1H), 4.00 (dd, *J* = 17.3, 9.6 Hz, 2H), 3.90 (s, 3H), 2.54 (s, 3H), 2.35 (d, *J* = 5.3 Hz, 2H), 1.47 (s, 9H).

LC-MS(ESI): $[M\text{-}Boc+H]^+$ =264.27.

Step 3: methyl 6'-methylspiro[azetidine-3,2'-chromene]-7'-carboxylate (compound 15c)

15c was prepared by referring to the similar step of Step 5 in Example 14.

LC-MS (ESI): $[M+H]^+$=246.22.

Step 4: 1-(tert-butyl) 7'-methyl 6'-methylspiro[azetidine-3,2'-chromene]-1,7'-dicarboxylate (compound 15d)

15d was prepared by referring to the similar procedure of Step 6 in Example 14.

$^1$H NMR (600 MHz, $CDCl_3$) δ 7.45 (s, 1H), 6.88 (s, 1H), 6.47 (d, *J* = 9.8 Hz, 1H), 6.06 (d, *J* = 9.7 Hz, 1H), 4.24 (d, *J* = 9.5, 2H), 4.01 (d, *J* = 9.5, 2H), 3.89 (s, 3H), 2.51 (s, 3H), 1.48 (s, 9H).

LC-MS(ESI): $[M\text{-}tBu+H]^+$ =290.20

Step 5: 1-(tert-butyl)7'-methyl 6'-(bromomethyl)spiro[azetidine-3,2'-chromene]-1,7'-dicarboxylate (Compound 15e)

15e was prepared by referring to the similar procedure of Step 6 in Example 12.

Step 6: 7'-(2,6-dioxopiperidin-3-yl)-8'-oxo-7',8'-dihydro-6'H-spiro[azetidine- 3,2'-pyrano[2,3-f]isoindole]-1-carboxylic acid tert-butyl ester (compound 15f)

3-amino-2,6-piperidinedione hydrochloride (173 mg, 1.05 mmol) and N,N-diisopropylethylamine ( 0.4mL) were added to a solution of compound 15e (2 g, 0.7 mmol) in acetonitrile (15 mL). The reaction solution was stirred at 80 °C for 12 hours. The resulting mixture was concentrated in vacuo, and purified by C18 reverse phase column to obtain compound 15f (120 mg, 38%) as a gray solid.

$^1$H NMR (400 MHz, DMSO-*d*$_6$) δ 10.97 (s, 1H), 7.35 (s, 1H), 7.13 (s, 1H), 6.71 (d, *J* = 9.9 Hz, 1H), 6.33 (d, *J* = 9.8 Hz, 1H), 5.08 (dd, *J* = 13.3, 5.1 Hz, 1H), 4.41 - 4.19 (m, 2H), 4.15 - 3.96 (m, 4H), 2.97 - 2.85 (m, 1H), 2.66 - 2.55 (m, 1H), 2.41 - 2.30 (m, 1H), 2.03 - 1.95 (m, 1H), 1.40 (s, 9H).
LC-MS(ESI): $[M\text{-}tBu+H]^+$ =384.35

Step 7: 3-(8'-oxo-6',8'-dihydro-7'H-spiro[azetidine-3,2'-pyrano[2,3-f]isoindole]-7'-yl)piperidine-2,6-dione (compound 15g)

Compound 15f (120 mg, 0.7 mmol) dichloromethane (3 mL), trifluoroacetic acid (1 mL). The reaction mixture was stirred at room temperature for 2 hours, and the reaction mixture was concentrated in vacuo. The crude product was used directly in the next step without further purification.
LC-MS (ESI): [M+H]+=340.34

Step 8: 3-(8'-oxo-3',4',6',8'-tetrahydro-7'H-spiro[azetidine-3,2'-pyrano[2 ,3-f]isoindole]-7'-yl)piperidine-2,6-dione (compound 15)

Compound 15 was prepared by referring to the step similar to Step 8 in Example 12.

$^1$H NMR (400 MHz, DMSO-$d_6$) δ 10.97 (s, 1H), 7.36 (s, 1H), 7.11 (s, 1H), 5.07 (dd, *J* = 13.3, 5.1 Hz, 1H), 4.34 (d, *J* = 16.8 Hz, 1H), 4.21 (d, *J* = 16.8 Hz, 1H), 4.15 - 4.04 (m, 4H), 2.91 (q, *J* = 5.6, 4.4 Hz, 2H), 2.67 - 2.56 (m, 2H), 2.40 - 2.31 (m, 1H), 2.20 (t, *J* = 6.9 Hz, 2H), 1.98 (m, 1H).
LC-MS(ESI): $[M+H]^+$ =342.43

Example 16

Synthesis of Compound 16

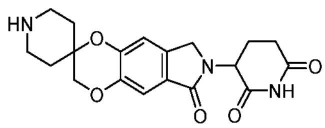

3-(6'-oxo-6',8'-dihydro-3'H,7'H-spiro[piperidine-4,2'-[1,4]dioxino[2,3-f]isoindole]-7'-yl)piperidine-2,6-dione

Synthesis scheme:

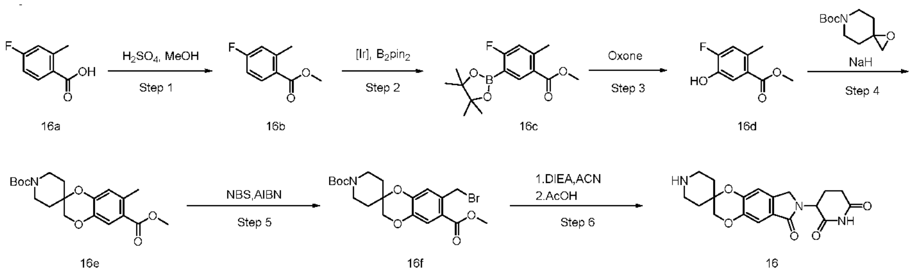


16a 16b 16c 16d 16e 16f 16

Step 1: methyl 4-fluoro-2-methylbenzoate (compound 16b)

16b was prepared by referring to the procedure similar to Step 1 in Example 1.
$^1$H NMR (600 MHz, DMSO-$d_6$) δ 7.90 (dd, *J* = 8.7, 6.2 Hz, 1H), 7.22 (dd, *J* = 10.1, 2.8 Hz, 1H), 7.15 (td, *J* = 8.5, 2.7 Hz, 1H), 3.82 (s, 3H), 2.53 (s, 3H).
LC-MS(ESI): $[M+H]^+$ = 169.19.

Step 2: methyl 4-fluoro-2-methyl-5-(4,4,5,5-tetramethyl-1,3,2-dioxaborolan-2-yl)benzoate (compound 16c)

16c was prepared by referring to the similar procedure of step 2 in Example 5.
$^1$H NMR (600 MHz, DMSO-$d_6$) δ 8.16 (d, *J*= 6.3 Hz, 1H), 7.19 (dd, *J* = 10.4, 3.2 Hz, 1H), 3.83 (s, 3H), 2.55 (s, 3H), 1.30 (s, 12H).

Step 3: methyl 4-fluoro-5-hydroxyphthalate (compound 16d)

16d was prepared by referring to the similar procedure of Step 3 in Example 5.
$^1H$ NMR (600 MHz, DMSO-$d_6$) δ 10.04 (s, 1H), 7.47 (d, *J* = 9.3 Hz, 1H), 7.13 (d, *J* = 12.2 Hz, 1H), 3.80 (s, 3H), 2.41 (s, 3H).
LC-MS(ESI): $[M+H]^-$ = 183.21.

Step 4: 1'-(tert-butyl)6-methyl 7-methyl-3H-spiro[benzo[b][1,4]dioxin-2,4'-piperidine]-1' ,6-dicarboxylate (compound 16e)

Compound 16d (1.0 g, 5.4 mmol) was dissolved in 10 ml of ultra-dry N,N-dimethylformamide, followed by the addition of tert-butyl 1-oxa-6-azaspiro[2.5]octane-6-carboxylate ester (1.2g, 5.3mmol) and sodium hydride (196mg, 4.9mmol), the reaction was heated to 110°C and stirred overnight. The reaction solution was subjected to silica gel column chromatography to obtain white solid compound 16e (1.4 g, 68%).
$^1H$ NMR (600 MHz, DMSO-$d_6$) δ 7.37 (s, 1H), 6.85 (s, 1H), 4.04 (s, 2H), 3.77 (s, 3H), 3.73 (d, *J* = 13.4 Hz, 2H), 3.24 - 3.04 (m, 2H), 2.42 (s, 3H), 1.66 - 1.61 (m, 4H), 1.41 (s, 9H).
LC-MS(ESI): $[M+H]^+$ = 278.29.

Step 5: 1'-(tert-butyl)6-methyl 7-(bromomethyl)-3H-spiro[benzo[b][1,4]dioxin-2,4'-piperidine] -1',6-dicarboxylate (compound 16f)

To a solution of compound 16e (1.0 g, 2.7 mmol) in carbon tetrachloride (10 mL) was added N-bromosuccinimide (613 mg, 3.4 mmol) and azobisisobutyronitrile (44 mg, 0.3 mmol), After nitrogen replacement three times, the reaction was stirred overnight at 85°C under nitrogen protection. After the reaction was completed, the reaction solution was filtered, and the filtrate was concentrated to obtain a crude product (1.2 g), which was used in the next reaction without further purification.

Step 6: 3-(6'-oxo-6',8'-dihydro-3'H,7'H-spiro[piperidine-4,2'-[1,4]dioxino[ 2,3-f]isoindole]-7'-yl)piperidine-2,6-dione (compound 16)

To a solution of compound 16f (1.2 g, 2.7 mmol) in acetonitrile (10 mL) was added 3-aminopiperidine-2,6-dione hydrochloride (655 mg, 4.0 mmol) and N,N-diisopropylethylamine (1.0g, 8.0mmol), the reaction solution was stirred overnight at 80°C, then the reaction solution was concentrated and dissolved in acetic acid (10mL), and the reaction solution was stirred at 110°C for 2h. The reaction solution was purified by reverse phase to obtain white solid compound 16 (235 mg, 24%).
$^1H$ NMR (400 MHz, DMSO-$d_6$) δ 10.96 (s, 1H), 7.22 (s, 1H), 7.17 (s, 1H), 5.06 (dd, *J* = 13.3, 5.1 Hz, 1H), 4.32 (d, *J* = 16.8 Hz, 1H), 4.20 (d, *J* = 16.8 Hz, 1H), 4.19 - 4.11 (m, 2H), 3.30 - 3.25 (m, 2H), 3.18 - 3.08 (m, 2H), 2.90 (ddd, *J* = 17.3, 13.6, 5.4 Hz, 1H), 2.59 (dt, *J* = 16.6, 3.4 Hz, 1H), 2.42 - 2.30 (m, 1H), 2.00 - 1.93 (m, 1H), 1.92 - 1.85 (m, 4H).
LC-MS(ESI): $[M+H]^+$ = 373.35.

Example 17

Synthesis of compound 17

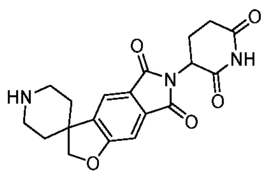

6-(2,6-dioxopiperidin-3-yl)-6-hydro-2H, 5H-spiro[[2,3-f]isoindole-3,4'-piperidin]-5, 7-dione Synthetic scheme

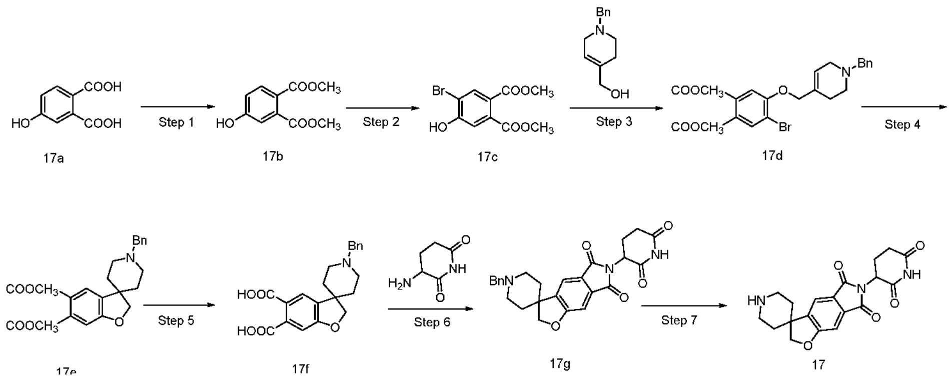


17a 17b 17c 17d 17e 17f 17g 17

Step 1: dimethyl 4-hydroxyphthalate (compound 17b)

17b was prepared by referring to the same procedure as Step 1 in Example 1.
LC-MS (ESI): [M-OMe+H]+=179.12.

Step 2: dimethyl 4-bromo-5-hydroxyphthalate (compound 17c)

Under nitrogen protection, compound 17b (10 g, 47 mmol) was dissolved in trifluoroacetic acid (100 mL), N-bromosuccinimide (4.24 g, 47 mmol) was added to the reaction system, and the reaction mixture was reacted overnight at room temperature. The resulting reaction system was concentrated under reduced pressure, and after adding water (100 mL), it was extracted with ethyl acetate (100 mL*3 times). Wash with saturated brine (100ml), combine the organic phases and dry over anhydrous sodium sulfate. The concentrated crude product was purified by reverse column chromatography to obtain light yellow solid compound 17c (4.6 g, 33%).
$^1$H NMR (600 MHz, $CD_3OD$) δ 7.98 (s, 1H), 7.07 (s, 1H), 3.88 (s, 3H), 3.86 (s, 3H).
LC-MS(ESI): $[M+H]^+$ = 289.04.

Step 3: dimethyl 4-((1-benzyl-1,2,3,6-tetrahydropyridin-4-yl)methoxy)-5-bromobenzoate (compound 17d)

Under nitrogen protection conditions, compound 17c (8g, 27mmol, 1.0eq), (1-benzyl-1,2,3,6-tetrahydropyridin-4-yl)methanol (5.64g, 27mmol) and triphenyl phosphine (7.2g, 27mmol) were dissolved in tetrahydrofuran (80mL), diethyl azodicarboxylate (4.08mL, 27mmol, 1.0eq) was added, and the reaction mixture was reacted at room temperature for 4 hours. The resulting reaction system was concentrated under reduced pressure, water (50 mL) was added to the system, and the reaction mixture was extracted with ethyl acetate (100 mL*3 times). Wash with saturated brine (100 mL), combine the organic phases and dry over anhydrous sodium sulfate. Compound 17d (6 g, 46%) was obtained as a reddish-brown solid after purification by column chromatography.
$^1$H NMR (600 MHz, $CDCl_3$) δ 8.04 (s, 1H), 7.47 (hd, *J* = 5.4, 1.9 Hz, 5H), 7.09 (s, 1H), 5.90 (tt, *J* = 3.2, 1.5 Hz, 1H), 4.67 - 4.55 (m, 2H), 4.38 (d, *J* = 12.9 Hz, 1H), 4.28 (d, *J* = 12.8 Hz, 1H), 3.98 (d, *J* = 16.7 Hz, 1H), 3.93 (s, 3H), 3.91 (s, 3H), 3.78 (dd, *J* = 19.0, 12.3 Hz, 1H), 3.48 (d, *J* = 16.5 Hz, 1H), 3.06 (s, 1H), 2.75 (s, 1H), 2.54 (d, *J* = 18.6 Hz, 1H).
LC-MS(ESI): $[M+H]^+$ = 474.26/476.27.

Step 4: dimethyl 1'-benzyl-2H-spiro[benzofuran-3,4'-piperidine]-5,6-dicarboxylate (compound 17e)

Under the condition of nitrogen protection, compound 17d (8.4g, 17mmol), tri-n-butyltin hydrogen (10.2g, 34mmol) and azobisisobutyronitrile (574mg, 3.4mmol) were dissolved in toluene (84mL), heated to 110°C for reaction 4 hours. The reaction system was cooled to room temperature, concentrated under reduced pressure, added water (100 mL), and extracted with ethyl acetate (100 mL*3 times). Wash with saturated brine (100 mL), combine the organic phases and dry over anhydrous sodium sulfate. After purification by column chromatography, reddish-brown oily compound 17e (5 g, 71%) was obtained.
$^1$H NMR (400 MHz, $CDCl_3$) δ 7.67 (s, 1H), 7.55 - 7.41 (m, 5H), 6.97 (s, 1H), 4.47 (s, 2H), 4.27 (s, 2H), 3.91 (s, 3H), 3.89 (s, 3H), 3.70 (d, *J* = 12.3 Hz, 2H), 2.78 - 2.63 (m, 2H), 2.52 (td, *J* = 14.3, 4.0 Hz, 2H), 1.94 (d, *J* = 14.5 Hz, 2H).
LC-MS(ESI): $[M+H]^+$ = 396.40.

Step 5: 1'-benzyl-2H-spiro[benzofuran-3,4'-piperidine]-5,6-dicarboxylic acid (compound 17f)

Compound 17e (2.3 g, 5.8 mmol) was dissolved in methanol (20 mL) and water (2 mL), and lithium hydroxide

(1.39 g, 58 mmol) was added to the system. React at room temperature for 4 hours. The reaction system was concentrated under reduced pressure, and compound 17f (1.28 g, 60%) was obtained as a light reddish brown solid after reverse purification.

$^1$H NMR (600 MHz, DMSO-$d_6$) δ 7.51 (ddd, *J* = 12.6, 6.6, 3.4 Hz, 6H), 6.98 (s, 1H), 4.64 (s, 2H), 4.36 (s, 2H), 3.39 (s, 2H), 3.13 (t, *J* = 13.4 Hz, 2H), 2.15 (dd, *J* = 20.6, 8.4 Hz, 2H), 1.96 (d, *J* = 14.1 Hz, 2H).

LC-MS(ESI): $[M+H]^+$ = 368.34.

Step 6: 1'-benzyl-6-(2,6-dioxopiperidin-3-yl)-6-hydro-2H,5H-spiro[[2,3-f]isoindole-3 ,4'-piperidine]-5,7-dione (compound 17g)

Under nitrogen protection conditions, compound 17f (3.0g, 8mmol), sodium acetate (3.3g, 24mmol) and compound 3-amino-2,6-piperidinedione hydrochloride (1.3g, 10mmol, 1.25eq) were dissolved in acetic acid (30 mL), the system was reacted at 110° C for 4 hours. The reaction system was cooled to room temperature, concentrated under reduced pressure, added water (50 mL), and extracted with ethyl acetate (100 mL*3 times). Wash with saturated brine (80 mL), combine the organic phases and dry over anhydrous sodium sulfate. After purification by column chromatography, 17g (1.8g, 48%) of light yellow solid compound was obtained.

$^1$H NMR (600 MHz, $CDCl_3$) δ 7.69 (s, 1H), 7.54 - 7.43 (m, 5H), 7.25 (s, 1H), 4.96 (dd, *J* = 12.7, 5.4 Hz, 1H), 4.56 (s, 2H), 4.28 (s, 2H), 2.97 - 2.75 (m, 4H), 2.70 (t, *J* = 13.6 Hz, 2H), 2.63 - 2.53 (m, 2H), 1.99 (d, *J* = 14.6 Hz, 2H), 1.63 - 1.57 (m, 2H).

LC-MS(ESI): $[M+H]^+$ = 460.34.

Step 7: 6-(2,6-dioxopiperidin-3-yl)-6-hydro-2H, 5H-spiro[[2,3-f]isoindole-3,4'-piperidine ]-5,7-dione (compound 17)

Under nitrogen protection, compound 17g (3.0g, 6.5mmol, 1.0eq) was dissolved in methanol (30mL), and 10% wet palladium on carbon (600mg) was added to the system. After hydrogen replacement for 3 times, the reaction was carried out at room temperature overnight. The reaction system was filtered, the filter cake was washed three times with methanol (15 mL), the obtained filtrate was spin-dried under reduced pressure, and the concentrated crude product was purified by reverse column chromatography to obtain white solid compound 17 (630 mg, 26%) .

$^1$H NMR (600 MHz, $CD_3OD$) δ 7.78 (s, 1H), 7.26 (d, *J* = 0.6 Hz, 1H), 5.14 - 5.10 (m, 1H), 4.74 (s, 2H), 3.38 (dt, *J* = 12.7, 3.2 Hz, 2H), 3.05 (td, *J* = 13.1, 3.0 Hz, 2H), 2.90 - 2.85 (m, 1H), 2.78 (dd, *J* = 4.4, 2.6 Hz, 1H), 2.77 - 2.69 (m, 2H), 2.14 (ddt, *J* = 13.2, 11.0, 4.1 Hz, 4H).

LC-MS(ESI): $[M+H]^+$ = 370.33.

Example 18

Synthesis of compound 18

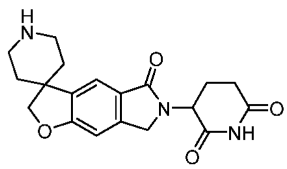

3-(5-oxo-5,7-dihydro-2H,6H-spiro[furo[2,3-f] isoindole-3,4'-piperidin]-6-yl)piperidine-2,6-dione Synthesis scheme:

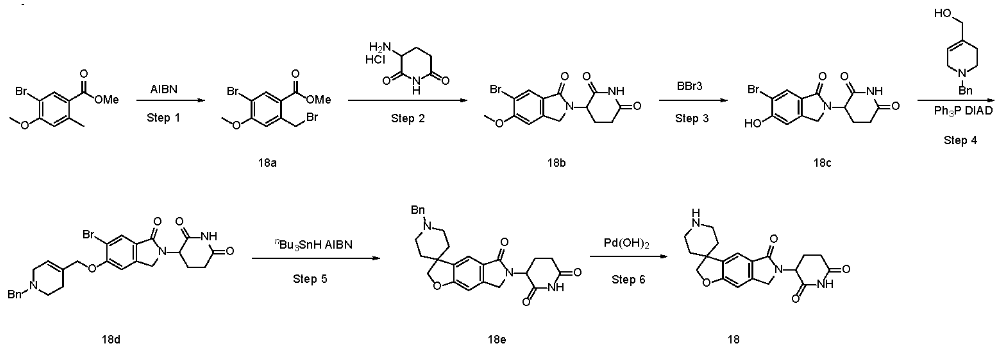


Step 1: methyl 5-bromo-2-(bromomethyl)-4-methoxybenzoate (compound 18a)

Under nitrogen protection conditions, methyl 5-bromo-4-methoxy-2-methylbenzoate (5g, 19.30mmol), NBS (3.61g, 20.30mmol) and AIBN (316.9mg, 1.90mmol) were dissolved in carbon tetrachloride (50 mL), the temperature was raised to 75° C, then react overnight. After the reaction was completely completed as detected by TLC, the reaction system was cooled to room temperature and then saturated aqueous sodium thiosulfate solution was added. After stirring for 30 minutes, it was extracted with dichloromethane, washed with saturated brine, and the organic phases were combined and dried over anhydrous sodium sulfate. After concentration, the crude product was purified by column chromatography to obtain white solid compound 18a (6.9 g, 96.7%).
LC-MS (ESI): $[M+H]^+$=336.90

Step 2: 3-(6-bromo-5-methoxy-1-oxoisoindol-2-yl)piperidine-2,6-dione (compound 18b)

Under nitrogen protection conditions, compound 18a (7g, 20.70mmol) and 3-amino-2,6-piperidinedione hydrochloride (5.1g, 31.10mmol) were dissolved in acetonitrile (100mL), and N , N-diisopropylethylamine (13.4g, 103.60mmol) was added to the reaction system, react overnight at 80°C. The obtained reaction system was concentrated under reduced pressure, and after adding acetic acid (50 mL), the reaction was carried out at 120°C for 2 hours. After the reaction was completely completed as detected by TLC, the reaction system was concentrated under reduced pressure and purified by reverse column chromatography to obtain solid compound 18b (5.3 g, 72.5%).
LC-MS (ESI): $[M+H]^+$=353.15

Step 3: 3-(6-bromo-5-hydroxyl-1-oxoisoindol-2-yl)piperidine-2,6-dione (compound 18c)

Under nitrogen protection, compound 18b (5 g, 14.20 mmol) was dissolved in dichloromethane (10 mL), and a solution of boron tribromide in dichloromethane (1 M, 140 mL) was slowly added at 0°C, and the reaction was carried out overnight at room temperature after the addition was complete. After the reaction was completely completed by TLC detection, the resulting reaction system was concentrated under reduced pressure to remove most of the boron tribromide, then dichloromethane (50 mL) was added to the system, and the reaction system was quenched with a small amount of methanol. The reaction system was concentrated under reduced pressure, and purified by reverse column chromatography to obtain an off-white solid compound 18c (3.6 g, 75.0%).
LC-MS (ESI): $[M+H]^+$=338.99

Step 4: 3-(5-((1-benzyl-1,2,3,6-tetrahydropyridin-4-yl)methoxy)-6-bromo-1-oxoisoindole-2 -yl) piperidine-2,6-dione (compound 18d)

Compound 18d was prepared by referring to the similar procedure of Step 3 in Example 17.

$^1$H NMR (600 MHz, DMSO-$d_6$) δ 11.00 (s, 1H), 7.90 (s, 1H), 7.55- 7.46 (m, 5H), 7.42 (s, 1H), 5.91 (s, 1H), 5.08 (dd, J = 13.3, 5.1 Hz, 1H), 4.75 (s, 2H), 4.45-4.34 (m, 3H), 4.27 (d, J = 17.5 Hz, 1H), 3.71 (s, 2H), 3.56 (s, 1H), 3.17 (s, 1H), 2.91 (ddd, J = 17.3, 13.6, 5.5 Hz, 1H), 2.63- 2.57 (m, 1H), 2.41 (ddd, J = 21.9, 16.2, 11.7 Hz, 3H), 2.00 (dtd, J = 12.7, 5.4, 2.3 Hz, 1H).
LC-MS(ESI): $[M+H]^+$ = 524.09

Step 5: 3-(1'-benzyl-5-oxo-5,7-dihydro-2H,6H-spiro[furo[2,3-f]isoindole-3,4'- piperidine]-6-yl)piperidine-2,6-dione (compound 18e)

Compound 18e was prepared by referring to the similar procedure of Step 4 in Example 17.

$^1$H NMR (600 MHz, DMSO-$d_6$) δ 10.97 (s, 1H), 7.56-7.47 (m, 5H), 7.42 (s, 1H), 7.04 (s, 1H), 5.03 (dd, J = 13.2, 4.9 Hz, 1H), 4.64 (q, J = 9.3 Hz, 2H), 4.39-4.33 (m, 3H), 4.23 (d, J = 17.3 Hz, 1H), 3.33-3.24 (m, 2H), 3.16 (d, J = 16.2 Hz, 2H), 2.94-2.84 (m, 1H), 2.59 (d, J = 16.7 Hz, 1H), 2.41-2.32 (m, 1H), 2.16 (t, J = 12.4 Hz, 2H), 2.00-1.92 (m, 3H).
LC-MS(ESI): $[M+H]^+$ = 446.35

Step 6: 3-(5-oxo-5,7-dihydro-2H, 6H-spiro[furo[2,3-f]isoindole-3,4'-piperidin]-6-yl) piperidine-2,6-dione (compound 18)

Under nitrogen protection, compound 18e (25 mg, 0.06 mmol) was dissolved in methanol (2 mL), and 10% wet palladium/carbon (24 mg) was added to the system. After hydrogen replacement for 3 times, the reaction was carried out at room temperature overnight. The reaction system was filtered, the filter cake was washed three times with methanol (5 mL), the obtained filtrate was spin-dried under reduced pressure, and the concentrated crude product was purified by reverse column chromatography to obtain a white solid compound 18 (8.7 mg, 43%).
$^1$H NMR (600 MHz, DMSO-$d_6$) δ 10.97 (s, 1H), 7.47 (s, 1H), 7.04 (s, 1H), 5.06 (dd, J = 13.3, 5.1 Hz, 1H), 4.65-4.60 (m, 2H), 4.36 (d, J = 17.2 Hz, 1H), 4.24 (d, J = 17.2 Hz, 1H), 3.36-3.34 (m, 2H), 3.04 (d, J = 8.5 Hz, 2H), 2.95-2.86 (m, 1H), 2.60 (d, J = 17.1 Hz, 1H), 2.38 (qd, J = 13.2, 4.4 Hz, 1H), 2.10-2.03 (m, 2H), 2.00-1.95 (m, 1H), 1.91-1.85 (m, 2H).
LC-MS(ESI): $[M+H]^+$ = 356.35

Example 19

Synthesis of compound 19

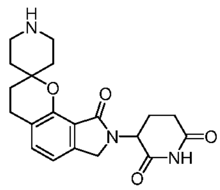

3-(9'-oxo-3',4',7',9'-tetrahydro-8'H-spiro[piperidine-4,2'-pyrano[2,3-e] isoindole ]-8'-yl)piperidine-2,6-dione

Synthesis scheme:

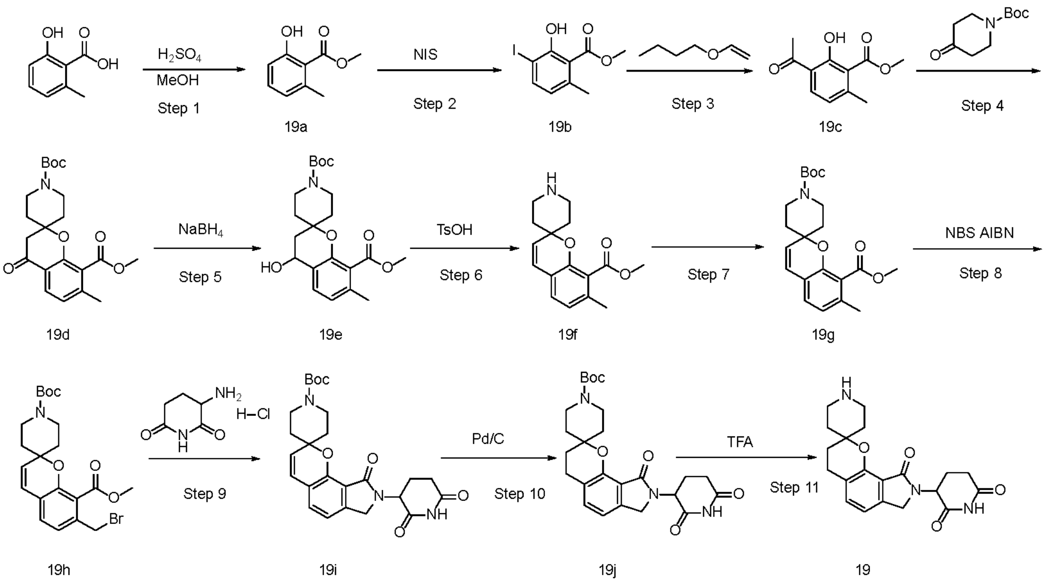


Step 1: methyl 2-hydroxy-6-methylbenzoate (compound 19a)

19a was prepared by referring to the similar procedure of Step 1 in Example 1.

$^1$H NMR (600 MHz, $CDCl_3$) δ 11.31 (s, 1H), 7.31-7.28 (m, 1H), 6.87-6.86 (m, 1H), 6.75-6.74 (m, 1H), 3.99 (s, 3H), 2.56 (s, 3H).
LC-MS (ESI): $[M+H]^+$ = 167.13

Step 2: methyl 2-hydroxy-3-iodo-6-methylbenzoate (compound 19b)

Under nitrogen protection, compound 19a (10 g, 60.24 mmol) was dissolved in trifluoroacetic acid (20 mL), NIS (16.2 g, 72.00 mmol) was added, and the mixture was kept at room temperature for 16 h. After the reaction was complete by TLC detection, the reaction was quenched with saturated $Na_2S_2O_3$ aqueous solution, washed with water, extracted with ethyl acetate, dried over anhydrous sodium sulfate, concentrated to obtain a crude product, which was purified in reverse to obtain compound 19b (3.8 g, 21.6%).
$^1$H NMR (600 MHz, $CDCl_3$) δ 12.19 (s, 1H), 7.77 (d, J = 8.0 Hz, 1H), 6.56 (dd, J = 8.0, 0.8 Hz, 1H), 4.01 (s, 3H), 2.54 (s, 3H).

Step 3: methyl 3-acetyl-2-hydroxy-6-methylbenzoate (compound 19c)

Under nitrogen protection condition, compound 19b (3.8g, 13.02mmol) and butyl vinyl ether (3.9g, 39.00mmol) were dissolved in methanol (20ml), $Pd(dppf)Cl_2$ (951mg, 1.30mmol) and TEA (3.95 g, 39.00 mmol) were added. Nitrogen replacement three times, and the reaction was performed at 60°C for 16h. The end of the reaction was detected by TLC, the reaction solution was spin-dried, dissolved by adding dichloromethane, washed with 5M/L HCl, washed with saturated brine, combined the organic phases, dried over anhydrous sodium sulfate, and concentrated through the column to obtain compound 19c (2.0 g, 73.8%).

$^1$H NMR (600 MHz, $CDCl_3$) δ 12.74 (s, 1H), 7.70 (d, J = 8.2 Hz, 1H), 6.79 (d, J = 8.2 Hz, 1H), 3.97 (s, 3H), 2.64 (s, 3H), 2.38 (s, 3H).
LC-MS (ESI): $[M-OCH_3+H]^+$ = 177.14

Step 4: 1'-tert-butyl-8-methyl-7-methyl-4-oxospiro[chroman-2,4'-piperidine]-1,8-dicarboxylate (compound 19d)

19d was prepared by referring to the similar procedure of Step 3 in Example 12.

$^1$H NMR (600 MHz, DMSO-$d_6$) δ 7.72 (d, J = 8.0 Hz, 1H), 6.99 (d, J = 8.0 Hz, 1H), 3.87 (s, 3H), 3.78 (br, 2H), 2.99 (br, 2H), 2.84 (s, 2H), 2.28 (s, 3H), 1.96-1.78 (m, 2H), 1.59 (td, J = 13.3, 4.8 Hz, 2H), 1.40 (s, 9H).
LC-MS (ESI): $[M+H]^+$ = 390.35

Step 5: 1'-tert-butyl-8-methyl-4-hydroxyl-7-oxospiro[chroman-2,4'-piperidine]-1,8-dicarboxylate (compound 19e)

19e was prepared by referring to the similar procedure of Step 4 in Example 12.

$^1$H NMR (600 MHz, DMSO-$d_6$) δ 7.37 (d, J = 7.8 Hz, 1H), 6.80 (d, J = 7.9 Hz, 1H), 5.44 (d, J = 5.5 Hz, 1H), 4.67 (q, J = 6.7 Hz, 1H), 3.80 (s, 3H), 3.76 (br, 2H), 2.97 (br, 2H), 2.17 (s, 3H), 2.06 (dd, J = 13.5, 6.2 Hz, 1H), 1.85-1.66 (m, 3H), 1.60-1.47 (m, 2H), 1.41 (s, 9H).
LC-MS (ESI): $[M+H]^+$ = 392.40

Step 6: methyl 7-methyloxospiro[chromene-2,4'-piperidine]-8-carboxylate (compound 19f)

Under nitrogen protection, compound 19e (1.5g, 3.83mmol) was dissolved in toluene (25mL), p-toluenesulfonic acid (659.8mg, 3.83mmol) was added, and the reaction mixture was carried out at 110°C for 12 hours. After the reaction was detected by TLC, the obtained reaction system was concentrated under reduced pressure and the crude compound 19f was directly used in the next step (850 mg, 81%).
LC-MS (ESI): $[M+H]^+$=274.25

Step 7: 1'-tert-butyl-8-methyl-7-methylspiro[chromene-2,4'-piperidine]-1',8-dicarboxylate (compound 19g)

Under the condition of nitrogen protection, compound 19f (850mg, 3.1 1mmol) was dissolved in dichloromethane (20mL), BoczO (1.36g, 6.22mmol) and TEA (600mg, 6.22mmol) were added to the reaction system, and the reaction mixture was carried out at room temperature to 25°C for 1 hour. After the reaction was detected by TLC, the reaction system was cooled to room temperature, concentrated under reduced pressure, added water, extracted with ethyl acetate, washed with saturated brine, combined with organic phases and dried over anhydrous sodium sulfate. After purification by column chromatography, 19g (1g, 86%) of white solid compound was obtained.

$^1$H NMR (600 MHz, DMSO-$d_6$) δ 7.07 (d, J = 7.6 Hz, 1H), 6.78 (d, J = 7.6 Hz, 1H), 6.48 (d, J = 9.8 Hz, 1H), 5.72 (d, J = 9.8 Hz, 1H), 3.83 (s, 5H), 3.07 (s, 2H), 2.18 (s, 3H), 1.81 (dq, J = 14.3, 2.6 Hz, 2H), 1.57 (td, J = 13.1, 4.8 Hz, 2H), 1.41 (s, 9H).
LC-MS(ESI): $[M+H]^+$ = 374.40

Step 8: 1'-tert-butyl-8-methyl-7-(bromomethyl)spiro[chromene-2,4'-piperidine]-1',8-dicarboxylate (compound 19h)

19h was prepared by referring to the similar procedure of Step 6 in Example 12.
LC-MS (ESI): $[M+H]^+$=452.25

Step 9: 8'-(2,6-dioxopiperidin-3-yl)-9'-oxo-8',9'-dihydro-7'H-spiro[piperidine-4,2 '-pyrano[2,3-e]isoindole]-1-carboxylic acid tert-butyl ester (compound 19i)

Under nitrogen protection, compound 19h (1.2g, 2.65mmol), DIPEA (1g, 7.95mmol) and 3-amino-2,6-piperidinedione hydrochloride (654.9mg, 3.98mmol) were dissolved in acetonitrile (30 mL), the system was reacted at 85°C for 16 hours. After the reaction was detected by TLC, the reaction system was cooled to room temperature, concentrated under reduced pressure, added water, extracted with ethyl acetate, washed with saturated brine, combined with organic phases and dried over anhydrous sodium sulfate. Compound 19i (400 mg, 32%) was obtained as a gray solid after purification by column chromatography.

$^1$H NMR (600 MHz, DMSO-$d_6$) δ 11.00 (s, 1H), 7.31 (d, J = 7.5 Hz, 1H), 7.04 (d, J = 7.5 Hz, 1H), 6.57 (d, J = 9.9 Hz, 1H), 5.79 (d, J = 9.9 Hz, 1H), 4.99 (d, J = 5.1 Hz, 1H), 4.44 - 4.16 (m, 2H), 3.79 (s, 2H), 3.19 (d, J = 22.8 Hz, 4H), 2.87 (ddd, J = 18.2, 13.6, 5.4 Hz, 1H), 2.73-2.56 (m, 1H), 2.51 (p, J = 1.9 Hz, 1H), 2.06-1.56 (m, 3H), 1.41 (s, 9H).
LC-MS(ESI): $[M\text{-}Boc+H]^+$ = 368.19

Step 10: 8'-(2,6-dioxopiperidin-3-yl)-9'-oxo-4',7',8',9'-tetrahydro-3'H-spiro[ tert-butyl piperidine-4,2'-pyrano[2,3-e]isoindole]-1-carboxylate (compound 19j)

Under nitrogen protection, compound 19i (400 mg, 0.85 mmol) was dissolved in THF (10 mL), and 10% wet palladium on carbon (250 mg) was added to the system. After hydrogen replacement for 3 times, the reaction was carried out overnight at 50°C. After the reaction was detected by TLC, the reaction system was filtered, the filter cake was washed three times with methanol (15mL), the obtained filtrate was spin-dried under reduced pressure, and the concentrated

crude solid compound 19j was directly used in the next step (380mg, 94%) .
LC-MS (ESI): $[M-Boc+H]^+$=370.29

The eleventh step: 3-(9'-oxo-3',4',7',9'-tetrahydro-8'H-spiro[piperidine-4,2'-pyrano[2,3 -e]isoindole]-8'-yl)piperidine-2,6-dione (compound 19)

Under nitrogen protection, compound 19j (380 mg, 0.80 mmol) was dissolved in dichloromethane (5 mL), and TFA (2 mL) was added to the system. React for 1 h at 25°C. After the reaction was detected by TLC, the reaction solution was spin-dried under reduced pressure, and a yellow solid compound 19 (290 mg, 97%) was prepared by pre-HPLC.

$^1$H NMR (600 MHz, DMSO-$d_6$) δ 10.98 (s, 1H), 7.34 (d, J = 7.7 Hz, 1H), 7.04 (d, J = 7.6 Hz, 1H), 4.97 (dd, J = 13.3, 5.2, 1H), 4.43 - 4.20 (m, 2H), 3.30-3.08 (m, 4H), 2.97-2.78 (m, 3H), 2.61 (dt, J = 17.1, 3.7 Hz, 1H), 2.47-2.31 (m, 1H), 2.04-1.88 (m, 5H), 1.80 (tt, J = 12.6, 5.0 Hz, 2H).
LC-MS(ESI): $[M+H]^+$ = 370.39

Example 20

Synthesis of compound 20

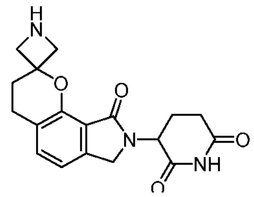

3-(9'-oxo-3',4',7',9'-tetrahydro-8'H-spiro[azetidine-3,2'-pyrano[2,3-e ]isoindole]-8'-yl)piperidine-2,6-dione

Synthesis scheme:

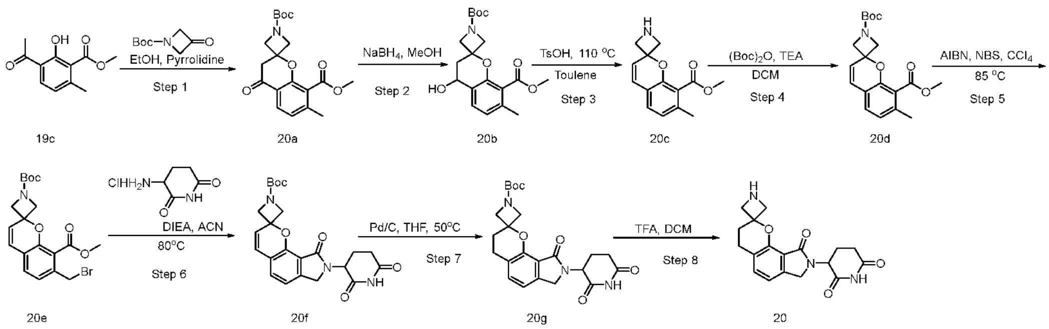


Step 1: 1-tert-butyl-8'-methyl-7'-methyl-4'-oxospiro[azetidine-3,2'-chroman]-1,8'-di carboxylate (Compound 20a)

Compound 20a was prepared by referring to the procedure similar to Step 3 in Example 12.

$^1$H NMR (600 MHz, $CDCl_3$) δ 7.84 (d, J = 8.0 Hz, 1H), 6.96 (d, J = 8.0 Hz, 1H), 4.09 (d, J = 9.5 Hz, 2H), 3.99 (s, 3H), 3.96 (d, J = 9.5 Hz, 2H), 3.06 (s, 2H), 2.39 (s, 3H), 1.46 (s, 9H).
LC-MS (ESI): $[M-Boc+H]^+$ = 262.17

Step 2: 1-tert-butyl-8'-methyl-4'-hydroxy-7'-methylspiro[azetidine-3,2'-chroman]-1,8'-dicarboxy ester (Compound 20b)

Compound 20b was prepared by referring to the similar steps of Step 4 in Example 12.
LC-MS (ESI): $[M-Boc+H]^+$=264.17

Step 3: 7'-methylspiro[azetidine-3,2'-chromene]-8'-methyl carboxylate (compound 20c)

Under nitrogen protection, compound 20b (900.0 mg, 2.48 mmol) was dissolved in toluene (10 mL), TsOH (471 mg, 2.48 mmol) was added, and the reaction mixture was carried out at 110° C for 2 h. After the reaction was detected by TLC, the reaction solution was spin-dried to obtain the crude compound 20c (1.1 g, 181.1%), which could be directly carried out in the next step without purification.
LC-MS (ESI): $[M+H]^+$=246.11

Step 4: 1-tert-butyl-8'-methyl-7'-methylspiro[azetidine-3,2'-chromene]-1,8'-dicarboxylate (compound 20d)

Under nitrogen protection, compound 20c (1.1g, 1eq) was dissolved in dichloromethane (15mL), $(Boc)_2O$ (1.95g, 2.0eq) and TEA (1.36g mg, 3.0eq) were added, and the reaction mixture was performed at room temperature for 2h. After the reaction was detected by TLC, the reaction solution was spin-dried and passed through the column to obtain compound 20d (300.0 mg, 19.4%).

$^1$H NMR (600 MHz, $CDCl_3$) δ 6.95 (d, J = 7.6 Hz, 1H), 6.76 (d, J = 7.6 Hz, 1H), 6.45 (d, J = 9.8 Hz, 1H), 5.93 (d, J = 9.8 Hz, 1H), 4.24 (d, J = 9.5 Hz, 2H), 3.99 (d, J = 9.5 Hz, 2H), 3.96 (s, 3H), 2.30 (s, 3H), 1.48 (s, 9H).
LC-MS (ESI): $[M-Boc+H]^+$ = 256.17

Step 5: 1-tert-butyl-8'-methyl-7'-(bromomethyl)spiro[azetidine-3,2'-chromene]-1,8'-dicarboxylate (Compound 20e)

Compound 20e was prepared by referring to the step similar to Step 6 in Example 12.
LC-MS (ESI): $[M-Boc+H]^+$=324.07

Step 6: 8'-(2,6-dioxopiperidin-3-yl)-9'-oxo-8',9'-dihydro-7'H-spiro[azetidine- 3,2'-pyrano[2,3-e]isoindole]-1-carboxylic acid tert-butyl ester (compound 20f)

Under nitrogen protection, compound 20e (600mg, 1eq) was dissolved in CAN (10mL), 3-aminopiperidine-2,6-dione hydrochloride (287mg, 1.5eq) and DIEA (448mg, 3.0eq) were added to the reaction solution and reacted at 80°C for 16h. After the reaction was detected by TLC, the reaction solution was spin-dried and passed through the column to obtain compound 20f (60 mg, two-step yield 21.4%).

$^1$H NMR (600 MHz, DMSO-$d_6$) δ 11.00 (s, 1H), 7.34 (d, J = 7.6 Hz, 1H), 7.08 (d, J = 7.6 Hz, 1H), 6.67 (d, J = 9.9 Hz, 1H), 6.20 (d, J = 9.9 Hz, 1H), 4.99 (dd, J = 13.2, 5.2 Hz, 1H), 4.37 (d, J = 17.6 Hz, 1H), 4.25 (d, J = 17.6 Hz, 1H), 4.04-4.01 (m, 4H), 2.93-2.87 (m, 1H), 2.62-2.57 (m, 1H), 2.41-2.33 (m, 1H), 2.00-1.96 (m, 1H), 1.41 (s, 9H).
LC-MS (ESI): $[M-Boc+H]^+$ =340.19

Step 7: 8'-(2,6-dioxopiperidin-3-yl)-9'-oxo-4',7',8',9'-tetrahydro-3'H-spiro[ Azetidine-3,2'-pyrano[2,3-e]isoindole]-1-carboxylic acid tert-butyl ester(compound 20g)

Under nitrogen protection, compound 20f (60.0mg, 1eq) was dissolved in THF (10mL), Pd/C (60mg, 1eq) was added, hydrogen was replaced three times, and the reaction was performed at 50°C for 16h. After the reaction was detected by TLC, the reaction solution was spin-dried to obtain 20 g (80 mg) of the crude compound, which could be directly carried out to the next reaction without purification.
LC-MS (ESI): $[M-Boc+H]^+$=342.17

Step 8: 3-(9'-oxo-3',4',7',9'-tetrahydro-8'H-spiro[azetidine-3,2'-pyrano[2 ,3-e]isoindole]-8'-yl)piperidine-2,6-dione (compound 20)

Under the condition of nitrogen protection, compound 20g (80mg, 1eq) was dissolved in dichloromethane (5mL), TFA (1mL) was added, and the reaction mixture was performed at room temperature for 2h. After the reaction was detected by TLC, the reaction solution was spin-dried to obtain compound 20 (15 mg, two-step yield 24.3%) by separation and lyophilization.

$^1$H NMR (600 MHz, DMSO-$d_6$) δ 10.98 (s, 1H), 7.35 (d, J = 7.6 Hz, 1H), 7.08 (d, J = 7.6 Hz, 1H), 4.97 (dd, J = 13.2, 5.2 Hz, 1H), 4.35 (d, J = 17.3 Hz, 1H), 4.23 (d, J = 17.3 Hz, 1H), 4.14-4.10 (m, 4H), 2.93 - 2.85 (m, 3H), 2.62 - 2.58 (m, 1H), 2.42-2.35 (m, 1H), 2.22 - 2.19 (m, 2H), 1.99-1.95 (m, 1H).
LC-MS (ESI): $[M+H]^+$ = 342.29

Example 21

Synthesis of compound 21

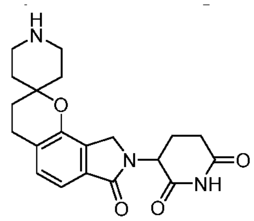

3-(7'-oxo-3',4',7',9'-tetrahydro-8'H-spiro[piperidine-4,2'-pyrano[2,3-e] isoindole]-8'-yl)piperidine-2,6-dione

Synthesis scheme:

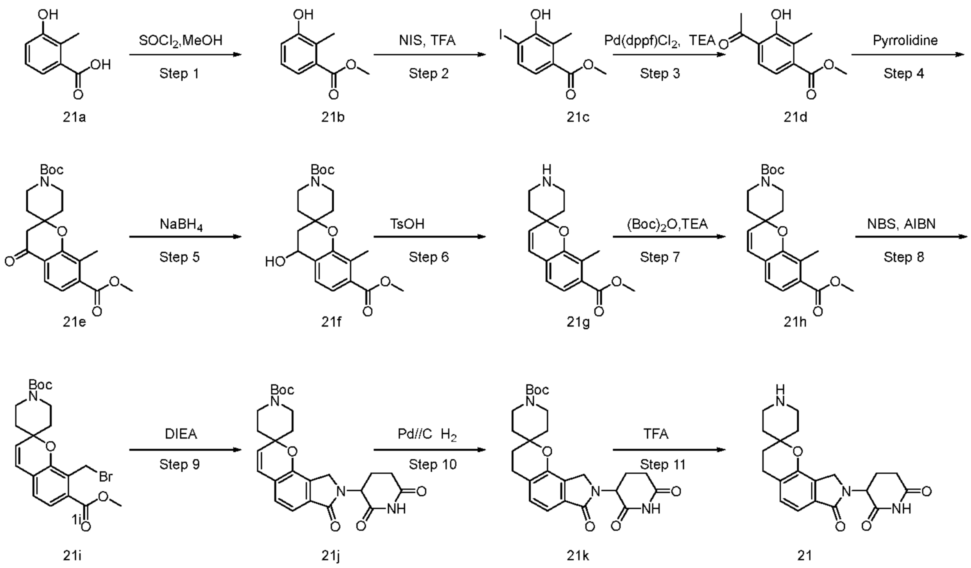


Step 1: methyl 3-hydroxy-2-methylbenzoate (compound 21b)

To a solution of 3-hydroxy-2-methylbenzoic acid (compound 21a) (10 g, 65.73 mmol) in methanol (100 mL) was slowly added thionyl chloride (15.64 g, 131.45 mmol). The reaction solution was stirred at 80° C for 2 hours. The reaction solution was spin-dried to obtain gray solid 21b (10.0 g, 91.56%).

$^1$H NMR (600 MHz, DMSO-$d_6$) δ 9.71 (s, 1H), 7.19 (dd, $J$ = 7.7, 1.3 Hz, 1H), 7.09 (t, $J$ = 7.9 Hz, 1H), 7.02 (dd, $J$ = 8.0, 1.4 Hz, 1H), 3.80 (s, 3H), 2.29 (s, 3H).
LC-MS(ESI): $[M+H]^+$ = 167.23.

Step 2: methyl 3-hydroxy-4-iodo-2-methylbenzoate (compound 21c)

N-iodosuccinimide (17.6 g, 78.23 mmol) was slowly added to a solution of methyl 3-hydroxy-2-methylbenzoate (compound 21b) (10.0 g, 60.18 mmol) in trifluoroacetic acid (80 mL) and methanol (40 mL). The reaction was stirred at room temperature for 2 hours. The resulting mixture was concentrated in vacuo and purified by C18 reverse phase column to obtain 21c (4 g, 22.76%) as a white solid.

$^1$H NMR (600 MHz, DMSO-$d_6$) δ 9.36 (s, 1H), 7.67 (d, $J$ = 8.2 Hz, 1H), 7.03 (d, $J$ = 8.2 Hz, 1H), 3.80 (s, 3H), 2.38 (s, 3H).

LC-MS(ESI): $[M+H]^+$ = 364.22.

Step 3: methyl 4-acetyl-3-hydroxy-2-methylbenzoate (compound 21d)

Under nitrogen protection, compound 21c (5.4 g, 18.49 mmol), butyl vinyl ether (5.56 g, 55.47 mmol) and Pd(dppf)Cl (1.35 g, 1.85 mmol) were dissolved in methanol (80 mL). Triethylamine (5.61 g, 55.47 mmol) was added to the reaction system. The reaction was raised up to 60° C, and stirred for 4 h. After the reaction, the mixture was filtered and the filtrate was concentrated to obtain a crude product, which was purified by column chromatography (PE:EA=20%) to obtain yellow oil 21d (3 g, 77.93%).

$^1$H NMR (600 MHz, $CD_3OD$) δ 7.28 (d, *J* = 8.2 Hz, 1H), 7.18 (d, *J* = 8.3 Hz, 1H), 3.87 (s, 3H), 2.38 (s, 3H), 1.59 (s, 3H).
LC-MS(ESI): $[M+H]^+$ = 209.15.

Step 4: 1'-(tert-butyl) 7-methyl 8-methyl-4-oxospiro[chroman-2,4'-piperidine]-1', 7-dicarboxylate (compound 21e)

21e was prepared by referring to the procedure similar to Step 3 in Example 12.

$^1$H NMR (600 MHz, DMSO-$d_6$) δ 7.65 (d, *J* = 8.2 Hz, 1H), 7.33 (d, *J* = 8.2 Hz, 1H), 3.86 (s, 3H), 2.89 (s, 2H), 2.38 (s, 3H), 1.92 (d, *J* = 2.5 Hz, 1H), 1.89 (q, *J* = 2.8 Hz, 1H), 1.64 (td, *J* = 13.1, 4.8 Hz, 2H), 1.43 (s, 4H), 1.40 (s, 9H).
LC-MS(ESI): $[M\text{-}Boc+H]^+$ = 290.18.

Step 5: 1'-(tert-butyl) 7-methyl 4-hydroxyl-8-methylspiro[chroman-2,4'-piperidine]-1',7-dicarboxylate (compound 21f)

Compound 21f was prepared by referring to the procedure similar to Step 4 in Example 12.
LC-MS (ESI): $[M\text{-}Boc+H]^+$=292.28.

Step 6: methyl 8-methylspiro[chromene-2,4'-piperidine]-7-carboxylate (compound 21g)

Compound 21g was prepared by referring to the procedure similar to Step 5 in Example 14.
LC-MS (ESI): $[M+H]^+$=274.47.

Step 7: 1'-(tert-butyl) 7-methyl 8-methylspiro[chromene-2,4'-piperidine]-1',7-dicarboxylate (compound 21h)

21h was prepared by referring to the similar procedure of Step 6 in Example 14.

$^1$H NMR (600 MHz, $CD_3OD$) δ 7.37 (d, *J* = 7.9 Hz, 1H), 6.94 (d, *J* = 7.9 Hz, 1H), 6.48 (d, *J* = 9.8 Hz, 1H), 5.77 (d, *J* = 9.7 Hz, 1H), 3.87 (s, 3H), 2.45 (s, 3H), 1.97 - 1.93 (m, 2H), 1.66 (td, *J* = 13.9, 12.2, 4.8 Hz, 2H), 1.58 (s, 2H), 1.53 (s, 2H), 1.49 (s, 9H).
LC-MS(ESI): $[M\text{-}Boc+H]^+$ = 274.17.

Step 8: 1'-(tert-butyl)7-methyl 8-(bromomethyl)spiro[chromene-2,4'-piperidine]-1',7-dicarboxylate (compound 21i)

Compound 21i was prepared by referring to the similar procedure of Step 6 in Example 12.
LC-MS (ESI): $[M+H]^+$=316.08.

Step 9: 8'-(2,6-dioxopiperidin-3-yl)-7'-oxo-8',9'-dihydro-7'H-spiro[piperidine-4,2 '-pyrano[2,3-e]isoindole]-1-carboxylic acid tert-butyl ester (compound 21j)

Compound 21i (4g, 8.84mmol) was dissolved in acetonitrile (100mL) solution, 3-aminopiperidine-2,6-dione hydrochloride (2.18g, 13.26mmol) and N,N-diisopropyl Ethylamine (3.43g, 26.53mmol) were added, the reaction was stirred overnight at 80°C. After the reaction was completed, the mixture was purified by normal phase column (100% EA) to obtain compound 21j (1 g, 24.19%) as a blue solid.
LC-MS (ESI): $[M\text{-}tBu+H]^+$=412.29.

Step 10: tert-butyl 8'-(2,6-dioxopiperidin-3-yl)-7'-oxo-4',7',8',9'-tetrahydro-3'H-spiro[piperidin-4,2'-pyrano[2,3-e]isoindole]-1-carboxylate (compound 21k)

Compound 21j (90mg, 192.50μmol) was dissolved in tetrahydrofuran (5mL) solution, palladium on carbon

(20mg) was added, hydrogen was replaced three times, and the reaction was stirred overnight at 50°C. After the reaction was completed, the mixture was filtered to obtain white solid compound 21k (90 mg, 99.57%).

LC-MS (ESI): $[M\text{-}tBu+H]^+$=414.39.

Step 11: 3-(7'-oxo-3',4',7',9'-tetrahydro-8'H-spiro[piperidine-4,2'-pyrano[2,3 -e]isoindole]-8'-yl)piperidine-2,6-dione (compound 21)

Compound 21k (90mg, 191.68μmol) was dissolved in dichloromethane (2mL) solution, trifluoroacetic acid (1mL) was added, and the reaction was stirred at room temperature for 2 hours. After the reaction was completed, it was spin-dried and purified by preparative liquid phase to obtain white solid compound 21 (50 mg, 70.61%).
$^1$H NMR (400 MHz, $CD_3OD$) δ 7.36 (d, J = 7.8 Hz, 1H), 7.32 (d, J = 7.8 Hz, 1H), 5.18 (dd, J = 13.3, 5.2 Hz, 1H), 4.55 - 4.40 (m, 2H), 3.38 (dd, J = 12.5, 3.3 Hz, 4H), 3.03 - 2.89 (m, 3H), 2.81 (m, J = 17.6, 4.6, 2.4 Hz, 1H), 2.51 (m, J = 13.3, 4.7 Hz, 1H), 2.24 - 2.09 (m, 3H), 2.02 (t, J = 6.8 Hz, 2H), 1.98 - 1.87 (m, 2H). LC-MS(ESI): $[M+H]^+$ = 370.39

Example 22

Synthesis of compound 22

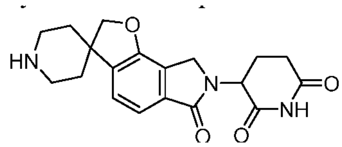

3-(6-oxo-6,8-dihydro-2H,7H-spiro[furo[2,3-e]isoindole-3,4'-piperidin]-7-yl)piperidine-2 ,6-dione Synthesis scheme:

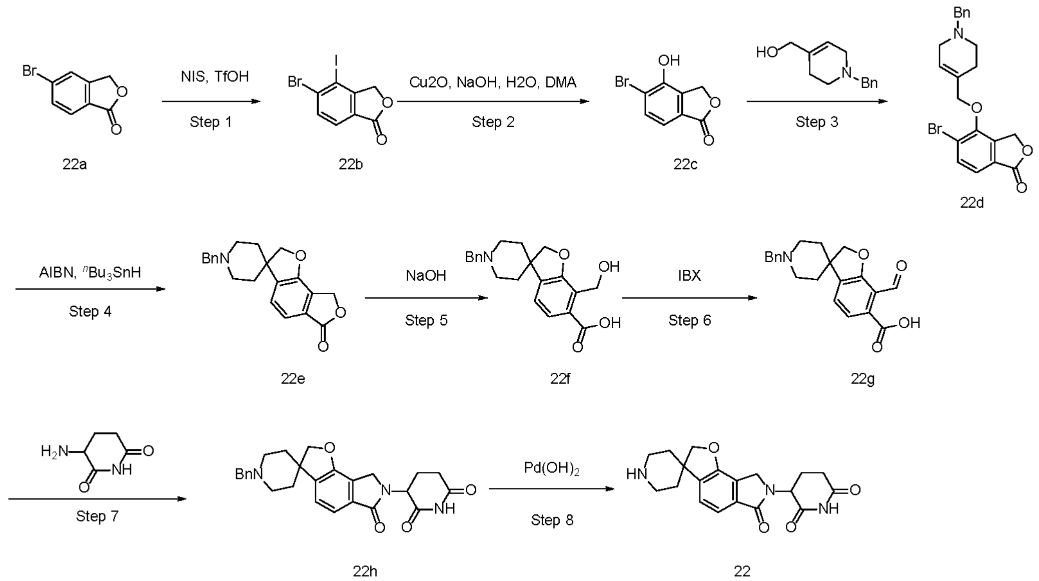


Step 1: 5-bromo-4-iodoisobenzofuran-1(3H)-one (compound 22b)

Compound 22a (1 g, 4.69 mmol) was dissolved in triacetic acid (10 mL), and the solution was protected with nitrogen and cooled to 0°C. N-iodosuccinimide (1.16 g, 5.16 mmol) was added to the reaction solution at 0°C. The reaction solution was raised to room temperature overnight. The resulting mixture was poured into ice water and filtered. The filter cake was washed with water and purified by column chromatography (EA:PE=0%-15%) to obtain white solid compound 22b (800 mg, 50.28%).

$^1$H NMR (600 MHz, DMSO-$d_6$) δ 7.94 - 7.91 (m, 1H), 7.78 (dd, *J* = 8.1, 1.1 Hz, 1H), 5.21 (s, 2H).

LC-MS(ESI): [M-H]- = 230.86.

Step 2: 5-bromo-4-hydroxyisobenzofuran-1(3H)-one (compound 22c)

A solution of sodium hydroxide (413.04 mg, 10.33 mmol) in water (7 mL)was added to a solution of compound 22b in N,N-dimethylacetamide (3.5 mL). Cuprous oxide (59.11 mg, 413.07 μmol) was added to the above solution. The reaction was warmed to 80 °C and stirred overnight. The reaction solution was neutralized to neutrality with 1N hydrochloric acid, extracted with ethyl acetate and the organic phase was washed with saturated brine. The organic phase was concentrated under vacuum and the resulting mixture was purified by column chromatography (EA:PE=0%-35%) to give white solid compound 22c (249 mg, 52.64%).

$^1$H NMR (600 MHz, DMSO-$d_6$) δ 10.91 (s, 1H), 7.73 (d, *J* = 8.0 Hz, 1H), 7.24 (d, *J* = 8.0 Hz, 1H), 5.35 (s, 2H).

LC-MS(ESI): [M-H]- = 230.86.

Step 3: 4-((1-benzyl-1,2,3,6-tetrahydropyridin-4-yl)methoxy)-5-bromoisobenzofuran-1(3H)-one (compound 22d)

Compound 22d was prepared by referring to the procedure similar to Step 3 in Example 17.

$^1$H NMR (600 MHz, DMSO-$d_6$) δ 7.83 (d, *J* = 8.0 Hz, 1H), 7.45 (d, *J* = 8.0 Hz, 1H), 7.37 - 7.30 (m, 4H), 7.29 - 7.19 (m, 1H), 5.84 (s, 1H), 5.68 (s, 2H), 4.64 (s, 2H), 3.56 (s, 2H), 2.93 (s, 2H), 2.57 (t, *J* = 5.7 Hz, 2H), 2.25 (s, 2H).

LC-MS(ESI): $[M+H]^+$ = 414.20.

Step 4: 1'-benzyl-2H-spiro[benzo[2,1-b:3,4-c']difuran-3,4'-piperidin]-6(8H)-one (compound 22e)

Compound 22e was prepared by referring to the procedure similar to Step 4 in Example 17.
$^1$H NMR (600 MHz, $CDCl_3$) δ 7.56 (d, *J* = 7.7 Hz, 1H), 7.52 - 7.46 (m, 6H), 5.26 (s, 2H), 4.54 (s, 2H), 4.29 (s, 2H), 2.73 (t, *J* = 13.1 Hz, 2H), 2.60 (td, *J* = 14.6, 4.0 Hz, 2H), 2.12 - 1.91 (m, 4H).

LC-MS(ESI): [M-H]- = 336.30.

Step 5: 1'-benzyl-7-(hydroxymethyl)-2H-spiro[benzofuran-3,4'-piperidine]-6-carboxylic acid (compound 22f)

A solution of sodium hydroxide (120.44 mg, 3.01 mmol) in water (2.0 mL)was added to a solution of compound 22e (202 mg, 602.26 μmol) in tetrahydrofuran (2.0 mL). The reaction was stirred overnight at room temperature. The resulting mixture was concentrated under vacuum and purified by reverse column to give compound 22f (98 mg, 46.04%) as a white solid.

LC-MS(ESI): [M-H] =354.59.

Step 6: 1'-benzyl-7-formyl-2H-spiro[benzofuran-3,4'-piperidine]-6-carboxylic acid (compound 22g)

Compound 22f (20 mg, 56.59 μmol) and IBX (31.69 mg, 113.18 μmol) were dissolved in tetrahydrofuran and dimethyl sulfoxide = 20:1 (0.5 mL), and the mixture was stirred at 80° C for 2 h. The reaction was filtered and the resulting mixture was concentrated in vacuo, and the crude product was used directly in the next step without purification.

LC-MS (ESI): $[M+H]^+$=352.30.

Step 7: 3-(1'-benzyl-6-oxo-6,8-dihydro-2H,7H spiro[furo[2,3-e]isoindole-3,4'-piperidine ]-7-yl)piperidine-2,6-dione (compound 22h)

Compound 22g (48 mg, 136.60 μmol) and 3-aminopiperidine-2,6-dione hydrochloride (44.96 mg, 273.19 μmol) were dissolved in tetrahydrofuran and dimethyl sulfoxide = 20:1 (1.0 mL), and the reaction mixture was added a drop of N,N-diisopropylethylamine. The reaction was stirred at 50 °C for 1 h. The mixed solution was cooled to room temperature, and sodium triacetoxyborohydride (86.85 mg, 409.79 μmol) and a drop of acetic acid were added, and the temperature was raised to 50° C for 1 h. The mixture was concentrated under vacuum and purified by reverse-phase HPLC to give compound 22h (16 mg, 26.29%) as a white solid.
$^1$H NMR (600 MHz, DMSO-$d_6$) δ 11.00 (s, 1H), 7.46 - 7.56 (m, 5H), 7.34 (d, *J* = 7.6 Hz, 1H), 7.26 (d, *J* = 7.7 Hz, 1H), 5.09 (dd, *J* = 13.3, 5.1 Hz, 1H), 4.67 (t, *J* = 6.3 Hz, 2H), 4.43 - 4.34 (m, 3H), 4.24 (d, *J* = 17.1 Hz, 1H), 3.16 (d, *J* = 14.4 Hz, 2H), 2.91 (ddd, *J* = 18.0, 13.7, 5.4 Hz, 1H), 2.64 - 2.56 (m, 1H), 2.44 (td, *J* = 13.2, 4.6 Hz, 2H), 2.12 (d, *J* = 15.4 Hz, 2H), 2.03 - 1.89 (m, 4H).

LC-MS(ESI): $[M+H]^+$ = 446.35.

Step 8: 3-(6-oxo-6,8-dihydro-2H,7H spiro[furo[2,3-e]isoindol-3,4'-piperidin]-7-yl) piperidine-2,6-dione (compound 22)

Compound 22h (150 mg, 336.69 μmol) was dissolved in hexafluoroisopropanol (4.5 mL) solution, 20% palladium hydroxide/carbon (75 mg) was added, hydrogen was replaced three times, and the reaction mixture was stirred at room temperature under hydrogen for 5 hours. After the reaction was completed, the reaction solution was filtered, concentrated under reduced pressure and purified by reverse-phase HPLC to obtain compound 22 (35 mg, 29.25%) as a white solid. $^1$H NMR (600 MHz, $CD_3OD$) δ 7.46 - 7.40 (m, 2H), 5.16 (dd, J = 13.4, 5.2 Hz, 1H), 4.70 (s, 2H), 4.52 - 4.40 (m, 2H), 3.54 - 3.47 (m, 2H), 3.24 - 3.14 (m, 2H), 2.96 - 2.87 (m, 2H), 2.80 (m, J = 17.7, 4.6, 2.2 Hz, 1H), 2.52 (m, J= 13.3, 4.6 Hz, 1H), 2.25 - 2.16 (m, 3H), 2.05 - 2.01 (m, 1H).

LC-MS(ESI): $[M+H]^+$ = 356.29.

Example 23

Synthesis of compound 23

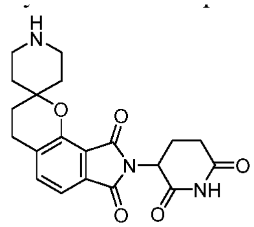

8'-(2,6-dioxopiperidin-3-yl)-3',4'-dihydro-7'H-spiro[piperidin-4,2'-pyrano[2,3-e]isoindole]-7',9'(8'H)-dione

Synthesis scheme:

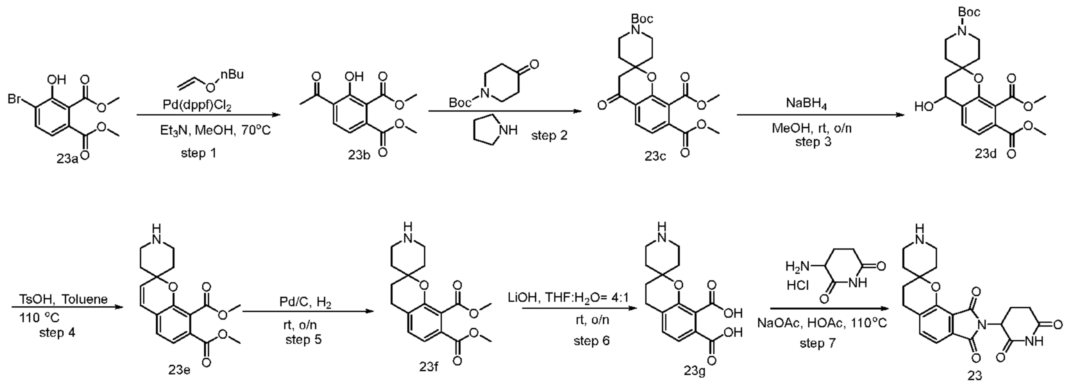


Step 1: dimethyl 4-acetyl-3-hydroxyphthalate (compound 23b)

Under nitrogen protection conditions, dimethyl 4-bromo-3-hydroxyphthalate (10g, 34.59mmol) was dissolved in MeOH (100mL), and 1-(vinyloxy)butane (13.86g, 138.87 mmol), $Pd(dppf)Cl_2$ (2.53 g, 3.46 mmol), and triethylamine (10.50 g, 103.78 mmol) were added. The reaction was stirred at 70 °C for 12 h. After the reaction was completed, HCl-1,4-dioxane (100 mL) was added and stirred at room temperature for 30 minutes. Then it was extracted three times with EA, the organic phases were combined, washed with saturated sodium chloride solution, dried over anhydrous sodium sulfate and concentrated to give the crude compound 23b (8 g, 91.6%) as a yellow oil, which was directly used in the next step. LC-MS (ESI): $[M-32+H]^+$=211.06

Step 2: 1'-(tert-butyl)-7,8-dimethyl-4-oxospiro[chroman-2,4'-piperidine]-1',7,8-tricarboxylate (Compound 23c)

Compound 23c was prepared by referring to the procedure similar to Step 3 in Example 12.

$^1H$ NMR (600 MHz, $CDCl_3$) δ 7.93 (d, *J* = 8.2 Hz, 1H), 7.68 (d, *J* = 8.2 Hz, 1H), 4.00 (s, 3H), 3.95 (s, 3H), 3.30 (t, *J* = 9.7 Hz, 2H), 3.17 (t, *J* = 9.7 Hz, 2H), 3.14 (s, 2H), 2.47 (t, *J* = 9.7 Hz, 2H), 2.06 (t, *J* = 9.7 Hz, 2H).
LC-MS(ESI):$[M+H]^+$= 334.19

Step 3: 1'-(tert-butyl)-7,8-dimethyl-4-hydroxy spiro[chroman-2,4'-piperidine]-1',7,8-tricarboxylate (compound 23d)

Compound 23d was prepared by referring to the similar procedure of Step 4 in Example 12.
LC-MS (ESI): $[M+H]^+$=336.29

Step 4: spiro[chromene-2,4'-piperidine]-7,8-dicarboxylic acid dimethyl ester (compound 23e)

Compound 23d (2.80g, 6.43mmol) was dissolved in 3mL of toluene, p-toluenesulfonic acid (2.21g, 12.86mmol) was added, and the mixture was refluxed at 110°C overnight. After the reaction was completed, the reaction mixture was concentrated, added water, extracted with ethyl ester and spin-dried, separated and purified by column chromatography to obtain yellow oily compound 23e (1.4 g, 68.6%).
LC-MS (ESI): $[M+H]^+$=318.38

Step 5: spiro[chroman-2,4'-piperidine]-7,8-dicarboxylic acid dimethyl ester (compound 23f)

Compound 23e (1.40 g, 4.41 mmol) was dissolved in MeOH, Pd/C (700 mg) was added and the reaction was replaced by hydrogen stirred under hydrogen for 4 hours. After the reaction was completed, compound 23f (1.3 g, 93.8%) was obtained as a yellow oily compound after filtration and concentration, and the obtained crude product was directly sent to the next step.
LC-MS (ESI): $[M+H]^+$=320.58

Step 6: spiro[chroman-2,4'-piperidine]-7,8-dicarboxylic acid (compound 23g)

Compound 23f (1.4 g, 4.38 mmol) was dissolved in a mixed solution of THF:$H_2O$ (4:1), LiOH (629.87 mg, 26.30 mmol) was added and stirred overnight at room temperature. The crude product (1.35 g, 96.84%) was obtained after the reaction was completed and concentrated under reduced pressure.
LC-MS (ESI): $[M+H]^+$=306.58

Step 7: 8'-(2,6-dioxopiperidin-3-yl)-3',4'-dihydro-7'H-spiro[piperidine-4,2'-pyrano[2,3-e]isoindole]-7',9'(8'H)-dione (compound 23)

Compound 23g (1.50 g, 4.91 mmol), 3-aminopiperidine-2,6-dione hydrochloride (970.30 mg, 5.90 mmol) and sodium acetate (806.02 mg, 9.83 mmol) were dissolved in acetic acid (5 mL). The mixture was stirred at 110 °C for 5 h. After the reaction, suction filtration, the filtrate was concentrated under reduced pressure and purified by prep-HPLC (phase A=water, phase B=ACN, 0.1% TFA, B%=0% ~ 50% in 30min) to obtain a white solid compound 23 (0.55 g, 31.3%).

> $^1H$ NMR (600 MHz, DMSO-$d_6$) δ 11.11 (s, 1H), 7.62 (d, *J* = 7.5 Hz, 1H), 7.39 (d, *J* = 7.4 Hz, 1H), 5.08 (dd, *J* = 12.9, 5.5 Hz, 1H), 3.28 (t, *J* = 11.8 Hz, 2H), 3.09 (t, *J* = 11.8 Hz, 2H), 2.96 (t, *J* = 12,4 Hz, 2H), 2.91 - 2.78 (m, 3H), 2.63 - 2.52 (m, 2H), 2.05 -1.94 (m, 3H), 1.91 - 1.81 (m, 2H).
> LC-MS(ESI): $[M+H]^+$= 384.19

Example 24

Synthesis of compound 24

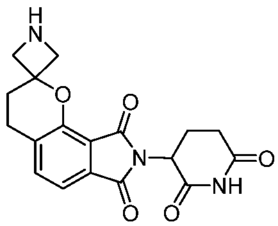

8'-(2,6-dioxopiperidin-3-yl)-3',4'-dihydro-7'H-spiro[azetidine-3,2'-pyrano[2, 3-e]isoindole]-7',9'(8'H)-dione

Synthesis scheme:

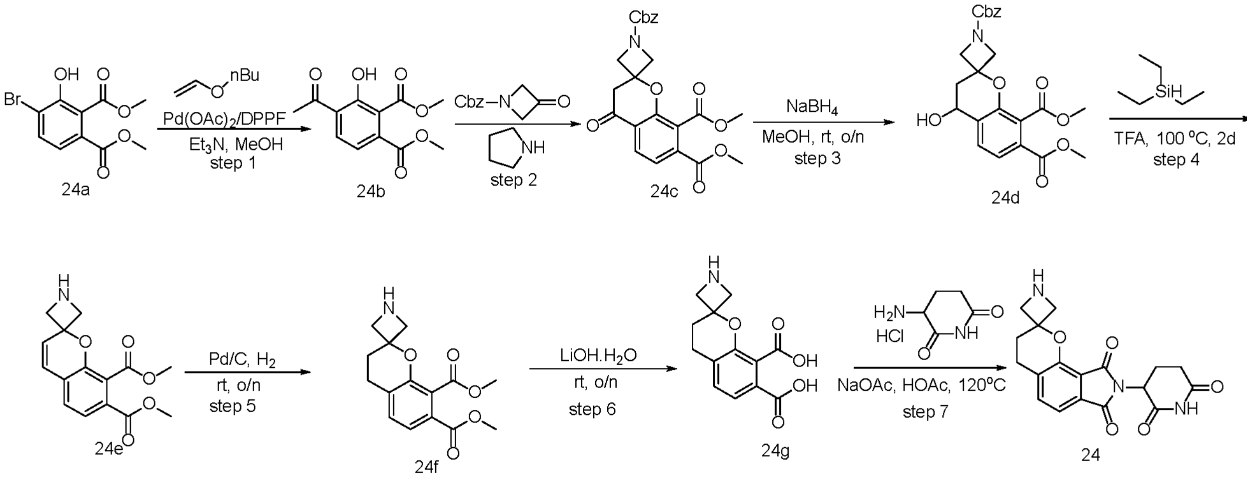


Step 1: dimethyl 4-acetyl-3-hydroxyphthalate (compound 24b)

Compound 24b was prepared by referring to the same procedure as Step 1 in Example 23.

$^1$H NMR (600 MHz, DMSO-$d_6$) δ 10.70 (s, 1H), 7.48 (d, J = 7.5 Hz, 1H), 7.12 (d, J = 7.4 Hz, 1H), 3.88 (s, 3H), 3.87 (s, 3H), 2.42 (s, 3H).
LC-MS(ESI): $[M-32+H]^+$= 221.16

Step 2: 1-benzyl-7',8'-dimethyl-4'-oxospiro[azetidine-3,2'-chroman]-1,7',8'-tricarboxylate (compound 24c)

Compound 24c was prepared by referring to the similar step of Step 3 in Example 12.

$^1$H NMR (600 MHz, $CDCl_3$) δ 7.99 (dd, *J* = 8.2, 0.9 Hz, 1H), 7.71 (dd, *J* = 8.2, 1.0 Hz, 1H), 7.42 - 7.32 (m, 5H), 5.13 (s, 2H), 4.20 (d, *J* = 9.7 Hz, 2H), 4.06 (d, *J* = 9.7 Hz, 2H), 4.00 (s, 3H), 3.95 (s, 3H), 3.14 (s, 2H).
LC-MS(ESI): $[M+H]^+$= 438.20

Step 3: 1-benzyl-7',8'-dimethyl-4'-hydroxy spiro[azetidine-3,2'-chroman]-1,7',8'-tricarboxylic ester (compound 24d)

Compound 24d was prepared by referring to the similar step of Step 4 in Example 12.
$^1$H NMR (600 MHz, DMSO-$d_6$) δ 7.63 (d, *J* = 7.5 Hz, 1H), 7.44 (d, *J* = 7.0 Hz, 1H), 7.42 - 7.30 (m, 5H), 5.11 (s, 2H), 4.50 (s, 1H), 4.25 -4.15 (m, 1H), 4.10 (d, *J* = 9.7 Hz, 4H), 3.88 (s, 3H), 3.86 (s, 3H), 2.60 (d, *J* = 17.6, 2H).
LC-MS(ESI):$[M+H]^+$= 442.29

Step 4: spiro[azetidine-3,2'-chromene]-7',8'-dicarboxylic acid dimethyl ester (compound 24e)

Compound 24d (180mg, 407.76μmol) was dissolved in trifluoroacetic acid, then triethylsilane (330mg, 2.84mmol) was added. The reaction mixture was stirred in an oil bath at 100°C for 6 hours and then cooled to room

temperature. Then another batch of triethylsilane (330mg, 2.84mmol) was added, the reaction was completed and spin-dried, the crude product was dissolved in methanol and stirred for 1h and then spin-dried, the supernatant was poured off, the lower layer was the product, and no purification was required to obtain yellow oily compound 24e (160mg, 96.6%), directly used in the next step.
LC-MS (ESI): $[M+H]^+$=289.98

Step 5: spiro[azetidine-3,2'-chroman]-7',8'-dicarboxylic acid dimethyl ester (compound 24f)

Under nitrogen protection, compound 24e (160 mg, 409.28 μmol) was dissolved in methanol. Pd/C (50 mg, 10%) was added. The reaction was replaced by hydrogen, and stirred overnight at room temperature under hydrogen. After the reaction was completed and filtered, the filtrate was spin-dried to obtain compound 24f (130 mg, 81.3%) as a yellow oil, which was directly used in the next step without purification.
LC-MS (ESI): $[M+H]^+$=292.18

Step 6: spiro[azetidine-3,2'-chroman]-7',8'-dicarboxylic acid (compound 24g)

Compound 24f (130mg, 410.57μmol) was dissolved in a mixed solvent of tetrahydrofuran/methanol/-water=1:1:5, and lithium hydroxide (150mg, 6.26mmol) was added in an ice-water bath. The reaction mixture was stirred overnight at room temperature. After the reaction was completed, the pH was adjusted to 7 with aqueous hydrochloric acid, and the filtrate was spin-dried to obtain 24 g (140 mg, 105%) of the yellow oil product, which was directly used in the next step without purification.
LC-MS (ESI): $[M+H]^+$=264.17

Step 7: 8'-(2,6-dioxopiperidin-3-yl)-3',4'-dihydro-7'H-spiro[azetidine-3,2'-pyrano [2,3-e]isoindole]-7',9'(8'H)-dione (compound 24)

Compound 24g (200mg, 1.22mmol) and sodium acetate (200mg, 2.44mmol) were dissolved in acetic acid. The reaction mixture was stirred overnight in an oil bath at 120°C, after spin-drying, it was dissolved with methanol, purified in reverse phase, ACN/$H_2O$ (0-20% 40min) , to obtain a white solid compound 24 (80 mg, 18.4%).

> $^1$H NMR (600 MHz, DMSO-$d_6$) δ 11.13 (s, 1H), 7.63 (d, $J$ = 7.5 Hz, 1H), 7.44 (d, $J$ = 7.0 Hz, 1H), 5.09 (dd, $J$ = 12.9, 5.5 Hz, 1H), 4.15 (s, 4H), 2.96 (t, $J$ = 6.5 Hz, 2H), 2.94-2.87(m, 1H), 2.60-2.54 (m, 2H), 2.31 - 2.20 (m, 2H), 2.03-1.94 (m, 1H).
> LC-MS(ESI): $[M+H]^+$= 356.30

Example 25

Synthesis of compound 25

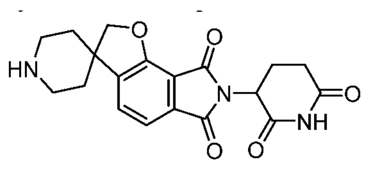

7-(2,6-dioxopiperidin-3-yl)-7-hydrogen-2H,6H-spiro[furo[2,3-e]isoindole-3,4'-piperidin]- 6,8-dione Synthesis scheme:

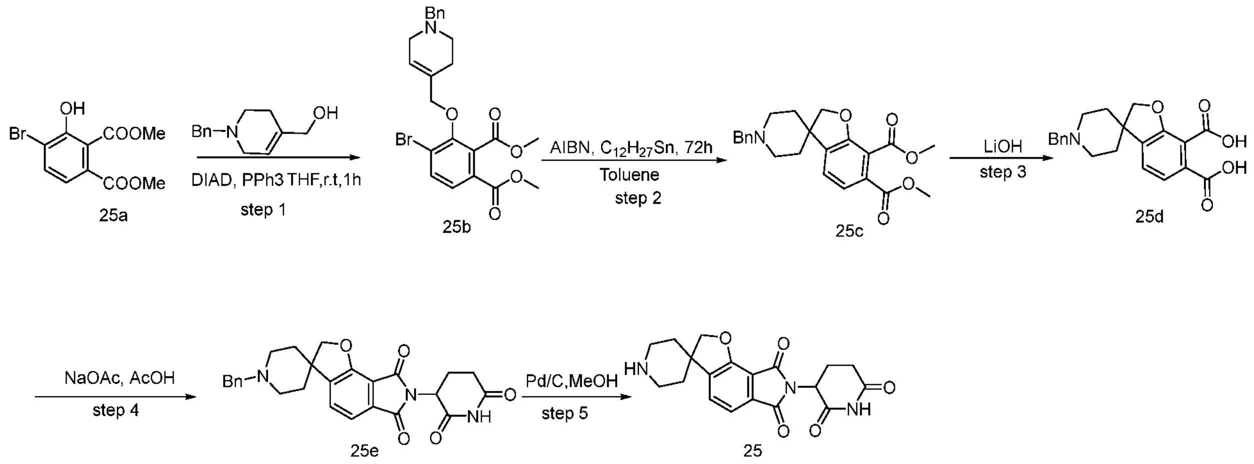


Step 1: dimethyl 3-((1-benzyl-1,2,3,6-tetrahydropyridin-4-yl)methoxy)-4-bromophthalate (compound 25b)

Compound 26b was prepared by referring to the similar procedure of step 3 in Example 17.

$^1$H NMR (600 MHz, DMSO-$d_6$) δ 7.95 (d, $J$ = 8.4 Hz, 1H), 7.70 (d, $J$ = 8.4 Hz, 1H), 7.58 - 7.45 (m, 5H), 5.87 - 5.83 (m, 1H), 4.45 (s, 2H), 3.84 (s, 3H), 3.83 (s, 3H), 3.81 (s, 2H), 3.47 - 3.30 (m, 2H), 3.25 - 3.10 (m, 2H), 2.47 - 2.34 (m, 2H). LC-MS(ESI): [M+H]$^+$= 474.09

Step 2: dimethyl 1'-benzyl-2H-spiro[benzofuran-3,4'-piperidine]-6,7-dicarboxylate (compound 25c)

Compound 25c was prepared by referring to the similar procedure of step 4 in Example 17.

$^1$H NMR (600 MHz, DMSO-$d_6$) δ 7.55-7.47 (m, 5H), 7.46 (d, $J$ = 8.4 Hz, 1H), 7.31 (d, $J$ = 8.4 Hz, 1H), 4.65 (s, 2H), 4.35 (s, 2H), 3.80 (s, 3H), 3.78 (s, 3H), 3.43 - 3.30 (m, 2H), 3.15 - 3.06 (m, 2H), 2.17 - 2.07 (m, 2H), 2.01 - 1.94 (m, 2H). LC-MS(ESI): [M+H]$^+$=360.29

Step 3: 1'-benzyl-2H-spiro[benzofuran-3,4'-piperidine]-6,7-dicarboxylic acid (compound 25d)

Compound 25d was prepared by referring to the procedure similar to Step 5 in Example 17.
LC-MS (ESI): [M+H]$^+$=367.99

Step 4: 1'-benzyl-7-(2,6-dioxopiperidin-3-yl)-7-hydrogen-2H,6H-spiro[furo[2,3-e]isoindole -3,4'-piperidine]-6,8-dione (compound 25e)

Compound 25e was prepared by referring to the procedure similar to Step 6 in Example 17.
$^1$H NMR (600 MHz, DMSO-$d_6$) δ 11.10 (s, 1H), 7.62 (d, $J$ = 7.4 Hz, 1H), 7.55-7.47 (m, 5H), 7.43 (d, $J$ = 7.3 Hz, 1H), 5.11 (dd, $J$ = 13.0, 5.4 Hz, 1H), 4.81 (s, 2H), 3.96 (s, 2H), 3.38 (d, $J$ = 13.0 Hz, 2H), 3.09 - 3.01 (m, 2H), 2.91 - 2.84 (m, 1H), 2.63-2.58 (m, 2H), 2.08 - 2.00 (m, 3H), 1.98 - 1.90 (m, 2H).
LC-MS(ESI): [M+H]$^+$=460.49

Step 5: 7-(2,6-dioxopiperidin-3-yl)-7-hydrogen-2H,6H-spiro[furo[2,3-e]isoindole-3,4'- piperidine]-6,8-dione (compound 25)

Compound 25 was prepared by referring to the similar procedure of Step 7 in Example 17.
$^1$H NMR (600 MHz, DMSO-$d_6$) δ 11.12 (s, 1H), 7.61 (d, $J$ = 7.4 Hz, 1H), 7.49 (d, $J$ = 7.3 Hz, 1H), 5.11 (dd, $J$ = 13.0, 5.4 Hz, 1H), 4.81 (s, 2H), 3.38 (d, $J$ = 13.0 Hz, 2H), 3.09 - 3.00 (m, 2H), 2.92 - 2.85 (m, 1H), 2.62-2.59 (m, 2H), 2.09 - 2.01 (m, 3H), 1.98 - 1.91 (m, 2H).
LC-MS(ESI): [M+H]$^+$=370.09

Example 26

Synthesis of compound 26

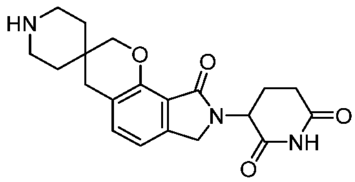

3-(9'-oxo-7',9'-dihydro-2'-spiro [piperidine-4,3'-pyrano [2,3-e]isoindole]-8'(4 'H)-yl)piperidine-2,6-dione

Synthesis scheme:

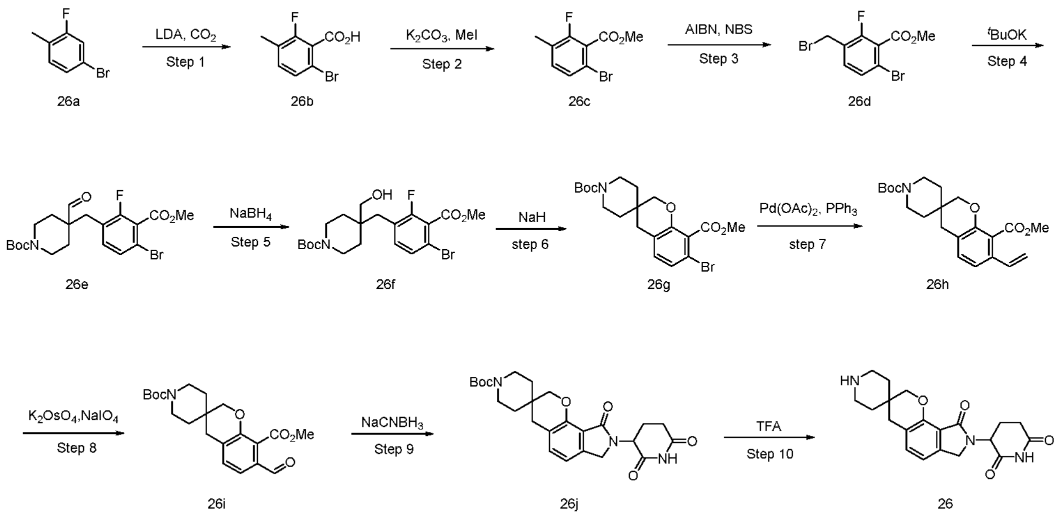


Step 1: 6-bromo-2-fluoro-3-methylbenzoic acid (compound 26b)

Compound 26a (2.0g, 10.6mmol)was subsequently added to a solution of lithium diisopropylamide (1.3M in THF/hexane, 6.9mL, 13mmol) in tetrahydrofuran (10.6mL) at -78°C, then react at this temperature for 2 hours, and quickly pour into dry ice. After it was warmed to room temperature, the solvent was spin-dried to obtain a white solid compound 26b (2.84 g, 115%), which was directly used in the next step.
LC-MS (ESI): [M-H] =230.95

Step 2: methyl 6-bromo-2-fluoro-3-methylbenzoate (compound 26c)

Iodomethane (2.5 g, 17.8 mmol), potassium carbonate (3.7 g, 26.7 mmol) were added to a solution of compound 26b (2.1 g, 8.9 mmol) in N,N-dimethylformamide (12 mL). The reaction was carried out at 80°C for 1 hour. After the reaction was completed, water (50 mL) was added, the reaction mixture was extracted with ethyl acetate (100 mL*3 times), washed with saturated brine (100 mL), and the combined organic phases were dried over anhydrous sodium sulfate. After spin-drying, the obtained solution was purified by column chromatography (PE:EA=0-20%) to obtain compound 26c (1.9 g, 86%) as a yellow oil.

$^1H$ NMR (600 MHz, $CDCl_3$) δ 7.27 (d, *J* = 9.2 Hz, 1H), 7.14 - 7.09 (m, 1H), 3.97 (s, 3H), 2.25 (d, *J* = 1.9 Hz, 3H).

LC-MS(ESI): $[M+H]^+$ = 247.66.

Step 3: methyl 6-bromo-3-(bromomethyl)-2-fluorobenzoate (compound 26d)

Under nitrogen atmosphere, N-bromosuccinimide (1.6 g, 9.2 mmol) and azobisisobutyronitrile (62.8 mg, 0.38mmol) were added to a solution of compound 26c (1.9 g, 7.6 mmol) in carbon tetrachloride (98 mL), and the reaction was performed overnight at 85°C. The solvent was spin-dried and purified by column chromatography (PE:EA=0-30%) to obtain compound 26d (219.9 mg, 8%) as a yellow oil.

$^1$H NMR (600 MHz, $CDCl_3$) δ 7.39 (d, *J* = 8.4 Hz, 1H), 7.35 - 7.31 (m, 1H), 4.45 (s, 2H), 3.98 (s, 3H).

Step 4: tert-butyl 4-(4-bromo-2-fluoro-3-(methoxycarbonyl)benzyl)-4-formylpiperidine-1-carboxylate (compound 26e)

Under a nitrogen atmosphere, at -30°C, potassium tert-butoxide (75.7 mg, 0.67 mmol) was added to a solution of 1-tert-butoxycarbonylpiperidine-4-carbaldehyde (158.4 mg, 0.74 mmol) in tetrahydrofuran (4 mL). The reaction was performed at this temperature for 1 hour, then slowly added a solution of compound 26d (219.9 mg, 0.67 mmol) in tetrahydrofuran (3 mL) to it, and reacted at this temperature for 2 hours, then the reaction mixture was moved to room temperature for overnight reaction. The solvent was spin-dried and purified by column chromatography (PE:EA=0-40%) to obtain compound 26e (147.6 mg, 48%) as a colorless oil.

$^1$H NMR (600 MHz, $CDCl_3$) δ 9.55 (s, 1H), 7.30 (d, *J* = 8.3 Hz, 1H), 7.00 - 6.95 (m, 1H), 3.96 (s, 3H), 4.00 - 3.75 (m, 2H), 2.95 - 2.70 (m, 2H), 2.77 (s, 2H), 1.94 (d, *J* = 13.0 Hz, 2H), 1.58 - 1.46 (m, 2H), 1.43 (s, 9H).

LC-MS(ESI): $[M-Boc+H]^+$ = 358.19.

Step 5: tert-butyl 4-(4-bromo-2-fluoro-3-(methoxycarbonyl)benzyl)-4-(hydroxymethyl)piperidine-1-carboxylate (compound 26f)

At 0°C, sodium borohydride (1.9 g, 51.4 mmol) was slowly added in portions to a solution of compound 26e (4.7 g, 10.3 mmol) in methanol (50 mL), and then moved to room temperature for 2 hours. Water was added slowly to quench the reaction. The reaction mixture was spin-dried and purified by column chromatography (PE:EA=0-50%) to obtain colorless oily liquid compound 26f (4.14g, 88%).

$^1$H NMR (600 MHz, DMSO-$d_6$) δ 7.51 (d, *J* = 8.3 Hz, 1H), 7.41 - 7.36 (m, 1H), 4.78 (t, *J* = 5.0 Hz, 1H), 3.90 (s, 3H), 3.46 - 3.39 (m, 2H), 3.20 (d, *J* = 4.5 Hz, 4H), 2.66 (s, 2H), 1.99 (s, 2H), 1.42 - 1.32 (m, 11H).

LC-MS(ESI): $[M-Boc+H]^+$ = 360.29.

Step 6: 1'-(tert-butyl)8-methyl 7-bromospiro[chroman-3,4'-piperidine]-1',8-dicarboxylate (compound 26g)

Under nitrogen atmosphere, compound 26f (125 mg, 0.27 mmol) was slowly added to a solution of sodium hydride (11.9 mg, 0.27 mmol, 60% dispersed in liquid paraffin) in N,N-dimethylformamide (1 mL), and the reaction was moved to 110°C for 1 hour. After cooling to room temperature, water (2 mL) was added slowly to quench the reaction. After adding water (10mL), the reaction solution was extracted with ethyl acetate (20ml*3 times), washed with saturated brine (40mL), combined the organic phases, and purified by column chromatography (PE:EA=0-40%) to obtain a white solid compound 26g (73.0mg, 61%).

$^1$H NMR (600 MHz, $CDCl_3$) δ 7.03 (d, *J* = 8.2 Hz, 1H), 6.92 (d, *J* = 8.2 Hz, 1H), 3.92 (s, 3H), 3.90 (s, 2H), 3.59-3.47 (m, 2H), 3.37 - 3.28 (m, 2H), 2.61 (s, 2H), 1.50 - 1.40 (m, 13H).
LC-MS(ESI): $[M-Boc+H]^+$ = 339.99

Step 7: 1'-(tert-butyl(8-methyl 7-vinylspiro[chroman-3,4'-piperidine]-1',8-dicarboxylate (compound 26h)

Under a nitrogen atmosphere, compound 26g (73.0mg, 0.17mmol) was dissolved in a mixed solvent of tetrahydrofuran/water (10:1, 1.1mL), and then potassium vinyl trifluoroborate (33.5mg, 0.25mmol), palladium acetate (7.5mg, 0.033mmol), triphenylphosphine (6.1mmol, 0.023mmol) and cesium carbonate (108.2mg, 0.33mmol) were added and reacted overnight in an oil bath at 85°C. The solvent was spin-dried and purified by column chromatography (PE:EA=0-40%) to obtain white solid compound 26h (38.9 mg, 45%).

$^1$H NMR (600 MHz, $CDCl_3$) δ 7.08 (d, *J* = 7.9 Hz, 1H), 7.04 (d, *J* = 7.8 Hz, 1H), 6.63 (dd, *J* = 17.4, 11.0 Hz, 1H), 5.69 (d, *J* = 17.4 Hz, 1H), 5.28 (d, *J*= 11.0 Hz, 1H), 3.96 - 3.88 (m, 5H), 3.60 - 3.50 (m, 2H), 3.38 - 3.29 (m, 2H), 2.67 (s, 2H), 1.52 - 1.42 (m, 13H).
LC-MS(ESI): $[M-Boc+H]^+$ = 288.18

Step 8: 1'-(tert-butyl)8-methyl-7-formylspiro[chroman-3,4'-piperidine]-1',8-dicarboxylate (compound 26i)

Sodium periodate (64.9mg, 0.3mmol) and potassium osmate dihydrate (1.4mg, 0.003mmol) were added to compound 26h (29.4mg, 0.076mmol) in acetone/water mixed solution (5:1,2.7 mL), react overnight at room temperature. The reaction mixture was spin-dried of the solvent and purified by column chromatography (PE:EA=0-40%) to obtain compound 26i (8.9 mg, 30%) as a white solid.

$^1$H NMR (600 MHz, $CDCl_3$) δ 9.89 (s, 1H), 7.37 (d, *J* = 7.7 Hz, 1H), 7.25 (d, *J* = 6.4 Hz, 1H), 4.00 - 3.94 (m, 5H), 3.59 - 3.48 (m, 2H), 3.42 - 3.33 (m, 2H), 2.75 (s, 2H), 1.54 - 1.48 (m, 2H), 1.48 - 1.41 (m, 11H).
LC-MS(ESI): $[M\text{-}Boc+H]^+$ = 290.18

Step 9: tert-butyl 8'-(2,6-dioxopiperidin-3-yl)-9'-oxo-4',7',8',9'-tetrahydro-2'H -spirofpiperidine-4,3'-pyrano[2,3-e]isoindole]-1-carboxylate (compound 26j)

Compound 26i (500mg, 1.3mmol) and 3-amino-2,6-piperidinedione hydrochloride (316mg, 1.9mmol) were mixed in a mixed solvent of dichloromethane/methanol (1/2, 39mL), After reacting at room temperature for 1 hour, sodium cyanoborohydride (241 mg, 3.8 mmol) was added, and the reaction was carried out overnight at room temperature. The solvent was spin-dried, and the mixture was purified by a reverse-phase column to obtain compound 26j (233 mg, 39%) as a white solid.

$^1$H NMR (600 MHz, DMSO-$d_6$) δ 10.95 (s, 1H), 7.28 (d, *J* = 7.6 Hz, 1H), 7.00 (d, *J* = 7.6 Hz, 1H), 5.02 (dd, *J* = 13.3, 5.1 Hz, 1H), 4.30 (d, *J* = 17.2 Hz, 1H), 4.17 (d, *J* = 17.1 Hz, 1H), 4.01 (s, 2H), 3.50 - 3.43 (m, 2H), 3.30 - 3.25 (m, 2H), 2.94 - 2.87 (m, 1H), 2.73 (s, 2H), 2.60 - 2.55 (m, 1H), 2.38 - 2.30 (m, 1H), 1.99 - 1.91 (m, 1H), 1.45 - 1.35 (m, 11H), 1.35 - 1.29 (m, 2H).
LC-MS(ESI): $[M+H]^+$ = 470.40

Step 10: 3-(9'-oxo-7',9'-dihydro-2'-spiro[piperidine-4,3'-pyrano[2,3-e]isoindole]-8'(4'H)-yl)piperidine-2,6-dione (compound 26)

Trifluoroacetic acid (2 mL) was added to a solution of compound 26j (230 mg, 0.49 mmol) in dichloromethane (5 mL), and the reaction was carried out at room temperature for 1.5 hours. The solvent was spin-dried, and the mixture was purified by a preparative column to obtain compound 26 (151.2 mg, 84%) as a white solid.
$^1$H NMR (600 MHz, DMSO-$d_6$) δ 10.96 (s, 1H), 7.30 (d, *J* = 7.6 Hz, 1H), 7.04 (d, *J* = 7.4 Hz, 1H), 5.01 (dd, *J* = 13.2, 4.9 Hz, 1H), 4.31 (d, *J* = 17.1 Hz, 1H), 4.18 (d, *J* = 17.2 Hz, 1H), 4.11 - 4.04 (m, 2H), 3.20 - 3.06 (m, 4H), 2.94 - 2.86 (m, 1H), 2.77 (s, 2H), 2.60 - 2.53 m, 1H), 2.39 - 2.30 (m, 1H), 1.98 - 1.91 (m, 1H), 1.69 - 1.53 (m, 4H).
LC-MS(ESI): $[M+H]^+$ = 370.29.

Example 27

Synthesis of compound 27

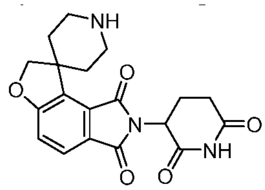

2-(2,6-dioxopiperidin-3-yl)-2-hydro-1H,7H-spiro[furo[3,2-e]isoindole-8,4'-piperidine]-1,3-dione Synthesis scheme:

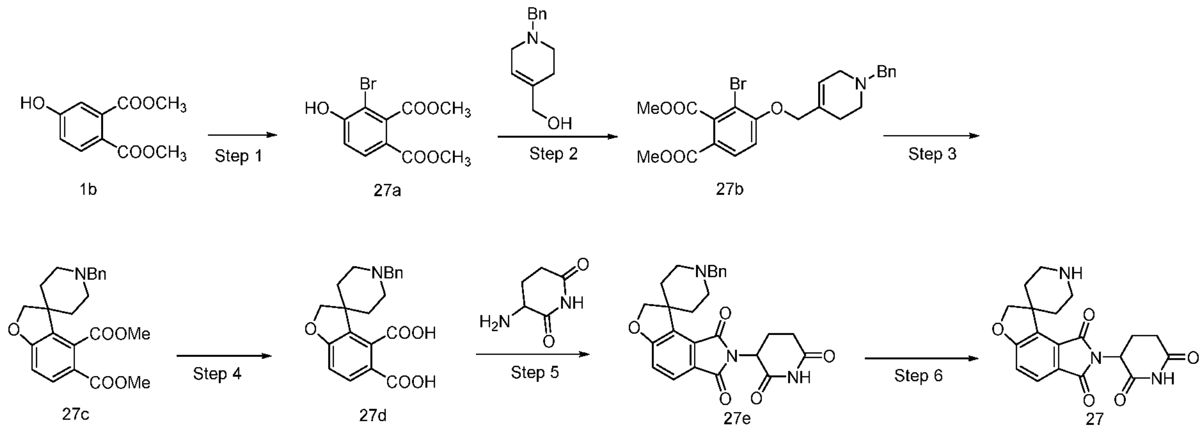


The first step: dimethyl 3-bromo-4-hydroxyphthalate (compound 27a)

Compound 1b (33.6g, 160mmol, 1.0eq) was dissolved in trifluoroacetic acid (100mL), N-bromosuccinimide (34.2g, 192mmol, 1.2eq) was added to the reaction system, and reacted overnight at room temperature. The resulting reaction system was concentrated under reduced pressure, water (100 mL) was added and extracted with ethyl acetate (100 ml*3 times). The mixture was washed with saturated brine (100 mL). The organic phases was combined and dried over anhydrous sodium sulfate. The concentrated crude product was purified by reverse column chromatography to obtain light yellow solid compound 27a (9.8 g, 21.3%).
$^1$H NMR (600MHz, DMSO-$d_6$) δ11.68(s, 1H), 7.87(d, *J*=12.0Hz, 1H), 7.10(d, *J*=12.0Hz, 1H), 3.84(s, 3H), 3.79 (s, 3H).
LCMS(ESI): [M-H]-=287.09.

The second step: dimethyl 4-((1-benzyl 1,2,3,6-tetrahydropyridin-4-yl) methoxy)-3-bromophthalate (compound 27b)

Compound 27b was prepared by referring to the procedure similar to step 3 in Example 17.
$^1$H NMR (600MHz, DMSO-d$_6$) δ8.00 (d, J = 8.8Hz, 1H), 7.40-7.29 (m, 5H), 7.27-7.24 (m, 1H), 5.84 (tt, J = 3.4, 1.6 Hz, 1H), 4.68(s, 2H), 3.86(s, 3H), 3.81(s, 3H), 3.54(s, 2H), 2.93-2.92(m, 2H), 2.54(t, J=5.7Hz, 2H), 2.16(s, 2H).
LCMS (ESI): $[M+H]^+$=474.09.

The third step: dimethyl 1'-benzyl-2H-spiro(benzofuran-3,4'-piperidine)-4,5-dicarboxylate (compound 27c)

27c was prepared by referring to the similar procedure of step 4 in Example 17.

$^1$H NMR (600MHz, DMSO-d$_6$) δ7.84(d, J=8.5Hz, 1H), 7.38-7.29(m, 4H), 7.29-7.23(m, 1H), 7.00(d, J=8.5Hz, 1H), 4.52(s, 2H), 3.86(s, 3H), 3.78(s, 3H), 3.48(s, 2H), 2.75(dd, J= 11.6, 3.7Hz, 2H), 2.08~ 1.93(m ,4H),1.58(d, J=12.2Hz, 2H).
LCMS (ESI): $[M+H]^+$=395.71

The fourth step: 1'-benzyl-2H-spiro[benzofuran-3,4'-piperidine]-4,5-dicarboxylic acid (compound 27d)

27d was prepared by referring to the similar procedure of step 5 in Example 17.

$^1$H NMR (400MHz, DMSO-d6) δ7.81(d, J=8.5Hz, 1H), 7.60-7.65(m, 2H), 7.44-7.39(m, 3H), 6.94(d, J=8.5Hz, 1H), 4.61(s, 2H), 4.25(s, 2H), 3.16(s, 4H), 2.39(s, 2H), 1.78(d, J=12.2Hz, 2H).
LCMS (ESI) $[M+H]^+$ = 368.30

The fifth step: 1'-benzyl-2-(2,6-dioxopiperidin-3-yl)-2-hydro-1H,7H-spiro[furo[3,2-e]isoindole -8,4'-piperidine]-1,3-dione (compound 27e)

Compound 27e was prepared by referring to the procedure similar to step 6 in Example 17.

$^1$H NMR (400MHz, $CD_3OD$) δ7.79(d, J=8.2Hz, 1H), 7.69-7.44(m, 5H), 7.19(d, J=8.1Hz, 1H), 5.13(dd, J=12.6, 5.4Hz, 1H), 4.80(s, 2H), 4.38(s, 2H), 3.55(d, J= 13.0Hz, 2H), 3.35-3.36(m, 1H), 3.17(t, J = 13.4Hz, 2H), 2.97-2.85(m, 2H),

2.81-2.65(m, 2H), 2.17-1.98(m, 3H).
LCMS (ESI) $[M+H]^+$=460.29

The sixth step: 2-(2,6-dioxopiperidin-3-yl)-2-hydro-1H,7H-spiro[furo[3,2-e]isoindole-8,4'-piperidine]-1,3-dione (compound 27)

Compound 27 was prepared by referring to the steps similar to step 7 in Example 17.

$^1$H NMR (600MHz, $CD_3OD$) δ7.79 (dd, J = 8.2, 1.2Hz, 1H), 7.19 (dd, J = 8.1, 1.3Hz, 1H), 5.15 (dd, J = 12.9, 5.4Hz, 1H), 4.78(s, 2H), 3.49(dt, J=13.3,2.7Hz, 2H), 3.14(td, J=13.7, 2.8Hz, 2H), 2.94-2.87(m, 3H), 2.80-2.70(m,2H), 2.18-2.14(m, 1H), 2.04(d, J= 14.3Hz, 2H).
LCMS (ESI) $[M+H]^+$=370.39

Example 28

Synthesis of compound 28

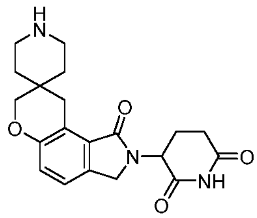

3-(1'-oxo-1',9'-dihydro-7'H-spiro[piperidin-4,8'-pyrano[3,2-e]isoindol]-2'(3'H)-yl)piperidine-2,6-dione Synthesis scheme:

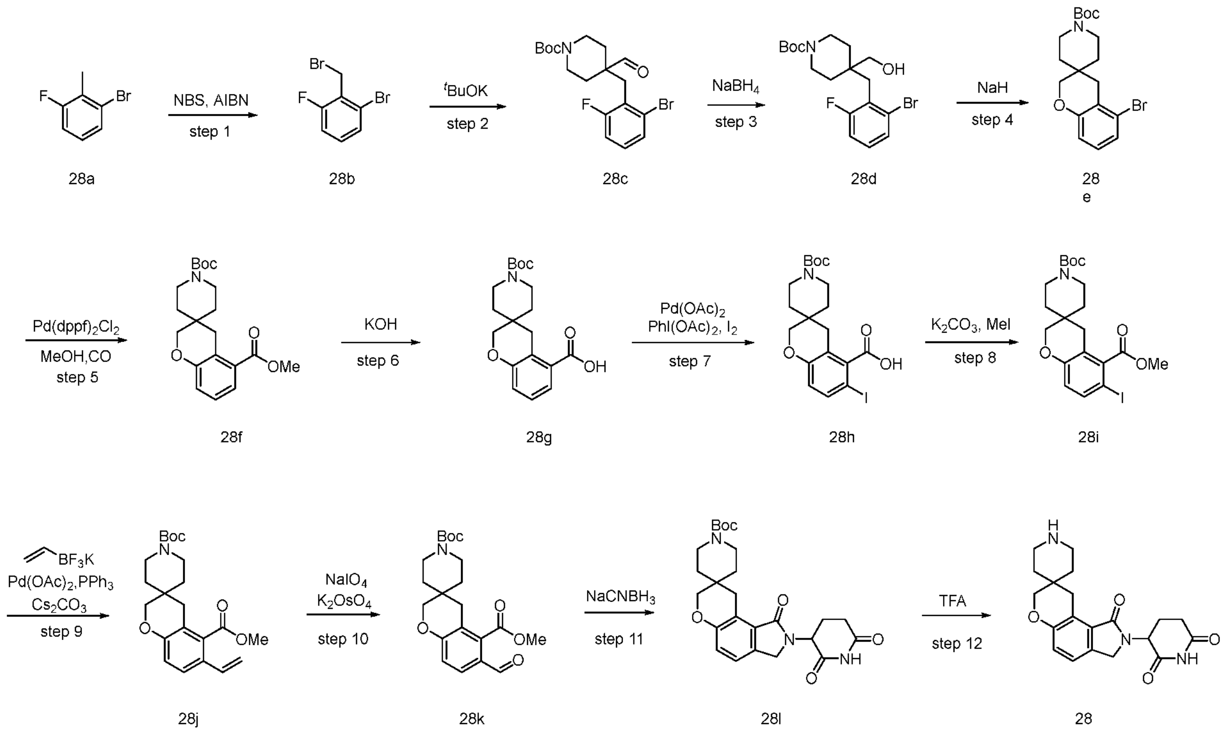

28a 28b 28c 28d 28e
28f 28g 28h 28i
28j 28k 28l 28

The first step: 1-bromo-2-(bromomethyl)-3-fluorobenzene (compound 28b)

Compound 28b was prepared by referring to the procedure similar to step 3 in Example 26.
$^1$H NMR (600MHz, $CDCl_3$) δ7.39 (d, J = 8.1Hz, 1H), 7.21-7.15 (m, 1H), 7.08-7.03 (m, 1H), 4.65 (d, J = 1.7Hz, 2H).

The second step: tert-butyl 4-(2-bromo-6-fluorobenzyl)-4-formylpiperidine-1-carboxylate (compound 28c)

Compound 28c was prepared by referring to the similar procedure of step 4 in Example 26.
$^1$H NMR (600MHz, $CDCl_3$) δ9.65 (d, J = 2.9Hz, 1H), 7.37 (d, J = 8.0Hz, 1H), 7.14-7.08 (m, 1H), 7.04-6.98 (m, 1H), 4.15-3.70(m,2H), 3.02(d, J=2.4Hz,2H), 2.90-2.60(m,2H), 2.18-1.94(m,2H), 1.76-1.62(m,2H), 1.44 (s,9H).
LC-MS (ESI): $[M-Boc+H]^+$=300.06.

The third step: tert-butyl 4-(2-bromo-6-fluorobenzyl)-4-(hydroxymethyl)piperidine-1-carboxylate (compound 28d)

Compound 28d was prepared by referring to the procedure similar to step 5 in Example 26.
$^1$H NMR (600MHz, $CDCl_3$) δ7.39(d, J=8.0Hz, 1H), 7.12-7.07(m, 1H), 7.05-6.99(m, 1H), 3.80-3.65(m, 2H), 3.62( d, J=6.2Hz, 2H), 3.11(t, J= 11.0Hz, 2H), 2.94(d, J=2.6Hz, 2H), 1.67-1.62(m, 2H), 1.62-1.59(m, 1H), 1.55-1.47(m, 2H), 1.44(s,9H).
LC-MS (ESI): $[M-Boc+H]^+$=301.98.

The fourth step: tert-butyl 5-bromospiro[chroman-3,4'-piperidine]-1'-carboxylate (compound 28e)

Compound 28e was prepared by referring to the procedure similar to step 6 in Example 26.
$^1$H NMR (400MHz, $CDCl_3$) δ7.15(dd, J=7.9, 1.1Hz, 1H), 7.02-6.95(m, 1H), 6.78(dd, J=8.2, 1.1Hz, 1H), 3.85(s, 2H), 3.64-3.51(m,2H), 3.45-3.32(m,2H), 2.64(s,2H), 1.52-1.44(m, 13H).
LC-MS (ESI): $[M-tBu+H]^+$=325.98.

Step 5: 1'-(tert-butyl)-5-methylspiro[chroman-3,4'-piperidine]-1',5-dicarboxylate (compound 28f)

Triethylamine (2.5 g, 24.6 mmol) and $Pd(dppf)_2Cl_2$•$CH_2Cl_2$ (670.7 mg, 0.8 mmol) were added to a methanol solution (60 mL) of compound 28e (3.1 g, 8.2 mmol) under the carbon monoxide atmosphere, and subsequently shifted to 65 °C to react overnight. After the reaction was completed, it was spin-dried and purified by column chromatography (PE:EA=0-20%) to obtain colorless oily liquid compound 28f (2.7 g, 90%).
$^1$H NMR (400MHz, $CDCl_3$) δ7.52(d, J=7.5Hz, 1H), 7.20-7.11(m, 1H), 6.99(d, J=8.0Hz, 1H), 3.90(s, 2H), 3.88 (s,3H), 3.60-3.46(m,2H), 3.46-3.34(m,2H), 3.00(s,2H), 1.55-1.40(m,13H).
LC-MS (ESI): $[M-Boc+H]^+$=262.17.

Step 6: 1'-(tert-butoxycarbonyl)spiro[chroman-3,4'-piperidine]-5-carboxylic acid (compound 28g)

An aqueous solution (20mL) of potassium hydroxide (1.5g, 37mmol) was added to compound 28f in methanol/tetrahydrofuran (1/1, 40mL) at 0°C, and shifted to a 60°C oil bath to react overnight. The solution was spin-dried, added 1M hydrochloric acid solution (50mL), extracted with ethyl acetate (40mL*3 times), washed with saturated brine (80mL), combined the organic phases, and spin-dried to obtain the white solid compound 28g (2.5g, 97% ), which was directly used for the next step.
LC-MS (ESI): [M-H]-=346.20.

The seventh step: 1'-(tert-butoxycarbonyl)-6-iodospiro[chroman-3,4'-piperidine]-5-carboxylic acid (compound 28h)

To a solution of compound 28g (84.9mg, 0.244mmol) in N, N-dimethylformamide (1mL) was added iodine simple substance (62.0mg, 0.244mmol), iodobenzenediethyl ester (78.7mg, 0.244mmol) and palladium acetate (2.7mg, 0.012mmol) under air atmosphere. The mixture was reacted in an oil bath at 80°C for half an hour. The resulting mixture was extracted with ethyl acetate (10mL*3 times), washed with saturated brine (20mL), combined the organic phases, spin-dried to undergo the preparative liquid phase, and freeze-dried to obtain white solid compound 28h (72.3mg, 65%).
$^1$H NMR (400MHz, $CDCl_3$) δ7.54 (d, J = 8.6Hz, 1H), 6.63 (d, J = 8.5Hz, 1H), 3.89 (s, 2H), 3.64-3.45 (m, 2H), 3.45 -3.26(m,2H), 2.71(s,2H), 1.52-1.36(m,13H).
LC-MS (ESI): [M-H] =472.10.

Step 8: 1'-(tert-butyl)-5-methyl 6-iodospiro[chroman-3,4'-piperidine]-1',5-dicarboxylate (compound 28i)

Compound 28i was prepared by referring to the procedure similar to step 2 in Example 26.
$^1$H NMR (600MHz, $CDCl_3$) δ7.51 (d, J = 8.7Hz, 1H), 6.62 (d, J = 8.7Hz, 1H), 3.95 (s, 3H), 3.88 (s, 2H), 3.57-3.47 (m,2H), 3.40-3.27(m, 2H), 2.59(s,2H), 1.49-1.41(m, 13H).
LC-MS (ESI): [M-Boc+H]$^+$=388.19.

Step 9: 1'-(tert-butyl)5-methyl 6-vinylspiro[chroman-3,4'-piperidine]-1',5-dicarboxylate (compound 28j)

Compound 28j was prepared by referring to the procedure similar to step 7 in Example 26.
LC-MS (ESI): [M+H]$^+$=388.09.

Step 10: 1'-(tert-butyl)-5 -methyl-6-formylspiro [chroman-3,4'-piperidine]-1',5-dicarboxylate (compound 28k)

Compound 28k was prepared by referring to the procedure similar to step 8 in Example 26.
$^1$H NMR (600MHz, $CDCl_3$) δ9.83 (s, 1H), 7.65 (d, J = 8.5Hz, 1H), 6.99 (d, J = 8.5Hz, 1H), 4.05-3.95 (m, 5H), 3.58 -3.47(m,2H), 3.40-3.31(m,2H), 2.64(s,2H), 1.55-1.29(m,13H).
LC-MS (ESI): [M-tBu+H]$^+$=334.19.

The eleventh step: 2'-(2,6-dioxopiperidin-3-yl)-1'-oxo-1',2',3',9'-tetrahydro-7'-H-spiro[piperidine-4,8'-pyrano[3,2-e]isoindole]-1-carboxylic acid tert-butyl ester (compound 28l)

Compound 28l was prepared by referring to the similar procedure of step 9 in Example 26.
$^1$H NMR (600MHz, DMSO-$d_6$) δ10.99(s, 1H), 7.29(d, J=8.2Hz, 1H), 7.02(d, J=8.2Hz, 1H), 5.01(dd, J=13.2, 5.1Hz, 1H), 4.30(d, J=16.9Hz, 1H), 4.19(d, J=16.9Hz, 1H), 3.98(s, 2H), 3.47-3.38(m, 2H), 3.31-3.24( m,2H), 3.12-3.02(m, 2H), 2.92-2.85(m, 1H), 2.63-2.56(m, 1H), 2.42-2.32(m, 1H), 2.00-1.94(m, 1H), 1.51-1.30(m, 11H), 1.38-1.30(m, 2H).
LC-MS (ESI): [M-Boc+H]$^+$=370.29.

The twelfth step : 3-(1'-oxo-1',9'-dihydro-7'H-spiro[piperidine-4,8'-pyrano[3,2-e]isoindole]-2'(3'H)-yl)piperidine-2,6-dione (compound 28)

Compound 28 was prepared by referring to the procedure similar to step 10 in Example 26.
$^1$H NMR (600MHz, DMSO-$d_6$) δ11.00(s, 1H), 7.31(d, J=8.2Hz, 1H), 7.04(d, J=8.2Hz, 1H), 5.01(dd, J=13.3, 5.1Hz, 1H), 4.31(d, J= 17.0Hz, 1H), 4.20(d, J= 17.0Hz, 1H), 4.05(s, 2H), 3.21-3.03(m, 6H), 2.94-2.84( m, 1H), 2.64-2.56(m, 1H), 2.38(qd, J= 13.0, 4.2Hz, 1H), 2.02-1.91(m, 1H), 1.70-1.52(m, 4H).
LC-MS (ESI): [M+H]$^+$=370.29.

Example 29

Synthesis of PROTAC 1

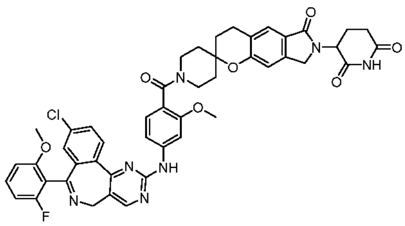

3-(1-(4-((9-chloro-7-(2-fluoro-6-methoxyphenyl)-SH-benzo[c]pyrimido[4,5-e]azepine- 2-yl)amino)-2-methoxybenzoyl)-6'-oxo-3',4',6',8'-tetrahydro-7'H-spiro[piperidine-4,2 '-pyrano[2,3-f]isoindol]-7'-yl)piperidine-2,6-dione

Synthesis scheme:

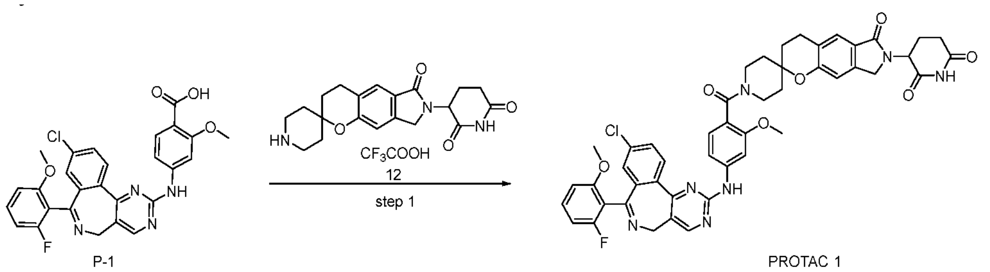


The first step: 3-(1-(4-((9-chloro-7-(2-fluoro-6-methoxyphenyl)-5H-benzo[c]pyrimido[4,5-e]azepine-2-yl)amino)-2-methoxybenzoyl)-6'-oxo-3',4',6',8'-tetrahydro-7'H-spiro[piperidine -4,2'-pyrano[2,3-f]isoindol]-7'-yl)piperidine-2,6-dione (compound PROTAC 1)

To a solution of compound 12 (15 mg, 31.03 μmol) and diisopropylethylamine (13 mg, 100.58 μmol) in N,N-dimethylformamide (1 mL) at room temperature was added the compound P-1 (17 mg, 32.76 μmol) and HATU (15 mg, 39.45 μmol). The reaction was stirred at room temperature for 1.5 hours, and then purified by preparative high performance liquid chromatography to obtain the yellow solid compound PROTAC 1 (8.7 mg, 32.22%).

$^1$H NMR (600MHz, $CD_3OD$) δ8.66(s, 1H), 8.49(d, J=8.5Hz, 1H), 7.93(d, J=8.4Hz, 1H), 7.87(s, 1H), 7.63(dd, J=15.4, 8.1Hz, 1H), 7.54(d, J=9.5Hz,1H), 7.49(s, 1H), 7.41(d, J=6.6Hz, 1H), 7.23(dd, J=37.6, 8.1Hz, 1H), 7.08-6.88(m, 3H), 5.17-5.07(m, 1H), 4.52-4.32(m, 3H), 4.29(s, 1H), 3.93(d, J=5.4Hz, 3H), 3.58-3.44(m,3H), 3.41-3.35(m,2H),3.02-2.86(m,3H), 2.80(t, J=15.9Hz,1H), 2.53-2.43(m,1H), 2.40-2.28(m,1H), 2.24-2.12(m,1H), 2.02-1.89(m,3H), 1.79(s, 3H), 1.69-1.56(m, 1H).

LC-MS(ESI): $[M+H]^+$=870.45/872.45.

Example 30

Synthesis of PROTAC 2

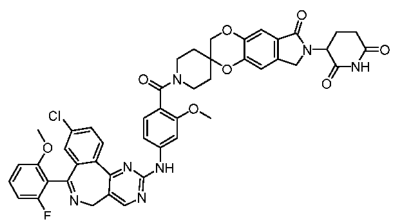

3-(1-(4-((9-chloro-7-(2-fluoro-6-methoxyphenyl)-5H-benzo[c]pyrimido[4,5-e]azepine- 2-yl)amino)-2-methoxybenzoyl)-6'-oxo-6',8'-dihydro-3'H,7'H-spiro[piperidine-4,2'- [1,4]dioxino[2,3-f]isoindole]-7'-yl)piperidine-2,6-dione

Synthesis scheme:

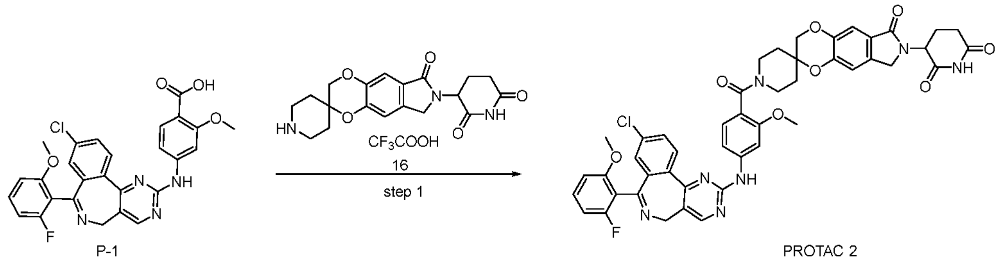

The first step: 3-(1-(4-((9-chloro-7-(2-fluoro-6-methoxyphenyl)-5H-benzo[c]pyrimido[4,5-e] azepine-2-yl)amino)-2-methoxybenzoyl)-6'-oxo-6',8'-dihydro-3'H,7'H-spiro[piperidine-4 2'-[1,4]dioxino[2,3-f]isoindol]-7'-yl)piperidine-2,6-dione (compound PROTAC 2)

To a solution of compound 16 (10 mg, 20.60 μmol) and diisopropylethylamine (8 mg, 61.90 μmol) in N, N-dimethylformamide (1 mL) at room temperature, compound P-1 (11 mg, 21.20 μmol) and HATU (10 mg, 26.30 μmol) were added. The reaction was stirred at room temperature for 1.5 hours, and then purified by preparative high performance liquid chromatography to obtain the yellow solid compound PROTAC 2 (4.2 mg, 23.37%).

$^1H$ NMR (600MHz, $CD_3OD$) δ8.64 (s, 1H), 8.45 (d, J = 8.5Hz, 1H), 7.92-7.84 (m, 2H), 7.57 (dd, J = 15.4, 8.1Hz, 1H), 7.43(s,1H), 7.40(d,J=7.8Hz,1H), 7.29(s,1H), 7.28-7.19(m,1H), 7.15(d,J=15.4Hz,1H), 6.92( s,2H), 5.16-5.07(m,1H), 4.55-4.46(m,1H), 4.46-4.31(m,2H), 4.25-4.14(m,1H), 4.13-4.05(m,2H), 3.92(d,J=8.4Hz,3H), 3.54(s,2H),3.50-3.35(m,2H), 2.97-2.85(m,1H), 2.85-2.74(m,1H), 2.53-2.42( m,1H), 2.20-2.12(m,1H), 1.96(s,1H), 1.91-1.77(m,2H), 1.74-1.58(m,1H).

LC-MS(ESI): $[M+H]^+$=872.09/873.97.

Example 31

Synthesis of PROTAC 3

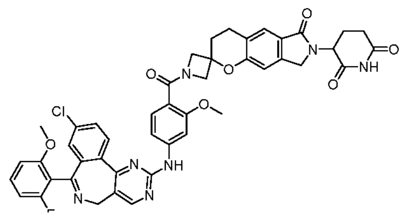

3-(1-(4-((9-chloro-7-(2-fluoro-6-methoxyphenyl)-5H-benzo[c]pyrimido[4,5-e]azepine- 2-yl)amino)-2-methoxybenzoyl)-6'-oxo-3',4',6',8'-tetrahydro-7'H-spiro[azetidine- 3,2'-pyrano[2,3-f]isoindole]-7'-yl)piperidine-2,6-dione

Synthesis scheme:

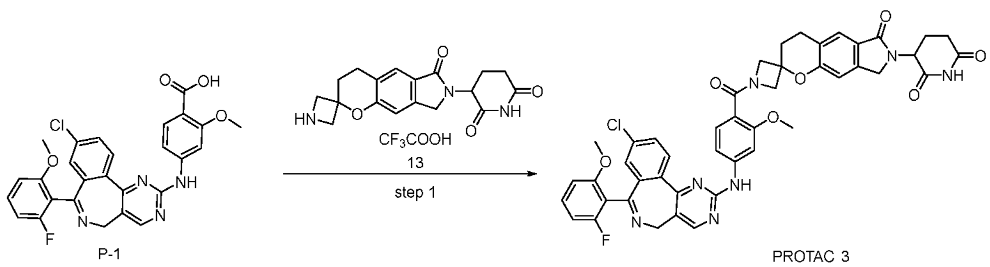


The first step: 3-(1-(4-((9-chloro-7-(2-fluoro-6-methoxyphenyl)-5H-benzo[c]pyrimido[4,5-e]azepine-2-yl)amino)-2-methoxybenzoyl)-6'-oxo-3',4',6',8'-tetrahydro-7'H-spiro[azetidine-3,2'-pyrano[2,3-f]isoindol]-7'-yl)piperidine-2,6-dione (compound PROTAC 3)

To a solution of compound 13 (10 mg, 21.96 μmol) and diisopropylethylamine (9 mg, 69.63 μmol) in N, N-dimethylformamide (1 mL) at room temperature, compound P-1 (12 mg, 23.12 μmol) and HATU (10 mg, 26.30 μmol) were added. The reaction was stirred at room temperature for 1.5 hours, and then purified by preparative high performance liquid chromatography to obtain the yellow solid compound PROTAC 3 (6.2 mg, 33.52%).

$^1H$ NMR (600MHz, $CD_3OD$) δ8.67(s, 1H), 8.49(d, J=8.5Hz, 1H), 7.94(d, J=8.4Hz, 1H), 7.91(s, 1H), 7.63(dd ,J= 15.3,8.2Hz,1H), 7.55(s,1H), 7.50(s,1H), 7.40(s,2H), 7.07(s,1H), 7.05-6.88(m,2H), 5.12( td, J=12.9,5.1Hz,1H), 4.47-4.34(m,2H), 4.27-4.17(m,4H), 4.14(d, J=10.1Hz,1H), 3.96(s,3H),3.78 - 3.53(m,3H), 3.04-2.86(m,4H), 2.82-2.75(m,1H), 2.53-2.43(m,1H), 2.28-2.21(m,2H), 2.19-2.12(m, 1H).

LC-MS(ESI): $[M+H]^+$=841.98/843.98.

Example 32

Synthesis of PROTAC 4

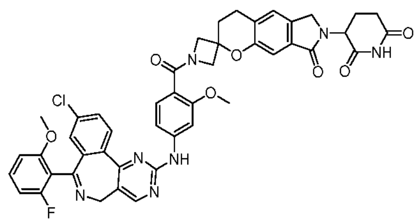

3-(1-(4-((9-chloro-7-(2-fluoro-6-methoxyphenyl)-SH-benzo[c]pyrimido[4,5-e]azepine- 2-yl)amino)-2-methoxybenzoyl)-8'-oxo-3',4',6',8'-tetrahydro-7'H-spiro[azetidine- 3,2'-pyrano[2,3-f]isoindole]-7'-yl)piperidine-2,6-dione

Synthesis scheme:

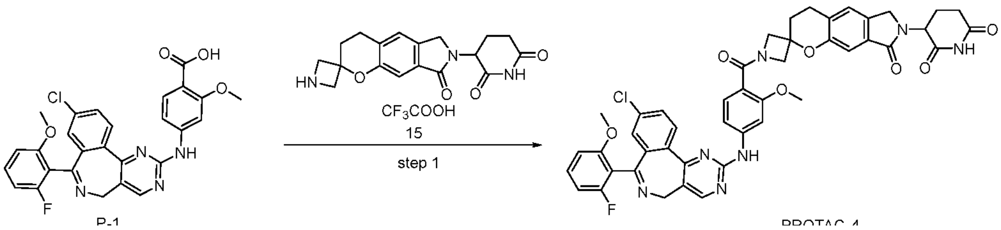


The first step: 3-(1-(4-((9-chloro-7-(2-fluoro-6-methoxyphenyl)-5H-benzo[c]pyrimido[4,5-e]azepine-2-yl)amino)-2-methoxybenzoyl)-8'-oxo-3',4',6',8'-tetrahydro-7'H-spiro[azetidine -3,2'-pyrano[2,3-f]isoindol]-7'-yl)piperidine-2,6-dione (compound PROTAC 4)

To a solution of compound 15 (10 mg, 21.96 μmol) and diisopropylethylamine (9 mg, 69.63 μmol) in N, N-dimethylformamide (1 mL), compound P-1 (12 mg, 23.12 μmol) and HATU (10 mg, 26.30 μmol) were added at room temperature. The reaction was stirred at room temperature for 1.5 hours, and then purified by preparative high performance liquid chromatography to obtain the yellow solid compound PROTAC 4 (7.8 mg, 42.17%).

$^1$H NMR (600MHz, $CD_3OD$) δ8.65(s, 1H), 8.45(d, J=8.5Hz, 1H), 7.92(s, 1H), 7.88(d, J=8.5Hz, 1H), 7.60-7.55 (m,1H), 7.44(s,1H), 7.39(s,2H), 7.31(s,1H), 7.28(s,1H), 6.86(s,2H), 5.14(td,J=13.0,5.1 Hz, 1H), 4.45-4.33(m, 2H), 4.26-4.16(m, 4H), 4.12(d, J=10.0Hz, 1H), 3.96(s, 3H), 3.06-2.86(m, 4H), 2.83-2.75(m,1H), 2.53-2.43(m,1H), 2.28-2.14(m,3H).

LC-MS(ESI): $[M+H]^+$=841.98/843.98.

Example 33

Synthesis of PROTAC 5

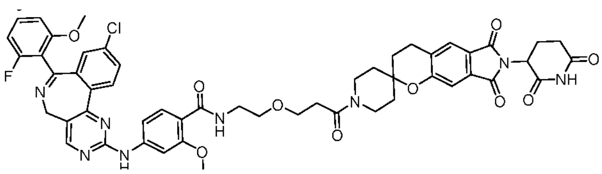

4-((9-chloro-7-(2-fluoro-6-methoxyphenyl)-5H-benzo[c]pyrimido[4,5-e]azepine-2-yl)amino)-N-(2-(3-(7'-(2,6-dioxopiperidin-3-yl)-6',8'-dioxo-4',6',7',8' -tetrahydro-3'H-spiro[piperidine-4,2'-pyrano[2,3-fjisoindol]-1-yl)-3-oxopropoxy)ethyl)- 2-methoxybenzamide

Synthesis scheme:

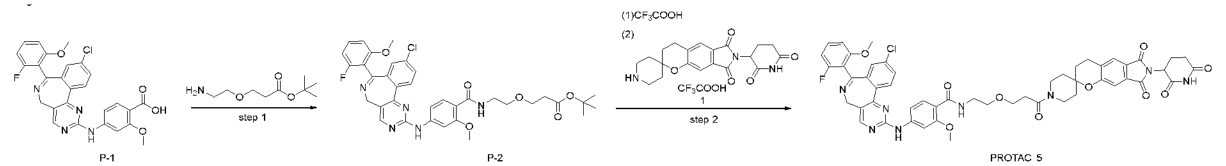


The first step: 3-(2-(4-((9-chloro-7-(2-fluoro-6-methoxyphenyl)-5H-benzo[c]pyrimido[4,5-e]azepine-2-yl)amino)-2-methoxybenzamido)ethoxy)propanoic acid tert-butyl ester (compound P-2)

At room temperature, to a solution of tert-butyl 3-(2-aminoethoxy)propionate (20 mg, 105.68 μmol) and diisopropylethylamine (38 mg, 294.01 μmol) in N,N-dimethylformamide (1 mL), compound P-1 (50 mg, 96.35 μmol) and HATU (45 mg, 118.35 μmol) were added. The reaction was stirred at room temperature for 1.5 hours. Compound P-2 (54 mg, 81.20%) was obtained as a yellow solid by reverse phase purification.

$^1$H NMR (600MHz, $CD_3OD$) δ8.67(s, 1H), 8.45(d, J=8.5Hz, 1H), 8.04(s, 1H), 7.97(d, J=8.7Hz, 1H), 7.86(d, J=8.5Hz,1H), 7.56(dd, J=15.2, 8.4Hz, 1H), 7.43(s,1H), 7.38(dd, J=8.7, 2.0Hz, 1H), 6.92(s,2H), 4.05(s, 3H), 3.77(t, J=6.0Hz,2H), 3.65(t, J=5.5Hz, 2H), 3.61(t, J=5.5Hz, 2H), 2.54(t,J= 6.1Hz, 2H), 1.44(s, 9H).

LC-MS(ESI): $[M+H]^+$=690.11/692.04.

The second step: 4-((9-chloro-7-(2-fluoro-6-methoxyphenyl)-5H-benzo[c]pyrimido[4,5-e]azepine-2-yl)atnino)-N-(2-(3-(7'-(2,6-dioxopiperidin-3-yl)-6',8'-dioxo-4',6',7 ',8'-tetrahydro-3'H-spiro[piperidin-4,2'-pyrano[2,3-f]isoindol]-1-yl)-3-oxopropoxy) ethyl)-2-methoxybenzamide (compound PROTAC 5)

To a solution of compound P-2 (54 mg, 78.24 μmol) in dichloromethane (3 mL) was added trifluoroacetic acid (1 mL) under stirring at room temperature. After the reaction was stirred at room temperature for 3 hours, the resulting mixture was concentrated in vacuo, dissolved by adding N, N-dimethylformamide (1 mL), to which was added diisopropylethylamine (52 mg, 402.33 μmol), then added compound 1 (15 mg, 30.16 μmol) and HATU (15 mg, 39.45 μmol), and then purified by preparative high performance liquid chromatography to obtain the white solid compound PROTAC 5 (8.4 mg, 27.87%).

$^1$H NMR (600MHz, $CD_3OD$) δ8.64(s, 1H), 8.37(d, J=8.4Hz, 1H), 8.07-7.95(m, 2H), 7.78(d, J=8.4Hz, 1H), 7.52 -7.43(m,2H), 7.36(s, 2H), 7.20(s, 1H), 6.87(s, 2H), 5.08-5.01(m, 1H), 4.31(d, J=11.9Hz,1H), 4.13-4.07(m,1H), 4.02(d, J=3.2Hz, 3H), 3.89-3.79(m, 3H), 3.72-3.66(m, 2H), 3.64-3.59(m, 2H), 3.52-3.44(m,2H), 3.13(t, J=12.6Hz, 2H), 2.86-2.62(m, 5H), 2.11-2.04(m,1H), 1.76(d, J=11.4Hz, 3H), 1.66 - 1.58(m, 1H), 1.52(d, J= 13.0Hz,1H).

LC-MS(ESI): $[M+H]^+$=999.13/1001.01.

Example 34

Synthesis of PROTAC 6

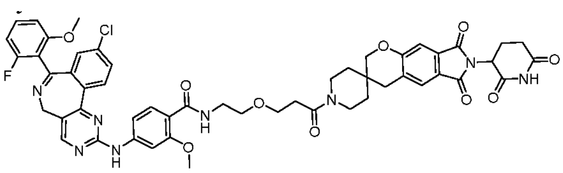

4-((9-chloro-7-(2-fluoro-6-methoxyphenyl)-5H-benzo[c]pyrimido[4,5-e]azepine-2-yl)amino)-N-(2-(3-(7'-(2,6-dioxopiperidin-3-yl)-6',8'-dioxo-4',6',7',8' -tetrahydro-2'H-spiro[piperidine-4,3'-pyrano[2,3-fjisoindol]-1-yl)-3-oxopropoxy)ethyl)- 2-methoxybenzamide

Synthesis scheme:

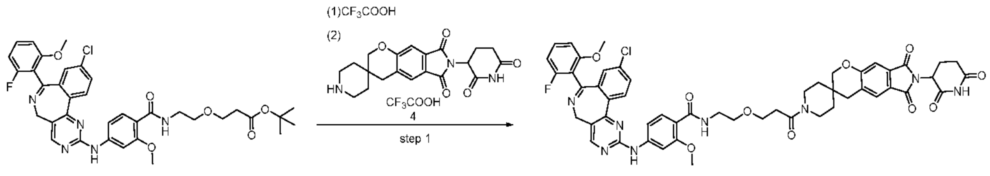


The first step: 4-((9-chloro-7-(2-fluoro-6-methoxyphenyl)-5H-benzo[c]pyrimido[4,5-e]azepine-2-Base)amino)-N-(2-(3-(7'-(2,6-dioxopiperidin-3-yl)-6',8'-dioxo-4',6',7',8'-tetrahydro-2'H-spiro[piperidin-4,3'-pyrano[2,3-f]isoindol]-1-yl)-3-oxopropoxy) ethyl)-2-methoxybenzamide (Compound PROTAC 6)

To a solution of compound P-2 (54 mg, 78.24 μmol) in dichloromethane (3 mL) was added trifluoroacetic acid (1 mL) with stirring at room temperature. After the reaction was stirred at room temperature for 3 hours, the resulting mixture was concentrated in vacuo, dissolved by adding N,N-dimethylformamide (1 mL), to which was added diisopropylethylamine (52 mg, 402.33 μmol), and then added compound 4 (15 mg, 30.16 μmol) and HATU (15 mg, 39.45 μmol). The resulting mixture was quenched with water and purified by preparative high performance liquid chromatography to obtain the white solid compound PROTAC 6 (5.4 mg, 17.92%).

$^1$H NMR (600MHz, $CD_3OD$) δ8.65(s, 1H), 8.40(d, J=8.3Hz, 1H), 8.07(s, 1H), 8.00(dd, J=8.7, 2.9Hz, 1H), 7.81 (d,J=8.3Hz,1H), 7.52(dd,J=15.5,7.6Hz,1H), 7.48(d,J=8.3Hz,1H), 7.44-7.31(m,2H), 7.06(d, J=6.5Hz,1H), 6.92(s,2H), 5.07-5.02(m,1H), 4.13(s,2H), 4.03(d,J=2.5Hz,2H), 4.01-3.94(m,2H), 3.89-3.80(m,2H), 3.72-3.54(m,6H), 2.88-2.63(m,6H), 2.10-2.04(m,1H), 1.63(s,1H), 1.55-1.33(m ,5H).

LC-MS(ESI): $[M+H]^+$=999.17/1001.17.

Example 35

Synthesis of PROTAC 7

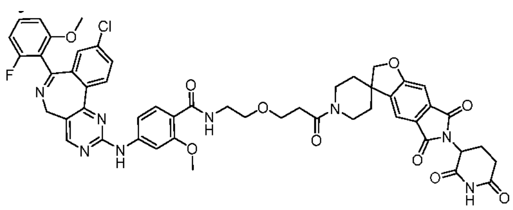

4-((9-chloro-7-(2-fluoro-6-methoxyphenyl)-5H-benzo[c]pyrimido[4,5-e]azepine-2-yl)amino) -N-(2-(3-(6-(2,6-dioxopiperidin-3-yl)-5,7-dioxo-6,7-dihydro-2H,5H-spiro[furan [2,3-f]isoindole-3,4'-piperidin]-1'-yl)-3 -oxopropoxy)ethyl)-2-methoxybenzamide

Synthesis scheme:

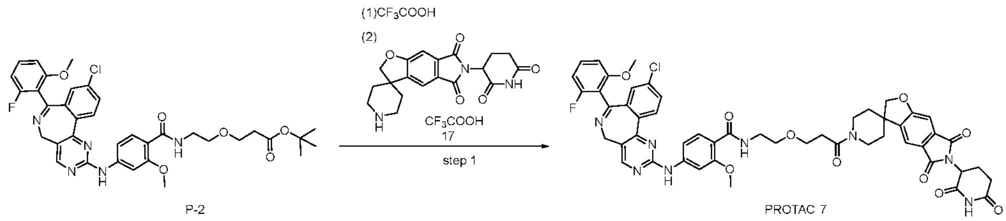

P-2
PROTAC 7

The first step: 4-((9-chloro-7-(2-fluoro-6-methoxyphenyl)-5H-benzo[c]pyrimido[4,5-e]azepine-2-yl)amino)-N-(2-(3-(6-(2,6-dioxopiperidin-3-yl)-5,7-dioxo-6,7-dihydro-2H,5H-spiro[furo[2,3-f]isoindole-3,4'-piperidin]-1'-yl)-3-oxopropoxy)ethyl)-2-methoxybenzylamide (Compound PROTAC 7)

To a solution of compound P-2 (54 mg, 78.24 μmol) in dichloromethane (3 mL) was added trifluoroacetic acid (1 mL) with stirring at room temperature. After the reaction was stirred at room temperature for 3 hours, the resulting mixture was concentrated in vacuo, dissolved by adding N, N-dimethylformamide (1 mL), to which was added diisopropylethylamine (52 mg, 402.33 μmol), and then added compound 17 (15 mg, 31.03 μmol) and HATU (15 mg, 39.45 μmol). The resulting mixture was quenched with water, and purified by preparative high performance liquid chromatography to obtain compound PROTAC 7 (8.2 mg, 26.82%) as a light yellow solid.

$^1H$ NMR (600MHz, $CD_3OD$) δ8.64 (d, J = 2.9Hz, 1H), 8.39 (s, 1H), 8.03-7.94 (m, 2H), 7.82 (d, J = 7.6Hz, 1H), 7.60 (d,J=10.9Hz,1H), 7.53(dd,J=15.3,8.3Hz,1H), 7.43-7.33(m,2H), 7.05(s,1H), 6.91(s,2H), 5.09( dd,J=12.7,5.4Hz,1H), 4.61(s,2H), 4.53(d,J=13.6Hz,1H), 4.23-4.05(m,3H), 4.04(s,3H), 3.90-3.81 (m,2H), 3.74-3.59(m,4H), 3.22-3.14(m,2H), 2.95-2.81(m,2H), 2.81-2.65(m,5H), 2.16-2.07(m,1H), 1.97-1.71(m,5H).

LC-MS(ESI): $[M+H]^+$=984.98/986.98.

Example 36

Synthesis of PROTAC 8

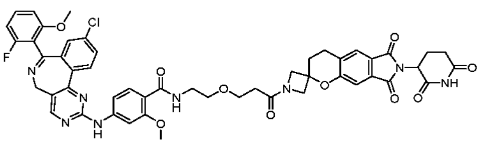

4-((9-chloro-7-(2-fluoro-6-methoxyphenyl)-5H-benzo[c]pyrimido[4,5-e]azepine-2-yl)amino) -N-(2-(3-(7'-(2,6-dioxopiperidin-3-yl)-6',8'-dioxo-4',6',7',8' -tetrahydro-3'H-spiro[azetidine-3,2'-pyrano[2,3-f]isoindol]-1-yl)-3-oxopropoxy)ethyl base)-2-methoxybenzamide

Synthesis scheme:

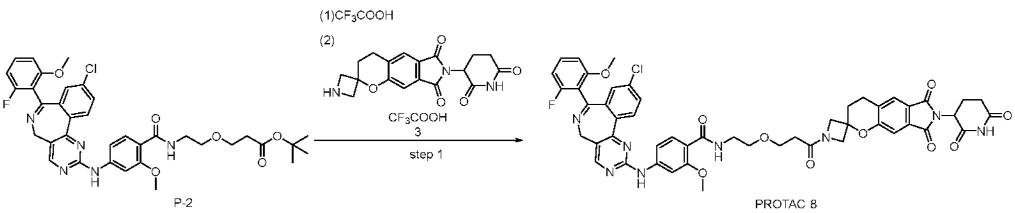

P-2
PROTAC 8

The first step: 4-((9-chloro-7-(2-fluoro-6-methoxyphenyl)-5H-benzo[c]pyrimido[4,5-e]azepine-2-yl)amino)-N-(2-(3-(7'-(2,6-dioxopiperidin-3-yl)-6',8'-dioxo-4',6',7 ',8'-tetrahydro-3'H-spiro[azetidine-3,2'-pyrano[2,3-f]isoindol]-1-yl)-3-oxypropoxy)ethyl)-2-methoxybenzamide (Compound PROTAC 8)

To a solution of compound P-2 (54 mg, 78.24 μmol) in dichloromethane (3 mL) was added trifluoroacetic acid (1 mL) with stirring at room temperature. After the reaction was stirred at room temperature for 3 hours, the resulting mixture

was concentrated in vacuo, dissolved by adding N,N-dimethylformamide (1 mL), to which was added diisopropylethylamine (52 mg, 402.33 μmol), and then added compound 3 (15 mg, 31.96 μmol) and HATU (15 mg, 39.45 μmol). The resulting mixture was quenched with water and purified by preparative HPLC to obtain the yellow solid compound PROTAC 8 (7.7 mg, 24.80 %).

$^1$H NMR (600MHz, $CD_3OD$) δ8.64(s, 1H), 8.39(d, J=8.3Hz, 1H), 8.06-7.99(m, 1H), 7.97(dd, J=8.6, 2.4Hz, 1H), 7.80(d, J=8.4Hz,1H), 7.50(dd, J=15.2,8.2Hz,1H), 7.46(s,1H), 7.40-7.28(m,2H), 7.16(s,1H), 6.89(s,2H),5.09-5.01(m,1H), 4.39-4.28(m,2H), 4.06-4.00(m,4H), 3.83(t, J=5.8Hz,2H), 3.71-3.66(m,2H), 3.66-3.59(m,2H), 2.89-2.79(m,3H), 2.76-2.60(m,3H), 2.56-2.44(m,2H), 2.19-2.04(m,3H).

LC-MS(ESI): $[M+H]^+$=970.97/972.97.

Example 37

Synthesis of PROTAC 9

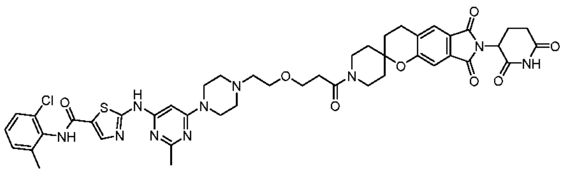

N-(2-chloro-6-methylphenyl)-2-((6-(4-(2-(3-(7'-(2,6-dioxopiperidin-3-yl)- 6',8'-dioxo-4',6',7',8'-tetrahydro-3'H-spiro[piperidine-4,2'-pyrano[2,3-f]isoindol]-1-yl)-3-oxopropoxy)ethyl)piperazin-1-yl)-2-methylpyrimidin-4-yl)amino)thiazole-5-carboxamide

Synthesis scheme:

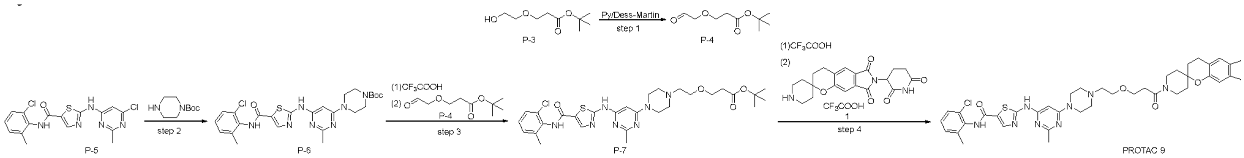


The first step: 3-(2-oxoethoxy) tert-butyl propionate (compound P-4)

To a dichloromethane (40 mL) solution of compound P-3 (1.5 g, 7.88 mmol) and pyridine (1.37 g, 17.35 mmol) was added Dess-Martin oxidizing agent (4.35 g, 10.25 mmol) at 0 °C under stirring. The reaction was stirred at room temperature for 14 hours. The resulting mixture was quenched with saturated aqueous sodium thiosulfate (50 mL) and saturated aqueous sodium bicarbonate (100 mL), and extracted three times by adding dichloromethane (150 mL). The organic phase was concentrated under vacuum, and the resulting mixture was purified by normal phase to obtain the colorless oily compound P-4 (1.2 g, 80.86 %).

$^1$H NMR (600MHz, DMSO-$d_6$) δ9.56(s, 1H), 4.17(s, 2H), 3.67(t, J=6.2Hz, 2H), 2.48(t, J=6.2Hz, 2H), 1.41 (s,9H).

The second step: tert-butyl 4-(6-((5-((2-chloro-6-methylphenyl)carbamoyl)thiazol-2-yl)amino)-2-methylpyrimidin-4-yl) piperazine-1-carboxylate (Compound P-6)

To a solution of N, N-dimethylformamide (15 mL) of N-tert-butoxycarbonylpiperazine (230 mg, 1.23 mmol) and diisopropylethylamine (380 mg, 2.94 mmol) was added compound P-5 (400 mg, 1.01 mmol). The reaction was stirred at 110 °C for 14 hours. The resulting mixture was quenched with water (150 mL), and extracted three times with the addition of ethyl acetate (150 mL), The organic phase was concentrated under vacuum and the resulting mixture was purified by normal phase to obtain the yellow solid compound P-6 (379 mg, 68.66 %).

$^1$H NMR (600MHz, DMSO-$d_6$) δ11.57(s, 1H), 9.90(s, 1H), 8.24(d, J=7.7Hz, 1H), 7.41(d, J=7.8Hz, 1H), 7.30 (d, J=6.9Hz,1H), 7.26(t, J=7.7Hz,1H), 6.11(s,1H), 3.55(s,4H), 3.43(s,4H), 2.43(s,3H), 2.24(s,3H), 1.43(s,9H).

LC-MS (ESI): $[M+H]^+$=544.12.

The third step: tert-butyl 3-(2-(4-(6-((5-((2-chloro-6-methylphenyl)carbamoyl)thiazol-2-yl)amino)-2-methylpyrimidine -4-yl) piperazin-1-yl) ethoxy) propionate (compound P-7)

To a solution of compound P-6 (380 mg, 78.24 μmol) in dichloromethane (20 mL) was added trifluoroacetic acid (4 mL) under stirring at room temperature. The reaction was stirred at room temperature for 3 hours, and the resulting mixture was concentrated in vacuo, dissolved by adding tetrahydrofuran (20 mL), to which was added diisopropylethylamine (420 mg, 3.25 mmol), and then added acetic acid (100 mg, 1.67 mmol) and titanic acid tetraisopropyl ester (0.5 mL). Finally compound P-4 (264 mg, 1.40 mmol) and sodium cyanoborohydride (176 mg, 2.80 mmol) were added. The reaction was stirred at room temperature for 3 hours, the resulting mixture was concentrated in vacuo, and light yellow oily compound P-7 (287 mg, 86.16%) was obtained by normal phase purification.

$^1$H NMR (600MHz, DMSO-$d_6$) δ11.65(s, 1H), 9.92(s, 1H), 8.25(s, 1H), 7.41(d, J=7.8Hz, 1H), 7.30(d, J=7.0Hz,1H), 7.27(t,J=7.7Hz,1H), 6.15(s,1H), 4.34(s,2H), 3.77-3.74(m,2H), 3.68-3.54(m,4H), 3.36(s,2H), 3.30-3.23(m,2H), 3.12(s,2H), 2.53(d,J=6.1Hz,2H), 2.45(s,3H), 2.24(s,3H), 1.42 (s,9H).

The fourth step: N-(2-chloro-6-methylphenyl)-2-((6-(4-(2-(3-(7'-(2,6-dioxopiperidine-3 -yl)-6',8'-dioxo-4',6',7',8'-tetrahydro-3'H-spiro[piperidin-4,2'-pyrano[2,3-f]isoindole]-1-yl)-3-oxopropoxy)ethyl)piperazin-1 -yl)-2-methylpyrimidin-4-yl) amino)thiazole-5 -carboxamide (Compound PROTAC 9)

To a solution of compound P-7 (35 mg, 56.80 μmol) in dichloromethane (2 mL) was added trifluoroacetic acid (0.5 mL) under stirring at room temperature. The reaction was stirred at room temperature for 3 hours. The resulting mixture was concentrated in vacuo, added N, N-dimethylformamide (1 mL) to dissolve, then added diisopropylethylamine (50 mg, 286.85 μmol) thereto, and then added compound 1 (30mg, 60.31μmol) and HATU (28mg, 73.64μmol). The reaction was stirred at room temperature for 3 hours, and then purified by preparative high performance liquid chromatography to obtain the white solid compound PROTAC 9 (16 mg, 28.67%).

$^1$H NMR (600MHz, $CD_3OD$) δ8.18(s, 1H), 7.64(s, 1H), 7.38(d, J=7.4Hz, 1H), 7.29(d, J=1.3Hz, 1H), 7.27(d ,J=5.3Hz,1H), 7.25(d,J=7.6Hz,1H), 6.18(s,1H), 5.10(dd,J=12.8,5.5Hz,1H), 4.62(s,2H), 4.38 (d, J=13.3Hz, 1H), 3.92(t, J=5.2Hz, 2H), 3.89-3.80(m, 3H), 3.74(s, 2H), 3.59(t, J=11.9Hz, 1H), 3.53-3.48(m,2H), 3.44(s,1H), 3.27-3.16(m,3H), 2.99(t,J=6.7Hz,2H), 2.92-2.67(m,6H), 2.53(s ,3H), 2.34(s,3H), 2.16-2.09(m,1H), 1.98-1.87(m,4H), 1.81-1.72(m,1H), 1.69-1.58(m,1H).

LC-MS (ESI): $[M+H]^+$=925.45.

Example 38

Synthesis of PROTAC 10

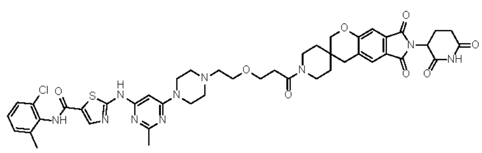

N-(2-chloro-6-methylphenyl)-2-((6-(4-(2-(3-(7'-(2,6-dioxopiperidin-3-yl)- 6',8'-dioxo-4',6',7',8'-tetrahydro-2'H-spiro[piperidine-4,3'-pyrano[2,3-f]isoindole]-1-yl)-3-oxopropoxy)ethyl)piperazin-1-yl)-2-methylpyrimidin-4-yl)amino)thiazole-5-carboxamide

Synthesis scheme:

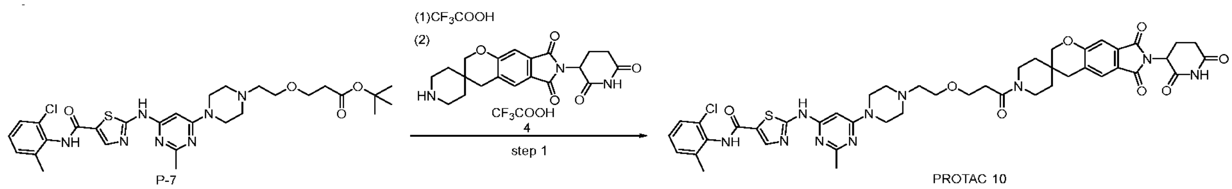


P-7

PROTAC 10

The first step: N-(2-chloro-6-methylphenyl)-2-((6-(4-(2-(3-(7'-(2,6-dioxopiperidine-3 -yl)-6',8'-dioxo-4',6',7',8'-tetrahydro-2'H-spiro[piperidine-4,3'-pyrano[2,3-f]isoindole]-1-yl)-3-oxopropoxy)ethyl)piperazin-1-yl)-2-methylpyrimidin-4-yl)atnino)thiazole-5-carboxatnide (Compound PROTAC 10)

To a solution of compound P-7 (35 mg, 56.80 μmol) in dichloromethane (2 mL) was added trifluoroacetic acid (0.5 mL) under stirring at room temperature. The reaction was stirred at room temperature for 3 hours. The resulting mixture was concentrated in vacuo, dissolved by adding N, N-dimethylformamide (1 mL), added diisopropylethylamine (50 mg, 286.85 μmol) thereto, and then added compound 4 (30mg, 60.31μmol) and HATU (28mg, 73.64μmol) . The reaction was stirred at room temperature for 3 hours, and then purified by preparative high performance liquid chromatography to obtain the white solid compound PROTAC 10 (9.7 mg, 17.38%).

$^1$H NMR (600MHz, $CD_3OD$) δ8.18(s, 1H), 7.62(s, 1H), 7.38(d, J=7.7Hz, 1H), 7.30-7.23(m, 2H), 7.21(d, J= 2.9Hz, 1H), 6.18(s, 1H), 5.10(dd, J= 12.8, 5.4Hz, 1H), 4.62(s, 3H), 4.12(s, 2H), 3.91(t, J= 5.2Hz, 2H), 3.87-3.80(m,3H), 3.78-3.64(m,3H), 3.63-3.55(m,2H), 3.54-3.47(m,2H), 3.46-3.41(m,1H), 3.26- 3.16(m,1H), 2.94-2.84(m,3H), 2.79-2.68(m,4H), 2.53(s,3H), 2.34(s,3H), 2.15-2.09(m,1H), 1.67-1.46(m,5H).

LC-MS(ESI): $[M+H]^+$=925.35/927.35.

Example 39

Synthesis of PROTAC 11

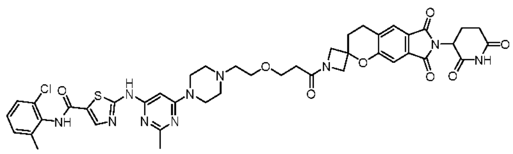

N-(2-chloro-6-methylphenyl)-2-((6-(4-(2-(3-(7'-(2,6-dioxopiperidin-3-yl)- 6',8'-dioxo-4',6',7',8'-tetrahydro-3'H-spiro[azetidine-3,2'-pyrano[2,3- f] isoindol]-1-yl)-3-oxopropoxy)ethyl)piperazin-1-yl)-2-methylpyrimidin-4-yl)amino)thiazole-5-carboxamide

Synthesis scheme:

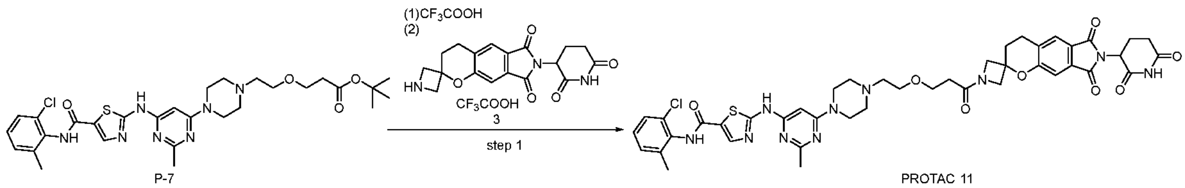


The first step: N-(2-chloro-6-methylphenyl)-2-((6-(4-(2-(3-(7'-(2,6-dioxopiperidine-3 -yl)-6',8'-dioxo-4',6',7',8'-tetrahydro-3'H-spiro[azetidine-3,2'-pyrano[2,3-f]isoindol]-1-yl)-3-oxopropoxy)ethyl)piperazin-1-yl)-2-methylpyrimidin-4-yl)amino)thiazole-5 - formamide (compound PROTAC 11)

To a solution of compound P-7 (35 mg, 56.80 μmol) in dichloromethane (2 mL) at room temperature was added trifluoroacetic acid (0.5 mL) under stirring. The reaction was stirred at room temperature for 3 h. The resulting mixture was concentrated under vacuum, dissolved by adding N, N-dimethylformamide (1 mL), to which diisopropylethylamine (50 mg, 286.85 μmol) was added, followed by adding the compound 3 (30 mg, 63.92 μmol) and HATU (28 mg, 73.64 μmol). The reaction was stirred at room temperature for 3 hours. The white solid compound PROTAC 11 (16 mg, 27.90 %) was obtained by preparative HPLC purification.

$^1$H NMR (600MHz, $CD_3OD$) δ8.18 (s, 1H), 7.64 (d, J = 5.8Hz, 1H), 7.38 (d, J = 7.6Hz, 1H), 7.30-7.22 (m, 3H), 6.15 (d,J=5.9Hz,1H), 5.08(dd,J=12.8,5.4Hz,1H), 4.61(s,2H), 4.38(s,2H), 4.13(q,J=10.9Hz,2H), 3.94-3.88(m,2H), 3.88-3.78(m,3H), 3.71(s,2H), 3.36-3.34(m,1H), 3.50(t,J= 4.4Hz,2H), 3.26-3.20 (m,1H), 3.04(t,J= 12.8Hz,2H), 2.90-2.82(m,1H), 2.78-2.59(m,4H), 2.49(s,4H), 2.35(s,3H), 2.31-2.22(m,2H), 2.17-2.09(m,1H).

LC-MS(ESI): $[M+H]^+$=897.35/899.35.

Example 40

Synthesis of PROTAC 12

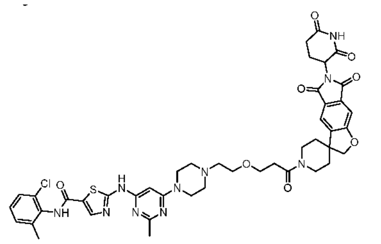

N-(2-chloro-6-methylphenyl)-2-((6-(4-(2-(3-(6-(2,6-dioxopiperidin-3-yl)-5 ,7-dioxo-6,7-dihydro-2H,5H-spiro[furo[2,3-f] isoindole-3,4'-piperidin]-1'-yl)-3- oxopropoxy)ethyl)piperazin-1-yl)-2-methylpyrimidin-4-yl)amino)thiazole-5-carboxamide

Synthesis scheme:

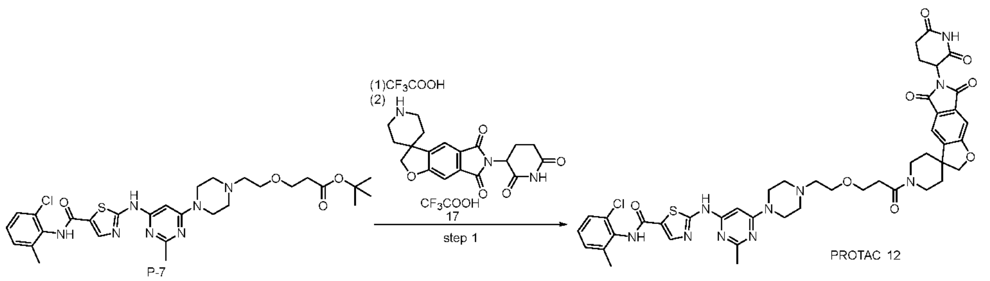


The first step: N-(2-chloro-6-methylphenyl)-2-((6-(4-(2-(3-(6-(2,6-dioxopiperidine-3-yl)-5,7-dioxo-6,7-dihydro-2H,5H-spiro[furo[2,3-f]isoindole-3,4'-piperidinyl]-1'-yl )-3-oxopropoxy)ethyl)piperazin-1-yl)-2-methylpyrimidin-4-yl)amino)thiazole-5-carboxamide (compound PROTAC 12)

To a solution of compound P-7 (35 mg, 56.80 μmol) in dichloromethane (2 mL) was added trifluoroacetic acid (0.5 mL) under stirring at room temperature. The reaction was stirred at room temperature for 3 hours, the resulting mixture was concentrated in vacuo, N, N-dimethylformamide (1 mL) was added to dissolve, diisopropylethylamine (50 mg, 286.85 μmol) was added thereto, and then compound 17 (30mg, 62.06μmol) and HATU (28mg, 73.64μmol) were added. The reaction was stirred at room temperature for 3 hours, and then purified by preparative high performance liquid chromatography to obtain the white solid compound PROTAC 12 (20 mg, 35.36%).

$^1H$ NMR (600MHz, $CD_3OD$) δ8.18(s,1H), 7.69(s,1H), 7.38(d, J=7.4Hz,1H), 7.30-7.21(m,3H), 6.18(s,1H), 5.10(dd, J=12.9, 5.4Hz, 1H), 4.75(s,2H), 4.69-4.56(m,3H), 4.05(d, J=14.2Hz,1H), 3.96-3.91(m,2H ), 3.86(t, J=5.1Hz,2H), 3.75(s,2H), 3.55-3.41(m,4H), 3.31-3.20(m,3H), 2.95-2.82(m,3H), 2.82- 2.68(m,3H), 2.52(s,3H), 2.34(s,3H), 2.17-2.10(m,1H), 2.07-1.99(m,1H), 1.94-1.86(m,3H).

LC-MS(ESI): $[M+H]^+$=911.35/913.25.

Example 41

Synthesis of PROTAC 13

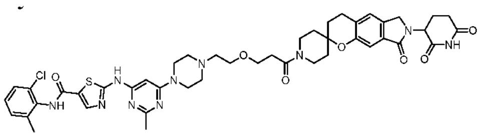

N-(2-chloro-6-methylphenyl)-2-((6-(4-(2-(3-(7'-(2,6-dioxopiperidin-3-yl)- 8'-oxo-4',6',7',8'-tetrahydro-3'H-spiro[piperidine-4,2'-pyrano[2,3-f]isoindole]- 1-yl)-3-oxopropoxy)ethyl)piperazin-1-yl)-2-methylpyrimidin-4-yl)amino)thiazole-5-carboxamide

Synthesis scheme:

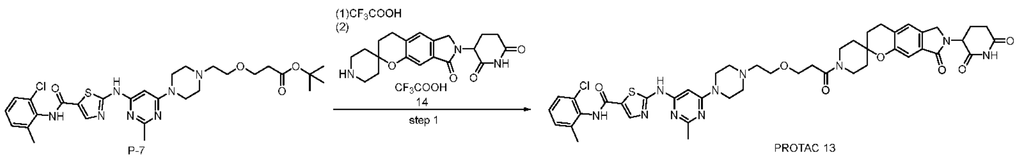


The first step: N-(2-chloro-6-methylphenyl)-2-((6-(4-(2-(3-(7'-(2,6-dioxopiperidine-3 -yl)-8'-oxo-4',6',7',8'-tetrahydro-3'H-spiro[piperidine-4,2'-pyrano[2,3-f]isoindole]-1-yl)-3-oxopropoxy)ethyl)piperazin-1-yl)-2-methylpyrimidin-4-yl) amino)thiazole-5-carboxamide (compound PROTAC 13 )

To a solution of compound P-7 (35 mg, 56.80 μmol) in dichloromethane (2 mL) was added trifluoroacetic acid (0.5 mL) under stirring at room temperature. The reaction was stirred at room temperature for 3 hours, the resulting mixture was concentrated under vacuum, N, N-dimethylformamide (1 mL) was added to dissolve, diisopropylethylamine (50 mg, 286.85 μmol) was added thereto, and then compound 14 (30mg, 62.05μmol) and HATU (28mg, 73.64μmol) were added. The reaction was stirred at room temperature for 3 hours, and then purified by preparative high performance liquid chromatography to obtain the white solid compound PROTAC 13 (14 mg, 24.75%).

$^1$H NMR (600MHz, $CD_3OD$) δ8.18(s, 1H), 7.38(d, J=7.4Hz, 1H), 7.31-7.22(m, 4H), 6.18(s, 1H), 5.14(dd, J= 13.3, 5.1Hz, 1H), 4.61(s, 2H), 4.45-4.32(m, 3H), 3.92(t, J=5.0Hz, 2H), 3.88-3.80(m, 3H), 3.79-3.67(m ,2H), 3.63-3.55(m,1H), 3.52-3.41(m,3H), 3.28-3.17(m,3H), 2.98-2.92(m,2H), 2.92-2.87(m,1H), 2.87 -2.71(m,3H), 2.55-2.42(m,4H), 2.36(s,3H), 2.20-2.14(m,1H), 1.96-1.84(m,4H), 1.77-1.68(m,1H), 1.65-1.55(m,1H), 1.41-1.37(m,1H).

LC-MS(ESI): $[M+H]^+$=911.45/913.25.

Example 42

Synthesis of PROTAC 14

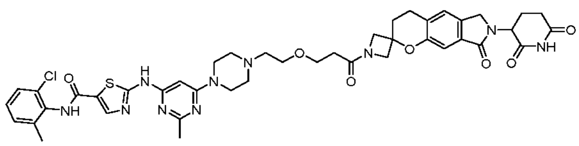

N-(2-chloro-6-methylphenyl)-2-((6-(4-(2-(3-(7'-(2,6-dioxopiperidin-3-yl)- 8'-oxo-4',6',7',8'-tetrahydro-3'H-spiro[azetidine-3,2'-pyrano[2,3-f]isoindol]-1-yl)-3-oxopropoxy)ethyl)piperazin-1-yl)-2-methylpyrimidin-4-yl)amino)thiazole-5-carboxamide

Synthesis scheme:

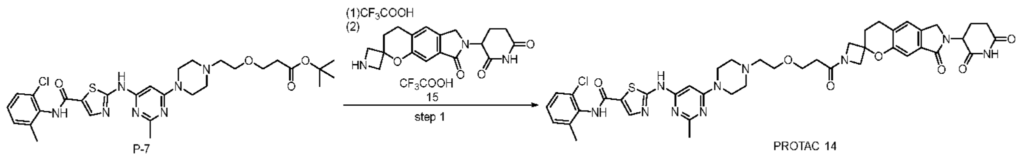


The first step: N-(2-chloro-6-methylphenyl)-2-((6-(4-(2-(3-(7'-(2,6-dioxopiperidine-3 -yl)-8'-oxo-4',6',7',8'-tetrahydro-3'H-spiro[azetidine-3,2'-pyrano[2,3-f]isoindol]-1-yl)-3-oxopropoxy)ethyl)piperazin-1-yl)-2-methylpyrimidin-4-yl)amino)thiazole-5-carboxamide ( Compound PROTAC 14)

To a solution of compound P-7 (35 mg, 56.80 μmol) in dichloromethane (2 mL) was added trifluoroacetic acid (0.5 mL) under stirring at room temperature. The reaction was stirred at room temperature for 3 hours, the resulting mixture was concentrated in vacuo, N,N-dimethylformamide (1 mL) was added to dissolve, and diisopropylethylamine (50 mg, 286.85 μmol) was added thereto, followed by adding the compound 15 (20mg, 43.92μmol) and HATU (20mg, 52.60μmol). The reaction was stirred at room temperature for 3 hours, and the purified by preparative high performance liquid chromatography to obtain the white solid compound PROTAC 14 (8.7 mg, 22.42%).

$^1$H NMR (600MHz, $CD_3OD$) δ8.18(d, J=2.8Hz, 1H), 7.38(d, J=7.6Hz, 1H), 7.32-7.18(m, 4H), 6.15(d, J=27.6Hz ,1H), 5.12(dd, J= 13.3, 5.1Hz, 1H), 4.61(s, 2H), 4.44-4.28(m, 4H), 4.16-4.01(m, 2H), 3.95-3.78(m, 4H), 3.73(s, 2H), 3.50(dd, J= 10.0, 5.0Hz, 2H), 3.46-3.36(m, 2H), 3.30-3.18(m, 2H), 3.06-2.94(m, 2H), 2.94-2.83(m, 2H), 2.83-2.73(m,1H), 2.71-2.60(m, 1H), 2.52-2.40(m, 4H), 2.35(s, 3H), 2.31-2.11(m, 3H ).

LC-MS(ESI): $[M+H]^+$=883.36/885.26.

Example 43

Synthesis of PROTAC 15

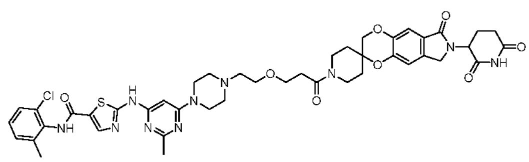

N-(2-chloro-6-methylphenyl)-2-((6-(4-(2-(3-(7 -(2,6-dioxopipendin-3-yl)- 6 -oxo-7,8 -dihydro-3'H,6'H-spiro[pipendine-4,2'-[1,4]dioxino[2,3-f]isoindol]-1-yl)-3-oxopropoxy)ethyl)piperazin-1-yl)-2-methylpyrimidin-4-yl)amino)thiazole-5-carboxamide

Synthesis scheme:

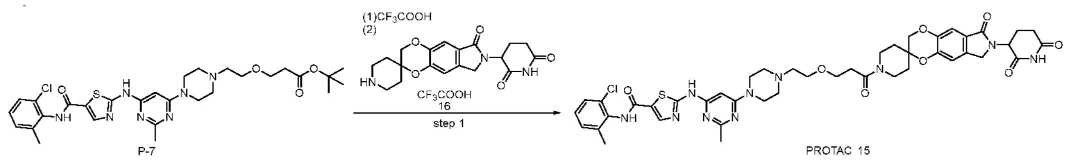


The first step: N-(2-chloro-6-methylphenyl)-2-((6-(4-(2-(3-(7 -(2,6-dioxopipendine-3 -yl)-6'-oxo-7',8'-dihydro-3'H,6'H-spiro[piperidine-4,2'-[1,4]dioxino[2,3 -f]isoindole]-1-yl)-3-oxopropoxy)ethyl)piperazin-1-yl)-2-methylpyrimidin-4-yl)amino)thiazole-5-carboxamide (Compound PROTAC 15)

To a solution of compound P-7 (35 mg, 56.80 μmol) in dichloromethane (2 mL) at room temperature was added trifluoroacetic acid (0.5 mL) under stirring. The reaction was stirred at room temperature for 3 h. The resulting mixture was concentrated under vacuum, dissolved by adding N, N-dimethylformamide (1 mL) to which diisopropylethylamine (50 mg, 286.85 μmol) was added, followed by adding the compound 16 (30 mg, 61.80 μmol) and HATU (28 mg, 73.64 μmol). The reaction was stirred at room temperature for 3 hours. The white solid compound PROTAC 15 (24 mg, 42.51 %) was

obtained by preparative HPLC purification.

$^1H$ NMR (600MHz, $CD_3OD$) δ8.18(s, 1H), 7.38(d, J=7.4Hz, 1H), 7.29(s, 1H), 7.27(d, J=5.2Hz, 1H), 7.25(d, J=7.7Hz,1H), 7.10(s, 1H), 6.19(s,1H), 5.11(dt, J=13.2,5.2Hz,1H), 4.63(s, 2H), 4.43-4.31(m, 3H), 4.11-4.02(m, 2H),3.95-3.80(m, 5H), 3.74(s, 2H), 3.61-3.53(m, 2H), 3.53-3.47(m, 2H), 3.44(s, 1H), 3.29-3.17(m,3H), 2.95-2.85(m, 1H), 2.85-2.74(m, 3H), 2.53(s, 3H), 2.50-2.41(m, 1H), 2.34(s, 3H), 2.19-2.13(m, 1H), 1.97-1.83(m, 2H), 1.83-1.75(m, 1H), 1.74-1.64(m, 1H).

LC-MS(ESI): $[M+H]^+$=913.45/915.35.

Example 44

Synthesis of PROTAC 16

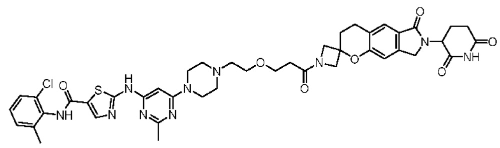

N-(2-chloro-6-methylphenyl)-2-((6-(4-(2-(3-(7'-(2,6-dioxopiperidin-3-yl)- 6'-oxo-4',6',7',8'-tetrahydro-3'H-spiro[azetidine-3,2'-pyrano[2,3-f] isoindole]-1-yl)-3-oxopropoxy)ethyl)piperazin-1-yl)-2-methylpyrimidin-4-yl)amino)thiazole-5-carboxamide

Synthesis scheme:

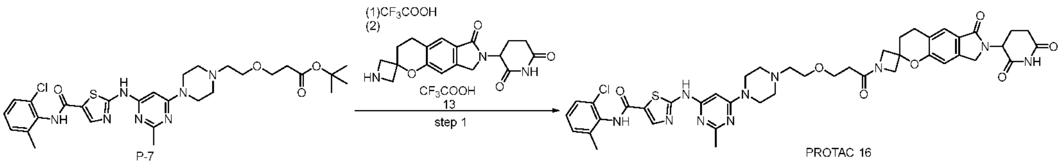


The first step: N-(2-chloro-6-methylphenyl)-2-((6-(4-(2-(3-(7'-(2,6-dioxopiperidine-3 -yl)-6'-oxo-4',6',7',8'-tetrahydro-3'H-spiro[azetidine-3,2'-pyrano[2,3- f] isoindol]-1-yl)-3-oxopropoxy)ethyl)piperazin-1-yl)-2-methylpyrimidin-4-yl)atnino)thiazole-5-carboxamide ( Compound PROTAC 16)

To a solution of compound P-7 (35 mg, 56.80 μmol) in dichloromethane (2 mL) was added trifluoroacetic acid (0.5 mL) under stirring at room temperature. The reaction was stirred at room temperature for 3 hours, the resulting mixture was concentrated in vacuo, N, N-dimethylformamide (1 mL) was added to dissolve, diisopropylethylamine (50 mg, 286.85 μmol) was added thereto, and then compound 13 (20mg, 43.92μmol) and HATU (20mg, 52.60μmol) were added. The reaction was stirred at room temperature for 3 hours, and the purified by preparative high performance liquid chromatography to obtain the white solid compound PROTAC 16 (9.0 mg, 23.20%).

$^1H$ NMR (600MHz, $CD_3OD$) δ8.18(s, 1H), 7.55(s, 1H), 7.38(d, J=7.4Hz, 1H), 7.30-7.23(m, 2H), 6.99(d, J= 19.3Hz, 1H), 6.16(d, J=9.1Hz, 1H), 5.14-5.05(m, 1H), 4.61(s, 2H), 4.43-4.29(m, 4H), 4.15-4.04(m, 2H), 3.93-3.86(m,2H), 3.86-3.77(m,2H), 3.72(s,2H), 3.53-3.46(m,2H), 3.46-3.39(m,2H), 3.27-3.17(m ,2H), 3.03-2.95(m,2H), 2.95-2.85(m,2H), 2.81-2.74(m,1H), 2.66-2.57(m,1H), 2.56-2.46(m,3H), 2.46 -2.37(m,1H), 2.35(s,3H), 2.24(t,J=6.4Hz,2H), 2.19-2.11(m,1H).

LC-MS(ESI): $[M+H]^+$=883.36/885.36.

Example 45

Synthesis of PROTAC 17

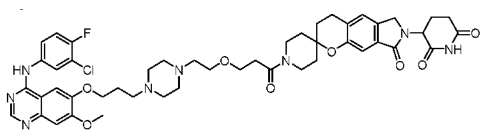

3-(1-(3-(2-(4-(3-((4-((3-chloro-4-fluorophenyl)amino)-7-methoxyquinazolin-6-yl)oxy) propyl) piperazin-1-yl) ethoxy) propionyl) -8'-oxo-3',4',6',8'-tetrahydro-7'H-spiro[piperidine- 4,2'-pyrano[2,3-f]isoindole]-7'-yl)piperidine-2,6-dione

Synthesis scheme:

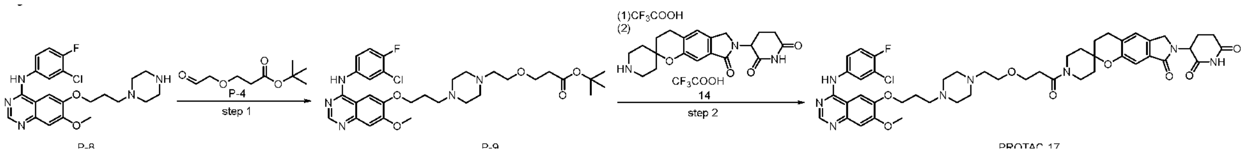


The first step: tert-butyl 3-(2-(4-(3-((4-((3-chloro-4-fluorophenyl)amino)-7-methoxyquinazolin-6-yl)oxy) propyl) piperazin-1-yl) ethoxy) propionate (compound P-9)

To a solution of compound P-8 (100 mg, 224.25 μmol) and compound P-4 (85 mg, 451.59 μmol) in methanol (4 mL) was added acetic acid (1 mL) and tetraisopropyl titanate (1 mL), and finally sodium cyanoborohydride (46 mg, 732.02 μmol) was added with stirring at room temperature. The reaction was stirred at room temperature for 14 hours. The resulting mixture was concentrated under vacuum and purified by preparative high performance liquid chromatography to obtain the light yellow oily compound P-9 (82 mg, 59.15 %).

$^1$H NMR (600MHz, $CD_3OD$) δ8.76(s, 1H), 8.00(s, 1H), 7.96(dd, J=6.6, 2.6Hz, 1H), 7.68(ddd, J=8.9, 4.1, 2.7Hz, 1H), 7.39(t, J=8.9Hz, 1H), 7.29(s, 1H), 4.38(t, J=5.6Hz, 2H), 4.11(s, 3H), 3.85-3.80(m, 2H), 3.75(t,J=5.9Hz,2H), 3.72(t,J=6.2Hz,1H), 3.68-3.64(m, 1H), 3.56-3.48(m, 4H), 3.43-3.36(m, 2H), 3.35(d,J=4.9Hz, 1H), 3.25(t,J=7.1Hz, 2H), 2.56(t,J=5.9Hz, 2H), 2.54-2.49(m, 1H), 2.38-2.31(m, 2H), 1.48(s, 9H).

LC-MS (ESI): $[M+H]^+$=618.02.

The second step: 3-(1-(3-(2-(4-(3-((4-((3-chloro-4-fluorophenyl)atnino)-7-methoxyquinazoline-6 - yl)oxy)propyl)piperazin-1-yl)ethoxy)propionyl)-8'-oxo-3',4',6',8'-tetrahydro-7'H-spiro [piperidine-4,2'-pyrano[2,3-f]isoindole]-7'-yl)piperidine-2,6-dione (compound PROTAC 17)

To a solution of compound P-9 (25 mg, 40.44 μmol) in dichloromethane (2 mL) was added trifluoroacetic acid (0.5 mL) under stirring at room temperature. The reaction was stirred at room temperature for 3 h. The resulting mixture was concentrated under vacuum, dissolved by adding N, N-dimethylformamide (1 mL) to which diisopropylethylamine (60 mg, 464.22 μmol) was added, followed by adding the compound 14 (20 mg, 41.37 μmol) and HATU (20 mg, 52.60 μmol). The reaction was stirred at room temperature for 1.5 hours. The white solid compound PROTAC 17 (6.0 mg, 19.06 %) was obtained by preparative HPLC purification.

$^1$H NMR (600MHz, $CD_3OD$) δ8.75 (d, J = 0.9Hz, 1H), 7.99 (d, J = 4.0Hz, 1H), 7.95 (dd, J = 6.6, 2.6Hz, 1H), 7.70-7.65 (m,1H), 7.39(t, J=8.9Hz, 1H), 7.32(s,1H), 7.28-7.24(m, 1H), 7.22(s, 1H), 5.13(ddd, J=21.6, 13.3, 5.1Hz,1H), 4.45-4.33(m, 5H), 4.07(d, J=20.7Hz, 2H), 3.90-3.77(m, 5H), 3.71-3.64(m, 1H), 3.62-3.53(m, 2H), 3.48-3.36(m, 5H), 3.27-3.14(m, 4H), 3.05(s, 2H), 2.97(t, J=6.6Hz, 2H), 2.93-2.87(m, 2H), 2.86-2.76(m, 2H), 2.76-2.66(m, 1H), 2.56-2.44(m, 1H), 2.31-2.24(m,2 H), 2.20-2.14(m, 1H), 1.98-1.84(m, 4H), 1.79-1.68(m, 1H), 1.68-1.57(m,1H).

LC-MS(ESI): $[M+H]^+$=913.35/915.35.

Example 46

Synthesis of PROTAC 18

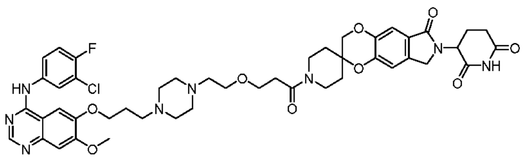

3-(1-(3-(2-(4-(3-((4-((3-chloro-4-fluorophenyl)amino)-7-methoxyquinazolin-6-yl)oxy)propyl)piperazin-1-yl)ethoxy)propionyl)-6'-oxo-6',8'-dihydro-3'H,7'H-spiro[piperidine-4, 2'-[1,4]dioxino[2,3-f]isoindol]-7'-yl)piperidine-2,6-dione

Synthesis scheme:

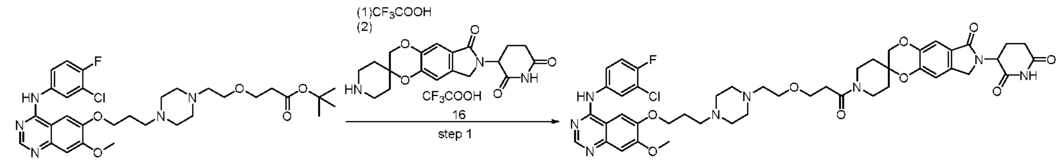


P-9 PROTAC 18

The first step: 3-(1-(3-(2-(4-(3-((4-((3-chloro-4-fluorophenyl)amino)-7-methoxyquinazoline-6 - yl)oxy)propyl)piperazin-1-yl)ethoxy)propionyl)-6'-oxo-6',8'-dihydro-3'H,7'H-spiro[piperidine-4,2'-[1,4]dioxino[2,3-f]isoindol]-7'-yl)piperidine-2,6-dione (compound PROTAC 18)

To a solution of compound P-9 (25 mg, 40.44 μmol) in dichloromethane (2 mL) was added trifluoroacetic acid (0.5 mL) under stirring at room temperature. The reaction was stirred at room temperature for 3 h. The resulting mixture was concentrated under vacuum, dissolved by adding N,N-dimethylformamide (1 mL), to which diisopropylethylamine (60 mg, 464.22 μmol) was added, followed by adding the compound 16 (20 mg, 41.20 μmol) and HATU (20 mg, 52.60 μmol). The reaction solution was stirred at room temperature for 1.5 hours. The white solid compound PROTAC 18 (5.8 mg, 18.48 %) was obtained by preparative HPLC purification.

$^1H$ NMR (600MHz, $CD_3OD$) δ8.75 (d, J = 2.7Hz, 1H), 7.98 (d, J = 6.0Hz, 1H), 7.95 (dd, J = 6.6, 2.6Hz, 1H), 7.70-7.65 (m, 1H), 7.39(t, J=8.9Hz, 1H), 7.30-7.23(m, 2H), 7.13(s, 1H), 5.12(ddd, J=23.3, 13.4, 5.1Hz, 1H), 4.44-4.31(m, 5H), 4.12-4.00(m, 5H), 3.92-3.78(m, 5H), 3.64-3.54(m, 2H), 3.53-3.36(m, 5H), 3.30-3.22(m, 3H), 3.20-3.13(m, 1H), 3.06(s, 2H), 2.96-2.86(m, 2H), 2.83-2.75(m, 3H), 2.53-2.42(m, 1H), 2.28(s , 2H), 2.21-2.12(m, 1H), 1.96-1.77(m, 3H), 1.75-1.66(m, 1H).

LC-MS(ESI): $[M+H]^+$=915.45/917.35.

Example 47

Synthesis of PROTAC 19

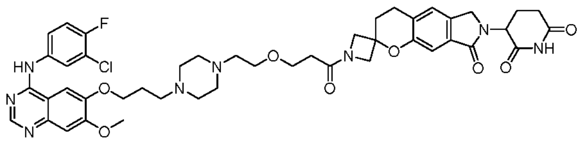

3-(1-(3-(2-(4-(3-((4-((3-chloro-4-fluorophenyl)amino)-7-methoxyquinazolin-6-yl)oxy)propyl)piperazin-1-yl)ethoxy)propionyl)-8'-oxo-3',4',6',8'-tetrahydro-7'H-spiro[azetidine-3,2'-pyrano[2,3-f]isoindole]-7'-yl)piperidine-2,6-dione

Synthetic scheme

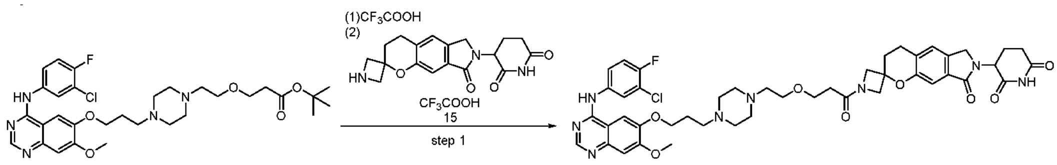


P-9

PROTAC 19

The first step: 3-(1-(3-(2-(4-(3-((4-((3-chloro-4-fluorophenyl)amino)-7-methoxyquinazoline-6 - yl)oxy)propyl)piperazin-1-yl)ethoxy)propionyl)-8'-oxo-3',4',6',8'-tetrahydro-7'H-spiro [azetidine-3,2'-pyrano[2,3-f]isoindol]-7'-yl)piperidine-2,6-dione (compound PROTAC 19)

To a solution of compound P-9 (25 mg, 40.44 μmol) in dichloromethane (2 mL) was added trifluoroacetic acid (0.5 mL) under stirring at room temperature. The reaction was stirred at room temperature for 3 h. The resulting mixture was concentrated under vacuum, dissolved by adding N, N-dimethylformamide (1 mL) to which diisopropylethylamine (60 mg, 464.22 μmol) was added, followed by adding the compound 15 (20 mg, 43.92 μmol) and HATU (20 mg, 52.60 μmol). The reaction was stirred at room temperature for 1.5 hours. The white solid compound PROTAC 19 (7.3 mg, 22.53 %) was obtained by preparative HPLC purification.

$^1$H NMR (600MHz, $CD_3OD$) δ8.75 (d, J = 2.2Hz, 1H), 7.98 (d, J = 7.6Hz, 1H), 7.96 (dd, J = 6.6, 2.6Hz, 1H), 7.70-7.66 (m, 1H), 7.54(d, J=11.0Hz, 1H), 7.39(t, J=8.9Hz,1H), 7.26(d, J=5.4Hz, 1H), 7.06(d, J=2.2Hz, 1H), 5.12(ddd, J= 13.3, 10.7, 5.1Hz, 1H), 4.46-4.38(m, 2H), 4.37-4.3 1(m, 4H), 4.13-4.06(m, 5H), 3.85-3.76 (m, 4H), 3.47-3.36(m, 4H), 3.32-3.29(m, 1H), 3.25-3.10(m, 4H), 3.05(s, 2H), 3.00-2.95(m, 2H), 2.94 -2.86(m, 1H), 2.81-2.75(m, 1H), 2.60-2.54(m, 1H), 2.54-2.42(m, 3H), 2.30-2.19(m, 4H), 2.19-2.12(m, 1H).

LC-MS(ESI): $[M+H]^+$=885.46/887.36.

Example 48

Synthesis of PROTAC 20

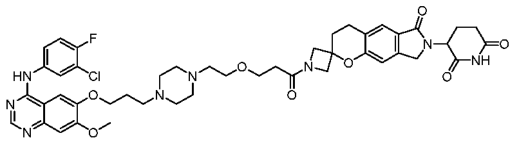

3-(1-(3-(2-(4-(3-((4-((3-chloro-4-fluorophenyl)amino)-7-methoxyquinazolin-6-yl)oxy yl)propyl)piperazin-1-yl)ethoxy) propionyl)-6'-oxo-3',4',6',8'-tetrahydro-7'H-spiro[azetidine-3,2'-pyrano[2,3-f]isoindole]-7'-yl)piperidine-2,6-dione

Synthesis scheme:

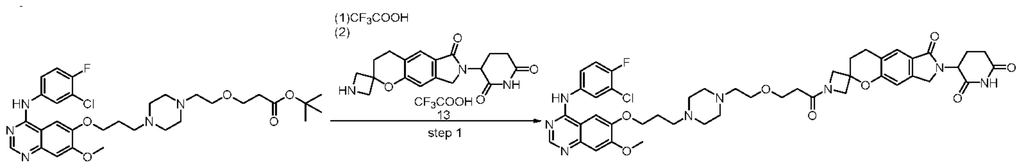


P-9

PROTAC 20

The first step: 3-(1-(3-(2-(4-(3-((4-((3-chloro-4-fluorophenyl)amino)-7-methoxyquinazoline-6 - yl)oxy)propyl)piperazin-1-yl)ethoxy)propionyl)-6'-oxo-3',4',6',8'-tetrahydro-7'H-spiro [azetidine-3,2'-pyrano[2,3-f]isoindol]-7'-yl)piperidine-2,6-dione (compound PROTAC 20)

To a solution of compound P-9 (25 mg, 40.44 μmol) in dichloromethane (2 mL) was added trifluoroacetic acid (0.5 mL) under stirring at room temperature. The reaction was stirred at room temperature for 3 h. The resulting mixture was concentrated under vacuum, dissolved by adding N,N-dimethylformamide (1 mL) to which diisopropylethylamine (60 mg,

464.22 μmol) was added, followed by adding the compound 13 (20 mg, 43.92 μmol) and HATU (20 mg, 52.60 μmol). The reaction was stirred at room temperature for 1.5 hours, and purified by preparative HPLC to obtain the white solid compound PROTAC 20 (8.0 mg, 24.69%)

$^1$H NMR (600MHz, $CD_3OD$) δ8.75 (d, J = 1.9Hz, 1H), 7.98 (d, J = 4.5Hz, 1H), 7.95 (dd, J = 6.6, 2.6Hz, 1H), 7.70-7.65 (m,1H), 7.39(t,J=8.9Hz,1H), 7.33(s,1H), 7.25(s,1H), 7.23(s,1H), 5.13-5.05(m,1H), 4.45- 4.28(m,6H), 4. 13-4.03(m,5H), 3.87-3.72(m,4H), 3.47-3.35(m,3H), 3.31-3.26(m,2H), 3.22-3.12(m,3H), 3.06(s,2H), 3.03-2.98(m,2H), 2.90-2.82(m,1H), 2.81-2.74(m,1H), 2.65-2.58(m,1H), 2.51-2.40(m ,3H), 2.30-2.18(m,5H), 2.17-2.11(m,1H).

LC-MS(ESI): [M+H]+=885.36/887.36.

Example 49

Synthesis of PROTAC 21

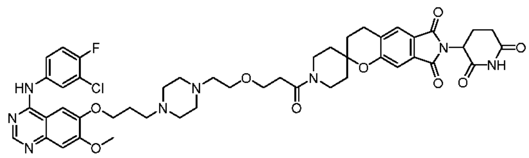

1-(3-(2-(4-(3-((4-((3-chloro-4-fluorophenyl)amino)-7-methoxyquinazolin-6-yl)oxy) propyl)piperazin-1-yl)ethoxy)propionyl)-7'-(2,6-dioxopiperidin-3-yl)-3',4'-dihydro-6'H-spiro [piperidine-4,2'-pyrano[2,3-f]isoindole]-6',8'(7'H)-dione

Synthesis scheme:

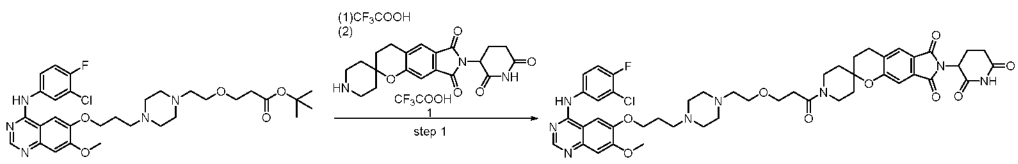


The first step: 1-(3-(2-(4-(3-((4-((3-chloro-4-fluorophenyl)amino)-7-methoxyquinazolin-6-yl) oxy)propyl)piperazin-1-yl) ethoxy)propionyl)-7'-(2,6-dioxopiperidin-3-yl)-3',4'-dihydro-6 'H-spiro[piperidine-4,2'-pyrano[2,3-f]isoindole]-6',8'(7'H)-dione (compound PROTAC21)

To a solution of compound P-9 (40 mg, 64.71 μmol) in dichloromethane (2 mL) was added trifluoroacetic acid (0.5 mL) under stirring at room temperature. The reaction was stirred at room temperature for 3 h. The resulting mixture was concentrated under vacuum, dissolved by adding N,N-dimethylformamide (1 mL) to which diisopropylethylamine (60 mg, 464.22 μmol) was added, followed by adding the compound 1 (40 mg, 80.41 μmol) and HATU (40 mg, 105.20 μmol). The reaction was stirred at room temperature for 1.5 hours. The white solid compound PROTAC 21 (14 mg, 18.77 %) was obtained by preparative HPLC purification.

$^1$H NMR (600MHz, DMSO-$d_6$) δ8.73(s, 1H), 7.96(s, 1H), 7.93(d, J=6.5Hz, 1H), 7.70-7.61(m, 2H), 7.37(t, J=8.9Hz, 1H), 7.28(s, 1H), 7.24(s, 1H), 5.08(dd, J= 12.8, 5.3Hz, 1H), 4.39(d, J= 12.7Hz, 1H), 4.33( t,J=5.2Hz,2H), 4.06(s,3H), 3.89-3.75(m,5H), 3.59-3.53(m,1H), 3.46-3.34(m,6H), 3.26-3.18(m, 2H), 3.17-3.06(m,2H), 3.04-2.95(m,4H), 2.89-2.65(m,6H), 2.30-2.20(m,2H), 2.14-2.05(m,1H), 2.00-1.86(m,4H), 1.79-1.71(m,1H), 1.69-1.61(m,1H).

LC-MS (ESI): $[M+H]^+$=927.60.

Example 50

Synthesis of PROTAC 22

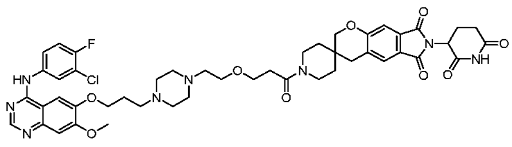

1-(3-(2-(4-(3-((4-((3-chloro-4-fluorophenyl)amino)-7-methoxyquinazolin-6-yl)oxy) propyl)piperazin-1-yl)ethoxy)propionyl)-7'-(2,6-dioxopiperidin-3-yl)-2'H-spiro[piperidine-4,3'- pyrano[2,3-f]isoindole]-6',8'(4'H,7'H)-dione

Synthesis scheme:

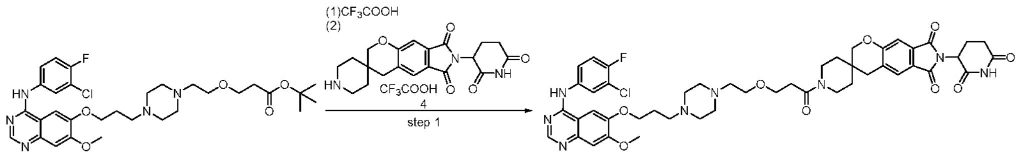


P-9

PROTAC 22

The first step: 1-(3-(2-(4-(3-((4-((3-chloro-4-fluorophenyl)amino)-7-methoxyquinazolin-6-yl) oxy)propyl)piperazin-1-yl)ethoxy)propionyl)-7'-(2,6-dioxopiperidin-3-yl)-2'H-spiro[piperidine-4 ,3'-pyrano[2,3-f]isoindole]-6',8'(4'H,7'H)-dione (compound PROTAC 22)

To a solution of compound P-9 (40 mg, 64.71 μmol) in dichloromethane (2 mL) was added trifluoroacetic acid (0.5 mL) under stirring at room temperature. The reaction was stirred at room temperature for 3 h. The resulting mixture was concentrated under vacuum, dissolved by adding N, N-dimethylformamide (1 mL) to which diisopropylethylamine (60 mg, 464.22 μmol) was added, followed by adding the compound 4 (40 mg, 80.41 μmol) and HATU (40 mg, 105.20 μmol). The reaction was stirred at room temperature for 1.5 hours. The white solid compound PROTAC 22 (16 mg, 21.45 %) was obtained by preparative HPLC purification.

$^1$H NMR (600MHz, DMSO-$d_6$) δ8.74(s, 1H), 7.98-7.92(m, 2H), 7.69-7.61(m, 2H), 7.38(t, J=8.9Hz, 1H), 7.24( s,1H), 7.20(d,J=6.2Hz,1H), 5.11-5.04(m,1H), 4.35-4.29(m,2H), 4.16-4.09(m,2H), 4.05(d,J= 4.3Hz, 3H), 3.89-3.81(m, 3H), 3.78(t, J=5.2Hz, 2H), 3.70-3.55(m, 4H), 3.47-3.35(m, 4H), 3.18-3.07(m ,2H), 3.03-2.97(m,2H), 2.93(s,2H), 2.90-2.80(m,2H), 2.77-2.65(m,5H), 2.28-2.20(m,2H), 2.13-2.06 (m,1H), 1.66-1.46(m,5H).

LC-MS (ESI): $[M+H]^+$=927.55.

Example 51

Synthesis of PROTAC 23

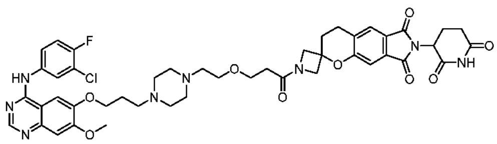

1-(3-(2-(4-(3-((4-((3-chloro-4-fluorophenyl)amino)-7-methoxyquinazolin-6-yl)oxy)propyl)piperazin-1-yl)ethoxy)propionyl)-7'-(2,6-dioxopiperidin-3-yl)-3',4'-dihydro-6'H-spiro [azetidine-3,2'-pyrano[2,3-f]isoindole]-6',8'(7'H)-dione

Synthesis scheme:

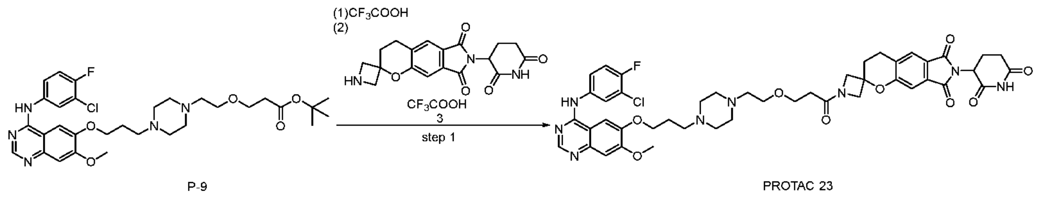


The first step: 1-(3-(2-(4-(3-((4-((3-chloro-4-fluorophenyl)amino)-7-methoxyquinazolin-6-yl) oxy)propyl)piperazin-1-yl) ethoxy)propionyl)-7'-(2,6-dioxopiperidin-3-yl)-3',4'-dihydro-6 'H-spiro[azetidine-3,2'-pyrano[2,3-f]isoindole]-6',8'(7'H)-dione (compound PROTAC 23)

To a solution of compound P-9 (40 mg, 64.71 μmol) in dichloromethane (2 mL) was added trifluoroacetic acid (0.5 mL) under stirring at room temperature. The reaction was stirred at room temperature for 3 h. The resulting mixture was concentrated under vacuum, dissolved by adding N,N-dimethylformamide (1 mL) to which diisopropylethylamine (60 mg, 464.22 μmol) was added, followed by adding the compound 3 (40 mg, 85.22 μmol) and HATU (40 mg, 105.20 μmol). The reaction was stirred at room temperature for 1.5 hours. The white solid compound PROTAC 23 (5.4 mg, 7.05 %) was obtained by preparative HPLC purification.

$^1$H NMR (600MHz, $CD_3OD$) δ8.75(s, 1H), 7.97(s, 1H), 7.96(dd, J=6.6, 2.5Hz, 1H), 7.70-7.65(m, 2H), 7.39(t, J=8.9Hz, 1H), 7.32(d, J= 1.9Hz, 1H), 7.26(d, J=8.9Hz, 1H), 5.09(dd, J= 12.8, 5.4Hz, 1H), 4.38-4.33( m, 3H), 4. 16-4. 10(m,2H), 4.09(d,J=2.0Hz, 3H), 3.86-3.74(m, 4H), 3.42-3.35(m, 2H), 3.30(s, 3H), 3.14(s, 4H), 3.07-2.98(m, 4H), 2.89-2.81(m, 2H), 2.77-2.65(m, 3H), 2.62-2.56(m, 1H), 2.51-2.44(m, 1H), 2.32-2.22(m, 4H), 2.13-2.07(m, 1H).

LC-MS (ESI): $[M+H]^+$=899.35.

Example 52

Synthesis of PROTAC 24

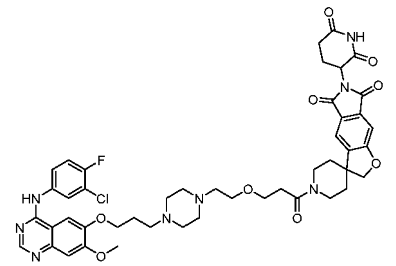

1'-(3-(2-(4-(3-((4-((3-chloro-4-fluorophenyl)amino)-7-methoxyquinazolin-6-yl)oxy) propyl)piperazin-1-yl)ethoxy)propionyl)-6-(2,6-dioxopiperidin-3-yl)-6-hydro-2H,5H-spiro[furo[2 ,3-f]isoindole-3,4'-piperidine]-5,7-dione

Synthesis scheme:

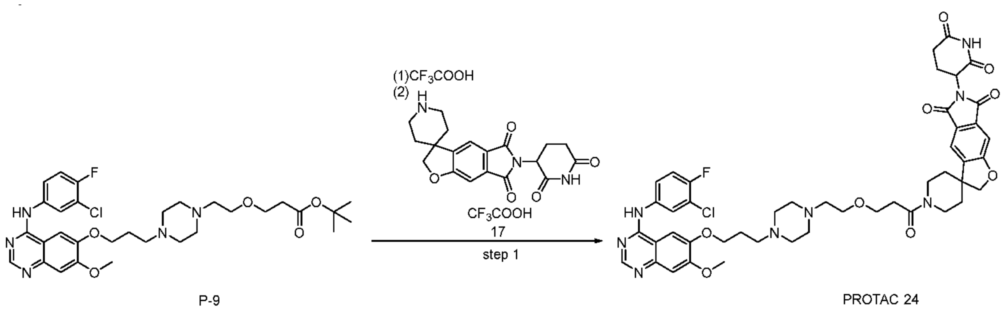

The first step: 1'-(3-(2-(4-(3-((4-((3-chloro-4-fluorophenyl)amino)-7-methoxyquinazolin-6-yl )oxy)propyl)piperazin-1-yl)ethoxy)propionyl)-6-(2,6-dioxopiperidin-3-yl)-6-hydro-2H,5H-spiro[furo[2,3-f]isoindole-3,4'-piperidine]-5,7-dione (compound PROTAC 24)

To a solution of compound P-9 (40 mg, 64.71 μmol) in dichloromethane (2 mL) was added trifluoroacetic acid (0.5 mL) under stirring at room temperature. The reaction was stirred at room temperature for 3 h. The resulting mixture was concentrated under vacuum, dissolved by adding N,N-dimethylformamide (1 mL) to which diisopropylethylamine (60 mg, 464.22 μmol) was added, followed by adding the compound 17 (40 mg, 82.75 μmol) and HATU (40 mg, 105.20 μmol). The reaction was stirred at room temperature for 1.5 hours. The white solid compound PROTAC 24 (20 mg, 26.46 %) was obtained by preparative HPLC purification.

$^1H$ NMR (600MHz, DMSO-$d_6$) δ8.73(s,1H), 7.95(s,1H), 7.93(dd, J=6.6,2.5Hz, 1H), 7.69(s, 1H), 7.68-7.63( m,1H), 7.37(t, J=8.9Hz, 1H), 7.24(s,1H), 7.21(d, J=2.5Hz, 1H), 5.11-5.04(m, 1H),4.74(s, 2H), 4.61(d, J=13.3Hz, 1H), 4.32(t, J=5.5Hz, 2H), 4.07(s, 3H), 4.04(d, J=14.0Hz, 1H), 3.89-3.79(m, 4H),3.52-3.35(m,6H),3.28(d, J=13.8Hz, 1H), 3.19(s, 2H), 3.03(s,2H), 2.94-2.80(m, 4H), 2.78- 2.65(m, 4H), 2.29-2.22(m, 2H), 2.14-2.07(m, 1H), 2.07-1.98(m, 1H), 1.94-1.84(m, 3H).

LC-MS (ESI): $[M+H]^+$=913.55.

Example 52

Synthesis of PROTAC 24

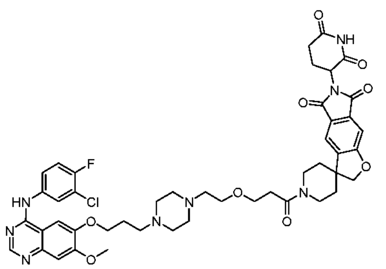

1'-(3-(2-(4-(3-((4-((3-chloro-4-fluorophenyl)amino)-7-methoxyquinazolin-6-yl)oxy)propyl)piperazin-1-yl)ethoxy)propionyl)-6-(2,6-dioxopiperidin-3-yl)-6-hydrogen-2H,SH-spiro[furo[2 ,3-f]isoindole-3,4'-piperidine]-5,7-dione

Synthesis scheme:

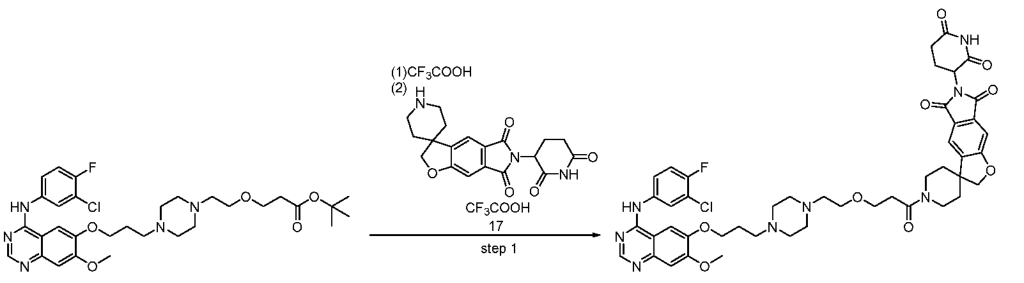


P-9

PROTAC 24

The first step: 1'-(3-(2-(4-(3-((4-((3-chloro-4-fluorophenyl)amino)-7-methoxyquinazolin-6-yl )oxy)propyl)piperazin-1-yl)ethoxy)propionyl)-6-(2,6-dioxopiperidin-3-yl)-6-hydrogen-2H,5H-spiro[furo[2,3-f]isoindole-3,4'-piperidine]-5,7-dione (compound PROTAC 24)

To a solution of compound P-9 (40 mg, 64.71 μmol) in dichloromethane (2 mL) was added trifluoroacetic acid (0.5 mL) with stirring at room temperature. The reaction was stirred at room temperature for 3 hours. The resulting mixture was concentrated in vacuo, and dissolved by adding N,N-dimethylformamide (1 mL). Diisopropylethylamine (60 mg, 464.22

μmol) was added thereto, and then compound 17 and HATU (40mg, 105.20μmol) were added (40mg, 82.75μmol). The reaction was stirred at room temperature for 1.5 hours. Purified by preparative high performance liquid chromatography to obtain white solid compound PROTAC 24 (20 mg, 26.46%).

$^1$H NMR (600MHz, DMSO-$d_6$) δ8.73(s,1H), 7.95(s,1H), 7.93(dd,J=6.6, 2.5Hz, 1H), 7.69(s, 1H), 7.68-7.63(m, 1H), 7.37(t, *J*=8.9Hz,1H), 7.24(s, 1H), 7.21(d,J=2.5Hz, 1H), 5.11-5.04(m, 1H), 4.74(s, 2H), 4.61(d, J=13.3Hz, 1H), 4.32(t, J=5.5Hz, 2H), 4.07(s, 3H), 4.04(d, J=14.0Hz, 1H), 3.89-3.79(m, 4H), 3.52-3.35(m, 6H), 3.28(d,J= 13.8Hz, 1H), 3.19(s, 2H), 3.03(s, 2H), 2.94-2.80(m, 4H), 2.78- 2.65(m, 4H), 2.29-2.22(m, 2H), 2.14-2.07(m, 1H), 2.07-1.98(m, 1H), 1.94-1.84(m, 3H).

LC-MS (ESI): $[M+H]^+$=913.55.

Example 53

Synthesis of PROTAC 25

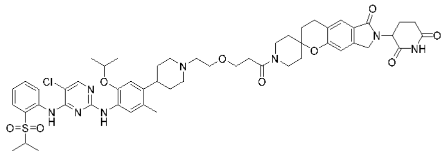

3-(1-(3-(2-(4-(4-((5-chloro-4-((2-(isopropylsulfonyl)phenyl)amino)pyrimidin-2-yl)amino)- 5-isopropoxy-2-methylphenyl) piperidin-1-yl)ethoxy)propionyl)-6'-oxo-3',4',6',8'-tetrahydro- 7'H-spiro[piperidine-4,2'-pyrano[2,3-f]isoindole]-7'-yl)piperidine-2,6-dione

Snthesis scheme:

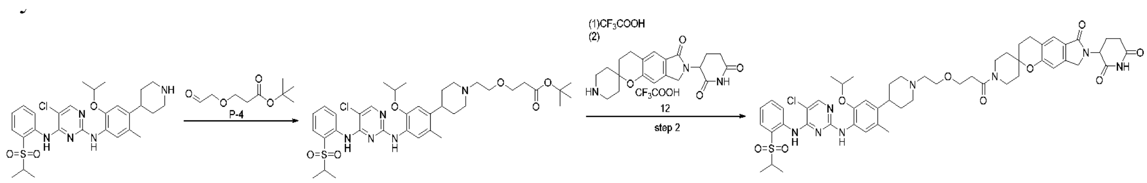


The first step: 3-(2-(4-(4-((5-chloro-4-((2-(isopropylsulfonyl)phenyl)amino)pyrimidin-2-yl)amino)-5-isopropoxy-2-methylphenyl)piperidin-1-yl)ethoxy)propanoic acid tert-butyl ester (compound P-11)

At room temperature, to a solution of compound P-10 (100 mg, 179.17 μmol) and compound P-4 (68 mg, 361.27 μmol) in methanol (5 mL) was added acetic acid (1 mL) and tetraisopropyl titanate (1 mL) , and finally sodium cyanoborohydride (34 mg, 541.04 μmol) was added with further stirring. The reaction was stirred at room temperature for 14 hours. The resulting mixture was concentrated in vacuo and purified by high performance liquid chromatography to obtain light yellow oily compound P-11 (84 mg, 64.19%).

$^1$H NMR (600MHz, $CD_3OD$) δ8.41(d, *J*=8.2Hz, 1H), 8.22(s, 1H), 7.98(dd, *J*=8.0, 1.4Hz, 1H), 7.76-7.68(m, 2H), 7.46(t, *J*=7.7Hz, 1H), 6.84(s, 1H), 4.60(dt, *J*=12.1, 6.0Hz, 1H), 3.90-3.83(m, 2H), 3.81-3.74(m, 3H), 3.44-3.40(m, 1H), 3.40-3.34(m, 1H), 3.27-3.20(m, 2H), 3.17-3.10(m, 1H), 2.60(t, *J* = 5.8Hz, 2H), 2.20(s, 3H), 2.09-1.95(m, 5H), 1.50(s, 9H), 1.49-1.47(m, 1H), 1.35(d, *J* = 6.1Hz, 6H), 1.28(d, *J* = 6.8Hz, 6H).

LC-MS (ESI): $[M+H]^+$=730.15.

The second step: 3-(1-(3-(2-(4-(4-((5-chloro-4-((2-(isopropylsulfonyl)phenyl)amino)pyrimidin-2-yl )atnino)-5-isopropoxy-2-methylphenyl)piperidin-1-yl)ethoxy)propionyl)-6'-oxo-3',4',6',8' -tetrahydro-7'H-spiro[piperidine-4,2'-pyrano [2,3-f]isoindol]-7'-yl)piperidine-2,6-dione (compound PROTAC 25)

At room temperature, to a solution of compound P-11 (25 mg, 34.23 μmol) in dichloromethane (2 mL) was added trifluoroacetic acid (0.5 mL) with stirring. The reaction was stirred at room temperature for 3 hours. The resulting mixture

was concentrated in vacuo, and dissolved by adding N,N-dimethylformamide (1 mL). Diisopropylethylamine (60 mg, 464.22 μmol) was added thereto, and then compound 12 (20mg, 41.37μmol) and HATU (20mg, 52.60μmol) were added. The reaction was stirred at room temperature for 1.5 hours. Purified by preparative high performance liquid chromatography to obtain white solid compound PROTAC 25 (5.5 mg, 15.55%).

$^1$H NMR (600MHz, $CD_3OD$) δ8.37 (d, *J* = 8.3Hz, 1H), 8.21 (d, *J* = 2.8Hz, 1H), 8.00-7.94 (m, 1H), 7.72-7.63 (m, 2H), 7.56(d, *J*=8.7Hz, 1H), 7.44(td, *J*=7.6,2.9Hz, 1H), 7.06-6.97(m, 1H), 6.84(d, *J*=9.4Hz, 1H), 5.13( td,*J*=13.4, 5.1Hz, 1H), 4.52-4.31(m, 4H), 3.98-3.74(m, 6H), 3.62(dd, *J*=22.7, 10.1Hz, 1H), 3.56-3.47(m, 1H), 3.47-3.40(m, 1H), 3.37(dd, *J* = 13.8, 7.0Hz, 1H), 3.28-3.12(m, 4H), 2.98-2.87(m, 3H), 2.87-2.74(m, 3H), 2.52-2.42(m, 1H), 2.21(s, 3H), 2.19-2.03(m, 4H), 1.99-1.85(m, 4H), 1.83-1.74(m, 1H), 1.63(ddd, *J*=26.4, 13.4, 4.7Hz, 1H), 1.41-1.37(m, 1H), 1.37-1.30(m, 1H), 1.25(t, *J*=8.8Hz,6H), 1.18-1.10(m, 6H).

LC-MS(ESI): $[M+H]^+$=1025.45/1027.45.

Example 54

Synthesis of PROTAC 26

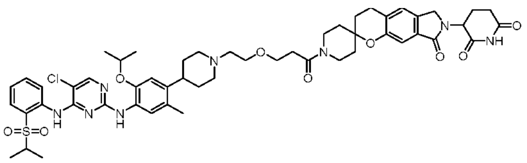

3-(1-(3-(2-(4-(4-((5-chloro-4-((2-(isopropylsulfonyl)phenyl)amino)pyrimidin-2-yl)amino)-5-isopropoxy-2-methylphenyl) piperidin-1-yl)ethoxy)propionyl)-8'-oxo-3',4',6',8'-tetrahydro- 7'H-spiro[piperidine-4,2'-pyrano[2,3-f]isoindole]-7'-yl)piperidine-2,6-dione

Synthesis scheme

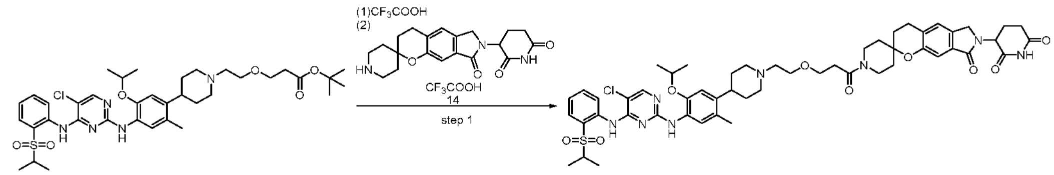


P-11 PROTAC 26

The first step: 3-(1-(3-(2-(4-(4-((5-chloro-4-((2-(isopropylsulfonyl)phenyl)amino)pyrimidin-2-yl )atnino)-5-isopropoxy-2-methylphenyl)piperidin-1-yl)ethoxy)propionyl)-8'-oxo-3',4',6',8' -tetrahydro-7'H-spiro[piperidine-4,2'-pyrano[2,3-f]isoindol]-7'-yl)piperidine-2,6-dione (compound PROTAC 26)

At room temperature, to a solution of compound P-11 (25 mg, 34.23 μmol) in dichloromethane (2 mL) was added trifluoroacetic acid (0.5 mL) with stirring. The reaction was stirred at room temperature for 3 hours. The resulting mixture was concentrated in vacuo, and dissolved by adding N,N-dimethylformamide (1 mL). Diisopropylethylamine (60 mg, 464.22 μmol)was added thereto, and then compound 14 (20mg, 41.37μmol) and HATU (20mg, 52.60μmol) were added. The reaction was stirred at room temperature for 1.5 hours. Purified by preparative high performance liquid chromatography to obtain white solid compound PROTAC 26 (6.4 mg, 18.10%).

$^1$H NMR (600MHz, $CD_3OD$) δ8.38(d, *J*=8.2Hz, 1H), 8.22(s, 1H), 7.95(td, *J*=8.1, 1.3Hz, 1H), 7.70-7.62(m, 2H), 7.43(dd, *J*=14.6, 7.2Hz, 1H), 7.32(d, *J*=8.0Hz, 1H), 7.25(d, *J*=8.3Hz, 1H), 6.83(d, *J*=8.3Hz, 1H), 5.19-5.08(m, 1H), 4.54-4.32(m, 4H), 3.99-3.73(m, 6H), 3.67-3.47(m, 3H), 3.47-3.39(m, 1H),3.39-3.34(m, 1H), 3.28-3.11(m, 4H), 2.99-2.88(m, 3H), 2.87-2.74(m, 3H), 2.56-2.44(m, 1H), 2.23(s,3H), 2.16-2.02(m, 4H), 1.98-1.83(m, 4H), 1.81-1.72(m, 1H), 1.60(ddd, *J* = 26.2, 12.9, 4.7Hz, 1H), 1.36-1.32(m, 1H), 1.26(d, *J*=6.8Hz, 6H), 1.20-1.12(m, 6H).

LC-MS(ESI): $[M+H]^+$=1025.45/1027.35.

Example 55

Synthesis of PROTAC 27

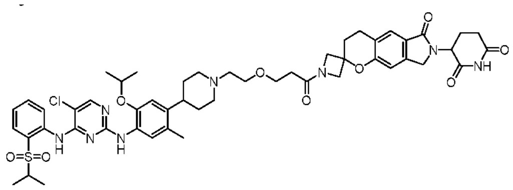

3-(1-(3-(2-(4-(4-((5-chloro-4-((2-(isopropylsulfonyl)phenyl)amino)pyrimidin-2-yl)amino)-5-isopropoxy-2-methylphenyl) piperidin-1-yl)ethoxy)propionyl)-6'-oxo-3',4',6',8'-tetrahydro- 7'H-spiro[azetidine-3,2'-pyrano[2,3-f]isoindole]-7'-yl)piperidine-2,6-dione

Synthesis scheme:

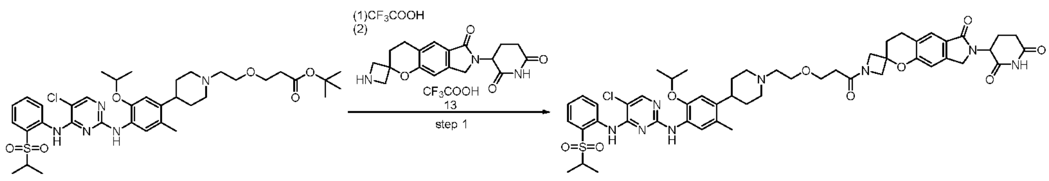


The first step: 3-(1-(3-(2-(4-(4-((5-chloro-4-((2-(isopropylsulfonyl)phenyl)amino)pyrimidin-2-yl )atnino)-5-isopropoxy-2-methylphenyl)piperidin-1-yl)ethoxy)propionyl)-6'-oxo-3',4',6',8' -tetrahydro-7'H-spiro[azetidine-3,2'-pyrano[2,3-f]isoindole]-7'-yl)piperidine-2,6-dione (compound PROTAC 27)

To a solution of compound P-11 (25 mg, 34.23 μmol) in dichloromethane (2 mL) was added trifluoroacetic acid (0.5 mL) with stirring at room temperature. The reaction was stirred at room temperature for 3 hours. The resulting mixture was concentrated in vacuo, and dissolved by adding N,N-dimethylformamide (1 mL), diisopropylethylamine (60 mg, 464.22 μmol) was added thereto, and then compound 13 (20mg, 43.92μmol) and HATU (20mg, 52.60μmol) were added. The reaction was stirred at room temperature for 1.5 hours. Purified by preparative high performance liquid chromatography to obtain white solid compound PROTAC 27 (7.5 mg, 20.54%).

$^1H$ NMR (600MHz, $CD_3OD$) δ8.37(d, *J*=8.3Hz, 1H), 8.21(d, *J*=4.7Hz, 1H), 7.98-7.92(m, 1H), 7.68(t, *J*=6.1Hz, 2H), 7.55(s, 1H), 7.44(dt, *J*=10.6, 4.6Hz, 1H), 6.88(d, *J*=5.0Hz, 1H), 6.87(d, *J*=5.0Hz, 1H), 5.10 (ddd, *J*= 13.4, 5.1, 2.3Hz, 1H), 4.58-4.51(m, 1H), 4.41-4.24(m, 4H), 4.14(d, *J*= 10.5Hz, 1H), 4.09-4.03(m, 1H), 3.95-3.84(m, 4H), 3.83-3.73(m, 3H), 3.49-3.40(m, 2H), 3.40-3.34(m, 1H), 3.28-3.20(m, 1H), 3.19-3.12(m, 1H), 2.98(t, *J* = 6.3Hz, 2H), 2.95-2.85(m, 1H), 2.80-2.73(m, 1H), 2.67-2.61(m, 1H), 2.56-2.50 (m, 1H), 2.48-2.38(m, 1H), 2.27-2.20(m, 4H), 2.16-2.05(m, 5H), 1.37-1.30(m, 1H), 1.27-1.24(m, 6H), 1.24-1.2 1(m, 6H).

LC-MS(ESI): $[M+H]^+$=997.35/999.25.

Example 56

Synthesis of PROTAC 28

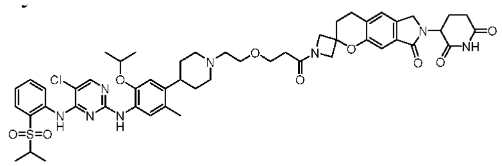

3-(1-(3-(2-(4-(4-((5-chloro-4-((2-(isopropylsulfonyl)phenyl)amino)pyrimidin-2-yl)amino)-5-isopropoxy-2-methylphenyl) piperidin-1-yl)ethoxy)propionyl)-8'-oxo-3',4',6',8'-tetrahydro-7'H-spiro[azetidine-3,2'-pyrano[2,3-f]isoindole]-7'-yl)piperidine-2,6-dione

Synthesis scheme:

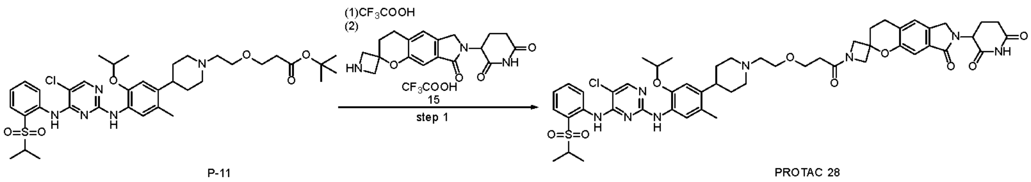


The first step: 3-(1-(3-(2-(4-(4-((5-chloro-4-((2-(isopropylsulfonyl)phenyl)amino)pyrimidin-2-yl )atnino)-5-isopropoxy-2-methylphenyl)piperidin-1-yl)ethoxy)propionyl)-8'-oxo-3',4',6',8' -tetrahydro-7'H-spiro[azetidine-3,2'-pyrano[2,3-f]isoindole]-7'-yl)piperidine-2,6-dione (compound PROTAC 28)

To a solution of compound P-11 (25 mg, 34.23 μmol) in dichloromethane (2 mL) was added trifluoroacetic acid (0.5 mL) with stirring at room temperature. The reaction was stirred at room temperature for 3 hours. The resulting mixture was concentrated under vacuum, and dissolved by adding N ,N-dimethylformamide (1 mL). Diisopropylethylamine (60 mg, 464.22 μmol) was added thereto, and then compound 15 (20mg, 43.92μmol) and HATU (20mg, 52.60μmol) were added. The reaction was stirred at room temperature for 1.5 hours. Purified by preparative high performance liquid chromatography to obtain white solid compound PROTAC 28 (7.2 mg, 19.72%).

$^1$H NMR (600MHz, $CD_3OD$) δ8.38(d, *J*=8.2Hz, 1H), 8.21(s, 1H), 7.98-7.93(m, 1H), 7.71-7.64(m, 1H), 7.63(d, *J*=4.3Hz, 1H), 7.47-7.39(m, 1H), 7.34(s, 1H), 7.25(s, 1H), 6.87(d, *J*=4.0Hz, 1H), 5.17-5.08(m, 1H), 4.57-4.49(m, 1H), 4.48-4.38(m, 2H), 4.38-4.28(m, 2H), 4.16-4.02(m, 2H), 3.96-3.83(m, 3H), 3.83-3.73 (m, 3H), 3.51-3.39(m, 2H), 3.39-3.35(m, 1H), 3.28-3.18(m, 2H), 3.17-3.10(m, 1H), 2.99(dd, *J* = 15.2, 8.5Hz, 2H), 2.95-2.86(m, 1H), 2.79(d, *J* = 17.4Hz, 1H), 2.65(ddd, *J* = 16.4, 8.1, 3.7Hz, 1H), 2.57-2.43(m, 2H ),2.29-2.22(m, 2H), 2.22-2.17(m, 3H),2.14-1.99(m, 4H),1.33(dt, *J* = 12.6, 6.7Hz, 1H), 1.26(d, *J* = 6.7Hz, 6H), 1.28-1.19(m, 6H).

LC-MS(ESI): $[M+H]^+$=997.45/999.35.

Example 57

Synthesis of PROTAC 29

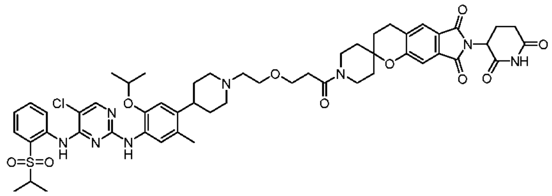

1-(3-(2-(4-(4-((5-chloro-4-((2-(isopropylsulfonyl)phenyl)amino)pyrimidin-2-yl)amino)-5-isopropoxy-2-methylphenyl)piperidin-1-yl)ethoxy)propionyl)-7'-(2,6-dioxopiperidin-3-yl)-3',4' -dihydro-6'H-spiro[piperidine-4,2'-pyrano[2,3-f]isoindole]-6',8'(7'H)-dione

Synthesis scheme:

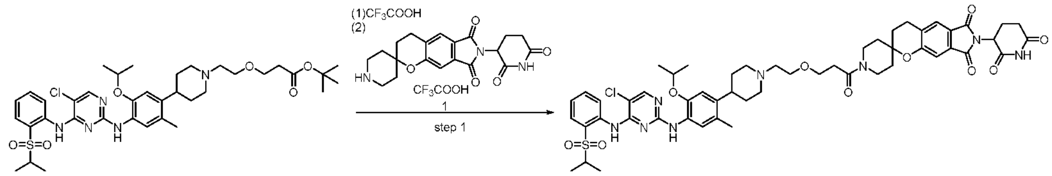


P-11 PROTAC 29

The first step: 1-(3-(2-(4-(4-((5-chloro-4-((2-(isopropylsulfonyl)phenyl)amino)pyrimidin-2-yl)amino) -5-isopropoxy-2-methylphenyl)piperidin-1-yl)ethoxy)propionyl)-7'-(2,6-dioxopiperidin-3-yl)-3 ',4'-dihydro-6'H-spiro[piperidine-4,2'-pyrano[2,3-f]isoindole]-6',8'(7'H)-dione (compound PROTAC 29)

To a solution of compound P-11 (40 mg, 54.77 μmol) in dichloromethane (2 mL) was added trifluoroacetic acid (0.5 mL) with stirring at room temperature. The reaction was stirred at room temperature for 3 hours. The resulting mixture was concentrated under vacuum, and dissolved by adding N,N-dimethylformamide (1 mL). Diisopropylethylamine (60 mg, 464.22 μmol) was added thereto, and then compound 1(35mg, 70.36μmol) and HATU (32mg, 84.16μmol) were added . The reaction was stirred at room temperature for 1.5 hours. Purified by preparative high performance liquid chromatography to obtain white solid compound PROTAC 29 (7.2 mg, 9.84%).

$^1H$ NMR (600MHz, $CD_3OD$) δ8.37(d, *J*=8.2Hz, 1H), 8.22(d, *J*=10.1Hz, 1H), 7.98(d, *J*=9.9Hz, 1H), 7.72-7.63(m, 3H), 7.45(t, *J*=7.7Hz, 1H), 7.31(d, *J*=10.8Hz, 1H), 6.85(d, *J*=1.7Hz, 1H), 5.11(dd, *J*=12.7, 5.5Hz, 1H), 4.52-4.45(m, 2H), 3.98-3.84(m, 4H), 3.84-3.73(m, 3H), 3.61(t, *J*= 12.4Hz, 1H), 3.54-3.48(m, 1H ), 3.48-3.40(m, 1H),3.40-3.34(m, 1H), 3.27-3.18(m, 3H), 3.18-3.11(m, 1H), 2.99(t, *J* = 6.3Hz, 2H), 2.91-2.68(m, 5H), 2.21(s, 3H), 2.17-2.03(m, 5H), 2.00-1.91(m, 3H), 1.88(d, *J*= 14.0Hz, 1H), 1.85-1.76(m, 1H), 1.69-1.60(m, 1H), 1.26(d, *J* = 6.8Hz, 6H), 1.22-1.16(m, 6H).

LC-MS (ESI): $[M+H]^+$=1039.44.

Example 58

Synthesis of PROTAC 30

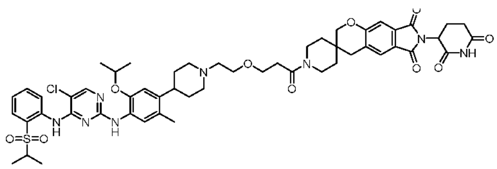

1-(3-(2-(4-(4-((5-chloro-4-((2-(isopropylsulfonyl)phenyl)amino)pyrimidin-2-yl)amino)-5-isopropoxy-2-methylphenyl)piperidin-1-yl)ethoxy)propionyl)-7'-(2,6-dioxopiperidin-3-yl)-2'H-spiro [piperidine-4,3'-pyrano[2,3-f]isoindole]-6',8'(4'H,7'H)-dione

Synthesis scheme:

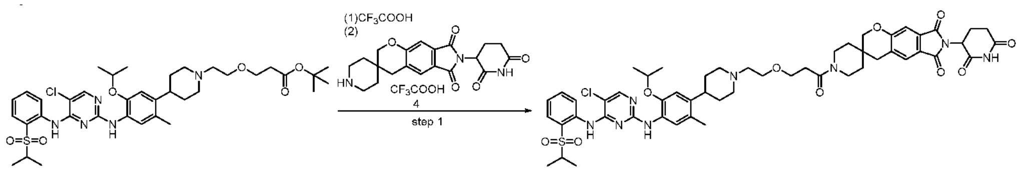


P-11 PROTAC 30

The first step: 1-(3-(2-(4-(4-((5-chloro-4-((2-(isopropylsulfonyl)phenyl)amino)pyrimidin-2-yl)amino) -5-isopropoxy-2-methylphenyl)piperidin-1-yl)ethoxy)propionyl)-7'-(2,6-dioxopiperidin-3-yl)-2'H-spiro[piperidine-4,3'-pyrano[2,3-f]isoindole]-6',8'(4'H,7'H)-dione (compound PROTAC 30)

To a solution of compound P-11 (40 mg, 54.77μmol) in dichloromethane (2 mL) was added trifluoroacetic acid (0.5 mL) with stirring at room temperature. The reaction was stirred at room temperature for 3 hours. The resulting mixture was concentrated under vacuum, and dissolved by adding N,N-dimethylformamide (1 mL). Diisopropylethylamine (60 mg, 464.22 μmol)was added thereto, and then compound 4 (35mg, 70.36μmol) and HATU (32mg, 84.16μmol) were added. The reaction was stirred at room temperature for 1.5 hours. Purified by preparative high performance liquid chromatography to obtain white solid compound PROTAC 30 (7.6 mg, 10.39%).

$^1$H NMR (600MHz, CD3OD) δ8.37(d, *J*=7.5Hz, 1H), 8.21(d, *J*=9.7Hz, 1H), 7.98(d, *J*=10.1Hz, 1H), 7.75-7.61 (m, 3H), 7.45(t, *J*=7.6Hz, 1H), 7.24(d, *J*=1.7Hz, 1H), 6.83(d, *J*=1.5Hz,1H), 5.11(dd, *J*=12.8, 5.4Hz, 1H), 4.46(dt, *J*=12.1, 5.9Hz, 1H), 4.17(d, *J*=11.1Hz, 1H), 4.10(d, *J*=11.0Hz, 1H), 3.99-3.88(m, 3H), 3.83(t, *J*= 5.1Hz, 2H), 3.77(d, *J* = 12.0Hz, 2H), 3.75-3.70(m, 1H), 3.66-3.58(m, 2H), 3.46(dd, *J*= 13.2, 3.1Hz, 2H), 3.40-3.35(m, 1H), 3.22(dd, *J*= 15.4, 6.8Hz, 2H), 3.14(dd, *J*= 13.6, 10.0Hz, 1H), 3.01-2.93(m, 2H), 2.93-2.84(m, 1H), 2.83-2.68(m, 4H), 2.21(s, 3H), 2.16-2.03(m, 5H), 1.68-1.53(m, 3H), 1.53-1.46(m, 1H), 1.26(d, *J*= 6.8Hz, 6H), 1.22-1.14(m, 6H).

LC-MS (ESI): $[M+H]^+$=1039.54.

Example 59

Synthesis of PROTAC 31

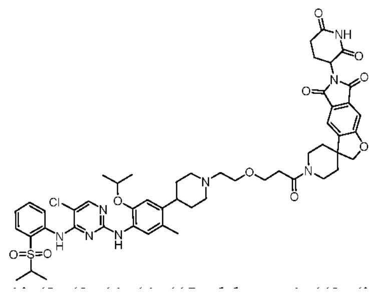

1'-(3-(2-(4-(4-((5-chloro-4-((2-(isopropylsulfonyl)phenyl)amino)pyrimidin-2-yl)amino)-5-isopropoxy-2-methylphenyl)piperidin-1-yl)ethoxy)propionyl)-6-(2,6-dioxopiperidin-3-yl)-6-hydrogen-2H,5H-spiro[furo[2,3-f]isoindole-3,4'-piperidine]-5,7-dione

Synthesis scheme:

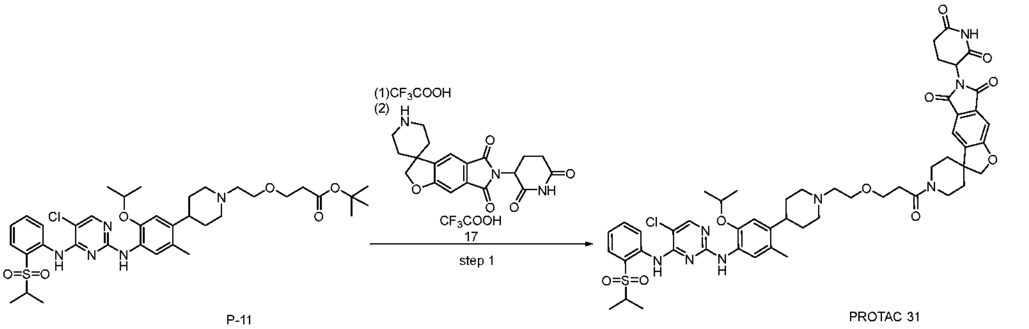


P-11 PROTAC 31

The first step: 1'-(3-(2-(4-(4-((5-chloro-4-((2-(isopropylsulfonyl)phenyl)amino)pyrimidin-2-yl)amino )-5-isopropoxy-2-methylphenyl)piperidin-1-yl)ethoxy)propionyl)-6-(2,6-dioxopiperidin-3-yl)-6 - hydro-2H,5H-spiro[furo[2,3-f]isoindole-3,4'-piperidine]-5,7-dione (compound PROTAC 31)

To a solution of compound P-11 (40 mg, 54.77 μmol) in dichloromethane (2 mL) was added trifluoroacetic acid (0.5 mL) with stirring at room temperature. The reaction was stirred at room temperature for 3 hours. The resulting mixture was concentrated in vacuo, and dissolved by adding N,N-dimethylformamide (1 mL). Diisopropylethylamine (60 mg, 464.22 μmol) was added thereto, and then compound 17 (35mg, 74.57μmol) and HATU (32mg, 84.16μmol) were added. The reaction was stirred at room temperature for 1.5 hours. Purified by preparative high performance liquid chromatography to obtain white solid compound PROTAC 31 (10.5 mg, 13.92%).

$^1$H NMR (600MHz, $CD_3OD$) δ8.39(d, *J*=8.2Hz, 1H), 8.19(s, 1H), 7.95(d, *J*=7.9Hz, 1H), 7.73(s, 1H), 7.71-7.66 (m, 2H), 7.43(t, *J*=7.6Hz, 1H), 7.25(s, 1H), 6.85(s, 1H), 5.09(dd, *J*=12.9, 5.4Hz, 1H), 4.75(s, 2H), 4.65(d, *J* = 13.8Hz, 1H), 4.56-4.49(m, 1H), 4.07(d, *J* = 14.3Hz, 1H), 3.96-3.90(m, 2H), 3.89-3.84(m, 2H), 3.84-3.75(m, 2H), 3.52-3.43(m, 2H), 3.38-3.34(m, 1H), 3.29-3.20(m, 2H), 3.17-3.11(m, 1H), 3.00-2.93(m, 1H), 2.90-2.69(m, 6H), 2.21(s, 3H), 2.15-2.03(m, 6H), 1.95-1.90(m, 2H), 1.87-1.82(m, 1H), 1.29-1.21(m, 12H).

LC-MS (ESI): $[M+H]^+$=1025.35.

Example 60

Synthesis of PROTAC 32

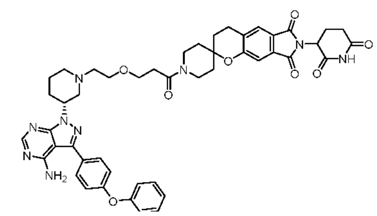

1-(3-(2-((R)-3-(4-amino-3-(4-phenoxyphenyl)-1H-pyrazolo[3,4-d]pyrimidin-1-yl)piperidin-1-yl)ethoxy)propionyl)-7'-(2,6-dioxopiperidin-3-yl)-3',4'-dihydro-6'H-spiro[piperidin -4,2'-pyrano[2,3-f]isoindole]-6',8'(7'H)-dione

Synthesis scheme:

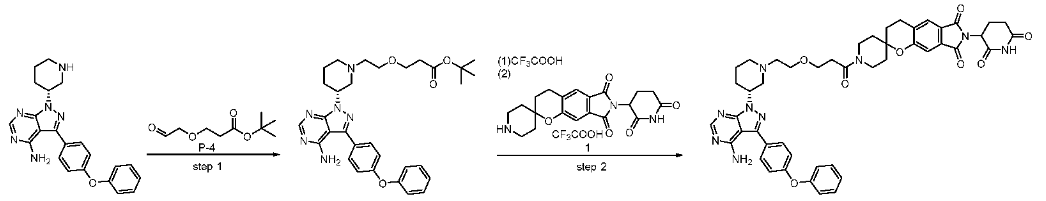


P-12 P-13 PROTAC 32

The first step: (R)-3-(2-(3-(4-amino-3-(4-phenoxyphenyl)-1H-pyrazolo[3,4-d]pyrimidin-1-yl ) piperidin-1-yl) ethoxy) tert-butyl propionate (compound P-13)

To a solution of compound P-12 (100 mg, 258.76 μmol) and compound P-4 (97.41 mg, 517.52 μmol) in methanol (5 mL) were added acetic acid (1 mL)at room temperature, finally sodium cyanoborohydride (49 mg, 776.28 μmol) was added with stirring. The reaction was stirred at room temperature for 14 hours. The resulting mixture was concentrated in vacuo and purified by high performance liquid chromatography to obtain compound P-13 (112 mg, 77.47%) as a colorless oil.

$^1H$ NMR (600MHz, $CD_3OD$) δ8.35(s, 1H), 7.78-7.68(m, 2H), 7.44(t, *J*=7.9Hz, 2H), 7.26-7.17(m, 3H), 7.16-7.10(m, 2H), 5.38-5.22(m, 1H), 3.98-3.82(m, 3H), 3.79-3.68(m, 3H), 3.57-3.47(m, 2H), 3.28-3.16(m, 1H), 2.59-2.52(m, 1H), 2.44-2.37(m, 1H), 2.36-2.15(m, 3H), 2.13-2.03(m, 1H), 2.01-1.94(m, 1H), 1.36(d, *J*=38.3Hz, 9H).

LC-MS (ESI): $[M+H]^+$=559.38.

The second step: 1-(3-(2-((R)-3-(4-amino-3-(4-phenoxyphenyl)-1H-pyrazolo[3,4-d]pyrimidine- 1-yl)piperidin-1-yl) ethoxy)propionyl)-7'-(2,6-dioxopiperidin-3-yl)-3',4'-dihydro-6'H -spiro[piperidine-4,2'-pyrano[2,3-f]isoindole]-6',8'(7'H)-dione (compound PROTAC 32)

To a solution of compound P-13 (40 mg, 71.60 μmol) in dichloromethane (2 mL) was added trifluoroacetic acid (0.5 mL) with stirring at room temperature. The reaction was stirred at room temperature for 3 hours. The resulting mixture was concentrated in vacuo, and dissolved by adding N,N-dimethylformamide (1 mL). Diisopropylethylamine (50 mg, 386.86 μmol) was added thereto, and then compound 1 (35mg, 74.57μmol) and HATU (20mg, 52.60μmol) were added. The reaction was stirred at room temperature for 1.5 hours. Purified by preparative high performance liquid chromatography to obtain white solid compound PROTAC 32 (10.5 mg, 13.25%).

$^1H$ NMR (600MHz, $CD_3OD$) δ8.41-8.35(m,1H), 7.79-7.63(m, 3H), 7.46-7.34(m, 2H), 7.30(d, *J* = 13.4Hz, 1H), 7.26-7.14(m, 3H), 7.14-7.06(m, 2H), 5.46-5.35(m, 1H), 5.11(dd,*J* = 10.4, 5.1Hz, 1H), 4.55-4.36(m, 1H), 4.14-3.97(m, 1H), 3.95-3.70(m, 7H), 3.65-3.49(m, 3H), 3.28-3.17(m, 2H), 3.04-2.93(m, 2H), 2.92-2.69(m, 5H), 2.40-2.09(m, 5H), 2.03-1.65(m, 6H).

LC-MS (ESI): $[M+H]^+$=868.46.

Example 61

Synthesis of PROTAC 33

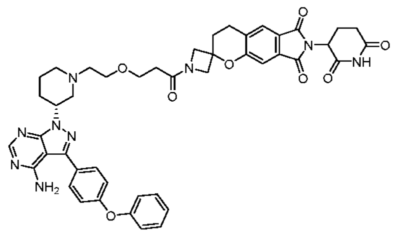

1-(3-(2-((R)-3-(4-amino-3-(4-phenoxyphenyl)-1H-pyrazolo[3,4-d]pyrimidin-1-yl) piperidin-1-yl)ethoxy)propionyl)-7'-(2,6-dioxopiperidin-3-yl)-3',4'-dihydro-6'H-spiro[azetidine-3,2'-pyrano[2,3-f]isoindole]-6',8'(7'H)-dione

Synthesis scheme:

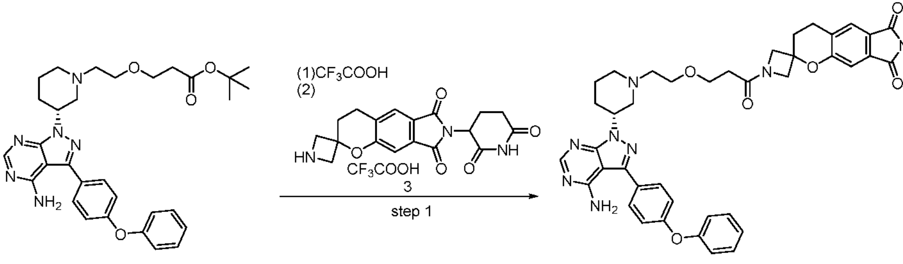


P-13

PROTAC 33

The first step: 1-(3-(2-((R)-3-(4-amino-3-(4-phenoxyphenyl)-1H-pyrazolo[3,4-d]pyrimidine- 1-yl)piperidin-1-yl)ethoxy) propionyl)-7'-(2,6-dioxopiperidin-3-yl)-3',4'-dihydro-6'H -Spiro[azetidine-3,2'-pyrano[2,3-f]isoindole]-6',8'(7'H)-dione (compound PROTAC33)

To a solutionof compound P-13 (40 mg, 71.60 μmol) in dichloromethane (2 mL) was added trifluoroacetic acid (0.5 mL) with stirring at room temperature. The reaction was stirred at room temperature for 3 hours. The resulting mixture was concentrated under vacuum, and dissolved by adding N,N-dimethylformamide (1 mL). Diisopropylethylamine (50 mg, 386.86 μmol) was added thereto, and then compound 3 (35mg, 74.57μmol) and HATU (20mg, 52.60μmol) were added thereto. The reaction was stirred at room temperature for 1.5 hours. Purified by preparative high performance liquid chromatography to obtain white solid compound PROTAC 33 (9.4 mg, 15.01%).

$^1$H NMR (600MHz, $CD_3OD$) δ8.43-8.32(m, 1H), 7.80-7.62(m, 3H), 7.46-7.38(m, 2H), 7.30-7.04(m, 6H), 5.46-5.34(m, 1H), 5.11(dd, *J* = 12.7, 5.2Hz, 1H), 4.37-4.22(m, 2H), 4.11(dd, *J* = 27.1, 13.2Hz, 2H), 3.98-3.68(m, 7H), 3.61-3.46(m, 2H), 3.29-3.17(m, 1H), 3.05-2.93(m,2H), 2.92-2.84(m, 1H), 2.80-2.68(m, 2H), 2.63-2.46(m, 1H), 2.40-1.97(m, 8H).

LC-MS (ESI): $[M+H]^+$=840.56.

Example 62

Synthesis of PROTAC 34

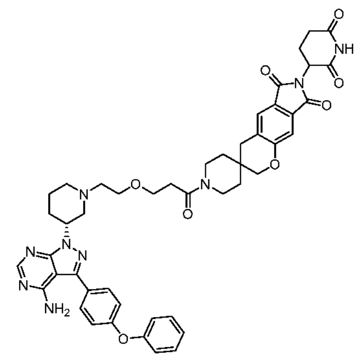

1-(3-(2-((R)-3-(4-amino-3-(4-phenoxyphenyl)-1H-pyrazolo[3,4-d]pyrimidin-1-yl) piperidin-1-yl)ethoxy)propionyl)-7'-(2,6-dioxopiperidin-3-yl)-2'H-spiro[piperidine-4,3'-pyrano[2,3-f]isoindole]-6',8'(4'H,7'H)-dione

Synthesis scheme:

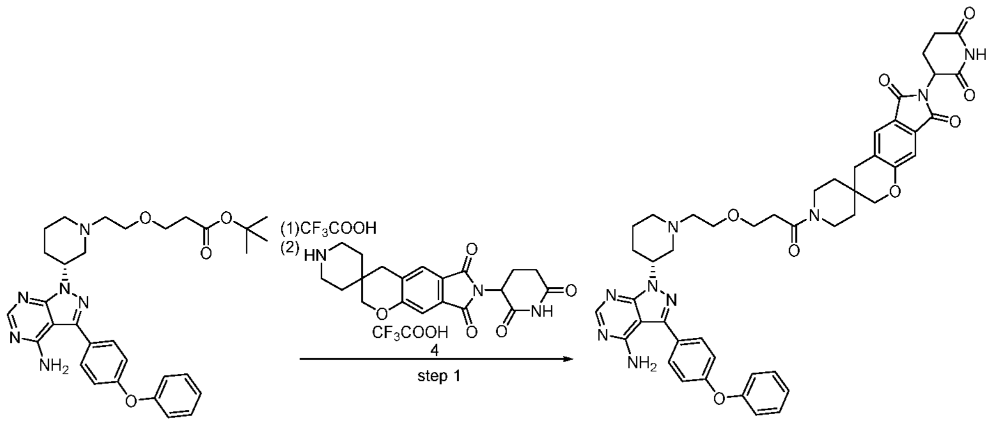


P-13

PROTAC 34

The first step: 1-(3-(2-((R)-3-(4-amino-3-(4-phenoxyphenyl)-1H-pyrazolo[3,4-d]pyrimidine- 1-yl)piperidin-1-yl)ethoxy) propionyl)-7'-(2,6-dioxopiperidin-3-yl)-2'H-spiro[piperidine-4,3 '-pyrano[2,3-f]isoindole]-6',8'(4'H,7'H)-dione (compound PROTAC 34)

To a solution of compound P-13 (40 mg, 71.60 μmol) in dichloromethane (2 mL) was added trifluoroacetic acid (0.5 mL) with stirring at room temperature. The reaction was stirred at room temperature for 3 hours. The resulting mixture was concentrated under vacuum, and dissolved by adding N,N-dimethylformamide (1 mL). Diisopropylethylamine (50 mg, 386.86 μmol) was added thereto, and then compound 4(35mg, 70.36μmol) and HATU (20mg, 52.60μmol) were added (35mg, 70.36μmol) . The reaction was stirred at room temperature for 1.5 hours. Purified by preparative high performance liquid chromatography to obtain white solid compound PROTAC 34 (10.6 mg, 17.36%).

$^1$H NMR (600MHz, $CD_3OD$) δ8.37(d, *J*=8.8Hz, 1H), 7.80-7.60(m, 3H), 7.43(t, *J*=7.9Hz, 2H), 7.29-7.07(m, 6H), 5.47-5.36(m, 1H), 5.11(dd, *J* = 12.8, 5.4Hz, 1H), 4.30-3.46(m, 15H), 3.28-3.13(m, 1H), 3.00-2.67(m, 6H), 2.41-1.96(m, 6H), 1.67-1.29(m, 4H).

LC-MS (ESI): $[M+H]^+$=868.46.

Example 63

Synthesis of PROTAC 35

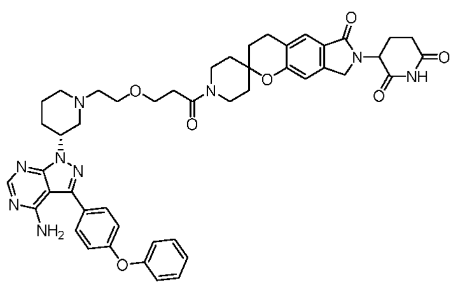

3-(1-(3-(2-((R)-3-(4-amino-3-(4-phenoxyphenyl)-1H-pyrazolo[3,4-d]pyrimidine-1 -yl)piperidin-1-yl)ethoxy)propionyl)-6'-oxo-3',4',6',8'-tetrahydro-7'H-spiro[piperidine-4, 2'-pyrano[2,3-f]isoindol]-7'-yl)piperidine-2,6-dione

Synthesis scheme:

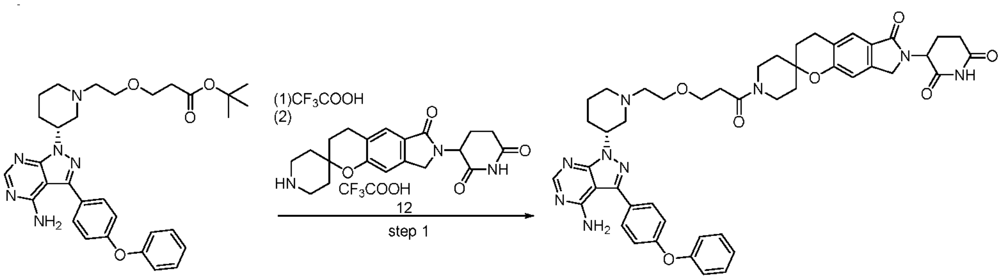


P-13 PROTAC 35

The first step: 3-(1-(3-(2-((R)-3-(4-amino-3-(4-phenoxyphenyl)-1H-pyrazolo[3,4-d ]pyrimidin-1-yl)piperidin-1-yl)ethoxy) propionyl)-6'-oxo-3',4',6',8'-tetrahydro-7'H-spiro[piperdine-4,2'-pyrano[2,3-f]isoindol]-7'-yl)piperidine-2,6-dione (compound PROTAC 35)

To a solutionof compound P-13 (40 mg, 71.60 μmol) in dichloromethane (2 mL) was added trifluoroacetic acid (0.5 mL) with stirring at room temperature. The reaction was stirred at room temperature for 3 hours. The resulting mixture was concentrated in vacuo, and dissolved by adding N,N-dimethylformamide (1 mL). Diisopropylethylamine (50 mg, 386.86 μmol) was added thereto, and then compound 12 (30mg, 60.05μmol) and HATU (20mg, 52.60μmol) were added. The reaction was stirred at room temperature for 1.5 hours. Purified by preparative high performance liquid chromatography to obtain white solid compound PROTAC 35 (7.0 mg, 13.21%).

$^1$H NMR (600MHz, $CD_3OD$) δ8.42-8.35(m,1H), 7.81-7.66(m,2H), 7.56(d, *J*=13.4Hz, 1H), 7.48-7.35(m, 2H), 7.25- 6.93(m, 6H), 5.47-5.34(m, 1H), 5.17-5.07(m, 1H), 4.53-4.28(m, 3H), 4.12-3.48(m, 11H), 3.29-3.15(m, 2H), 2.98-2.74(m, 5H), 2.58-2.12(m, 6H), 2.07-1.46(m, 7H).

LC-MS (ESI): $[M+H]^+$=854.56.

Example 64

Synthesis of PROTAC 36

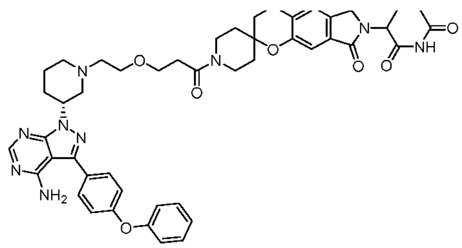

3-(1-(3-(2-((R)-3-(4-amino-3-(4-phenoxyphenyl)-1H-pyrazolo[3,4-d]pyrimidine-1 -yl)piperidin-1-yl)ethoxy)propionyl)-8'-oxo-3',4',6',8'-tetrahydro-7'H-spiro[piperidine-4, 2'-pyrano[2,3-f]isoindol]-7'-yl)piperidine-2,6-dione

Synthesis scheme:

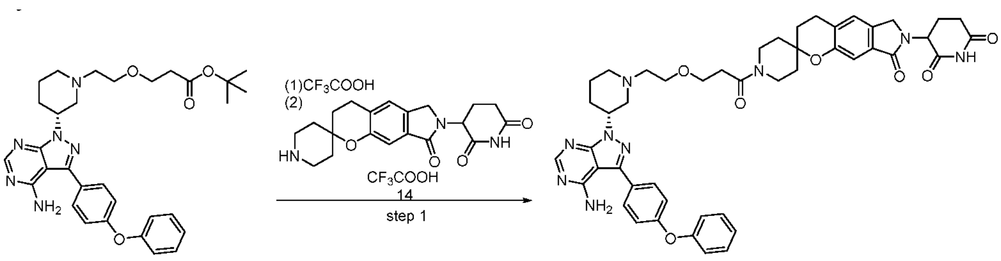


P-13 PROTAC 36

The first step: 3-(1-(3-(2-((R)-3-(4-amino-3-(4-phenoxyphenyl)-1H-pyrazolo[3,4-d ]pyrimidin-1-yl)piperidin-1-yl)ethoxy) propionyl)-8'-oxo-3',4',6',8'-tetrahydro-7'H-spiro[piperidine-4,2'-pyrano[2,3-f]isoindol]-7'-yl)piperidine-2,6-dione (compound PROTAC 36)

To a solutionof compound P-13 (40 mg, 71.60 μmol) in dichloromethane (2 mL) was added trifluoroacetic acid (0.5 mL) with stirring at room temperature. The reaction was stirred at room temperature for 3 hours. The resulting mixture was concentrated in vacuo, and dissolved by adding N,N-dimethylformamide (1 mL). Diisopropylethylamine (50 mg, 386.86 μmol) was added thereto, and then compound 14 (30mg, 62.05μmol) and HATU (20mg, 52.60μmol) were added. The reaction was stirred at room temperature for 1.5 hours. Purified by preparative high performance liquid chromatography to obtain white solid compound PROTAC 36 (10.0 mg, 18.87%).

$^1H$ NMR (600MHz, $CD_3OD$) δ8.40-8.34(m, 1H), 7.78-7.66(m, 2H), 7.46-7.27(m,3H), 7.26-7.06(m,6H), 5.46-5.33(m, 1H), 5.14(dd, *J* = 13.0,4.8Hz,1H), 4.51-4.26(m,3H), 3.96-3.48(m,10H), 3.29-3.16(m,2H), 3.00-2.72(m ,5H), 2.56-2.13(m,7H), 2.07-1.59(m, 7H).

LC-MS (ESI): $[M+H]^+$=854.46.

Example 65

Synthesis of PROTAC 37

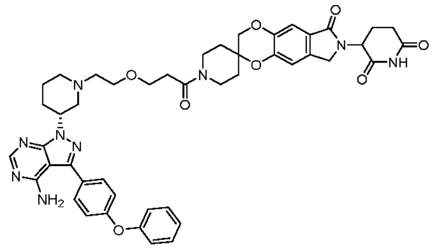

3-(1-(3-(2-((R)-3-(4-amino-3-(4-phenoxyphenyl)-1H-pyrazolo[3,4-d]pyrimidine-1 -yl)piperidin-1-yl)ethoxy)propionyl)-6'-oxo-6',8'-dihydro-3'H,7'H-spiro[piperidine-4,2' -[1,4]dioxino[2,3-f]isoindole]-7'-yl)piperidine-2,6-dione

Synthesis scheme:

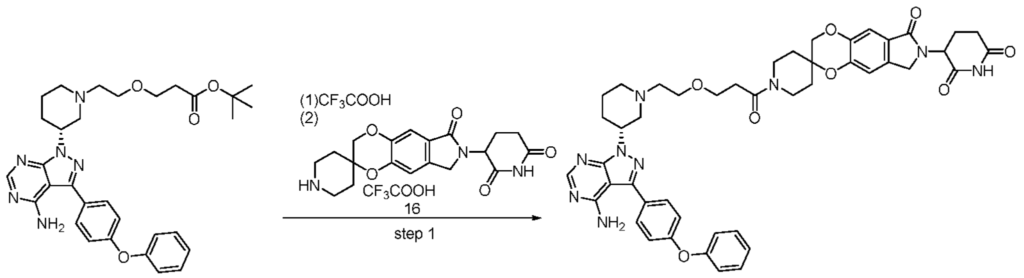


P-13

PROTAC 37

The first step: 3-(1-(3-(2-((R)-3-(4-amino-3-(4-phenoxyphenyl)-1H-pyrazolo[3,4-d ]pyrimidin-1-yl)piperidin-1-yl)ethoxy) propionyl)-6'-oxo-6',8'-dihydro-3'H,7'H-spiro[piperidine- 4,2'-[1,4]dioxino[2,3-f]isoindol]-7'-yl)piperidine-2,6-dione (compound PROTAC 37)

To a solutionof compound P-13 (40 mg, 71.60 μmol) in dichloromethane (2 mL) was added trifluoroacetic acid (0.5 mL) with stirring at room temperature. The reaction was stirred at room temperature for 3 hours. The resulting mixture was concentrated in vacuo, and dissolved by adding N,N-dimethylformamide (1 mL). Diisopropylethylamine (50 mg, 386.86 μmol) was added thereto, and then compound 16 (30mg, 61.80μmol) and HATU (20mg, 52.60μmol) were added. The reaction was stirred at room temperature for 1.5 hours. Purified by preparative high performance liquid chromatography to obtain white solid compound PROTAC 37 (6.4 mg, 12.10%).

$^1$H NMR (600MHz, $CD_3OD$) δ8.39-8.34(m, 1H), 7.80-7.67(m,2H), 7.48-7.35(m,2H),7.34-7.27(m, 1H), 7.25-7.01(m, 6H),5.46-5.34(m, 1H), 5.18-5.09(m, 1H), 4.54-4.27(m, 3H), 4.12-3.48(m, 12H), 3.28-3.14(m, 2H), 2.96-2.75(m, 4H), 2.61-2.41(m, 2H), 2.40-2.13(m, 4H), 2.06-1.63(m, 5H).

LC-MS (ESI): $[M+H]^+$=856.36.

Example 66

Synthesis of PROTAC 38

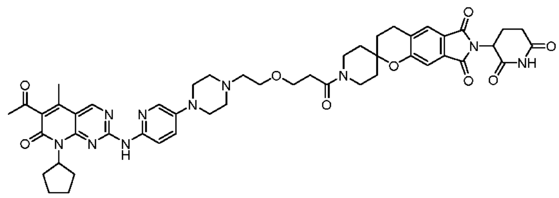

1-(3-(2-(4-(6-((6-acetyl-8-cyclopentyl-5-methyl-7-oxo-7,8-dihydropyridone[2,3 -d]pyrimidin-2-yl)amino)pyridin-3-yl)piperazin-1-yl)ethoxy)propionyl)-7'-(2,6-dioxopiperidin-3-yl) -3',4'-dihydro-6'H-spiro[piperidine-4,2'-pyrano[2,3-f]isoindole]-6',8'(7'H)- dione

Synthesis scheme:

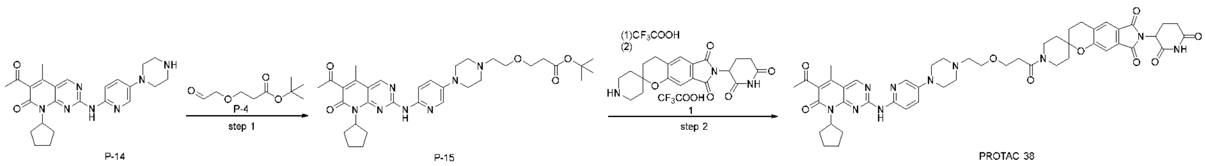


The first step: 3-(2-(4-(6-((6-acetyl-8-cyclopentyl-5-methyl-7-oxo-7,8-dihydropyridone[2, 3-d]pyrimidin-2-yl)atnino)pyridin-3-yl)piperazin-1-yl)ethoxy)propionate tert-butyl ester (compound P-15)

To a solution of compound P-14 (110 mg, 245.79 μmol) and compound P-4 (92.53 mg, 491.57 μmol) in methanol (3 mL) were added acetic acid (1 mL) and tetraisopropyl titanate (1 mL) at room temperature, sodium triacetoxyborohydride (156.28 mg, 737.36 μmol) was added with stirring. The reaction was stirred at 40°C for 2 hours. The resulting mixture was concentrated under vacuum and purified by preparative high performance liquid chromatography to obtain colorless oily compound P-15 (131 mg, 86.00%).

$^1$H NMR (600MHz, $CD_3OD$) δ8.96(s,1H), 8.12-8.05(m,2H), 7.59(dd, *J* = 9.1,2.7Hz,1H), 6.03-5.95(m,1H), 3.91-3.86(m, 2H), 3.79(t, *J*=5.8Hz, 2H), 3.71-3.22(m, 10H), 2.60(t, *J*=5.8Hz, 2H), 2.51(s, 3H), 2.40(s ,3H),2.37-2.30(m, 2H), 2.08-2.00(m, 2H),1.94-1.87(m, 2H),1.74-1.66(m, 2H), 1.54-1.45(m, 9H).

LC-MS (ESI): $[M+H]^+$=620.48.

The second step: 1-(3-(2-(4-(6-((6-acetyl-8-cyclopentyl-5-methyl-7-oxo-7,8-dihydropyridone [2,3-d]pyrimidin-2-yl)atnino)pyridin-3-yl)piperazin-1-yl)ethoxy)propionyl)-7'-(2,6-dioxopiperidine- 3-yl)-3',4'-dihydro-6'H-spiro[piperidine-4,2'-pyrano[2,3-f]isoindole]-6',8'(7 'H)-dione (compound PROTAC 38)

To a solution of compound P-15 (45 mg, 72.61 μmol) in dichloromethane (2 mL) was added trifluoroacetic acid (0.5 mL) with stirring at room temperature. The reaction was stirred at room temperature for 3 hours, The resulting mixture was concentrated in vacuo, and dissolved by adding N,N-dimethylformamide (1 mL). Diisopropylethylamine (50 mg, 386.86 μmol) was added thereto, and then compound 1 (35 mg, 70.36 μmol) and HATU (20 mg, 52.60 μmol) were added. The reaction was stirred at room temperature for 1.5 hours. Purified by preparative high performance liquid chromatography to obtain yellow solid compound PROTAC 38 (10.0 mg, 15.30%).

$^1$H NMR (600MHz, $CD_3OD$) δ9.06(s, 1H), 8.08-8.02(m, 2H), 7.75(d, *J*=9.5Hz, 1H), 7.64(s, 1H), 7.29(s, 1H),

6.04-5.96(m,1H), 5.11-5.06(m,1H), 4.38(d, *J* = 13.2Hz,1H), 3.99-3.80(m,7H), 3.64-3.50(m,4H), 3.48 -3.37(m,3H), 3.26-3.18(m,1H), 2.99(t,*J* = 6.7Hz,2H),2.91-2.66(m,6H), 2.52(s,3H), 2.43(s,3H), 2.37-2.28(m,2H), 2.14-2.03(m,3H), 1.99-1.85(m,6H), 1.82-1.61(m,5H).

LC-MS (ESI): $[M+H]^+$=929.55.

Example 67

Synthesis of PROTAC 39

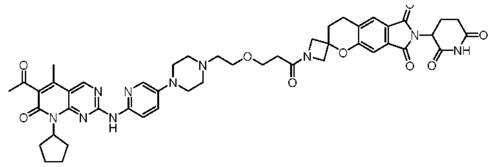

1-(3-(2-(4-(6-((6-acetyl-8-cyclopentyl-5-methyl-7-oxo-7,8-dihydropyridone[2,3 -d]pyrimidin-2-yl)amino)pyridin-3-yl)piperazin-1-yl)ethoxy)propionyl)-7'-(2,6-dioxopiperidin-3-yl)-3',4'-dihydro-6'H-spiro[azetidine-3,2'-pyrano[2,3-f]isoindole]-6',8'(7' H)-dione

Synthesis scheme:

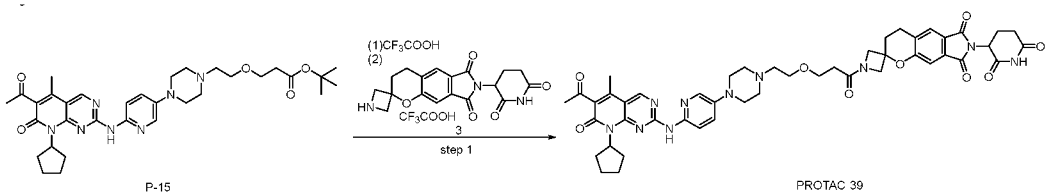


P-15

PROTAC 39

The first step: 1-(3-(2-(4-(6-((6-acetyl-8-cyclopentyl-5-methyl-7-oxo-7,8-dihydropyridone [2,3-d]pyrimidin-2-yl)atnino) pyridin-3-yl)piperazin-1-yl)ethoxy)propionyl)-7'-(2,6-dioxopiperidine- 3-yl)-3',4'-dihydro-6'H-spiro[azetidine-3,2'-pyrano [2,3-f]isoindole]-6',8 '(7'H)-dione (compound PROTAC 39)

To a solution of compound P-15 (45 mg, 72.61 μmol) in dichloromethane (2 mL) was added trifluoroacetic acid (0.5 mL) with stirring at room temperature. The reaction was stirred at room temperature for 3 hours. The resulting mixture was concentrated in vacuo, and dissolved by adding N,N-dimethylformamide (1 mL). Diisopropylethylamine (50 mg, 386.86 μmol) was added thereto, and then compound 3 (35mg, 74.57μmol) and HATU (20mg, 52.60μmol) were added. The reaction was stirred at room temperature for 1.5 hours. Purified by preparative high performance liquid chromatography to obtain yellow solid compound PROTAC 39 (10.0 mg, 14.88%).

$^1H$ NMR (600MHz, $CD_3OD$) δ9.07(s, 1H), 8.11-7.99(m, 2H), 7.74-7.64(m, 2H), 7.28(d, *J*=10.2Hz, 1H), 6.01(dt, *J* = 17.8, 8.8Hz, 1H), 5.10-5.03(m, 1H), 4.39(d, *J* = 1.6Hz, 2H), 4.12(q, *J* = 10.9Hz, 2H), 3.97-3.88(m, 3H), 3.87-3.79(m,3H), 3.53(t,*J* = 4.8Hz,3H), 3.06-3.00(m,2H), 2.90-2.81(m,2H), 2.76-2.61(m,3H), 2.54-2.48(m,4H), 2.44(s,3H),2.37-2.30(m,2H), 2.27(t,*J*=6.4Hz,2H), 2.09(d,*J*=5.3Hz,3H), 1.94-1.87(m,2H), 1.75-1.66(m,2H).

LC-MS (ESI): $[M+H]^+$=901.45.

Example 68

Synthesis of PROTAC 40

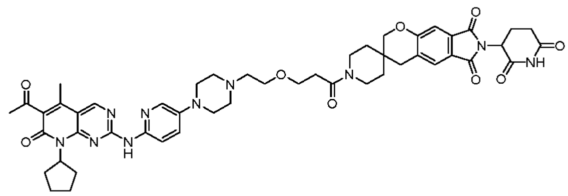

1-(3-(2-(4-(6-((6-acetyl-8-cyclopentyl-5-methyl-7-oxo-7,8-dihydropyridone[2,3 -d]pyrimidin-2-yl)amino)pyridin-3-yl)piperazin-1-yl)ethoxy)propionyl)-7'-(2,6-dioxopiperidin-3-yl)-2'H-spiro[piperidine-4,3'-pyrano[2,3-f]isoindole]-6',8'(4'H,7'H)-dione

Synthesis scheme:

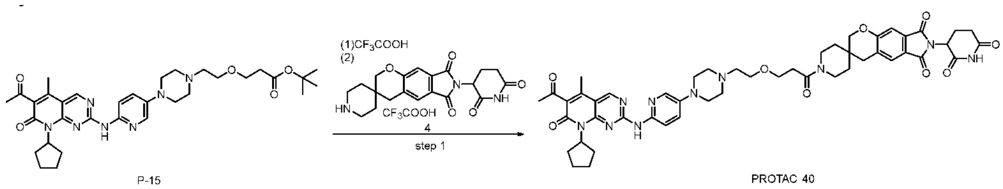


The first step: 1-(3-(2-(4-(6-((6-acetyl-8-cyclopentyl-5-methyl-7-oxo-7,8-dihydropyridone [2,3-d]pyrimidin-2-yl)atnino) pyridin-3-yl)piperazin-1-yl)ethoxy)propionyl)-7'-(2,6-dioxopiperidine- 3-yl)-2'H-spiro[piperidine-4,3'-pyrano[2,3-f]isoindole]-6',8'(4'H,7'H)-dione (compound PROTAC 40)

To a solution of compound P-15 (45 mg, 72.61 μmol) in dichloromethane (2 mL) was added trifluoroacetic acid (0.5 mL) with stirring at room temperature. The reaction was stirred at room temperature for 3 hours. The resulting mixture was concentrated under vacuum, and dissolved by adding N,N-dimethylformamide (1 mL). Diisopropylethylamine (50 mg, 386.86 μmol) was added thereto, and then compound 4 (35mg, 70.36μmol) and HATU (20mg, 52.60μmol) were added. The reaction was stirred at room temperature for 1.5 hours. Purified by preparative high performance liquid chromatography to obtain yellow solid compound PROTAC 40 (10.0 mg, 15.30%).

$^1$H NMR (400MHz, $CD_3OD$) δ9.07(s, 1H), 7.98(s, 2H), 7.62(d, *J*=12.9Hz, 2H), 7.17(s, 1H), 5.99(dt, *J*=17.5, 8.7Hz, 1H), 5.06(dd, *J*= 12.7, 5.3Hz, 1H), 4.09(s, 2H), 3.96-3.89(m, 2H), 3.86-3.79(m, 3H), 3.71-3.51(m ,7H), 2.99(s,3H), 2.91(s,2H), 2.86(s,3H), 2.80-2.69(m,4H), 2.50(s,3H), 2.45-2.38(m,3H), 2.36-2.28(m,2H), 2.13-2.02(m,3H), 1.95-1.84(m,2H), 1.74-1.40(m,7H).

LC-MS (ESI): $[M+H]^+$=929.12.

Example 69

Synthesis of PROTAC 41

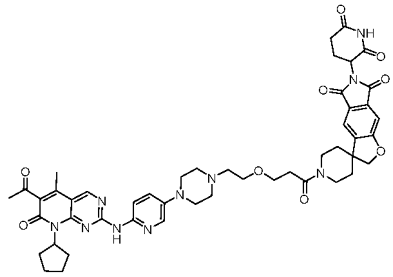

1'-(3-(2-(4-(6-((6-acetyl-8-cyclopentyl-5-methyl-7-oxo-7,8-dihydropyridone[2, 3-d]pyrimidin-2-yl)atnino)pyridin-3-yl)piperazin-1-yl)ethoxy)propionyl)-6-(2,6-dioxopiperidin-3-yl) -6-hydro-2H,5H-spiro[furo[2,3-f]isoindole-3,4'-piperidine]-5,7-dione

Synthesis scheme:

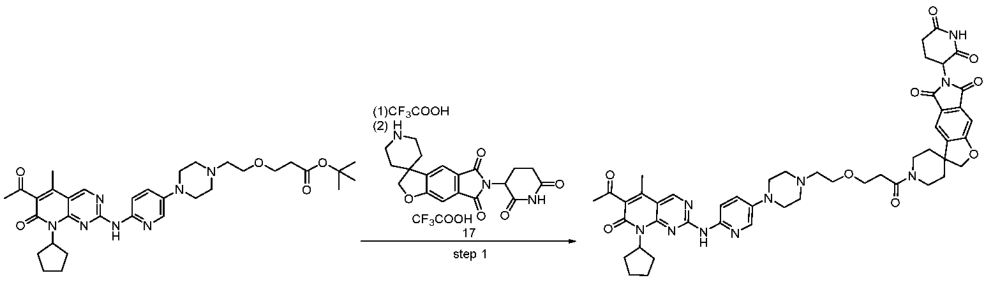


P-15

PROTAC 41

The first step: 1'-(3-(2-(4-(6-((6-acetyl-8-cyclopentyl-5-methyl-7-oxo-7,8-dihydropyridone [2,3-d]pyrimidin-2-yl)atnino) pyridin-3-yl)piperazin-1-yl)ethoxy)propionyl)-6-(2,6-dioxopiperidine- 3-yl)-6-hydro-2H,5H-spiro[furo[2,3-f]isoindole-3,4'-piperidine]-5,7-dione (compound PROTAC 41)

To a solution of compound P-15 (45 mg, 72.61 μmol) in dichloromethane (2 mL) was added trifluoroacetic acid (0.5 mL) with stirring at room temperature. The reaction was stirred at room temperature for 3 hours. The resulting mixture was concentrated in vacuo, and dissolved by adding N,N-dimethylformamide (1 mL). Diisopropylethylamine (50 mg, 386.86 μmol) was added thereto, and then compound 17 (35 mg, 70.36 μmol) and HATU (20 mg, 52.60 μmol) were added. The reaction was stirred at room temperature for 1.5 hours. Purified by preparative high performance liquid chromatography to obtain yellow solid compound PROTAC 41 (10.0 mg, 15.30%).

$^1H$ NMR (600MHz, $CD_3OD$) δ9.05(s, 1H), 8.08-8.01(m, 2H), 7.75-7.69(m, 2H), 7.22(s, 1H), 6.00(dt, *J*=17.8, 8.9 Hz, 1H), 5.13-5.07(m, 1H), 4.75(s, 2H), 4.60(d, *J*= 13.4Hz, 1H), 4.06(d, *J*= 14.1Hz, 1H), 4.01-3.73(m, 8H), 3.57(t, *J*=5.0Hz, 2H), 3.53-3.36(m, 3H), 2.95-2.83(m, 3H), 2.82-2.67(m, 4H), 2.52(d, *J*=4.8 Hz, 3H), 2.43(s, 3H), 2.37-2.28(m, 2H), 2.16-2.01(m, 5H), 1.95-1.83(m, 5H), 1.74-1.66(m, 2H).

LC-MS (ESI): $[M+H]^+$=916.45.

Example 70

Synthesis of PROTAC 42

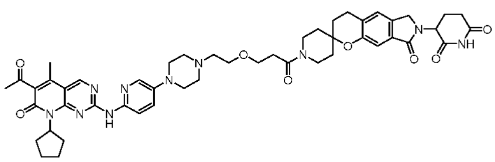

3-(1-(3-(2-(4-(6-((6-acetyl-8-cyclopentyl-5-methyl-7-oxo-7,8-dihydropyridone[2,3-d]pyrimidin-2-yl)atnino)pyridin-3-yl) piperazin-1-yl)ethoxy)propionyl)-8'-oxo-3',4',6', 8'-tetrahydro-7'H-spiro[piperidine-4,2'-pyrano[2,3-f]isoindole]-7'-yl)piperidine-2,6-dione

Synthesis scheme:

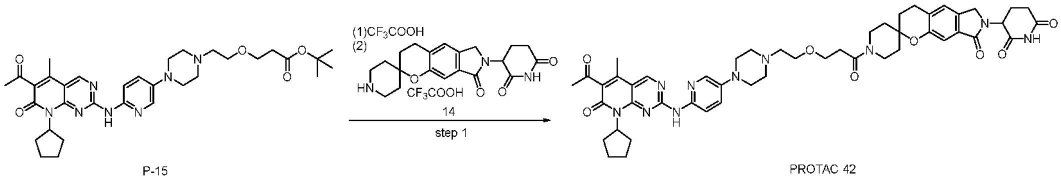


The first step: 3-(1-(3-(2-(4-(6-((6-acetyl-8-cyclopentyl-5-methyl-7-oxo-7,8- dihydropyridone[2,3-d]pyrimidin-2-yl)atnino)pyridin-3-yl)piperazin-1-yl)ethoxy)propionyl)-8'-oxo-3',4' ,6',8'-tetrahydro-7'H-spiro[piperidine-4,2'-pyrano[2,3-f]isoindole]-7'-yl)piperidine-2,6 - dione (compound PROTAC 42)

To a solutionof compound P-15 (45 mg, 72.61 μmol) in dichloromethane (2 mL) was added trifluoroacetic acid (0.5 mL) with stirring at room temperature. The reaction was stirred at room temperature for 3 hours. The resulting mixture was concentrated under vacuum, and dissolved by adding N,N-dimethylformamide (1 mL). Diisopropylethylamine (50 mg, 386.86 μmol) was added thereto, and then compound 14 (30mg, 62.05μmol) and HATU (20mg, 52.60μmol) were added. The reaction was stirred at room temperature for 1.5 hours. Purified by preparative high performance liquid chromatography to obtain yellow solid compound PROTAC 42 (10.0 mg, 15.30%).

$^1$H NMR (600MHz, $CD_3OD$) 89.06(s,1H),8.06-8.00(m,2H),7.77-7.72(m,1H), 7.31(s,1H), 7.23(s,1H), 5.97(td, *J*=17.1,8.7Hz,1H), 5.12(dt, *J*=13.3, 4.7Hz, 1H), 4.44-4.33(m, 3H), 3.97-3.80(m, 8H), 3.61-3.50(m, 4H), 3.48-3.37(m, 2H), 3.24-3.18(m,1H), 2.98-2.72(m,7H), 2.53-2.41(m,7H), 2.36-2.27(m, 2H), 2.23- 2.12(m,1H), 2.07-2.01(m, 2H), 1.96-1.84(m, 6H), 1.78-1.57(m, 5H).

LC-MS (ESI): $[M+H]^+$=916.45.

Example 71

Synthesis of PROTAC 43

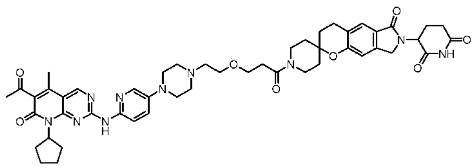

3-(1-(3-(2-(4-(6-((6-acetyl-8-cyclopentyl-5-methyl-7-oxo-7,8-dihydropyridone[2,3-d]pyrimidin-2-yl)atnino)pyridin-3-yl) piperazin-1-yl)ethoxy)propionyl)-6'-oxo-6',8'-dihydro- 3'H,7'H-spiro[piperidine-4,2'-[1,4]dioxino[2,3-f]isoindole]-7'-yl)piperidine-2,6-dione

Synthesis scheme:

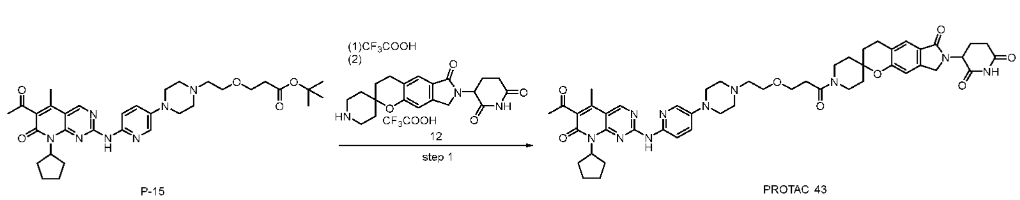


The first step: 3-(1-(3-(2-(4-(6-((6-acetyl-8-cyclopentyl-5-methyl-7-oxo-7,8- dihydropyridone [2,3-d]pyrimidin-2-yl)amino)pyridin-3-yl)piperazin-1-yl)ethoxy)propionyl)-6'-oxo-6',8' -dihydro-3'H,7'H-spiro[piperidin-4,2'-[1,4]dioxino[2,3-f]isoindole]-7'-yl)piperidine -2,6-dione (compound PROTAC 43)

To a solutionof compound P-15 (45 mg, 72.61 μmol) in dichloromethane (2 mL) was added trifluoroacetic acid (0.5 mL) with stirring at room temperature. The reaction was stirred at room temperature for 3 hours. The resulting mixture was concentrated in vacuo, and was dissolved by adding N,N-dimethylformamide (1 mL). Diisopropylethylamine (50 mg,

386.86 μmol) was added thereto, and then compound 12 (30mg, 62.05μmol) and HATU (20mg, 52.60μmol) were added. The reaction was stirred at room temperature for 1.5 hours. Purified by preparative high performance liquid chromatography to obtain yellow solid compound PROTAC 43 (10.0 mg, 15.30%).

$^1$H NMR (600MHz, $CD_3OD$) δ9.05(s, 1H), 8.03(t, *J*=6.9Hz, 2H), 7.78-7.73(m, 1H), 7.53(d, *J*=5.7Hz, 1H), 7.01 (s, 1H),6.03-5.96(m, 1H), 5.11(dd, *J* = 13.3, 5.2Hz,1H), 4.44-4.33(m, 3H), 3.98-3.81(m, 7H), 3.62-3.53 (m, 3H), 3.46-3.35(m, 3H), 3.24-3.17(m, 1H), 2.96-2.88(m, 3H), 2.84-2.75(m, 3H), 2.54-2.41(m, 7H), 2.36-2.28(m, 2H), 2.23-2.12(m, 2H), 2.11-2.02(m, 3H), 1.97-1.84(m, 6H), 1.79-1.58(m, 5H).

LC-MS (ESI): $[M+H]^+$=916.45.

Example 72

Synthesis of PROTAC 44

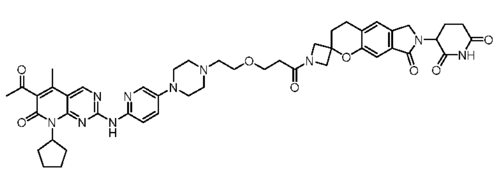

3-(1-(3-(2-(4-(6-((6-acetyl-8-cyclopentyl-5-methyl-7-oxo-7,8-dihydropyridone[2,3-d]pyrimidin-2-yl)atnino)pyridin-3-yl) piperazin-1-yl)ethoxy)propionyl)-8'-oxo-3',4',6', 8'-tetrahydro-7'H-spiro[azetidine-3,2'-pyrano [2,3 -fJisoindole]-7'-yl)piperidine-2,6- dione

Synthesis scheme:

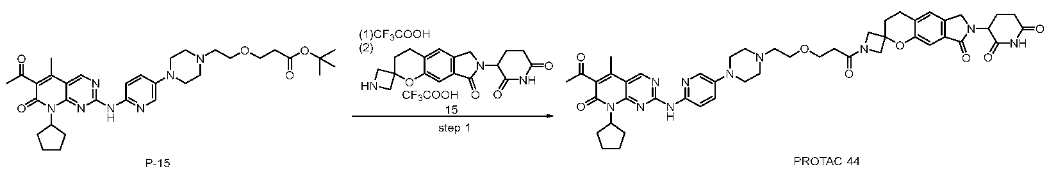


P-15

PROTAC 44

The first step: 3-(1-(3-(2-(4-(6-((6-acetyl-8-cyclopentyl-5-methyl-7-oxo-7,8- dihydropyridone[2,3-d]pyrimidin-2-yl)atnino)pyridin-3-yl)piperazin-1-yl)ethoxy)propionyl)-8'-oxo-3',4' ,6',8'-tetrahydro-7'H-spiro[azetidine-3,2'-pyrano[2,3-f]isoindole]-7'-yl)piperidine- 2,6-dione (compound PROTAC 44)

To a solution of compound P-15 (45 mg, 72.61 μmol) in dichloromethane (2 mL) was added trifluoroacetic acid (0.5 mL) with stirring at room temperature. The reaction was stirred at room temperature for 3 hours. The resulting mixture was concentrated in vacuo, and dissolved by adding N,N-dimethylformamide (1 mL). Diisopropylethylamine (50 mg, 386.86 μmol) was added thereto, and then compound 15 (35mg, 65.88μmol) and HATU (20mg, 52.60μmol) were added. The reaction was stirred at room temperature for 1.5 hours. Purified by preparative high performance liquid chromatography to obtain yellow solid compound PROTAC 44 (10.0 mg, 15.30%).

$^1$H NMR (600MHz, $CD_3OD$) δ9.06(s, 1H), 8.06-8.01(m, 2H), 7.71(t, *J*=9.5Hz, 1H), 7.33(d, *J*=2.8Hz, 1H), 7.23 (d, *J*=6.1Hz,1H), 6.03-5.95(m,1H), 5.09(dt,*J*=13.2,5.4Hz, 1H), 4.45-4.32(m, 4H), 4.13-4.03(m, 2H), 3.96-3.78(m, 6H), 3.57-3.50(m, 3H), 3.48-3.37(m, 2H), 3.01(t, *J*= 5.6Hz, 2H), 2.91-2.83(m, 1H), 2.81-2.74(m, 1H), 2.69-2.62(m, 1H), 2.54-2.42(m, 8H), 2.36-2.20(m, 5H), 2.18-2.02(m, 4H), 1.94-1.85(m, 2H), 1.73-1.60(m, 3H).

LC-MS (ESI): $[M+H]^+$=888.46.

Example 73

Synthesis of PROTAC 45

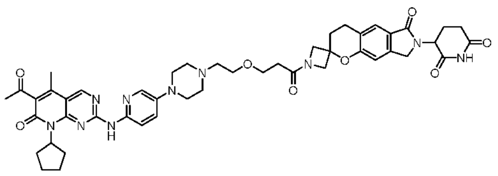

3-(1-(3-(2-(4-(6-((6-acetyl-8-cyclopentyl-5-methyl-7-oxo-7,8-dihydropyridone[2,3-d]pyrimidin-2-yl)amino)pyridin-3-yl) piperazin-1-yl)ethoxy)propionyl)-6'-oxo-3',4',6', 8'-tetrahydro-7'H-spiro[azetidine-3,2'-pyrano [2,3 -fJisoindole]-7'-yl)piperidine-2,6- dione

Synthesis scheme:

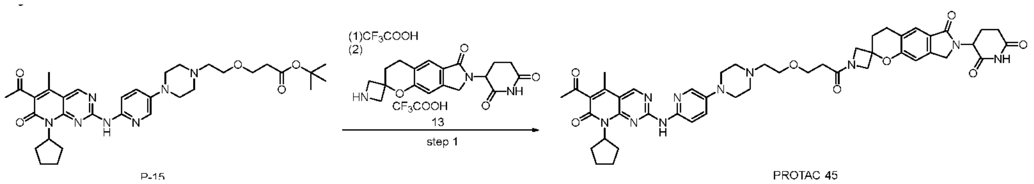


P-15

PROTAC 45

The first step: 3-(1-(3-(2-(4-(6-((6-acetyl-8-cyclopentyl-5-methyl-7-oxo-7,8- dihydropyridone [2,3-d]pyrimidin-2-yl)amino)pyridin-3-yl)piperazin-1-yl)ethoxy)propionyl)-6'-oxo-3',4' ,6',8'-tetrahydro-7'H-spiro[azetidine-3,2'-pyrano[2,3-f]isoindole]-7'-yl)piperidine- 2,6-dione (compound PROTAC 45)

To a solutionof compound P-15 (45 mg, 72.61 μmol) in dichloromethane (2 mL) was added trifluoroacetic acid (0.5 mL) with stirring at room temperature. The reaction was stirred at room temperature for 3 hours. The resulting mixture was concentrated in vacuo, and dissolved by adding N,N-dimethylformamide (1 mL). Diisopropylethylamine (50 mg, 386.86 μmol) was added thereto, and then compound 13 (30mg, 65.88μmol) and HATU (20mg, 52.60μmol) were added. The reaction was stirred at room temperature for 1.5 hours. Purified by preparative high performance liquid chromatography to obtain yellow solid compound PROTAC 45 (10.0 mg, 15.30%).

$^1$H NMR (600MHz, $CD_3OD$) δ9.05(s, 1H), 8.05-7.97(m, 2H), 7.75(t, J--10.1Hz, 1H), 7.55(s, 1H), 7.01(s, 1H), 6.04-5.96(m,1H), 5.13-5.07(m,1H), 4.43-4.31(m,4H), 4.11(d, *J*= 10.7Hz,1H), 4.07(d,*J* = 10.7Hz,1H ),3.96-3.80(m, 6H),3.57-3.48(m, 3H),3.26-3.20(m, 2H),2.99(t,*J*= 6.4Hz, 2H), 2.94-2.84(m,1H), 2.80-2.73(m, 1H), 2.66-2.59(m, 1H), 2.56-2.49(m, 4H), 2.49-2.39(m, 4H), 2.37-2.19(m, 5H), 2.17-2.03(m ,4H), 1.96-1.85(m, 2H) ,1.74-1.59(m, 3H).

LC-MS (ESI): $[M+H]^+$=888.46.

Example 74

Synthesis of PROTAC 46

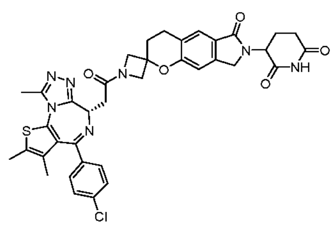

3-(1-(2-((S)-4-(4-chlorophenyl)-2,3,9-trimethyl-6H-thieno[3,2-f][1,2,4 ]triazolo[4,3-a][1,4]diazepine-6-yl)acetyl)-6'-oxo-3',4',6',8'-tetrahydro -7'H-spiro[azetidine-3,2'-pyrano[2,3-f]isoindole]-7'-yl)piperidine-2,6-dione

Synthesis scheme:

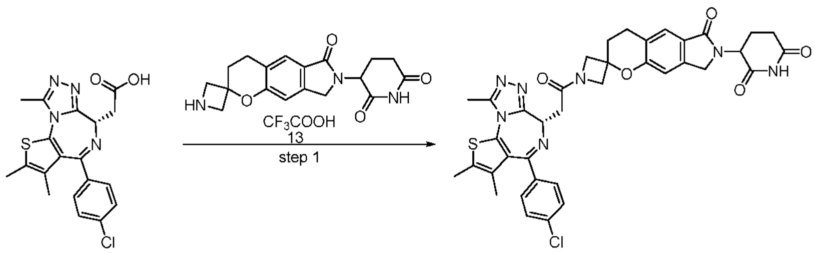


P-16 PROTAC 46

The first step: 3-(1-(2-((S)-4-(4-chlorophenyl)-2,3,9-trimethyl-6H-thieno[3,2-f][1,2,4]triazolo[4,3-a][1,4]diazepine-6-yl) acetyl)-6'-oxo-3',4',6',8'-tetrahydro-7'H-spiro[azetidine-3,2'-pyrano[2,3-f]isoindole]-7'-yl)piperidine-2,6-dione (compound PROTAC 46)

To a solution of compound 13 (15 mg, 32.94 μmol) and diisopropylethylamine (13 mg, 100.58 μmol) in N,N-dimethylformamide (1 mL) at room temperature, was added compound P-16 and HATU ( 15mg, 39.45μmol). The reaction was stirred at room temperature for 1.5 hours. Purified by preparative high performance liquid chromatography to obtain white solid compound PROTAC 46 (8.4 mg, 35.21%).

$^1$H NMR (600MHz, $CD_3OD$) δ7.60 (d, *J* = 3.0Hz, 1H), 7.52-7.44 (m, 4H), 7.10 (d, *J* = 7.4Hz, 1H), 5.13 (dd, *J* = 13.0, 4.6Hz, 1H), 4.71-4.64(m, 2H), 4.58-4.54(m, 1H), 4.51-4.39(m, 2H),4.21-4.11(m, 2H), 3.48-3.35(m, 2H), 3.08-3.02(m, 2H), 2.96-2.87(m, 1H), 2.83-2.77(m, 1H), 2.73(s,3H), 2.54-2.48(m, 1H), 2.47(s, 3H), 2.40-2.27(m, 2H), 2.21-2.14(m, 1H), 1.72(d, *J*=5.0Hz, 3H).

LC-MS(ESI): $[M+H]^+$=724.10/726.05.

Example 75

Synthesis of PROTAC 47

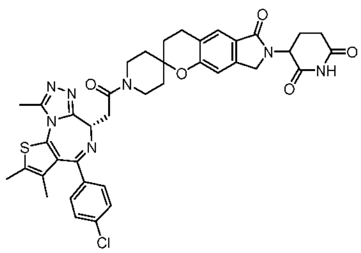

3-(1-(2-((S)-4-(4-chlorophenyl)-2,3,9-trimethyl-6H-thieno[3,2-f][1,2,4]triazolo[4,3-a][1,4]diazepine-6-yl)acetyl)-6'-oxo-3',4',6',8'-tetrahydro-7'H-spiro[piperidine-4,2'-pyrano[2,3-f]isoindole]-7'-yl)piperidine-2,6-dione

Synthesis scheme:

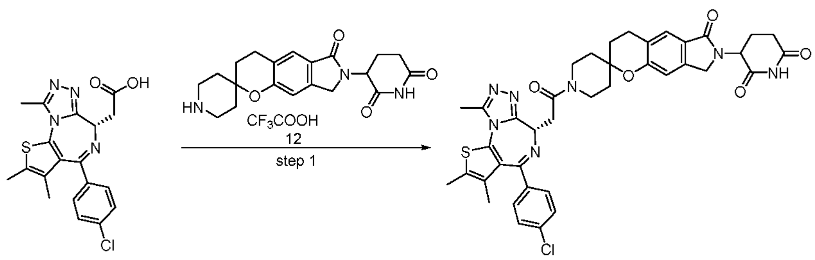


P-16 PROTAC 47

The first step: 3-(1-(2-((S)-4-(4-chlorophenyl)-2,3,9-trimethyl-6H-thieno[3,2-f][1,2,4]triazolo[4,3-a][1,4]diazepine-6-yl) acetyl)-6'-oxo-3',4',6',8'-tetrahydro-7'H-spiro[piperidine-4,2'-pyrano[2,3-f]isoindol]-7'-yl)piperidine-2,6-dione (compound PROTAC 47)

To a solution of compound 12 (15 mg, 31.03 μmol) and diisopropylethylamine (13 mg, 100.58 μmol) in N,N-dimethylformamide (1 mL) at room temperature, was added compound P-16 (13 mg, 32.43 μmol) and HATU (15 mg, 39.45 μmol). The reaction was stirred at room temperature for 1.5 hours. Purified by preparative high-performance liquid chromatography to obtain PROTAC 47 (10.20 mg, 43.70%) as a light yellow solid.

$^1$H NMR (600MHz, $CD_3OD$) δ7.58 (d, *J* = 11.5Hz, 1H), 7.52-7.46 (m, 4H), 7.08 (d, *J* = 3.9Hz, 1H), 5.13 (dd, *J* = 13.3, 5.2Hz, 1H), 4.80(t, *J*=6.9Hz, 1H), 4.46(dd, *J*=17.0, 8.7Hz, 1H), 4.40(dd, *J*=20.5, 6.0Hz, 2H), 4.11(t, *J* = 14.8Hz, 1H), 3.76-3.63(m, 3H), 3.26(dd, *J* = 17.6, 8.4Hz, 1H), 2.99(dd, *J* = 11.9, 5.9Hz, 2H), 2.96-2.88(m, 1H), 2.83-2.75(m, 4H), 2.54-2.45(m, 4H), 2.20-2.15(m, 1H), 2.06-1.83(m, 5H), 1.76-1.65(m, 4H).

LC-MS (ESI): $[M+H]^+$=752.12/754.01.

Example 76

Synthesis of PROTAC 48

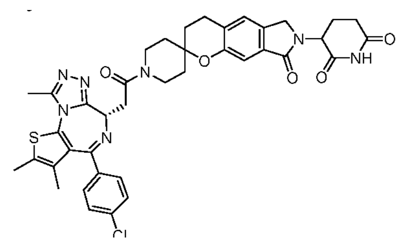

3-(1-(2-((S)-4-(4-chlorophenyl)-2,3,9-trimethyl-6H-thieno[3,2-f][1,2,4]triazolo[4,3-a][1,4]diazepine-6-yl)acetyl)-8'-oxo-3',4',6',8'-tetrahydro -7'H-spiro[piperidine-4,2'-pyrano[2,3-f]isoindole]-7'-yl)piperidine-2,6-dione

Synthesis scheme:

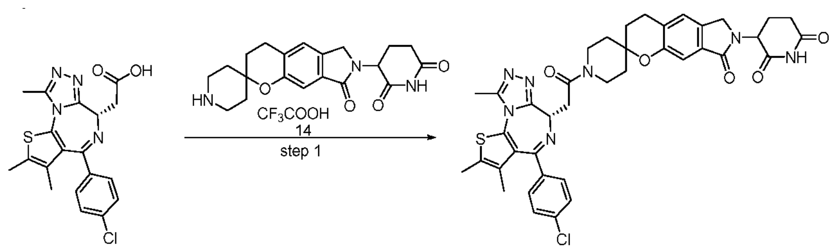


P-16

PROTAC 48

The first step: 3-(1-(2-((S)-4-(4-chlorophenyl)-2,3,9-tnmethyl-6H-thieno[3,2-f][1,2,4]triazolo[4,3-a][1,4]diazepine-6-yl) acetyl)-8'-oxo-3',4',6',8'-tetrahydro-7'H-spiro[piperidine-4,2'-pyrano[2,3-f]isoindol]-7'-yl)piperidine-2,6-dione (compound PROTAC 48)

To a solution of compound 14 (30 mg, 62.05 μmol) and diisopropylethylamine (25 mg, 193.43 μmol) in N,N-dimethylformamide (1 mL) at room temperature, was added compound P-16 (25 mg, 62.36 μmol) and HATU (29 mg, 76.26 μmol). The reaction was stirred at room temperature for 1.5 hours. Purified by preparative high performance liquid chromatography to obtain light yellow solid compound PROTAC 48 (13.2 mg, 28.28%) .

$^1$H NMR (600MHz, $CD_3OD$) δ7.52-7.45 (m, 4H), 7.34 (s, 1H), 7.29 (d, *J* = 6.0Hz, 1H), 5.15 (dd, *J* = 13.3, 4.8Hz, 1H), 4.79 (t, *J*=6.9Hz, 1H), 4.44(d, *J*=16.4Hz, 1H), 4.41-4.35(m, 2H), 4.14-4.05(m, 1H), 3.72(dd, *J*=16.4, 7.8Hz, 2H), 3.69-3.61(m, 1H), 3.27(t, *J* = 11.5Hz, 1H), 3.00(dd, *J* = 11.6, 6.2Hz, 2H), 2.97-2.88(m, 1H), 2.80(d, *J*=18.0Hz, 1H), 2.77(d, *J*=3.2Hz, 3H), 2.54-2.46(m, 4H), 2.22-2.15(m, 1H), 2.04-1.83(m, 5H), 1.73(s, 3H), 1.72-1.62(m, 1H).

LC-MS(ESI): $[M+H]^+$=752.17/754.27.

Example 77

Synthesis of PROTAC 49

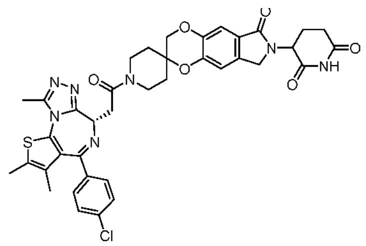

3-(1-(2-((S)-4-(4-chlorophenyl)-2,3,9-trimethyl-6H-thieno[3,2-f][1,2,4]triazolo[4,3-a][1,4]diazepine-6-yl)acetyl)-6'-oxo-6',8'-dihydro-3'H,7'H-spiro[piperidin-4,2'-[1,4]dioxino[2,3-f]isoindole]-7'-yl)piperidine-2,6-dione

Synthesis scheme:

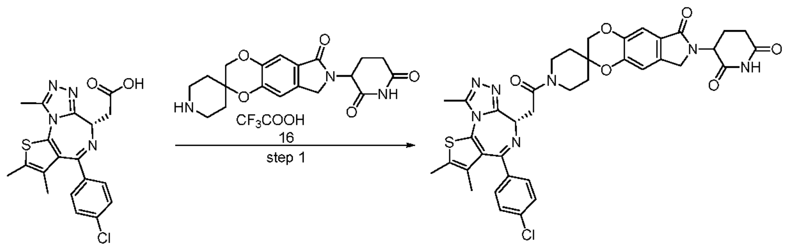


P-16

PROTAC 49

The first step: 3-(1-(2-((S)-4-(4-chlorophenyl)-2,3,9-trimethyl-6H-thieno[3,2-f][1,2,4]triazolo[4,3-a][1,4]diazepine-6-yl) acetyl)-6'-oxo-6',8'-dihydro-3'H,7'H-spiro[piperidine-4,2'-[1,4]dioxino[2,3-f]isoindole]-7'-yl)piperidine-2,6-dione (compound PROTAC 49)

To a solution (1 mL) of compound 16 (15 mg, 30.90 μmol) and diisopropylethylamine (12 mg, 92.85 μmol) in N,N-dimethylformamide at room temperature, was added compound P-16 (13 mg, 32.43 μmol) and HATU (15 mg, 39.45 μmol). The reaction was stirred at room temperature for 1.5 hours. Purified by preparative high performance liquid chromatography to obtain white solid compound PROTAC 49 (3.5 mg, 15.02%).

$^1H$ NMR (600MHz, $CD_3OD$) δ7.50-7.44 (m, 4H), 7.33 (s, 1H), 7.17 (d, *J* = 3.7Hz, 1H), 5.13 (dd, *J* = 13.3, 5.1Hz, 1H) ,4.76(t, *J* = 6.9Hz, 1H), 4.46-4.34(m, 2H),4.19-4.08(m, 2H), 3.76-3.64(m, 3H), 3.31-3.25(m, 1H), 2.96-2.88(m, 1H), 2.84-2.76(m, 1H), 2.74(s, 3H),2.54-2.49(m, 1H), 2.47(d, *J* = 9.6Hz, 3H), 2.21-2.15(m, 1H), 2.05(d, *J* = 5.2Hz, 3H), 2.04-1.86(m, 2H), 1.81-1.75(m, 1H), 1.73(s, 3H).

LC-MS(ESI): $[M+H]^+$=754.11/755.98.

Example 78

Synthesis of PROTAC 50

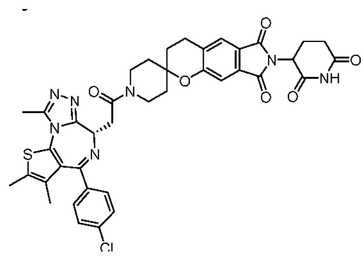

1-(2-((S)-4-(4-chlorophenyl)-2,3,9-trimethyl-6H-thieno[3,2-f][1,2,4]triazole[4,3-a][1,4]diazepine-6-yl)acetyl)-7'-(2,6-dioxopiperidin-3-yl)-3',4'-dihydro-6'H-spiro[piperidine-4,2'-pyrano[2,3-f]isoindole]-6',8'(7'H)-dione

Synthesis scheme:

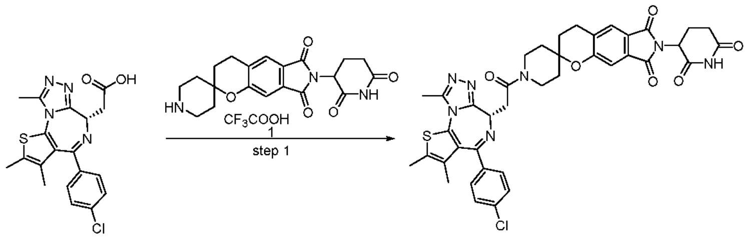


P-16

PROTAC 50

The first step: 1-(2-((S)-4-(4-chlorophenyl)-2,3,9-trimethyl-6H-thieno[3,2-f][1,2,4] triazolo[4,3-a][1,4]diazepine-6-yl) acetyl)-7'-(2,6-dioxopiperidin-3-yl)- 3',4'-dihydro-6'H-spiro[piperidine-4,2'-pyrano[2,3-f]isoindole]-6',8'(7'H)-dione (compound PROTAC50)

To a solution of compound 1 (15 mg, 30.90 μmol) and diisopropylethylamine (12 mg, 92.8 μmol) in N,N-dimethylformamide (1 mL) at room temperature, was added compound P-16 (13 mg, 32.43 μmol) and HATU (15 mg, 39.45 μmol). The reaction was stirred at room temperature for 1.5 hours. Purified by preparative high performance liquid chromatography to obtain white solid compound PROTAC 50 (3.5 mg, 15.02%).

$^1$H NMR (400MHz, $CD_3OD$) δ7.69 (s, 1H), 7.54-7.46 (m, 4H), 7.36 (d, *J* = 2.5Hz, 1H), 5.11 (dd, *J* = 12.6, 5.4Hz, 1H) ,4.84(t, *J*=7.0Hz, 1H),4.39(d, *J*=9.1Hz, 1H),4.11(t, *J*=12.8Hz, 1H), 3.79-3.67(m, 4H), 3.04(d, *J* = 2.7Hz, 2H), 2.91-2.83(m, 1H), 2.80(t, *J* = 3.0Hz, 4H), 2.74(dd, *J* = 13.8, 4.0Hz, 1H), 2.50(s, 3H), 2.18-2.08(m, 1H),2.04-1.89(m, 5H),1.77-1.70(m, 4H).

LC-MS(ESI): $[M+H]^+$=766.26/768.26.

Example 79

Synthesis of PROTAC 51

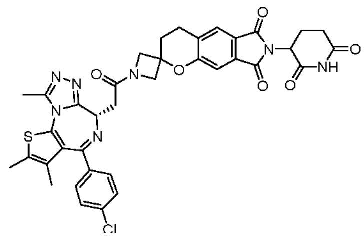

1-(2-((S)-4-(4-chlorophenyl)-2,3,9-trimethyl-6H-thieno[3,2-f][1,2,4]triazole[4,3-a][1,4]diazepine-6-yl)acetyl)-7'-(2,6-dioxopiperidin-3-yl)-3',4 '-dihydro-6'H-spiro[azetidine-3,2'-pyrano[2,3-f]isoindole]-6',8'(7'H)-dione

Synthesis scheme:

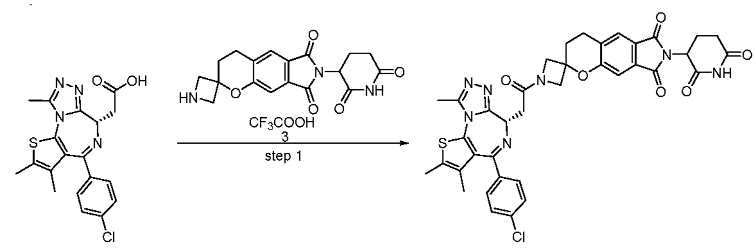


P-16 PROTAC 51

The first step: 1-(2-((S)-4-(4-chlorophenyl)-2,3,9-trimethyl-6H-thieno[3,2-f][1,2,4] triazolo[4,3-a][1,4]diazepine-6-yl) acetyl)-7'-(2,6-dioxopiperidin-3-yl)- 3',4'-dihydro-6'H-spiro[azetidine-3,2'-pyrano[2,3-f]isoindole]-6',8'(7'H )-dione (compound PROTAC 51)

To a solutionof compound 1 (15 mg, 30.90 μmol) and diisopropylethylamine (12 mg, 92.8 μmol) in N,N-dimethylformamide (1 mL) at room temperature, was added compound P-16 (13 mg, 32.43 μmol) and HATU (15 mg, 39.45 μmol). The reaction was stirred at room temperature for 1.5 hours. Purified by preparative high performance liquid chromatography to obtain white solid compound PROTAC 51 (3.5 mg, 15.02%).

$^1$H NMR (400MHz, $CD_3OD$) δ7.71(d, *J*=1.7Hz, 1H), 7.54-7.45(m, 4H), 7.37(d, *J*=4.7Hz, 1H), 5.12(dd, *J*=12.4, 5.4Hz, 1H), 4.76-4.70(m, 1H), 4.70-4.54(m, 2H), 4.23-4.14(m, 2H), 3.47-3.37(m, 2H), 3.10(t, *J* = 6.5Hz, 2H), 2.94-2.83(m, 1H), 2.81-2.70(m, 5H), 2.48(s, 3H), 2.40-2.29(m, 2H), 2.18-2.10(m, 1H), 1.73(d ,*J*=2.2Hz,3H).

LC-MS(ESI): $[M+H]^+$=738.27/740.27.

Example 80

Synthesis of PROTAC 52

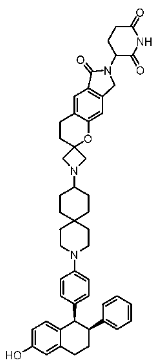

3-1-(3-(4-((1R,2S)-6-hydroxy-2-phenyl-1,2,3,4-tetrahydronaphthalen-1-yl)phenyl)-3-azaspiro [5.5]undec-9-yl)-6'-oxo-3',4',6',8'-tetrahydro-7'H-spiro[azetidine-3,2'-pyrano[2,3]isoindol]-7'-yl)piperidine-2,6-dione

Synthesis scheme:

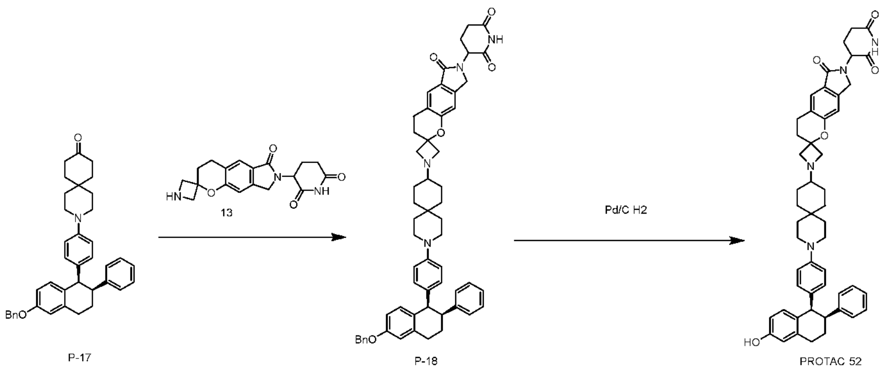


The first step: 3-(1-(3-(4-((1R,2S)-6-(benzyloxy)-2-phenyl-1,2,3,4-tetrahydronaphthalene-1-yl )phenyl)-3-azaspiro[5.5] undecane-9-yl)-6'-oxo-3',4',6',8'-tetrahydro-7'H-spiro[azetidine -3,2'-pyrano[2,3]isoindol]-7'-yl)piperidine-2,6-dione (compound P-18)

Under the condition of nitrogen protection, 3-(4-((1R,2S)-6-(benzyloxy)-2-phenyl-1,2,3,4-tetrahydronaphthalene-1- yl) phenyl)-3-azaspiro[5.5]undecane-9-one (120mg, 0.21mmol), 3mL DCM, 3mL DMF and TEA (0.5ml) were added to the reaction flask in order, after stirring at room temperature for half an hour. $NaBH_3CN$ (40mg , 0.63mmol) and compound 13 (88mg, 0.21mmol) were added, heated to 50°C for 12 hours. The reaction solution was prepared by reverse preparation to obtain white solid P-18 (100 mg, 54%).

$^1$H NMR(400MHz, DMSO-$d_6$)δ10.96(s, 1H), 7.55-7.30(m, 6H), 7.20-7.02(m, 4H), 6.93-6.66(m, 7H), 6.32(s, 2H), 5.08(s, 2H),4.50-4.31(m, 4H),4.25(d, *J* = 4.0Hz, 3H),3.39-3.30(m, 2H),3.09(d, *J* = 23.4Hz, 5H), 2.95-2.83(m, 3H), 2.65-2.55(m, 1H), 2.42-2.27(m, 2H), 2.25-2.05(m, 3H), 2.02-1.94(m, 1H), 1.78(d, *J* = 19.7Hz, 5H), 1.53(d, *J* = 44.4Hz, 4H), 1.39-1.21(m, 3H), 1.11(t, *J* = 7.3Hz, 2H).
LC-MS (ESI): $[M+H]^+$=881.46

The second step: 3-(1-(3-(4-((1R,2S)-6-hydroxy-2-phenyl-1,2,3,4-tetrahydronaphthalen-1-yl)phenyl) -3-azaspiro[5.5] undec-9-yl)-6'-oxo-3',4',6',8'-tetrahydro-7'H-spiro[azetidine -3,2'-pyrano[2,3]isoindol]-7'-yl)piperidine-2,6-dione (compound PROTAC 52)

Under the condition of nitrogen protection, compound P-18 (100mg, 0.11mmol)was dissolved in 5mL methanol solution, 10% Pd/C (20mg) was added, after being replaced with hydrogen three times, and heated to 50°C under hydrogen atmosphere for 16 hours. After the reaction was completed, the reaction solution was prepared by reverse preparation to obtain PROTAC 52 (70 mg, 80%) as a white solid.

$^1$H NMR(400MHz, DMSO-$d_6$)δ10.96(s, 1H), 9.15(s, 1H), 7.53(s, 1H), 7.22-7.00(m, 4H), 6.94-6.70(m, 4H), 6.67-6.59(m, 2H), 6.48(dd, *J*=8.3, 2.6Hz, 1H), 6.31(d, *J*=8.1Hz, 2H), 5.06(dd, *J*=13.2, 5.1Hz, 1H), 4.44-4.16(m, 7H), 3.38-3.30(m, 1H), 3.17(d, *J* = 41.0Hz, 4H), 3.01-2.82(m, 5H), 2.66-2.55(m, 1H), 2.42-2.30 (m, 1H), 2.25-1.95(m, 4H), 1.89-1.66(m, 5H), 1.54(d, *J* = 45.2Hz, 4H), 1.39-1.25(m, 2H), 1.11(dd,*J* =11.3, 9.6Hz, 3H).
LC-MS (ESI): $[M+H]^+$=791.41

Example 81

Synthesis of PROTAC 53

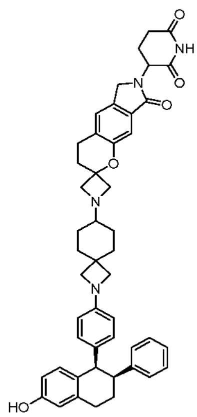

3-(1-(2-(4-((1R,2S)-6-hydroxy-2-phenyl-1,2,3,4-tetrahydronaphthalen-1-yl)phenyl)-2- azaspiro [3.5]non-7-yl)-8'-oxo-3',4',6',8'-tetrahydro-7'H-spiro[azetidine-3,2'- pyrano[2,3]isoindol]-7'-yl]piperidine-2,6-dione

Synthesis scheme:

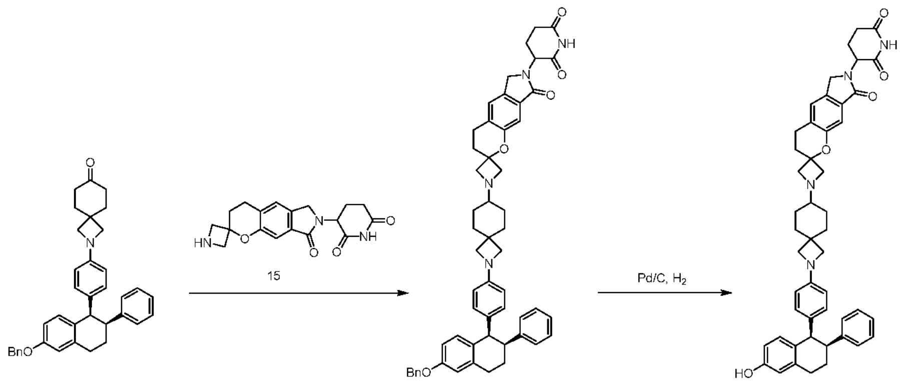


P-19 PROTAC 53

The first step: 3-(1-(2-(4-((1R, 2S)-6-(benzyloxy)-2-phenyl-1,2,3,4-tetrahydronaphthalene-1-yl )phenyl)-2-azaspiro[3.5] non-7-yl)-8'-oxo-3',4',6',8'-tetrahydro-7'H-spiro[azetidine -3,2'-pyrano[2,3]isoindol]-7'-yl)piperidine-2,6-dione (compound P-19)

Under the condition of nitrogen protection, 2-(4-(6-(benzyloxy)-2-phenyl-3,4-dihydronaphthalen-1-yl)phenyl)-2-azaspiro[3.5]non-7-one (60mg, 0.11mmol) and compound 15 (38.8mg, 0.11mmol) were dissolved in DCE:DMF=2:1 (6mL). 3 drops of acetic acid (catalytic amount) were added, stirred at room temperature for 1h. And sodium cyanoborohydride (21.5mg, 0.33mmol) was added to the above reaction solution, and the reaction was carried out at room temperature for 2h. Purified by preparative HPLC to obtain compound P-19 (20 mg, 20.6%).

$^1$H NMR (600MHz, DMSO-$d_6$) $\delta$10.98 (d, *J* = 2.3Hz, 1H), 7.46 (d, *J* = 7.0Hz, 2H), 7.43-7.37 (m, 3H), 7.35 (d, *J* =7.3Hz, 1H), 7.18-7.08(m, 4H),6.89-6.82(m, 3H),6.77-6.70(m, 2H),6.18(d, *J* = 8.2Hz, 2H),6.06(d, *J* =8.1Hz, 2H),5.08(s, 3H),4.44-4.39(m, 1H),4.34(td, *J*=11.5, 10.7, 5.3Hz, 2H),4.25-4.16(m, 3H),3.42-3.37(m, 4H), 3.33-3.27(m, 2H), 3.04(d, *J* = 5.7Hz, 1H), 3.01-2.83(m, 5H), 2.63-2.56(m, 1H), 2.40-2.35(m, 1H), 2.15(ddt, *J* = 31.6, 12.8, 6.9Hz, 3H), 1.99-1.92(m, 3H), 1.87-1.82(m, 1H), 1.74(ddt, *J* = 12.3, 6.1Hz, 1H), 1.46(dt, *J*=24.2, 13.1Hz, 3H), 1.18(t, *J*=11.8Hz, 2H).

LC-MS(ESI): $[M+H]^+$=853.43.

The second step: 3-(1-(2-(4-((1R,2S)-6-hydroxy-2-phenyl-1,2,3,4-tetrahydronaphthalen-1-yl)phenyl) -2-azaspiro[3.5] non-7-yl)-8'-oxo-3',4',6',8'-tetrahydro-7'H-spiro[azetidine-3 ,2'-pyrano[2,3]isoindol]-7'-yl]piperidine-2,6-dione (compound PROTAC 53)

Under the condition of nitrogen protection, compound P-19 (25mg, 1eq)was dissolved in methanol (5mL), 10% Pd/C (5mg, 20%wt) was added. After three replacements with hydrogen, heated to 50°C under hydrogen atmosphere for

16 hours. After the reaction was completed, the reaction solution was prepared by reverse preparation to obtain white solid PROTAC 53 (3.5 mg, 19.6%) .

$^1$H NMR(400MHz, DMSO-$d_6$)δ10.97(s, 1H), 9.10(s, 1H), 7.54(s, 1H), 7.19-7.03(m, 4H), 6.89-6.80(m, 2H), 6.65-6.57(m, 2H), 6.48(dd, *J*=8.3, 2.5Hz, 1H), 6.18(d, *J*=8.2Hz, 2H), 6.05(d, *J*=8.3Hz, 2H), 5.09-5.04(m, 1H), 4.47-4.32(m, 4H), 4.27-4.19(m, 2H), 4.12(d, *J* = 4.9Hz, 1H), 3.43-3.37(m, 3H), 3.28(d, *J* =12.5Hz, 2H), 3.01-2.83(m, 5H), 2.69-2.57(m, 2H), 2.42-2.31(m, 2H), 2.18(t, *J* = 6.2Hz, 2H),2.09(dd, *J*=12.4, 6.4Hz, 1H), 1.96(d, *J*=14.0Hz, 3H), 1.85(d, *J*=11.4Hz, 1H), 1.70(d, *J*=11.9Hz, 1H),1.51-1.41(m, 2H), 1.17(d, *J*=12.2Hz, 2H).

LC-MS (ESI): [M+H]$^+$=763.38.

Example 82

Synthesis of PROTAC 54

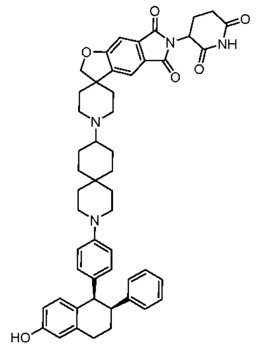

6-(2,6-dioxopiperidin-3-yl)-1'-(3-(4-((1R,2S)-6-hydroxy-2-phenyl-1,2,3,4 -tetralin-1-yl)phenyl)-3-azaspiro[5.5]undec-9-yl)-6-hydrogen-2H,5H-spiro[furo[2,3-f]isoindole-3,4'-piperidine]-5,7-dione

Synthesis scheme:

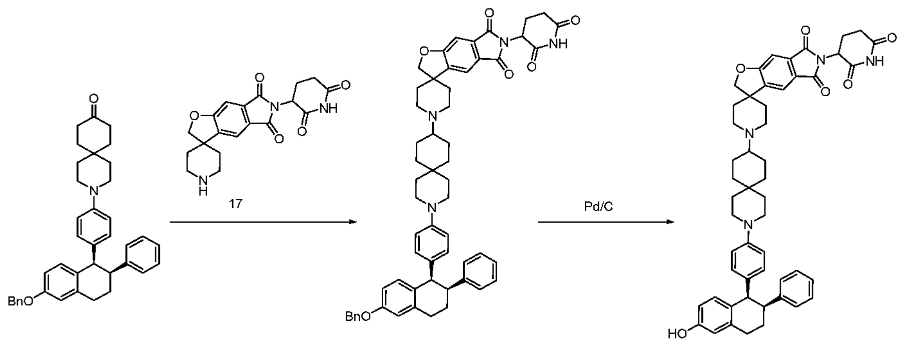


P-20

PROTAC 54

The first step: 1'-(3-(4-((1R,2S)-6-(benzyloxy)-2-phenyl-1,2,3,4-tetrahydronaphthalen-1-yl) phenyl)-3-azaspiro[5.5]undecane-9-yl)-6-(2,6-dioxopiperidin-3-yl)-6-hydrogen-2H,5H-spiro[furo[2,3-f]isoindole-3,4'-piperidine]-5,7-dione (compound P-20)

Under the condition of nitrogen protection, 3-(4-((1R,2S)-6-(benzyloxy)-2-phenyl-1,2,3,4-tetrahydronaphthalene-1- yl) phenyl)-3-azaspiro[5.5]undecane-9-one (90mg, 0.16mmol), 3mL DCM, 3mL DMF and TEA (0.5ml) were added to the reaction flask in order. After stirring at room temperature for half an hour, $NaBH_3CN$ (30mg , 0.49mmol) and compound 17 (88mg, 0.21mmol) were added, heated to 50°C 12 hours. White solid compound P-20 (100 mg, 52%) was obtained by reverse preparation of the reaction solution.

$^1$H NMR (400MHz, DMSO-$d_6$) δ11.11(s, 1H), 7.66(s, 1H), 7.48-7.29(m, 6H), 7.11(t, *J*=7.4Hz, 2H), 7.07-6.98(m,3H), 6.95-6.82(m, 5H), 6.72(dd, *J*=8.6, 2.7Hz, 1H), 6.56(dd, *J*=8.6, 2.8Hz, 1H), 5.09(s, 2H), 4.73 (d,

*J*=10.6Hz, 2H), 3.46(d, *J*=11.8Hz, 2H), 3.14(d, *J*=36.2Hz, 5H), 2.99-2.79(m, 8H), 2.72-2.57(m, 6H), 2.22(dd, *J* = 19.5, 11.3Hz, 4H), 2.11-1.93(m, 5H), 1.80(s, 1H), 1.75-1.50(m, 4H), 0.87(t, *J* = 7.3Hz ,1H).

LC-MS (ESI): $[M+H]^+$=909.45.

The second step: 6-(2,6-dioxopiperidin-3-yl)-1'-(3-(4-((1R,2S)-6-hydroxy-2-phenyl-1,2,3,4-tetrahydronaphthalen-1-yl)phenyl)-3-azaspiro[5.5]undecane-9-yl)-6-hydrogen-2H,5H-spiro[furo[2,3-f]isoindole-3,4'-piperidine]-5,7-dione (compound PROTAC 54)

Under the condition of nitrogen protection, compound P-20 (30mg, 0.03mmol) was dissolved in methanol (5mL), and 10% Pd/C (6mg, 20%wt) was added. After being replaced with hydrogen three times, the reaction was heated to 50°C under hydrogen atmosphere for 16 hours. After the reaction was completed, the reaction solution was prepared in reverse to obtain PROTAC 54 (6.5 mg, 26.6%) as a white solid.

$^1H$ NMR(600MHz, DMSO-$d_6$)$\delta$11.12(s, 1H), 9.80(s, 1H), 8.24(s, 1H), 7.65(s, 1H), 7.36(d, *J* = 13.7Hz, 1H), 7.16(ddt, *J*=19.8, 12.5, 6.7Hz, 3H), 6.84(d, *J*=7.2Hz, 2H),6.73-6.56(m, 3H), 6.49(dd, *J*=8.3, 2.5Hz, 1H), 6.26(d, *J*=8.1Hz, 2H), 5.12(ddd, *J*=12.9, 7.7, 5.3Hz, 1H), 4.72(d, *J*=13.8Hz, 2H), 4.17(d, *J*=5.0Hz, 1H), 3.46-3.19(m, 8H), 3.13-2.75(m, 9H), 2.60(dt, *J* = 17.0, 3.4Hz, 1H), 2.30-1.92(m, 10H), 1.78-1.50(m,5H), 1.34-1.19(m, 1H).

LC-MS (ESI): $[M+H]^+$=819.40.

Example 83

Synthesis of PROTAC 55

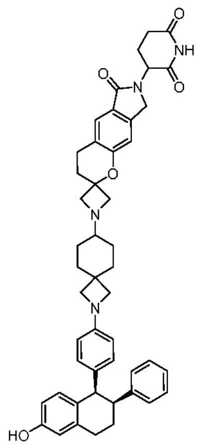

3-(1-(2-(4-((1R,2S)-6-hydroxy-2-phenyl-1,2,3,4-tetrahydronaphthalen-1-yl)phenyl)-2-azaspiro [3.5]non-7-yl)-6'-oxo-3',4',6',8'-tetrahydro-7'H-spiro[azetidine-3,2'-pyrano[2,3-f]isoindol]-7'-yl)piperidine-2,6-dione

Synthesis scheme:

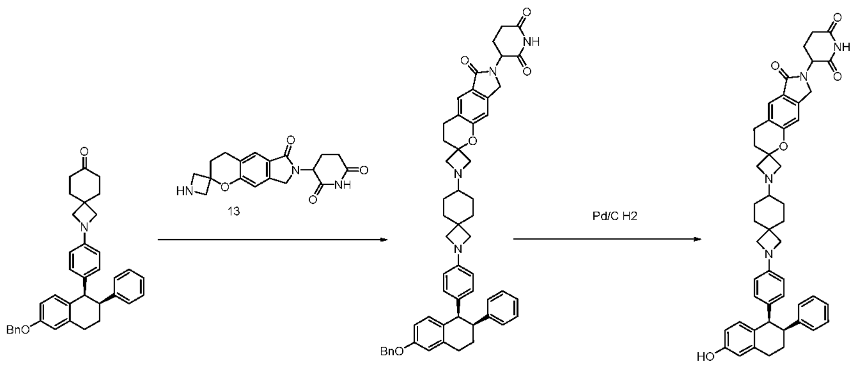


The first step: 3-(1-(2-(4-((1R, 2S)-6-(benzyloxy)-2-phenyl-1,2,3,4-tetrahydronaphthalene-1-yl )phenyl)-2-azaspiro[3.5] non-7-yl)-6'-oxo-3',4',6',8'-tetrahydro-7'H-spiro[azetidine -3,2'-pyrano[2,3-f]isoindol]-7'-yl)piperidine-2,6-dione (compound P-21)

Under the condition of nitrogen protection, 2-(4-(6-(benzyloxy)-2-phenyl-3,4-dihydronaphthalen-1-yl)phenyl)-2-azaspiro[3.5] non-7- ketone (90mg, 0.17mmol) and compound 13 (38.8mg, 0.17mmol) were dissolved in DCE:DMF=2:1 (6mL). 3 drops of acetic acid (catalytic amount) was added, stirred at room temperature for 1h. Sodium cyanoborohydride (21.5mg, 0.51mmol) was added to the above reaction solution, and the reaction was carried out at room temperature for 2h. Purified by preparative HPLC to obtain the product P-21 (30 mg, 20.6%).

$^1$H NMR (600MHz, DMSO-$d_6$) δ10.97(d, *J*=2.5Hz, 1H), 7.54(d, *J*=7.7Hz, 1H), 7.46(d, J=7.0Hz, 2H), 7.41(t ,*J* = 7.5Hz, 2H), 7.36-7.32(m, 1H), 7.16-7.04(m, 4H), 6.89-6.83(m, 3H), 6.77-6.72(m, 2H), 6.18(d, *J* = 8.1Hz, 2H), 6.06(d, *J* = 8.1Hz, 2H), 5.10-5.04(m, 3H), 4.42(q, *J* = 5.6Hz, 1H), 4.36(dd, *JJ* 17.5, 7.5Hz ,3H), 4.23(dt, *J* = 13.6, 7.0Hz, 2H), 4.17(d, *J* = 5.0Hz, 1H), 3.40-3.37(m, 4H), 3.31-3.29(m, 1H), 3.07-3.02(m, 1H), 3.01-2.85(m, 5H), 2.62-2.57(m, 1H),2.39-2.35(m, 1H), 2.15(ddt, *J* = 34.0, 12.7, 6.5Hz, 4H), 2.00-1.90(m, 4H), 1.87-1.82(m, 1H), 1.75-1.72(m, 1H), 1.48-1.42(m, 2H), 1.18(d, *J*=12.1Hz, 2H).

LC-MS(ESI): $[M+H]^+$=853.43.

The second step: 3-(1-(2-(4-((1R,2S)-6-hydroxy-2-phenyl-1,2,3,4-tetrahydronaphthalen-1-yl)phenyl) -2-azaspiro[3.5] non-7-yl)-6'-oxo-3',4',6',8'-tetrahydro-7'H-spiro[azetidine-3,2'-pyrano[2,3-f]isoindol]-7'-yl)piperidine-2,6-dione (compound PROTAC 55)

Under the condition of nitrogen protection, compound P-21 (25mg, 0.03mmol) was dissolved in methanol (5mL). 10% Pd/C (5mg, 20%wt) was added. After three times of hydrogen replacements, and heated to 50°C under hydrogen atmosphere for 16 hours. After the reaction was completed, the reaction solution was prepared in reverse to obtain PROTAC 55 (7.5 mg, 32.7%) as a white solid.

$^1$H NMR (600MHz, DMSO-$d_6$) δ10.98(d, *J*=2.1Hz, 1H), 9.11(s, 1H), 7.38(d, *J*=3.1Hz, 1H), 7.23-7.02(m, 4H) ,6.88-6.79(m,2H), 6.66-6.57(m, 2H), 6.48(dd, *J* = 8.3, 2.5Hz, 1H), 6.18(d, *J* = 8.2Hz, 2H), 6.05(d, *J* = 8.1Hz, 2H), 5.08(dd, *J* = 13.3, 5.2Hz, 1H), 4.44-4.32(m, 4H), 4.25-4.20(m, 2H), 4.12(d, *J* = 5.0Hz, 1H), 3.48-3.32(m, 4H), 3.29-3.18(m, 2H), 3.02-2.83(m, 5H), 2.63-2.56(m, 1H), 2.38(dq, *J* = 13.3, 9.2, 7.0Hz, 1H), 2.19(dt, *J* = 20.4, 7.1Hz, 2H), 2.09(dt, *J* = 12.5, 6.2Hz, 1H), 2.03-1.88(m, 4H), 1.84(d, *J* = 11.7Hz, 1H), 1.70(dd, *J*=12.9, 6.1Hz, 1H), 1.46(dt, *J*=24.0, 13.2Hz, 2H), 1.19(d, *J*=13.9Hz, 2H).

LC-MS (ESI): $[M+H]^+$=763.38.

Example 84

Synthesis of PROTAC 56

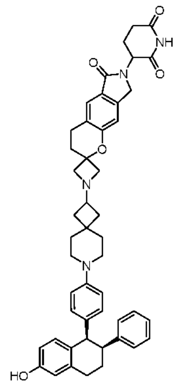

3-(1-(7-(4-((1R,2S)-6-hydroxy-2-phenyl-1,2,3,4-tetrahydronaphthalen-1-yl)phenyl)-7-azaspiro[3.5]non-2-yl)-6'-oxo-3',4',6',8'-tetrahydro-7'H-spiro[azetidine-3,2'-pyrano[2,3-f]isoindol]-7'-yl)piperidine-2,6-dione

Synthesis scheme:

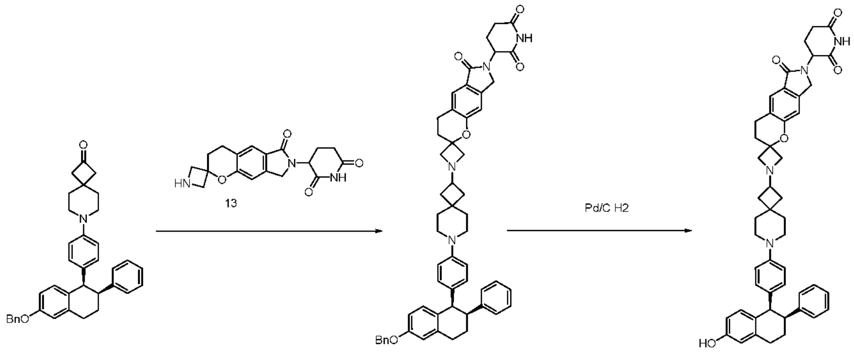

P-22
PROTAC 56

The first step: 3-(1-(7-(4-((1R,2S)-6-(benzyloxy)-2-phenyl-1,2,3,4-tetrahydronaphthalen-1-yl )phenyl)-7-azaspiro[3.5] non-2-yl)-6'-oxo-3',4',6',8'-tetrahydro-7'-hydrogen-spiro[azetidine-3,2'-pyrano[2,3-f]isoindol]-7'-yl)piperidine-2,6-dione (compound P-22)

Under the condition of nitrogen protection, 7-(4-((1R, 2S)-6-(benzyloxy)-2-phenyl-1,2,3,4-tetrahydronaphthalen-1-yl)phenyl)-7-azaspiro[3.5]non-2-one (100mg, 0.19mmol) and compound 13 (64.8mg, 0.19mmol) were dissolved in DCE:DMF=2:1 (6mL), and 3 drops of acetic acid ( catalytic amount) were added, stirred at room temperature for 1 h. Sodium cyanoborohydride (35.9 mg, 0.57 mmol) was added into the above reaction solution, and the reaction was performed at room temperature for 2 h. Purified by preparative HPLC to obtain the product compound P-22 (32 mg, 19.7%).

$^1$H NMR (600MHz, DMSO-$d_6$) $\delta$10.97(s, 1H), 7.53(s, 1H), 7.46(d, *J*=7.3Hz, 2H), 7.41(t, *J*=7.5Hz, 2H), 7.34 (t, *J*=7.3Hz, 1H), 7.14(dq, *J*=14.2, 7.0Hz, 3H), 7.08(d, *J*=8.8Hz, 1H), 6.90(d, *J*=2.0Hz, 1H), 6.84 (d, *J*=7.0Hz, 2H), 6.78-6.71(m, 2H),6.64(s,2H),6.26(d,*J*=7.0Hz,2H),5.11-5.04(m,3H),4.42- 4.33(m, 2H), 4.30-4.19(m, 4H), 4.10(dd, *J* = 30.4, 9.5Hz, 2H), 3.09-3.01(m, 3H),2.99-2.91(m, 4H), 2.87(d, *J*=6.0Hz, 2H), 2.60(d, *J*=18.5Hz, 1H), 2.40-2.33(m, 1H), 2.21-2.09(m, 5H), 2.01-1.95(m, 1H), 1.88 -1.81(m, 2H), 1.78-1.72(m, 1H), 1.64(s, 4H).

LC-MS(ESI): $[M+H]^+$=853.43.

The second step: 3-(1-(7-(4-((1R,2S)-6-hydroxy-2-phenyl-1,2,3,4-tetrahydronaphthalen-1-yl)phenyl) -7-azaspiro[3.5] non-2-yl)-6'-oxo-3',4',6',8'-tetrahydro-7'H-spiro[azetidine-3 ,2'-pyrano[2,3-f]isoindol]-7'-yl)piperidine-2,6-dione (compound PROTAC 56)

Under the condition of nitrogen protection, compound P-22 (30mg, 0.03mmol) was dissolved in methanol (5mL). 10% Pd/C (6mg, 20%wt) was added, and after being replaced with hydrogen three times, heated to 50°C under hydrogen atmosphere for 16 hours. After the reaction was completed, white solid PROTAC 56 (9.5 mg, 41.4%) was obtained by

revers preparation of reaction solution.

$^1H$ NMR (600MHz, DMSO-$d_6$) δ10.97(s, 1H), 9.16(s, 1H), 7.53(s, 1H), 7.19-7.10(m, 3H), 7.08(d, *J*=7.8Hz, 1H), 6.83(d, *J*=7.1Hz, 2H), 6.69-6.60(m, 4H), 6.48(dd, *J*=8.3, 2.3Hz, 1H), 6.26(d, *J*=7.5Hz, 2H), 5.06(dd, *J*=13.2, 5.0Hz, 1H), 4.37(dd, *J*=23.1, 6.9Hz, 2H), 4.25(dd, *J*=18.6, 12.8Hz, 3H), 4.18-4.07(m, 5H), 3.31(d, *J*=14.1Hz, 1H), 3.04(s, 2H), 2.99-2.85(m, 7H), 2.60(d, *J*=17.2Hz, 1H), 2.41-2.32(m, 1H), 2.19(s, 2H), 2.16-2.06(m, 2H), 1.99(d, *J*=7.0Hz, 1H), 1.84(d, *J*=8.4Hz, 2H), 1.72(d, *J*=5.9Hz, 1H ), 1.65(s, 3H).

LC-MS (ESI): $[M+H]^+$=763.38.

Example 85

Synthesis of PROTAC 57

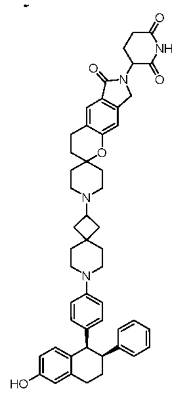

3-(1-(7-(4-((1R,2S)-6-hydroxy-2-phenyl-1,2,3,4-tetrahydronaphthalen-1-yl)phenyl)-7-nitrogen heterospiro[3.5]non-2-yl)-6'-oxo-3',4',6',8'-tetrahydro-7'H-spiro[piperidin-4,2'-pyrano [2,3-f]isoindole]-7'-yl)piperidine-2,6-dione

Synthesis scheme:

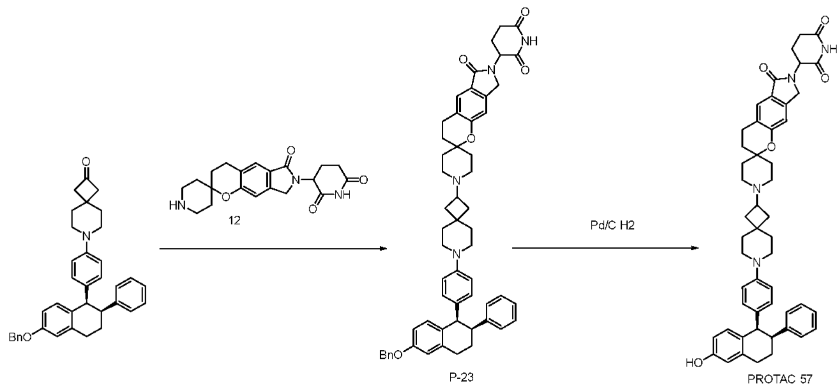


The first step: 3-(1-(7-(4-((1R,2S)-6-(benzyloxy)-2-phenyl-1,2,3,4-tetrahydronaphthalen-1-yl )phenyl)-7-azaspiro[3.5] non-2-yl)-6'-oxo-3',4',6',8'-tetrahydro-7'H-spiro[piperidine- 4,2'-pyrano[2,3-f]isoindol]-7'-yl)piperidine-2,6-dione (compound P-23)

Under the condition of nitrogen protection, 7-(4-((1R, 2S)-6-(benzyloxy)-2-phenyl-1,2,3,4-tetrahydronaphthalen-1-yl)phenyl)-7-azaspiro[3.5]nonan-2-one (90mg, 0.17mmol) and compound 12 (62.8mg, 0.17mmol) were dissolved in DCE:DMF=2:1 (6mL), and 3 drops of acetic acid ( catalytic amount) were added, stirried at room temperature for 1 h. Sodium cyanoborohydride (32.1 mg, 0.51 mmol) was added into the above reaction solution, and the reaction was perfomeded at room temperature for 2 h. Purified by preparative HPLC to obtain the product compound P-23 (24 mg, 16.6%).

$^1$H NMR (600MHz, DMSO-$d_6$) δ10.97(s, 1H), 7.51(d, *J*=7.3Hz, 1H), 7.46(d, *J*=7.3Hz, 2H), 7.41(t, *J*=7.5Hz, 2H), 7.34(t, *J*=7.3Hz, 1H), 7.14(dq, *J*=14.5, 7.6, 7.1Hz, 3H), 7.02(s, 1H), 6.90(d, *J*=1.9Hz, 1H), 6.84(d, *J*=7.2Hz, 2H), 6.79-6.72(m, 2H), 6.66(s, 2H), 6.26(d, *J*=7.3Hz, 2H), 5.10-5.03(m, 3H), 4.35(d, *J*=16.9Hz, 1H), 4.22(dd, *J*=10.7, 6.0Hz, 2H), 3.32(d, *J*=11.2Hz, 3H), 3.09-2.90(m, 11H), 2.60(d, *J*=17.0Hz, 1H), 2.36(s, 1H), 2.19(s, 2H), 2.12(dd, *J*=12.6, 5.9Hz, 1H), 2.00(dt, *J*=20.6, 11.4Hz, 5H), 1.90(t, *J*=6.8Hz, 2H), 1.86-1.80(m, 3H), 1.75(d, *J*=5.6Hz, 1H), 1.64(d, *J*=20.5Hz, 4H).
LC-MS (ESI): $[M+H]^+$=881.46.

The second step: 3-(1-(7-(4-((1R,2S)-6-hydroxy-2-phenyl-1,2,3,4-tetrahydronaphthalen-1-yl)phenyl) -7-azaspiro[3.5] non-2-yl)-6'-oxo-3',4',6',8'-tetrahydro-7'H-spiro[piperidine-4,2'-pyrano[2,3-f]isoindol]-7'-yl)piperidine-2,6-dione (compound PROTAC 57)

Under the condition of nitrogen protection, compound P-23 (30mg, 0.03mmol)was dissolved in methanol (5mL). 10% Pd/C (6mg, 20%wt) was added, and after three replacements with hydrogen, and heated to 50°C under hydrogen atmosphere for 16 hours. After the reaction was completed, the reaction solution was prepared by reverse preparation to obtain PROTAC 57 (10.5 mg, 50.6%) as a white solid.
$^1$H NMR (600MHz, DMSO-$d_6$) δ10.97(s, 1H), 9.84(s, 1H), 7.51(d, *J*=6.7Hz, 1H), 7.14(dt, *J*=11.5, 6.7Hz, 3H), 7.02(s, 1H), 6.86-6.74(m, 4H), 6.66-6.60(m, 2H), 6.49(dd, *J* = 8.3, 2.3Hz, 1H), 6.31(d,*J* = 8.1Hz, 2H), 5.07(dd, *J*=13.2, 5.1Hz, 1H), 4.35(d, *J*=16.9Hz, 1H), 4.25-4.18(m, 2H), 3.32(d,*J*=11.4Hz, 3H), 3.13(s, 2H), 3.08-2.97(m, 6H), 2.95-2.85(m, 5H), 2.60(d, *J* = 16.7Hz, 1H), 2.36(dd, *J* = 13.2, 4.6Hz, 1H), 2.22(s, 2H), 2.11-1.95(m, 6H), 1.92-1.84(m, 3H), 1.70(d, *J*=23.7Hz, 5H).
LC-MS (ESI): $[M+H]^+$=791.41.

Example 86

Synthesis of PROTAC 58

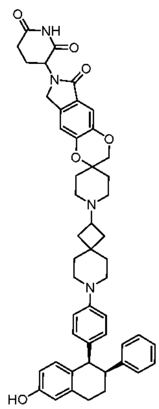

3-(1-(7-(4-((1R,2S)-6-hydroxy-2-phenyl-1,2,3,4-tetrahydronaphthalen-1-yl)phenyl)-7- azaspiro [3.5]non-2-yl)-6'-oxo-6',8'-dihydro-3'H,7'H-spiro[piperidine-4,2'-[1,4] dioxino[2,3-f]isoindol]-7'-yl)piperidine-2,6-dione

Synthesis scheme:

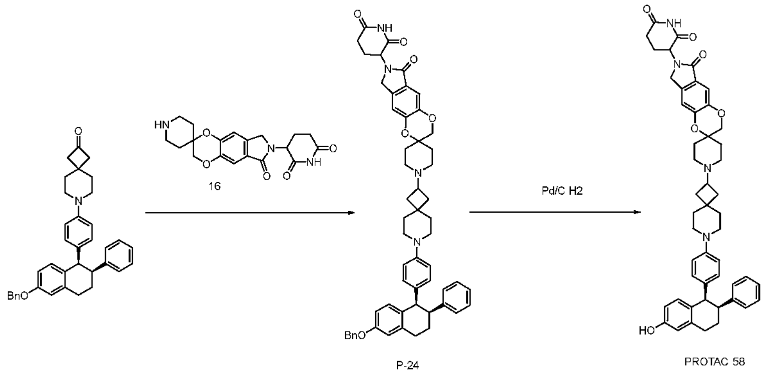


The first step: 3-(1-(7-(4-((1R,2S)-6-(benzyloxy)-2-phenyl-1,2,3,4-tetrahydronaphthalen-1-yl )phenyl)-7-azaspiro[3.5] non-2-yl)-6'-oxo-6',8'-dihydro-3'H,7'H-spiro[piperidine-4, 2'-[1,4]dioxino[2,3-f]isoindol]-7'-yl)piperidine-2,6-dione (compound P-24)

Under the condition of nitrogen protection, 7-(4-((1R, 2S)-6-(benzyloxy)-2-phenyl-1,2,3,4-tetrahydronaphthalene-1-yl)phenyl )-7-azaspiro[3.5]nonan-2-one (60mg, 0.11mmol) and compound 16 (40.8mg, 0.11mmol) were dissolved in DCE:DMF=2:1 (6mL). 3 drops of acetic acid ( catalytic amount) were added, stirred at room temperature for 1 h. Sodium cyanoborohydride (21.5 mg, 0.33 mmol) was added into the above reaction solution, and the reaction was performed at room temperature. Purified by preparative HPLC to obtain the product P-24 (22 mg, 21.6%).

$^1$H NMR (600MHz, DMSO-$d_6$) δ10.98(s, 1H), 7.46(d, *J*=7.3Hz, 2H), 7.41(t, *J*=7.5Hz, 2H), 7.34(t, *J*=7.3Hz, 1H), 7.24(s, 1H), 7.14(dt, *J*=12.4, 5.4Hz, 4H), 6.91-6.89(m, 1H), 6.84(d, *J*=7.2Hz, 2H), 6.78-6.72(m, 2H), 6.65(s, 2H), 6.26(d, *J*=7.3Hz, 2H), 5.07(d, *J*=15.1Hz, 3H), 4.33(d, *J*=17.0Hz, 2H), 4.24-4.13(m, 4H), 3.41-3.31(m, 4H), 2.99(ddt, *J* = 50.0, 25.9, 11.9Hz, 10H), 2.60(d, *J* = 16.8Hz, 1H), 2.40-2.34(m, 1H), 2.22-2.12(m, 3H), 1.99(t, *J* = 14.4Hz, 4H), 1.92-1.85(m, 2H),1.75(d, *J* = 7.7Hz, 1H), 1.64(d,*J* =18.9Hz, 4H).

LC-MS (ESI): $[M+H]^+$=883.44.

The second step: 3-(1-(7-(4-((1R,2S)-6-hydroxy-2-phenyl-1,2,3,4-tetrahydronaphthalen-1-yl)phenyl) -7-azaspiro[3.5] non-2-yl)-6'-oxo-6',8'-dihydro-3'H,7'H-spiro[piperidine-4,2'-[1,4]dioxino[2,3-f]isoindol]-7'-yl)piperidine-2,6-dione (compound PROTAC 58)

Under the condition of nitrogen protection, compound P-24 (30mg, 0.03mmol) was dissolved in methanol (5mL). 10% Pd/C (6mg, 20%wt) was added, and after three replacement with hydrogen, then heated to 50°C in hydrogen atmosphere for 16 hours. After the reaction was completed, white solid PROTAC 58 (8.5 mg, 35.7%) was obtained by reverse preparation of the reaction solution.

$^1$H NMR (600MHz, DMSO-$d_6$) δ10.97(s, 1H), 9.54(s, 1H), 7.24(s, 1H), 7.14(dd, *J*=12.2, 4.7Hz, 3H), 6.83(d, *J* = 7.2Hz, 2H), 6.69-6.57(m, 4H), 6.48(dd, *J*= 8.3, 2.2Hz, 1H), 6.24(d, *J* = 8.0Hz, 2H), 7.24(s, 1H), 5.07(dd, *J* = 13.3,4.9Hz, 1H), 4.36-4.30(m, 1H), 4.30-4.19(m, 2H), 4.18-4.13(m, 2H), 3.84(dd, *J* = 16.4, 8.1Hz, 1H),2.96(dtd, *J*=38.2, 20.2, 13.1Hz, 8H), 2.60(d, *J*=16.8Hz, 1H),2.40-2.33(m, 5H), 2.19(s, 3H), 2.09 (dd, *J*=11.7, 5.2Hz,2H), 1.98(d, *J*=11.5Hz,4H), 1.87(t, *J*=13.7Hz, 2H), 1.71(d, *J*=5.7Hz, 1H), 1.63 (d,*J*=18.2Hz,3H).

LC-MS (ESI): $[M+H]^+$=793.39.

Example 87

Synthesis of PROTAC 59

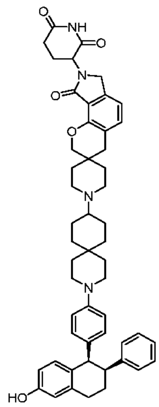

3-(1-(3-(4-((1R,2S)-6-hydroxy-2-phenyl-1,2,3,4-tetrahydronaphthalen-1-yl)phenyl)-3-azaspiro[5.5]undec-9-yl)-9'-oxo-7',9'-dihydro-2'H-spiro[piperidin-4,3'-pyrano[2,3 -f]isoindole]-8'(4'H)-yl)piperidine-2,6-dione

Synthesis scheme:

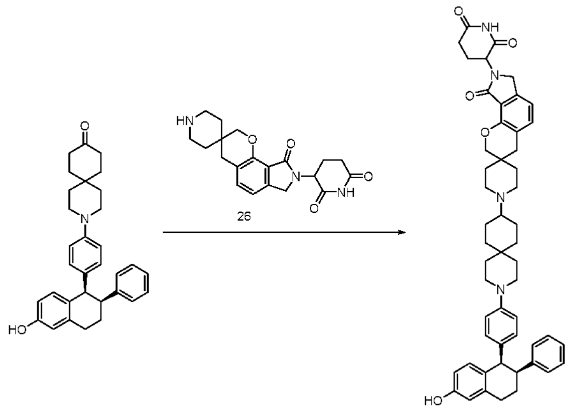


PROTAC 59

The first step: 3-(1-(3-(4-((1R,2S)-6-hydroxy-2-phenyl-1,2,3,4-tetrahydronaphthalen-1-yl)phenyl) -3-azaspiro[5.5]undec-9-yl)-9'-oxo-7',9'-dihydro-2'H-spiro[piperidin-4,3'-pyrano[2,3-f]isoindole]-8'(4'H)-yl)piperidine-2,6-dione (compound PROTAC 59)

Under the condition of nitrogen protection, 3-(4-((1R,2S)-6-hydroxyl-2-phenyl-1,2,3,4-tetrahydronaphthalene-1-yl)phenyl)-3-azaspiro[5.5]undecane-9-one (30mg, 0.06mmol) and compound 26 (36mg, 0.09mmol) were dissolved in DCE:DMSO=1:1 (4mL), triethylamine (15mg, 0.18 mmol) and acetic acid (15mg, 0.18mmol) were added, stirred at room temperature for 1h. Sodium triacetoxyborohydride (42mg, 0.18mmol) was added to the above reaction solution, and the reaction was performed at room temperature for 24h. Purified by preparative HPLC to obtain the product PROTAC 59 (13 mg, 23.6%).

$^1$H NMR (600MHz, DMSO-$d_6$) δ10.98(s, 1H), 9.07(s, 1H), 7.30(dd, *J*=12.9, 7.7Hz, 1H), 7.14(dd, *J*=11.7, 7.0Hz, 3H), 7.05(t, *J* = 8.2Hz, 1H), 6.84(d, *J* = 7.3Hz, 4H), 6.66-6.61(m, 2H), 6.51-6.47(m, 1H), 6.35(s, 2H), 5.02(dt, *J*=11.8, 5.4Hz, 1H), 4.32(d, *J*=17.3Hz, 1H), 4.23(dd, *J*=19.4, 8.0Hz, 2H), 3.95(s, 1H), δ3.37-3.30(m, 3H), 3.26-3.07(m, 7H), 2.94(ddq, *J*=30.1, 12.9, 8.7, 6.2Hz, 5H), 2.68-2.55(m, 2H), 2.34(td, *J*=13.3, 4.7Hz, 1H), 2.08(q, *J*=7.2, 5.9Hz, 1H), 1.94(ddt, *J*=34.1, 13.4, 7.0Hz, 4H), 1.69(dddd, *J*=63.4, 41.3, 26.0, 13.2Hz, 9H), 1.48(d, *J*=22.9Hz, 2H), 1.25-1.12(m, 3H).

LC-MS (ESI): $[M+H]^+$=819.44.

Example 88

Intermediate 18

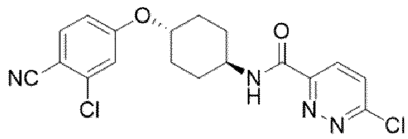

Intermediate 18

6-chloro-N-((1r,4r)-4-(3-chloro-4-cyanophenoxy)cyclohexyl)pyridazine-3-carboxamide Synthesis scheme:

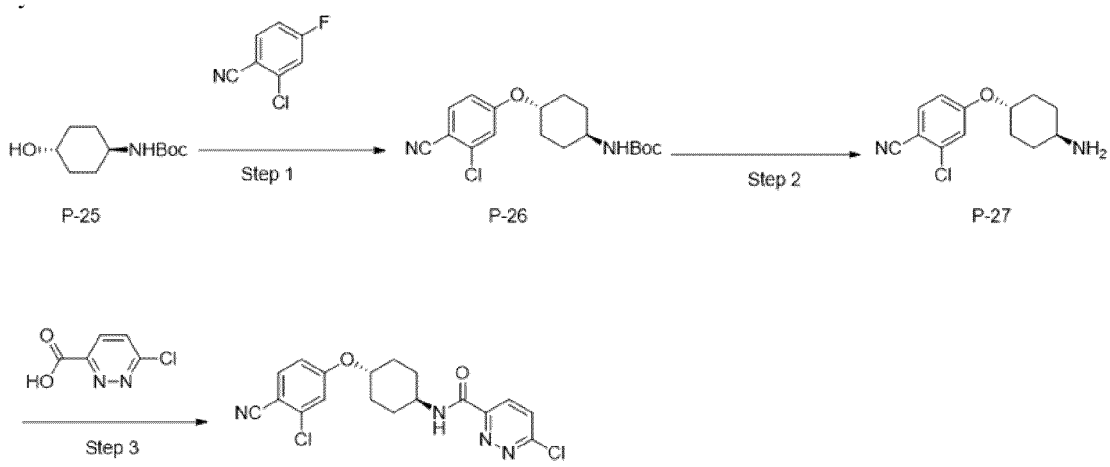


Intermediate 18

The first step: tert-butyl (1r,4r)-4-(3-chloro-4-cyanophenoxy)cyclohexyl)carbamate (intermediate P-26)

Intermediate P-25 (20g, 92.9mmol) was dissolved in a one-necked flask containing tetrahydrofuran (100mL), cooled to 0°C, sodium hydrogen (8.36g, 209.02mmol) was slowly added, and stirred at 0°C for 1h. A solution of 2-chloro-4-fluorobenzonitrile (20 g, 92.9 mmol) in tetrahydrofuran (100 mL) was added into the one-necked flask, and stirred at 0°C for 0.5 h. Transfer to room temperature and continue stirring for 3h. After the reaction was completed, the reaction mixture was cooled to 0 °C, quenched with ice water, and extracted three times with ethyl acetate (100 mL). And the organic phase was washed with saturated brine, dried over anhydrous sodium sulfate, filtered and concentrated to obtain a crude product. The crude product was purified by column (0-25% ethyl acetate) to obtain white solid intermediate P-26 (21.92 g).

$^1$H NMR (600MHz, $CD_3OD$) δ7.69(d, *J*=8.8Hz, 1H), 7.18(d, *J*=2.4 Hz, 1H), 7.03(dd, *J*=8.8, 2.4Hz, 1H), 4.43(tt, *J*=10.2, 4.1Hz, 1H), 3.42(dq, *J*=10.9, 5.8Hz, 1H), 2.18-2.10(m, 2H), 2.07-1.97(m, 2H), 1.61-1.51(m, 2H), 1.46(s, 9H), 1.43-1.40(m, 2H).
LC-MS (ESI): $[M+H]^+$=351.11

The second step: 4-(((1r,4r)-4-aminocyclohexyl)oxy)-2-chlorobenzonitrile (intermediate P-27)

Intermediate P-26 (21.92 g, 62.48 mmol)was added to a one-necked flask containing dioxane (25 mL). A solution (of hydrochloric acid in dioxane (25 mL) was added to the reaction flask. Stir at room temperature for 2h after addition. After the reaction was completed, it was directly concentrated to obtain the crude white solid intermediate P-27 (20.05 g). LC-MS (ESI): $[M+H]^+$=251.13

The third step: 6-chloro-N-((1r,4r)-4-(3-chloro-4-cyanophenoxy)cyclohexyl)pyridazine-3-carboxamide (intermediate 18)

The intermediate P-27 (20.05g, 79.97mmol), triethylamine (32.37g, 319.87mmol), 1-propyl phosphoric anhydride (12.2g, 96mmol) and dichloromethane (200mL) were added to a single-necked flask in order, stirred at room temperature for 5 min after addition. 6-Chloropyridazine-3-carboxylic acid (16.48g, 103.96mmol) was slowly added into the one-necked flask, and the temperature was raised to 30°C and stirring was continued for 2.5h. After the reaction was

completed, the reaction mixture was concentrated and purified by pulping (petroleum ether: ethyl acetate = 1:1) to obtain intermediate 18 (13.45 g) as a brown solid.

$^1$H NMR (600MHz, DMSO-$d_6$) δ9.17(d, *J*=8.2Hz, 1H), 8.23(d, *J*=8.9Hz, 1H), 8.11(d, *J*=8.9Hz, 1H), 7.87(d, *J*=8.7Hz, 1H), 7.41(d, *J*=2.4Hz, 1H), 7.15(dd, *J*=8.8, 2.4Hz, 1H), 4.54(td, *J*=10.5, 5.1Hz, 1H), 3.91(dtd, *J*=11.5, 7.7, 4.1Hz, 1H), 2.13(d, *J*=12.3Hz, 2H), 1.90(s, 2H), 1.71(q, *J*=13.4Hz, 2H), 1.57-1.48(m, 2H).
LC-MS (ESI): $[M+H]^+$=391.33

Example 89

Synthesis of PROTAC 60

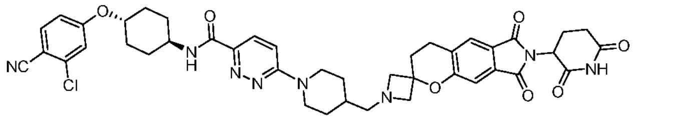

N-((1r,4r)-4-(3-chloro-4-cyanophenoxy)cyclohexyl)-6-(4-((7'-(2,6-dioxopiperidine-3- base)-6',8'-dioxo-4',6',7',8'-tetrahydro-3'hydrospiro[azetidine-3,2'-pyrano[2, 3-f]isoindole]-1-methyl)piperidin-1-yl)pyridazine-3 -carboxamide

Synthetic scheme

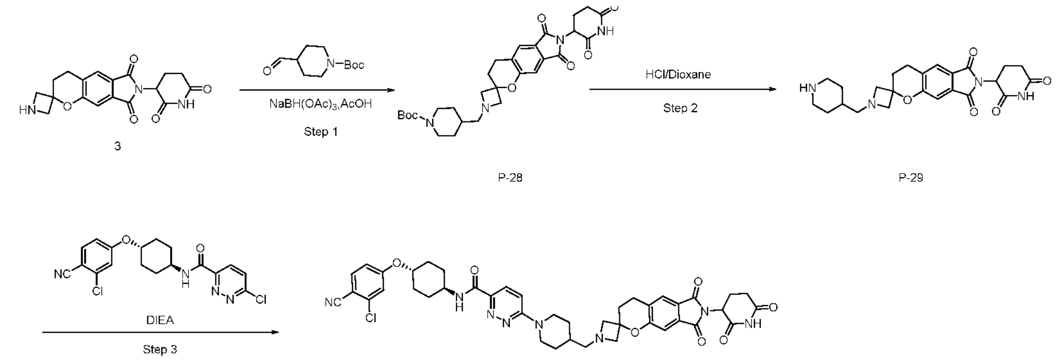


PROTAC 60

The first step: tert-butyl 4-((7'-(2,6-dioxopiperidin-3-yl)-6',8'-dioxo-4',6',7',8'-tetrahydro-3'H-spiro[azetidine-3,2'-pyran [2,3-f]isoindol]-1-yl)methyl)-4-methylpiperidine- 1-carboxylate (compound P-28)

Compound 3 (100mg, 0.281mmol) and tert-butyl 4-formylpiperidine-1-carboxylate (90mg, 0.422mmol) were added the solvent DCE (2mL), AcOH (4drop), reacted at room temperature for 1h. Then $NaBH(OAc)_3$ (179 mg, 0.844 mmol) was added, and the reaction was continued for 1 h at room temperature. After the reaction solution was concentrated, it was washed with 5 mL of saturated aqueous ammonium chloride solution, extracted with ethyl acetate, concentrated and purified by preparative thin-layer plate chromatography (DCM:MeOH=10:1) to obtain white solid compound P-28 (120 mg) .

LC-MS (ESI): $[M+H]^+$=497.40

The second step: 7'-(2,6-dioxopiperidin-3-yl)-1-(piperidin-4-ylmethyl)-3',4'-dihydro-6'H-spiro [azetidine-3,2'-pyrano [2,3-f]isoindole]-6',8'(7'H)-dione (Compound P-29)

Compound P28 (100 mg, 0.181 mmol), dioxane (1 mL) and 4M dioxane hydrochloride solution (1 mL) were reacted at room temperature for 0.5 h, and concentrated to obtain white solid compound P-29 (40 mg).

The third step: N-((1r,4r)-4-(3-chloro-4-cyanophenoxy)cyclohexyl)-6-(4-((7'-(2,6- dioxopiperidin -3-yl)-6',8'-dioxo-4',6',7',8'-tetrahydro-3'hydrospiro[azetidine-3,2'-pyran [2,3-f]isoindole]-1-methyl)piperidin-1-yl)pyridazine-3-carboxamide (compound PROTAC 60)

Compound P-29 (40mg, 0.088mmol), intermediate 18 (69.17mg, 0.176mmol), DIEA (22.85mg, 0.177mmol), and DMSO (1mL) were sequentially added into a 8mL sealed tube. Under the protection of $N_2$, react at 80°C for 2h. After the reaction was completed, it was cooled to room temperature, concentrated, and purified by Prep-HPLC (15-50% acetonitrile) to obtain white solid compound PROTAC 60 (1.23 mg).

$^1$H NMR (400MHz, DMSO-$d_6$) $\delta$11.11(s, 1H), 8.57(d, *J*=8.2Hz, 1H), 7.84(dd, *J*=11.0, 9.1Hz, 2H), 7.78(s, 1H), 7.41-7.34(m, 2H), 7.29(s, 1H), 7.13(dd, *J* = 8.8, 2.5Hz, 1H), 5.32(s, 1H), 5.10(dd, *J* = 12.8, 5.5Hz, 2H), 4.43 (m, *J*=65.0Hz, 8H), 3.85(s, 3H), 2.60(d, *J*=17.5Hz, 3H), 2.25(s, 3H), 2.15-1.86(m, 8H), 1.78(d, *J*=12.5Hz, 3H), 1.63(q, *J*=12.3Hz, 3H).

LC-MS (ESI): $[M+H]^+$=807.65

Example 90

Synthesis of PROTAC 61

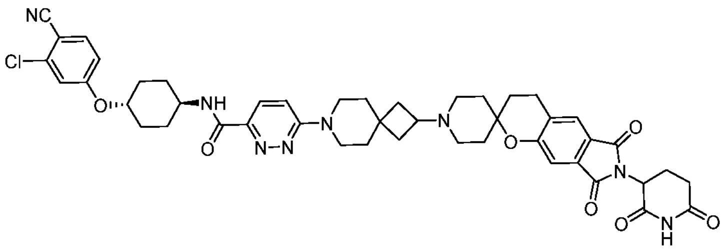

N-((1r,4r)-4-(3-chloro-4-cyanophenoxy)cyclohexyl)-6-(2-(7'-(2,6-dioxopiperidin-3-yl )-6',8'-dioxo-4',6',7',8'-tetrahydro-3'hydrospiro[piperidine-4,2'-pyrano[2,3-f]isoindol]-1-yl)-7-azaspiro[3,5]non-7-yl]pyridazine-3 -carboxamide

Synthetic scheme:

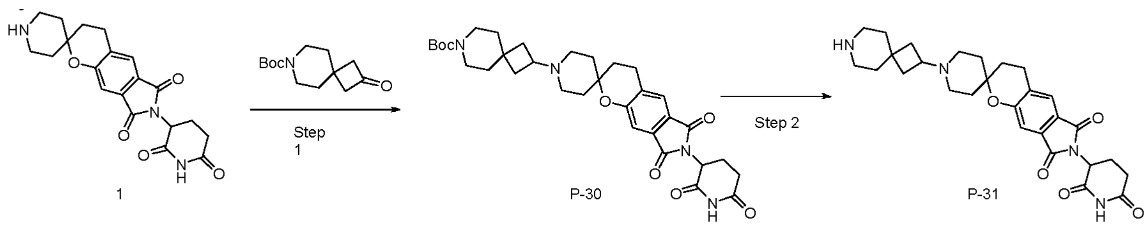


1 P-30 P-31

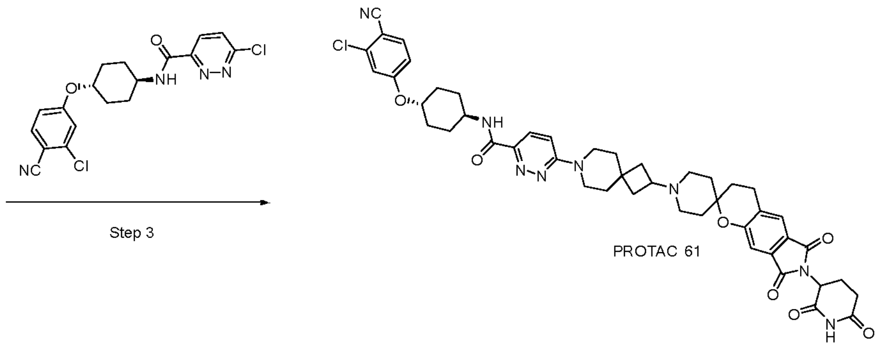


PROTAC 61

The first step: 2-(7'-(2,6-dioxopiperidin-3-yl)-6',8'-dioxo-4',6',7',8'-tetrahydro-3'H-spiro[piperidine-4,2'-pyrano[2,3-f]isoindol]-1-yl)-7-azaspiro[3,5]nonane-7-tert-butyl carboxylate (compound P-30)

Compound 1 (200mg, 0.521mmol) and tert-butyl 2-oxo-7-azaspiro[3,5]nonane-7-tert-butyl carboxylate (249.67mg, 1.04mmol) were added in a glass vial in order, solvent THF (2mL) and $Ti(O\text{-}iPr)_4$ (296.5mg, 1.04mmol) was added, stirred and reacted at 60°C for 1h, then $NaBH_3CN$ (98.3mg, 1.56mmol) was added, the reaction was continued to react at 60°C for 1h, after the reaction was completed , it was concentrated, and then purified by preparative thin-layer plate chromatography (DCM:MeOH=10:1) to obtain white solid compound P-30 (200 mg).
LC-MS: $[M+H]^+$=607.53

The second step: 7'-(2,6-dioxopiperidin-3-yl)-1-(7-azaspiro[3.5]non-2-yl)-3',4'-dihydro- 6'H-spiro[piperidine-4,2'-pyrano [2,3-f]isoindole]-6',8',7'H-dione (compound P-31)

Compound P-30 (200mg, 0.346mmol), DCM (1mL) solution and TFA (1mL) were sequentially added into a one-necked bottle in order. Stir at room temperature for 1 h. After the reaction was completed, it was directly concentrated to obtain white solid compound P-31 (150 mg).
LC-MS: $[M+H]^+$=507.50

The third step: N-((1r,4r)-4-(3-chloro-4-cyanophenoxy)cyclohexyl)-6-(2-(7'-(2,6-dioxypiperidine) -3-yl)-6',8'-dioxo-4',6',7',8'-tetrahydro-3'hydrospiro[piperidine-4,2'-pyrano[2, 3-f]isoindol]-1-yl)-7-azaspiro[3,5]non-7-yl]pyridazine-3-carboxamide (PROTAC 61)

The preparation refers to the third step of Example 89.

$^1$H NMR (600MHz, DMSO-$d_6$) δ11.11(s, 1H), 9.84(q, *J*=9.5Hz, 1H), 8.57(d, *J*=8.2Hz, 1H), 7.84(dd, *J*=21.0, 9.1Hz, 2H), 7.75(d, *J*=4.2Hz, 1H), 7.42-7.33(m, 3H), 7.14(dd, *J*=8.8, 2.4Hz, 1H), 5.10(dd, *J*=12.9, 5.5 Hz, 1H), 4.54(tt, *J*=9.9, 4.4Hz, 1H), 3.92-3.81(m, 2H), 3.74(t, *J*=5.5Hz, 2H), 3.66(t, *J*=5.4Hz, 2H), 3.33(d, *J*=11.4Hz, 2H), 3.08(q, *J*=11.8Hz, 2H), 2.97(t, *J*=7.0Hz, 2H), 2.89(ddd, *J*=17.7, 13.9, 5.5Hz, 1H), 2.64-2.52(m, 2H), 2.27(dd, *J* = 12.0, 7.9Hz, 2H), 2.14-2.01(m, 7H), 1.92(dddd, *J* = 27.9, 23.3, 16.9, 5.0Hz, 6H),1.72-1.57(m, 6H),1.57-1.47(m, 2H).
LC-MS: $[M+H]^+$=861.66

Example 91

Synthesis of PROTAC 62

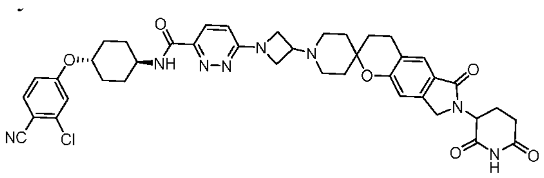

N-((1r,4r)-4-(3-chloro-4-cyanophenoxy)cyclohexyl)-6-(4-((7'-(2,6-dioxopiperidine-3-yl)-6',8'-dioxo-4',6',7',8'-tetrahydro-3'H-spiro[azetidine-3,2'-pyrano[2,3-f]isoindole]-1-methyl)-4-methylpiperidin-1-yl)pyridazine-3 -carboxamide

Synthesis scheme:

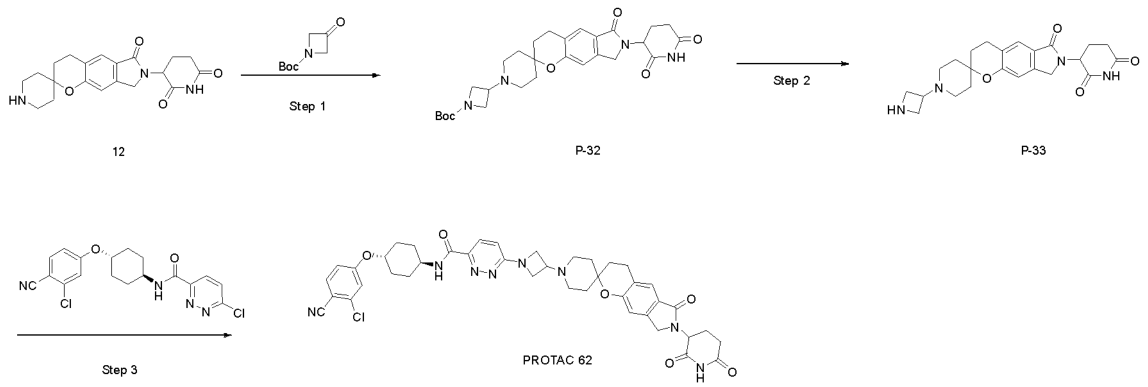


The first step: tert-butyl 3-(7'-(2,6-dioxopiperidin-3-yl)-6'-oxo-4',6',7',8'-tetrahydro-3 'H-spiro[piperidine-4,2'-pyrano [2,3-f]isoindol]-1-yl)azetidine-1-carboxylate (compound P-32)

Compound 12 (100mg, 0.271mmol) and tert-butyl 3-oxoazetidine-1-carboxylate (139.03mg, 0.812mmol) added the solvent DCE (1mL), AcOH (4 drops), reacted 1h at room temperature. $NaBH(OAc)_3$ (172.11 mg, 0.812 mmol) was added, and the reaction was continued for 1 h at room temperature. After the reaction solution was concentrated, it was washed with 5 mL of saturated aqueous ammonium chloride solution, extracted with ethyl acetate. After concentration, the white solid compound P-32 (109 mg) was purified by thin-layer plate chromatography (DCM: MeOH=10:1).

The second step: 3-(1-(azetidin-3-yl)-6'-oxo-3',4',6',8'-tetrahydro-7'H-spiro[piperidine -4,2'-pyran[2,3-f]isoindole]-7'-yl) piperidine-2,6-dione (compound P-33)

Refer to the second step of Example 89 for preparation.

The third step: N-(1r,4r)-4-(3-chloro-4-cyano)cyclohexyl)-6-(4-(7'-(2,6-dioxypiperidin-3-yl) )-6',8'-dioxo-4',6',7',8'-tetrahydro-3'H-spiro[azetidine-3,2'-pyran[2,3 -f]isoindol]-1-yl)methyl)piperidin-1-yl)pyridazine-3-carboxamide (compound PROTAC 62).

Refer to the third step of Example 89 for preparation.

$^1$H NMR (400MHz, DMSO-$d_6$) δ10.95(s, 1H), 8.60(d, *J*=8.1Hz, 1H), 7.93(d, *J*=9.2Hz, 1H), 7.85(d, *J*=8.8Hz, 1H), 7.51(s, 1H), 7.37(d, *J* = 2.4Hz, 1H), 7.12(dd, *J* = 8.8, 2.4Hz, 1H), 7.06-6.96(m, 2H), 5.05(dd, *J* = 13.3, 5.1Hz, 1H), 4.57-4.29(m, 6H), 4.21(d, *J* = 17.0Hz, 1H), 3.32-3.12(s, 5H), 2.89(m, 2H), 2.58(d ,*J*=16.7Hz, 2H), 2.36(d, *J*=13.2Hz,2 H), 2.17-1.77(m, 11H), 1.64(q, *J*=11.5Hz, 2H), 1.57-1.43(m, 2H).
LC-MS (ESI): $[M+H]^+$=779.63

Example 92

Synthesis of PROTAC 63

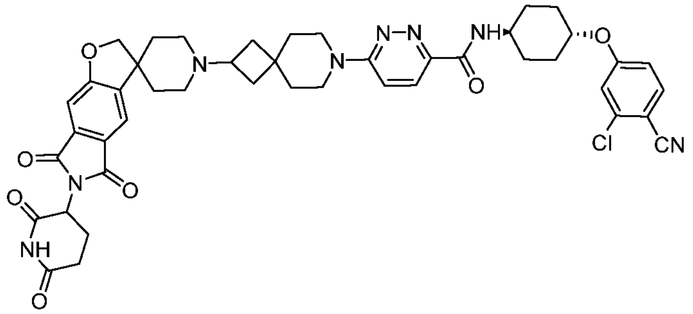

N-(1r,4r)-4-(3-chloro-4-cyanophenoxy)cyclohexyl)-6-(2-(6-(2,6-dioxopiperidin-3-yl) -5,7-dioxo-6,7-dihydro-2H,5H-spiro [furo[2,3-f]isoindole-3,4'-piperidinyl]-1'-yl)-7-azaspiro[3.5]nonan-7-yl)pyridazine-3-carboxamide

Synthesis scheme:

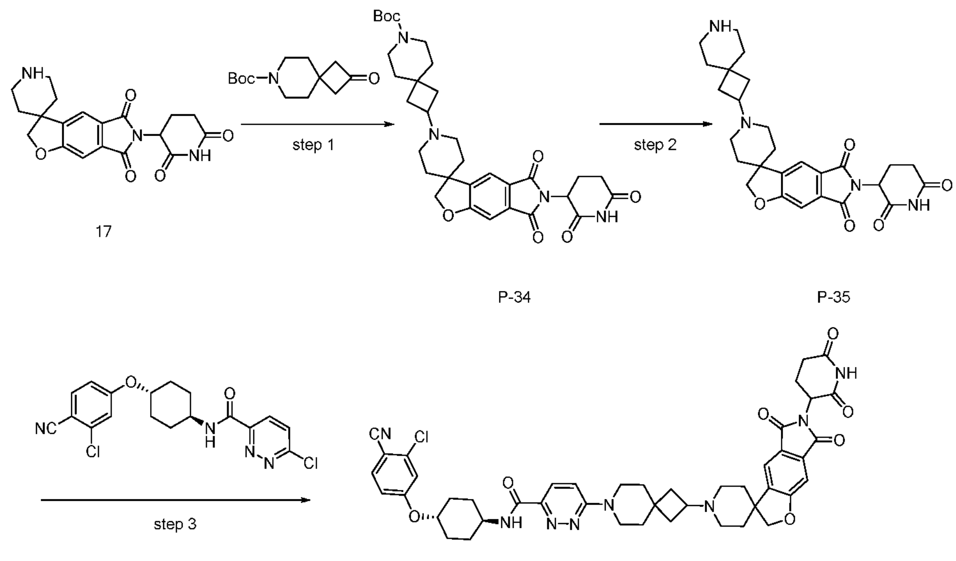


17

P-34

P-35

PROTAC 63

The first step: 2-(6-(2,6-dioxypiperidin-3-yl)-5,7-dioxo-6,7-dihydro-2H, 5H-spiro[furan[2,3 - f]isoindole-3,4'-piperidin]-1'-yl)-7-azaspiro[3,5]nonane-7-carboxylic acid tert-butyl ester (compound P-34)

Compound 17 (20mg, 0.054mmol), tert-butyl 2-oxo-7-azaspiro[3,5]nonane-7-carboxylate (25.9mg, 0.108mmol) and Ti( O-iPr)$_4$ (30mg, 0.10mmol) were added in a glass vial in order. THF (1mL) was added dropwise, and the reaction was stirred at 60°C with for 2h. And then $NaBH_3CN$ (11mg, 0.162mmol) was added. The reaction was stirred at 60°C for 1h. After the reaction was completed, it was concentrated and purified by TLC (DCM:MeOH=10:1) to obtain white solid compound P-34 (20 mg).

The second step: 6-(2,6-dioxopiperidin-3-yl)-1'-(7-azaspiro[3.5]non-2-yl)-6-hydrogen-2H,5H-spiro[furo[2,3-f]isoindole-3,4'-piperidine]-5,7-dione (compound P-35)

Refer to the second step of PROTAC 60 for preparation.

The third step: N-(1r,4r)-4-(3-chloro-4-cyanophenoxy)cyclohexyl)-6-(2-(6-(2,6-dioxopiperidine- 3-yl)-5,7-dioxo-6,7-dihydro-2H,5H-spiro[furo[2,3-f]isoindole-3,4'-piperidine]-1'-yl)-7-azaspiro[3.5]nonan-7-yl)pyridazine-3-carboxamide (PROTAC 63)

Refer to the third step of Example 89 for preparation.

$^1$H NMR (600MHz, DMSO-$d_6$) δ11.13(s, 1H), 8.59(t, *J*=6.9Hz, 1H), 7.90-7.80(m, 2H), 7.66(s, 1H), 7.44-7.34(m, 3H), 7.14(dd, *J*=8.8, 2.4Hz, 1H),5.16-5.08(m, 1H), 4.74(d, *J*=12.9Hz, 3H), 4.55(dd, *J*=9.3, 5.2Hz, 2H), 4.02-3.99(m, 1H), 3.93-3.61(m, 6H), 3.47(d, *J* = 10.8Hz, 1H), 3.28(dd, *J* = 29.5, 23.9Hz, 2H), 2.98-2.85(m, 2H), 2.61(dd, *J* = 9.5, 7.6Hz, 1H), 2.32-2.22(m, 2H), 2.09(ddd, *J* = 27.3, 21.5, 20.0Hz, 7H), 1.90(d, *J* = 12.2Hz, 3H), 1.79-1.46(m, 7H).
LC-MS (ESI): $[M+H]^+$=847.65

Example 93

Synthesis of PROTAC 64

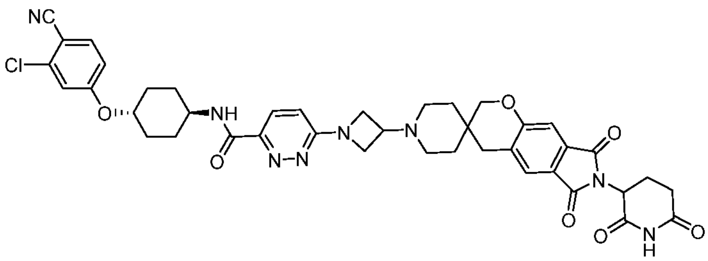

N-((1r,4r)-4-(3-chloro-4-cyanophenoxy)cyclohexyl)-6-(3-(7'-(2,6-dioxopiperidin-3-yl )-6',8'-dioxo-4',6',7',8'-tetrahydro-2'H-spiro[piperidine-4,3'-pyran[2,3-f] isoindole]-1-yl)azetidin-1-yl]pyridazine-3-carboxamide

Synthetic scheme:

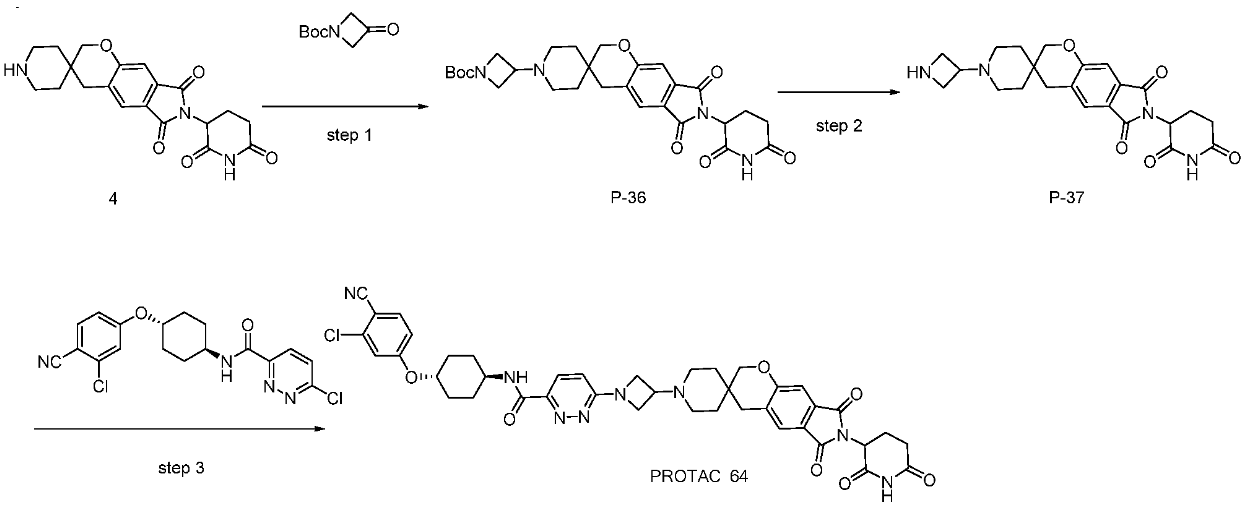

4
P-36
P-37
PROTAC 64

The first step: 3-(7'-(2,6-dioxopiperidin-3-yl)-6',8'-dioxo-4',6',7',8'-tetrahydro-2'H-spiro[piperidine-4,3'-pyrano[2,3-f]isoindol]-1-yl)azetidine-1-carboxylic acid tert-butyl ester (compound P-36 )

Compound 4 (200mg, 0.522mmol) and tert-butyl 3-oxoazetidine-1-carboxylate (178.61mg, 1.04mmol) were successively added into a glass vial. Solvent THF (2mL) and Ti(O -iPr)$_4$ (296.52mg, 1.04mmol) were added and reacted 1h at 60°C with stirring. And then $NaBH_3CN$ (98.34mg, 1.56mmol) was added, continued to react 1h at 60°C with stirring. After

the reaction was completed, it was concentrated, and then purified by preparing thin layer chromatography (DCM:MeOH=10:1) to obtain compound P-36 (170 mg) as a white solid.

$^1$H NMR (600MHz, $CD_3OD$) δ7.68(s, 1H), 7.30(s, 1H), 5.14-5.10(m, 1H), 4.36-4.24(m, 4H), 4.14(t, *J* = 11.1Hz, 4H), 3.52(s, 2H), 3.14(s, 4H), 2.96-2.82(m, 4H), 2.79-2.69(m, 3H), 1.48(s,9H).
LC-MS: $[M+H]^+$=539.43

The second step: 1-(azetidin-3-yl)-7'-(2,6-dioxopiperidin-3-yl)-2'H-spiro[piperidine-4,3' -pyrano[2,3-f]isoindole]-6',8'(4'H,7'H)-dione (Compound P-37)

Refer to the second step of Example 89 for preparation.
LC-MS: $[M+H]^+$=439.36

The third step: N-((1r,4r)-4-(3-chloro-4-cyanophenoxy)cyclohexyl)-6-(3-(7'-(2,6-dioxypiperidine) -3-yl)-6',8'-dioxo-4',6',7',8'-tetrahydro-2'H-spiro[piperidine-4,3'-pyran[2, 3-f]isoindol]-1-yl)azetidin-1-yl]pyridazine-3-carboxamide (compound PROTAC 64)

Refer to the third step of Example 89 for preparation.

$^1$H NMR (400MHz, DMSO-$d_6$) δ11.09(s, 1H), 8.55(d, *J*=8.2Hz, 1H), 7.84(dd, *J*=9.0, 7.2Hz, 2H), 7.69(s, 1H), 7.38(d, *J*=2.4Hz, 1H), 7.25(s, 1H), 7.13(dd, *J*=8.8, 2.4Hz, 1H), 6.85(d, *J*=9.3Hz, 1H), 5.08(dd, *J* = 12.8, 5.4Hz, 1H), 4.53(dt, *J*= 10.3, 5.6Hz, 1H), 4.26-4.12(m, 2H), 4.07(s, 2H), 3.95(dd, *J* = 9.0, 5.0Hz, 2H),3.86(d, *J*=11.6Hz, 1H),2.86(s, 3H), 2.69-2.51(m, 3H), 2.46(d, *J*=6.1Hz, 2H), 2.32(d, *J*=11.0Hz, 2H), 2.16-2.06(m, 2H), 2.06-1.93(m, 2H), 1.90(dt, *J*=8.1, 4.1Hz, 2H), 1.71-1.58(m, 2H), 1.52(td, *J*=9.7, 4.6Hz, 3H),1.42(d, *J*=14.3Hz, 2H).
LC-MS: $[M+H]^+$=793.8

Example 94

Synthesis of PROTAC 65

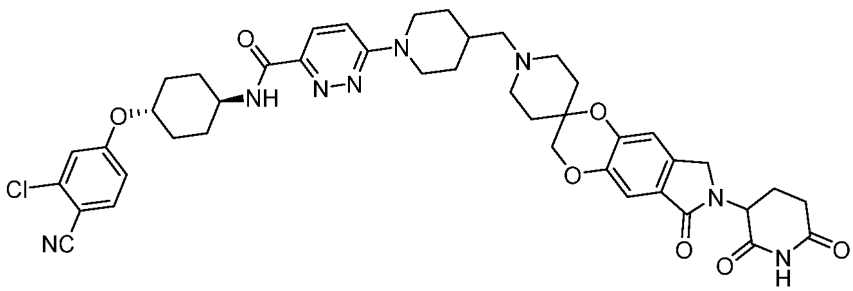

N-((1r,4r)-4-(3-chloro-4-cyanophenoxy)cyclohexyl)-6-(4-((7'-(2,6-dioxopiperidine-3-yl)-6'-oxo-7',8'-dihydro-3'H,6'-spiro [piperidine-4,2'-[1,4]dioxy[2,3-f ]isoindole]-1-methyl)piperidin-1-yl)pyridazine-3-carboxamide

Synthetic scheme:

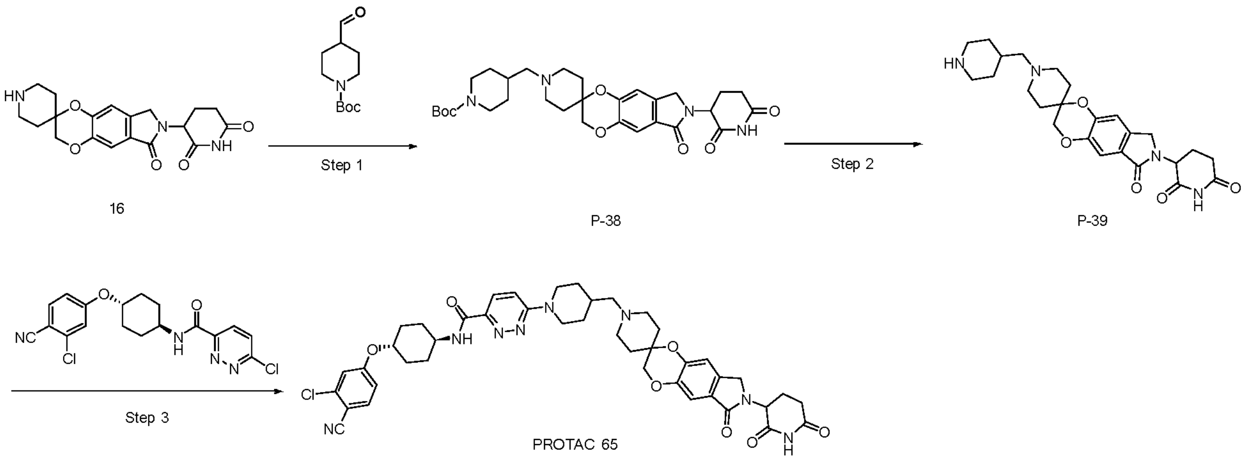


16

P-38

P-39

PROTAC 65

The first step: tert-butyl 4-piperidine-1-carboxylate (7'-(2,6-dioxopiperidin-3-yl)-6'-oxo-7',8'-dihydro- 3'H, 6'H-spiro[piperidin-4,2'-[1,4]dioxy[2,3-f]isoindol]-1-yl) (compound P-38)

Compound 16 (150mg, 0.404mmol) and tert-butyl 4-formylpiperidine-1-carboxylate (172.1mg, 0.808mmol) were successively added into a reaction flask, added solvent DCE (2mL), AcOH (4drop) in drops, and reacted 1 h at room temperature with stirring. Then $NaBH(OAc)_3$ (257 mg, 1.21 mmol) was added, and continued to react 1 h at room temperature with stirring. After reaction was completed, it was concentrated, and then purified by preparing thin layer chromatography to obtain white solid compound P-38 (130mg).

$^1$H NMR (600MHz, DMSO-$d_6$) δ10.95(s, 1H), 7.16(s, 1H), 7.09(s, 1H), 5.05(dd, *J*=13.4, 5.1Hz, 1H), 4.46(t, *J* = 5.3Hz, 1H), 4.30(d, *J* = 16.6Hz, 1H), 4.17(d, *J* = 16.7Hz, 1H), 4.09-4.00(m, 2H), 3.91(s, 3H), 3.27- 3.18(m, 1H), 2.94-2.83(m, 2H), 2.63-2.53(m, 3H), 2.40-2.24(m, 3H), 2.17-2.12(m, 2H), 2.04-1.91(m, 2H), 1.74-1.57(m, 6H), 1.38(s, 9H).
LC-MS: $[M+H]^+$=469.50

The second step: 3-(6'-oxo-1-(piperidin-4-ylmethyl)-6',8'-dihydro-3'H, 7'H-spiro[piperidin-2 ,2'-[1,4]dioxo[2,3-f]isoindol]-7'-yl)piperidine-1,6-dione (compound P-39)

Refer to the second step of Example 89 for preparation.
LC-MS: $[M+H]^+$=469.50

The third step: N-((1r,4r)-4-(3-chloro-4-cyanophenoxy)cyclohexyl)-6-(4-((7'-(2,6- dioxopiperidine-3-yl)-6'-oxo-7',8'-dihydro-3'H,6'-spiro[piperidin-4,2'-[1,4]dioxy[2 ,3-f]isoindole]-1-methyl)piperidin-1-yl)pyridazine-3-carboxamide (compound PROTAC 65)

Refer to the third step of Example 89 for preparation.

$^1$H NMR (400MHz, DMSO-$d_6$) δ10.96(s, 1H), 8.57(d, *J*=8.2Hz, 1H), 7.90-7.80(m, 2H), 7.44-7.35(m, 2H), 7.24(s, 1H), 7.18-7.10(m, 2H), 5.07(dd, *J* = 13.3, 5.0Hz, 1H), 4.60-4.46(m, 2H), 4.39-4.28(m, 1H), 4.26-4.10(m, 2H), 3.92-3.80(m, 1H), 3.63-3.45(m, 10H), 3.23-3.00(m, 5H), 2.98-2.83(m, 1H), 2.68-2.56(m, 1H), 2.43-2.28(m, 1H), 2.26-2.07(m, 3H), 2.04-1.81(m, 6H), 1.71-1.44(m, 4H).
LC-MS: $[M+H]^+$=823.77

Example 95

Synthesis of PROTAC 66

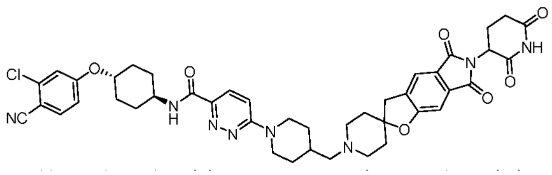

N-((1r,4r)-4-(3-chloro-4-cyanophenoxy)cyclohexyl)-6-(4-((6-(2,6-dioxopiperidine-3-yl)-5,7-dioxo-3,5,6,7-tetrahydrospiro[furo[2,3-f]isoindole-2,4'-piperidinyl]-1'-yl)methyl) piperidin-1-yl) pyridazine-3-carboxamide

Synthetic scheme:

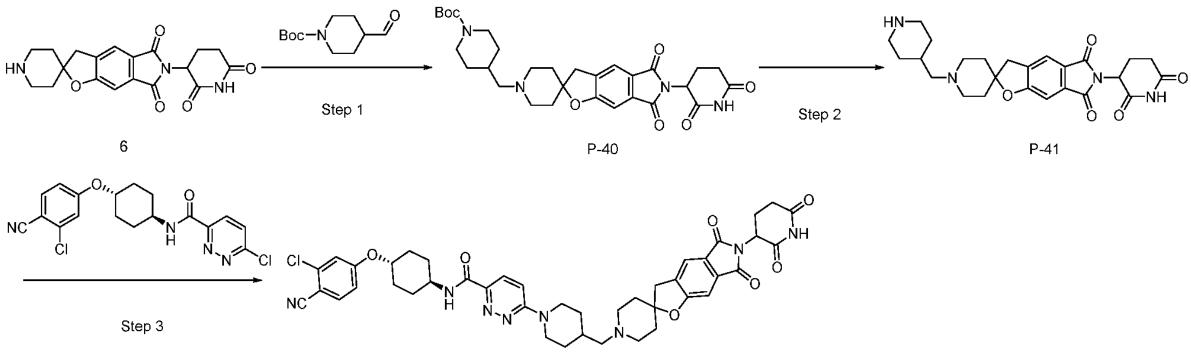


6

P-40

P-41

PROTAC 66

The first step: 4-(6-(2,6-dioxypiperidin-3-yl)-5,7-dioxo-3,5,6,7-tetrahydrospiro[furan[2,3-f] isoindole-2,4'-piperidin]-1'-yl) methyl)piperidine-1-carboxylate tert-butyl ester (compound P-40)

Compound 6 (80mg, 0.216mmol) and tert-butyl 4-formylpiperidine-1-carboxylate (92.4mg, 433mmol) were added successively into a glass vial, added solvent DCM (2mL) and AcOH (2drops) in drops, and reacted 1h at room temperature with stirring. Then $NaBH(OAc)_3$ (138 mg, 648 mmol) was added, and continued to react 1 h at room temperature with stirring. After the reaction was completed, the reaction mixture was concentrated, and then purified by TLC (DCM:MeOH=10:1) to obtain white solid compound P-40 (67.3mg).

The second step: 6-(2,6-dioxopiperidin-3-yl)-1'-(piperidin-4-ylmethyl)spiro[furo[2,3-f]isoindole-2 ,4'-piperidine]-5,7(3H,6H)-dione (compound P-41)

Refer to the second step of Example 89 for preparation.

The third step: N-((1r,4r)-4-(3-chloro-4-cyanophenoxy)cyclohexyl)-6-(4-((6-(2,6-dioxopiperene Pyridin-3-yl)-5,7-dioxo-3,5,6,7-tetrahydrospiro[furo[2,3-f]isoindole-2,4'-piperidine]-1'-yl)methyl)piperidin-1-yl)pyridazine-3-carboxamide (compound PROTAC 66)

Refer to the third step of Example 89 for preparation.

$^1$H NMR (600MHz, $CD_3OD$) δ7.99(d, *J*=9.7Hz, 1H), 7.71(d, *J*=8.8Hz, 1H), 7.63(s, 1H), 7.47(d, *J*=9.7Hz, 1H), 7.28(s, 1H), 7.21(d, *J*=2.3Hz, 1H), 7.06(dd, *J*=8.8, 2.3Hz, 1H), 5.11(dd, *J*=12.8, 5.4Hz, 1H), 4.62-4.49(m, 3H), 4.05-3.96(m, 1H), 3.75(dd, *J* = 59.4, 9.9Hz, 2H), 3.25-3.03(m, 6H), 2.94-2.60(m, 8H), 2.39 -2.28(m, 1H), 2.27-1.97(m, 8H), 1.66(dd, *J*=13.0, 7.1Hz, 4H), 1.42(ddd, *J*=77.8, 40.4, 18.7Hz, 3H).
LC-MS (ESI): $[M+H]^+$=821.68

Example 96

Synthesis of PROTAC 67

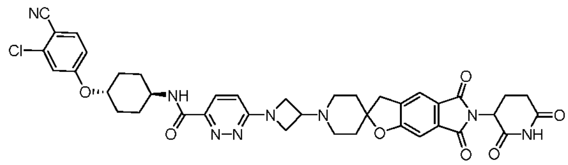

N-((1r,4r)-4-(3-chloro-4-cyanophenoxy)cyclohexyl)-6-(3-(6-(2,6-dioxypiperidin-3-yl )-5,7-dioxo-3,5,6,7-tetrahydrospiro [furo[2,3-f]isoindole-2,4'-piperidin]-1'-yl) azetidin-1-yl)pyridazine-3-carboxamide

Synthetic scheme:

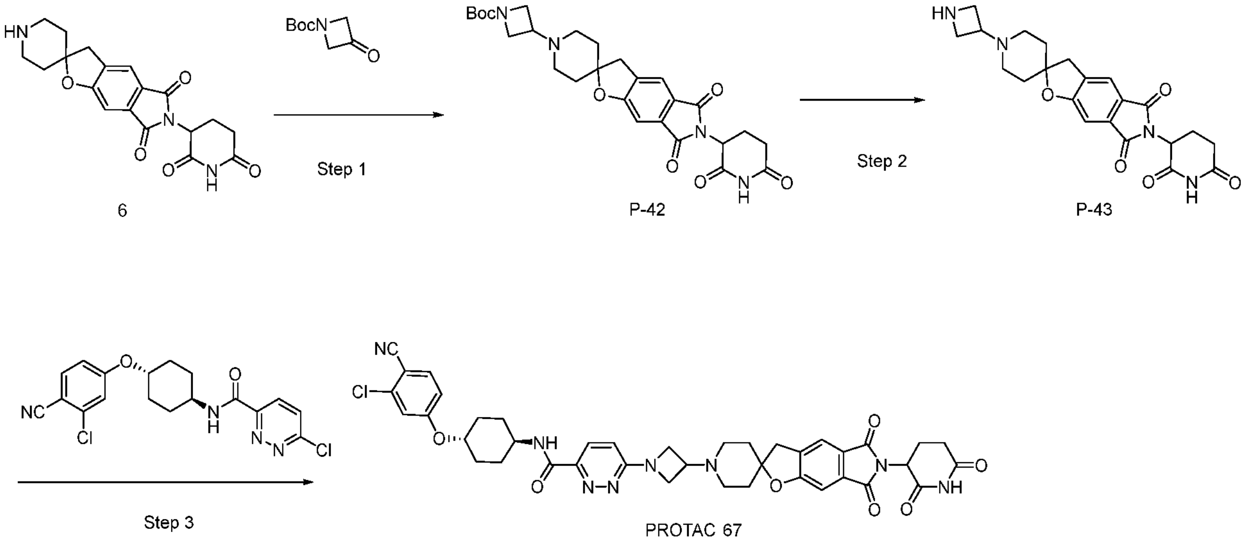


The first step: 3-(6-(2,6-dioxypiperidin-3-yl)-5,7-dioxo-3,5,6,7-tetrahydrospiro[furan[2,3-f]isoindole-2,4'-piperidin]-1'-yl) azetidinyl ester (compound P-42)

Compound 6 (60mg, 0.162mmol) and tert-butyl 3-oxoazetidine-1-carboxylate (41.71mg, 0.243mmol) were added successively in a glass vial. Solvent THF (2mL) and $Ti(O\text{-}iPr)_4$ (92.33mg, 0.324mmol) were added, stirred and reacted at 60°C for 1h. Then $NaBH_3CN$ (30.62mg, 0.487mmol) was added and stirring was continued to react at 60°C for 1h. After the reaction was completed, the reaction mixture was subjected to concentrated treatment, and then purified by prepared thin layer chromatography (DCM:MeOH=10:1) to obtain the white solid compound P-42 (30 mg).
LC-MS: $[M+H]^+$=525.43

The second step: 1'-(azetidin-3-yl)-6-(2,6-dioxopiperidin-3-yl)spiro[furo[2,3-f]isoindole- 2,4'-piperidine]-5,7(3H,6H)-dione (Compound P-43)

Refer to the second step of PROTAC 60 for preparation.
LC-MS: $[M+H]^+$=425.43

The third step: N-((1r,4r)-4-(3-chloro-4-cyanophenoxy)cyclohexyl)-6-(3-(6-(2,6-dioxypiperidine -3-yl)-5,7-dioxo-3,5,6,7-tetrahydrospiro[furo[2,3-f]isoindole-2,4'-piperidine]-1'- yl) azetidin-1-yl) pyridazine-3-carboxamide (compound PROTAC 67)

Refer to the third step of Example 89 for preparation.

$^1$H NMR (400MHz, $CD_3OD$) δ 7.97(d, J=8.4Hz, 1H), 7.69(d, J=8.7Hz, 1H), 7.60(s, 1H), 7.25(s, 1H), 7.20(d ,J=2.4Hz, 1H), 7.05(dd,J=8.8, 2.4Hz, 1H), 6.95(d,J=8.2Hz, 1H), 5.34(s, 1H), 4.42(m,4H), 4.24 (s,2H), 3.98(s, 1H), 3.89-3.58(m, 3H), 3.04-2.51(m, 9H), 2.27-1.96(m,6H), 1.63(q, J = 13.6, 11.5Hz, 4H).
LC-MS: $[M+H]^+$=779.59

Example 97

Synthesis of Intermediate 30

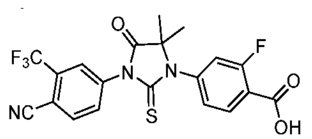

4-(3-(4-cyano-3-(trifluoromethyl)phenyl)-5,5-dimethyl-4-oxo-2-thioimidazolidin-1-yl)-2-fluorobenzoic acid

Synthetic scheme:

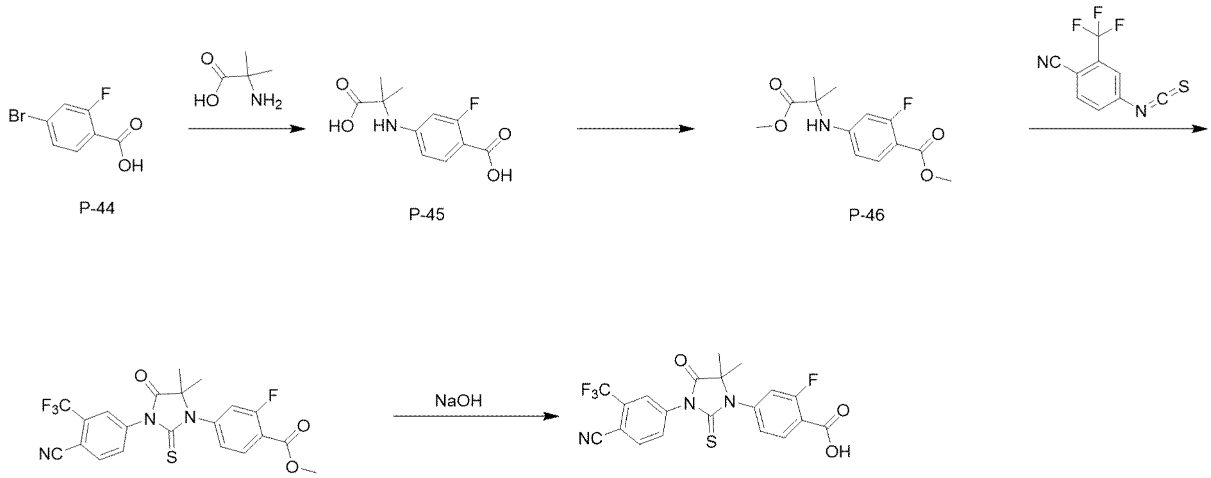


P-44 P-45 P-46 P-47 Intermediate 30

The first step: 4-((2-carboxyprop-2-yl)amino)-2-fluorobenzoic acid (P-45)

4-bromo-2-fluorobenzoic acid (5g, 22.83mmol), 2-metylalanine (7.06g, 68.49mmol), copper powder 2.90g, 45.66mmol), copper iodile (8.70g, 45.66mmol), potassium carbonate (15.78g, 114.15mmol) and N,N-dimetylglycine (1.17g, 11.41mmol) were added. Solvent DMF (100mL) and water (10mL) were added , and the reaction was stirred at 110°C for 16h. After cooling the reaction solution to room temperature, ice water (200mL) was added, and added, and ice hydrocloric acid was added dropwise to adjust the pH to 3-4. The mixture was extracted with ethyl acetate 3 times (500mL*3). The organic phase was concentrated and was then purified by positive phase column ( Ethyl acetate: petroleum ether = 35%) to obtain a yellow solid (P-45) (4g).
LC-MS: $[M+H]^+$=242.2

The second step: methyl 2-fluoro-4-((1-methoxy-2-methyl-1-oxopropan-2-yl)amino) benoate (P-46)

P-45 (3 g. 12.24 mmol) and solvent methanol (50 mL) were sequentially added into a one-necked flask. Thionulchloride (3.70g, 31.09mmol0 was added dropwise under ice-bath conditions, and the reaction was stirred at 80°C for 16h. After the reaction was completed, is was directly concentrated to obtain a yellow solid (P-46) (3 g).
LC-MS: $[M+H]^+$=270.7

The third step: methyl 4-(3-(4-cyano-3-(trifluoromehyl)phenyl)-5,5-dimethyl-4-oxo-2-thioimidazolidin-1-yl )-2-fluorobenzoate (P-47)

P-46 (1g, 3.71mmol), 4-isothiocyanate-2-(trifluoromethyl)benzonitrile (1.02g, 4.46mmol), isopropyl acetate

(10mL) and DMSO (5 mL) were sequentially added to a single-necked flask. The reaction was carried out at 95°C for 60 h under the protection of nitrogen. After the reaction was completed, it was concentrated until no solvent was evaporated, methanol was added dropwise, stirred and crystallized at 0-5°C, and intermediate )-47 (500 mg) was obtained by filtration. LC-MS (ESI): $[M+H]^+$=466.08

The fourth step: 4-(3-(4-cyano-3-(trifluoromethyl)phenyl)-5,5-dimethyl-4-oxo-2-thioimidazolidin-1-yl )-2-fluorobenzoic acid (intermediate 30)

Intermadiate P-47 (0.5g, 1.07mmol), sodium hydroxide (214.84mg, 5.37mmol), solvent methanol (5mL), tetrohydrofuran (5mL) and water (2.5mL) were sequentially added into a one-necked flask. The reaction mixture was reacted at 40°C for 2h. After the reaction is completed, water (10mL) was added and glacial hydrocloric acid was added dropwise to adjust the pH to 3-4. The reaction mixture was extracted 3 times with ethyl acetate (10mL*3). The organic phase was concentrated, and then purified by reverse phase column (acetonitrile: water=60%) to obtain a white solid (Intermediate 30) (300 mg).

$^1$H NMR (600MHz, DMSO-$d_6$) $\delta$13.55(s, 1H), 8.41(d, J=8.2Hz, 1H), 8.30(d, J = 1.0Hz, 1H), 8.13-8.01(m, 2H), 7.47(dd, J=11.0,1.9Hz,1H), 7.38(dd,J=8.3, 1.9Hz), 1.56(s,6H).

LC-MS (ESI): $[M+H]^+$=450.22

Example 98

Synthesis of PROTAC 68

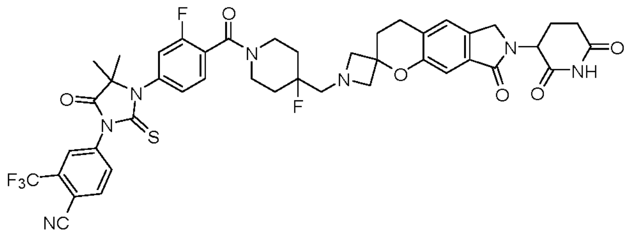

4-(3-(4-(4-(7'-(2,6-dioxopiperidin-3-yl)-8'-oxo-4',6',7',8'-tetrahyro-3'H-spiro[azetidin-3,2'-pyrano[2,3-f]isoindol]-1-yl) methyl)-4-fluoropiperidine-1-carbonyl)-3-fluorophenyl)-4,4-dimethyl-5-oxo-2-thioimidazolidin-1 -yl)-2-(trifluoromethyl) benzonitrile

Synthetic scheme:

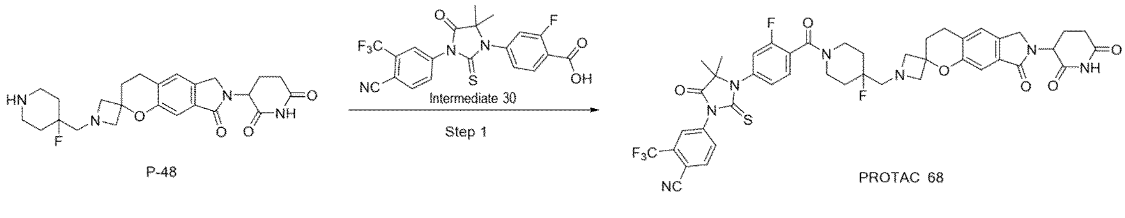


The first step: 4-(3-(4-(4-(7'-(2,6-dioxopiperidin-3-yl)-8'-oxo-4', 6', 7', 8'-tetrahydro-3'H-spiro [azetidine-3,2'-pyrano [2,3-f] isoindol]-1-yl)methyl)-4- fluoropiperidine-1-carbonyl)-3-fluorophenyl)-4,4-dimethyl-5-oxo-2-thioimidazolidin-1-yl)-2-(trifluoromethyl) benzonitrile (Compound PROTAC 68)

Compound P-48 (40mg, 0.08mmol), intermediate 30 (52.2mg, 0.12mmol), DIEA (3eq), BOP (3eq) and DMF (2mL) were sequentially added into an 8mL sealed tube. The reaction mixture was reacted at room temperature for 3 h under N2 conditions. After the reaction was completed, it was cooled to room temperature, concentrated, and then purified by preparation to obtain white solid compound PROTAC 68 (5 mg).

$^1H$ NMR (600MHz, MeOD) δ 8.21(t, J=20.7Hz, 2H), 8.02(dd, J=8.2, 1.8Hz, 1H), 7.63(s, 1H), 7.45-7.37(m, 1H), 7.29(s,1H), 7.23(s,1H), 5.13(dd,J=13.3,5.2Hz,1H), 4.57(d,J=13.2Hz,1H), 4.39(q,J=16.5Hz, 2H), 3.64(d, J=8.6Hz, 2H), 3.56(d, J=12.9Hz, 1H), 3.47(dd, J=26.8, 4.7Hz, 3H), 3.24(td, J=12.9, 2.4 Hz, 1H), 3.00-2.87(m, 5H), 2.82-2.75(m, 1H), 2.49(ddd, J = 26.5, 13.3, 4.6Hz, 1H), 2.22(t, J = 6.5Hz, 2H) ,2.18-2.13(m,1H),2.12-2.05(m,1H),1.96(d,J = 4.7Hz,1H), 1.78(ddd,J = 19.9,19.0,5.4Hz,2H),1.63(s ,6H).

LC-MS (ESI): $[M+H]^+$=890.27

Example 99

Synthesis of PROTAC 69

4-(3-(4-(4-(7'-(2,6-dioxopiperidin-3-yl)-6',8'-dioxo-4',6',7',8'-tetrahydro-2'H-spiro[piperidine-4,3'-pyrano[2,3-f]isoindole]-1-yl)methyl)piperidine-1-carbonyl)- 3-fluorophenyl)-4,4-dimethyl-5-oxo-2-thioimidazolidin-1-yl)-2-(trifluoromethyl) benzonitrile

Synthetic scheme:

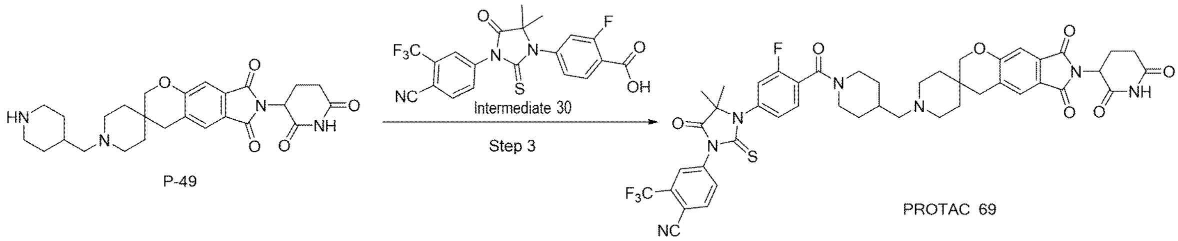


The first step: 4-(3-(4-(4-(7'-(2,6-dioxopiperidin-3-yl)-6',8'-dioxo-4',6' ,7',8'-tetrahydro-2'H-spiro[piperidine-4,3'-pyrano [2,3-f]isoindol]-1-yl)methyl)piperidine-1-carbonyl)-3-fluorophenyl)-4,4-dimethyl-5-oxo-2-thioimidazolidin-1-yl)-2-(trifluoromethyl)benzonitrile (compound PROTAC 69 )

Refer to the first step of Example 98 for preparation.

$^1H$ NMR (600MHz, DMSO-$d_6$) δ11.11(s, 1H), 8.42(d, J=8.2Hz, 1H), 8.30(d, J=1.3Hz, 1H), 8.09(dd, J=8.2, 1.5Hz, 1H), 7.73(s, 1H), 7.61(t, J = 7.7Hz, 1H), 7.46(dd, J = 9.9, 1.4Hz, 1H), 7.37-7.29(m, 2H), 5.10( dd,J=12.9,5.4Hz,1H),4.54(d,J=11.9Hz,1H),4.32(s,1H),4.04(s,1H),3.45(d,J=13.0Hz,4H), 3.21-3.02(m,7H), 2.88(dd,J = 20.1,9.0Hz,2H),2.80(s, 1H),2.57(dd,J = 38.4,10.8Hz,2H),2.16(s, 1H) ,2.06-2.00(m,1H),1.91(t,J=5.7Hz,1H),1.81-1.68(m,4H),1.62(d,J=13.6Hz,1H),1.57(s,6H).

LC-MS (ESI): $[M+H]^+$=914.29

Example 100

Synthesis of PROTAC 70

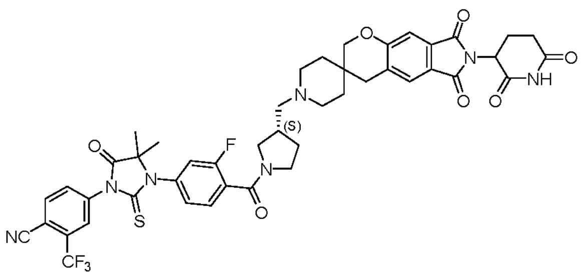

4-(3-(4-((3S)-3-((7'-(2,6-dioxopiperidin-3-yl)-6',8'-dioxo-4',6',7',8'-tetrahydro-2'H-spiro[piperidine-4,3'-pyrano[2,3-f]isoindole]-1-yl)methyl)pyrrolidine- 1-carbonyl)-3-fluorophenyl)-4,4-dimethyl-5-oxo-2-thioimidazolidin-1 -yl)-2-(trifluoromethyl)benzonitrile

Synthetic scheme:

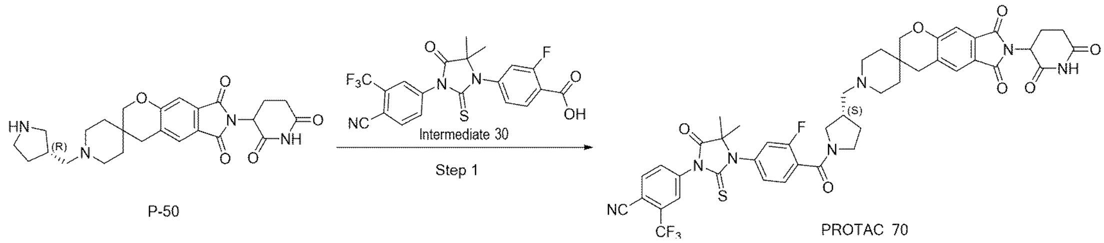


The first step: 4-(3-(4-((3S)-3-((7'-(2,6-dioxopiperidin-3-yl)-6',8'-dioxo- 4',6',7',8'-tetrahydro-2'H-spiro[piperidin-4,3'-pyrano[2,3-f]isoindol]-1-yl)methyl )pyrrolidin-1 -carbonyl)-3 -fluorophenyl)-4,4-dimethyl-5-oxo-2-thioimidazolidin-1-yl)-2-(trifluoromethyl) benzonitrile (Compound PROTAC 70)

Refer to the first step of Example 98 for preparation.

$^1$H NMR(600MHz,DMSO-$d_6$) δ11.11(s,1H),8.42(dd,J = 8.2, 2.6Hz, 1H), 8.30(s, 1H), 8.09(d, J = 8.2Hz,1H), 7.65(ddd,J=28.3,20.7,13.8Hz,2H), 7.52-7.44(m,1H),7.39-7.34(m,1H), 7.30(dd,J=13.4,5.0Hz,1H), 5.10 (dt, J=12.3,6.0Hz,1H), 4.31(d,J=32.9Hz,1H), 4.04(d,J=16.9Hz,1H), 3.89(dd,J=12.0,7.7Hz,1H), 3.67(d,J = 8.8Hz,1H), 3.40(s,3H), 3.30(s,2H),3.24-2.98(m,5H), 2.92-2.83(m,1H), 2.77(dd,J = 32.9,11.9Hz,2H), 2.63-2.56(m,1H), 2.21-1.98(m,3H), 1.81-1.60(m,5H), 1.56(s,5H).
LC-MS (ESI): $[M+H]^+$=890.27

Example 101

Synthesis of PROTAC 71

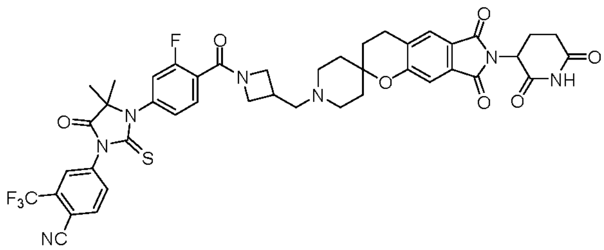

4-(3-(4-(3-(7'-(2,6-dioxopiperidin-3-yl)-6',8'-dioxo-4',6',7',8'-tetrahydro-3'H-spiro[piperidine-4,2'-pyrano[2,3-f]isoindol]-1-yl)methyl)azetidine-1- Carbonyl)-3-fluorophenyl)-4,4-dimethyl-5-oxo-2-thioimidazolidin-1-yl)-2-(trifluoromethyl) benzonitrile

Synthetic scheme:

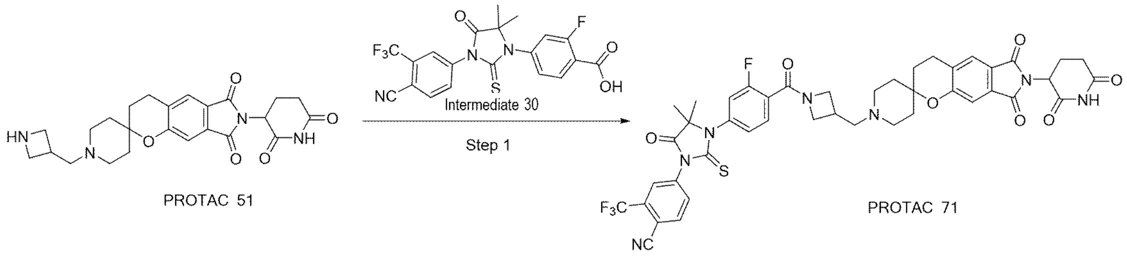


The first step: 4-(3-(4-(3-(7'-(2,6-dioxopiperidin-3-yl)-6',8'-dioxo-4',6',7',8'-tetrahydro-3'H-spiro[piperidine-4,2'-pyrano [2,3-f]isoindol]-1-yl)methyl) azetidine-1 -carbonyl)-3 -fluorophenyl)-4,4-dimethyl-5-oxo-2-thioimidazolidin-1-yl)-2-(trifluoromethyl)benzonitrile ( Compound PROTAC 71)

Refer to the first step of Example 98 for preparation.

$^1$H NMR (600MHz, DMSO-$d_6$) δ11.10(s, 1H), 8.42(d, J=8.2Hz, 1H), 8.29(d, J=1.2Hz, 1H), 8.10-8.07(m, 1H), 7.77-7.71(m,2H), 7.47(dd,J = 10.4,1.4Hz,1H), 7.42-7.34(m,2H), 5.10(dd,J = 12.9, 5.4Hz,1H), 4.26(t, J=9.1Hz, 2H), 3.96(m,3H), 3.52(s,2H), 3.37(dd,J=24.5,10.7Hz,2H), 3.21(dd,J=13.3,7.9Hz,3H), 2.99-2.84(m,3H), 2.60 (d, J = 17.4Hz, 2H), 2.09-1.95(m, 3H), 1.90(dd, J = 18.2, 11.2Hz,3H), 1.55(s, 6H).
LC-MS (ESI): $[M+H]^+$=886.26

Example 102

Synthesis of PROTAC 72

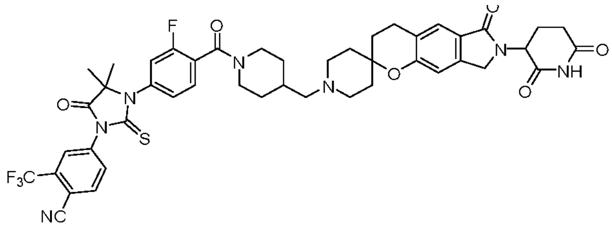

4-(3-(4-(4-(7'-(2,6-dioxopiperidin-3-yl)-6'-oxo-4',6',7',8'-tetrahydro-3'H-spiro[piperidine-4,2'-pyrano[2,3-f]isoindol]-1-yl) methyl)piperidine-1-carbonyl)-3- fluorophenyl )-4,4-dimethyl-5-oxo-2-thioimidazolidin-1-yl)-2-(trifluoromethyl)benzonitrile

Synthetic scheme:

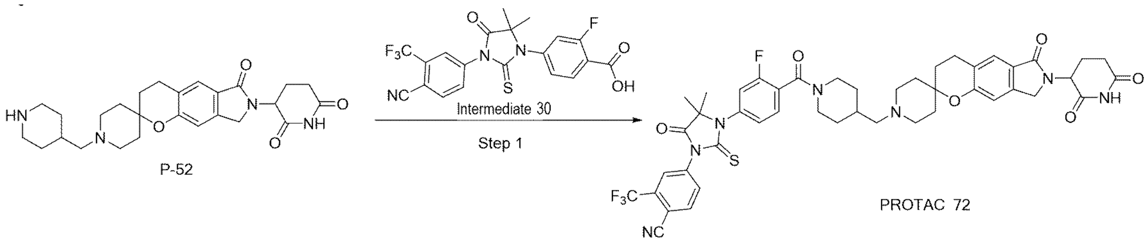


The first step: (3-(4-(4-(7'-(2,6-dioxopiperidin-3-yl)-6'-oxo-4', 6', 7', 8' -tetrahydro-3'H-spiro[piperidine-4,2'-pyrano[2,3-f] isoindol]-1-yl)methyl)piperidine-1-carbonyl)-3- fluorophenyl)-4,4-dimethyl-5-oxo-2-thioimidazolidin-1-yl)-2-(trifluoromethyl)benzonitrile (compound PROTAC 72)

Refer to the first step of Example 98 for preparation.

$^1$H NMR (600MHz, DMSO-d6) δ10.97(s, 1H), 8.42(d, J=8.2Hz, 1H), 8.30(d, J=1.4Hz, 1H), 8.09(dd, J=8.3, 1.5Hz, 1H), 7.61(t, J=7.8Hz, 1H), 7.52(d, J=5.6Hz, 1H), 7.46(dd, J=10.0, 1.3Hz, 1H), 7.37-7.32(m, 1H), 7.03(d, J=17.1Hz, 1H), 5.07(dd, J=13.3, 5.2Hz, 1H), 4.55(d, J=12.8Hz, 1H), 4.34(t, J=13.7Hz, 2H), 4.26-4.19(m, 2H), 3.46(d, J=10.5Hz, 3H), 3.16(d, J=29.2Hz, 5H), 2.94-2.82(m, 4H), 2.60(d,J =17.0Hz,1H),2.37 (dd,J=13.2,4.6Hz,1H),2.19(s,1H),2.09-2.03(m,1H),1.96(m,7H),1.76(s,1H) ,1.57(s,6H).
LC-MS (ESI): $[M+H]^+$=890.31

Example 103

Synthesis of PROTAC 73

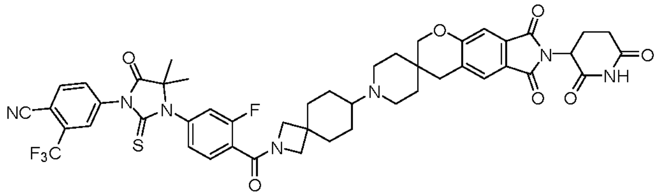

4-(3-(4-(7-(7'-(2,6-dioxopiperidin-3-yl)-6',8'-dioxo-4',6',7',8'-tetrahydro-2'H-spiro[piperidin-4,3'-pyrano[2,3-f]isoindol]-1-yl)-2-azaspiro[3.5]nonane -2-carbonyl)-3-fluorophenyl)-4,4-dimethyl-5-oxo-2-thioimidazolidin-1 -yl)-2-(trifluoromethyl) benzonitrile

Synthetic scheme:

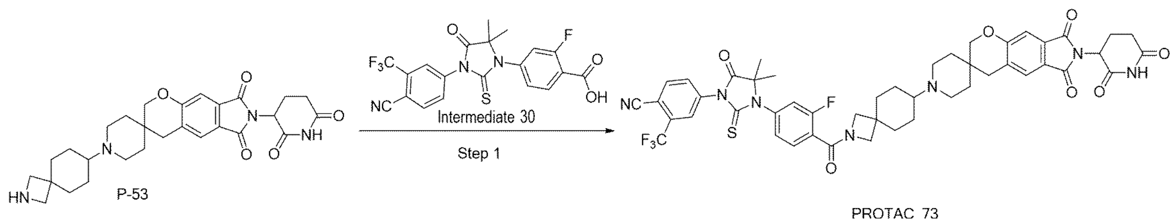


P-53

PROTAC 73

The first step: 4-(3-(4-(7-(7'-(2,6-dioxopiperidin-3-yl)-6',8'-dioxo-4',6', 7',8'-tetrahydro-2'H-spiro[piperidine-4,3'-pyrano [2,3-f]isoindol]-1-yl)-2-azaspiro[ 3.5] nonane-2-carbonyl)-3-fluorophenyl)-4,4-dimethyl-5-oxo-2-thioimidazolidin-1-yl)-2-(trifluoromethyl) benzonitrile (compound PROTAC 73)

Refer to the first step of Example 98 for preparation.

$^1$H NMR (600MHz, DMSO-$d_6$) δ11.11(d, J=4.8Hz, 1H), 8.42(d, J=8.3Hz, 1H), 8.30(s, 1H), 8.09(d, J=8.3Hz , 1H), 7.71(ddd,J=62.7,35.5,12.2Hz,2H),7.46(ddd, J=10.4, 4.8, 1.6Hz,1H), 7.39-7.33(m, 1H),7.29(dd, J=11.7, 3.9Hz,1H), 5.12-5.07(m, 1H), 4.31(d,J=19.1Hz,1H), 4.02(d,J=17.4Hz,1H),3.78(dd,J=39.2, 11.7Hz, 4H), 3.40-3.29(m, 2H), 3.14(ddd, J = 50.3, 31.4, 16.0Hz, 4H), 2.93-2.83(m, 1H), 2.77(d, J = 17.9Hz, 1H), 2.63-2.55(m,3H), 2.12-1.86(m,5H), 1.78-1.40(m, 13H).
LC-MS (ESI): $[M+H]^+$=940.30

Example 104

Synthesis of PROTAC 74

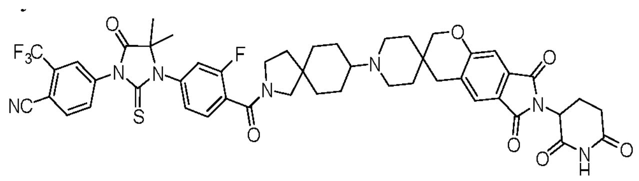

4-(3-(4-(8-(7'-(2,6-dioxopiperidin-3-yl)-6',8'-dioxo-4',6',7',8'-tetrahydro-2'H-spiro[piperidin-4,3'-pyrano[2,3-f]isoindol]-1-yl)-2-azaspiro[4.5]decane -2-carbonyl)-3-fluorophenyl)-4,4-dimethyl-5-oxo-2-thioimidazolidin-1 -yl)-2-(trifhioromethyl) benzonitrile

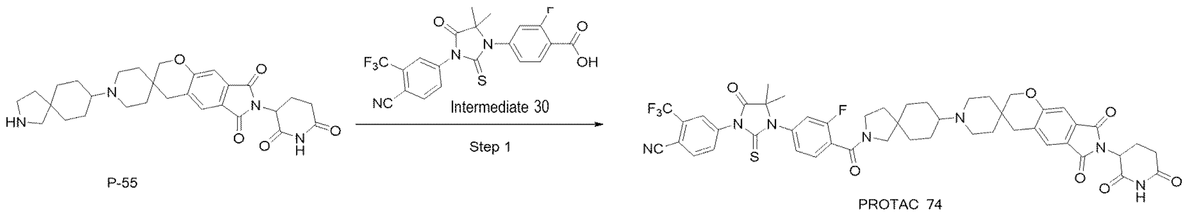


P-55

PROTAC 74

The first step: 4-(3-(4-(8-(7'-(2,6-dioxopiperidin-3-yl)-6',8'-dioxo-4',6' , 7',8'-tetrahydro-2'H-spiro[piperidine-4,3'-pyrano [2,3-f]isoindol]-1-yl)-2-azaspiro[4.5] decane-2-carbonyl)-3-fluorophenyl)-4,4-dimethyl-5-oxo-2-thioimidazolidin-1-yl)-2-(trifluoromethyl) benzonitrile (compound P-55)

Refer to the first step of Example 98 for preparation

$^1$H NMR (600MHz, DMSO) δ11.11(s, 1H), 8.42(d, J=8.2Hz, 1H), 8.33-8.26(m, 1H), 8.08(t, J=10.5Hz, 1H), 7.78 -7.58(m,2H), 7.45(d,J=10.2Hz,1H), 7.35-7.27(m,2H), 5.15-5.03(m,1H), 4.33(d,J=8.5Hz,1H), 4.03(t, J=12.5Hz, 1H), 3.49-3.30(m,6H), 3.27-3.03(m,6H), 2.93-2.83(m,1H), 2.77(dd,J=28.0,9.5Hz, 1H), 2.64-2.55(m,1H), 2.09-1.94(m,3H), 1.91-1.66(m,7H), 1.63-1.53(m,7H), 1.50(d, J = 2.8Hz,1H), 1.45-1.34(m, 2H).
LC-MS (ESI): $[M+H]^+$=954.35

Example 105

Synthesis of PROTAC 75

4-(3-(4-(4-(7'-(2,6-dioxopiperidin-3-yl)-6',8'-dioxo-4',6',7',8'-tetrahydro-3'H-spiro[azetidine-3,2'-pyrano[2,3-f]isoindol]-1-yl)methyl)-4- methoxypiperidine-1-carbonyl)-3-fluorophenyl)-4,4-dimethyl-5-oxo-2-thioimidazolidin-1-yl)-2-(trifluoromethyl) benzonitrile

Synthetic scheme:

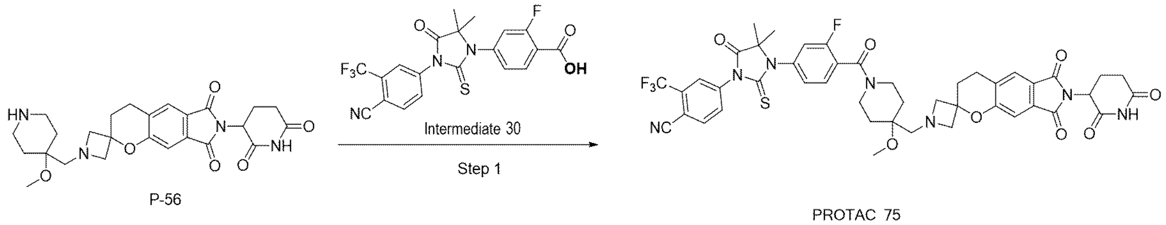


P-56

PROTAC 75

The first step: 4-(3-(4-(4-(7'-(2,6-dioxopiperidin-3-yl)-6',8'-dioxo-4',6' ,7',8'-tetrahydro-3'H-spiro[azetidine-3,2'-pyrano [2,3-f]isoindol]-1-yl)methyl)- 4-methoxypiperidine-1-carbonyl)-3-fluorophenyl)-4,4-dimethyl-5-oxo-2-thioimidazolidin-1-yl)-2-(trifluoromethyl)benzonitrile (Compound PROTAC 75)

Refer to the first step of Example 98 for preparation.

$^1$H NMR (600MHz, DMSO-$d_6$) δ11.12(s, 1H), 8.41(t, J=13.1Hz, 1H), 8.30-8.24(m, 1H), 8.10(t, J=11.0Hz, 1H), 7.79(d,J=13.4Hz,1H), 7.64-7.57(m,1H), 7.46(d,J=9.8Hz,1H),7.36(d,J=7.8Hz, 1H),7.29(s, 1H), 5.11(dd, J = 12.9, 5.4Hz, 1H), 4.61-4.21(m, 6H), 3.66(s, 1H), 3.56(s, 1H), 3.26(d, J = 21.9Hz, 2H), 3.23(s,3H), 3.11(d,J = 9.6Hz,1H), 3.00-2.83(m,4H),2.63-2.54(m,2H),2.29(s,2H), 2.08-1.99( m,1H), 1.87(d,J=9.9Hz, 1H), 1.76(s,1H), 1.59(s,6H).
LC-MS (ESI): $[M+H]^+$=916.92

Example 106

Synthesis of PROTAC 76

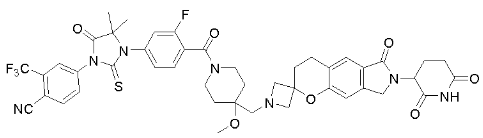

4-(3-(4-(4-(7'-(2,6-dioxopiperidin-3-yl)-6'-oxo-4',6',7',8'-tetrahydro-3'H-spiro[azetidine-3,2'-pyrano[2,3-f]isoindol]-1-yl) methyl)-4-methoxypiperidine- 1-carbonyl)-3-fluorophenyl)-4,4-dimethyl-5 - oxo-2-thioimidazolidin-1-yl)-2-(trifluoromethyl)benzonitrile

Synthetic scheme:

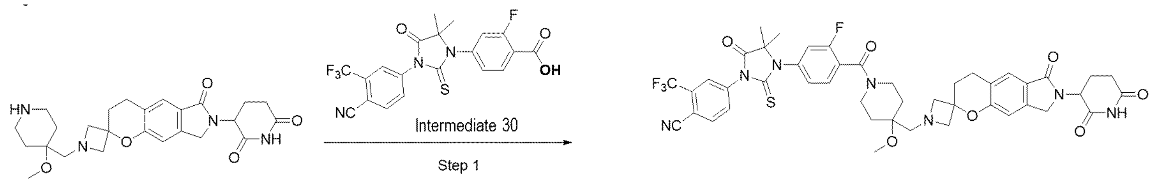

P-57
PROTAC 76

The first step: 4-(3-(4-(4-(7'-(2,6-dioxopiperidin-3-yl)-6'-oxo-4', 6', 7', 8'-tetrahydro-3'H-spiro[azetidine-3,2'-pyrano[2,3-f]isoindol]-1-yl)methyl)-4-methoxypiperidin-1-carbonyl)-3-fluorophenyl)-4,4-dimethyl-5-oxo-2-thioimidazolidin-1-yl)-2-(trifluoromethyl)benzyl Nitrile (compound PROTAC 76)

Refer to the first step of Example 98 for the preparation.

$^1$H NMR (600 MHz, DMSO-d6) δ 10.97 (s, 1H), 8.42 (d, J = 8.3 Hz, 1H), 8.29 (d, J = 1.4 Hz, 1H), 8.09 (dd, J = 8.2, 1.6 Hz, 1H), 7.61 (t, J = 7.8 Hz, 1H), 7.52 (s, 1H), 7.46 (d, J = 9.9 Hz, 1H), 7.36 (d, J = 8.1 Hz, 1H), 7.05 (t, J = 22.5 Hz, 1H), 5.07 (dd, J = 13.3, 5.0 Hz, 1H), 4.55 (s, 1H), 4.44 (s, 1H), 4.41 - 4.20 (m, 4H), 3.68 - 3.51 (m, 3H), 3.28 (s, 2H), 3.22 (s, 3H), 3.11 (d, J = 9.5 Hz, 1H), 2.95 - 2.85 (m, 3H), 2.63 - 2.56 (m, 1H), 2.42 - 2.32 (m, 1H), 2.25 (s, 2H), 2.03 - 1.94 (m, 1H), 1.87 (d, J = 10.6 Hz, 1H), 1.76 (s, 1H), 1.68 - 1.58 (m, 1H), 1.56 (s, 6H).
LC-MS (ESI): $[M+H]^+$=902.93

Example 107

Synthesis of PROTAC 77

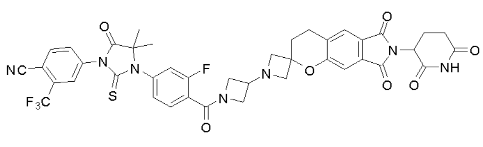

4-(3-(4-(3-(7'-(2,6-dioxopiperidin-3-yl)-6',8'-dioxo-4',6',7',8'-tetrahydro-3'H-spiro[azetidine-3,2'-pyrano[2,3-f]isoindole]-1-yl)azetidine-1- carbonyl)-3-fluorophenyl)-4,4-dimethyl-5-oxo-2-thioimidazolidin-1 -yl)-2-(trifhioromethyl)benzonitrile

Synthetic scheme:

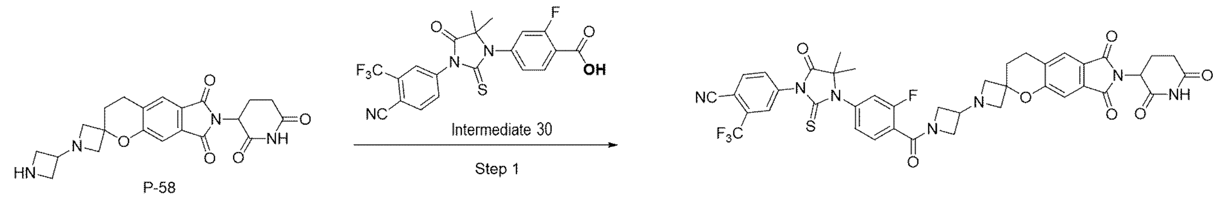


P-58

PROTAC 77

The first step: 4-(3-(4-(3-(7'-(2,6-dioxopiperidin-3-yl)-6',8'-dioxo-4',6' ,7',8'-tetrahydro-3'H-spiro[azetidine-3,2'-pyrano[2,3-f]isoindol]-1-yl) azetidine-1-carbonyl)-3-fluorophenyl)-4,4-dimethyl-5-oxo-2-thioimidazolidin-1-yl)-2-(trifluoromethyl)benzonitrile ( Compound PROTAC77)

Refer to the first step of Example 98 for preparation.

$^1$H NMR (600 MHz, DMSO-d6) $\delta$ 11.12 (s, 1H), 8.42 (d, J = 8.3 Hz, 1H), 8.28 (t, J = 19.0 Hz, 1H), 8.16 - 7.98 (m, 1H), 7.81 - 7.67 (m, 2H), 7.48 (dt, J = 26.3, 13.1 Hz, 1H), 7.41 - 7.29 (m, 1H), 7.30 (s, 1H), 5.11 (dd, J = 12.9, 5.4 Hz, 1H), 4.45 - 3.99 (m, 8H), 2.95 (t, J = 6.0 Hz, 2H), 2.89 (ddd, J = 17.1, 14.1, 5.4 Hz, 1H), 2.61 (m, 2H), 2.22 (t, J = 6.2 Hz, 2H), 2.07 - 1.98 (m, 2H), 1.55 (s, 6H).
LC-MS(ESI): $[M+H]^+$ = 902.93

Example 108

Synthesis of PROTAC 78

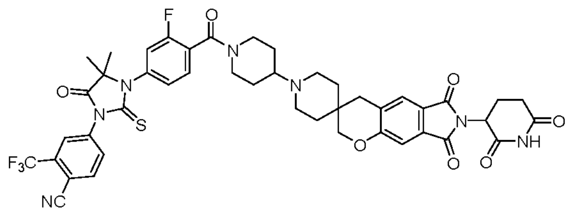

4-(3-(4-(4-(7'-(2,6-dioxopiperidin-3-yl)-6',8'-dioxo-4',6',7',8'-tetrahydro-2'H-spiro[piperidine-4,3'-pyrano[2,3-f]isoindol]-1-yl)piperidine-1-carbonyl)-3-fluorophenyl)-4,4-dimethyl-5-oxo-2-thioimidazolidin-1-yl)-2-(trifluoromethyl)benzonitrile

Synthetic scheme:

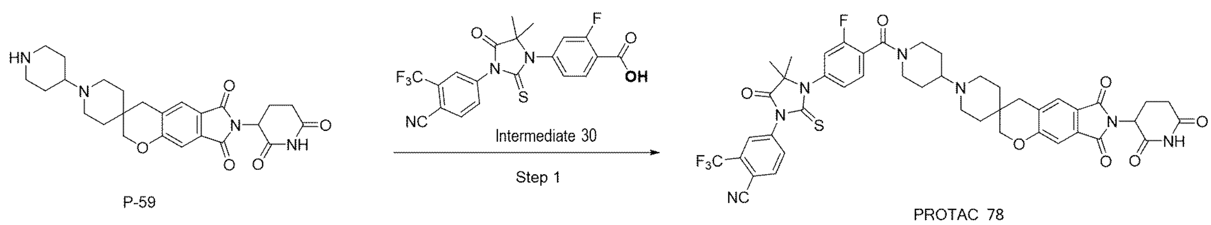


P-59

PROTAC 78

The first step: 4-(3-(4-(4-(7'-(2,6-dioxopiperidin-3-yl)-6',8'-dioxo-4',6' , 7',8'-tetrahydro-2'H-spiro[piperidine-4,3'-pyrano [2,3-f]isoindole]-1-yl)piperidine-1-carbonyl) -3-fluorophenyl)-4,4-dimethyl-5-oxo-2-thioimidazolidin-1-yl)-2-(trifluoromethyl)benzonitrile (compound PROTAC 78)

Refer to the first step of Example 98 for preparation.

$^1$H NMR (600 MHz, DMSO-d6) δ 11.11 (s, 1H), 8.42 (d, J = 8.3 Hz, 1H), 8.30 (s, 1H), 8.09 (dd, J = 8.2, 1.3 Hz, 1H), 7.75 - 7.60 (m, 2H), 7.48 (dd, J = 10.0, 1.4 Hz, 1H), 7.36 (dt, J = 12.7, 6.3 Hz, 1H), 7.30 (d, J = 3.9 Hz, 1H), 5.10 (dd, J = 12.9, 5.4 Hz, 1H), 4.70 (d, J = 12.3 Hz, 1H), 4.33 (s, 1H), 4.04 (s, 1H), 3.63 - 3.51 (m, 3H), 3.18 (d, J = 11.1 Hz, 3H), 3.08 (t, J = 21.2 Hz, 1H), 2.95 - 2.83 (m, 2H), 2.80 (s, 1H), 2.66 - 2.56 (m, 2H), 2.22 (s, 1H), 2.09 (d, J = 12.4 Hz, 1H), 2.01 (ddd, J = 19.3, 11.2, 6.0 Hz, 2H), 1.69 (dd, J = 33.8, 13.8 Hz, 6H), 1.57 (s, 6H).
LC-MS(ESI): $[M+H]^+$ = 900.92

Example 109

Synthesis of PROTAC 79

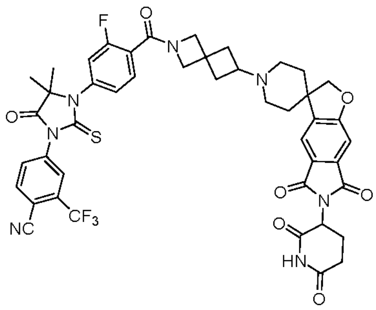

4-(3-(4-(6-(6-(2,6-dioxopiperidin-3-yl)-5,7-dioxo-6,7-dihydro-2H,SH-spiro [furo[2,3-f]isoindole-3,4'-piperidin]-1'-yl)-2-azaspiro[3.3]heptane-2-carbonyl)-3-fluorophenyl) -4,4-dimethyl-5-oxo-2-thioimidazolidin-1 -yl)-2-(trifluoromethyl)benzonitrile

Synthetic scheme:

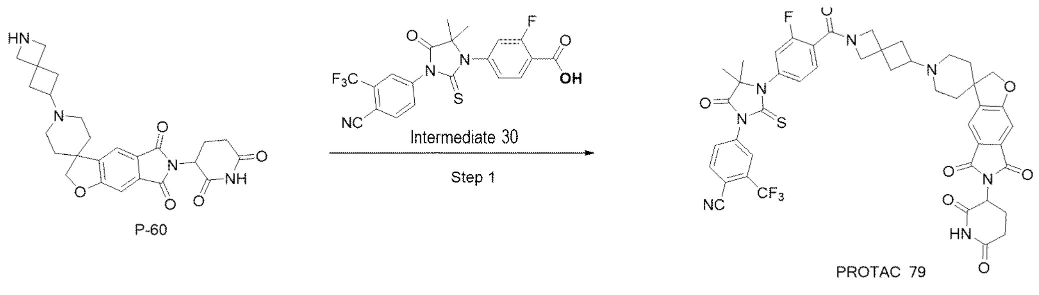


P-60

PROTAC 79

The first step: 4-(3-(4-(6-(6-(2,6-dioxopiperidin-3-yl)-5,7-dioxo-6,7-dihydro-2H , 5H-spiro[furo[2,3-f]isoindole-3,4'-piperidin]-1'-yl)-2-azaspiro[3.3]heptane-2-carbonyl)-3-fluorophenyl)-4,4-dimethyl-5-oxo-2-thioimidazolidin-1-yl)-2-(trifluoromethyl)benzonitrile (compound PROTAC 79)

Refer to the first step of Example 98 for preparation.

$^1$H NMR (600 MHz, DMSO-d6) δ 11.14 - 11.08 (m, 1H), 8.42 (d, J = 8.2 Hz, 1H), 8.29 (t, J = 9.1 Hz, 1H), 8.13 - 8.03 (m, 1H), 7.74 (dt, J = 7.8, 4.7 Hz, 1H), 7.66 (d, J = 13.0 Hz, 1H), 7.47 (t, J = 10.4 Hz, 1H), 7.35 (dd, J = 22.0, 13.7 Hz, 2H), 5.12 (ddd, J = 12.6, 11.9, 5.6 Hz, 1H), 4.72 (dd, J = 18.7, 3.4 Hz, 2H), 4.25 - 4.16 (m, 2H), 4.10 (s, 2H), 3.33 - 3.17 (m, 2H), 2.97 - 2.82 (m, 3H), 2.68 - 2.56 (m, 3H), 2.55 - 2.45 (m, 1H), 2.44 - 2.36 (m, 2H), 2.28 - 2.08 (m, 2H), 2.02 (dd, J =

23.3, 18.5 Hz, 3H), 1.85 (s, 1H), 1.55 (s, 6H).
LC-MS(ESI): $[M+H]^+$ = 898.90

Example 110

Synthesis of PROTAC 80

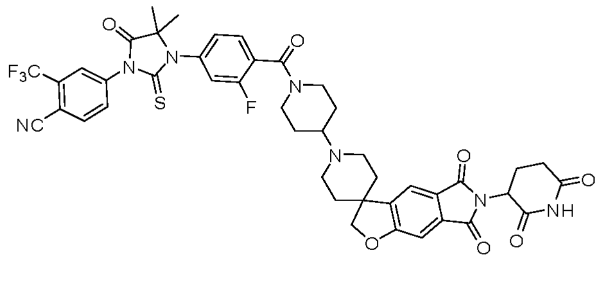

4-(3-(4-(4-(6-(2,6-dioxopiperidin-3-yl)-5,7-dioxo-6,7-dihydro-2H,SH-spiro [furo[2,3-f]isoindole-3,4'-piperidin]-1'-yl)piperidine-1-carbonyl)-3-fluorophenyl)-4,4-dimethyl- 5-oxo-2-thioimidazolidin-1-yl)-2-(trifluoromethyl)benzonitrile

Synthetic scheme:

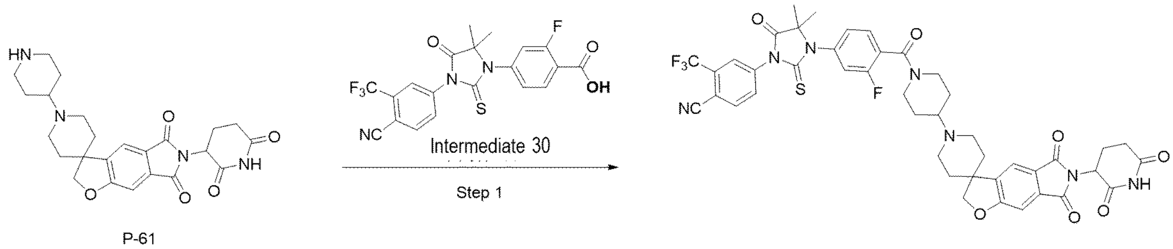

P-61

PROTAC 80

The first step: 4-(3-(4-(4-(6-(2,6-dioxopiperidin-3-yl)-5,7-dioxo-6,7-dihydro-2H, 5H-spiro[furo[2,3-f]isoindole-3,4'-piperidin]-1'-yl)piperidine-1-carbonyl)-3-fluorophenyl)-4,4-dimethyl-5-oxo-2-thioimidazolidin-1-yl)-2-(trifluoromethyl)benzonitrile (compound PROTAC 80)

Refer to the first step of Example 98 for preparation.

$^1$H NMR (600 MHz, DMSO-d6) δ 11.12 (s, 1H), 8.42 (d, J = 8.3 Hz, 1H), 8.28 (t, J = 14.1 Hz, 1H), 8.09 (dd, J = 8.2, 1.6 Hz, 1H), 7.65 (d, J = 12.6 Hz, 2H), 7.52 - 7.45 (m, 1H), 7.43 - 7.34 (m, 2H), 5.16 - 5.07 (m, 1H), 4.79 - 4.68 (m, 3H), 3.59 (d, J = 10.8 Hz, 4H), 3.18 (dd, J = 57.5, 11.3 Hz, 3H), 2.94 - 2.83 (m, 2H), 2.69 - 2.56 (m, 2H), 2.23 (s, 3H), 2.06 (dd, J = 20.0, 6.9 Hz, 3H), 1.97 - 1.85 (m, 1H), 1.72 (d, J = 47.8 Hz, 2H), 1.57 (s, 6H).
LC-MS(ESI): $[M+H]^+$ = 886.89

Example 111

Synthesis of PROTAC 81

4-(3-(4-(3-(6-(2,6-dioxopiperidin-3-yl)-5,7-dioxo-6,7-dihydro-2H,5H-spiro[furo[2,3-f]isoindole-3,4'-piperidin]-1'-yl)azetidine-1-carbonyl)-3-fluorophenyl)-4,4-dimethyl-5-oxo-2-thioimidazolidin-1-yl)-2-(trifluoromethyl)benzonitrile

Synthetic scheme:

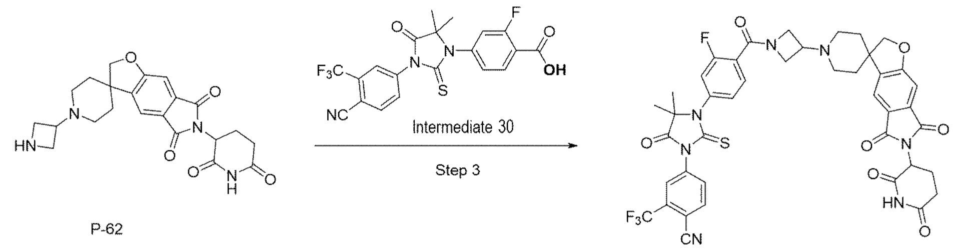


P-62

PROTAC 81

The first step: 4-(3-(4-(3-(6-(2,6-dioxopiperidin-3-yl)-5,7-dioxo-6,7-dihydro-2H, 5H-spiro[furo[2,3-f]isoindole-3,4'-piperidin]-1'-yl)azetidine-1-carbonyl)-3-fluorophenyl)-4,4-dimethyl-5-oxo-2-thioimidazolidin-1-yl)-2-(trifluoromethyl)benzonitrile (compound PROTAC 81)

Refer to the first step of Example 98 for preparation.

$^1$H NMR (600 MHz, DMSO-d6) δ 11.12 (s, 1H), 8.41 (d, J = 8.3 Hz, 1H), 8.30 (d, J = 1.6 Hz, 1H), 8.19 - 8.04 (m, 1H), 7.76 (t, J = 7.7 Hz, 2H), 7.48 (d, J = 10.0 Hz, 1H), 7.41 - 7.25 (m, 2H), 5.10 (dd, J = 13.0, 5.3 Hz, 1H), 4.66 (s, 2H), 4.07 (m, 7H), 2.93 - 2.83 (m, 2H), 2.64 - 2.55 (m, 2H), 2.02 (ddd, J = 20.5, 19.7, 14.6 Hz, 4H), 1.76 (s, 2H), 1.55 (s, 6H). LC-MS(ESI): $[M+H]^+$ = 858.84

Example 112

Synthesis of PROTAC 82

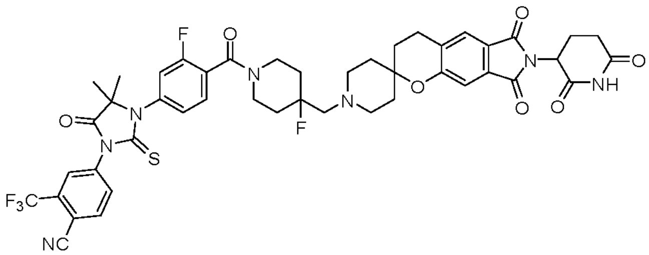

4-(3-(4-(4-(7'-(2,6-dioxopiperidin-3-yl)-6',8'-dioxo-4',6',7',8'-tetrahydro-3'H-spiro[piperidine-4,2'-pyrano[2,3-f]isoindol]-1-yl)methyl)-4-fluoropiperidine-1-carbonyl)-3-fluorophenyl)-4,4-dimethyl-5-oxo-2-thioimidazolidin-1-yl)-2-(trifluoromethyl)benzonitrile

Synthetic scheme:

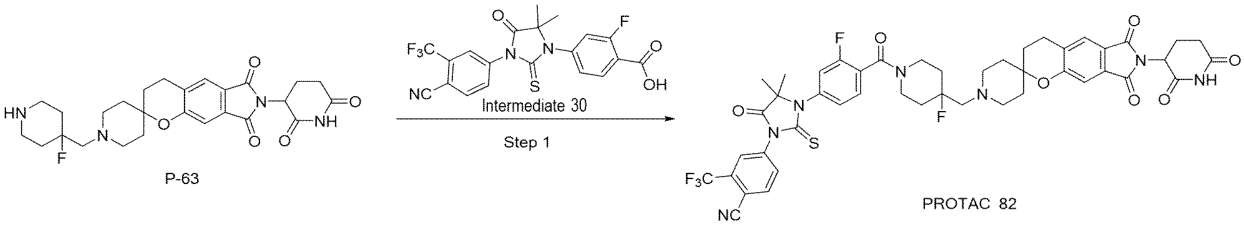


P-63

PROTAC 82

The first step: 4-(3-(4-(4-(7'-(2,6-dioxopiperidin-3-yl)-6',8'-dioxo-4',6' ,7',8'-tetrahydro-3'H-spiro[piperidine-4,2'-pyrano [2,3-f ]isoindol]-1-yl)methyl)-4-fluoropiperidine-1-carbonyl)-3-fluorophenyl)-4,4-dimethyl-5-oxo-2-thioimidazolidin-1-yl)-2-(trifluoromethyl)benzonitrile (Compound PROTAC 82)

Refer to the first step of Example 98 for preparation

$^1$H NMR (600 MHz, DMSO-d6) δ 11.10 (s, 1H), 8.42 (d, J = 8.2 Hz, 1H), 8.30 (s, 1H), 8.09 (d, J = 8.2 Hz, 1H), 7.70 (s, 1H), 7.64 (t, J = 7.8 Hz, 1H), 7.45 (dd, J = 10.0, 1.4 Hz, 1H), 7.35 (d, J = 8.1 Hz, 1H), 7.22 (s, 1H), 5.09 (dd, J = 12.9, 5.5 Hz, 1H), 4.31 (d, J = 13.0 Hz, 2H), 3.20 - 3.12 (m, 2H), 2.95 - 2.87 (m, 2H), 2.71 - 2.54 (m, 6H), 2.06 - 1.94 (m, 3H), 1.92 - 1.81 (m, 3H), 1.75 - 1.62 (m, 5H), 1.54 (d, J = 36.8 Hz, 5H).
LC-MS(ESI): $[M+H]^+$ = 932.01

Example 113

Synthesis of PROTAC 83

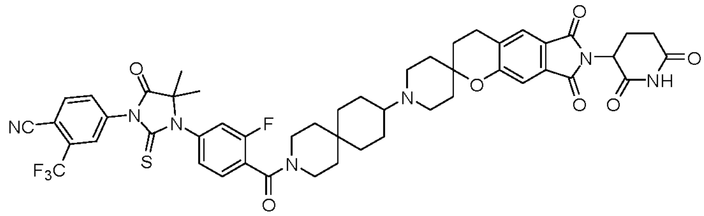

4-(3-(4-(9-(7'-(2,6-dioxopiperidin-3-yl)-6',8'-dioxo-4',6',7',8'-tetrahydro-3'H-spiro[piperidine-4,2'-pyrano [2,3-f]isoindol]-1-yl)-3-azaspiro[5.5]undecane-3-carbonyl)-3-fluorophenyl)-4,4-dimethyl-5-oxo-2-thioimidazolidin-1-yl)-2-(trifluoromethyl)benzonitrile

Synthetic scheme:

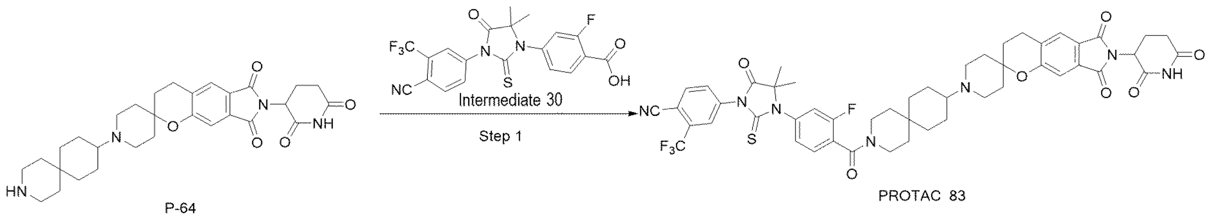


P-64

PROTAC 83

The first step: 4-(3-(4-(9-(7'-(2,6-dioxopiperidin-3-yl)-6',8'-dioxo-4',6', 7',8'-tetrahydro-3'H-spiro[piperidine-4,2'-pyrano [2,3-f]isoindol]-1-yl)-3-azaspiro[5.5] undecane-3-carbonyl)-3-fluorophenyl)-4,4-dimethyl-5-oxo-2-thioimidazolidin-1-yl)-2-(trifluoromethyl ) benzonitrile (compound PROTAC 83)

Refer to the first step of Example 98 for preparation.

$^1$H NMR (600 MHz, DMSO-$d_6$) δ 11.10 (s, 1H), 8.41 (d, J = 8.3 Hz, 1H), 8.30 (d, J = 1.5 Hz, 1H), 8.09 (dd, J = 8.2, 1.7 Hz, 1H), 7.69 (d, J = 8.4 Hz, 1H), 7.60 (td, J = 7.8, 4.9 Hz, 1H), 7.43 (ddd, J = 9.9, 4.4, 1.7 Hz, 1H), 7.38 - 7.29 (m, 1H), 720 (d, J = 14.6 Hz, 1H), 5.12 - 5.04 (m, 1H), 3.62 (d, J = 26.6 Hz, 2H), 3.23 (d, J = 7.3 Hz, 5H), 2.88 (m, 3H), 2.66 - 2.56 (m, 4H), 2.32 (d, J = 10.9 Hz, 1H), 2.02 (dd, J = 11.3, 5.2 Hz, 1H), 1.79 (m, 5H), 1.64 (d, J = 27.9 Hz, 5H), 1.56 (s, 5H), 1.47 - 1.21 (m, 6H), 1.17 - 1.06 (m, 2H).
LC-MS(ESI): $[M+H]^+$ = 968.34

Example 114

Synthesis of PROTAC 84

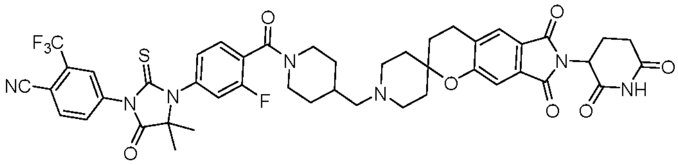

4-(3-(4-(4-(7'-(2,6-dioxopiperidin-3-yl)-6',8'-dioxo-4',6',7',8'-tetrahydro-3'H-spiro[piperidine-4,2'-pyrano[2,3-f]isoindol]-1-yl)methyl)piperidine-1-carbonyl)- 3-fluorophenyl)-4,4-dimethyl-5-oxo-2-thioimidazolidin-1-yl)-2-(trifhioromethyl) benzonitrile

Synthetic scheme:

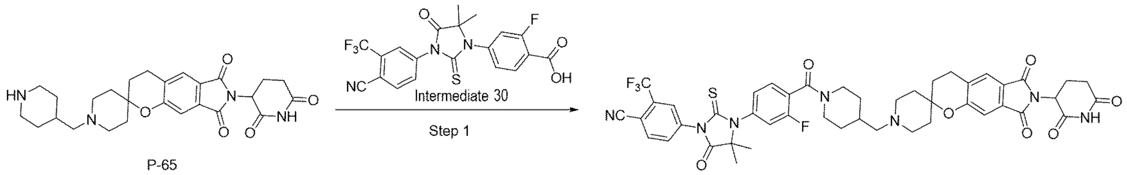


P-65

PROTAC 84

The first step: 4-(3-(4-(4-(7'-(2,6-dioxopiperidin-3-yl)-6',8'-dioxo-4',6' ,7',8'-tetrahydro-3'H-spiro[piperidine-4,2'-pyrano [2,3-f]isoindol]-1-yl)methyl)piperidine-1 -carbonyl)-3-fluorophenyl)-4,4-dimethyl-5-oxo-2-thioimidazolidin-1-yl)-2-(trifluoromethyl)benzonitrile (compound PROTAC 84 )

Refer to the first step of Example 98 for preparation.

$^1$H NMR (600 MHz, DMSO-d6) δ 11.10 (s, 1H), 8.41 (d, J = 8.2 Hz, 1H), 8.30 (d, J = 1.6 Hz, 1H), 8.09 (dd, J = 8.2, 1.6 Hz, 1H), 7.67 (d, J = 30.4 Hz, 1H), 7.61 (d, J = 6.9 Hz, 1H), 7.44 (dd, J = 9.9, 1.7 Hz, 1H), 7.37 - 7.30 (m, 1H), 720 (s, 1H), 5.09 (dd, J = 12.9, 5.5 Hz, 1H), 4.52 (d, J = 12.9 Hz, 1H), 3.42 (d, J = 21.6 Hz, 3H), 3.08 (d, J = 35.2 Hz, 2H), 2.93 - 2.78 (m, 4H), 2.66 - 2.53 (m, 3H), 2.40 - 2.29 (m, 2H), 2.26 - 2.15 (m, 2H), 2.02 (dd, J = 14.2, 8.9 Hz, 1H), 1.83 (dd, J = 23.5, 17.1 Hz, 3H), 1.76 - 1.61 (m, 5H), 1.58 - 1.38 (m, 7H).
LC-MS(ESI): $[M+H]^+$ = 914.29

Example 115

Synthesis of PROTAC 85

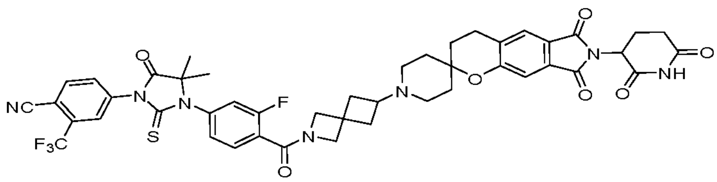

4-(3-(4-(6-(7'-(2,6-dioxopiperidin-3-yl)-6',8'-dioxo-4',6',7',8'-tetrahydro-3'H-spiro[piperidin-4,2'-pyrano[2,3-f]isoindol]-1-yl)-2-azaspiro[3.3]heptane -2-carbonyl)-3-fluorophenyl)-4,4-dimethyl-5-oxo-2-thioimidazolidin-1 -yl)-2-(trifhioromethyl) benzonitrile

Synthetic scheme:

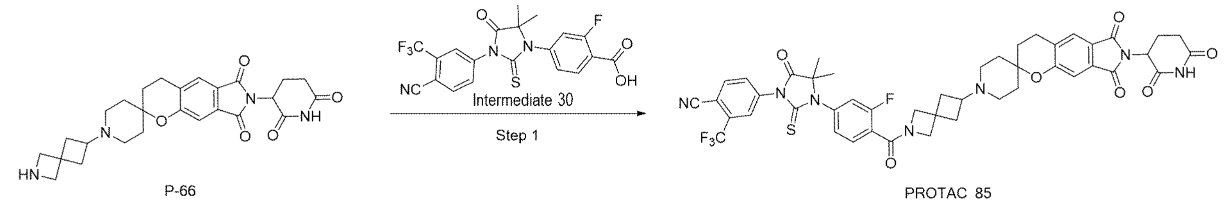


P-66

PROTAC 85

The first step: 4-(3-(4-(6-(7'-(2,6-dioxopiperidin-3-yl)-6',8'-dioxo-4',6', 7',8'-tetrahydro-3'H-spiro[piperidine-4,2'-pyrano [2,3-f]isoindol]-1-yl)-2-azaspiro[ 3.3] heptane-2-carbonyl)-3-fluorophenyl)-4,4-dimethyl-5-oxo-2-thioimidazolidin-1-yl)-2-(trifluoromethyl) benzonitrile (compound PROTAC 85)

Refer to the first step of Example 98 for preparation.

$^1$H NMR (600 MHz, DMSO-d6) δ 11.08 (t, J = 22.1 Hz, 1H), 8.40 (dt, J = 49.2, 24.6 Hz, 1H), 8.27 (d, J = 36.8 Hz, 1H), 8.09 (d, J = 8.2 Hz, 1H), 7.77 - 7.62 (m,2H), 7.47 - 7.39 (m, 1H), 7.37 - 7.28 (m, 1H), 7.28 (d, J = 9.9 Hz, 1H), 5.07 (ddd, J = 64.2, 34.7, 29.5 Hz, 1H), 4.15 (s, 2H), 4.04 (s, 2H), 3.59 (dd, J = 13.2, 7.4 Hz, 2H), 2.89 (m, 4H), 2.64 - 2.55 (m, 2H), 2.48 - 2.41 (m, 2H), 2.00 (m, 1H), 1.93 - 1.76 (m, 4H), 1.59 - 1.39 (m, 11H).
LC-MS(ESI): $[M+H]^+$ = 912.27

Example 116

Synthesis of PROTAC 86

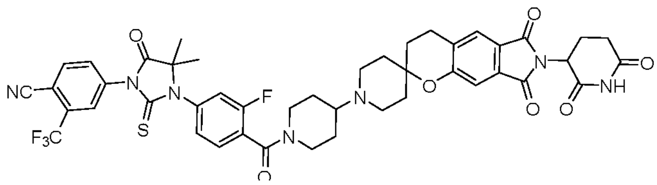

4-(3-(4-(4-(7'-(2,6-dioxopiperidin-3-yl)-6',8'-dioxo-4',6',7',8'-tetrahydro-3'H-spiro[piperidine-4,2'-pyrano[2,3-f]isoindol]-1-yl)piperidine-1-carbonyl)-3-fluorophenyl)-4,4-dimethyl-5-oxo-2-thioimidazolidin-1-yl)-2-(trifluoromethyl)benzonitrile

Synthetic scheme:

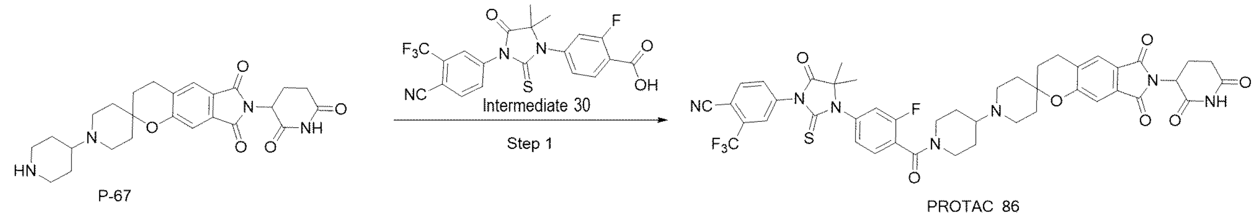


P-67

PROTAC 86

The first step: 4-(3-(4-(4-(7'-(2,6-dioxopiperidin-3-yl)-6',8'-dioxo-4',6', 7',8'-tetrahydro-3'H-spiro[piperidine-4,2'-pyrano [2,3-f]isoindole]-1-yl)piperidine-1-carbonyl) -3-fluorophenyl)-4,4-dimethyl-5-oxo-2-thioimidazolidin-1-yl)-2-(trifluoromethyl)benzonitrile (compound PROTAC 86)

Refer to the first step of Example 98 for preparation.

$^1$H NMR (600 MHz, MeOD) δ8.19 (m, 2H), 8.02 (d, J = 8.2 Hz, 1H), 7.71 (s, 1H), 7.65 (s, 1H), 7.44 (m, 3H), 5.12 (dd, J = 12.8, 5.5 Hz, 1H), 4.95 (d, J = 12.6 Hz, 1H), 3.88 (d, J = 12.8 Hz, 1H), 3.63 (d, J = 35.9 Hz, 3H), 3.52 - 3.42 (m, 2H), 3.02 (dt, J = 23.9, 8.8 Hz, 3H), 2.88 (m, 2H), 2.80 - 2.69 (m, 3H), 2.39 (s, 1H), 2.24 (d, J = 13.4 Hz, 2H), 2.13 (dd, J = 8.8, 3.7

Hz, 2H), 2.01 (t, J = 29.6 Hz, 3H), 1.84 (s, 2H), 1.63 (s, 6H).
LC-MS(ESI): $[M+H]^+$ = 900.27

Example 117

Synthesis of PROTAC 87

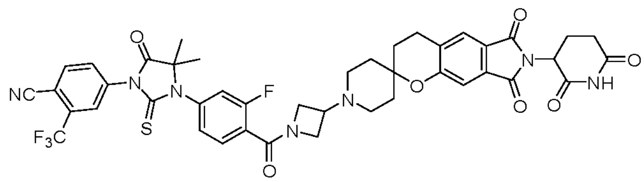

4-(3-(4-(3-(7'-(2,6-dioxopiperidin-3-yl)-6',8'-dioxo-4',6',7',8'-tetrahydro-3'H-spiro[piperidine-4,2'-pyrano[2,3-f]isoindol]-1-yl)azetidine-1-carbonyl)- 3-fluorophenyl)-4,4-dimethyl-5-oxo-2-thioimidazolidin-1-yl)-2-(trifluoromethyl)benzonitrile

Synthetic scheme:

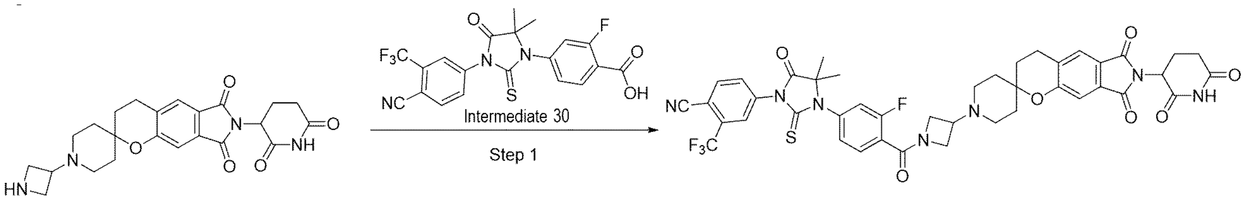


P-68

PROTAC 87

The first step: 4-(3-(4-(3-(7'-(2,6-dioxopiperidin-3-yl)-6',8'-dioxo-4',6' ,7',8'-tetrahydro-3'H-spiro[piperidine-4,2'-pyrano [2,3-f]isoindole]-1-yl)azetidine-1 -carbonyl)-3-fluorophenyl)-4,4-dimethyl-5-oxo-2-thioimidazolidin-1-yl)-2-(trifluoromethyl)benzonitrile (compound PROTAC 87 )

Refer to the first step of Example 98 for preparation.

$^1$H NMR (600 MHz, MeOD) δ 8.20 - 8.18 (m, 2H), 8.01 (d, J = 8.3 Hz, 1H), 7.79 (t, J = 7.7 Hz, 1H), 7.70 (s, 1H), 7.45 (dd, J = 16.7, 9.3 Hz, 1H), 7.38 (s, 1H), 5.11 (dd, J = 12.9, 5.4 Hz, 1H), 4.58 - 4.52 (m, 2H), 4.45 (d, J = 36.2 Hz, 2H), 4.25 (s, 1H), 3.52 - 3.37 (m, 2H), 3.29 - 3.16 (m, 2H), 3.04 (t, J = 6.4 Hz, 2H), 2.94 - 2.67 (m, 4H), 2.24 - 2.09 (m, 3H), 2.03 (d, J = 6.3 Hz,3H), 1.62 (s, 6H).
LC-MS(ESI): $[M+H]^+$ = 872.24

Example 118

Synthesis of PROTAC 88

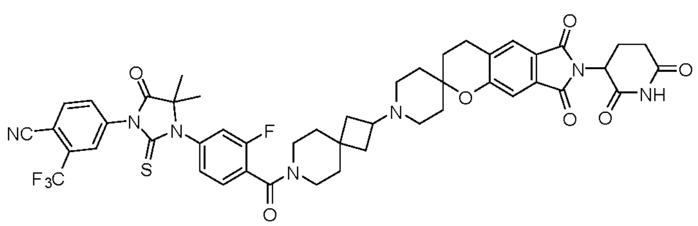

4-(3-(4-(2-(7'-(2,6-dioxopiperidin-3-yl)-6',8'-dioxo-4',6',7',8'-Tetrahydro-3'H-spiro[piperidin-4,2'-pyrano[2,3-f]isoindol]-1-yl)-7-azaspiro[3.5]nonane -7-carbonyl)-3-fluorophenyl)-4,4-dimethyl-5-oxo-2-thioimidazolidin-1 -yl)-2-(trifhioromethyl) benzonitrile

Synthetic scheme:

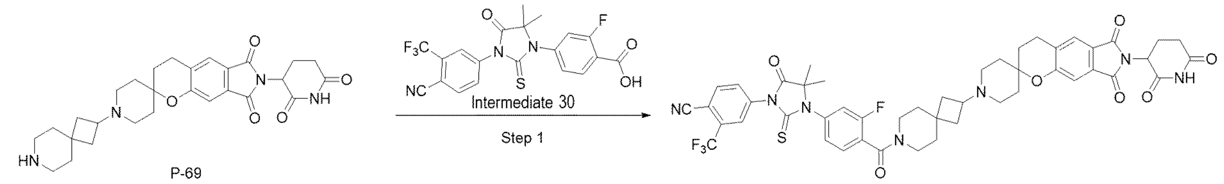


P-69

PROTAC 88

The first step: 4-(3-(4-(2-(7'-(2,6-dioxopiperidin-3-yl)-6',8'-dioxo-4',6' , 7',8'-tetrahydro-3'H-spiro[piperidine-4,2'-pyrano [2,3-f]isoindol]-1-yl)-7-azaspiro[ 3.5] nonane-7-carbonyl)-3-fluorophenyl)-4,4-dimethyl-5-oxo-2-thioimidazolidin-1-yl)-2-(trifluoromethyl) benzonitrile (compound PROTAC 88)

Refer to the first step of Example 98 for preparation.

$^1$H NMR (600 MHz, MeOD) δ 8.19 (d, J = 5.7 Hz, 2H), 8.02 (d, J = 8.3 Hz, 1H), 7.70 (s, 1H), 7.60 (dd, J = 14.3, 7.2 Hz, 1H), 7.45 - 7.35 (m, 3H), 5.16 - 5.07 (m, 1H), 3.83 (t, J = 42.4 Hz, 3H), 3.56 - 3.36 (m, 4H), 3.21 (dd, J = 6.4, 4.8 Hz, 2H), 3.04 (s, 2H), 2.93 - 2.67 (m, 4H), 2.47 (m, 2H), 2.19-2.13 (m, 4H), 2.01 (t, J = 29.6 Hz, 4H), 1.77 (m, 4H), 1.63 (s, 6H).
LC-MS(ESI): [M+H]$^+$ = 940.30

Example 119

Synthesis of PROTAC 89

4-(3-(4-(4-(7'-(2,6-dioxopiperidin-3-yl)-6'-oxo-4',6',7',8'-tetraHydrogen-3'H-spiro[piperidin-4,2'-pyrano[2,3-f]isoindol]-1-yl)methyl)-4-methylpiperidine-1-carbonyl) -3-fluorophenyl)-4,4-dimethyl-5-oxo-2-thioimidazolidin-1-yl)-2-(trifluoromethyl)benzonitrile

Synthetic scheme

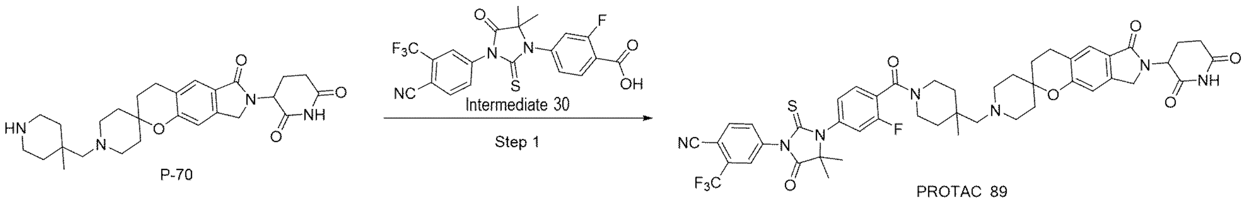


P-70

PROTAC 89

The first step: 4-(3-(4-(4-(7'-(2,6-dioxopiperidin-3-yl)-6'-oxo-4', 6', 7', 8'-tetrahydro-3'H-spiro [piperidine-4,2'-pyrano [2,3 -f]isoindol]-1-yl)methyl)-4-methylpiperidine- 1-carbonyl)-3-fluorophenyl)-4,4-dimethyl-5-oxo-2-thioimidazolidin-1-yl)-2-(trifluoromethyl)benzonitrile (compound PROTAC 89)

Refer to the first step of Example 98 for preparation.

$^1$H NMR (600 MHz, MeOD) δ 8.19 (dd, J = 4.9, 3.1 Hz, 2H), 8.01 (dd, J = 8.3, 1.7 Hz, 1H), 7.62 (dd, J = 14.7, 6.7 Hz, 2H), 7.45 - 7.39 (m, 2H), 7.10 (d, J = 35.3 Hz, 1H), 5.13 (dd, J = 13.3, 5.1 Hz, 1H), 4.41 (dt, J = 33.6, 16.8 Hz, 3H), 3.71 - 3.35 (m, 10H), 3.04 - 2.97 (m, 2H), 2.92 (m, 2H), 2.85 - 2.72 (m, 1H), 2.49 (ddd, J = 26.4, 13.3, 4.3 Hz, 2H), 2.14 (m, 6H), 1.99 (t, J = 6.7 Hz, 2H), 1.79 - 1.58 (m, 9H).
LC-MS(ESI): [M+H]$^+$ = 914.32

Example 120

Synthesis of PROTAC 90

4-(3-(4-(3-(7'-(2,6-dioxopiperidin-3-yl)-8'-oxo-4',6',7',8'-tetrahydro-3'H-spiro[piperidine-4,2'-pyrano[2,3-f]isoindol]-1-yl) azetidine-1-carbonyl)-3- fluorophenyl)-4,4-dimethyl-5-oxo-2-thioimidazolidin-1-yl)-2-(trifluoromethyl)benzonitrile

Synthetic scheme:

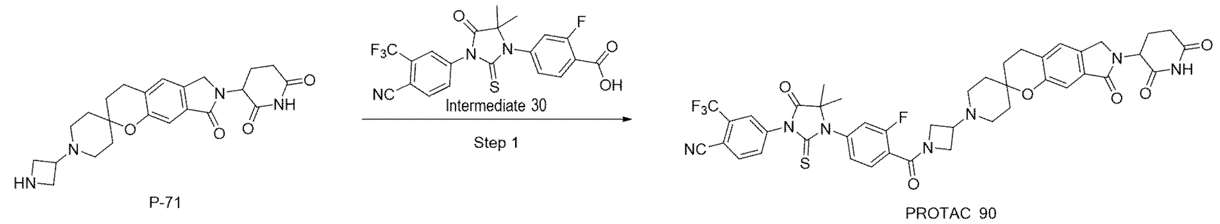


P-71

PROTAC 90

The first step: 4-(3-(4-(3-(7'-(2,6-dioxopiperidin-3-yl)-8'-oxo-4', 6', 7', 8'-tetrahydro-3'H-spiro[piperidine-4,2'-pyrano [2,3-f]isoindol]-1-yl)azetidine-1-carbonyl) - 3-fluorophenyl)-4,4-dimethyl-5-oxo-2-thioimidazolidin-1-yl)-2-(trifluoromethyl)benzonitrile (compound PROTAC 90)

Refer to the first step of Example 98 for preparation.

$^1$H NMR (600 MHz, MeOD) δ 8.22 - 8.13 (m, 2H), 8.01 (d, J = 8.2 Hz, 1H), 7.80 (t, J = 7.8 Hz, 1H), 7.52 - 7.41 (m, 2H), 7.36 (s, 1H), 7.32 (s, 1H), 5.14 (dd, J = 13.2, 4.8 Hz, 1H), 4.63 - 4.55 (m, 2H), 4.52 (m, 1H), 4.47 - 4.33 (m, 2H), 4.48 - 4.33 (m, 4H), 3.63 - 3.35 (m,4H), 3.01 (t, J = 6.5 Hz, 2H), 2.95 - 2.87 (m, 1H), 2.84 - 2.75 (m, 1H), 2.52 - 2.43 (m, 1H), 2.25 - 2.11 (m, 3H), 2.06 - 1.91 (m, 4H), 1.62 (s,6H).
LC-MS(ESI): $[M+H]^+$ = 858.26

Example 121

Synthesis of PROTAC 91

4-(3-(4-(4-(7'-(2,6-dioxopiperidin-3-yl)-6',8'-dioxo-4',6',7', 8'-tetrahydro-3'H-spiro[piperidine-4,2'-pyrano[2,3-f]isoindol]-1-yl)methyl)-4-methylpiperidine-1-carbonyl)-3 -fluorophenyl)-4,4-dimethyl-5-oxo-2-thioimidazolidin-1-yl)-2-(trifluoromethyl)benzonitrile

Synthetic scheme:

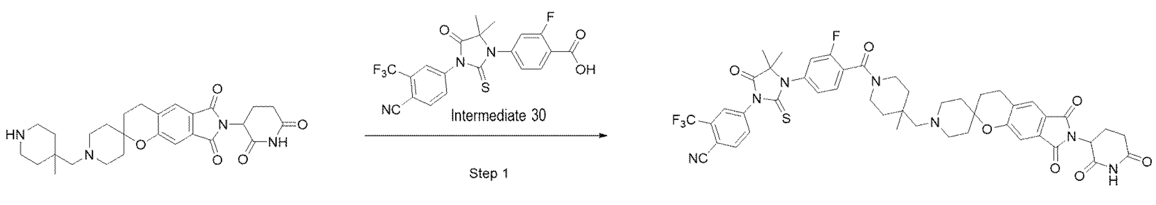


P-72

PROTAC 91

The first step: 4-(3-(4-(4-(7'-(2,6-dioxopiperidin-3-yl)-6',8'-dioxo-4',6' ,7',8'-tetrahydro-3'H-spiro[piperidine-4,2'-pyrano [2,3-f]isoindol]-1-yl)methyl)-4-methylpiperidine-1-carbonyl)-3-fluorophenyl)-4,4-dimethyl-5-oxo-2-thioimidazolidin-1-yl)-2-(trifluoromethyl) benzonitrile (compound PROTAC 91)

Refer to the first step of Example 98for preparation.

$^1$H NMR (600 MHz, DMSO-d6) δ 11.11 (s, 1H), 8.42 (d, J = 8.2 Hz, 1H), 8.30 (s, 1H), 8.09 (d, J = 8.2 Hz, 1H), 7.75 (s, 1H), 7.61 (t, J = 7.8 Hz, 1H), 7.48 - 7.32 (m, 3H), 5.10 (dd, J = 12.9, 5.4 Hz, 1H), 4.08 (s, 2H), 3.22 (m, 10H), 2.96 (s, 2H), 2.88 (m, 1H), 2.66 - 2.52 (m, 5H), 2.17 - 1.83 (m, 7H), 1.67 - 1.46 (m, 8H).
LC-MS(ESI): $[M+H]^+$ = 928.03

Example 122

Synthesis of PROTAC 92

4-(3-(4-(4-((6-(2,6-dioxopiperidin-3-yl)-5,7-dioxo-6,7-dihydro-2H, 5H- Spiro[furo[2,3-f]isoindole-3,4'-piperidin]-1'-yl) methyl)piperidine-1-carbonyl)-3-fluorophenyl)-4,4-dimethyl-5-oxo-2-thioimidazolidin-1-yl)-2-(trifluoromethyl)benzonitrile

Synthetic scheme:

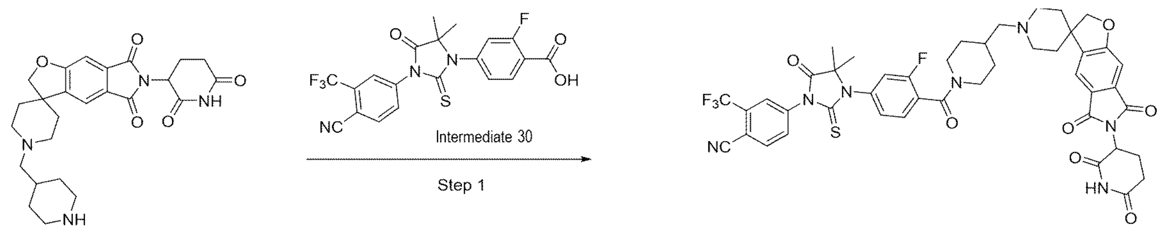


P-73

PROTAC 92

The first step: 4-(3-(4-(4-((6-(2,6-dioxopiperidin-3-yl)-5,7-dioxo-6,7-dihydro- 2H, 5H-spiro[furo[2,3-f]isoindole-3,4'-piperidin]-1'-yl)methyl)piperidine-1-carbonyl)-3-fluorophenyl)- 4,4-dimethyl-5-oxo-2-thioimidazolidin-1-yl)-2-(trifluoromethyl)benzonitrile (compound PROTAC 92)

Refer to the first step of Example 98 for preparation.

$^1$H NMR (600 MHz, DMSO-d6) δ 11.12 (s, 1H), 8.42 (d, J = 8.3 Hz, 1H), 8.30 (t, J = 4.3 Hz, 1H), 8.09 (dd, J = 8.2, 1.6 Hz, 1H), 7.63 (dd, J = 19.8, 11.9 Hz, 2H), 7.46 (dd, J = 14.3, 12.8 Hz, 1H), 7.39 - 7.30 (m, 2H), 5.15 - 5.08 (m, 1H), 4.74 (d, J = 19.1 Hz, 2H), 4.56 (d, J = 12.3 Hz, 1H), 3.59 (d, J = 13.2 Hz, 2H), 3.37 - 3.30 (m, 2H), 3.22 - 3.01 (m, 5H), 2.94 - 2.82 (m, 2H), 2.65 - 2.56 (m, 2H), 2.23 (m, 3H), 2.10 - 1.88-1.79 (m, 6H), 1.57 (s, 6H).
LC-MS(ESI): $[M+H]^+$ = 857.69

Example 123

Synthesis of PROTAC 93

4-(3-(4-(3-((6-(2,6-dioxopiperidin-3-yl)-5,7-dioxo-6,7-dihydro-2H, 5H- Spiro[furo[2,3-f]isoindole-3,4'-piperidin]-1'-yl) methyl)azetidine-1-carbonyl)-3-fluorophenyl)-4,4-dimethyl-5-oxo-2-thioimidazolidin-1-yl)-2-(trifluoromethyl)benzonitrile

Synthetic scheme:

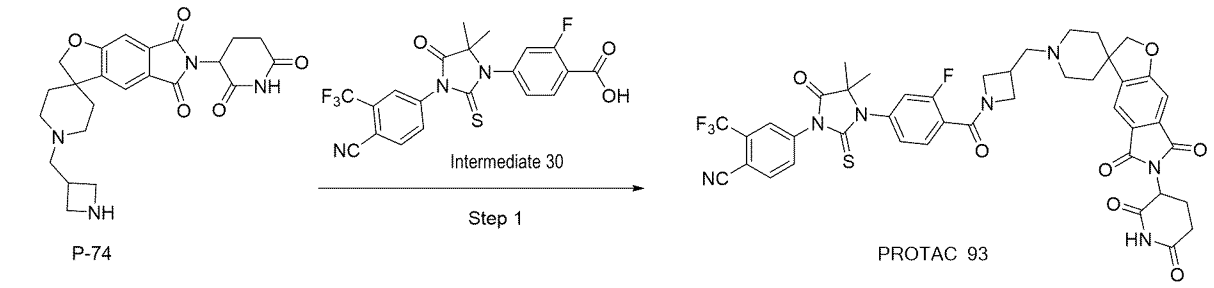


The first step: 4-(3-(4-(3-((6-(2,6-dioxopiperidin-3-yl)-5,7-dioxo-6,7-dihydro- 2H,5H-spiro[furo[2,3-f]isoindole-3,4'-piperidin]-1'-yl)methyl)azetidine-1-carbonyl)-3-fluorophenyl)-4,4-dimethyl-5-oxo-2-thioimidazolidin-1-yl)-2-(trifluoromethyl)benzonitrile (compound PROTAC 93)

Refer to the first step of Example 98 for preparation.

$^1$H NMR (600 MHz, DMSO-d6) δ 11.12 (s, 1H), 8.42 (d, J = 8.3 Hz, 1H), 8.29 (t, J = 7.9 Hz, 1H), 8.09 (dd, J = 8.3, 1.4 Hz, 1H), 7.77 - 7.66 (m, 1H), 7.64 (s, 1H), 7.49 (dd, J = 10.5, 1.6 Hz, 1H), 7.37 (dd, J = 13.9, 5.7 Hz, 2H), 5.15 - 5.07 (m, 1H), 4.78 - 4.65 (m, 2H), 4.29 (dd, J = 16.0, 8.6 Hz, 2H), 4.01 - 3.90 (m, 2H), 3.34 (s, 2H), 3.25 - 3.03 (m, 3H), 2.94 - 2.84 (m, 1H), 2.64 - 2.52 (m, 2H), 2.24 (s, 1H), 2.14 (t, J = 13.2 Hz,2H), 2.03 (dd, J = 17.3, 10.0 Hz, 3H), 1.93 - 1.84 (m, 1H), 1.55 (s, 6H).
LC-MS(ESI): $[M+H]^+$ = 872.09

Example 124

Synthesis of PROTAC 94

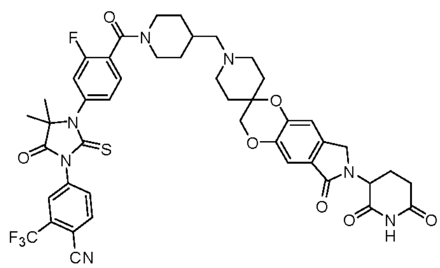

4-(3-(4-(4-(7'-(2,6-dioxopiperidin-3-yl)-6'-oxo-7',8'-dihydro-3'H, 6'H-spiro[piperidine-4,2'-[1,4]dioxo[2,3-f]isoindol]-1-yl)methyl)piperidine-1-carbonyl)-3 -fluorophenyl)-4,4-dimethyl-5-oxo-2-thioimidazolidin-1-yl)-2-(trifluoromethyl)benzonitrile

Synthetic scheme:

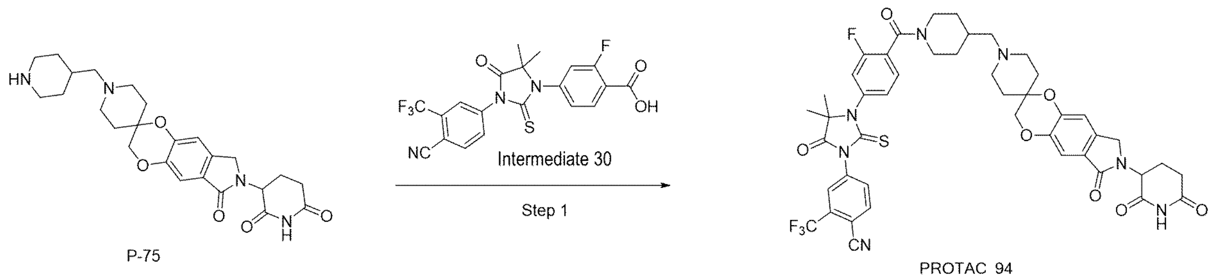

The first step: 4-(3-(4-(4-(7'-(2,6-dioxopiperidin-3-yl)-6'-oxo-7',8'-dihydro- 3'H, 6'H-spiro[piperidine-4,2'-[1,4]dioxo[2,3-f]isoindol]-1-yl)methyl)piperidine-1- carbonyl)-3-fluorophenyl)-4,4-dimethyl-5-oxo-2-thioimidazolidin-1-yl)-2-(trifluoromethyl)benzonitrile (compound PROTAC 94)

Refer to the first step of Example 98 for preparation.

$^1$H NMR (600 MHz, DMSO-d6) δ 10.98 (s, 1H), 8.42 (d, J = 8.3 Hz, 1H), 8.30 (d, J = 1.7 Hz, 1H), 8.09 (dd, J = 8.2, 1.7 Hz, 1H), 7.61 (t, J = 7.8 Hz, 1H), 7.46 (dd, J = 10.0, 1.5 Hz, 1H), 7.36 (dd, J = 8.1, 1.5 Hz, 1H), 7.23 (d, J = 7.4 Hz, 1H), 7.12 (d, J = 25.8 Hz, 1H), 5.11 - 5.04 (m, 1H), 4.55 (d, J = 12.3 Hz, 1H), 4.38 - 4.26 (m, 2H), 4.18 (m, 4H), 3.60 - 3.40 (m, 4H), 320 (d, J = 60.7 Hz, 5H), 2.91 (dt, J = 24.4, 8.1 Hz, 2H), 2.60 (d, J = 16.8 Hz, 1H), 2.42 - 2.30 (m, 1H), 2.16 (s, 2H), 2.02 - 1.89 (m, 5H), 1.76 (s, 1H), 1.57 (s, 5H).
LC-MS(ESI): [M+H]$^+$ = 902.17

Example 125

Synthesis of PROTAC 95

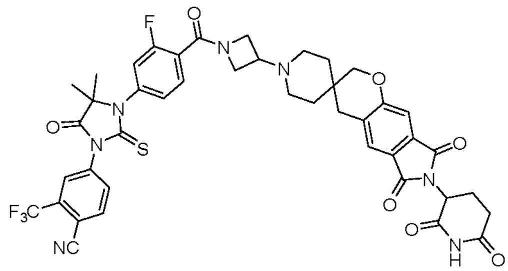

4-(3-(4-(3-(7'-(2,6-dioxopiperidin-3-yl)-6',8'-dioxo-4',6',7', 8'-tetrahydro-2'H-spiro[piperidine-4,3'-pyrano[2,3-f]isoindol]-1-yl)azetidine-1-carbonyl)- 3-fluorophenyl)-4,4-dimethyl-5-oxo-2-thioimidazolidin-1-yl)-2-(trifluoromethyl)benzonitrile

Synthetic scheme:

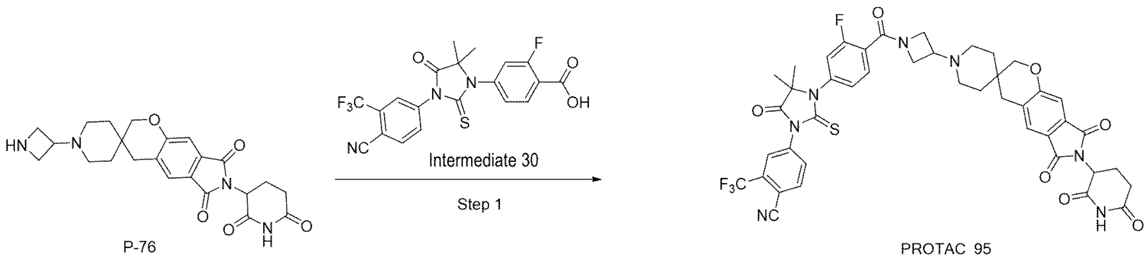


P-76

PROTAC 95

The first step: 4-(3-(4-(3-(7'-(2,6-dioxopiperidin-3-yl)-6',8'-dioxo-4',6',7',8'-tetrahydro-2'H-spiro[piperidine-4,3'-pyrano [2,3-f]isoindole]-1-yl)azetidine-1-carbonyl)-3-fluorophenyl)-4,4-dimethyl-5-oxo-2-thioimidazolidin-1-yl)-2-(trifluoromethyl)benzonitrile (compound PROTAC 95 )

Refer to the first step of Example 98 for preparation.

$^1$H NMR (600 MHz, MeOD) δ 8.19 (d, J = 7.6 Hz, 2H), 8.02 - 7.98 (m, 1H), 7.79 (t, J = 7.8 Hz, 1H), 7.67 (s, 1H), 7.45 (ddd, J = 9.8, 9.4, 1.5 Hz,2H), 7.29 (s, 1H), 5.11 (dd, J = 12.8, 5.4 Hz, 1H), 4.61 - 4.54 (m, 2H), 4.51 (dd, J = 10.7, 4.9 Hz, 1H), 4.44 (dd, J = 11.8, 4.8 Hz, 1H), 4.33 - 4.11 (m, 4H), 3.42 (m, 1H), 3.01 (s, 3H), 2.92 - 2.66 (m, 4H), 2.14 - 2.07 (m, 1H), 1.82 (t, J = 48.7 Hz, 4H), 1.62 (s, 6H).
LC-MS(ESI): [M+H]$^+$ = 872.36

Example 126

Synthesis of PROTAC 96

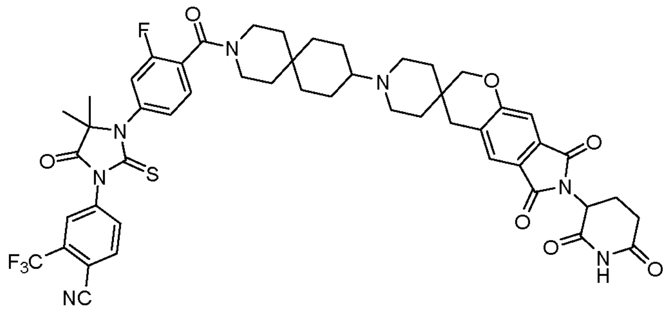

4-(3-(4-(9-(7'-(2,6-dioxopiperidin-3-yl)-6',8'-dioxo-4',6',7', 8'-tetrahydro-2'H-spiro[piperidine-4,3'-pyrano[2,3-f]isoindol]-1-yl)-3-azaspiro[5.5]undecane-3-carbonyl)-3-fluorophenyl)-4,4-dimethyl-5-oxo-2-thioimidazolidin-1 -yl)-2-(trifluoromethyl)benzonitrile

Synthetic scheme:

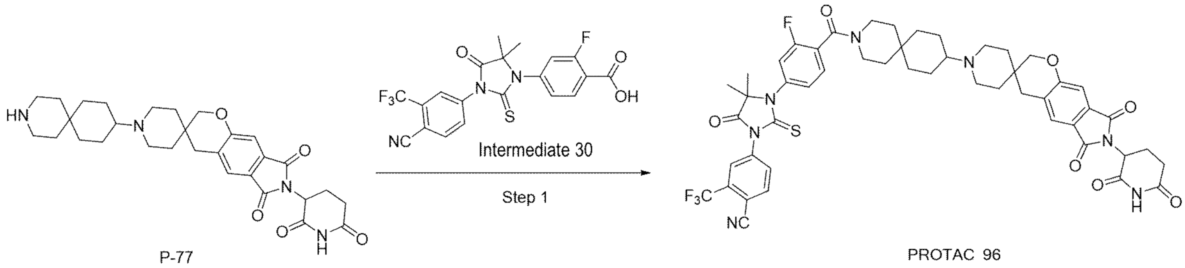


P-77

PROTAC 96

The first step: 4-(3-(4-(9-(7'-(2,6-dioxopiperidin-3-yl)-6',8'-dioxo-4',6' , 7',8'-tetrahydro-2'H-spiro[piperidine-4,3'-pyrano [2,3-f]isoindol]-1-yl)-3-azaspiro[5.5] undecane-3-carbonyl)-3-fluorophenyl)-4,4-dimethyl-5-oxo-2-thioimidazolidin-1-yl)-2-(trifluoromethyl) benzonitrile (compound PROTAC 96)

Refer to the first step of Example 98 for preparation.

$^1$H NMR (600 MHz, DMSO-d6) δ 11.11 (d, J = 4.4 Hz, 1H), 8.42 (d, J = 8.3 Hz, 1H), 8.30 (s, 1H), 8.09 (d, J = 8.4 Hz, 1H), 7.64 (ddd, J = 31.3, 21.8, 7.8 Hz, 2H), 7.49 - 7.40 (m, 1H), 7.38 - 7.25 (m, 2H), 5.14 - 5.06 (m, 1H), 4.32 (d, J = 11.2 Hz, 1H), 4.03 (d, J = 14.1 Hz, 1H), 3.43 - 3.32 (m, 7H), 3.28 - 3.10 (m, 5H), 3.07 (d, J = 12.8 Hz, 1H), 2.89 (dd, J = 20.1, 10.2 Hz, 1H), 2.79 (d, J = 13.4 Hz, 1H), 2.58 (m, 2H), 2.08 - 1.98 (m, 1H), 1.89 (s, 3H), 1.79 - 1.61 (m, 4H), 1.53 (m, 7H), 1.47 (d, J = 27.9 Hz, 1H), 1.40 (s, 1H), 1.32 (s, 1H), 1.26 - 1.12 (m, 2H).
LC-MS(ESI): $[M+H]^+$ = 968.45

Example 127

Synthesis of PROTAC 97

4-(3-(4-(4-(7'-(2,6-dioxopiperidin-3-yl)-6',8'-dioxo-4',6',7', 8'-tetrahydro-2'H-spiro[piperidine-4,3'-pyrano [2,3-f]isoindol]-1-yl)methyl)-4-methylpiperidine- 1-carbonyl)-3-fluorophenyl)-4,4-dimethyl-5-oxo-2-thioimidazolidin-1-yl)-2-(trifluoromethyl)benzonitrile

Synthetic scheme:

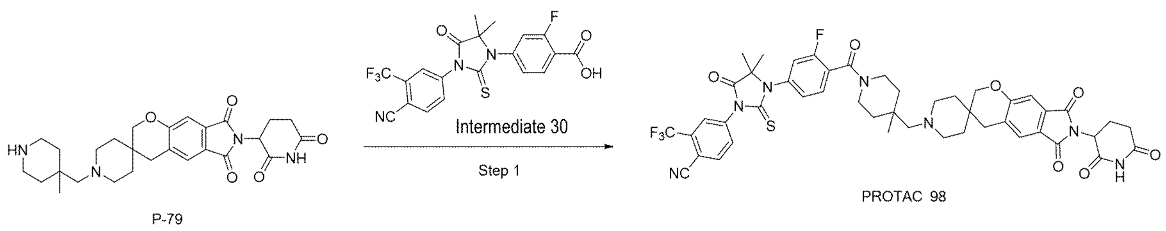


The first step: 4-(3-(4-(4-(7'-(2,6-dioxopiperidin-3-yl)-6',8'-dioxo-4',6' ,7',8'-tetrahydro-2'H-spiro[piperidine-4,3'-pyrano [2,3-f]isoindol]-1-yl)methyl)-4-methylpiperidine-1-carbonyl)-3-fluorophenyl)-4,4-dimethyl-5-oxo-2-thioimidazolidin-1-yl)-2-(trifluoromethyl) benzonitrile (Compound PROTAC 98)

Refer to the first step of Example 98 for preparation.

$^1$H NMR (600 MHz, DMSO-d6) δ 11.11 (s, 1H), 8.42 (d, J = 8.2 Hz, 1H), 8.29 (s, 1H), 8.09 (d, J = 8.2 Hz, 1H), 7.75 -7.61 (m, 2H), 7.46 (d, J = 9.9 Hz, 1H), 7.36 (d, J = 8.0 Hz, 1H), 7.30 (s, 1H), 5.10 (dd, J = 12.9, 5.4 Hz, 1H), 4.30 (s, 1H), 4.10 (s, 1H), 4.05 (s, 1H), 3.32 (s, 11H), 3.17 (s, 2H), 3.07 (s, 1H), 2.95 - 2.84 (m, 1H), 2.81 (s, 1H), 2.58 (m, 1H), 2.08 - 1.99 (m, 1H), 1.85 (dd, J = 34.1, 28.7 Hz, 3H), 1.71 (s, 1H), 1.64 - 1.42 (m, 10H).
LC-MS(ESI): $[M+H]^+$ = 928.45

Example 128

Synthesis of PROTAC 98

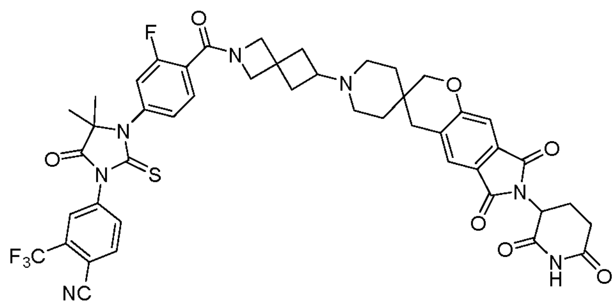

4-(3-(4-(6-(7'-(2,6-dioxopiperidin-3-yl)-6',8'-dioxo-4',6',7', 8'-tetrahydro-2'H-spiro[piperidin-4,3'-pyrano[2,3-f]isoindol]-1-yl)-2-azaspiro[3.3]heptane -2-carbonyl)-3-fluorophenyl)-4,4-dimethyl-5-oxo-2-thioimidazolidin-1 -yl)-2-(trifhioromethyl) benzonitrile

Synthetic scheme:

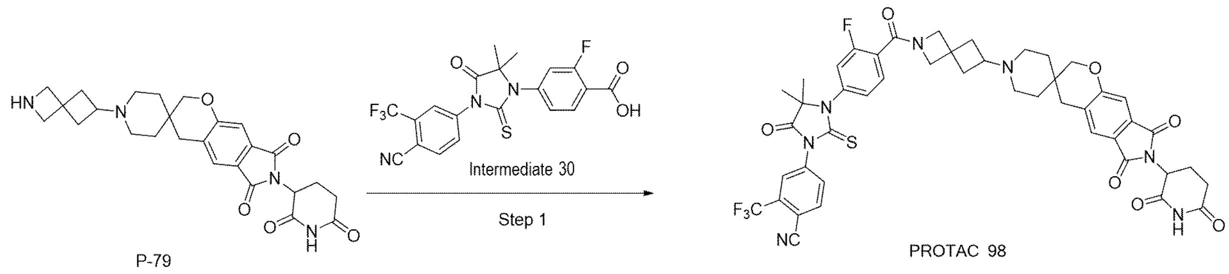


The first step: 4-(3-(4-(6-(7'-(2,6-dioxopiperidin-3-yl)-6',8'-dioxo-4',6' , 7',8'-tetrahydro-2'H-spiro[piperidine-4,3'-pyrano [2,3-fJisoindol]-1-yl)-2-azaspiro[3.3] heptane-2-carbonyl)-3-fluorophenyl)-4,4-dimethyl-5-oxo-2-thioimidazolidin-1-yl)-2-(trifluoromethyl) benzonitrile (compound PROTAC 98)

Refer to the first step of Example 98 for preparation.

$^1$H NMR (600 MHz, DMSO-d6) δ 11.10 (s, 1H), 8.42 (d, J = 8.2 Hz, 1H), 8.29 (s, 1H), 8.09 (d, J = 8.2 Hz, 1H), 7.72 (dd, J = 15.9, 5.9 Hz, 1H), 7j61 (d, J = 7.4 Hz, 1H), 7.46 (dd, J = 10.4, 3.4 Hz, 1H), 7.35 (d, J = 6.8 Hz, 1H), 7.30 (d, J = 7.2 Hz, 1H), 5.10 (dd, J = 12.8, 5.2 Hz, 1H), 4.30 (d, J = 5.9 Hz, 1H), 4.18 (s, 1H), 4.09 (s, 1H), 4.05 (d, J = 5.0 Hz, 1H), 3.68 (m, 2H), 3.32 - 3.22 (m, 3H), 3.05 (d, J = 6.5 Hz, 1H), 3.02 - 2.83 (m, 3H), 2.80 (d, J = 5.8 Hz, 1H), 2.60 (d, J = 16.0 Hz, 2H), 2.48 - 2.35 (m, 2H), 2.05 - 2.00 (m, 1H), 1.78 - 1.72 (m, 1H), 1.62 (m, 8H).
LC-MS(ESI): $[M+H]^+$ = 912.45

Example 129

Synthesis of PROTAC 99

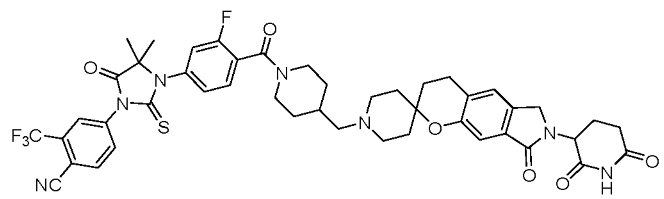

4-(3-(4-(4-(7'-(2,6-dioxopiperidin-3-yl)-8'-oxo-4',6',7', 8'-tetrahydro-3'H-spiro[piperidine-4,2'-pyrano[2,3-f]isomdol]-1-yl) methyl)piperidine-1-carbonyl)-3- fluorophenyl)-4,4-dimethyl-5-oxo-2-thioimidazolidin-1-yl)-2-(trifluoromethyl)benzonitrile

Synthetic scheme:

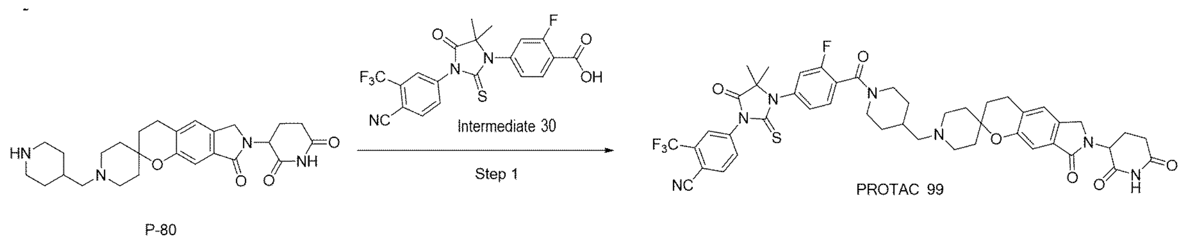


The first step: 4-(3-(4-(4-(7'-(2,6-dioxopiperidin-3-yl)-8'-oxo-4', 6', 7', 8'-tetrahydro-3'H-spiro[piperidine-4,2'-pyrano [2,3-f]isoindol]-1-yl)methyl)piperidine-1-carbonyl)- 3-fluorophenyl)-4,4-dimethyl-5-oxo-2-thioimidazolidin-1-yl)-2-(trifluoromethyl)benzonitrile (compound PROTAC 99)

Refer to the first step of Example 98 for preparation.

$^1H$ NMR (600 MHz, DMSO-d6) δ 10.98 (s, 1H), 8.42 (d, J = 8.2 Hz, 1H), 8.30 (s, 1H), 8.09 (d, J = 8.3 Hz, 1H), 7.61 (d, J = 7.7 Hz, 1H), 7.46 (d, J = 9.9 Hz, 1H), 7.35 (d, J = 8.3 Hz, 2H), 7.24 (s, 1H), 5.07 (dd, J = 13.2, 5.2 Hz, 1H), 5.07 (dd, J = 13.2, 5.2 Hz, 1H), 4.55 (d, J = 12.6 Hz, 1H), 4.26 m, 2H), 3.46 (m, 3H), 3.30 - 3.07 (m, 6H), 2.89 (dd, J = 19.3, 6.0 Hz, 4H), 2.64 - 2.55 (m, 1H), 2.42 - 2.34 (m, 1H), 2.19 (s, 1H), 2.02 - 1.81 (m, 8H), 1.76 (s, 1H), 1.57 (s, 6H). LC-MS(ESI): $[M+H]^+$ = 900.45

Example 130

Synthesis of PROTAC 100

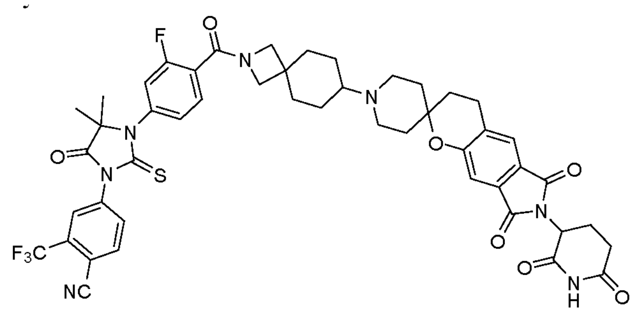

4-(3-(4-(7-(7'-(2,6-dioxopiperidin-3-yl)-6',8'-dioxo-4',6',7', 8'-tetrahydro-3'H-spiro[piperidin-4,2'-pyrano[2,3-f]isoindol]-1-yl)-2-azaspiro[3.5]nonane -2-carbonyl)-3-fluorophenyl)-4,4-dimethyl-5-oxo-2-thioimidazolidin-1 -yl)-2-(trifhioromethyl) benzonitrile

Synthetic scheme:

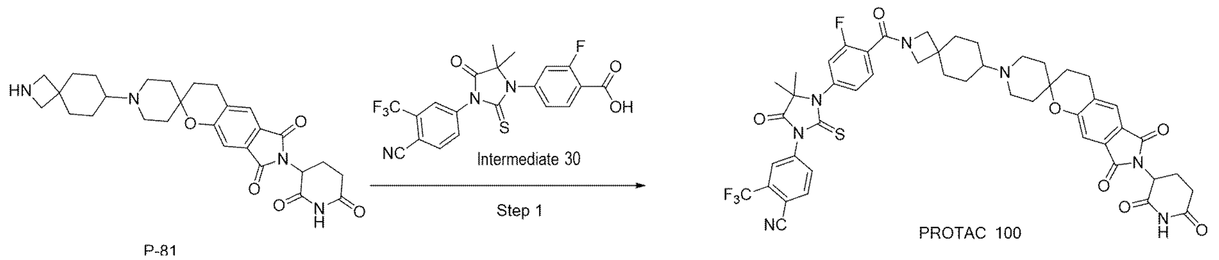

P-81
PROTAC 100

The first step: 4-(3-(4-(7-(7'-(2,6-dioxopiperidin-3-yl)-6',8'-dioxo-4',6', 7',8'-tetrahydro-3'H-spiro[piperidine-4,2'-pyrano [2,3-f]isoindol]-1-yl)-2-azaspiro[ 3.5] nonane-2-carbonyl)-3-fluorophenyl)-4,4-dimethyl-5-oxo-2-thioimidazolidin-1-yl)-2-(trifluoromethyl) benzonitrile (compound PROTAC 100)

Refer to the first step of Example 98 for preparation.

$^1H$ NMR (600 MHz, DMSO-d6) δ 11.11 (d, J = 6.2 Hz, 1H), 8.42 (d, J = 8.2 Hz, 1H), 8.29 (s, 1H), 8.09 (d, J = 8.2 Hz, 1H), 7.80 - 7.67 (m, 2H), 7.49 - 7.42 (m, 1H), 7.40 (s, 1H), 7.38 - 7.32 (m, 1H), 5.14 - 5.02 (m, 1H), 3.85 - 3.72 (m, 5H), 3.44 - 3.15 (m, 7H), 3.00 - 2.79 (m, 4H), 2.60 (d, J = 18.0 Hz, 1H), 2.48 - 2.42 (m, 1H), 2.12 - 1.80 (m, 10H), 1.63 - 1.40 (m, 9H). LC-MS(ESI): $[M+H]^+$ = 940.45

Example 131

Synthesis of PROTAC 101

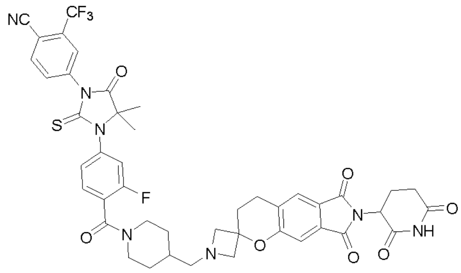

4-(3-(4-(4-(7'-(2,6-dioxopiperidin-3-yl)-6',8'-dioxo-4',6',7', 8'-tetrahydro-3'H-spiro[azetidine-3,2'-pyrano[2,3-f]isoindole]-1-yl)methyl)piperidine-1- carbonyl)-3-fluorophenyl)-4,4-dimethyl-5-oxo-2-thioimidazolidin-1-yl)-2-(trifluoromethyl) benzonitrile

Synthetic scheme:

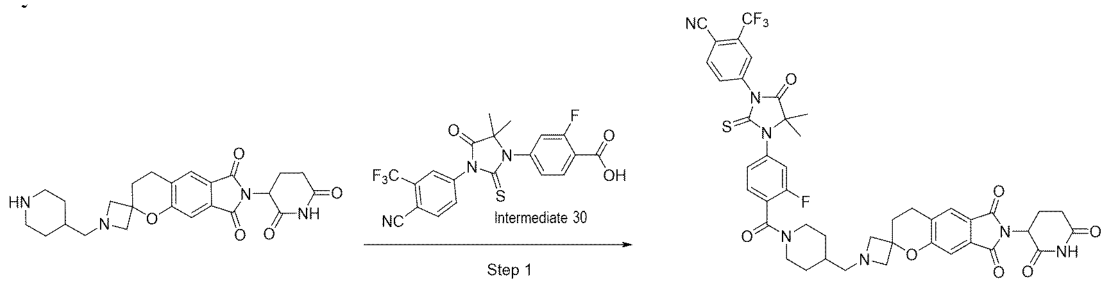


P-82

PROTAC 101

The first step: 4-(3-(4-(4-(7'-(2,6-dioxopiperidin-3-yl)-6',8'-dioxo-4',6', 7',8'-tetrahydro-3'H-spiro[azetidine-3,2'-pyrano [2,3-f]isoindol]-1-yl)methyl)piperidin-1-carbonyl)-3-fluorophenyl)-4,4-dimethyl-5-oxo-2-thioimidazolidin-1-yl)-2-(trifluoromethyl)benzonitrile ( Compound PROTAC 101)

Refer to the first step of Example 98 for preparation.

$^1$H NMR (600 MHz, DMSO-d6) δ 11.12 (s, 1H), 8.42 (d, J = 8.2 Hz, 1H), 8.29 (d, J = 1.3 Hz, 1H), 8.09 (dd, J = 8.2, 1.4 Hz, 1H), 7.79 (s, 1H), 7.60 (t, J = 7.7 Hz, 1H), 7.46 (dd, J = 9.9, 1.3 Hz, 1H), 7.35 (dd, J = 8.1, 1.3 Hz, 1H), 7.30 (d, J = 12.7 Hz, 1H), 5.11 (dd, J = 12.9, 5.4 Hz, 1H), 4.56 - 4.29 (m, 5H), 3.33 (s, 2H), 3.25 (s, 2H), 3.12 (s, 1H), 3.00 (d, J = 5.9 Hz, 1H), 2.96 - 2.79 (m, 3H), 2.64 - 2.54 (m, 2H), 2.25 (d, J = 5.8 Hz, 2H), 2.09 - 1.88 (m, 3H), 1.84 (d, J = 11.4 Hz, 1H), 1.69 (s, 1H), 1.56 (s, 6H).
LC-MS(ESI): $[M+H]^+$ = 886.11

Example 132

Synthesis of PROTAC 102

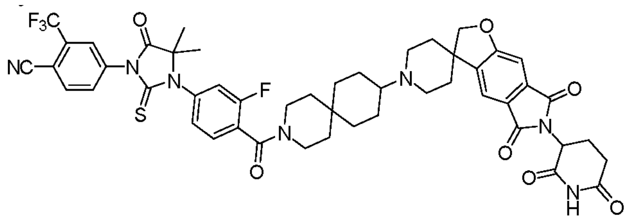

4-(3-(4-(9-(6-(2,6-dioxopiperidin-3-yl)-5,7-dioxo-6,7-dihydro-2H,5H-spiro [furo[2,3-f]isoindole-3,4'-piperidin]-1'-yl)-3-azaspiro[5.5]undecane-3-carbonyl)-3-fluorophenyl )-4,4-dimethyl-5-oxo-2-thioimidazolidin-1-yl)-2-(trifluoromethyl)benzonitrile

Synthetic scheme:

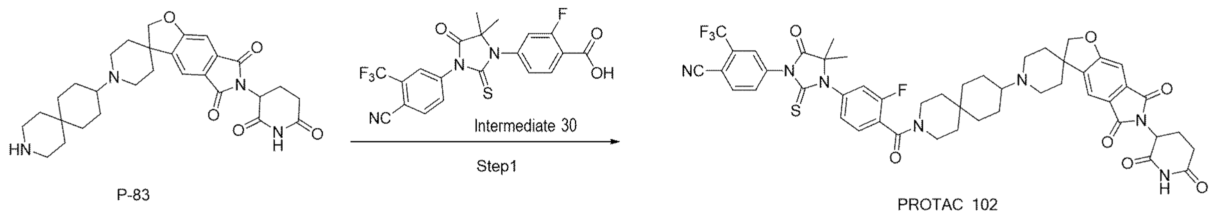


P-83

PROTAC 102

The first step: 4-(3-(4-(9-(6-(2,6-dioxopiperidin-3-yl)-5,7-dioxo-6,7-dihydro-2H, 5H-spiro[furo[2,3-f]isoindole-3,4'-piperidin]-1'-yl)-3-azaspiro[5.5]undecane-3-carbonyl)- -fluorophenyl)-4,4-dimethyl-5-oxo-2-thioimidazolidin-1-yl)-2-(trifluoromethyl)benzonitrile (compound PROTAC 102)

Refer to the first step of Example 98 for preparation.

$^1$H NMR (600 MHz, DMSO-d6) δ 11.12 (d, J = 4.6 Hz, 1H), 8.42 (d, J = 8.2 Hz, 1H), 8.29 (d, J = 7.1 Hz, 1H), 8.09 (d, J = 8.3 Hz, 1H), 7.68 - 7.52 (m, 2H), 7.48 - 7.42 (m, 1H), 7.40 - 7.30 (m, 2H), 5.16 - 5.06 (m, 1H), 4.81 - 4.68 (m, 2H), 3.68 (s, 2H), 3.62 - 3.53 (m, 1H), 3.25 (dd, J = 12.5, 5.2 Hz, 4H), 3.17 - 3.02 (m, 2H), 2.96 - 2.83 (m, 1H), 2.64 - 2.56 (m, 2H), 2.26 - 2.14 (m, 2H), 2.09 - 1.98 (m, 3H), 1.90 (d, J = 9.5 Hz, 4H), 1.69 (d, J = 11.3 Hz, 1H), 1.63 - 1.48 (m, 8H), 1.42 - 1.27 (m, 5H).

LC-MS(ESI): $[M+H]^+$ = 954.11

Example 133

Synthesis of PROTAC 103

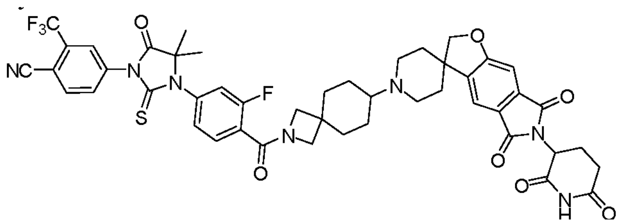

4-(3-(4-(7-(6-(2,6-dioxopiperidin-3-yl)-5,7-dioxo-6,7-dihydro-2H,5H-spiro [furo[2,3-f] isoindole-3,4'-piperidin]-1'-yl)-2-azaspiro[3.5]nonane-2-carbonyl)-3-fluorophenyl) -4,4-dimethyl-5-oxo-2-thioimidazolidin-1 -yl)-2-(trifluoromethyl)benzonitrile

Synthetic scheme:

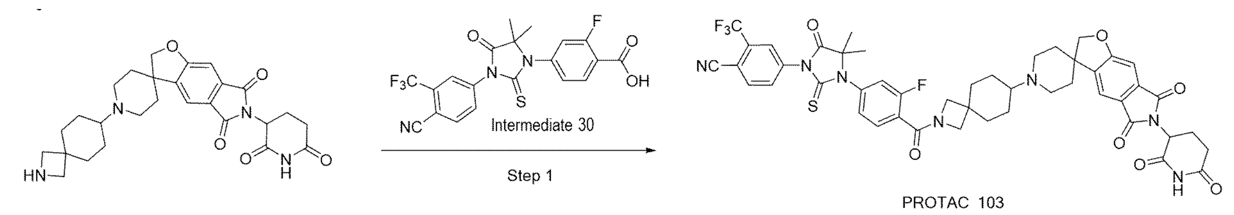


P-84

PROTAC 103

The first step: 4-(3-(4-(7-(6-(2,6-dioxopiperidin-3-yl)-5,7-dioxo-6,7-dihydro-2H, 5H-spiro[furo[2,3-f]isoindole-3,4'-piperidin]-1'-yl)-2-azaspiro[3.5]nonane-2-carbonyl)-3- fluorophenyl)-4,4-dimethyl-5-oxo-2-thioimidazolidin-1-yl)-2-(trifluoromethyl)benzonitrile (compound PROTAC 103)

Refer to the first step of Example 98 for preparation.

$^1H$ NMR (600MHz, DMSO-d6) δ11.12(d, J=5.1Hz, 1H), 8.42(d, J=8.3Hz, 1H), 8.30(s, 1H), 8.09(d, J=8.3Hz ,1H),7.74(dt,J=19.1,7.9Hz,1H),7.62(d,J=11.4Hz,1H),7.50-7.42(m,1H),7.36(dd,J=12.1,7.9Hz, 2H), 5.20-5.02(m, 1H), 4.83-4.67(m, 2H), 3.81(t, J = 16.5Hz, 2H), 3.79-3.70(m, 2H), 3.54(d, J = 11.1Hz ,2H),3.26(s,2H),3.10(m,2H),2.97-2.82(m,1H),2.60(dd,J = 24.1,8.5Hz,1H),2.26-1.81(m,10H), 1.66-1.41(m,9H).
LC-MS (ESI): $[M+H]^+$=926.15

Example 134

Synthesis of PROTAC 104

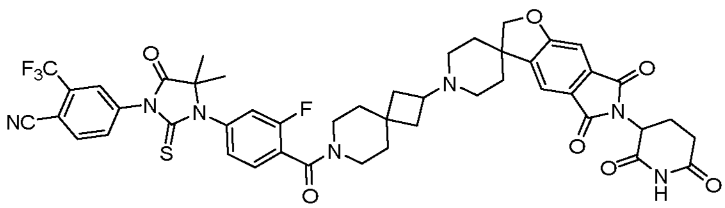

4-(3-(4-(2-(6-(2,6-dioxopiperidin-3-yl)-5,7-dioxo-6,7-dihydro-2H, 5H-spiro [furo[2,3-f]isoindole-3,4'-piperidin]-1'-yl)-7-azaspiro[3.5]nonane-7-carbonyl)-3-fluorophenyl) -4,4-dimethyl-5-oxo-2-thioimidazolidin-1 -yl)-2-(trifluoromethyl)benzonitrile

Synthetic scheme:

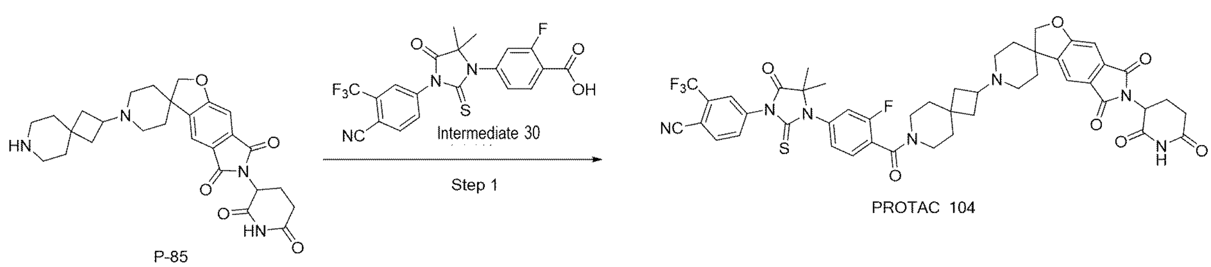


P-85

PROTAC 104

The first step: 4-(3-(4-(2-(6-(2,6-dioxopiperidin-3-yl)-5,7-dioxo-6,7-dihydro-2H, 5H-spiro[furo[2,3-f]isoindole-3,4'-piperidin]-1'-yl)-7-azaspiro[3.5]nonane-7-carbonyl)-3- fluorophenyl)-4,4-dimethyl-5-oxo-2-thioimidazolidin-1-yl)-2-(trifluoromethyl)benzonitrile (compound PROTAC 104)

Refer to the first step of Example 116 for preparation.

$^1$H NMR (600 MHz, DMSO-d6) δ 11.15 - 11.09 (m, 1H), 8.42 (d, J = 8.2 Hz, 1H), 8.30 (s, 1H), 8.09 (d, J = 8.4 Hz, 1H), 7.66 (t, J = 11.8 Hz, 1H), 7.62 (td, J = 8.0, 3.4 Hz, 1H), 7.46 (t, J = 9.9 Hz, 1H), 7.36 (dd, J = 12.8, 10.0 Hz, 2H), 5.16 - 5.07 (m, 1H), 4.73 (dd, J = 15.6, 9.0 Hz, 2H), 3.79 - 3.56 (m, 3H), 3.32 - 3.14 (m, 4H), 2.90 (dd, J = 16.5, 14.0 Hz, 3H), 2.63 (t, J = 24.7 Hz, 2H), 2.29 (m, 3H), 2.17 - 1.98 (m, 6H), 1.66 (s, 2H), 1.57 (s, 8H).

LC-MS(ESI): $[M+H]^+$ = 926.11

Example 135

Synthesis of PROTAC 105

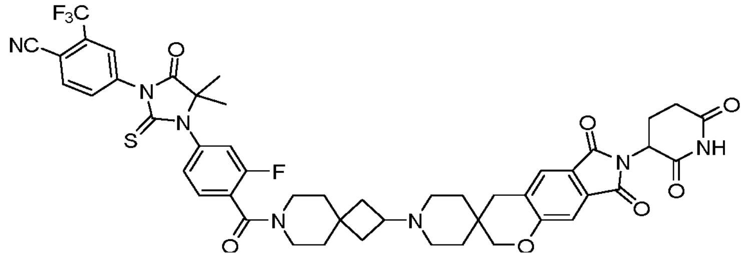

4-(3-(4-(2-(7'-(2,6-dioxopiperidin-3-yl)-6',8'-dioxo-4',6',7', 8'-tetrahydro-2'H-spiro[piperidin-4,3'-pyrano[2,3-f]isoindol]-1-yl)-7-azaspiro[3.5]nonane -7-carbonyl)-3-fluorophenyl)-4,4-dimethyl-5-oxo-2-thioimidazolidin-1 -yl)-2-(trifluoromethyl) benzonitrile

Synthetic scheme:

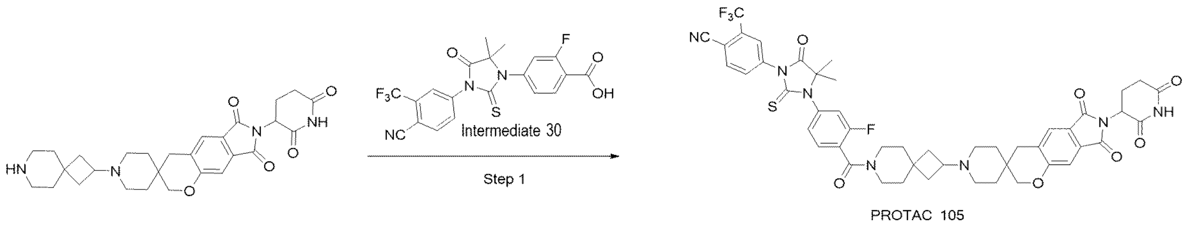


P-86

PROTAC 105

The first step: 4-(3-(4-(2-(7'-(2,6-dioxopiperidin-3-yl)-6',8'-dioxo-4',6', 7',8'-tetrahydro-2'H-spiro[piperidine-4,3'-pyrano [2,3-f]isoindol]-1-yl)-7-azaspiro[ 3.5] Nnonane-7-carbonyl)-3-fluorophenyl)-4,4-dimethyl-5-oxo-2-thioimidazolidin-1-yl)-2-(trifluoromethyl) benzonitrile (compound PROTAC 105)

Refer to the first step of Example 98 for preparation.

$^1$H NMR (400 MHz, DMSO-d6) δ 11.11 (s, 1H), 8.42 (d, J = 8.3 Hz, 1H), 8.30 (s, 1H), 8.09 (d, J = 8.3 Hz, 1H), 7.71 (t, J = 5.5 Hz, 1H), 7.64 - 7.55 (m, 1H), 7.45 (dd, J = 9.9, 2.5 Hz, 1H), 7.37 - 7.22 (m, 2H), 5.10 (dd, J = 12.7, 5.3 Hz, 1H), 4.31 (s, 1H), 4.06 (s, 1H), 3.79 (m, 2H), 3.37 - 3.13 (m, 5H), 3.09 - 2.77 (m, 6H), 2.68 - 2.55 (m, 2H), 2.25 (d, J = 9.4 Hz, 2H), 2.13 - 1.95 (m, 3H), 1.77 (d, J = 11.7 Hz, 1H), 1.66- 1.56 (s, 12H).

LC-MS(ESI): $[M+H]^+$ = 940.35

Example 136

Synthesis of PROTAC 106

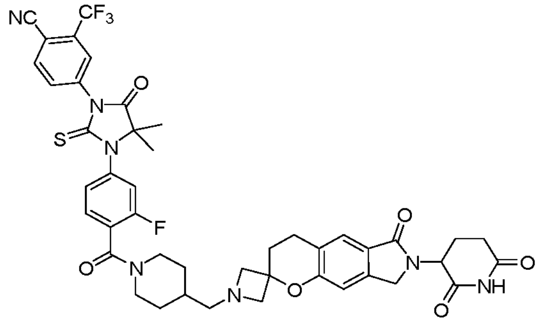

4-(3-(4-(4-(7'-(2,6-dioxopiperidin-3-yl)-6'-oxo-4',6',7',8'-tetrahydro-3'H-spiro[azetidine-3,2'-pyrano[2,3-f]isoindol]-1-yl)methyl)piperidine-1-carbonyl)-3 -fluorophenyl)-4,4-dimethyl-5-oxo-2-thioimidazolidin-1-yl)-2-(trifluoromethyl)benzonitrile

Synthetic scheme:

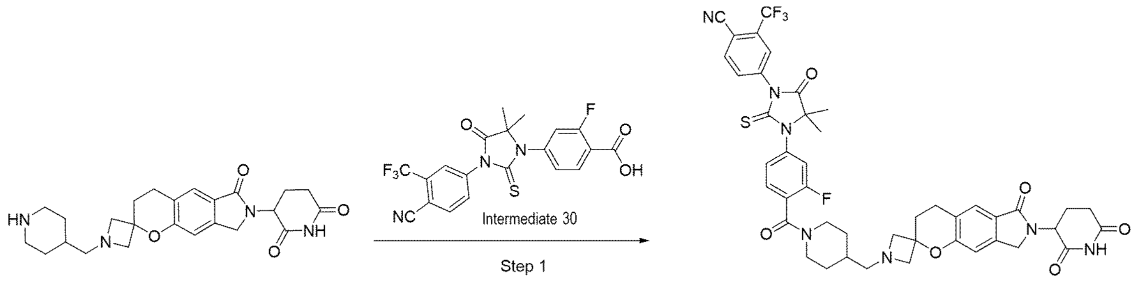


P-87

PROTAC 106

The first step: 4-(3-(4-(4-(7'-(2,6-dioxopiperidin-3-yl)-6'-oxo-4', 6', 7', 8'-tetrahydro-3'H-spiro [azetidine-3,2'-pyrano[2,3-f]isoindole]-1-yl)methyl)piperidine-1-carbonyl)-3-fluorophenyl)-4,4-dimethyl-5-oxo-2-thioimidazolidin-1-yl)-2-(trifluoromethyl)benzonitrile (compound PROTAC 106)

Refer to the first step of Example 98 for preparation.

$^1$H NMR (600 MHz, DMSO-d6) δ 10.97 (s, 1H), 8.42 (d, J = 8.2 Hz, 1H), 8.29 (s, 1H), 8.09 (d, J = 8.1 Hz, 1H), 7.60 (t, J = 7.5 Hz, 1H), 7.53 (s, 1H), 7.45 (d, J = 10.0 Hz, 1H), 7.35 (dd, J = 8.0, 1.2 Hz, 1H), 7.07 (d, J = 19.3 Hz, 1H), 5.07 (dd, J = 13.2, 4.8 Hz, 1H), 4.53 - 4.42 (m, 2H), 4.37 - 4.19 (m, 6H), 3.11 (s, 2H), 2.95 - 2.77 (m, 4H), 2.60 (d, J = 17.8 Hz, 1H), 2.40 - 2.28 (m, 1H), 2.27 - 2.13 (m, 2H), 2.03 - 1.88 (m, 2H), 1.83 (s, 1H), 1.69 (s, 1H), 1.56 (s, 6H), 1.32 - 1.11 (m, 3H). LC-MS(ESI): $[M+H]^+$ = 872.36

Example 137

Synthesis of PROTAC 107

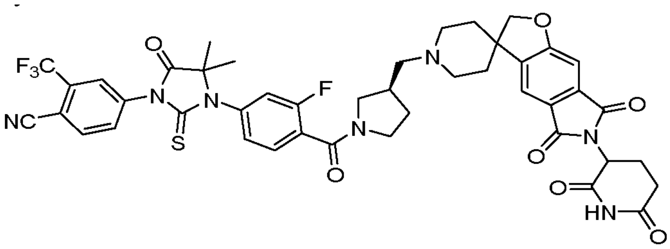

4-(3-(4-((3R)-3-((6-(2,6-dioxopiperidin-3-yl)-5,7-dioxo-6,7-dihydro- 2H, 5H-spiro[furo[2,3-f]isoindole-3,4'-piperidin-1'-yl) methyl)pyrrolidine-1-carbonyl)-3-fluorophenyl)- 4,4-dimethyl-5-oxo-2-thioimidazolidin-1-yl)-2-(trifluoromethyl)benzonitrile

Synthetic scheme:

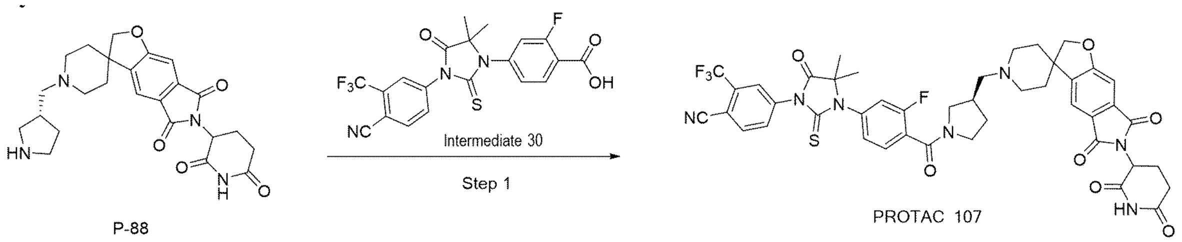


P-88

PROTAC 107

The first step: 4-(3-(4-((3R)-3-((6-(2,6-dioxopiperidin-3-yl)-5,7-dioxo-6,7 -dihydro-2H,5H-spiro[furo[2,3-f]isoindole-3,4'-piperidin-1'-yl)methyl)pyrrolidine-1-carbonyl)-3-fluorophenyl)-4,4-dimethyl-5-oxo-2-thioimidazolidin-1-yl)-2-(trifluoromethyl)benzonitrile (compound PROTAC 107)

Refer to the first step of Example 98 for preparation.

$^1$H NMR (600 MHz, DMSO-d6) δ 11.12 (d, J = 5.7 Hz, 1H), 8.42 (d, J = 8.2 Hz, 1H), 8.30 (s, 1H), 8.09 (d, J = 8.2 Hz, 1H), 7.74 - 7.59 (m, 2H), 7.48 (dd, J = 12.6, 7.5 Hz, 1H), 7.36 (dd, J = 16.4, 9.9 Hz, 2H), 5.17 - 5.03 (m, 1H), 4.80 - 4.67 (m, 2H), 3.91 (m, 1H), 3.39-3.31 (m, 4H), 3.23-3.04 (m, 3H), 2.96 - 2.83 (m, 2H), 2.83 - 2.65 (m, 1H), 2.61 (d, J = 16.4 Hz, 1H), 2.57 - 2.52 (m, 1H), 2.35 - 2.11 (m, 3H), 2.10 - 1.97 (m, 3H), 1.97 - 1.68 (m, 2H), 1.56 (s, 6H).
LC-MS(ESI): $[M+H]^+$ = 886.36

Example 138

Binding activity of the compounds disclosed by the invention to E3 ubiquitin ligase CRBN

The binding activity of compounds disclosed in the invention and positive control samples lenalidomide and pomalidomide with E3 ubiquitin ligase CRBN was determined by the way of compounds disclosed by the present invention, lenalidomide and pomalidomide with thalidomide marked with XL665 competitively binding to CRBN protein, which prevented the occurrence of fluorescence resonance energy transfer (FRET). From this, the binding force of the compounds to the CRBN protein was determined.
CRBN binding activity test kit (CEREBLON BINDING KITS (PE, 64BDCRBNPEG).
The detection is based on homogeneous time-resolved fluorescence (HTRF) technology. When the donor and the acceptor are close, the donor can transfer energy to the acceptor, exciting it to emit light at 665nm.
The donor used in this kit was Europium-labeled GST antibody (GST Eu Cryptate Antibody), and the acceptor was thalidomide labeled with XL665 (Thalidomide-Red reagent) . The donor binded to the CRBN protein containing the GST tag. The disclosed compounds in the present, along with lenalidomide and pomalidomide, competitively binded to the CRBN protein with thalidomide. The binding activity of the compounds was determined based on the fluorescence value emitted at 665 nm. The stronger the binding affinity of the compounds, the weaker the signal.
The donor and acceptor were diluted 50 times using the binding buffer from the kit (PROTAC binding buffer 1). The CRBN protein was diluted 45 times. The disclosed compound solution at 8mM was diluted 10 times with the diluent from the kit (1 X diluent), followed by a five-fold gradient dilution, performed 7 times sequentially.
5 μl of compound, 5 μl of diluted CRBN protein and 10 μl of donor-receptor mixture in equal volume were sequentially added to wells of a white 384-well plate (PE, Part number: 6008280). Incubate at room temperature for 3 hours.
The ratio of the emission signals from the acceptor and donor in each individual well is calculated.

$$\text{Ratio} = \text{665nm signal value/620nm signal value}*10^4$$

$$\text{The coefficient of variation (CV)} = \text{standard deviation (SD) / mean Ratio}*100$$

Using GraphPad Prism, $IC_{50}$ values were calculated based on compound concentration, Ratio and coefficient of variation.

Table 1 shows the $IC_{50}$ values of the disclosed compounds, lenalidomide, and pomalidomide.

| Compound | $IC_{50}$ (μM) | Compound | $IC_{50}$ (μM) |
|---|---|---|---|
| Lenalidomide | 3.1 | Compound14 | 3.5 |
| pomalidomide | 1.3 | Compound15 | 8.6 |
| Compound1 | 14.2 | Compound16 | 1.2 |
| Compound2 | 14.0 | Compound17 | 15.2 |
| Compound3 | 5.7 | Compound18 | 8.4 |
| Compound4 | 12.7 | Compound19 | 9.3 |
| Compound5 | 30.5 | Compound20 | 7.9 |
| Compound6 | 0.8 | Compound21 | 1.0 |
| Compound7 | 9.9 | Compound22 | 0.8 |
| Compound8 | 2.1 | Compound23 | 15.2 |
| Compound9 | 8.6 | Compound24 | 7.0 |
| Compound10 | 27.3 | Compound25 | 10.4 |
| Compound11 | 13.9 | Compound26 | 5.0 |
| Compound12 | 3.0 | Compound27 | 36.4 |
| Compound13 | 3.9 | Compound28 | 8.9 |

Conclusion: The compounds disclosed in the present invention can well bind to CRBN protein, and the binding ability of some compounds to CRBN is equivalent to or even better than that of positive control samples lenalidomide and pomalidomide.

Example 139

Inhibitory effect of the compounds disclosed in the present invention on cell proliferation in HeLa cells (sourced from ATCC)

The complete medium required for HeLa cells is DMEM high glucose (Gibco, Product number: 11995-065) basal medium containing 10% fetal bovine serum. HeLa cells were seeded in a 96-well plate at a density of 4,000 cells/well, and the next day, different concentrations of the compound of the present invention and the positive control sample Ceritinib (source: Bide Pharmaceuticals) were prepared to treat the cells. The cells were incubated at 37°C, 5% $CO_2$ cell culture incubator for 3 days. Cell viability was detected using the CCK-8 kit (Beyotime, Product number: C0040) according to the manufacturer's instructions. According to the inhibition rate, GraphPad Prism software was used to calculate the $IC_{50}$ value of each compound for 3 days, and the results are shown in Table 2.

Table 2. $IC_{50}$ values of the compounds of the present invention and the positive control on the inhibition of HeLa cell proliferation.

| Compound | $IC_{50}$(HeLa) [μM] |
|---|---|
| Ceritinib | 5.99 |
| PROTAC 25 | 2.19 |
| PROTAC 26 | 4.33 |
| PROTAC 27 | 4.27 |
| PROTAC 28 | 4.36 |
| PROTAC 29 | 5.41 |

(continued)

| Compound | $IC_{50}$(HeLa) [μM] |
|---|---|
| PROTAC 30 | 3.09 |
| PROTAC 31 | 4.65 |

Conclusion: the compounds of the present invention can effectively inhibit the proliferation of HeLa cells, and the inhibitory effect is better than that of the positive control.

Example 140

Inhibitory effect of the compounds disclosed in the present invention on cell proliferation in A549 cells (sourced from ATCC)

The complete medium required for A549 cells is F-12K (ATCC, Product number: 30-2004) basal medium containing 10% fetal bovine serum. A549 cells were seeded in a 96-well plate at a density of 2,800 cells/well, and the next day, different concentrations of the compound of the present invention and a positive control sample Ceritinib (source: Bide Pharmaceuticals) were prepared to treat the cells. The cells were incubated at 37°C, 5% $CO_2$ cell incubator for 4 days. Cell viability was detected using the CCK-8 kit (Beyotime, Product number: C0040) according to the manufacturer's instructions. According to the inhibition rate, the GraphPad Prism software was used to calculate the $IC_{50}$ value of each compound for 4 days, and the results are shown in Table 3.

Table 3 $IC_{50}$ values of the compounds of the present invention and the positive control on the inhibition of A549 cell proliferation.

| Compound | $IC_{50}$(A549) [μM] |
|---|---|
| Ceritinib | 4.31 |
| PROTAC 25 | 7.02 |
| PROTAC 26 | 4.27 |
| PROTAC 27 | 11.41 |
| PROTAC 28 | 6.81 |
| PROTAC 29 | 4.34 |
| PROTAC 30 | 4.38 |
| PROTAC 31 | 10.24 |

Conclusion: The compounds of the present invention can effectively inhibit the proliferation of A549 cells, and the inhibitory effect of some compounds is equivalent to that of the positive control.

Example 141

Inhibition of cell proliferation by compounds disclosed in the present invention in 22RV1 cells (sourced from ATCC)

The complete medium required for 22RV1 cells is RPMI1640 (ATCC, Product number: 30-2001) basal medium containing 10% fetal bovine serum. 22RV1 cells were seeded in a 96-well plate at a density of 4,000 cells/well, and the next day, different concentrations of the compound of the present invention and the positive control sample JQ1 (source: Bide Pharmaceuticals) were prepared to treat the cells, and the cells were cultured at 37°C and 5% $CO_2$ for 7 days in the incubator. Cell viability was detected using the CCK-8 kit (Beyotime, Product number: C0040) according to the manufacturer's instructions. According to the inhibition rate, the GraphPad Prism software was used to calculate the $IC_{50}$ value of each compound for 7 days, and the results are shown in Table 4.

Table 4. $IC_{50}$ values of the compounds of the present invention and the positive control on the inhibition of 22RV1 cell proliferation.

| Compound | $IC_{50}$(22RV1) [μM] |
|---|---|
| JQ1 | 0.044 |
| PROTAC 46 | 0.226 |
| PROTAC 47 | 0.103 |
| PROTAC 48 | 0.024 |
| PROTAC 49 | 0.141 |
| PROTAC 50 | 0.015 |
| PROTAC 51 | 0.123 |

Conclusion: The compounds in the present invention can effectively inhibit the proliferation of 22RV1 cells, and the inhibitory effect of some compounds is better than that of the positive control.

Example 142

Inhibitory effect of the compounds disclosed in the present invention on cell proliferation in NCI-H358 cells (sourced from ATCC)

The complete medium required for NCI-H358 cells is RPMI1640 (ATCC, Product number: 30-2001) basal medium containing 10% fetal bovine serum. NCI-H358 cells were seeded in a 96-well plate at a density of 2,800 cells/well, and the next day, different concentrations of the compounds of the present invention were prepared to treat the cells, and the cells were treated for 4 days in a 37°C, 5% $CO_2$ cell culture incubator. Cell viability was detected using a CCK-8 kit (Beyotime, Product number: C0040) according to the manufacturer's instructions. According to the inhibition rate, the GraphPad Prism software was used to calculate the $IC_{50}$ value of each compound for 4 days, and the results are shown in Table 5.

Table 5 $IC_{50}$ values of the compounds of the present invention on the inhibition of NCI-H358 cell proliferation.

| Compound | $IC_{50}$(NCI-H358) [μM] |
|---|---|
| PROTAC 17 | 16.81 |
| PROTAC 18 | 42.60 |
| PROTAC 21 | 23.15 |
| PROTAC 22 | 52.10 |
| PROTAC 23 | 71.18 |
| PROTAC 24 | 31.17 |

Conclusion: the compounds of the present invention can effectively inhibit the proliferation of NCI-H358 cells.

Example 143

Inhibition of cell proliferation by the compounds disclosed in the present invention in MV-4-11 cells (sourced from ATCC)

The complete medium required for MV-4-11 cells is IMDM (ATCC, Product number: 30-2005) basal medium containing 10% fetal bovine serum. MV-4-11 cells were seeded in a 96-well plate at a density of 7,500 cells/well, and different concentrations of the compound of the present invention and positive control samples Alisertib (source: Bide Pharmaceuticals), Palbociclib (source: Bide Pharmaceuticals), Ibrutinib (source Bide Pharmaceuticals) treated cells. The cells were incubated in a 37°C, 5% $CO_2$ cell incubator for 4 days. Cell viability was detected using the CCK-8 kit (Beyotime, Product number: C0040) according to the manufacturer's instructions. According to the inhibition rate, GraphPad Prism software was used to calculate the $IC_{50}$ value of each compound for 4 days, and the results are shown in Table 6-8.

Table 6 $IC_{50}$ values of the disclosed compounds and the positive control on the inhibition of MV-4-11 cell proliferation.

| Compound | $IC_{50}$(MV-4-11) [μM] |
|---|---|
| Alisertib | 0.026 |
| PROTAC 1 | 0.12 |
| PROTAC 2 | 0.081 |
| PROTAC 3 | 0.077 |
| PROTAC 4 | 0.025 |
| PROTAC 5 | 0.42 |
| PROTAC 6 | 0.32 |
| PROTAC 7 | 0.36 |
| PROTAC 8 | 0.38 |

Table 7 $IC_{50}$ values of the disclosed compounds and the positive control on the inhibition of MV-4-11 cell proliferation.

| Compound | $IC_{50}$(MV-4-11) [μM] |
|---|---|
| Palbociclib | 2.09 |
| PROTAC 38 | 5.03 |
| PROTAC 39 | 12.74 |
| PROTAC 41 | 2.16 |
| PROTAC 42 | 0.94 |
| PROTAC 43 | 2.49 |
| PROTAC 44 | 3.31 |
| PROTAC 45 | 4.25 |

Table 8 $IC_{50}$ values of the compounds of the present invention and the positive control on the inhibition of MV-4-11 cell proliferation.

| Compound | $IC_{50}$(MV-4-11) [μM] |
|---|---|
| Ibrutinib | 0.077 |
| PROTAC 46 | 0.0054 |
| PROTAC 47 | 0.017 |
| PROTAC 48 | 0.00096 |
| PROTAC 49 | 0.0082 |
| PROTAC 50 | 0.0069 |
| PROTAC 51 | 0.020 |

Conclusion: The compounds of the present invention can effectively inhibit the proliferation of MV-4-11 cells, wherein the inhibitory effect of some compounds is equivalent to or even better than that of the positive control.

Example 144

Inhibitory effect of the compounds disclosed in the present invention on cell proliferation in MOLM-13 cells (Nanjing Cobioer Biotechnology Co., Ltd.)

The complete medium required for MOLM-13 cells is 1640 (ATCC, Product number: 30-2001) basal medium containing 20% fetal bovine serum. MOLM-13 cells were seeded in a 96-well plate at a density of 7,500 cells/well, and different concentrations of the compounds of the present invention were prepared to treat the cells at the same time. The

cells were incubated in a 37°C, 5% $CO_2$ cell culture incubator for 4 days. Cell viability was detected using the CCK-8 kit (Beyotime, Product number: C0040) according to the manufacturer's instructions. According to the inhibition rate, GraphPad Prism software was used to calculate the $IC_{50}$ value of each compound for 4 days, and the results are shown in Table 9-10.

Table 9 $IC_{50}$ values of the compounds of the present inventionon the inhibition of MOLM-13 cell proliferation.

| Compounds | $IC_{50}$(MOLM-13) [μM] |
|---|---|
| PROTAC 9 | 1.11 |
| PROTAC 10 | 1.05 |
| PROTAC 12 | 4.77 |

Table 10 $IC_{50}$ values of the compounds of the present invention on the inhibition of MOLM-13 cell proliferation.

| Compounds | $IC_{50}$(MOLM-13) [μM] |
|---|---|
| PROTAC 39 | 12.43 |
| PROTAC 40 | 4.87 |
| PROTAC 41 | 2.19 |
| PROTAC 42 | 0.83 |
| PROTAC 43 | 1.87 |
| PROTAC 44 | 1.31 |
| PROTAC 45 | 4.34 |

Conclusion: the compounds of the present invention can effectively inhibit the proliferation of MOLM-13 cells.

Example 145

Inhibitory effect of compounds disclosed by the invention on cell proliferation in K562 cells (sourced from ATCC)

The complete medium required for K562 cells is IMDM (ATCC, Product number: 30-2005) basal medium containing 10% fetal bovine serum. K562 cells were seeded in a 96-well plate at a density of 7,500 cells/well, and different concentrations of the compound of the present invention and positive control samples Alisertib (source: Bide Pharmaceuticals) and dBET6 (source: Bide Pharmaceuticals) were prepared to treat the cells. The cells were incubated at a 37°C, 5% $CO_2$ cell culture incubator for 4 days. Cell viability was detected using the CCK-8 kit (Beyotime, Product number: C0040) according to the manufacturer's instructions. According to the inhibition rate, GraphPad Prism software was used to calculate the $IC_{50}$ value of each compound for 4 days, and the results are shown in Table 11-12.

Table 11 $IC_{50}$ values of the compounds of the present invention and the positive control on the inhibition of K562 cell proliferation.

| Compounds | IC50(K562) [μM] |
|---|---|
| Alisertib | 3.77 |
| PROTAC 1 | 0.15 |
| PROTAC 3 | 0.41 |
| PROTAC 4 | 0.22 |
| PROTAC 7 | 1.47 |

Table 12 $IC_{50}$ values of the compounds of the present invention and the positive control on the inhibition of K562 cell proliferation.

| Compound | $IC_{50}$(K562) [μM] |
|---|---|
| dBET6 | 0.001 |

(continued)

| Compound | $IC_{50}$(K562) [μM] |
|---|---|
| PROTAC 9 | 0.016 |
| PROTAC 10 | 0.017 |
| PROTAC 11 | 0.066 |
| PROTAC 12 | 0.040 |
| PROTAC 13 | 0.020 |
| PROTAC 14 | 0.057 |
| PROTAC 15 | 0.005 |
| PROTAC 16 | 0.048 |

Conclusion: The compounds of the present invention can effectively inhibit the proliferation of K562 cells, wherein the inhibitory effect of some compounds is equivalent to or even better than that of the positive control.

Example 146

Inhibitory effect of compound disclosed by the invention on cell proliferation in JeKo-1 cells (sourced from ATCC)

The complete medium required for JeKo-1 cells is RPMI1640 (ATCC, Product number: 30-2001) basal medium containing 20% fetal bovine serum. The JeKo-1 cells were seeded in a 96-well plate at a density of 10,000 cells/well, and different concentrations of the compounds of the present invention were prepared to treat the cells at the same time. The cells were incubated at a 37° C, 5% $CO_2$ cell culture incubator for 4 days. Cell viability was detected using the CCK-8 kit (Beyotime, Product number: C0040) according to the manufacturer's instructions. According to the inhibition rate, the GraphPad Prism software was used to calculate the $IC_{50}$ value of each compound for 4 days, and the results are shown in Table 13.

Table 13 $IC_{50}$ values of the compounds of the present invention on the inhibition of Jeko-1 cell proliferation.

| Compound | $IC_{50}$(Jeko-1) [μM] |
|---|---|
| PROTAC 32 | 18.40 |
| PROTAC 33 | 63.75 |
| PROTAC 35 | 19.14 |
| PROTAC 36 | 19.21 |
| PROTAC 37 | 32.42 |

Conclusion: the compounds of the present invention can effectively inhibit the proliferation of Jeko-1 cells.

Example 147

Inhibitory effect of compounds disclosed in the present invention on cell proliferation in Z138 cells (sourced from ATCC)

The complete medium required for Z138 cells is IMDM (ATCC, Product number: 30-2005) basal medium containing 10% horse serum. Z138 cells were seeded in a 96-well plate at a density of 10,000 cells/well, and different concentrations of the compound of the present invention and a positive control sample Ibrutinib (source: Bide Pharmaceuticals) were prepared to treat the cells. The cells were incubated in a 37°C, 5% $CO_2$ cell incubator for 4 days. Cell viability was detected using the CCK-8 kit (Beyotime, Product number: C0040) according to the manufacturer's instructions. According to the inhibition rate, the GraphPad Prism software was used to calculate the $IC_{50}$ value of each compound for 4 days, and the results are shown in Table 14.

Table 14 $IC_{50}$ values of the compounds of the present invention on the inhibition of Z138 cell proliferation.

| Compound | $IC_{50}$(Z138) [μM] |
|---|---|
| Ibrutinib | 21.09 |
| PROTAC 32 | 16.03 |
| PROTAC 33 | 34.88 |
| PROTAC 34 | 13.93 |
| PROTAC 35 | 22.62 |
| PROTAC 36 | 25.41 |
| PROTAC 37 | 36.07 |

Conclusion: The compounds of the present invention can effectively inhibit the proliferation of Z138 cells, wherein the inhibitory effect of some compounds is equivalent to or even better than that of the positive control.

Example 148

Inhibitory effect of compounds disclosed by the invention on cell proliferation in VCaP cells (sourced from ATCC)

The complete medium required for VCaP cells is DMEM high glucose (Gibco, Product number: 11995-065) basal medium containing 10% fetal bovine serum. The VCaP cells were seeded in a 96-well plate at a density of 15,000 cells/well, and the next day, different concentrations of the compound of the present invention and the positive control sample ARV-110 (source: Anaiji) were prepared to treat the cells, and the cells were incubated at 37°C and 5% $CO_2$ for 7 days in the cell culture incubator. Cell viability was detected using the CCK-8 kit (Beyotime, Product number: C0040) according to the manufacturer's instructions. According to the inhibition rate, the GraphPad Prism software was used to calculate the $IC_{50}$ value of each compound for 7 days, and the results are shown in Table 15.

Table 15 $IC_{50}$ values of the compounds of the present invention and the positive control on the inhibition of VCaP cell proliferation.

| Compound | $IC_{50}$(VCaP) [μM] |
|---|---|
| ARV-110 | 0.19 |
| PROTAC 60 | 1.20 |
| PROTAC 61 | 0.080 |
| PROTAC 62 | 0.10 |
| PROTAC 63 | 0.051 |
| PROTAC 64 | 0.96 |
| PROTAC 65 | 0.0011 |
| PROTAC 66 | 0.05 |
| PROTAC 67 | 6.50 |
| PROTAC 68 | 0.03 |
| PROTAC 69 | 4.26 |
| PROTAC 70 | 9.73 |
| PROTAC 71 | 4.57 |
| PROTAC 72 | 7.40 |
| PROTAC 73 | 2.49 |
| PROTAC 74 | 3.26 |
| PROTAC 75 | 0.17 |
| PROTAC 76 | 0.35 |

(continued)

| Compound | $IC_{50}$(VCaP) [μM] |
|---|---|
| PROTAC 77 | 2.03 |
| PROTAC 78 | 2.70 |
| PROTAC 79 | 4.09 |
| PROTAC 80 | 9.48 |
| PROTAC 81 | 9.51 |
| PROTAC 82 | 1.3 |
| PROTAC 83 | 0.89 |
| PROTAC 84 | 3.80 |
| PROTAC 85 | 0.62 |

Conclusion: The compounds of the present invention can effectively inhibit the proliferation of VCaP cells, and the inhibitory effect of some compounds is equivalent to or even better than that of the positive control.

Example 149

Inhibitory effect of compounds disclosed in the present invention on cell proliferation in LNCaP FGC cells (sourced from ATCC)

The complete medium required for LNCaP FGC cells is RPMI 1640 (ATCC, Product number: 30-2001) basal medium containing 10% fetal calf serum. Seed LNCAP FGC cells in a 96-well plate at a density of 5,000 cells/well, prepare different concentrations of the compound of the present invention and the positive control sample ARV-110 (source: Energy chemical) to treat the cells on the next day, and store them at 37°C, 5% $CO_2$ for 4 days in a cell incubator. Cell viability was detected using the CCK-8 kit (Beyotime, Product number: C0040) according to the manufacturer's instructions. According to the inhibition rate, the GraphPad Prism software was used to calculate the $IC_{50}$ value of each compound for 4 days, and the results are shown in Table 16.

Table 16 $IC_{50}$ values of the compounds of the present invention and the positive control on the inhibition of LNCaP cell proliferation.

| Compound | $IC_{50}$(LNCaP) [μM] |
|---|---|
| ARV-110 | 0.32 |
| PROTAC 60 | 0.93 |
| PROTAC 61 | 2.20 |
| PROTAC 62 | 1.16 |
| PROTAC 63 | 0.17 |
| PROTAC 64 | 0.13 |
| PROTAC 65 | 2.37 |
| PROTAC 66 | 3.00 |
| PROTAC 67 | 6.10 |
| PROTAC 68 | 3.86 |
| PROTAC 69 | 3.39 |
| PROTAC 70 | 7.06 |
| PROTAC 72 | 5.34 |
| PROTAC 73 | 4.53 |
| PROTAC 74 | 2.66 |

(continued)

| Compound | $IC_{50}$(LNCaP) [μM] |
|---|---|
| PROTAC 75 | 0.53 |
| PROTAC 76 | 3.24 |
| PROTAC 77 | 6.45 |
| PROTAC 80 | 3.24 |
| PROTAC 83 | 2.63 |
| PROTAC 85 | 27.9 |

Conclusion: The compounds of the present invention can effectively inhibit the proliferation of LNCaP cells, wherein the inhibitory effect of some compounds is equivalent to or even better than that of the positive control.

Example 150

Inhibition of cell proliferation by compounds disclosed in the present invention in T-47D cells (sourced from ATCC)

The complete medium required for T-47D cells is 1640 (ATCC, Product number: A10491-01) basal medium containing 10% fetal bovine serum. T-47D cells were seeded in a 96-well plate at a density of 3,000/well, and different concentrations of the compound of the present invention and the positive control sample ARV-471 (source: Innochem) were prepared to treat the cells. The cells were incubated at 37°C, 5% for 6 days in a $CO_2$ cell incubator. Cell viability was detected using the CCK-8 kit (Beyotime, Product number: C0040) according to the manufacturer's instructions. According to the inhibition rate, the GraphPadPrism software was used to calculate the $IC_{50}$ value of each compound for 6 days, and the results are shown in Table 17.

Table 17 $IC_{50}$ values of the compounds of the present invention and the positive control on the inhibition of T-47D cell proliferation.

| Compound | $IC_{50}$(T-47D) [μM] |
|---|---|
| ARV-471 | 0.0017 |
| PROTAC 52 | 0.0092 |
| PROTAC 53 | 0.0029 |
| PROTAC 54 | 0.068 |
| PROTAC 55 | 0.0066 |
| PROTAC 56 | 0.0016 |
| PROTAC 57 | 0.0020 |
| PROTAC 58 | 0.0010 |
| PROTAC 59 | 0.0052 |

Conclusion: The compounds of the present invention can effectively inhibit the proliferation of T-47D cells, wherein the inhibitory effect of some compounds is equivalent to or even better than that of the positive control.

Example 151

Inhibitory effect of compounds disclosed by the invention on cell proliferation in MCF-7 cells (sourced from ATCC)

The complete medium required for MCF-7 cells is DMEM (gibco, Product number: 11995-065) basal medium containing 10% fetal bovine serum. MCF-7 cells were seeded in a 96-well plate at a density of 800/well, and different concentrations of the compound of the present invention and the positive control sample ARV-471 (source: Enochem) were prepared to treat the cells. The cells were incubated at 37°C, 5% 9 days in a $CO_2$ cell incubator. Cell viability was detected using the CCK-8 kit (Beyotime, Product number: C0040) according to the manufacturer's instructions. According to the inhibition rate, GraphPadPrism software was used to calculate the $IC_{50}$ value of each compound for 9 days, and the

results are shown in Table 18.

Table 18 $IC_{50}$ values of the compounds of the present invention and the positive control on the inhibition of MCF-7 cell proliferation.

| Compound | $IC_{50}$(MCF-7) [μM] |
|---|---|
| ARV-471 | 0.07 |
| PROTAC 52 | 0.03 |
| PROTAC 53 | 0.09 |
| PROTAC 54 | 0.17 |
| PROTAC 55 | 0.04 |
| PROTAC 56 | 0.07 |
| PROTAC 57 | 0.01 |
| PROTAC 58 | 0.02 |
| PROTAC 59 | 0.13 |

Conclusion: The compounds of the present invention can effectively inhibit the proliferation of MCF-7 cells, wherein the inhibitory effect of some compounds is equivalent to or even better than that of the positive control.

## Claims

1. A compound, wherein the compound is a compound represented by formula I, or an isomer, an isotopic derivative, a polymorph, a prodrug thereof, or a pharmaceutically acceptable salt or a solvate thereof:

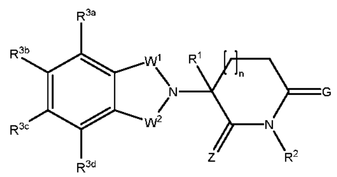

I,

wherein:

$W^1$ and $W^2$ are identical or different, and are each independently $CR^aRb$, C(=O), $NR^a$ or $SO_2$, and at least one of $W^1$ and $W^2$ is C(=O);
G and Z are identical or different, and are each independently selected from O, S, and Se $R^{3b}$ and $R^{3c}$ together with the carbon atoms to which they are attached form

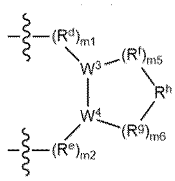

or

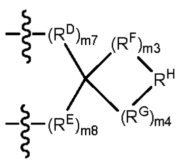

,

and $R^{3a}$ and $R^{3d}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkyloxyacyl, alkylaminoacyl, cycloalkyl, heterocyclyl, aryl and heteroaryl, wherein the alkyl, heteroalkyl, alkenyl, alkynyl, alkoxy, cycloalkyl, heterocyclyl, aryl and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkyloxyacyl, alkylaminoacyl, aryl and heteroaryl;

or $R^{3c}$ and $R^{3d}$ together with the carbon atoms to which they are attached form

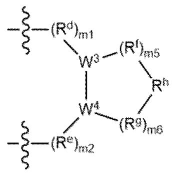

or

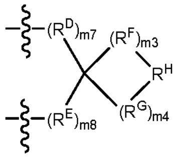

,

and $R^{3a}$ and $R^{3b}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkyloxyacyl, alkylaminoacyl, cycloalkyl, heterocyclyl, aryl and heteroaryl, wherein the alkyl, heteroalkyl, alkenyl, alkynyl, alkoxy, cycloalkyl, heterocyclyl, aryl and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkanoyl, alkyloxyacyl, alkylaminoacyl, aryl and heteroaryl;

or $R^{3a}$ and $R^{3b}$ together with the carbon atoms to which they are attached form

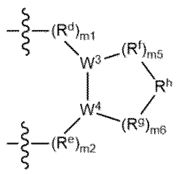

or

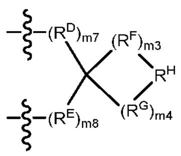

,

and $R^{3c}$ and $R^{3d}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkyloxyacyl, alkylaminoacyl, cycloalkyl, heterocyclyl, aryl and heteroaryl, wherein the alkyl, heteroalkyl, alkenyl, alkynyl, alkoxy, cycloalkyl, heterocyclyl, aryl and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkanoyl, alkyloxyacyl, alkylaminoacyl, aryl and heteroaryl;

$R^d$, $R^e$, $R^f$, $R^g$, $R^D$, $R^E$, $R^F$, and $R^G$ at each occurrence are each independently $C(R^m)_2$, $NR^m$, C(=O), O, or S;

$W^3$ and $W^4$ at each occurrence are each independently $CR^m$ or N;

$R^h$ and $R^H$ at each occurrence are each independently $NR^{1h}$, C(=O), $SO_2$, or $CR^{2h}R^{3h}$, and when $R^D$, $R^E$, $R^F$, and $R^G$ are all $C(R^m)_2$, $R^H$ is C(=O), or $CR^{2h}R^{3h}$;

m1 and m2 at each occurrence are independently an integer of 0, 1, 2, 3, 4, 5, or 6, and m1+ m2 ≤ 6;

m3 at each occurrence is independently an integer of 0, 1, 2, 3, 4, 5, 6, or 7, and m4 is an integer of 1, 2, 3, 4, 5, 6, 7, or 8, and m3+ m4≤8;

m5 and m6 at each occurrence are each independently an integer of 0, 1, 2, 3, 4, 5, 6, or 7, and m5+ m6 7;

m7 and m8 at each occurrence are each independently an integer of 0, 1, 2, 3, 4, 5, 6, or 7, and m7+ m8≤7;

$R^m$ at each occurrence is independently selected from H, deuterium atom, halogen, alkyl, deuteroalkyl, heteroalkyl, alkenyl, alkynyl, alkoxy, haloalkyl, haloalkoxy, hydroxyl, hydroxyalkyl, nitro, cyano, amino, alkylamino, alkylacyl, alkyloxyacyl, alkylaminoacyl, cycloalkyl, heterocyclyl, aryl and heteroaryl, wherein the alkyl , heteroalkyl, alkenyl, alkynyl, alkoxy, cycloalkyl, heterocyclyl, aryl and heteroaryl are each independently and optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkyloxyacyl, alkylaminoacyl, aryl and heteroaryl;

$R^{1h}$, $R^{2h}$, and $R^{3h}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkyloxyacyl, alkylaminoacyl, cycloalkyl, heterocyclyl, aryl, and heteroaryl, wherein the alkyl, heteroalkyl, alkoxy, cycloalkyl, heterocyclyl, aryl, and heteroaryl are each independently substituted with one or more substituents selected from halogen, alkyl, heteroalkyl, alkenyl, alkynyl, alkoxy, haloalkyl, hydroxyl, hydroxyalkyl, cyano, amino, nitro, cycloalkyl, heterocyclyl, alkylamino, alkylacyl, alkyloxyacyl, alkylaminoacyl, aryl and heteroaryl;

$R^1$ is selected from H, halogen, deuterium atom, $C_1$-$C_6$ alkyl, $C_1$-$C_6$ alkoxy, hydroxyl, cycloalkyl, $C_1$-$C_6$ haloalkyl, and hydroxyalkyl, preferably, $R^1$ is selected from H, F, Cl, Br, I, $C_1$-$C_3$ alkyl, $C_1$-$C_3$ alkoxy, $C_3$-$C_6$ cycloalkyl, $C_1$-$C_3$ haloalkyl, hydroxyl, and $C_1$-$C_6$ hydroxyalkyl;

$R^2$, $R^a$, and $R^b$ are each independently selected from H, deuterium atom, F, Cl, Br, I, $C_1$-$C_6$ alkyl, hydroxyl, $C_1$-$C_6$ haloalkyl, $C_1$-$C_6$ hydroxyalkyl and $C_1$-$C_6$ alkoxy; and

n is 0, 1, 2 or 3.

**2.** A compound, wherein the compound is a compound represented by formula I, or an isomer, an isotope derivative, a polymorph, a prodrug thereof, or a pharmaceutically acceptable salt or a solvate thereof:

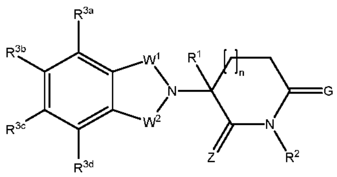 I,

wherein:

$W^1$ and $W^2$ are identical or different, and are each independently $CR^aR^b$ or C(=O), and at least one of $W^1$ and $W^2$ is C(=O);

G and Z are identical or different, and are each independently selected from O, S, and Se;

$R^{3b}$ and $R^{3c}$ together with the carbon atoms to which they are attached form

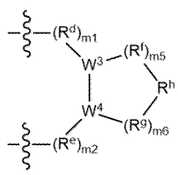

or

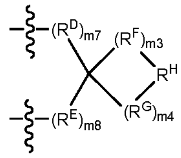

,

and $R^{3a}$ and $R^{3d}$ are each independently selected from H, deuterium atom, halogen, alkyl, deuterated alkyl, heteroalkyl, alkenyl, alkynyl, alkoxy, haloalkyl, haloalkoxyl, hydroxyl, hydroxyalkyl, nitro, cyano, amino, cycloalkyl, heterocyclyl, aryl and heteroaryl, wherein the alkyl, heteroalkyl, alkenyl, alkynyl, alkoxy, cycloalkyl, heterocyclyl, aryl and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, aryl and heteroaryl;

or $R^{3c}$ and $R^{3d}$ together with the carbon atoms to which they are attached form

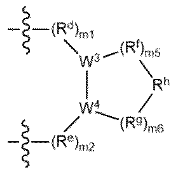

or

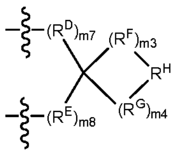

,

and $R^{3a}$ and $R^{3b}$ are each independently selected from H, deuterium atom halogen, alkyl, deuterated alkyl, heteroalkyl, alkenyl, alkynyl, alkoxy, haloalkyl, haloalkoxy, hydroxyl, hydroxyalkyl, nitro, cyano, amino, cycloalkyl, heterocyclyl, aryl and heteroaryl, wherein the alkyl, heteroalkyl, alkenyl, alkynyl, alkoxy, cycloalkyl, and heterocyclyl, aryl and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, aryl and heteroaryl;

or $R^{3a}$ and $R^{3b}$ together with the carbon atoms to which they are attached form

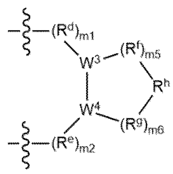

or

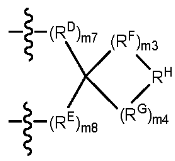

,

and $R^{3c}$ and $R^{3d}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, cycloalkyl, heterocyclyl, aryl and heteroaryl, wherein the alkyl, heteroalkyl, alkenyl, alkynyl, alkoxy, cycloalkyl, heterocyclyl, aryl and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, aryl and heteroaryl;

$R^d$, $R^e$, $R^f$, $R^g$, $R^D$, $R^E$, $R^F$, and $R^G$ at each occurrence are each independently $C(R^m)_2$, $NR^m$, O, or S;
$W^3$ and $W^4$ at each occurrence are each independently CH or N;
$R^h$ and $R^H$ at each occurrence are each independently $NR^{1h}$, C(=O), or $CR^{2h}R^{3h}$, and when $R^D$, $R^E$, $R^F$, and $R^G$ are all $C(R^m)_2$, $R^H$ is C(=O), or $CR^{2h}R^{3h}$;
m1 and m2 at each occurrence are independently an integer of 0, 1, 2, 3, 4, 5, or 6, and $m1+m2\leq6$;
m3 at each occurrence is independently an integer of 0, 1, 2, 3, 4, 5, 6, or 7, and m4 is an integer of 1, 2, 3, 4, 5, 6, 7, or 8, and $m3+m4\leq8$;
m5 and m6 at each occurrence are independently an integer of 0, 1, 2, 3, 4, 5, 6, or 7, and $m5+m6\leq7$;
m7 and m8 at each occurrence are independently an integer of 0, 1, 2, 3, 4, 5, 6, or 7, and $m7+m8\leq7$;
$R^m$ at each occurrence is independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, alkyl, nitro, cyano, amino, cycloalkyl, heterocyclyl, aryl and heteroaryl, wherein the alkyl, heteroalkyl, alkenyl, alkynyl, alkoxy, cycloalkyl, heterocyclyl, aryl and heteroaryl are each independently optionally substituted with one or more substituents selected from halogen, alkyl, heteroalkyl, alkoxy, haloalkyl, hydroxyl, hydroxyalkyl, cyano, amino, nitro, cycloalkyl, heterocyclyl, aryl and heteroaryl;

$R^{1h}$, $R^{2h}$, or $R^{3h}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, cycloalkyl, heterocyclyl, aryl, and heteroaryl, wherein the alkyl, heteroalkyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, haloalkyl, hydroxyl, hydroxyalkyl, cyano, amino, nitro, cycloalkyl, heterocyclyl, aryl and heteroaryl;
$R^1$ is selected from H, halogen, $C_1$-$C_3$ alkyl, $C_1$-$C_3$ alkoxy, hydroxyl, and hydroxyalkyl, preferably $R^1$ is selected from H, F, Cl, Br, I, $C_1$-$C_3$ alkyl, $C_1$-$C_3$ alkoxy, $C_1$-$C_3$ haloalkyl, hydroxyl, and $C_1$-$C_3$ hydroxyalkyl;
$R^2$, $R^a$, and $R^b$ are each independently selected from H, $C_1$-$C_3$ alkyl, and $C_1$-$C_3$ alkoxy; and
n is 0, 1, or 2.

**3.** The compound according to claims 1 or 2, wherein:

$W^1$ and $W^2$ are identical or different, and are each independently $CH_2$ or C(=O), and at least one of $W^1$ and $W^2$ is C(=O); and/or
G and Z are O; and/or
$R^{3b}$ and $R^{3c}$ together with the carbon atoms to which they are attached form

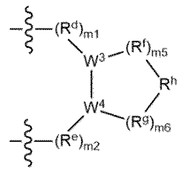

or

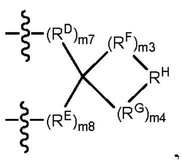

,

and $R^{3a}$ and $R^{3d}$ are each independently selected from H, deuterium atom halogen, $C_1$-$C_6$ alkyl, heteroalkyl, $C_2$-$C_6$ alkenyl, $C_2$-$C_6$ alkynyl, $C_1$ -$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, aryl and heteroaryl are each independently substituted with one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, aryl and heteroaryl , preferably $R^{3a}$ and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy , hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$-$C_6$

alkyl, $C_1$- $C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano , amino , nitro , $C_3$-$C_8$ cycloalkyl, $C_4$-$C_{10}$ heterocyclyl, $C_6$-$C_{10}$ aryl and $C_5$-$C_{10}$ heteroaryl, preferably $R^{3a}$ and $R^{3d}$ are each independently selected from H, deuterium atom, halogen, $C_1$-$C_3$ alkyl, and $C_1$-$C_3$ alkoxy;

or $R^{3c}$ and $R^{3d}$ together with the carbon atoms to which they are attached form

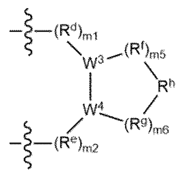

or

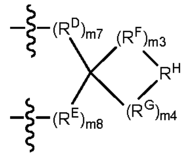

and $R^{3a}$ and $R^{3b}$ are each independently selected from H, deuterium atom halogen. $C_1$-$C_6$ alkyl, heteroalkyl, $C_2$-$C_6$ alkenyl, $C_2$-$C_6$ alkynyl, $C_1$-$C_6$ alkoxy, $C_1$-$C_6$ haloalkyl , $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$ -$C_6$ alkoxy, Cs-Cs cycloalkyl, $C_4$-$C_{10}$ heterocyclyl, $C_6$ -$C_{10}$ aryl, $C_5$-$C_{10}$ heteroaryl are each independently optionally substituted with one or more substituents selected from the groups of halogen, $C_1$ -$C_6$ alkyl, $C_1$ - $C_6$ heteroalkyl, $C_1$-$C_6$ alkoxy, $C_1$ -$C_6$ haloalkyl, hydroxyl, $C_1$-$C_6$ hydroxyalkyl, cyano , amino , nitro , $C_3$-$C_8$ cycloalkyl, $C_4$-$C_{10}$ heterocyclyl, $C_6$-$C_{10}$ aryl and $C_5$-$C_{10}$ heteroaryl, preferably $R^{3a}$ and $R^{3b}$ are each independently selected from H, deuterium atom, F, Cl, Br, I, $C_1$ -$C_6$ alkyl, $C_1$-$C_6$ heteroalkyl, $C_2$ -$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$ -$C_{10}$ aryl and $C_5$-$C_{10}$ heteroaryl, wherein the $C_1$-$C_6$ alkyl, $C_1$ -$C_6$ heteroalkyl, $C_2$ -$C_6$ alkenyl, $C_2$-$C_6$ alkynyl, $C_1$ -$C_6$ alkoxy, $C_3$ -$C_8$ cycloalkyl, $C_4$ -$C_{10}$ heterocyclyl, $C_6$ -$C_{10}$ aryl and $C_5$ -$C_{10}$ heteroaryl are each independently optionally substituted with one or more substituents selected from F, Cl, Br, I, $C_1$-$C_6$ alkyl, $C_1$ -$C_6$ heteroalkyl, $C_1$-$C_6$ alkoxy, $C_1$ -$C_6$ haloalkyl, hydroxyl, $C_1$-$C_6$ hydroxyalkyl, cyano, amino, nitro, $C_3$-$C_8$ cycloalkyl, $C_4$ -$C_{10}$ heterocyclyl, $C_6$-$C_{10}$ aryl, and $C_5$ -$C_{10}$ heteroaryl, preferably $R^{3a}$ and $R^{3b}$ are each independently selected from H, deuterium atom, halogen, $C_1$-$C_3$ alkyl, and $C_1$-$C_3$ alkoxy;

or $R^{3a}$ and $R^{3b}$ together with the carbon atoms to which they are attached form

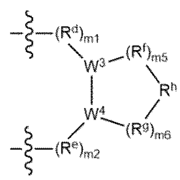

or

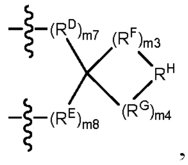

,

and $R^{3c}$ and $R^{3d}$ are each independently selected from H, deuterium, halogen, $C_1$ -$C_6$ alkyl, heteroalkyl, $C_2$ -$C_6$ alkenyl, $C_2$ -$C_6$ alkynyl, $C_1$ -$C_6$ alkoxy, $C_1$ -$C_6$ haloalkoxy, $C_1$-$C_6$ haloalkoxy, hydroxyl, $C_1$ -$C_6$ hydroxyalkyl, nitro, cyano, amino, $C_3$ -$C_8$ cycloalkyl, $C_4$ -$C_{10}$ heterocyclyl, $C_6$ -$C_{10}$ aryl, and $C_5$-$C_{10}$ heteroaryl, wherein the $C_1$-$C_6$

alkyl, $C_1$ -$C_6$ heteroalkyl, $C_2$ -$C_6$ alkenyl, $C_2$ -$C_6$ alkynyl, $C_1$ -$C_6$ alkoxy, $C_3$-$C_8$ cycloalkyl, $C_4$-$C_{10}$ heterocyclyl, $C_6$-$C_{10}$ aryl, $C_5$ -$C_{10}$ heteroaryl are each independently optionally substituted with one or more substituents selected from the group of halogen, $C_1$-$C_6$ alkyl, $C_1$-$C_6$ heteroalkyl, $C_1$-$C_6$ alkoxy, $C_1$ -$C_6$ haloalkyl, hydroxyl, $C_1$ -$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl, preferably $R^{3c}$ and $R^{3d}$ each independently selected from H, deuterium atom, F, Cl, Br, I, $C_1$ -$C_6$ alkyl, $C_1$-$C_6$ heteroalkyl, $C_2$ -$C_6$ alkenyl, $C_2$-$C_6$ alkynyl, $C_1$ -$C_6$ alkoxy, hydroxyl, nitro, cyano, amino, $C_3$ -$C_8$ cycloalkyl, $C_4$ -$C_{10}$ heterocyclyl, $C_6$ -$C_{10}$ aryl and $C_5$ -$C_{10}$ heteroaryl, wherein the $C_1$ -$C_6$ alkyl, $C_1$ -$C_6$ heteroalkyl, $C_2$ -$C_6$ alkenyl, $C_2$ -$C_6$ alkynyl, $C_1$ -$C_6$ alkoxy, $C_3$ -$C_8$ cycloalkyl, $C_4$ -$C_{10}$ heterocyclyl, $C_6$ -$C_{10}$ aryl and $C_5$ -$C_{10}$ heteroaryl are each independently optionally substituted with one or more substituents selected from F, Cl, Br, I, $C_1$ -$C_6$ alkyl, $C_1$ -$C_6$ heteroalkyl, $C_1$ -$C_6$ alkoxy, $C_1$ -$C_6$ haloalkyl, hydroxyl, $C_1$-$C_6$ hydroxyalkyl, cyano, amino, nitro, $C_3$ -$C_8$ cycloalkyl, $C_4$ -$C_{10}$ heterocyclyl, $C_6$ -$C_{10}$ aryl, and $C_5$ -$C_{10}$ heteroaryl, preferably $R^{3c}$ and $R^{3d}$ each independently selected from H, deuterium atom, halogen, $C_1$ -$C_3$ alkyl, and $C_1$ -$C_3$ alkoxy; and/or

$R^d$ , $R^e$ , $R^f$ , $R^g$ , $R^D$ , $R^E$ , $R^F$ , and $R^G$ at each occurrence are independently $C(R^m)_2$ or O; and/or

$W^3$ and $W^4$ are CH; and/or

$R^{1h}$, $R^{2h}$ , and $R^{3h}$ are each independently selected from H, deuterium atom, halogen, $C_1$ -$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, $C_1$ -$C_6$ haloalkyl, $C_1$-$C_6$ haloalkoxy, hydroxyl, $C_1$ -$C_6$ hydroxyalkyl, nitro, cyano, amino, $C_3$ -$C_8$ cycloalkyl, $C_4$ -$C_{10}$ heterocyclyl, $C_6$-$C_{10}$ aryl, and $C_5$-$C_{10}$ heteroaryl, wherein the $C_1$-$C_6$ alkyl, $C_1$ -$C_6$ heteroalkyl, $C_2$ -$C_6$ alkenyl, $C_2$ -$C_6$ alkynyl, $C_1$ -$C_6$ alkoxy, $C_3$ -$C_8$ cycloalkyl, $C_4$ -$C_{10}$ heterocyclyl, $C_6$ -$C_{10}$ aryl, and $C_5$ -$C_{10}$ heteroaryl are independently and optionally substituted with one or more substituents selected from the group consisting of halogen, $C_1$ -$C_6$ alkyl, $C_1$ -$C_6$ heteroalkyl, $C_1$ -$C_6$ alkoxy, $C_1$ -$C_6$ haloalkyl, hydroxyl, $C_1$ -$C_6$ hydroxyalkyl, cyano, amino, nitro, $C_3$-$C_8$ cycloalkyl, $C_4$-$C_{10}$ heterocyclyl, $C_6$ -$C_{10}$ aryl, and $C_5$ -$C_{10}$ heteroaryl; preferably, $R^{1h}$ , $R^{2h}$ , and $R^{3h}$ are each independently selected from H, deuterium atom, F, Cl, Br, I, $C_1$ -$C_6$ alkyl, $C_1$ -$C_6$ deuterated alkyl, $C_1$-$C_6$ heteroalkyl, $C_2$ -$C_6$ alkenyl, $C_2$ -$C_6$ alkynyl, $C_1$ -$C_6$ alkoxy, hydroxyl, nitro, cyano, amino, $C_3$-$C_8$ cycloalkyl, $C_4$ -$C_{10}$ heterocyclyl, $C_6$-$C_{10}$ aryl, and $C_5$-$C_{10}$ heteroaryl, wherein the $C_1$ -$C_6$ alkyl, $C_1$ -$C_6$ heteroalkyl, $C_2$ -$C_6$ alkenyl, $C_2$ -$C_6$ alkynyl, $C_1$ -$C_6$ alkoxy, $C_3$-$C_8$ cycloalkyl, $C_4$ -$C_{10}$ heterocyclyl, $C_6$ -$C_{10}$ aryl, and $C_5$ -$C_{10}$ heteroaryl are each independently and optionally substituted with one or more substituents selected from F, Cl, Br, I, $C_1$-$C_6$ alkyl, $C_1$ -$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$ -$C_8$ cycloalkyl, $C_4$ -$C_{10}$ heterocyclyl, $C_6$-$C_{10}$ aryl, and $C_5$-$C_{10}$ heteroaryl; preferably, $R^{1h}$ , $R^{2h}$ , and $R^{3h}$ are each independently selected from H, deuterium atom, halogen, $C_1$-$C_3$ alkyl, and $C_1$ -$C_3$ alkoxy; and/or

m1 and m2 at each occurrence are each independently an integer of 0, 1, 2, or 3, and $m1+m2 \leq 3$; preferably m1+m2=1 or m1+m2=2; and/or

m3 at each occurrence is independently an integer of 0, 1, 2, 3, or 4, and m4 is an integer of 1, 2, 3, 4, or 5, and $m3+m4 \leq 5$; preferably m3+m4=2 , m3+m4=3 or m3+m4=4; and/or

m5 and m6 at each occurrence are each independently an integer of 0, 1, 2, 3 or 4, and $m5+m6 \leq 4$, preferably m5+m6=2 or m5+m6=3; and/or

m7 and m8 at each occurrence are independently an integer of 0, 1, 2, 3 or 4, and $m7+m8 \leq 4$, preferably m7+m8=2 or m7+m8=3; and/or

$R^m$ at each occurrence is independently selected from H, deuterium atom, halogen, $C_1$ -$C_6$ alkyl, $C_1$-$C_6$ heteroalkyl, $C_2$ -$C_6$ alkenyl, $C_2$ -$C_6$ alkynyl, $C_1$-$C_6$ alkoxy, $C_1$-$C_6$ haloalkyl, $C_1$-$C_6$ haloalkoxy, hydroxyl, $C_1$ -$C_6$ hydroxyalkyl, nitro, cyano, amino, $C_3$ -$C_8$ cycloalkyl, $C_4$ -$C_{10}$ heterocyclyl, aryl and heteroaryl, wherein the $C_1$ -$C_6$ alkyl, $C_1$ -$C_6$ heteroalkyl, $C_2$-$C_6$ alkenyl, $C_2$ -$C_6$ alkynyl, $C_1$ -$C_6$ alkoxy, $C_3$ -$C_8$ cycloalkyl, $C_4$ -$C_{10}$ heterocyclyl, $C_6$ -$C_{10}$ aryl, and $C_5$ -$C_{10}$ heteroaryl are each independently optionally substituted with one or more substituents selected from the group consisting of halogen, $C_1$ -$C_6$ alkyl, $C_1$ - $C_6$ heteroalkyl, $C_1$ -$C_6$ alkoxy, $C_1$ -$C_6$ haloalkyl, hydroxyl, $C_1$ -$C_6$ hydroxyalkyl, cyano, amino, nitro, $C_3$ - $C_8$ cycloalkyl, $C_4$ -$C_{10}$ heterocyclyl, $C_6$-$C_{10}$ aryl and $C_5$-$C_{10}$ heteroaryl ; preferably , $R^m$ at each occurrence is independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$ -$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; Preferably $R^m$ at each occurrence is independently selected from H, deuterium atom, halogen, $C_1$-$C_3$ alkyl, and $C_1$-$C_3$ alkoxy; and/or

$R^1$ is selected from H, halogen, $C_1$ -$C_3$ alkyl, and hydroxyl; preferably, $R^1$ is selected from H, F, Cl, Br, I, $C_1$ -$C_3$ alkyl, and hydroxyl; and/or

$R^2$ is selected from H, and $C_1$ -$C_3$ alkyl; and/or

n is 0 or 1.

**4.** The compound according to any one of claims 1-3, wherein the compound is selected from the following structures:

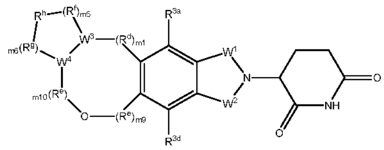
,
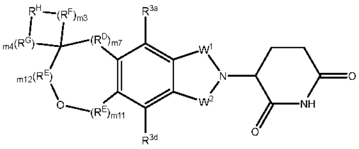
,
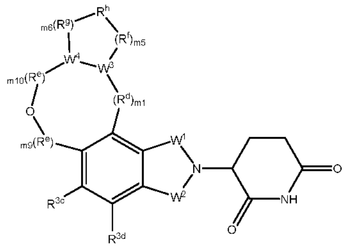
,
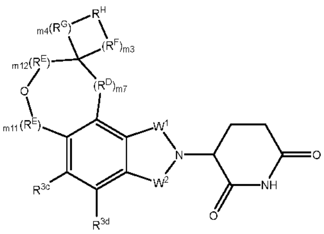
,
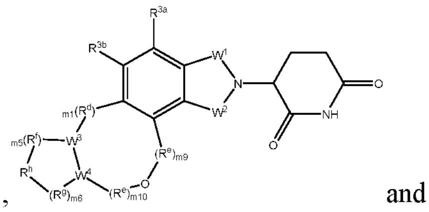
and
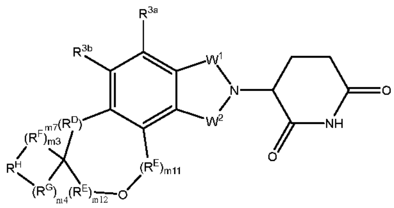

wherein:

$W^1$, $W^2$, $W^3$, $W^4$, $R^{3a}$, $R^{3b}$, $R^{3c}$, $R^{3d}$, $R^d$, $R^e$, $R^f$, $R^h$, $R^g$, $R^D$, $R^E$, $R^F$, $R^H$, $R^G$, m3, m4, m5 and m6 are as defined in claims 1, 2 or 3, preferably, $R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; and

m1, m9 and m10 at each occurrence are each independently an integer of 0, 1, 2, 3, 4, or 5, and m1+m9+m10$\leq$5; preferably, m1, m9 and m10 at each occurrence are each independently an integer of 0, 1, or 2, and m1+m9+m10$\leq$2, preferably m1+m9+m10=1 or m1+m9+m10=0; and

m7, m11 and m12 at each occurrence are each independently an integer of 0, 1, 2, 3, 4, 5, or 6, and m7+m11+m12$\leq$6; preferably, m7, m11 and m 12 at each occurrence are each independently an integer of 0, 1, 2, or 3, and m7+m11+m12$\leq$3, preferably m7+m11+m12=2 or m7+m11+m12=1.

**5.** The compound according to any one of claims 1-4, wherein the compound is selected from the following structures:

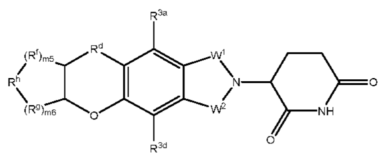
,
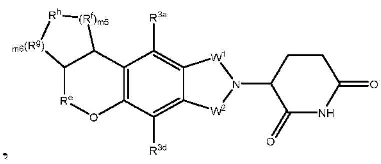
,
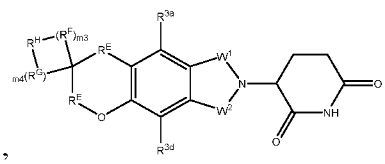
,
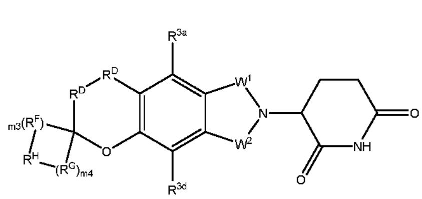
,
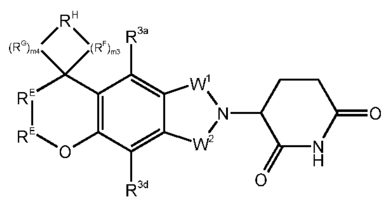
,
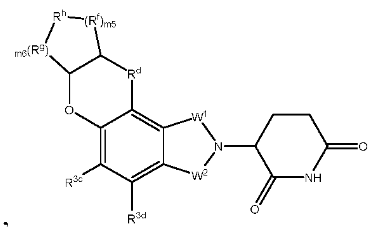
,

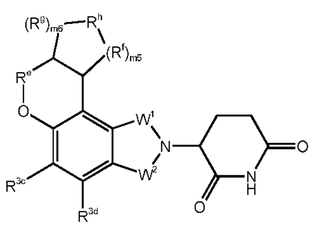,
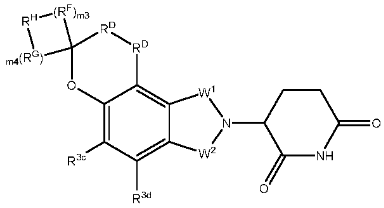,
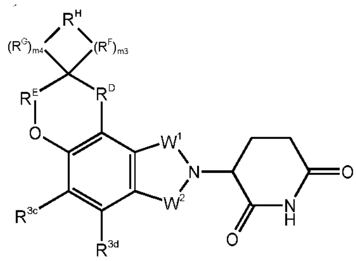,
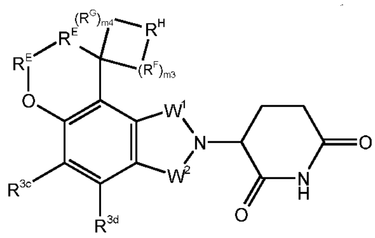,
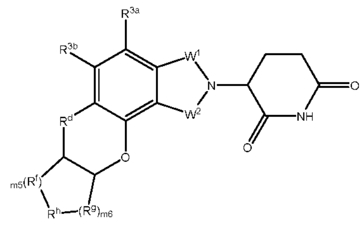,
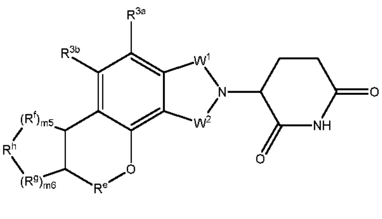,
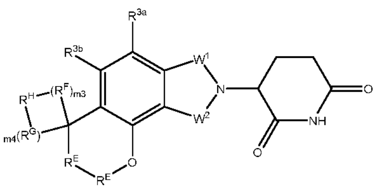,
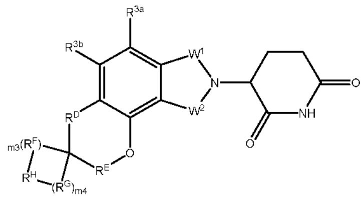,
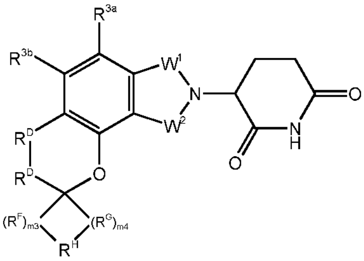,
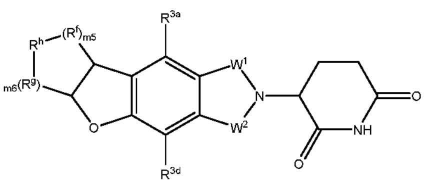,
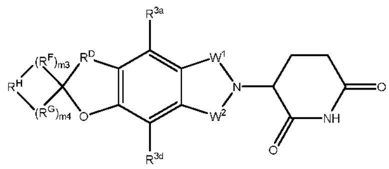,
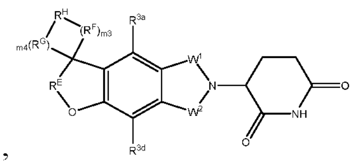,
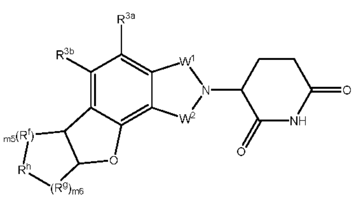,
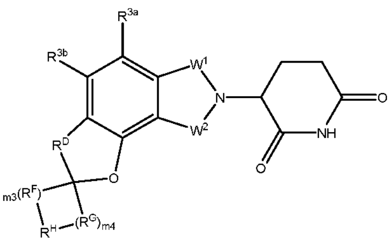,
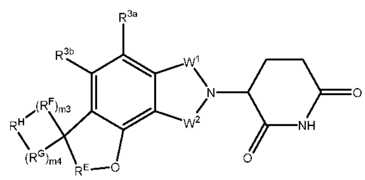,
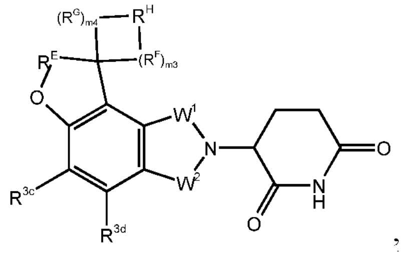,
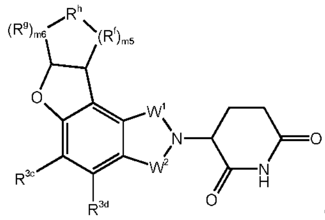
and
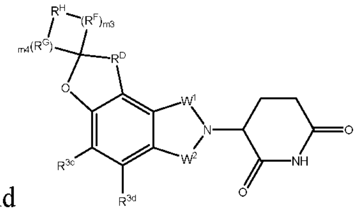;

wherein: $W^1$ , $W^2$ , $R^{3a}$ , $R^{3b}$ , $R^{3c}$ , $R^{3d}$ , $R^d$ , $R^e$ , $R^f$ , $R^h$ , $R^g$ , $R^D$ , $R^E$ , $R^F$ , $R^H$ , $R^G$ , m3, m4, m5, and m6 are as defined in claim 1, 2, or 3;
preferably:

$R^{3a}$, $R^{3b}$, $R^{3c}$, $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and the $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; and/or

m3 at each occurrence is independently an integer of 0, 1, 2, 3, or 4, and m4 is an integer of 1, 2, 3, 4, or 5, and m3+m4≤5, preferably m3+m4=2 , m3+m4=3 or m3+m4=4; and/or

m5 and m6 at each occurrence is independently an integer of 0, 1, 2, 3 or 4, and m5+m6≤4, preferably m5+m6=2 or m5+m6=3; and/or

$W^1$ and $W^2$ are identical or different, and are each independently $CH_2$ or C (=O), and at least one of $W^1$ and $W^2$ is C (=O); and/or

$R^d$, $R^e$, $R^f$, $R^g$, $R^D$, $R^E$, $R^F$, and $R^G$ at each occurrence are each independently $C(R^m)_2$ or O, $R^m$ at each occurrence is independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; and/or

$R^h$ and $R^H$ at each occurrence are each independently $NR^{1h}$, C(=O), or $CR^{2h3h}$, and $R^{1h}$, $R^{2h}$, and $R^{3h}$ are each independently selected from H, deuterium atom, F, Cl, Br, I, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkyne, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl.

**6.** The compound according to any one of claims 1-3, wherein the compound is selected from the following structures:

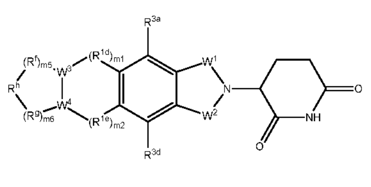,
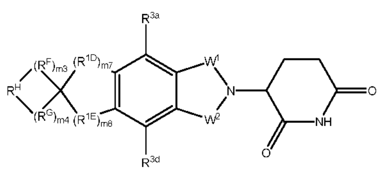,
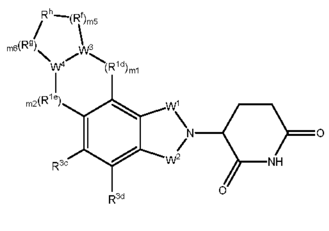,

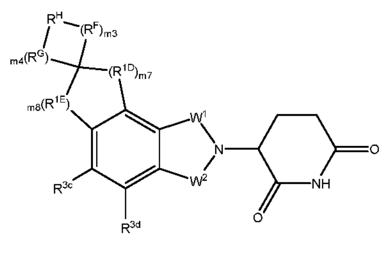,
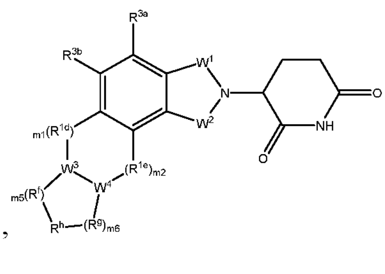,and
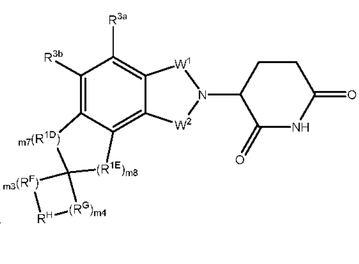,

wherein:

$W^1$, $W^2$, $W^3$, $W^4$, $R^{3a}$, $R^{3b}$, $R^{3c}$, $R^{3d}$, $R^f$, $R^h$, $R^g$, $R^F$, $R^G$, m1 , m2, m3, m4, m5, m6, m7, and m8 are as defined in claim 1, 2, or 3, and preferably, $R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl, and $C_5$-$C_{10}$ heteroaryl are each independently optionally substituted with one or more substituents selected from

F, Cl, Br, I, $C_1$-$C_6$ alkyl, $C_1$-$C_6$ heteroalkyl, $C_1$ -$C_6$ alkoxy, $C_1$ -$C_6$ haloalkyl, hydroxyl, $C_1$ -$C_6$ hydroxyalkyl, cyano, amino, nitro, $C_3$ -$C_8$ cycloalkyl, $C_4$ -$C_{10}$ heterocyclyl, $C_6$ -$C_{10}$ aryl, and $C_5$ -$C_{10}$ heteroaryl;

$R^{1d}$ , $R^{1e}$ , $R^{1D}$ and $R^{1E}$ at each occurrence are independently $C(R^m)_2$;
$R^H$ is $NR^{1h}$, C(=O), or $CR^{2h}R^{3h}$, and when $R^F$ and $R^G$ are both $C(R^m)_2$, $R^H$ is C(=O), or $CR^{2h}R^{3h}$; and

$R^{1h}$ , $R^{2h}$ , $R^{3h}$ , and $R^m$ are as defined in claims 1, 2 or 3.

**7.** The compound according to any one of claims 1-3 or 6, wherein the compound is selected from the following structures:

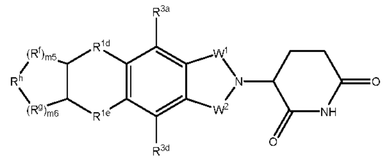,
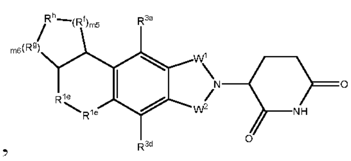,
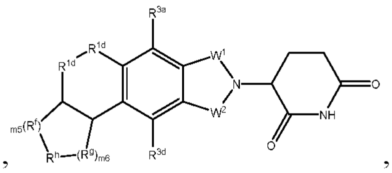,
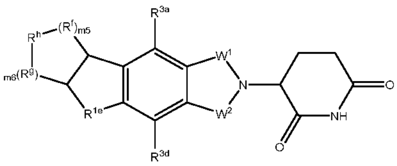,
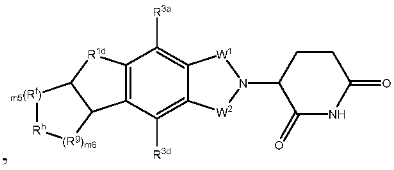,
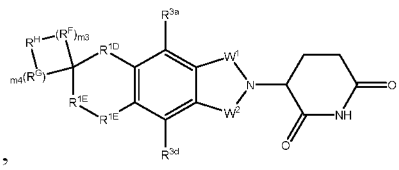,
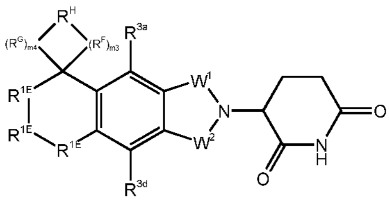,
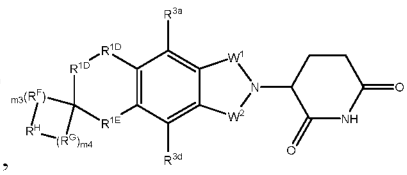,
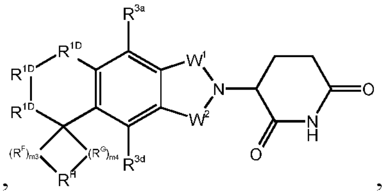,
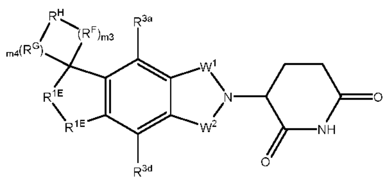,
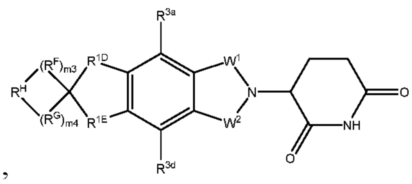,
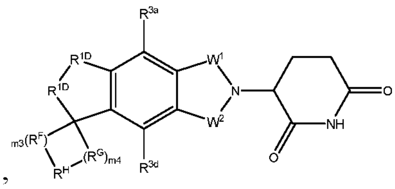,
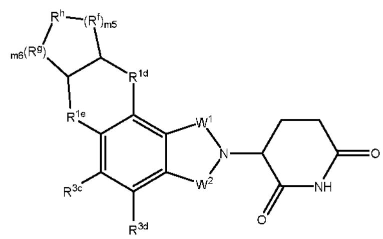,
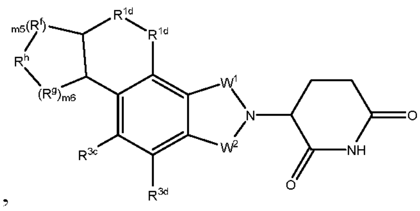,
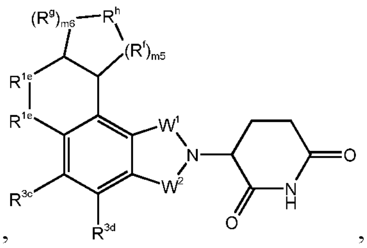,
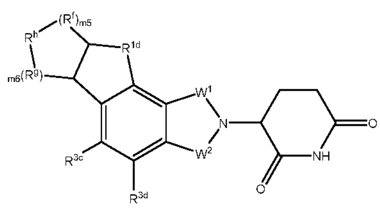,
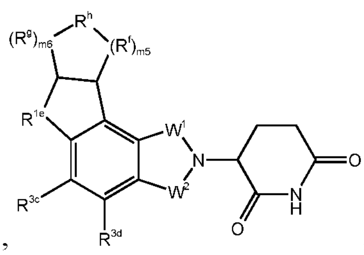,
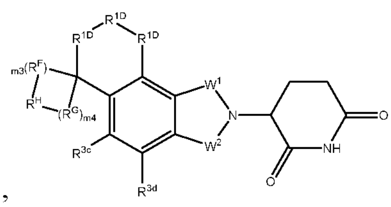,

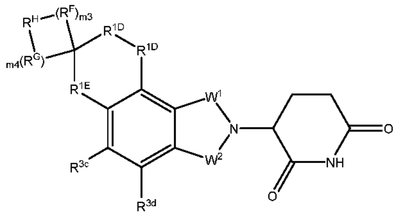, 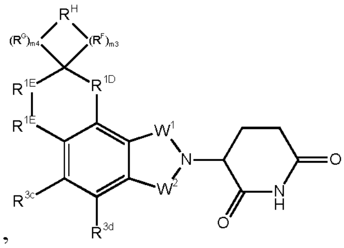, 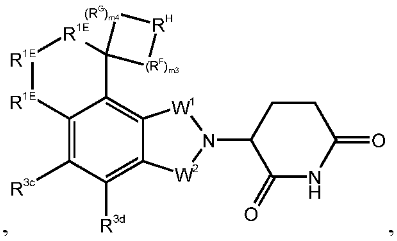,

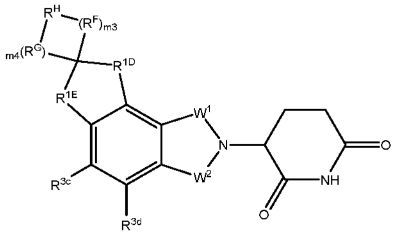, 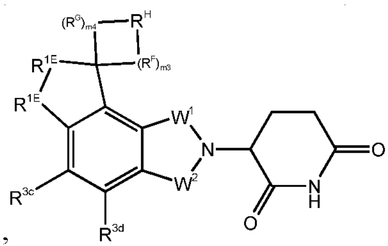, 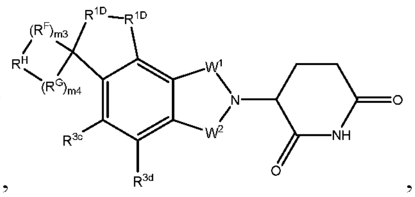,

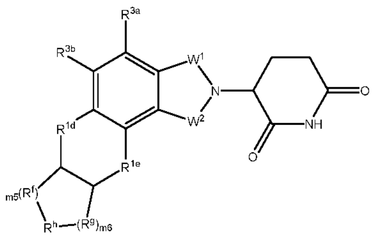, 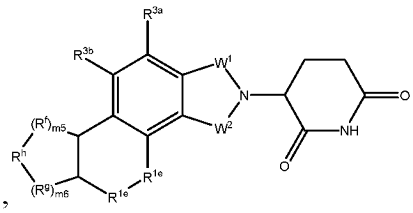, 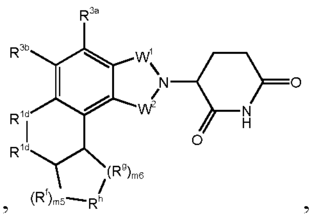,

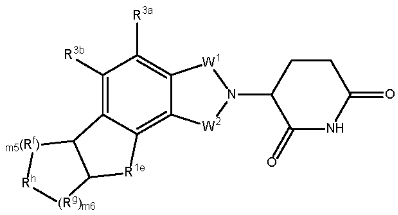, 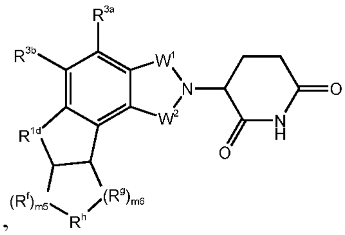, 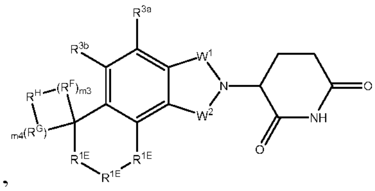,

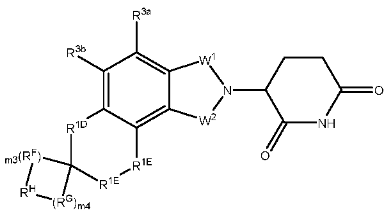, 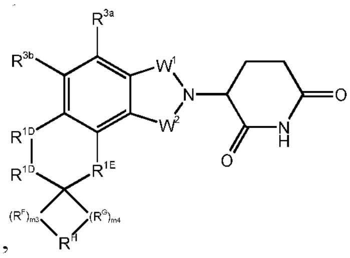, 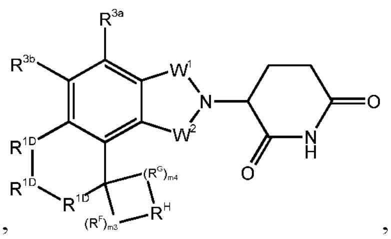,

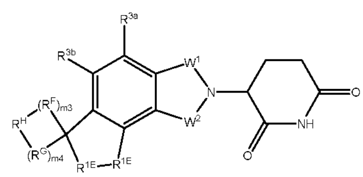, 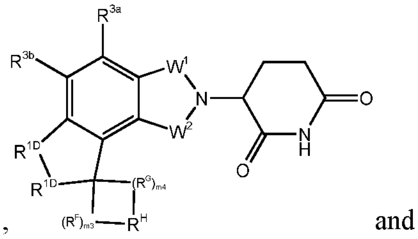 and 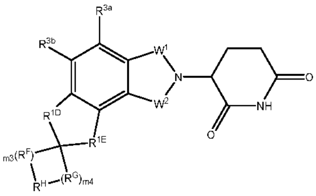;

wherein, $W^1$, $W^2$, $R^{3a}$, $R^{3b}$, $R^{3c}$, $R^{3d}$, $R^f$, Rh, $R^g$, $R^F$, $R^G$, m3, m4, m5, and m6 are as defined in claim 1, 2 or 3; $R^{1d}$, $R^{1e}$, $R^{1D}$, and $R^{1E}$ at each occurrence are each independently $C(R^m)_2$; $R^H$ is $NR^{1h}$, C(=O), or $CR^{2h}R^{3h}$, and when $R^F$ and $R^G$ are both $C(R^m)_2$, $R^H$ is C(=O), or $CR^{2h}R^{3h}$; and,

$R^{1h}$, $R^{2h}$, $R^{3h}$, and $R^m$ are as defined in claim1, 2 or 3;

preferably,

$R^{3a}$, $R^{3b}$, $R^{3c}$, $R^{3d}$ are each independently selected from H, deuterium atom, F, Cl, Br, I, $C_1$ -$C_6$ alkyl, $C_1$ -$C_6$ heteroalkyl, $C_2$ -$C_6$ alkenyl, $C_2$ -$C_6$ alkynyl, $C_1$ -$C_6$ alkoxy, hydroxyl, nitro, cyano, amino, $C_3$-$C_8$ cycloalkyl, $C_4$-$C_{10}$ heterocyclyl, $C_6$ -$C_{10}$ aryl and $C_5$ -$C_{10}$ heteroaryl, wherein the $C_1$ -$C_6$ alkyl, $C_1$ -$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from the group consisting of F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$ -$C_6$ hydroxyalkyl, cyano, amino, nitro, $C_3$ -$C_8$ cycloalkyl, $C_4$ -$C_{10}$ heterocyclyl, $C_6$-$C_{10}$ aryl and $C_5$-$C_{10}$ heteroaryl; and/or

m3 at each occurrence is each independently an integer of 0, 1, 2, 3, or 4, and m4 is an integer of 1, 2, 3, 4, or 5, and $m3+m4 \leq 5$, preferably m3+m4=2 , m3+m4=3 or m3+m4=4; and/or

m5 and m6 at each occurrence are each independently an integer of 0, 1, 2, 3 or 4, and $m5+m6 \leq 4$, preferably m5+m6=2 or m5+m6=3; and/or

$W^1$ and $W^2$ are identical or different, and they are each independently $CH_2$ or C(=O), and at least one of $W^1$ and $W^2$ is C(=O); and/or

$R^{1d}$, $R^{1e}$ , $R^{1D}$ , $R^{1E}$ , $R^f$ , $R^g$ , $R^F$ , and $R^G$ at each occurrence are each independently $C(R^m)_2$ , and $R^m$ at each occurrence are each independently selected from H, deuterium atom, F, Cl, Br, I, $C_1$ -$C_6$ alkyl, $C_1$ - $C_6$ heteroalkyl, $C_2$ -$C_6$ alkenyl, $C_2$ -$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$ -$C_6$ alkyl, $C_1$ -$C_6$ heteroalkyl, $C_2$ -$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently substituted with one or more substituents selected from F, Cl, Br, I, $C_1$ -$C_6$ alkyl, $C_1$ -$C_6$ heteroalkyl, $C_1$ -$C_6$ alkoxy, $C_1$ -$C_6$ haloalkyl, hydroxyl, $C_1$ -$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; and/or

$R^h$ at each occurrence is independently $NR^{1h}$, C(=O), or $CR^{2h}R^{3h}$;

$R^H$ at each occurrence is independently C(=O), or $CR^{2h}R^{3h}$, and $R^{1h}$, $R^{2h}$, and $R^{3h}$ at each occurrence are each independently selected from H, deuterium atom, F, Cl, Br, I, $C_1$ -$C_6$ alkyl, $C_1$ -$C_6$ deuterated alkyl, $C_1$-$C_6$ heteroalkyl, $C_2$-$C_6$ alkenyl, $C_2$-$C_6$ alkynyl, $C_1$ -$C_6$ alkoxy, hydroxyl, nitro, cyano, amino, $C_3$-$C_8$ cycloalkyl, $C_4$ -$C_{10}$ heterocyclyl, $C_6$ -$C_{10}$ aryl, and $C_5$ -$C_{10}$ heteroaryl, wherein the $C_1$ -$C_6$ alkyl, $C_1$ -$C_6$ heteroalkyl, $C_2$ -$C_6$ alkenyl, $C_2$ -$C_6$ alkynyl, $C_1$ -$C_6$ alkoxyl, $C_3$ -$C_8$ cycloalkyl, $C_4$ -$C_{10}$ heterocyclyl, $C_6$ -$C_{10}$ aryl, and $C_5$ -$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl , cyano, amino, nitro, $C_3$-$C_8$ cycloalkyl, $C_4$ -$C_{10}$ heterocyclyl, $C_6$ -$C_{10}$ aryl, and $C_5$ -$C_{10}$ heteroaryl.

**8.** The compound according to any one of claims 1-3, wherein the compound is selected from the following structures:

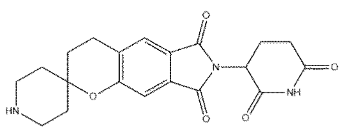,
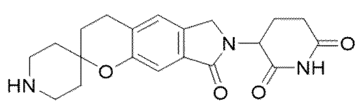,
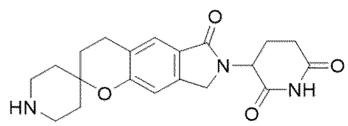,

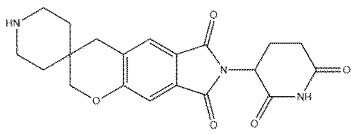,
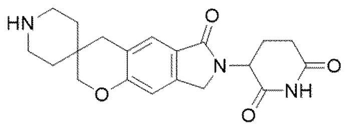,
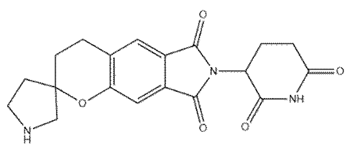,

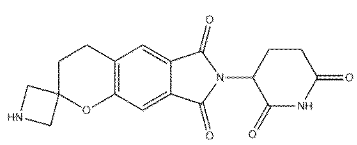,
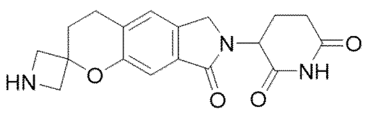,
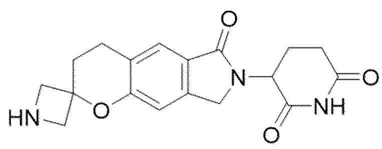,

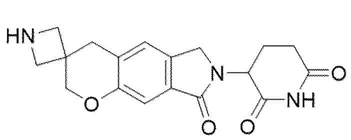, 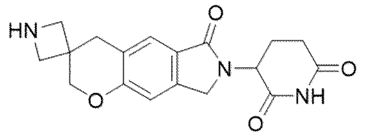, 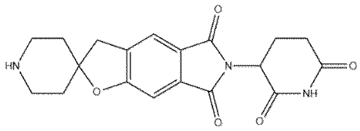,

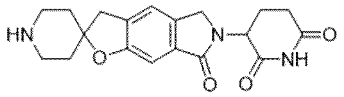, 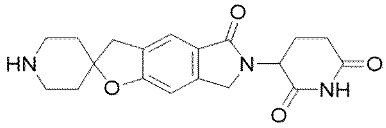, 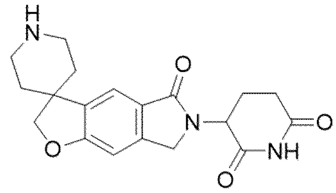,

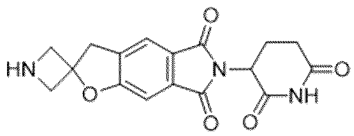, 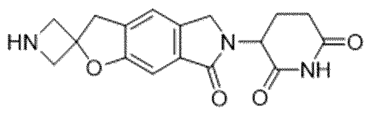, 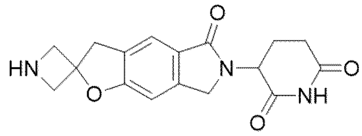,

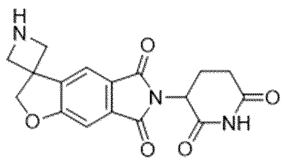, 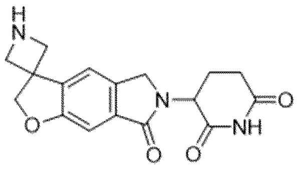, 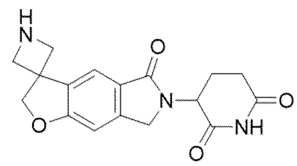,

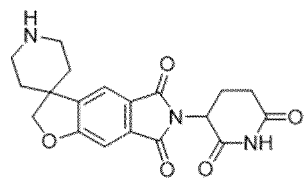, 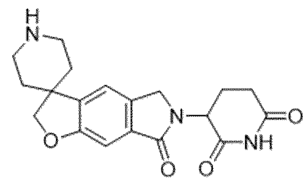, 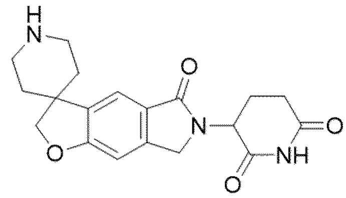,

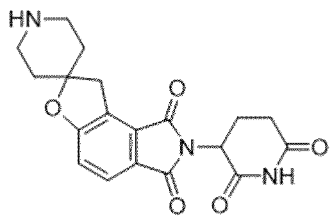, 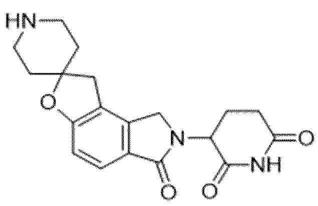, 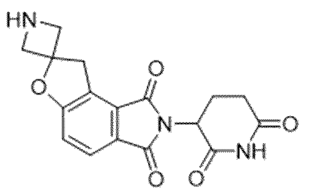,

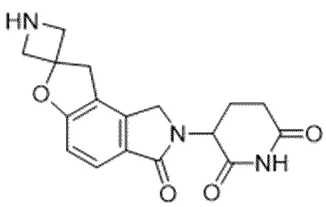, 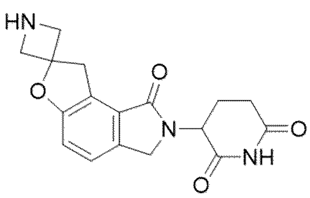, 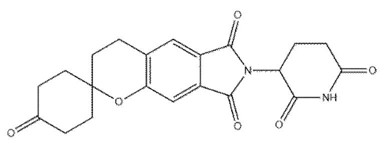,

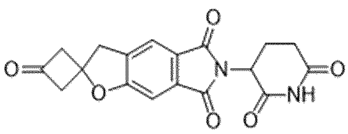 , 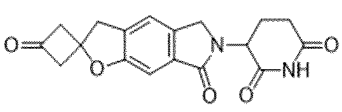 , 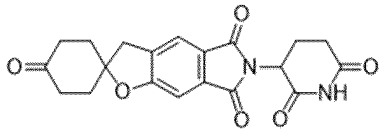 ,

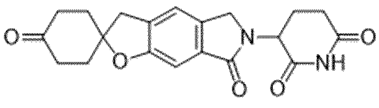 , 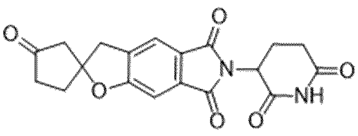 , 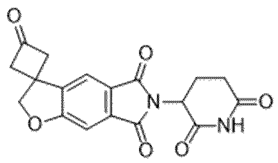 ,

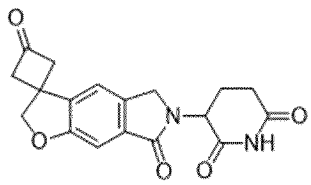 , 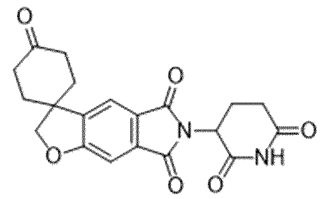 , 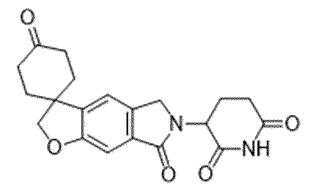 ,

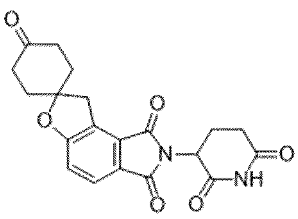 , 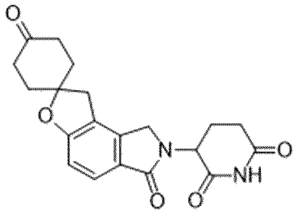 , 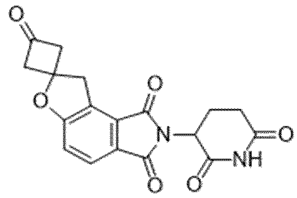 ,

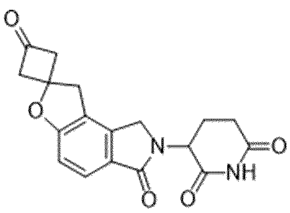 , 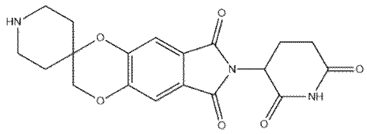 , 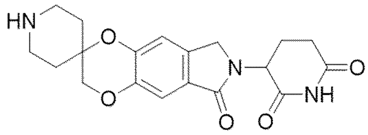 ,

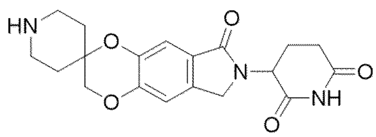 , 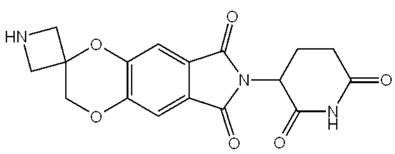 , 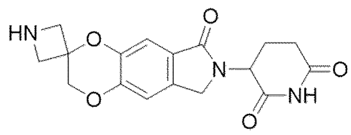 ,

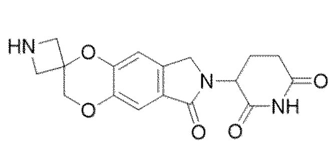 , 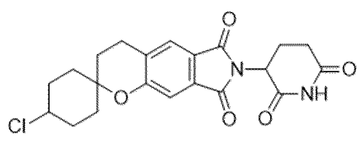 , 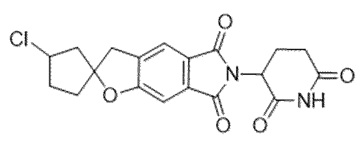 ,

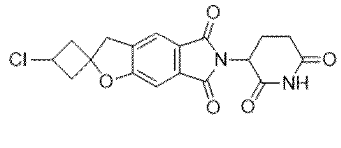 , 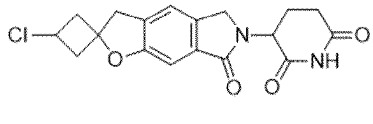 , 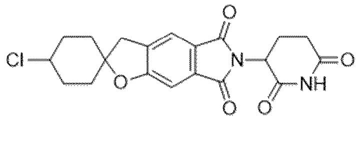 ,

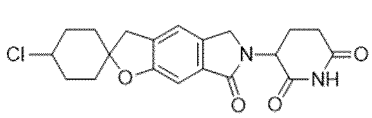,
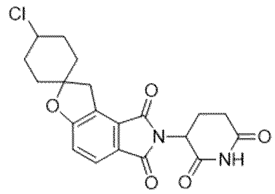,
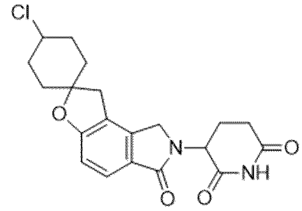,

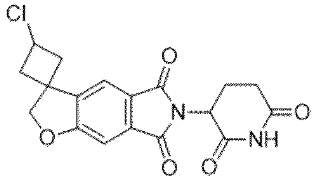,
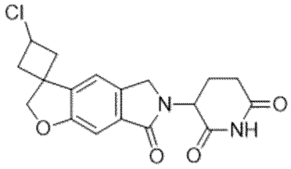,
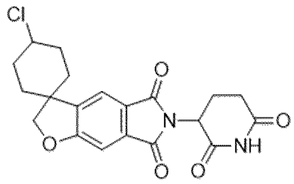,

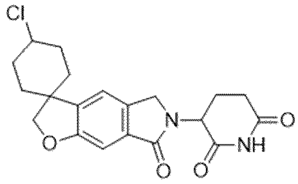,
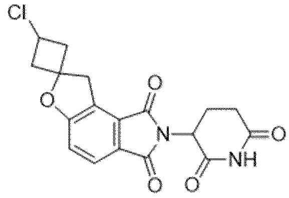,
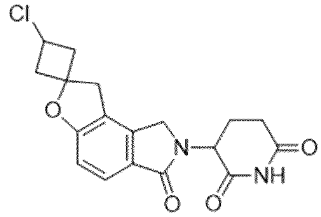,

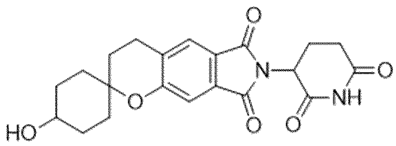,
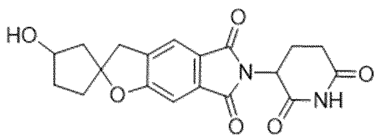,
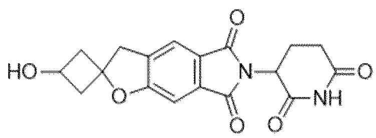,

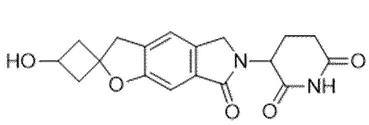,
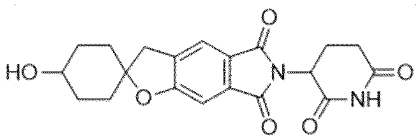,
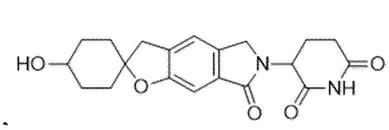,

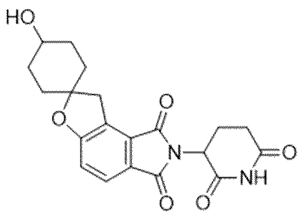,
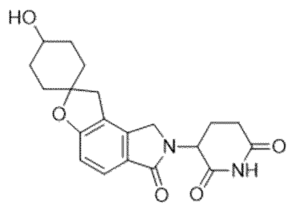,
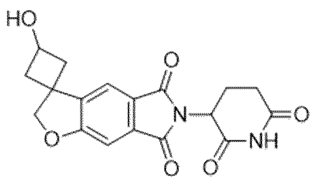,

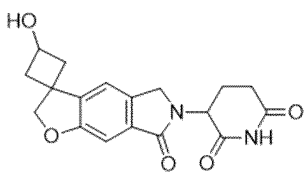,
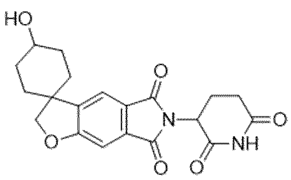,
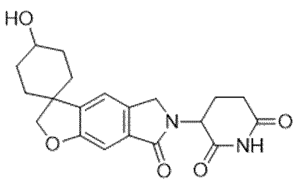,

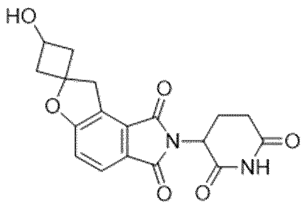 ,
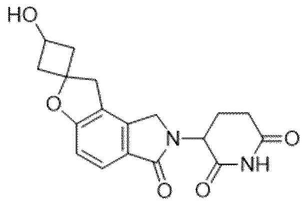 ,
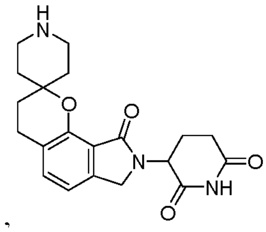 ,
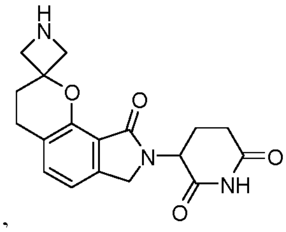 ,

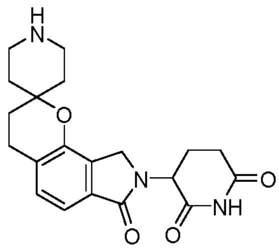 ,
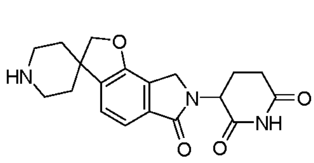 ,
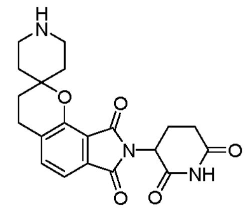 ,
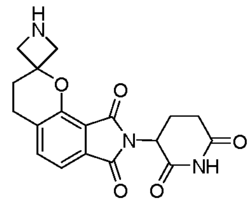 ,

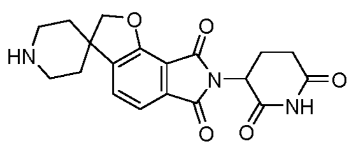 ,
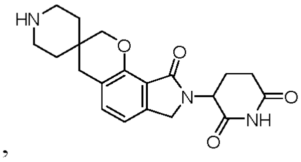 ,
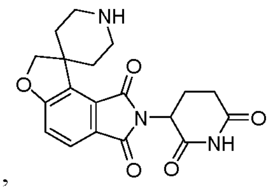 ,
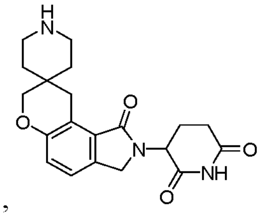 ,

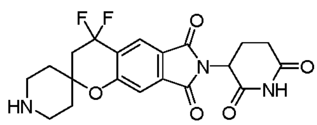 ,
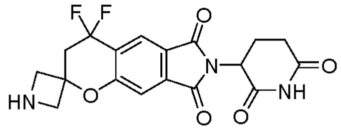 ,
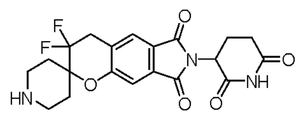 ,

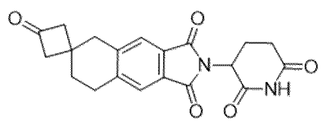 ,
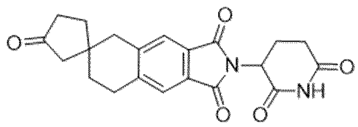 ,
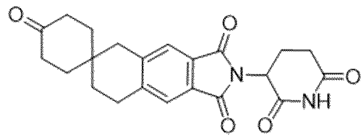 ,

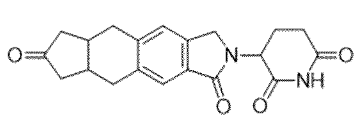 ,
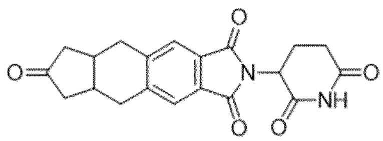 ,
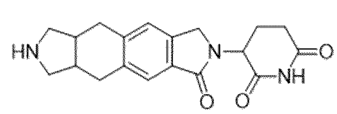 ,

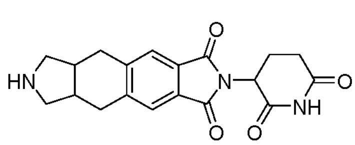 ,
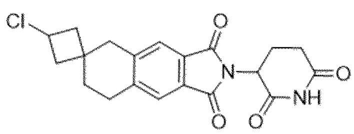 ,
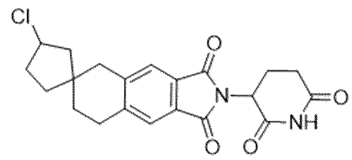 ,

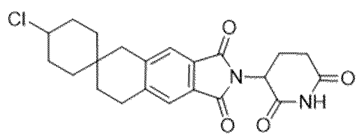 ,
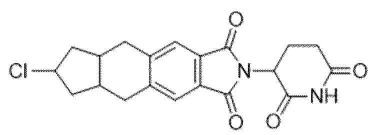 ,
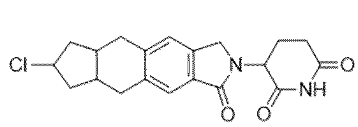 ,

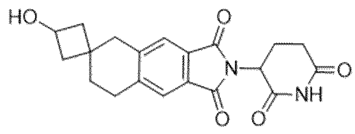 , 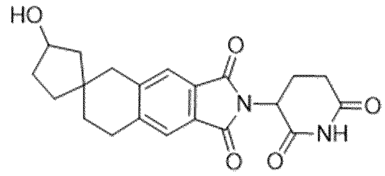 , 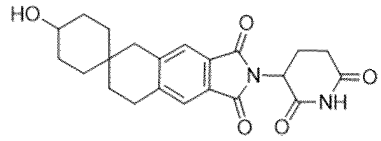 ,

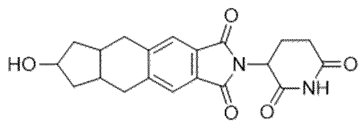 , 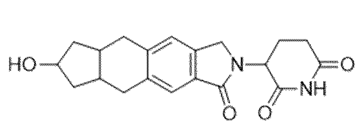 , 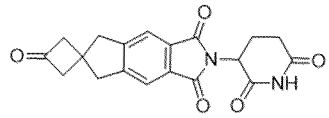 ,

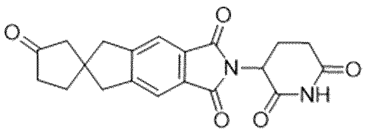 , 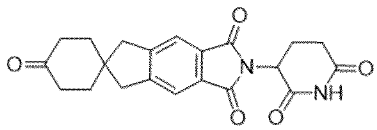 , 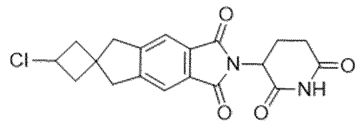 ,

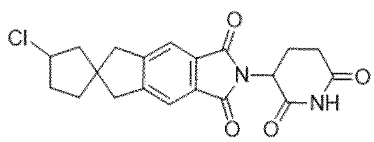 , 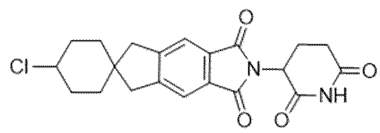 , 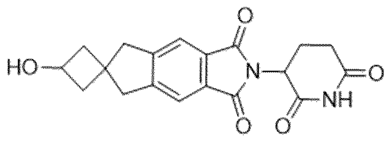 ,

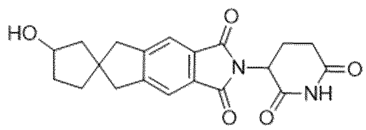 , 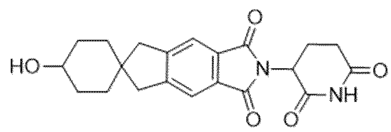 , 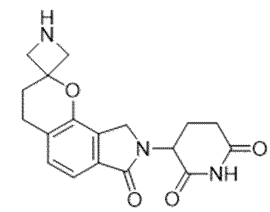 ,

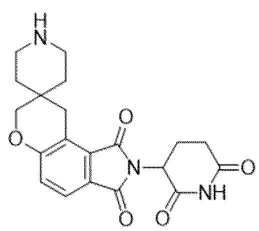 , 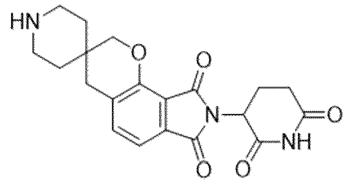 , 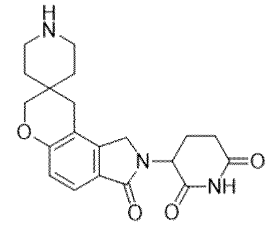 ,

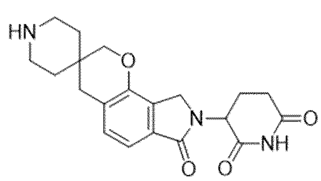 , 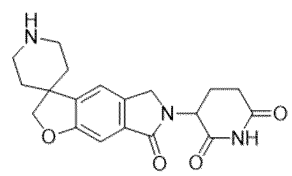 , 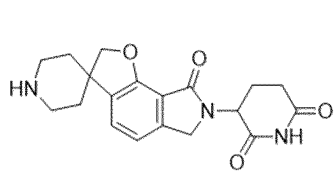 ,

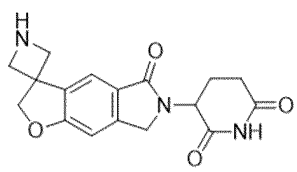 , 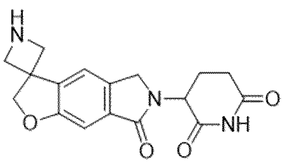 , 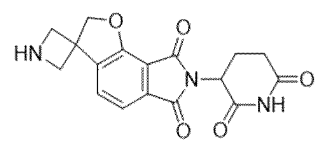 ,

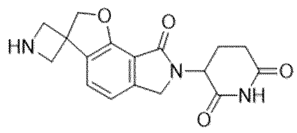,
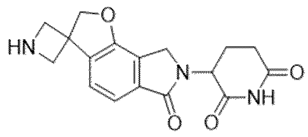,
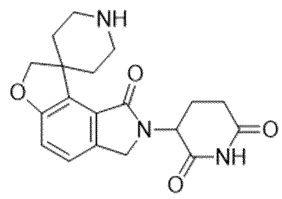,

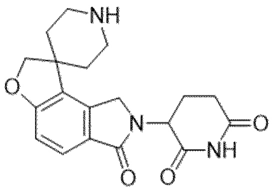,
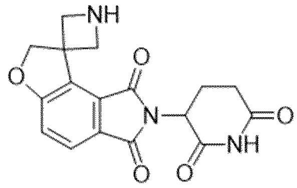,
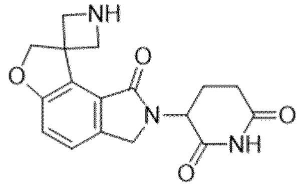,

or

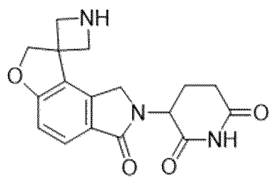.

**9.** A compound, wherein the compound is a compound having the following chemical structure: CLM-L-PTM,

or an isomer, an isotopic derivative, a polymorph, a prodrug thereof, or a pharmaceutically acceptable salt or a solvate thereof,
wherein:

PTM is a target protein binding moiety;
L is a bond or chemical linker moiety covalently connecting the CLM and the PTM, and
CLM is a cereblon E3 ubiquitin ligase binding moiety selected from the following structures:

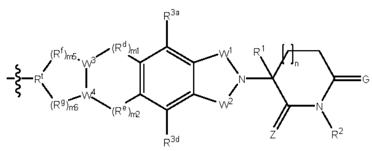,
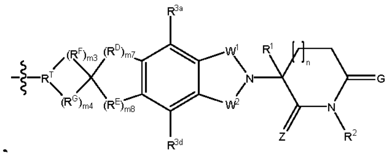,
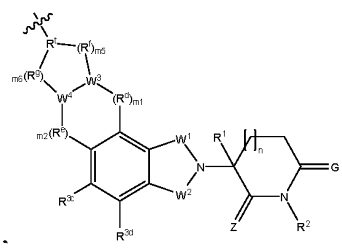,

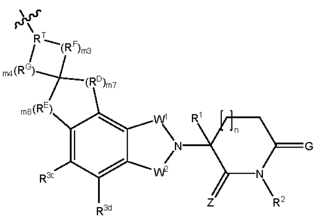,
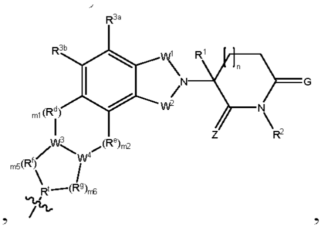, and
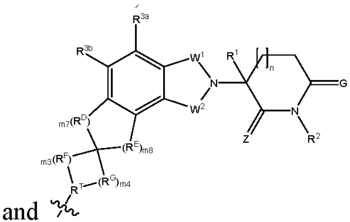;

wherein:

$W^1$ and $W^2$ are identical or different, and are each independently $CR^aR^b$ or C(=O), and at least one of $W^1$ and $W^2$ is C(=O);
G and Z are identical or different, and are each independently selected from O, S, and Se;

$R^{3a}$ , $R^{3b}$ , $R^{3c}$ , and $R^{3d}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkyloxyacyl, alkylaminoacyl, cycloalkyl, heterocyclyl, aryl and heteroaryl, wherein the alkyl, heteroalkyl, alkenyl, alkynyl, alkoxy, cycloalkyl, heterocyclyl, aryl and heteroaryl are each independently optionally substituted with one or more substituents selected from the group consisting of halogen, alkyl, heteroalkyl, alkoxyl, haloalkyl, hydroxyl, hydroxyalkyl, cyano, amino, nitro, cycloalkyl, heterocyclyl, alkenyl, alkynyl, alkylamino, alkylacyl, alkyloxyacyl, alkylaminoacyl, aryl and heteroaryl;

$R^d$ , $R^e$ , $R^f$, $R^g$ , $R^D$ , $R^E$ , $R^F$ , and $R^G$ at each occurrence are each independently $C(R^m)_2$, $NR^m$, C(=O), O, or S;
$W^3$ and $W^4$ at each occurrence are each independently $CR^m$ or N;

$R^t$ and $R^T$ at each occurrence are each independently N or $CR^{2h}$, and when $R^D$, $R^E$, $R^F$, and $R^G$ are all $C(R^m)_2$, $R^T$ is $CR^{2h}$;

m1 and m2 at each occurrence are each independently an integer of 0, 1, 2, 3, 4, 5, or 6, and m1+m2≤6;
m3 at each occurrence is each independently an integer of 0, 1, 2, 3, 4, 5, 6, or 7, m4 is an integer of 1, 2, 3, 4, 5, 6, 7, or 8, and m3+m4≤8;
m5 and m6 at each occurrence are each independently an integer of 0, 1, 2, 3, 4, 5, 6, or 7, and m5+m6≤7;
m7 and m8 at each occurrence are each independently an integer of 0, 1, 2, 3, 4, 5, 6, or 7, and m7+m8<7;
$R^m$ at each occurrence is each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, alkylamino, alkylacyl, alkyloxyacyl, alkylaminoacyl, cycloalkyl, heterocyclyl, aryl and heteroaryl, wherein the alkyl , heteroalkyl, alkenyl, alkynyl, alkoxy, cycloalkyl, heterocyclyl, aryl and heteroaryl are each independently optionally substituted with one or more substituents selected from the group consisting of halogen, alkyl, heteroalkyl, alkoxy, haloalkyl, hydroxyl, hydroxyalkyl, cyano, amino, nitro, cycloalkyl, heterocyclyl, alkenyl, alkynyl, alkylamino, alkylacyl, alkyloxyacyl, alkylaminoacyl, aryl and heteroaryl;

$R^{2h}$ is selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano , amino, alkylamino, alkylacyl, alkyloxyacyl, alkylaminoacyl, cycloalkyl, heterocyclyl, aryl, heteroaryl, alkenyl and alkynyl, wherein the alkyl, heteroalkyl, alkoxy, cycloalkyl, heterocyclyl, aryl, and heteroaryl are each independently optionally substituted with one or more substituents selected from the group consisting of halogen, alkyl, heteroalkyl, alkenyl, alkynyl, alkoxy, haloalkyl, hydroxyl, hydroxyalkyl, cyano, amino, nitro, cycloalkyl, heterocyclyl, alkylamino, alkylacyl, alkyloxyacyl, alkylaminoacyl, aryl, and heteroaryl;
$R^1$ is selected from H, halogen, deuterium atom, $C_1$ -$C_6$ alkyl, $C_1$ -$C_6$ alkoxy, hydroxyl, cycloalkyl, $C_1$-$C_6$ haloalkyl, and hydroxyalkyl;
$R^2$, $R^a$, and $R^b$ are each independently selected from H, $C_1$ -$C_3$ alkyl, $C_3$ -$C_6$ cycloalkyl, and $C_1$ -$C_6$ alkoxy; and
n is 0, 1, 2 or 3.

**10.** A compound, wherein the compound is a compound having the following chemical structure: CLM-L-PTM,

or an isomer, an isotopic derivative, a polymorph, a prodrug thereof, or a pharmaceutically acceptable salt or a solvate thereof,
wherein:

PTM is a target protein binding moiety;
L is a bond or chemical linker moiety covalently connecting the CLM and PTM, and
CLM is a cereblon E3 ubiquitin ligase binding moiety selected from the following structures:

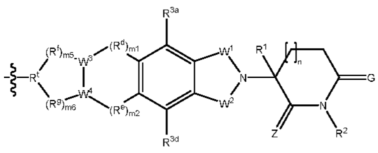,
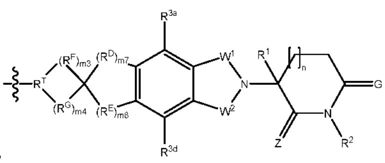,
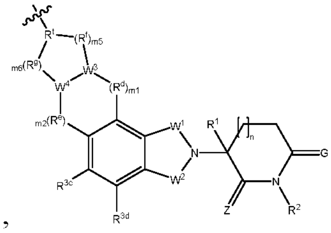,

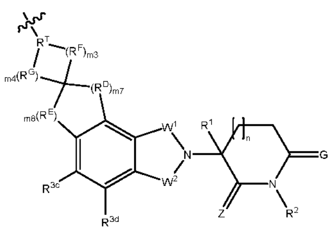,
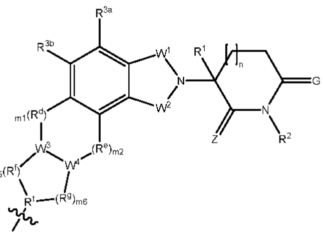, and
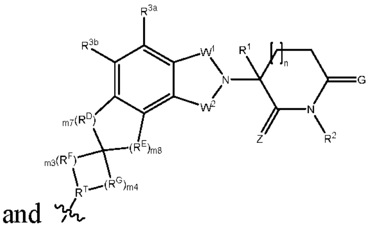;

wherein:

$W^1$ and $W^2$ are identical or different, and are each independently $CR^aR^b$ or C(=O), and at least one of $W^1$ and $W^2$ is C(=O);

G and Z are identical or different, and are each independently selected from O, S, and Se;

$R^{3a}$, $R^{3b}$, $R^{3c}$, and $R^{3d}$ are each independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, cycloalkyl, heterocyclyl, aryl and heteroaryl, wherein the alkyl, heteroalkyl, alkenyl, alkynyl, alkoxy, cycloalkyl, heterocyclyl, aryl and heteroaryl are each independently optionally substituted with one or more substituents selected from the group consisting of halogen, alkyl, heteroalkyl, alkoxy, haloalkyl, hydroxyl, hydroxyalkyl, cyano, amino, nitro , cycloalkyl, heterocyclyl, aryl and heteroaryl;

$R^d$, $R^e$, $R^f$, $R^g$, $R^D$, $R^E$, $R^F$, and $R^G$ at each occurrence are each independently $C(R^m)_2$, $NR^m$, O, or S;

$W^3$ and $W^4$ at each occurrence are each independently CH or N;

$R^t$ and $R^T$ at each occurrence are each independently N or $CR^{2h}$, and, when $R^D$, $R^E$, $R^F$, and $R^G$ are all $C(R)^m{}_2$ , $R^T$ is $CR^{2h}$;

m1 and m2 at each occurrence are each independently an integer of 0, 1, 2, 3, 4, 5, or 6, and m1+m2≤6;

m3 at each occurrence is independently an integer of 0, 1, 2, 3, 4, 5, 6, or 7, and m4 is an integer of 1, 2, 3, 4, 5, 6, 7, or 8, and m3+m4≤8;

m5 and m6 at each occurrence are each independently an integer of 0, 1, 2, 3, 4, 5, 6, or 7, and m5+m6≤7;

m7 and m8 at each occurrence are each independently an integer of 0, 1, 2, 3, 4, 5, 6, or 7, and m7+m8≤7;

$R^m$ at each occurrence is independently selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, cycloalkyl, heterocyclyl, aryl and heteroaryl, wherein the alkyl, heteroalkyl, alkenyl, alkynyl, alkoxy, cycloalkyl, heterocyclyl, aryl and heteroaryl are each independently optionally substituted with one or more substituents selected from the group consisting of halogen, alkyl, heteroalkyl, alkoxy, haloalkyl, hydroxyl, hydroxyalkyl, cyano, amino, nitro, cycloalkyl, heterocyclyl, aryl and heteroaryl;

$R^{2h}$ is selected from H, deuterium atom, halogen, alkyl, deuterated alkyl, heteroalkyl, alkenyl, alkynyl, alkoxy, haloalkyl, haloalkoxy, hydroxyl, hydroxyalkyl, nitro, cyano, amino, cycloalkyl, heterocyclyl, aryl, heteroaryl, alkenyl and alkynyl, wherein the alkyl, heteroalkyl, alkoxy, cycloalkyl, heterocyclyl, aryl, and heteroaryl are each independently optionally substituted with one or more substituents selected from the group consisting of halogen, alkyl, heteroalkyl, alkenyl, alkynyl, alkoxy, haloalkyl, hydroxyl, hydroxyalkyl, cyano, amino, nitro, cycloalkyl, heterocyclyl, aryl and heteroaryl;

$R^1$ is selected from H, halogen, $C_1$-$C_3$ alkyl, $C_1$ -$C_3$ alkoxy, hydroxyl, and hydroxyalkyl;

$R^2$, $R^a$, and $R^b$ are each independently selected from H, $C_1$ -$C_3$ alkyl, and $C_1$ -$C_3$ alkoxy; and

n is 0, 1, or 2.

**11.** The compound according to claims 9 or 10, wherein:

$W^1$ and $W^2$ are identical or different, and are each independently $CH_2$ or C(=O), and at least one of $W^1$ and $W^2$ is

C(=O); and/or

$R^{3a}$, $R^{3b}$ , $R^{3c}$ , and $R^{3d}$ are each independently selected from H, deuterium atom, halogen, $C_1$ -$C_6$ alkyl, heteroalkyl, $C_2$ -$C_6$ alkenyl, $C_2$ -$C_6$ alkynyl, $C_1$ -$C_6$ alkoxy, $C_1$ -$C_6$ haloalkyl, $C_1$-$C_6$ haloalkoxy, hydroxyl, $C_1$ -$C_6$ hydroxyalkyl, nitro, cyano, amino, $C_3$ -$C_8$ cycloalkyl, $C_4$ -$C_{10}$ heterocyclyl, $C_6$ -$C_{10}$ aryl, and $C_5$ -$C_{10}$ heteroaryl, wherein the $C_1$-$C_6$ alkyl, $C_1$-$C_6$ heteroalkyl, $C_2$ -$C_6$ alkenyl, $C_2$ -$C_6$ alkynyl, $C_1$ -$C_6$ alkoxy, $C_3$ -$C_8$ cycloalkyl, $C_4$ -$C_{10}$ heterocyclyl, $C_6$ -$C_{10}$ aryl and $C_5$ -$C_{10}$ heteroaryl are each independently optionally substituted with one or more substituents selected from the group consisting of halogen, $C_1$ -$C_6$ alkyl, $C_1$ -$C_6$ heteroalkyl, $C_1$ -$C_6$ alkoxy, $C_1$ -$C_6$ haloalkyl, hydroxyl, $C_1$ -$C_6$ hydroxyalkyl, cyano, amino, nitro, $C_3$ -Cs cycloalkyl, $C_4$-$C_{10}$ heterocyclyl, $C_6$-$C_{10}$ aryl and $C_5$-$C_{10}$ heteroaryl; preferably $R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$ -$C_6$ alkenyl, $C_2$ -$C_6$ alkynyl, $C_1$ -$C_6$ alkyl, hydroxyl, nitro, cyano, amino, $C_3$ -$C_8$ cycloalkyl, $C_4$ -$C_{10}$ heterocyclyl, $C_6$ -$C_{10}$ aryl, and $C_5$ -$C_{10}$ heteroaryl, wherein the $C_1$-$C_6$ alkyl, $C_1$ -$C_6$ heteroalkyl, $C_2$ - $C_6$ alkenyl, $C_2$ -$C_6$ alkynyl, $C_1$ -$C_6$ alkoxy, $C_3$ -$C_8$ cycloalkyl, $C_4$ -$C_{10}$ heterocyclyl, $C_6$ -$C_{10}$ aryl and $C_5$-$C_{10}$ heteroaryl are each independently optionally substituted with one or more of the substituents selected from F, Cl, Br, I, $C_1$-$C_6$ alkyl, $C_1$-$C_6$ heteroalkyl, $C_1$-$C_6$ alkoxy, $C_1$ -$C_6$ haloalkyl, hydroxyl, $C_1$ -$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl ; preferably $R^{3a}$ and $R^{3b}$ are each independently selected from H, deuterium atom, halogen, $C_1$-$C_3$ alkyl, and $C_1$-$C_3$ alkoxy, preferably $R^{3c}$ and $R^{3d}$ are each independently selected from H, deuterium atom, F, Cl, Br, I, $C_1$-$C_3$ alkyl, and $C_1$-$C_3$ alkoxy; and/or

$R^d$, $R^e$, $R^f$ and $R^g$ at each occurrence are each independently $C(R^m)_2$ or O; and/or

$R^D$, $R^E$, $R^F$ and $R^G$ at each occurrence are each independently $C(R^m)_2$ or O; and/or

$W^3$ and $W^4$ are CH; and/or

$R^{2h}$ is selected from H, deuterium atom, halogen, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; preferably $R^{2h}$ is selected from H, deuterium atom, F, Cl, Br, I, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$- $C_{10}$ heterocyclyl, $C_6$-$C_{10}$ aryl and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; preferably $R^{2h}$ is selected from H, deuterium atom, halogen, $C_1$-$C_3$ alkyl, and $C_1$-$C_3$ alkoxy; preferably $R^{2h}$ is selected from H, deuterium atom, F, Cl , Br, I, $C_1$-$C_3$ alkyl, $C_1$-$C_3$ haloalkyl, $C_1$-$C_3$ alkoxy, and $C_1$-$C_3$ haloalkoxy; and/or

m1 and m2 at each occurrence are each independently an integer of 0, 1, 2, or 3, and $m1+m2\leq3$, preferably m1+m2=1 or m1+m2=2; and/or

m3 at each occurrence is independently an integer of 0, 1, 2, 3, or 4, and m4 is an integer of 1, 2, 3, 4, or 5, and $m3+m4\leq5$, preferably m3+m4=2, m3+m4=3 or m3+m4=4; and/or

m5 and m6 at each occurrence are each independently an integer of 0, 1, 2, 3 or 4, and $m5+m6\leq4$, preferably m5+m6=2 or m5+m6=3; and/or

m7 and m8 at each occurrence are each independently an integer of 0, 1, 2, 3 or 4, and $m7+m8\leq4$, preferably m7+m8=2, or m7+m8=3; and/or;

$R^m$ at each occurrence is each independently selected from H, deuterium atom, halogen, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_1$-$C_6$ haloalkyl, $C_1$-$C_6$ haloalkoxy, hydroxyl, $C_1$-$C_6$ hydroxyalkyl, nitro, cyano, amino, $C_3$-$C_8$ cycloalkyl, $C_4$-$C_{10}$ heterocyclyl, aryl and heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from halogen, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; preferably, $R^m$ at each occurrence is independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$ -$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$

cycloalkyl, $C_4$-$C_{10}$ heterocyclyl, $C_6$-$C_{10}$ aryl and $C_5$-$C_{10}$ heteroaryl; Preferably $R^m$ at each occurrence is independently selected from H, deuterium atom, halogen, $C_1$-$C_3$ alkyl, and $C_1$-$C_3$ alkoxy; and/or

$R^1$ is selected from H, halogen, $C_1$-$C_3$ alkyl, and hydroxyl; $R^1$ is preferably selected from H, F, Cl, Br, I, $C_1$-$C_3$ alkyl and hydroxyl; and/or

$R^2$ is selected from H, and $C_1$-$C_3$ alkyl; and/or

n is 0 or 1.

**12.** The compound according to any one of claims 9-11, wherein the CLM is selected from the following structures:

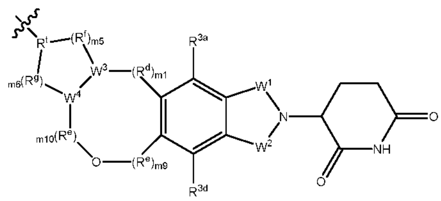,
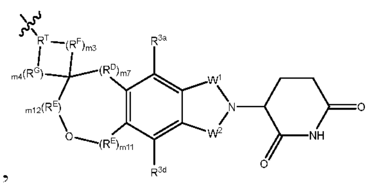,
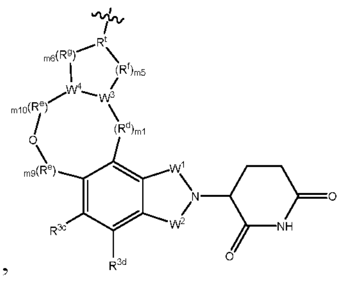,
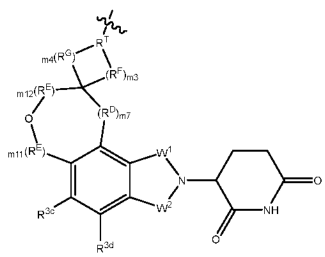,
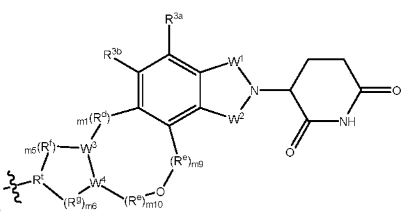, and
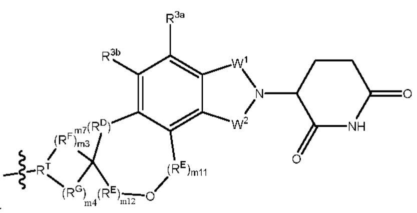

wherein:

$W^1$, $W^2$, $W^3$, $W^4$, $R^{3a}$, $R^{3b}$, $R^{3c}$, $R^{3d}$, $R^d$, $R^e$, $R^f$, $R^t$, $R^g$, $R^D$, $R^E$, $R^F$, $R^T$, $R^G$, m3, m4, m5 and m6 are as defined in claim 9, 10 or 11, preferably, $R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl;

m1, m9 and m10 at each occurrence are each independently an integer of 0, 1, 2, 3, 4, or 5, and m1+m9+m10≤5; preferably, m1, m9 and m10 at each occurrence are each independently an integer of 0, 1, or 2, and m1+m9+m10≤2, preferably m1+m9+m10=1 or m1+m9+m10=0; and

m7, m11 and m12 at each occurrence are each independently an integer of 0, 1, 2, 3, 4, 5, or 6, and m7+m11+m12≤6; preferably, m7, m11 and m12 at each occurrence are each independently an integer of 0, 1, 2, or 3, and m7+m11+m12≤3, preferably m7+m11+m12=2 or m7+m11+m12=1.

**13.** The compound according to any one of claims 9-12, wherein the CLM is selected from the following structures:

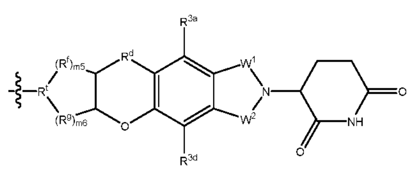,
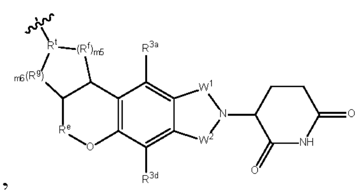,
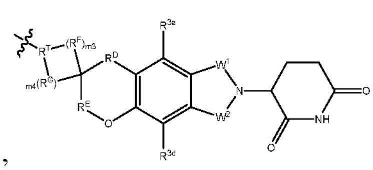,

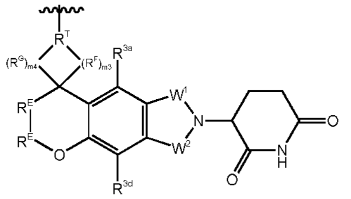, 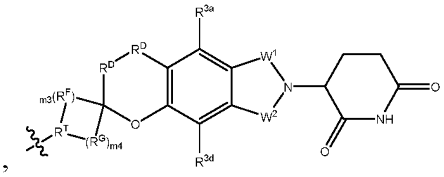, 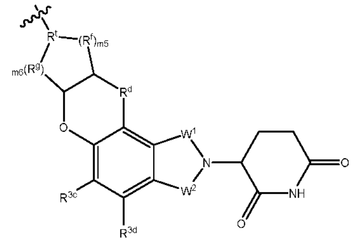,

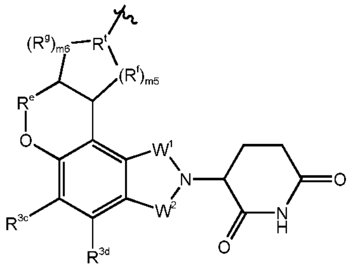, 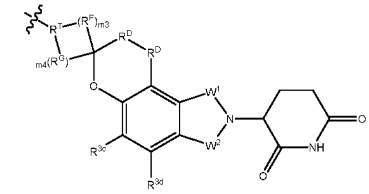, 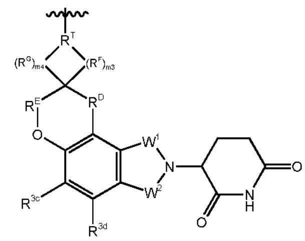,

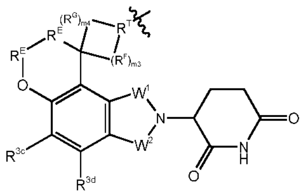, 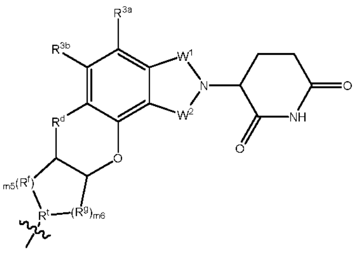, 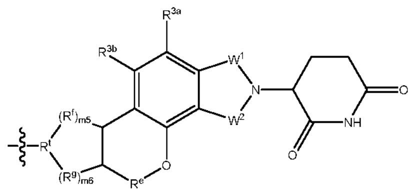,

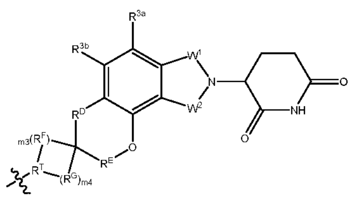, 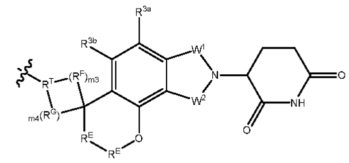, 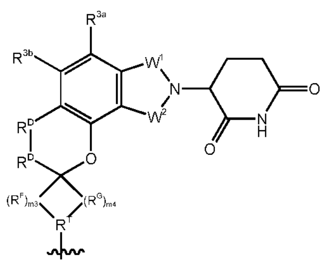,

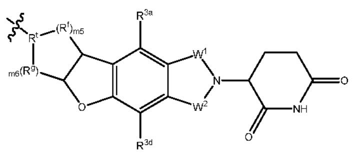, 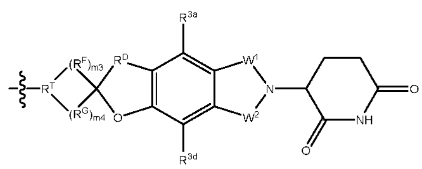, 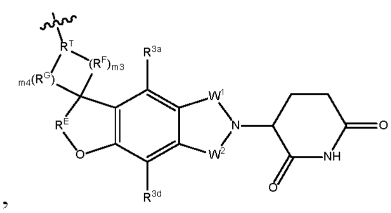,

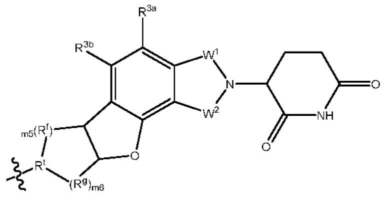, 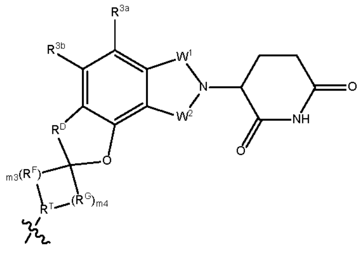, 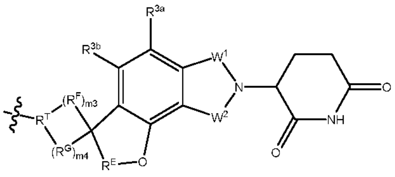,

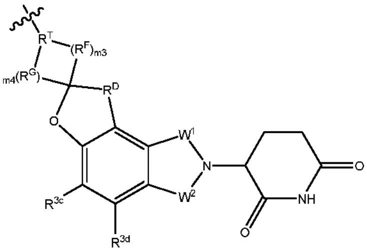
,
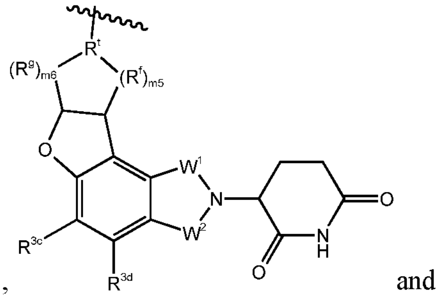
and
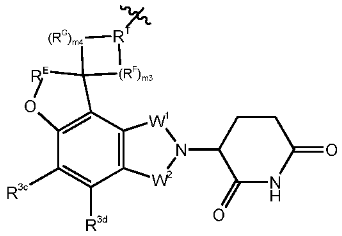

wherein:

$W^1$, $W^2$, $R^{3a}$, $R^{3b}$, $R^{3c}$, $R^{3d}$, $R^d$, $R^e$, $R^f$, $R^t$, $R^g$, $R^D$, $R^E$, $R^F$, $R^T$, $R^G$, m3, m4, m5, and m6 are as defined in claim 9, 10, or 11 ;
preferably:

$R^{3a}$, $R^{3b}$ , $R^{3c}$, $R^{3a}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$- $C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; and/or
m3 at each occurrence is independently an integer of 0, 1, 2, 3, or 4, and m4 is an integer of 1, 2, 3, 4, or 5, and m3+m4≤5, preferably m3+m4=2 , m3+m4=3 or m3+m4=4; and/or
m5 and m6 at each occurrence are each independently an integer of 0, 1, 2, 3 or 4, and m5+m6≤4, preferably m5+m6=2 or m5+m6=3; and/or
$W^1$ and $W^2$ are identical or different, and are each independently $CH_2$ or C(=O), and at least one of $W^1$ and $W^2$ is C(=O); and/or
$R^d$, $R^e$ , $R^f$ , $R^g$ , $R^D$ , $R^E$ , $R^F$ , and $R^G$ at each occurrence are each independently $C(R^m)_2$ or O, $R^m$ at each occurrence is independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more of the substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; and/or
$R^t$, and $R^T$ at each occurrence are each independently N or $CR^{2h}$, and $R^{2h}$ is selected from H, deuterium atom, F, Cl, Br, I, $C_1$-$C_6$ alkyl, $C_1$-$C_6$ deuterated alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl.

**14.** The compound according to any one of claims 9-11, wherein the CLM is selected from the following structures:

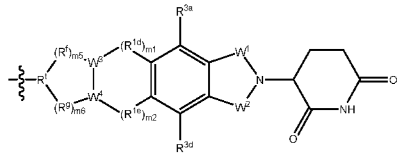
,
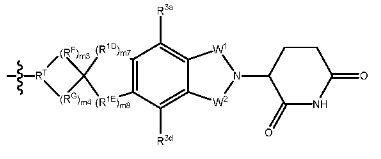
,
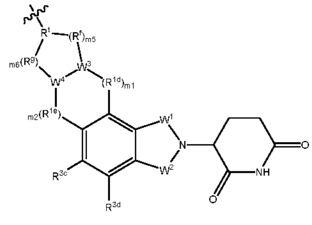
,

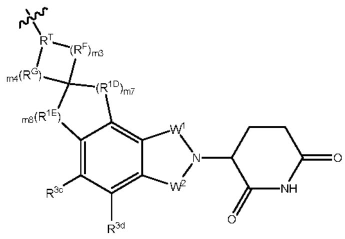, 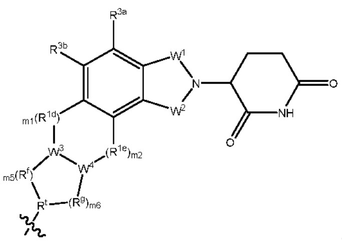, and 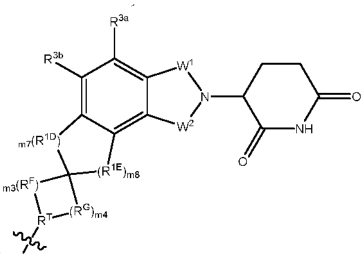

wherein:

$W^1$, $W^2$, $W^3$, $W^4$, $R^{3a}$, $R^{3b}$, $R^{3c}$, $R^{3d}$, $R^f$, $R^t$, $R^g$, $R^F$, $R^G$, m1, m2, m3, m4, m5, m6, m7, and m8 are as defined in claim 9, 10 or 11, preferably, $R^{3a}$, $R^{3b}$, $R^{3c}$, and $R^{3d}$ are each independently selected from H, deuterium atom, F, Cl, Br, I, $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl;
$R^{1d}$, $R^{1e}$, $R^{1D}$ and $R^{1E}$ at each occurrence are each independently $C(R^m)_2$;
$R^T$ is N, or $CR^{2h}$, when $R^F$ and $R^G$ are both $C(R^m)_2$, $R^T$ is $CR^{2h}$; and
$R^{2h}$, and $R^m$ are as defined in claim 9, 10 or 11.

**15.** The compound according to any one of claims 9-11 or 14, wherein the CLM is selected from the following structures:

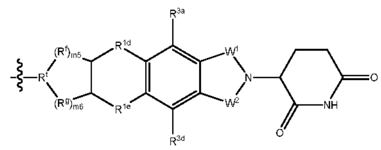, 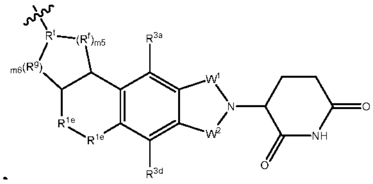, 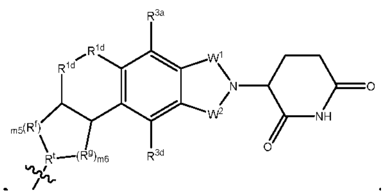,

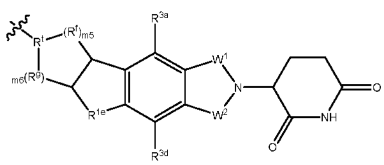, 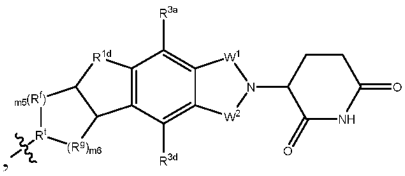, 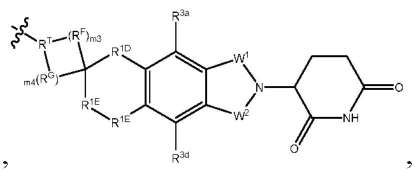,

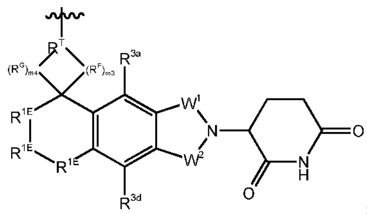, 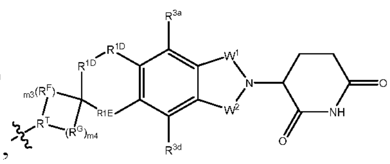, 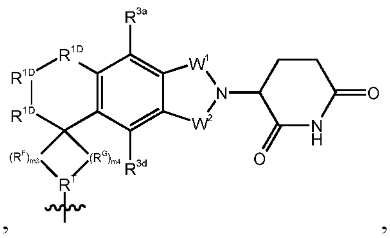,

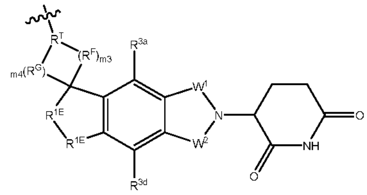, 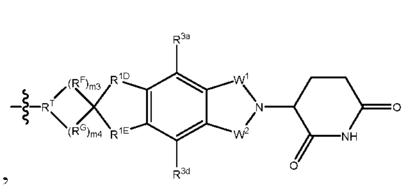, 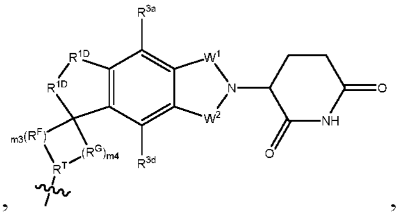,

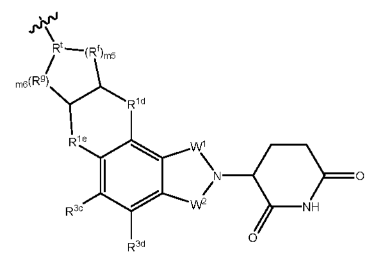,
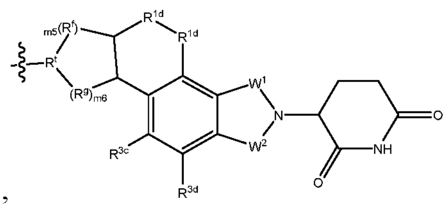,
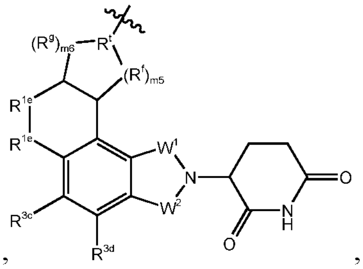,

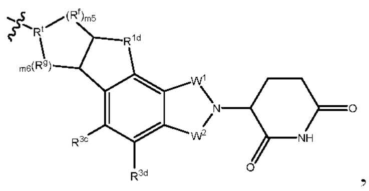,
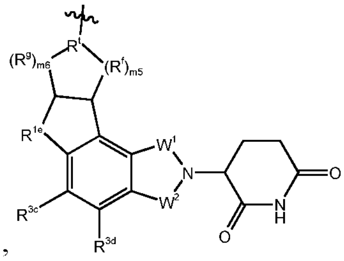,
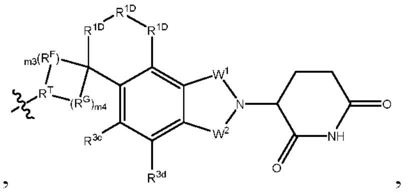,

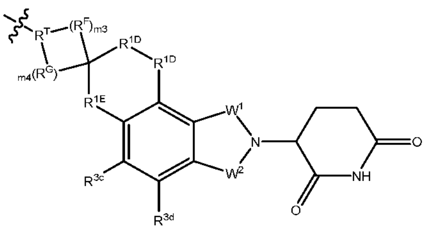,
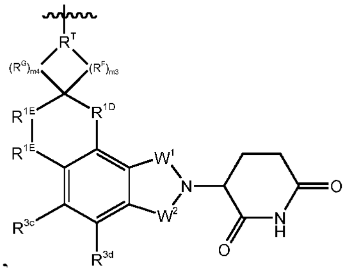,
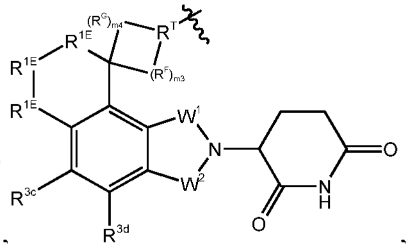,

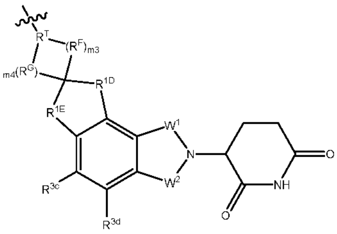,
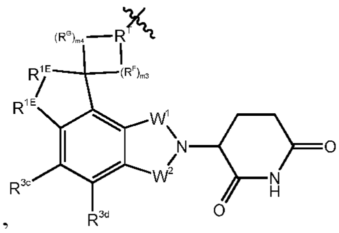,
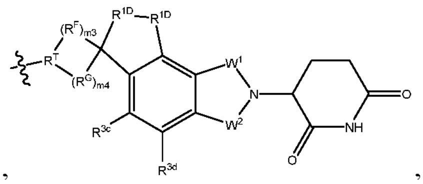,

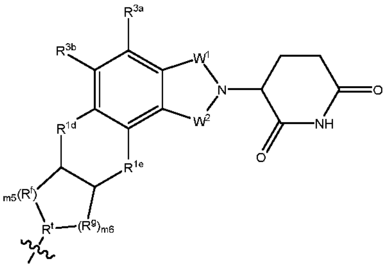,
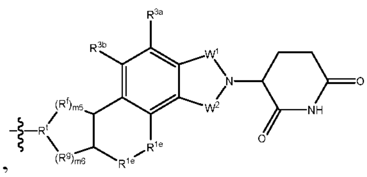,
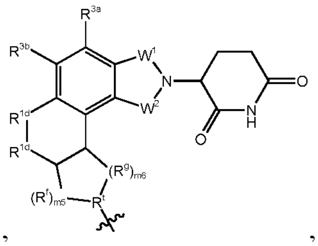,

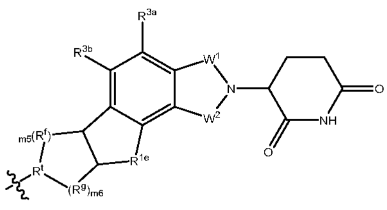,
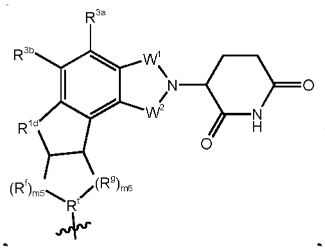,
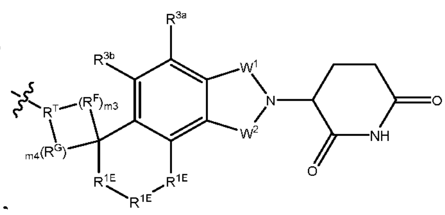,

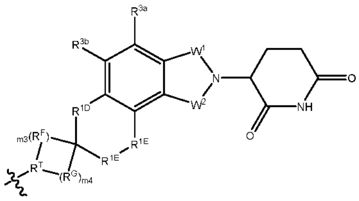
, 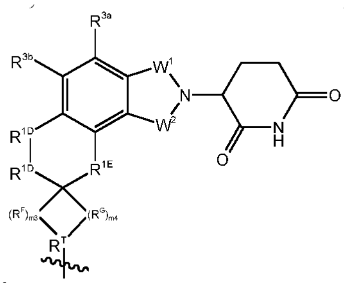
, 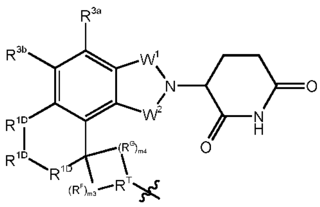
,

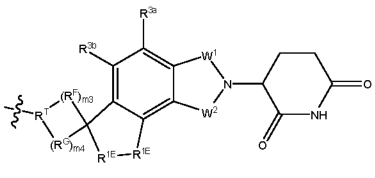
, 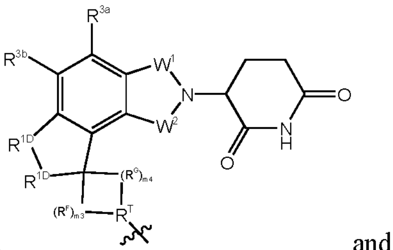
and 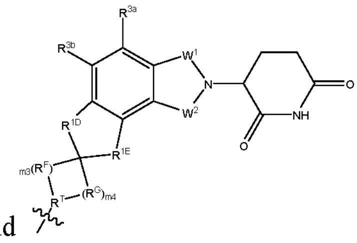
;

wherein, $W^1$, $W^2$, $R^{3a}$, $R^{3b}$, $R^{3c}$, $R^{3a}$, $R^f$, $R^t$, $R^g$, $R^F$, $R^G$, m3, m4, m5, and m6 are as defined in claim 9, 10, or 11; $R^{1d}$, $R^{1e}$, $R^{1D}$, and $R^{1E}$ at each occurrence are each independently $C(R^m)_2$; $R^T$ is N or $CR^{2h}$ and when $R^F$ and $R^G$ are both $C(R^m)_2$, $R^T$ is $CR^{2h}$; and, $R^{2h}$ and $R^m$ are as defined in claim 9 , 10 or 11;
preferably:

$R^{3a}$, $R^{3b}$, $R^{3c}$, $R^{3a}$ are each independently selected from H, deuterium atom, F, Cl, Br, I, $C_1$-$C_6$ alkyl, $C_1$-$C_6$ heteroalkyl, $C_2$ -$C_6$ alkenyl, $C_2$ -$C_6$ alkynyl, $C_1$ -$C_6$ alkoxy, hydroxyl, nitro, cyano, amino, $C_3$-$C_8$ cycloalkyl, $C_4$-$C_{10}$ heterocyclyl, $C_6$-$C_{10}$ aryl and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; and/or
m3 at each occurrence is independently an integer of 0, 1, 2, 3, or 4, and m4 is an integer of 1, 2, 3, 4, or 5, and m3+m4≤5, preferably m3+m4=2 , m3+m4=3 or m3+m4=4; and/or
m5 and m6 at each occurrence are each independently an integer of 0, 1, 2, 3 or 4, and m5+m6≤4, preferably m5+m6=2 or m5+m6=3; and/or
$W^1$ and $W^2$ are identical or different, and are each independently $CH_2$ or C(=O), and at least one of $W^1$ and $W^2$ is C(=O); and/or

$R^{1d}$, $R^{1e}$, $R^{1D}$, $R^{1E}$, $R^f$, $R^g$, $R^F$, and $R^G$ at each occurrence are each independently $C(R^m)_2$, $R^m$ at each occurrence is independently selected from H, deuterium atom, F, Cl, Br, I, $C_1$ -$C_6$ alkyl, $C_1$ -$C_6$ heteroalkyl, $C_2$ -$C_6$ alkenyl, $C_2$ -$C_6$ alkynyl, $C_1$ -$C_6$ alkoxy, hydroxyl, nitro, cyano, amino, $C_3$ -$C_8$ cycloalkyl, $C_4$ -$C_{10}$ heterocyclyl, $C_6$-$C_{10}$ aryl and $C_5$-$C_{10}$ heteroaryl, wherein the $C_1$ -$C_6$ alkyl, $C_1$ -$C_6$ heteroalkyl, $C_2$ -$C_6$ alkenyl, $C_2$ -$C_6$ alkynyl, $C_1$ -$C_6$ alkoxy, $C_3$ -$C_8$ cycloalkyl, $C_4$ -$C_{10}$ heterocyclyl, $C_6$ -$C_{10}$ aryl, and $C_5$-$C_{10}$ heteroaryl are each independently optionally substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl; and/or
$R^t$ at each occurrence is independently N or $CR^{2h}$, $R^T$ at each occurrence is independently $CR^{2h}$; $R^{1h}$, $R^{2h}$, and $R^{3h}$ are each independently selected from H, deuterium atom, F, Cl, Br, I, $C_1$ -$C_6$ alkyl, $C_1$ - $C_6$ deuterated alkyl, $C_1$ -$C_6$ heteroalkyl, $C_2$ -$C_6$ alkenyl, $C_2$-$C_6$ alkynyl, $C_1$ -$C_6$ alkoxy, hydroxyl, nitro, cyano, amino, $C_3$-$C_8$ cycloalkyl, $C_4$-$C_{10}$ heterocyclyl, $C_6$-$C_{10}$ aryl, and $C_5$-$C_{10}$ heteroaryl, wherein the $C_1$-$C_6$ alkyl, $C_1$-$C_6$ heteroalkyl, $C_2$-$C_6$ alkenyl, $C_2$-$C_6$ alkynyl, $C_1$-$C_6$ alkoxy, $C_3$-$C_8$ cycloalkyl, $C_4$-$C_{10}$ heterocyclyl, $C_6$-$C_{10}$ aryl and $C_5$-$C_{10}$ heteroaryl are each independently substituted with one or more substituents selected from F, Cl, Br, I, $C_1$-$C_6$ alkyl, $C_1$-$C_6$ heteroalkyl, $C_1$-$C_6$ alkoxy, $C_1$-$C_6$ haloalkyl, hydroxyl, $C_1$-$C_6$ hydroxyalkyl, cyano, amino, nitro, $C_3$-$C_8$ cycloalkyl, $C_4$-$C_{10}$ heterocyclyl, $C_6$-$C_{10}$ aryl and $C_5$-$C_{10}$ heteroaryl.

**16.** The compound according to any one of claims 9-11, wherein the CLM is selected from the following structures:

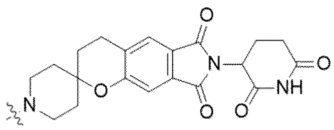,
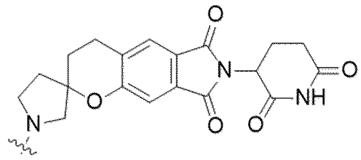,
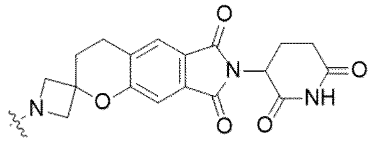,
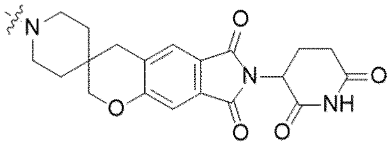,
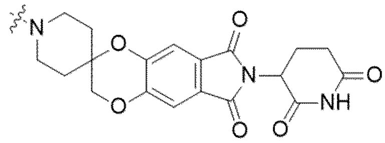,
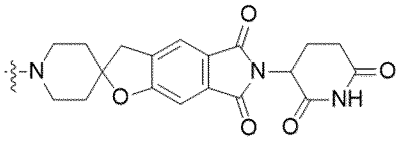,
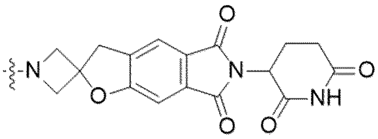,
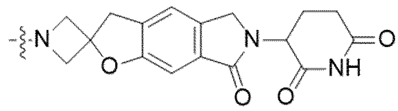,
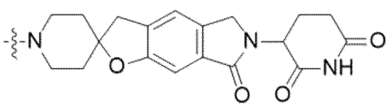,
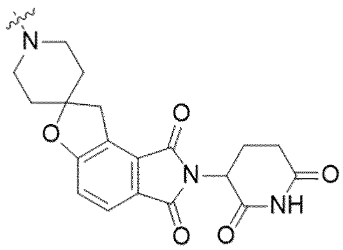,
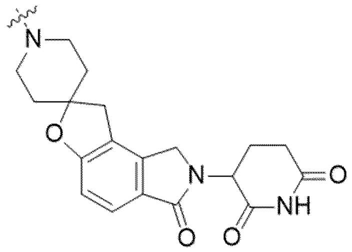,
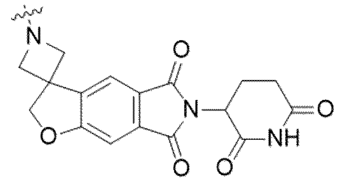,
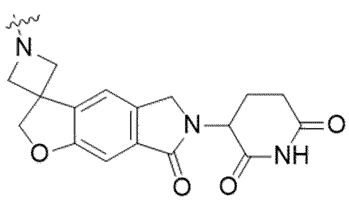,
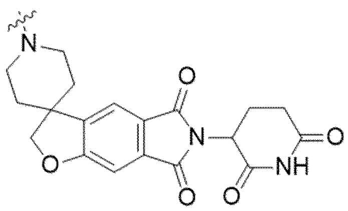,
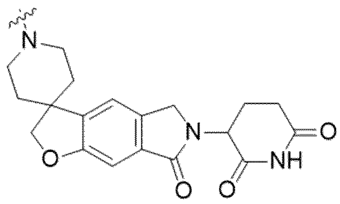,
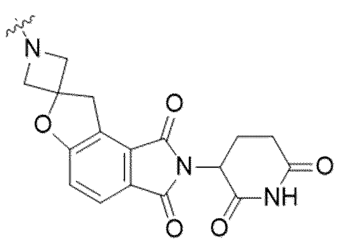,
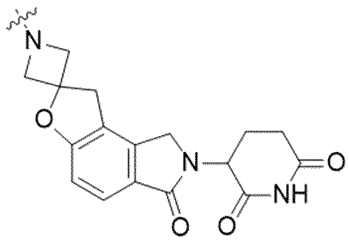,
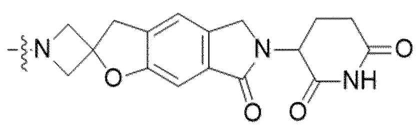,
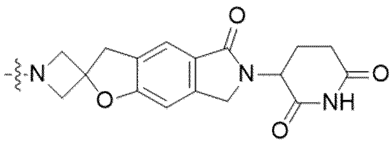,
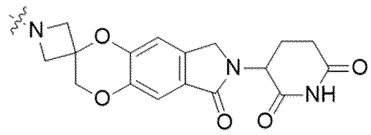,
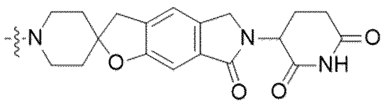,

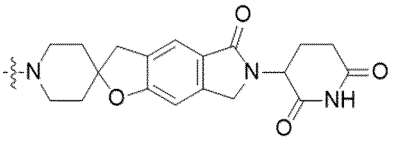 , 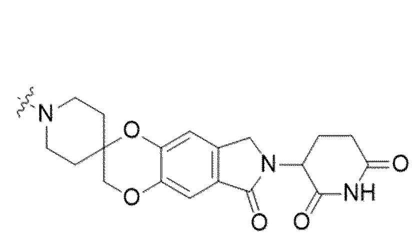 , 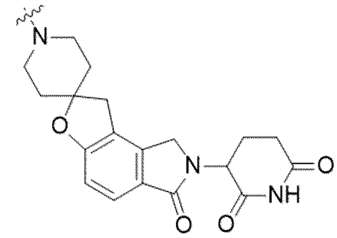 ,

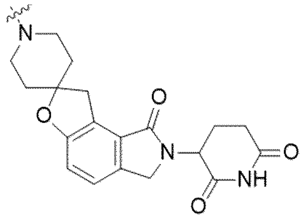 , 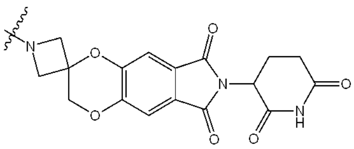 , 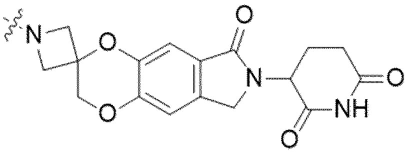 ,

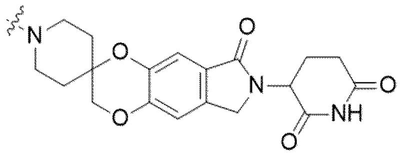 , 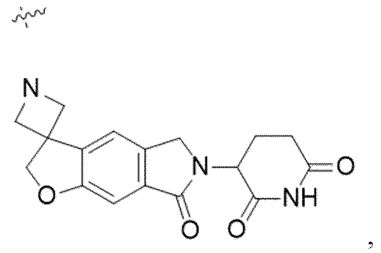 , 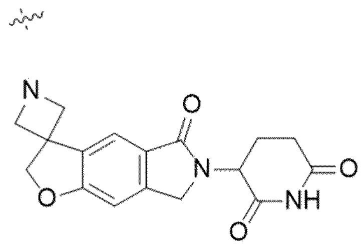 ,

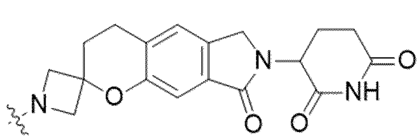 , 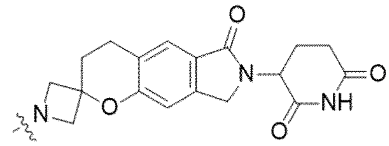 , 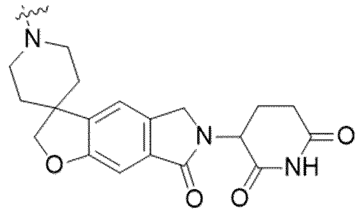 ,

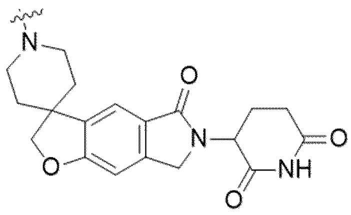 , 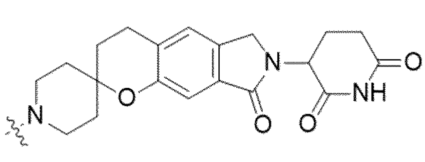 , 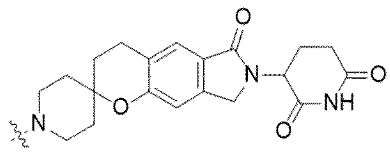 ,

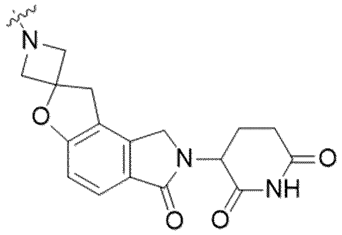 , 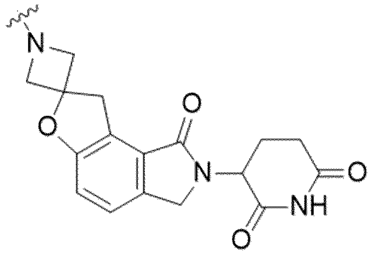 , 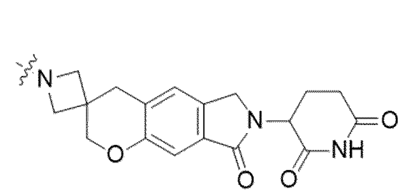 ,

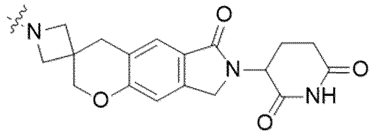 , 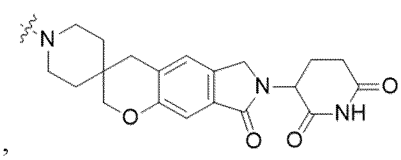 , 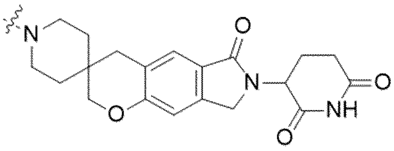 ,

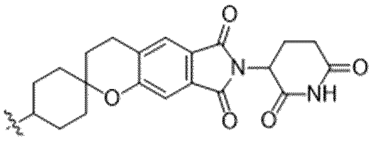 , 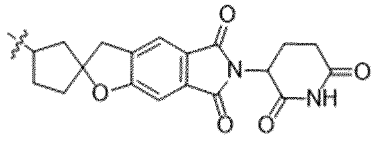 , 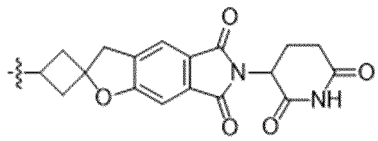 ,

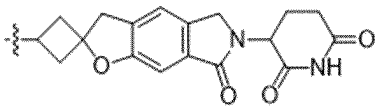 , 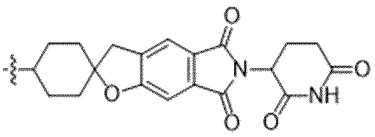 , 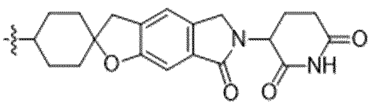 ,

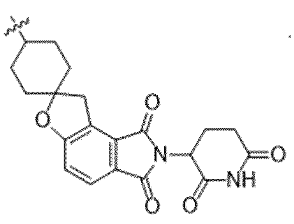 , 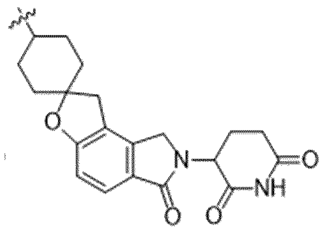 , 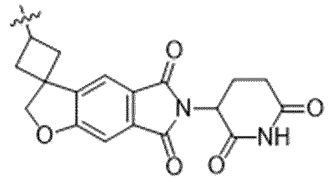 ,

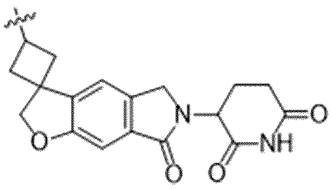 , 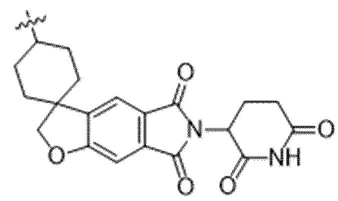 , 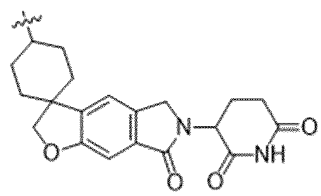 ,

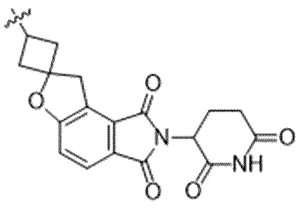 , 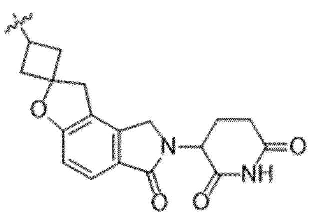 , 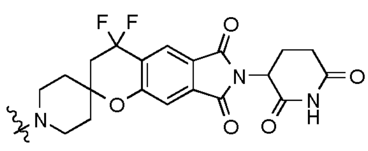 ,

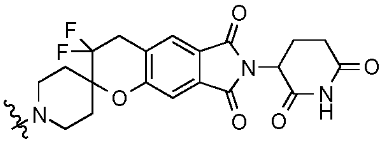 , 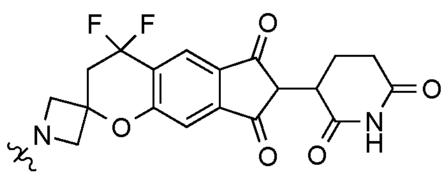 , 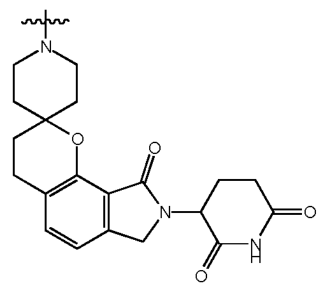 ,

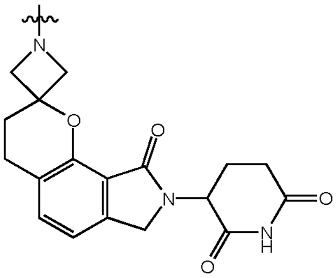 , 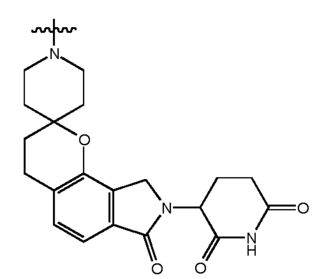 , 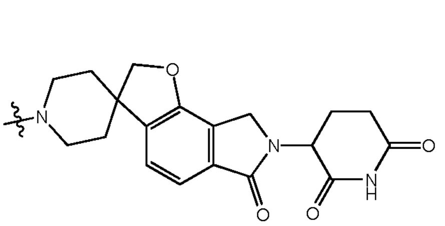 ,

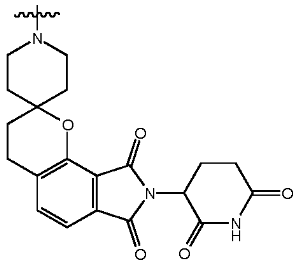 ,
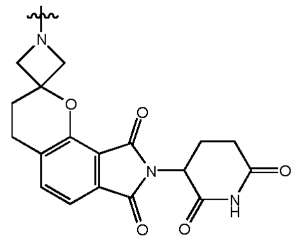 ,
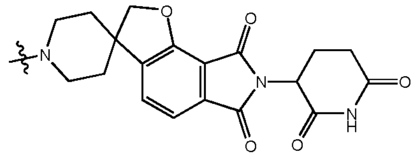 ,

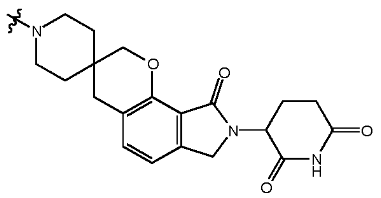 ,
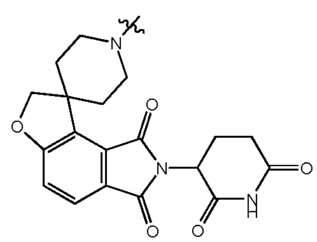 ,
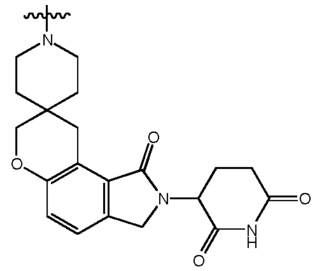 ,

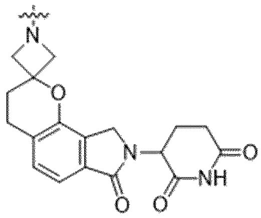 ,
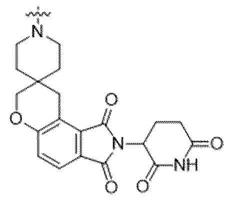 ,
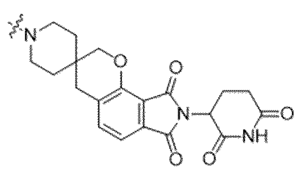 ,
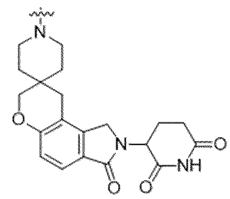 ,

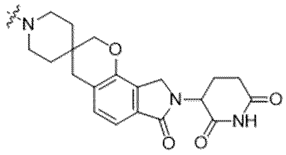 ,
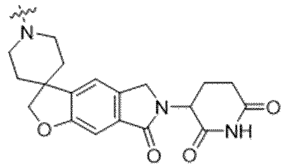 ,
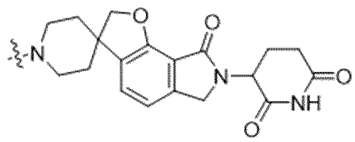 ,

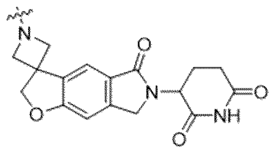 ,
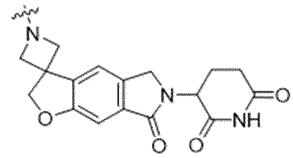 ,
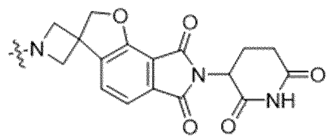 ,

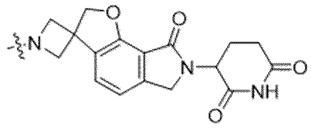 ,
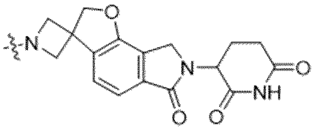 ,
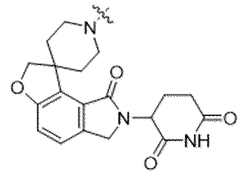 ,
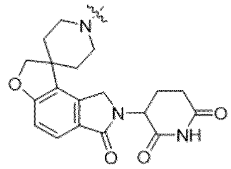 ,

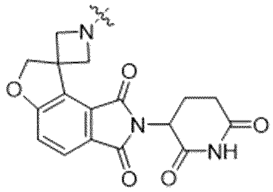 ,
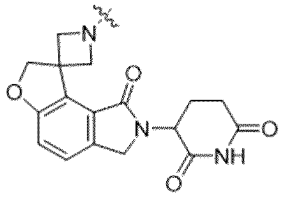 ,
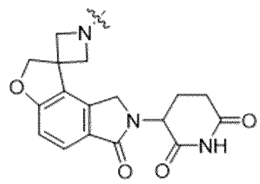 ,

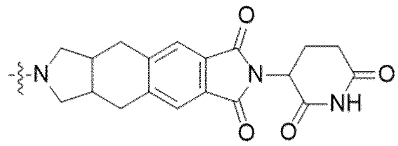 , 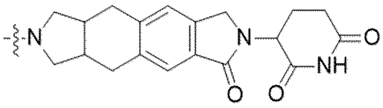 , 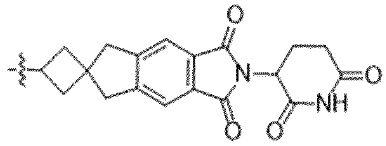 ,

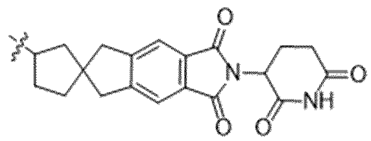 , 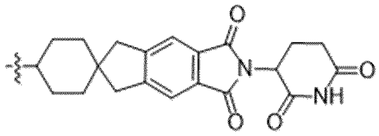 , 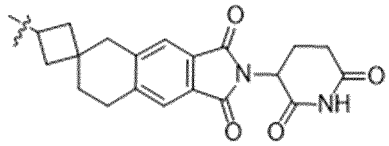 ,

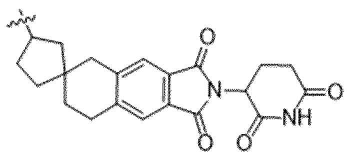 , 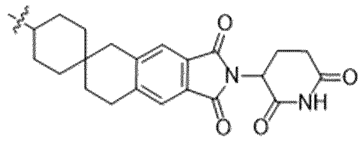 , 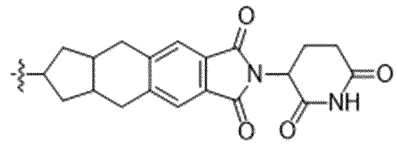 ,

and

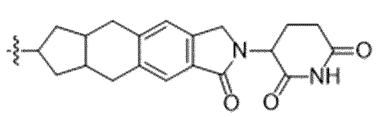

**17.** The compound according to any one of claims 9-16, wherein L is a bond or $-(B^L)_q-$:

$B^L$ at each occurrence is identical or different, and is each independently selected from: $CR^{L1}R^{L2}$, O, S, SO, $SO_2$, $NR^{L3}$, $SO_2NR^{L3}$, $SONR^{L3}$, $CONR^{L3}$, $NR^{L3}CONR^{L4}$, $NR^{L3}SO_2NR^{L4}$, CO, $CR^{L1}=CR^{L2}$, C≡C, $SiR^{L1}R^{L2}$, $P(O)R^{L1}$, $P(O)OR^{L1}$, $NR^{L3}C(=NCN)NR^{L4}$, $NR^{L3}C(=NCN)$, $NR^{L3}C(=CNO_2)NR^{L4}$, cycloalkylene, heterocyclylene, arylene, or heteroarylene, wherein the cycloalkylene, heterocyclylene, arylene, and heteroarylene are optionally substituted with 0-6 $R^{L1}$ and/or $R^{L2}$;

$R^{L1}$ , $R^{L2}$ , $R^{L3}$ , and $R^{L4}$ at each occurrence are independently selected from H, halogen, $C_{1-8}$ alkyl, O-$C_{1-8}$ alkyl, S-$C_{1-8}$ alkyl, NH-$C_{1-8}$ alkyl, N($C_{1-8}$ alkyl)$_2$ , $C_{3-11}$ cycloalkyl, aryl, heteroaryl, $C_{3-11}$ heterocyclyl, O-$C_{3-8}$ cycloalkyl, O-$C_{3-11}$ heterocyclyl, O-aryl, O-heteroaryl, S-$C_{3-8}$ cycloalkyl, NH-$C_{3-8}$ cycloalkyl, N ($C_{3-8}$ cycloalkyl)$_2$ , N($C_{3-8}$ cycloalkyl)($C_{1-8}$ alkyl), NH-$C_{3-8}$ heterocyclyl, N ($C_{3-8}$ heterocyclyl)$_2$ , N($C_{3-8}$ heterocyclyl)($C_{1-8}$ alkyl), NH-aryl, N(aryl)($C_{1-8}$ alkyl), NH-heteroaryl, N(heteroaryl)($C_{1-8}$ alkyl), OH, $NH_2$, SH, $SO_2P(O)$(O-$C_{1-8}$ alkyl)($C_{1-8}$ alkyl), P(O)(O-$C_{1-8}$ alkyl)$_2$ , C≡C-$C_{1-8}$ alkyl, C≡CH, CH=CH-($C_{1-8}$ alkyl), C($C_{1-8}$ alkyl)=CH-($C_{1-8}$ alkyl), C($C_{1-8}$ alkyl) =C($C_{1-8}$ alkyl)$_2$, $Si(OH)_3$, Si($C_{1-8}$alkyl)$_3$ , Si(OH)($C_{1-8}$ alkyl)$_2$ , CO-$C_{1-8}$alkyl, COzH, CN, $CF_3$, $CHF_2$, $CH_2F$, $NO_2$, $SF_5$, $SO_2$NH-$C_{1-8}$ alkyl, $SO_2$N($C_{1-8}$ alkyl)$_2$ , SONH-$C_{1-8}$ alkyl, SON($C_{1-8}$ alkyl)$_2$ , CONH-$C_{1-8}$ alkyl, CON($C_{1-8}$ alkyl)$_2$ , N($C_{1-8}$ alkyl)CONH($C_{1-8}$ alkyl), N($C_{1-8}$ alkyl)CON($C_{1-8}$ alkyl)$_2$ , NHCONH($C_{1-8}$ alkyl), NHCON($C_{1-8}$ alkyl)$_2$ , $NHCONH_2$ , N($C_{1-8}$ alkyl)$SO_2$NH($C_{1-8}$ alkyl), N($C_{1-8}$ alkyl)$SO_2$N($C_{1-8}$ alkyl)$_2$ , $NHSO_2$NH($C_{1-8}$ alkyl), $NHSO_2$N($C_{1-8}$ alkyl)$_2$ , and $NHSO_2NH_2$, optionally, the $C_{1-8}$ alkyl, $C_{3-11}$ cycloalkyl, $C_{3-11}$ heterocyclyl, $C_{6-10}$ aryl, and $C_{5-10}$ heteroaryl are each independently substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, halocycloalkyl, haloheterocyclyl, alkylamino, aryl, heteroaryl, haloaryl, and haloheteroaryl; and

q is an integer greater than or equal to 1; preferably, q is 1, 2, 3, 4, 5, 6, 7, 8, 9, 10, 11, 12, 13, 14, 15, 16, 17, 18, 19 or 20.

**18.** The compound according to claim 17, wherein $B^L$ is selected from one or more of the following structures: -O-, -S-, -SO-, $-SO_2-$, $-CH_2-$, -CO-, -NH-,

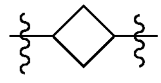 , 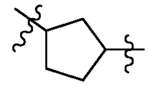 , 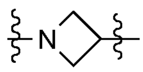 ,

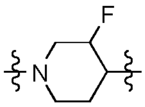, 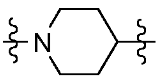, 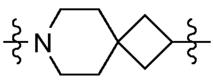, 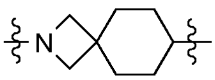, 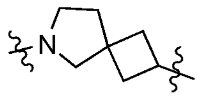,

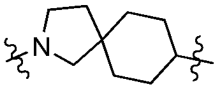, 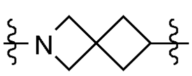, 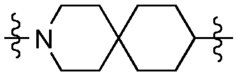, 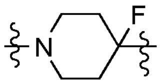, 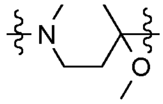,

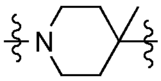, 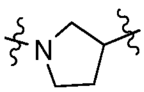, 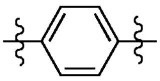, 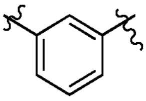, 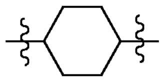, 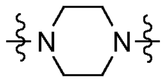,

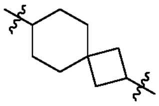, 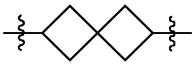, 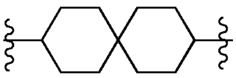, 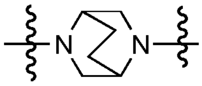, 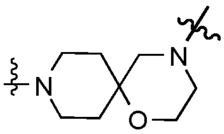,

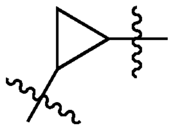 and 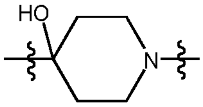

and

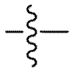

is the point of attachment.

**19.** The compound according to any one of claims 9-18, wherein L is selected from the following structures:

covalent bond, $-(CH_2)_j-$, $-(CH_2)_p-NH-(CH_2)_s-$, $-(CH_2)_y-NH-(CH_2)_j-NH-(CH_2)_s-$, $-(CH_2)_p-CO-(CH_2)_s-$, $-(CH_2)_p-O-(CH_2)_s-$, $-(CH_2)_y-CO-(CH_2)_j-CO-(CH_2)_s-$, $-(CH_2)_y-O-(CH_2)_j-O-(CH_2)_s-$, $-(CH_2)y-O-(CH_2)_j-CO-(CH_2)_s-$, $-(CH_2)_y-CO-(CH_2)_j-CO-(CH_2)_s-$, $-(CH_2)_p-NH-(CH_2)_y-O-(CH_2)_j-CO-(CH_2)_s-$, $-(CH_2)_y-CO-(CH_2)_j-O-(CH_2)_s-NH-(CH_2)_p-$,

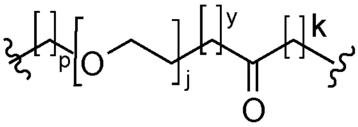, 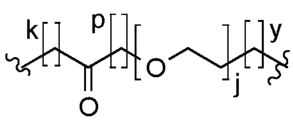, 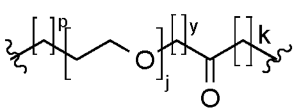,

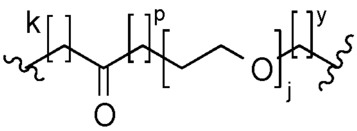, 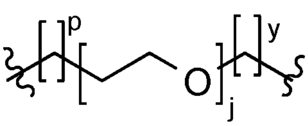, 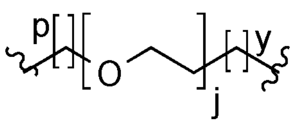,

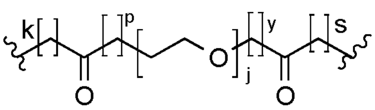 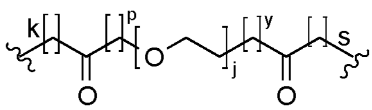 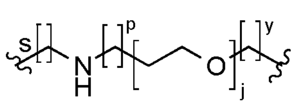

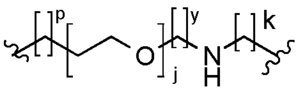, 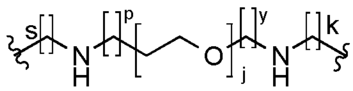, 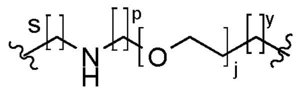,

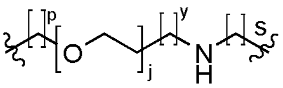, 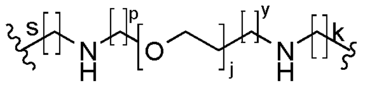, 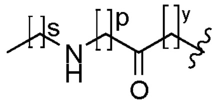, 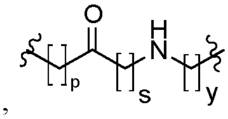,

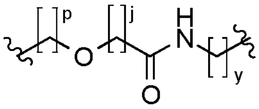, 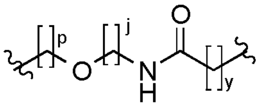, 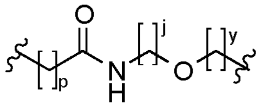, 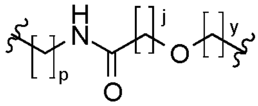,

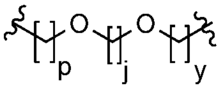, 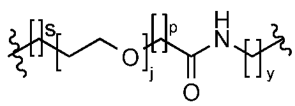, 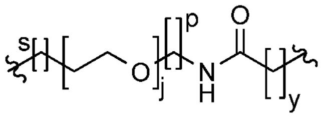,

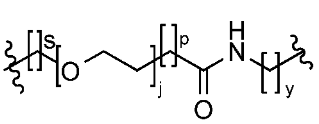, 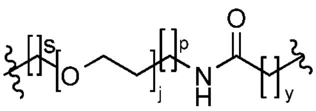, 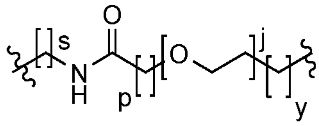,

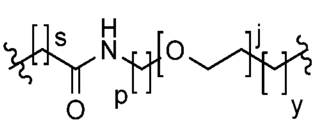, 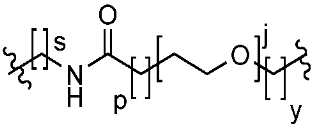, 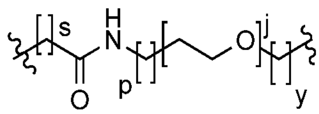,

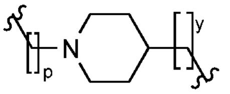, 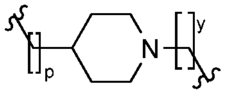, 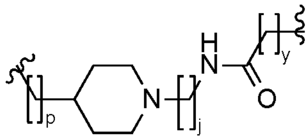, 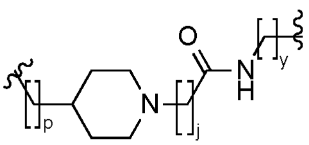,

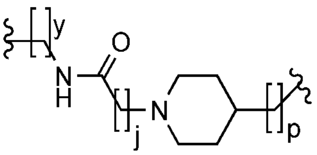, 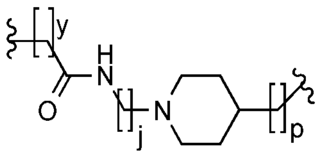, 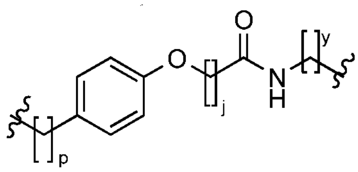,

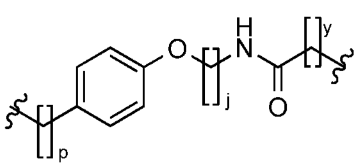, 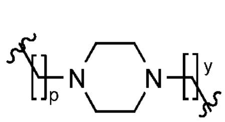, 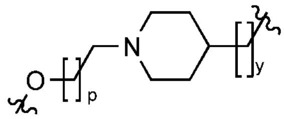,

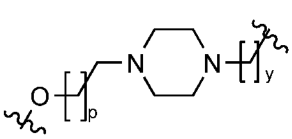, 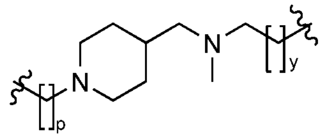, 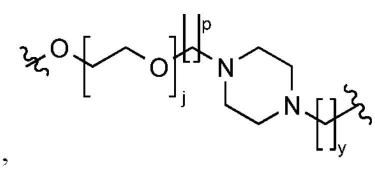,

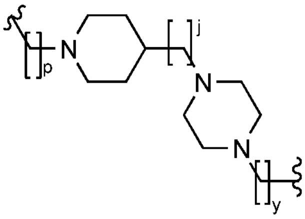, 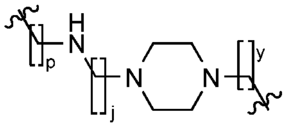, 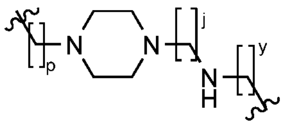,

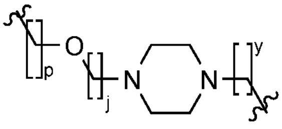, 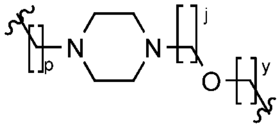, 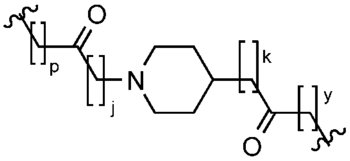,

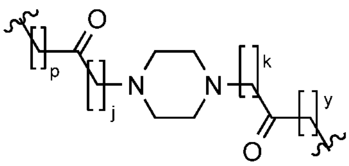, 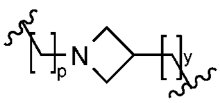, 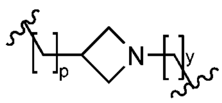, 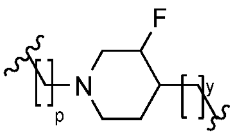,

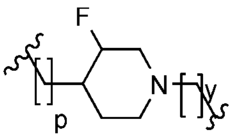, 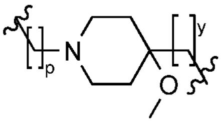, 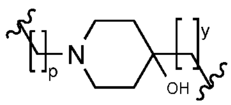, 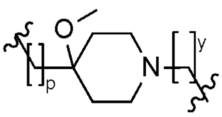,

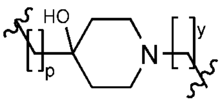, 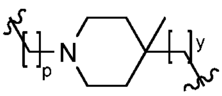, 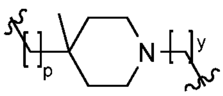, 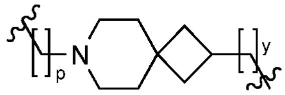,

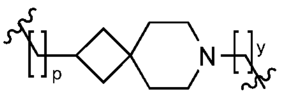, 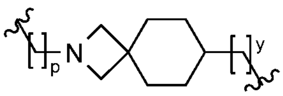, 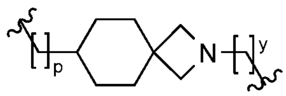,

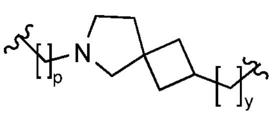, 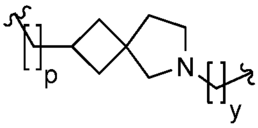, 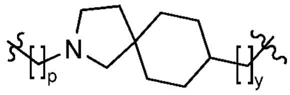,

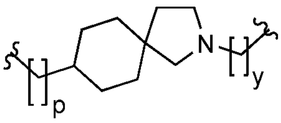, 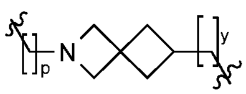, 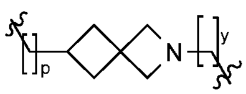, 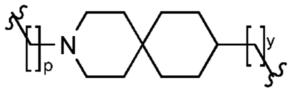,

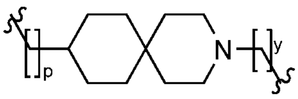, 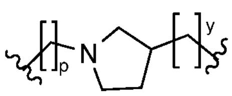, 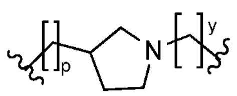, 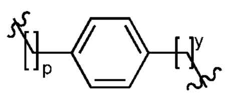

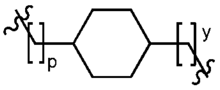, 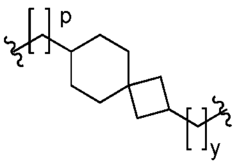, 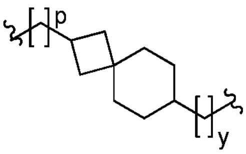, 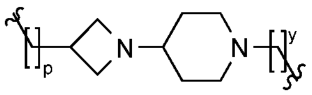,

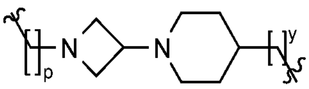, 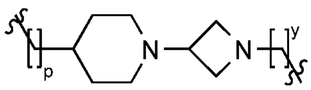, 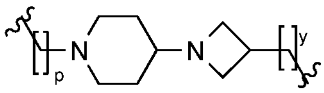,

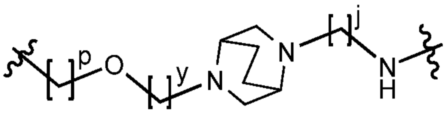, 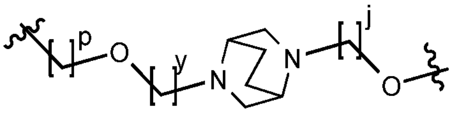,

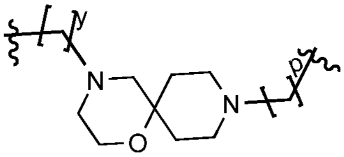, 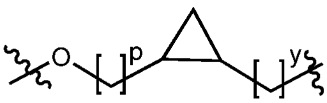, 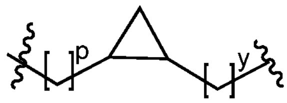,

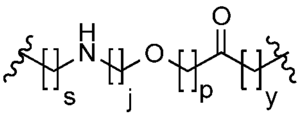, 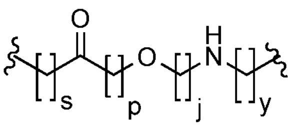, 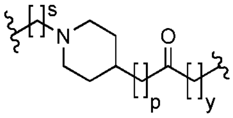, 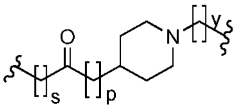,

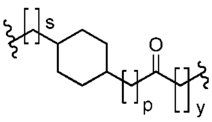, 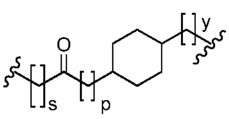, 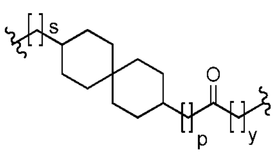, 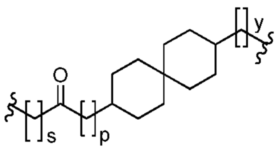,

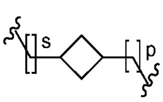, 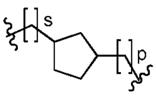, 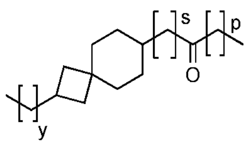, 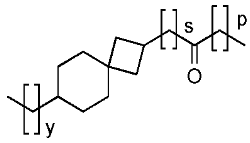, 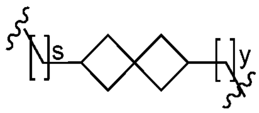,

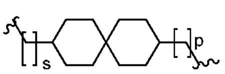, 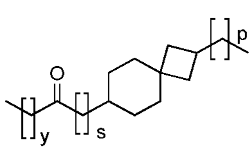, and 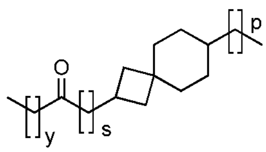;

wherein, j is 1, 2, 3, 4, 5, 6, 7, 8, 9 or 10;
k, s, p and y are 0, 1, 2, 3, 4, 5, 6, 7, 8, 9 or 10;

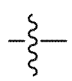

is the point of attachment with CLM or PTM;
preferably L is selected from covalent bond, $-CH_2-$,$-(CH_2)_2-$,$-(CH_2)_3-$,$-(CH_2)_4-$,$-(CH_2)_5-$,$-(CH_2)_6-$,$-(CH_2)_7-$,$-(CH_2)_8-$,$-NH-CH_2-$,$-NH-(CH_2)_2-$,$-NH-(CH_2)_3-$,$-NH-(CH_2)_4-$,$-NH-(CH_2)_5-$,$-NH-(CH_2)_6-$,$-NH-(CH_2)_7-$,$-NH-(CH_2)_8-$,-CO-

NH-$CH_2$-,-CO-NH-$(CH_2)_2$-,-CO-NH-$(CH_2)_3$-,-CO-NH-$(CH_2)_4$-,-CO-NH-$(CH_2)_5$-,-CO-NH-$(CH_2)_6$-,-CO-NH-$(CH_2)_7$-,-CO-NH-$(CH_2)_8$-,-$CH_2$-NH-,-$(CH_2)_2$-NH-,-$(CH_2)_3$-NH-,-$(CH_2)_4$-NH-,-$(CH_2)_5$-NH-,-$(CH_2)_6$-NH-,-$(CH_2)_7$-NH-,-$(CH_2)_8$-NH-,-NH-$CH_2$-NH-,-NH-$(CH_2)_2$-NH-,-NH-$(CH_2)_3$-NH-,-NH-$(CH_2)_4$-NH-,-NH-$(CH_2)_5$-NH-,-NH-$(CH_2)_6$-NH-,-NH-$(CH_2)_7$-NH-,-NH-$(CH_2)_8$-NH-,-($CH_2$-$CH_2$-O)-$CH_2$-$CH_2$-,-($CH_2$-$CH_2$-O)$_2$-$CH_2$-$CH_2$-,-($CH_2$-$CH_2$-O)$_3$-$CH_2$-$CH_2$-,-NH-($CH_2$-$CH_2$-O)-$CH_2$-$CH_2$-,-NH-($CH_2$-$CH_2$-O)$_2$-$CH_2$-$CH_2$-,-NH-($CH_2$-$CH_2$-O)$_3$-$CH_2$-$CH_2$-,-CO-NH-($CH_2$-$CH_2$-O)-$CH_2$-$CH_2$-,-CO-NH-($CH_2$-$CH_2$-O)$_2$-$CH_2$-$CH_2$-,-CO-NH-($CH_2$-$CH_2$-O)$_3$-$CH_2$-$CH_2$-,-($CH_2$-$CH_2$-O)-$CH_2$-$CH_2$-NH-,-($CH_2$-$CH_2$-O)$_2$-$CH_2$-$CH_2$-NH-, -($CH_2$-$CH_2$-O)$_3$-$CH_2$-$CH_2$-NH-,-NH-($CH_2$-$CH_2$-O)-$CH_2$-$CH_2$-NH-,-NH-($CH_2$-$CH_2$-O)$_2$-$CH_2$-$CH_2$-NH-,-NH-($CH_2$-$CH_2$-O)$_3$-$CH_2$-$CH_2$-NH-,-CO-NH-($CH_2$-$CH_2$-O)-$CH_2$-$CH_2$-NH-,-CO-NH-($CH_2$-$CH_2$-O)$_2$-$CH_2$-$CH_2$-NH-,-CO-NH-($CH_2$-$CH_2$-O)$_3$-$CH_2$-$CH_2$-NH-,-$CH_2$-$CH_2$-(O-$CH_2$-$CH_2$)-,-$CH_2$-$CH_2$-(O-$CH_2$-$CH_2$)$_2$-, -$CH_2$-$CH_2$-(O-$CH_2$-$CH_2$)$_3$-,-NH-$CH_2$-$CH_2$-(O-$CH_2$-$CH_2$)-,-NH-$CH_2$-$CH_2$-(O-$CH_2$-$CH_2$)$_2$-,-NH-$CH_2$-$CH_2$-(O-$CH_2$-$CH_2$)$_3$-,-CO-NH-$CH_2$-$CH_2$-(O-$CH_2$-$CH_2$)-,-CO-NH-$CH_2$-$CH_2$-(O-$CH_2$-$CH_2$)$_2$-,-CO-NH-$CH_2$-$CH_2$-(O-$CH_2$-$CH_2$)$_3$-,-$CH_2$-$CH_2$-(O-$CH_2$-$CH_2$)-NH-,-$CH_2$-$CH_2$-(O-$CH_2$-$CH_2$)$_2$-NH-,-$CH_2$-$CH_2$-(O-$CH_2$-$CH_2$)$_3$-NH-,-NH-$CH_2$-$CH_2$-(O-$CH_2$-$CH_2$)-NH-,-NH-$CH_2$-$CH_2$-(O-$CH_2$-$CH_2$)$_2$-NH-,-NH-$CH_2$-$CH_2$-(O-$CH_2$-$CH_2$)$_3$-NH-,-NH-$CH_2$-$CH_2$-O-$CH_2$-$CH_2$-CO-,-CO-$CH_2$-$CH_2$-O-$CH_2$-$CH_2$-NH-,-NH-$(CH_2)_4$-CO-,-NH-$(CH_2)_5$-CO-,-NH-$(CH_2)_6$-CO-,-CO-$(CH_2)_4$-NH-,-CO-$(CH_2)_5$-NH-,-CO-$(CH_2)_6$-NH-,-NH-($CH_2$-$CH_2$-O)-$(CH_2)_3$-,-NH-($CH_2$-$CH_2$-O)-$(CH_2)_4$-,-NH-($CH_2$-$CH_2$-O)-$(CH_2)_5$-,-NH-($CH_2$-$CH_2$-O)-$(CH_2)_6$-,-$(CH_2)_3$-(O-$CH_2$-$CH_2$)-NH-,-$(CH_2)_4$-(O-$CH_2$-$CH_2$)-NH-,-$(CH_2)_5$-(O-$CH_2$-$CH_2$)-NH-,-$(CH_2)_6$-(O-$CH_2$-$CH_2$)-NH-,-$CH_2$-$CH_2$-O-$(CH_2)_2$-CO-,-$CH_2$-$CH_2$-O-$(CH_2)_3$-CO-,-$CH_2$-$CH_2$-O-$(CH_2)_4$-CO-,-CO-$(CH_2)_2$-O-$CH_2$-$CH_2$-,-CO-$(CH_2)_3$-O-$CH_2$-$CH_2$-,-CO-$(CH_2)_4$-O-$CH_2$-$CH_2$-,-CO-$(CH_2)_2$-,-CO-$(CH_2)_3$-,-CO-$(CH_2)_4$-,-CO-$(CH_2)_5$-,-CO-$(CH_2)_6$-, -$(CH_2)_2$-CO-,-$(CH_2)_3$-CO-,-$(CH_2)_4$-CO-,-$(CH_2)_5$-CO-,-$(CH_2)_6$-CO-,-CO-$(CH_2)_2$-CO-,-CO-$(CH_2)_3$-CO-,-CO-$(CH_2)_4$-CO-,-CO-$(CH_2)_5$-CO-,-CO-$(CH_2)_6$-CO-,-$CH_2$-CO-$CH_2$-,-$CH_2$-CO-$(CH_2)_2$-,-$CH_2$-CO-$(CH_2)_3$-, -$CH_2$-CO-$(CH_2)_4$-,-$(CH_2)_2$-CO-$CH_2$-,-$(CH_2)_2$-CO-$(CH_2)_2$-,-$(CH_2)_2$-CO-$(CH_2)_3$-,-$(CH_2)_2$-CO-$(CH_2)_4$-,-$(CH_2)_3$-CO-$CH_2$-,-$(CH_2)_3$-CO-$(CH_2)_2$-,-$(CH_2)_3$-CO-$(CH_2)_3$-,-$(CH_2)_3$-CO-$(CH_2)_4$-,-$(CH_2)_4$-CO-$CH_2$-,-$(CH_2)_4$-CO-$(CH_2)_2$-,-$(CH_2)_4$-CO-$(CH_2)_3$-,-$(CH_2)_4$-CO-$(CH_2)_4$-,-$CH_2$-O-$CH_2$-,-$CH_2$-O-$(CH_2)_2$-,-$CH_2$-O-$(CH_2)_3$-, -$CH_2$-O-$(CH_2)_4$-,-$(CH_2)_2$-O-$CH_2$-,-$(CH_2)_2$-O-$(CH_2)_2$-,-$(CH_2)_2$-O-$(CH_2)_3$-,-$(CH_2)_2$-O-$(CH_2)_4$-,-$(CH_2)_3$-O-$CH_2$-,-$(CH_2)_3$-O-$(CH_2)_2$-,-$(CH_2)_3$-O-$(CH_2)_3$-,-$(CH_2)_3$-O-$(CH_2)_4$-,-$(CH_2)_4$-O-$CH_2$-,-$(CH_2)_4$-O-$(CH_2)_2$-,-$(CH_2)_4$-O-$(CH_2)_3$-,-$(CH_2)_4$-O-$(CH_2)_4$-,

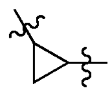, 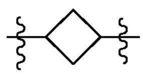, 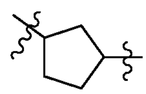,

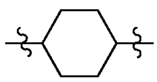, 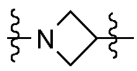, 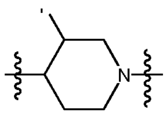, 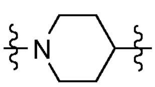, 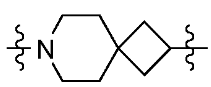, 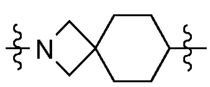,

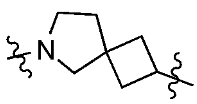, 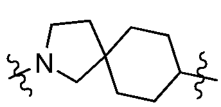, 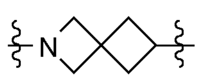, 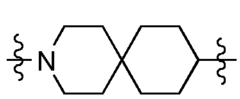, 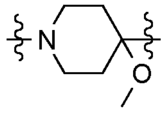,

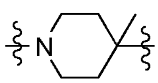, 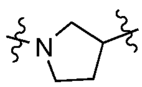, 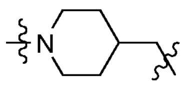, 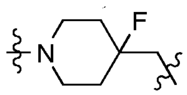, 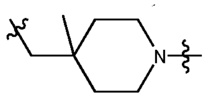, 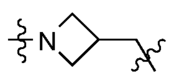,

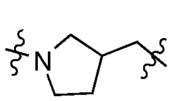, 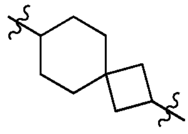, 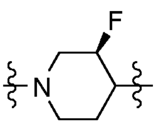, 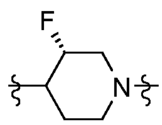, 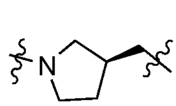, 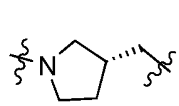,

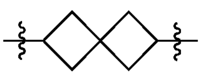, 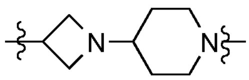, 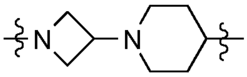, 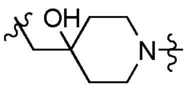, 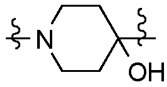,

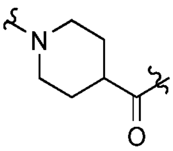, 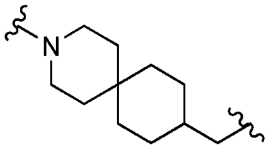, 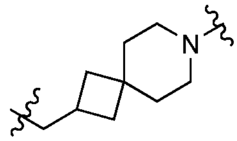, 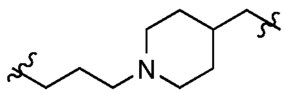,

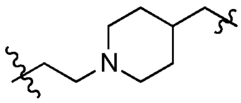, 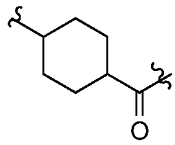, 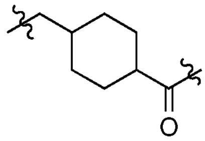, 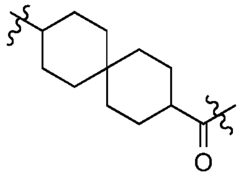, 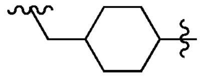,

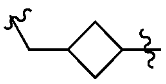, 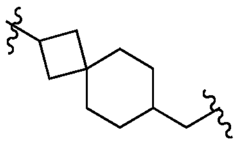, 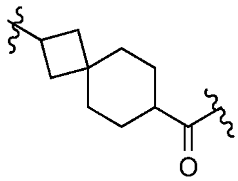, 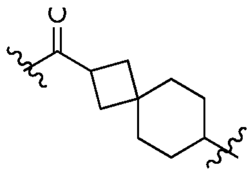,

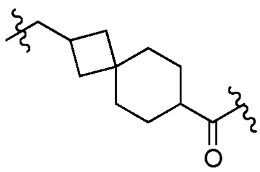, 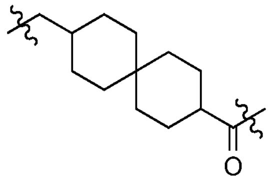, 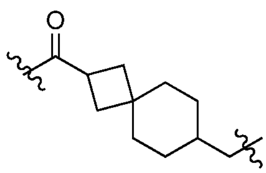, 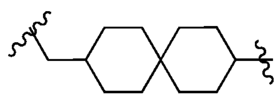,

and

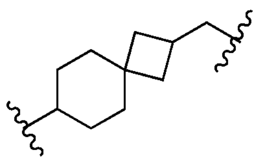

**20.** The compound according to any one of claims 9-19, wherein the PTM is a moiety that binds to a target protein or polypeptide, wherein the target protein is selected from the following proteins: structural proteins, receptors, enzymes, cell surface proteins; proteins pertinent to the integrated function of a cell, including proteins involved in catalytic activity, aromatase activity, motor activity, helicase activity, metabolic process, antioxidant activity, proteolysis, biosynthesis; proteins with kinase activity, oxidoreductase activity, transferase activity, hydrolase activity, lyase activity, isomerase activity, ligase activity, enzyme regulator activity, signal transducer activity, structural molecule activity, binding activity, receptor activity, cell motility, membrane fusion, cell communication, regulation of biological process, development, cell differentiation, response to stimulus; behavioral proteins; cell adhesion proteins; proteins involved in cell death; and proteins involved in transportation, including proteins of protein transporter activity, nuclear transporter activity, ion transporter activity, channel transporter activity, carrier activity, permease activity, secretion activity, electron transporter activity, pathogenesis, chaperone regulator activity, nucleic acid binding activity, transcription regulator activity, extracellular tissues and biogenesis activity, and translation regulator activity.

**21.** The compound according to any one of claims 9-20, wherein the PTM is a moiety binding to a target protein or polypeptide, wherein the target protein is selected from the group consisting of B7.1 and B7, TNFR2, NADPH oxidase, BclIBax and other conjugates in the apoptosis pathway, C5a receptor, HMG-CoA reductase, PDE V phosphodiesterase type, PDEIV phosphodiesterase type 4, PDEI I, PDEI II, PDE III, squalene epoxidation enzyme, CXCR1, CXCR2, nitric oxide (NO) synthase, cyclo-oxygenase 1, cyclo-oxygenase 2, 5HT receptors, dopamine receptors, G proteins (ie Gq), histamine receptors, 5-lipoxygenase, tryptase serine protease, thymidylate synthase, purine nucleoside phosphorylase, GAPDH trypanosomal, glycogen phosphorylase, carbonic anhydrase, chemokine receptor, JAW STAT, RXR and its analog, HIV1 protease, HIV1 integrase, influenza neuraminidase, hepatitis B reverse transcriptase, sodium channel, multi drug resistant bacteria, protein P-glycoprotein, tyrosine kinases, CD23, CD124, tyrosine kinase p561ck , CD4, CD5, IL-2 receptor, IL-1 receptor, TNF-αR, ICAM1, Cat+ channels, VCAM, VLA-4 integrin, selectins, CD40/CD40L, inosine monophosphate dehydrogenase, p38 MAP kinase, RaslRaflMEWERK pathway, interleukin-1 converting enzyme, caspase, HCV, NS3 protease, HCV NS3 RNA helicase, glycinamide ribonucleotide formyl transferase, rhinovirus, 3C protease, herpes simplex virus-I (HSV-I), protease, cytomegalovirus (CMV) protease, poly(ADP-ribose) polymerase, cyclindependent kinase 4/6, vascular endothelial growth factor, oxytocin receptor, microsomal transfer protein inhibitor, bile acid transport inhibitor, 5α reductase inhibitors, angiotensin 11, glycine receptor, noradrenaline reuptake receptor, endothelin receptor, neuropeptide Y and receptor, adenosine receptors, adenosine kinase and AMP deaminase, purinergic receptors (P2Y1, P2Y2, P2Y4, P2Y6, P2X1-7), farnesyltransferases, geranylgeranyl transferase, TrkA a receptor for NGF, β-amyloid, tyrosine kinase Flk-II KDR, vitronectin receptor, integrin receptor, Her-21 neu, telomerase inhibition, cytosolic phospholipase A2, EGF receptor tyrosine kinase, ecdysone 20-monooxygenase, ion channel of GABA-gated chloride channel, acetylcholinesterase, voltage-sensitive sodium channel protein, calcium release channel and chloride ion channel, acetyl-CoA carboxylase, adenylosuccinate synthetase, protoporphyrinogen oxidase, enolpyruvylshikimate phosphate synthase, MYC protein, androgen receptor, estrogen receptor, aurora kinase A, Bcr-Abl protein, epidermal growth factor receptor, anaplastic lymphoma kinase, Bruton's tyrosine kinase, bromodomain protein 4.

**22.** The compound according to any one of claims 9-21, wherein the PTM is an Hsp90 inhibitor, a kinase inhibitor, a phosphatase inhibitor, a MDM2 inhibitor, a compound that binds to a protein comprising the human BET bromodomain, a HDAC inhibitor, a human lysine methyltransferase inhibitor, a compound that binds to RAF receptor, a compound that binds to FKBP, an angiogenesis inhibitor, an immunosuppressive compound, an compound that binds to aryl hydrocarbon receptor, a compound that binds to androgen receptor, a compound that binds to estrogen receptor, a compound that binds to thyroid hormone receptor, a compound that binds to HIV protease, a compound that binds to HIV integrase, a compound that binds to HCV protease, a compound that binds to acyl protein thioesterase 1 and/or 2, a compound that binds to c-MYC protein, a compound that binds to laser kinase A, a compound that binds to Bcr-Abl protein, a compound that binds to bromodomain protein 4 (BRD4), a compound that binds to anaplastic lymphoma kinase (ALK), a compound that binds to Bruton's tyrosine kinase (BTK), a compound that binds to cyclin-dependent kinase 4/6 (CDK4/6), or a compound that binds to epidermal growth factor receptor.

**23.** The compound according to any one of claims 9-22, wherein the PTM is a moiety binding to androgen receptor.

**24.** The compound according to claim 23, wherein PTM is selected from the following structures:

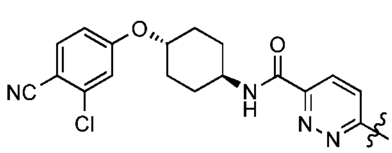 or 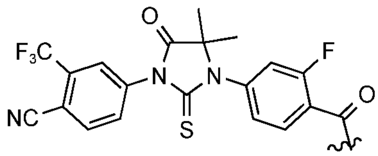

**25.** The compound according to claim 23 or 24, wherein the compound is selected from the following structures:

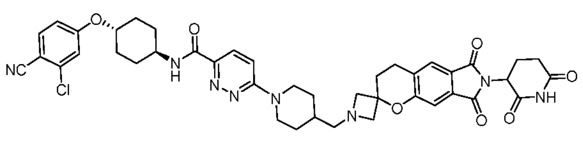 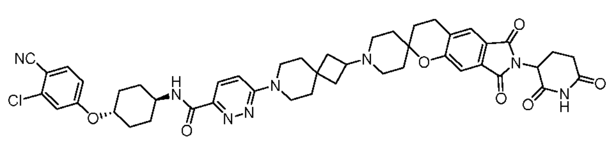

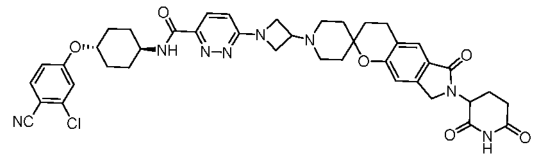

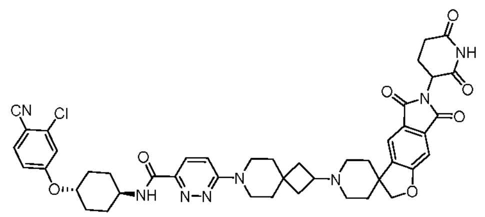

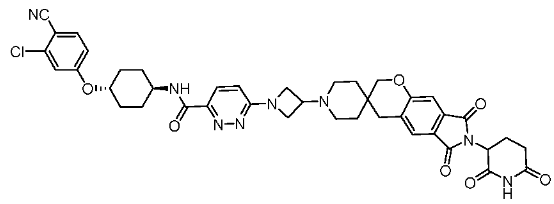

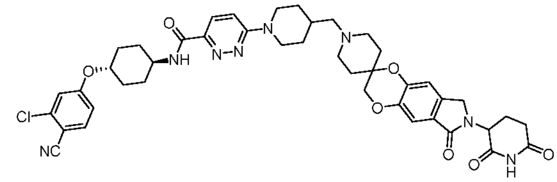

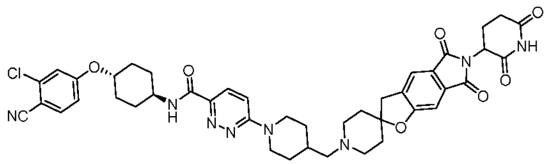

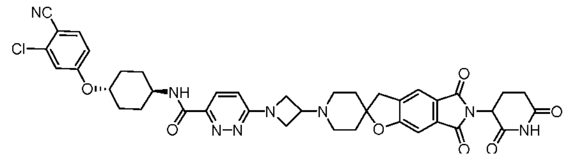

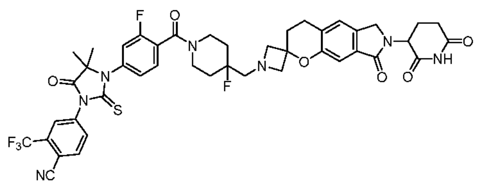

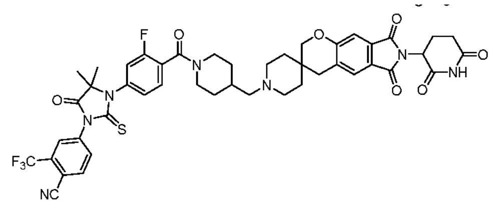

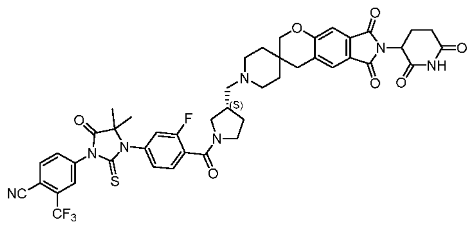

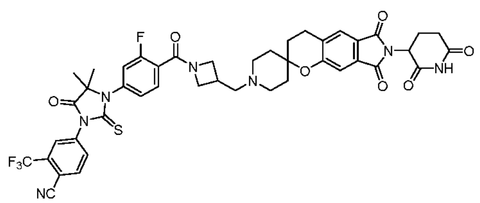

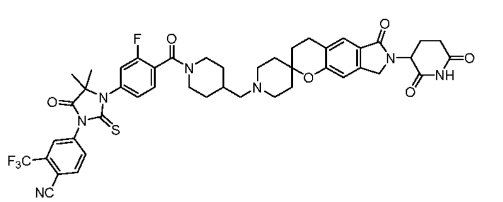

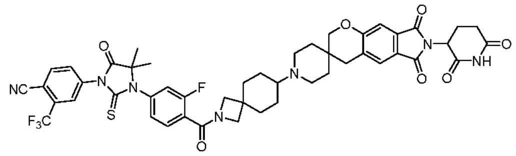

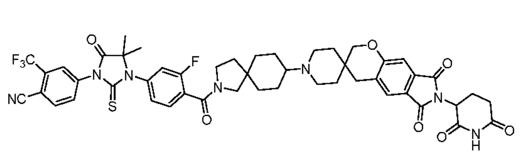

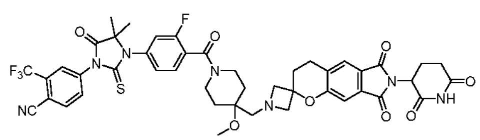

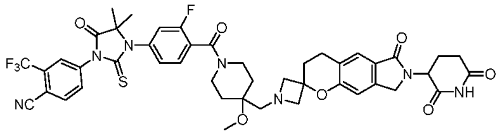

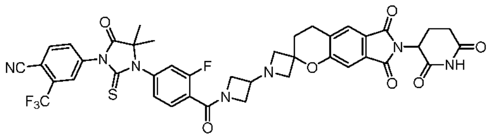

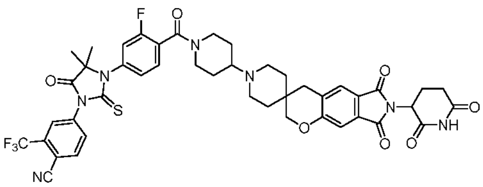

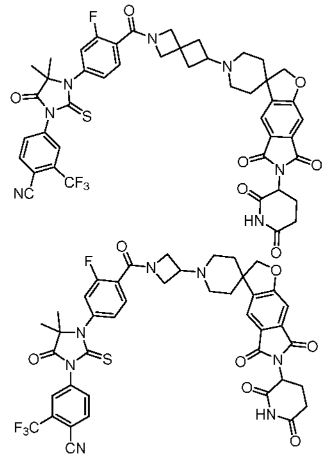

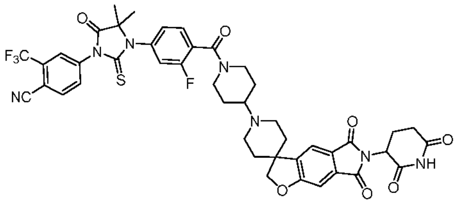

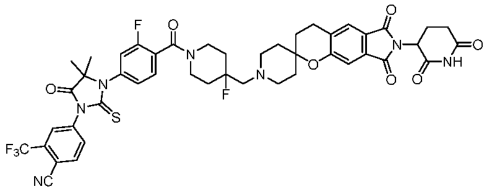

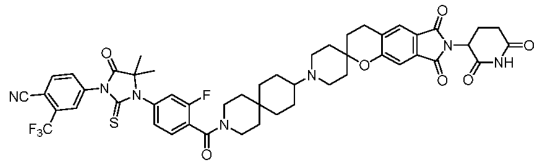

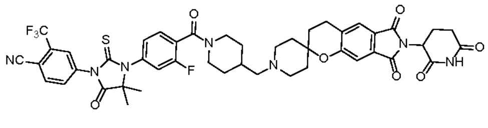

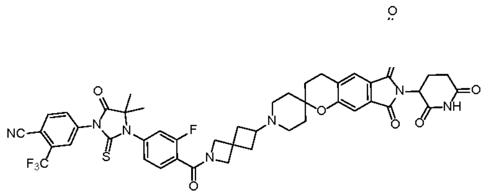

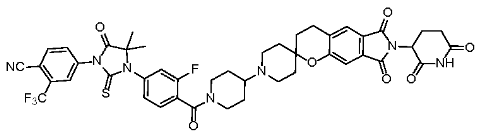

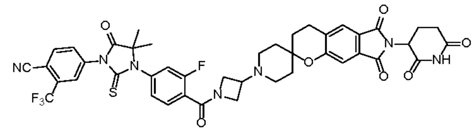

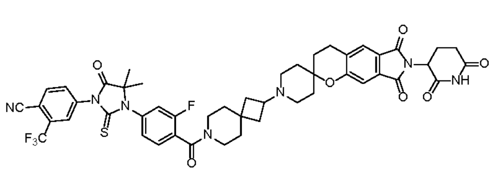

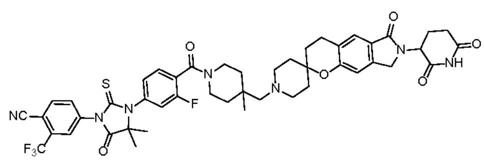

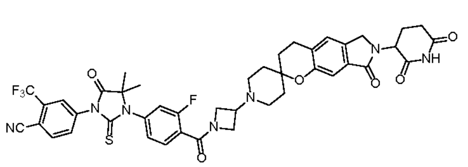

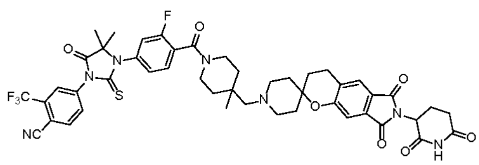

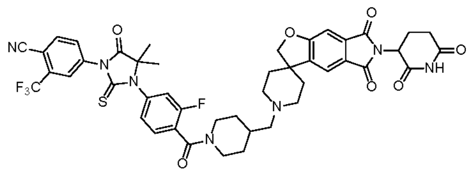

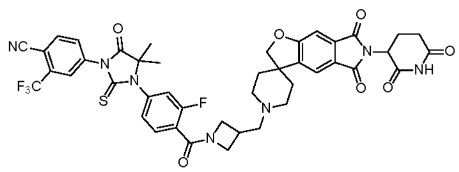

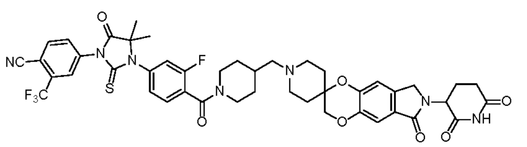

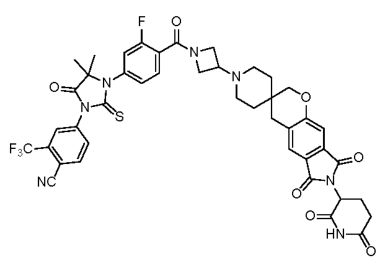

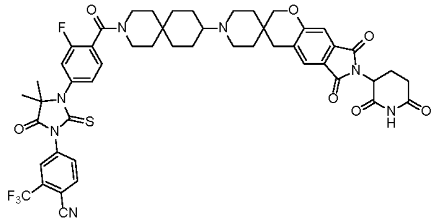

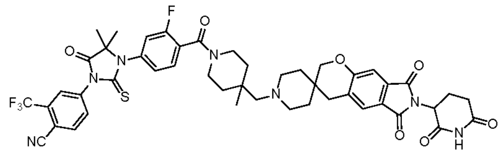

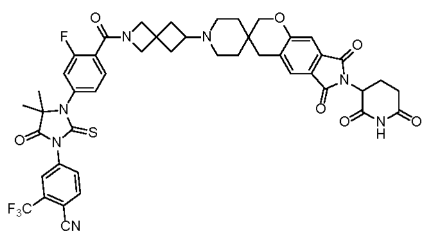

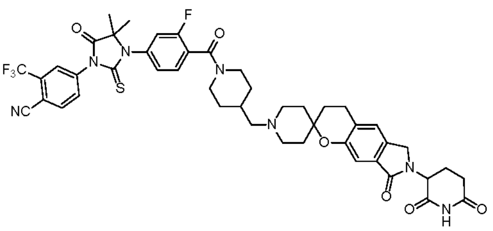

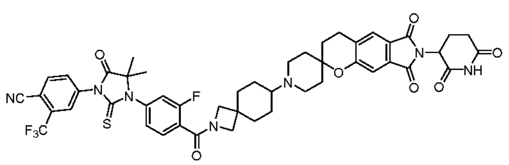

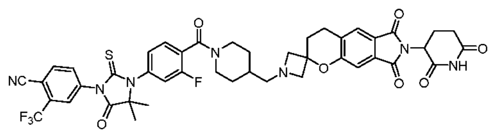

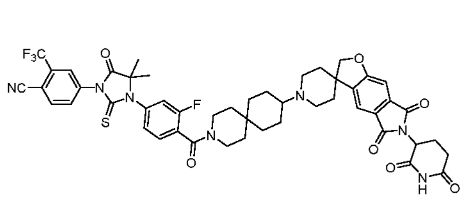

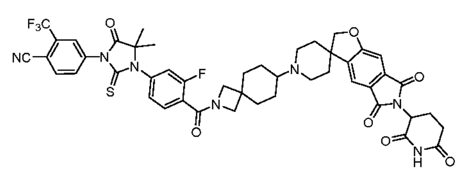

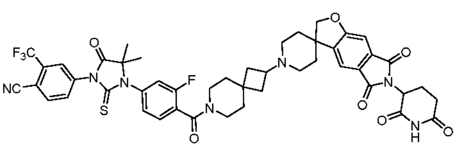

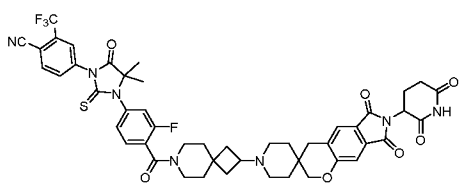

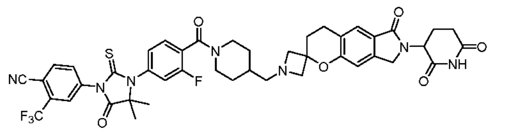

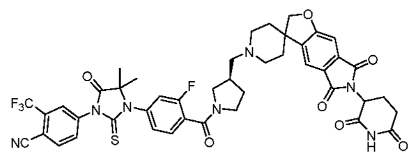

**26.** The compound according to any one of claims 9-22, wherein the PTM is a moiety binding to aurora kinase A (AURORA-A).

**27.** The compound according to claim 26, wherein PTM has the following structure:

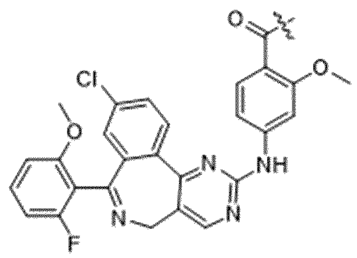

**28.** The compound according to claims 26 or 27, wherein the compound has the following structures:

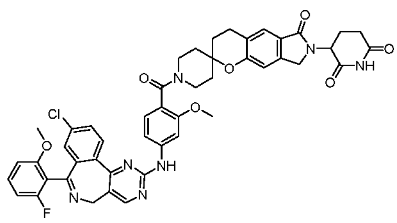

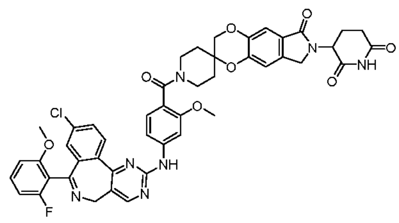

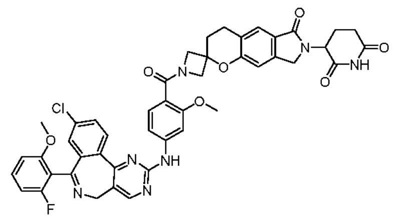

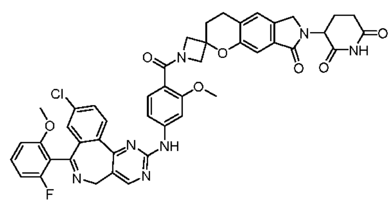

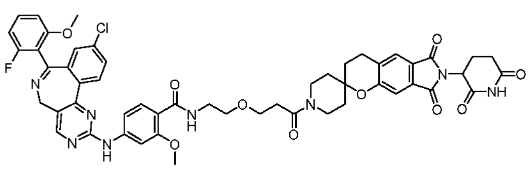

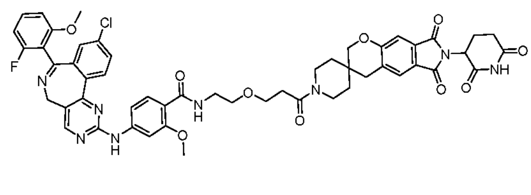

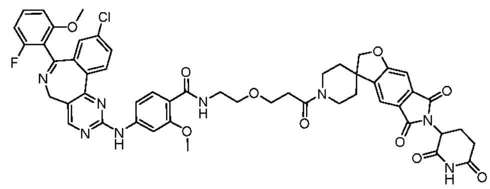

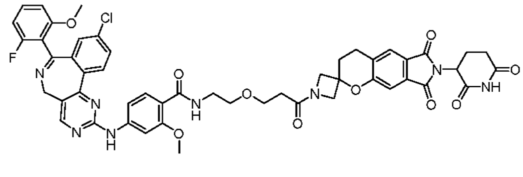

**29.** The compound according to any one of claims 9-22, wherein the PTM is a moiety binding to Bcr-Abl protein.

**30.** The compound according to claim 29, wherein the PTM has the following structure:

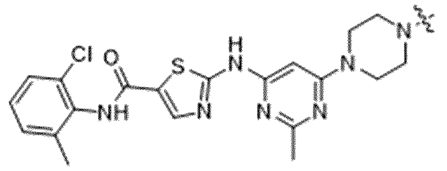

**31.** The compound according to claims 29 or 30, wherein the compound has the following structure:

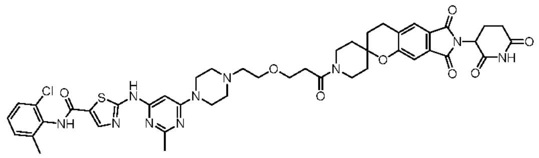

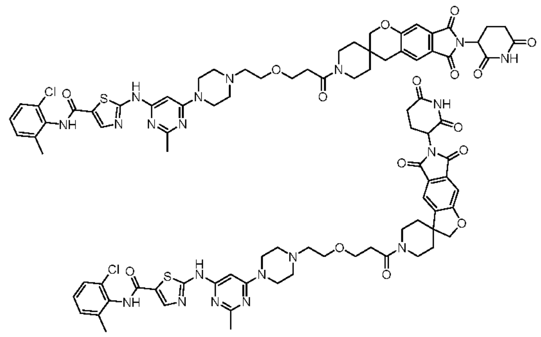

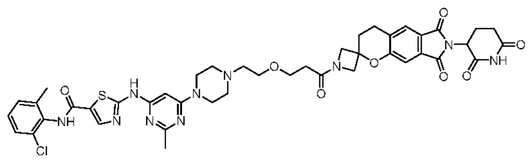

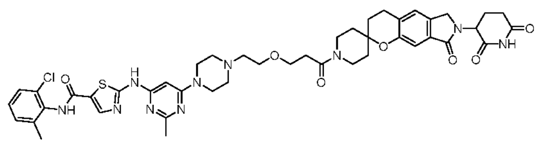

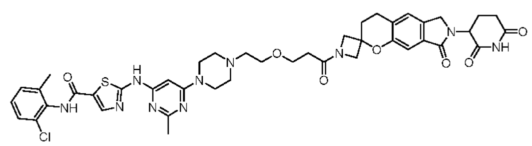

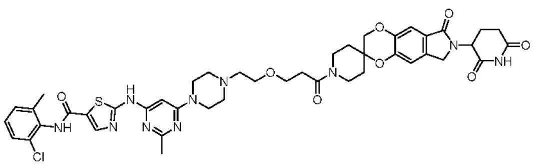

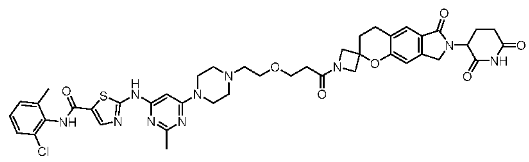

**32.** The compound according to any one of claims 9-22, wherein the PTM is a moiety binding to epidermal growth factor receptor (EGFR).

**33.** The compound according to claim 32, wherein the PTM has the following structure:

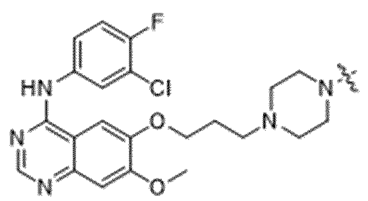

**34.** The compound according to claims 32 or 33, wherein the comopund has the following structures:

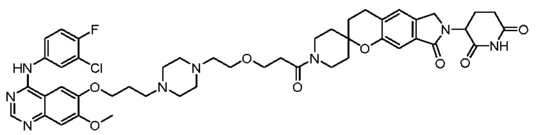

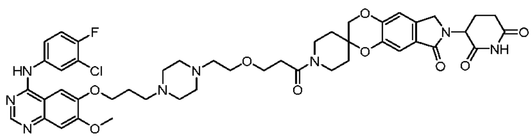

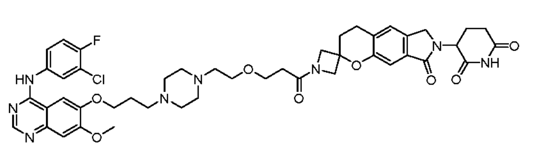

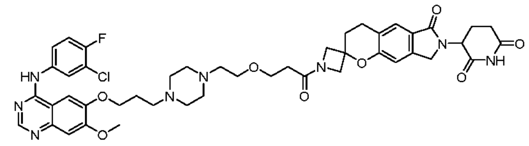

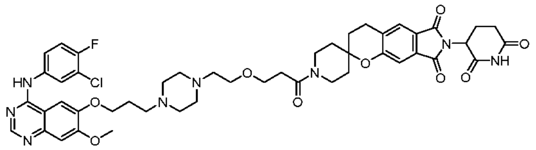

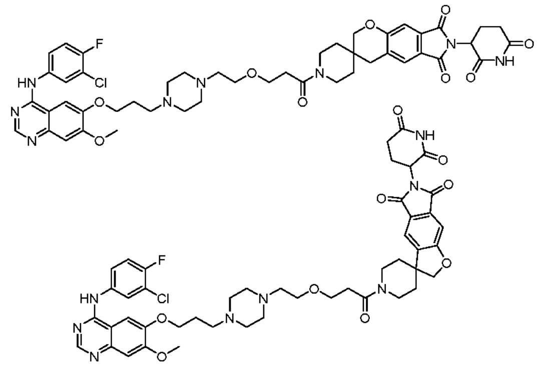

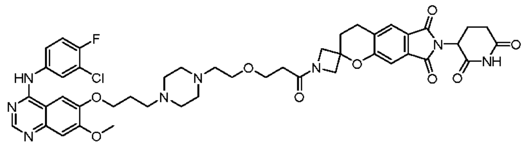

**35.** The compound according to any one of claims 9-22, wherein the PTM is a moiety that binds to anaplastic lymphoma kinase (ALK).

**36.** The compound according to claim 35, wherein PTM has the following structure:

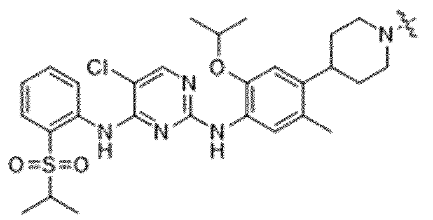

**37.**

The compound according to claims 35 or 36, wherein the comopund has the following structures:

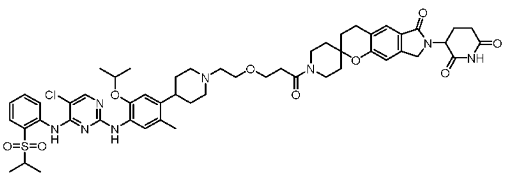

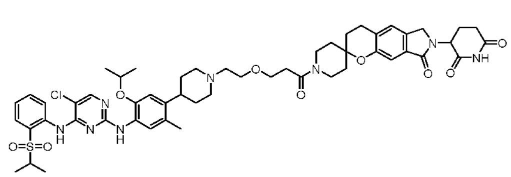

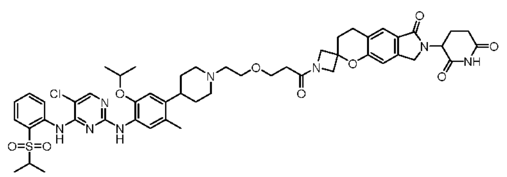

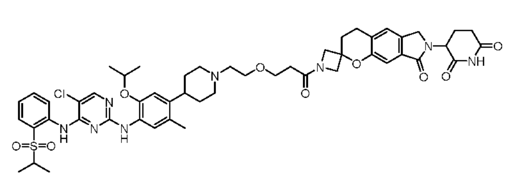

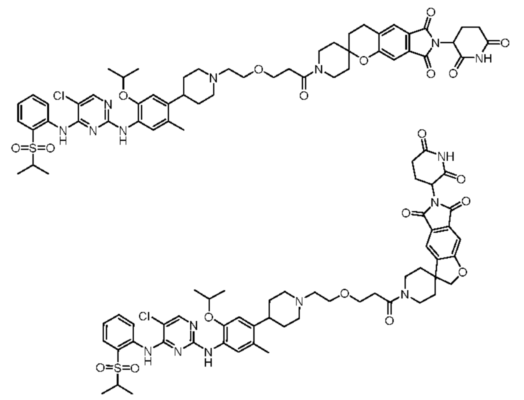

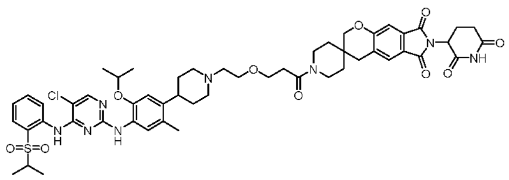

**38.** The compound according to any one of claims 9-22, wherein the PTM is a moiety binding to Bruton's tyrosine kinase (BTK).

**39.** The compound according to claim 38, wherein the PTM has the following structure:

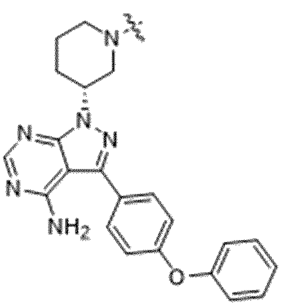

**40.** The compound according to claims 38 or 39, wherein the compound has the following structures:

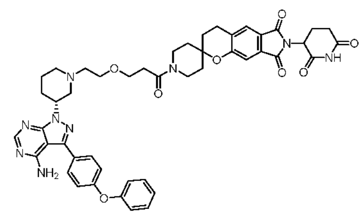

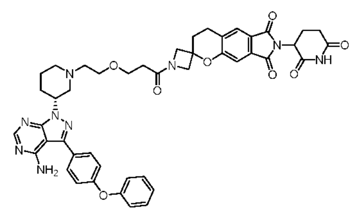

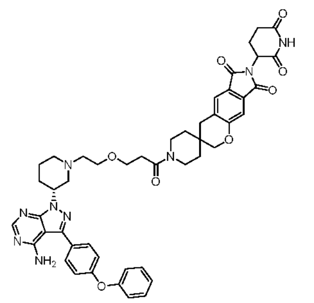

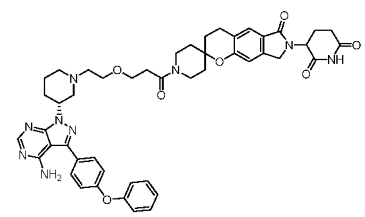

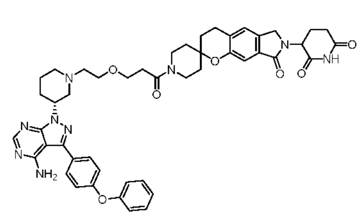

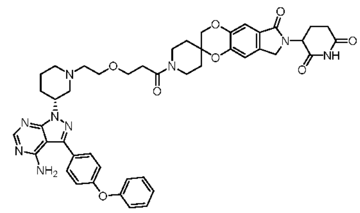

**41.** The compound according to any one of claims 9-22, wherein the PTM is a moiety binding to cyclin-dependent kinase 4/6 (CDK4/6).

**42.** The compound according to claim 41, wherein the PTM has the following structures:

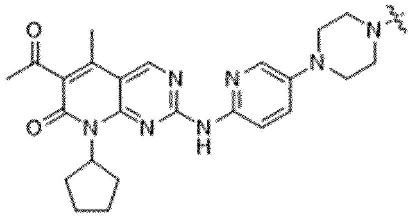

and 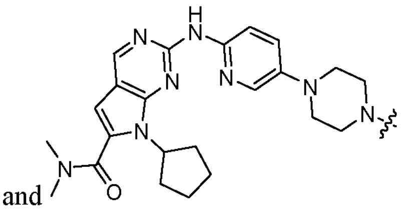

**43.** The compound according to claims 41 or 42, wherein the comopund has the following structures:

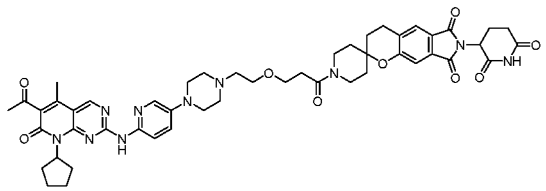

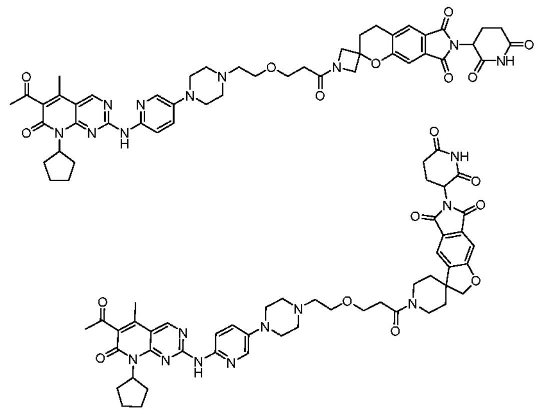

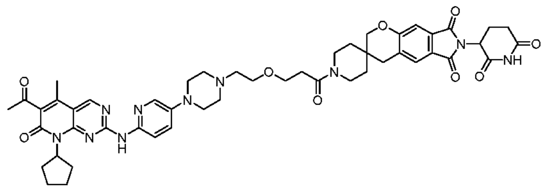

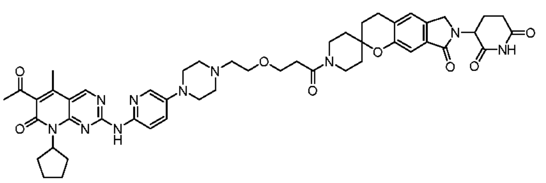

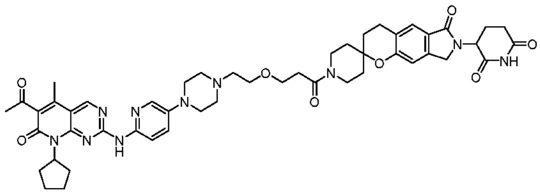

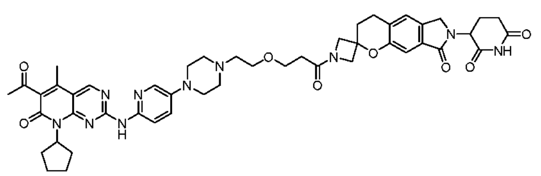

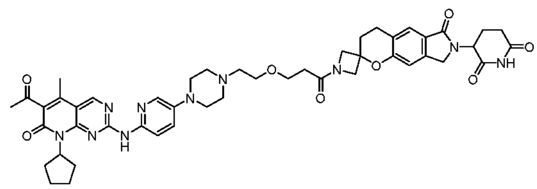

**44.** The compound according to any one of claims 9-22, wherein the PTM is a moiety binding to bromodomain protein 4 (BRD4).

**45.** The compound according to claim 44, wherein the PTM has the following structure:

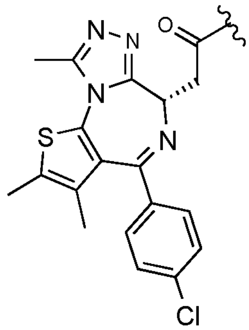

**46.** The compound according to claims 44 or 45, wherein the comopund has the following structures:

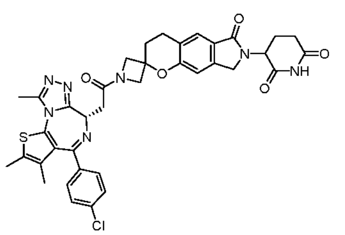
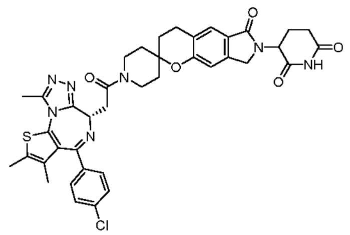
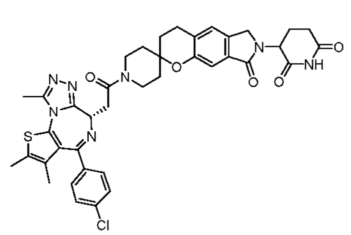
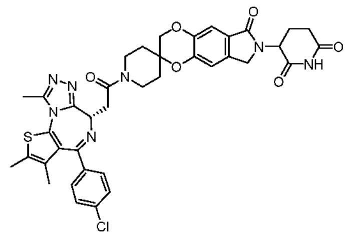
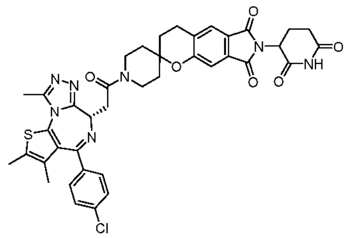
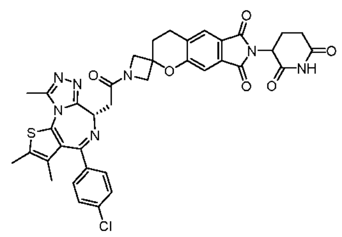

**47.** The compound according to any one of claims 9-22, wherein the PTM is a moiety binding to estrogen receptor (ER).

**48.** The compound according to claim 47, wherein PTM has the following structure:

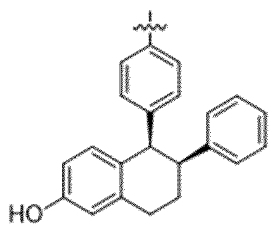

**49.** The compound according to claims 47 or 48, wherein the comopund has the following structures:

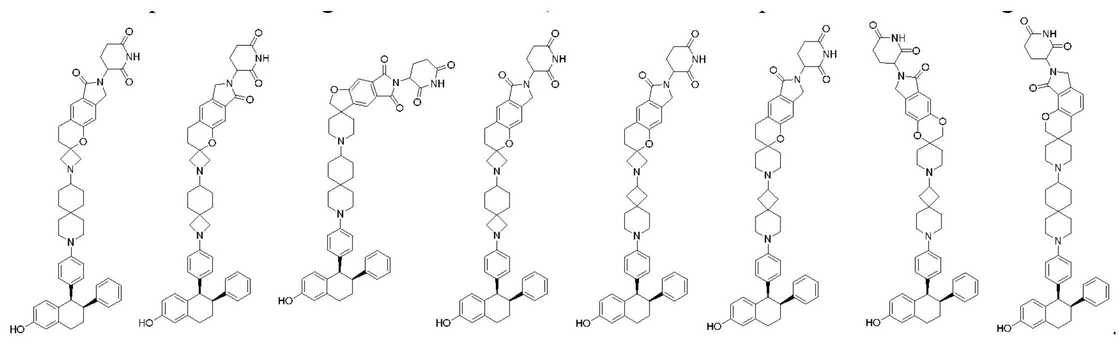

**50.** A pharmaceutical composition, wherein the pharmaceutical composition comprises a compound of any one of claims 1-49.

**51.** Use of the compound according to any one of claims 9 to 49, or the pharmaceutical composition according to claim 50, in the preparation of a medicament for the treatment or prevention of diseases treated by degrading target protein bound to a ligand of the target protein, and the target protein is preferably a cyclin-dependent kinase 4/6, androgen receptor, estrogen receptor, aurora kinase A, Bcr-Abl protein, epidermal growth factor receptor, anaplastic lymphoma kinase, Bruton's tyrosine kinase, or bromodomain protein 4.

**52.** Use of the compound according to any one of claims 9 to 49, or the pharmaceutical composition according to claim 50, in the preparation of a medicament for the treatment or prevention of diseases treated by binding to cereblon in vivo.

**53.** Use of the compound according to any one of claims 9 to 49, or the pharmaceutical composition according to claim 50, in the treatment or prevention of a disorder associated with the accumulation and/or aggregation of a target protein,

preferably the target protein is a cyclin-dependent kinase 4/6, androgen receptor, estrogen receptor, aurora kinase A, Bcr-Abl protein, epidermal growth factor receptor, anaplastic lymphoma kinase, Bruton's tyrosine kinase, or bromodomain protein 4.

**54.** The use according to any one of claims 51 to 53, wherein the disease is a tumor or cancer, preferably the tumor or cancer is breast cancer, breast ductal carcinoma, prostate cancer, mantle cell lymphoma, chronic myelogenous leukemia, acute myeloid leukemia, myelomonocytic leukemia, non-small cell lung cancer, lung adenocarcinoma, and/or cervical cancer.

**55.** A compound according to any one of claims 9 to 49, or a pharmaceutical composition according to claim 50, for use in the treatment or prevention of a disorder treated by degrading a target protein bound to a ligand of the target protein, and the target protein is preferably a cyclin-dependent kinase 4/6, androgen receptor, estrogen receptor, aurora kinase A, Bcr-Abl protein, epidermal growth factor receptor, anaplastic lymphoma kinase, Bruton's tyrosine kinase, or bromodomain protein 4.

**56.** A compound according to any one of claims 9 to 49, or a pharmaceutical composition according to claim 50, for use in the treatment or prevention of a disorder treated by binding to a cereblon protein.

**57.** A compound according to any one of claims 9 to 49, or a pharmaceutical composition according to claim 50, for use in the treatment or prevention of a disorder associated with the accumulation and/or aggregation of a target protein, preferably the target protein is a cyclin-dependent kinase 4/6, androgen receptor, estrogen receptor, aurora kinase A, Bcr-Abl protein, epidermal growth factor receptor, anaplastic lymphoma kinase, Bruton's tyrosine kinase, or bromodomain protein 4.

**58.** The compound, or pharmaceutical composition thereof, for use as claimed in any one of claims 55 to 57, wherein the disorder is a tumor or cancer, preferably the tumor or cancer is breast cancer, breast ductal carcinoma, prostate cancer, mantle cell lymphoma, chronic myeloid leukemia, acute myeloid leukemia, myelomonocytic leukemiaa, non-small cell lung cancer, lung adenocarcinoma, and/or cervical cancer.

**59.** A method for treating or preventing a disease treated by degrading a target protein bound to a ligand of the target protein, which includes administering a therapeutically effective amount of the compound according to any one of claims 9 to 49 or the pharmaceutical composition according to claim 50 to a subject in need thereof, and the target protein is preferably a cyclin-dependent kinase 4/6, androgen receptor, estrogen receptor, aurora kinase A, Bcr-Abl protein, epidermal growth factor receptor, interstitial Anaplastic lymphoma kinase, Bruton's tyrosine kinase, or bromodomain protein 4.

**60.** A method for treating or preventing a disease treated by binding to cereblon in vivo, including administering a therapeutically effective amount of the compound according to any one of claims 9 to 49, or the pharmaceutical composition according to claim 50.

**61.** A method for treating or preventing a disease associated with the accumulation and/or aggregation of a target protein, comprising administering a therapeutically effective amount of the compound according to any one of claims 9 to 49, or the pharmaceutical composition according to claim 50, to a subject in need thereof, and the target protein is preferably a cyclin-dependent kinase 4/6, androgen receptor, estrogen receptor, aurora kinase A, Bcr-Abl protein, epidermal growth factor receptor, anaplastic lymphoma kinase, Bruton's tyrosine kinase, or bromodomain protein 4.

**62.** The method according to any one of claims 59 to 62, wherein the disease is a tumor or cancer, preferably the tumor or cancer is a breast cancer, breast ductal carcinoma, prostate cancer, mantle cell lymphoma, chronic myelogenous leukemia, acute myeloid leukemia, myelomonocytic leukemia, non-small cell lung cancer, lung adenocarcinoma, and/or cervical cancer.

**63.** A method for inducing the degradation of a target protein in cells, wherein the method comprises administering an effective amount of the compound according to any one of claims 9 to 49, or the pharmaceutical composition according to claim 50, and the target protein is preferably cyclin-dependent kinase 4/6, androgen receptor, estrogen receptor, aurora kinase A, Bcr-Abl protein, epidermal growth factor receptor, anaplastic lymphoma kinase, Bruton's tyrosine kinase, or bromodomain protein 4.

**INTERNATIONAL SEARCH REPORT**

International application No.
**PCT/CN2023/073745**

**A. CLASSIFICATION OF SUBJECT MATTER**

C07D 487/04(2006.01)i; C07D 471/04(2006.01)i; C07D 471/10(2006.01)i; C07D 471/14(2006.01)i; C07D 471/20(2006.01)i; C07D 487/10(2006.01)i; C07D 498/04(2006.01)i; C07D 498/14(2006.01)i; A61P 35/00(2006.01)i; A61K 31/407(2006.01)i; A61K 31/435(2006.01)i; A61K 31/437(2006.01)i; A61K 31/438(2006.01)i

According to International Patent Classification (IPC) or to both national classification and IPC

**B. FIELDS SEARCHED**

Minimum documentation searched (classification system followed by classification symbols)

C07D487/-,C07D471/-,C07D498/-,A61P35/00

Documentation searched other than minimum documentation to the extent that such documents are included in the fields searched

Electronic data base consulted during the international search (name of data base and, where practicable, search terms used)

CNABS, CNKI, VEN, STN(CAPLUS, REGISTRY): 甘李药业, 小脑蛋白E3泛素连接酶, 泛素酶, 蛋白降解靶向嵌合体, 螺环, cereblon, ubiquitin, ligase, CRBN, PROTAC, CLM-L-PTM, spirocyclic, 根据通式I中限定结构进行的子结构检索, substructure search according to a qualified structure in general formula I

**C. DOCUMENTS CONSIDERED TO BE RELEVANT**

| Category* | Citation of document, with indication, where appropriate, of the relevant passages | Relevant to claim No. |
|---|---|---|
| X | WO 2021143822 A1 (JIANGSU HENGRUI MEDICINE CO., LTD.; SHANGHAI HENGRUI PHARMACEUTICAL CO., LTD.) 22 July 2021 (2021-07-22)<br>description, pages 2-14, 20-23, 27-28, 32-34 | 1-63 |
| Y | WO 2021143822 A1 (JIANGSU HENGRUI MEDICINE CO., LTD.; SHANGHAI HENGRUI PHARMACEUTICAL CO., LTD.) 22 July 2021 (2021-07-22)<br>description, pages 2-14, 20-23, 27-28, 32-34 | 1-63 |
| X | WO 2021126974 A1 (ORIONIS BIOSCIENCES INC.) 24 June 2021 (2021-06-24)<br>claims 1-24, description, pages 20-23, 295 | 1-63 |
| Y | WO 2021231927 A1 (UNIV MICHIGAN REGENTS) 18 November 2021 (2021-11-18)<br>description, pages 5-10, 48, 85-101 | 1-63 |
| Y | WO 2021041664 A1 (UNIV MICHIGAN REGENTS) 04 March 2021 (2021-03-04)<br>description, pages 8-9, 52-86, 88-90, 149, 153 | 1-63 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 May 2023** | **24 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/073745**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **53-54, 59-63**
because they relate to subject matter not required to be searched by this Authority, namely:

Claims 53-54 and 59-63 relate to a method for treatment implemented on the human or animal body (PCT Rule 39.1(iv)). However, a search is still carried out on the basis of the technical subject matter of the use of the compound in the preparation of the corresponding drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/073745**

| Patent document cited in search report | Publication date (day/month/year) | Patent family member(s) | Publication date (day/month/year) |
|---|---|---|---|
| WO 2021143822 A1 | 22 July 2021 | None | |
| WO 2021126974 A1 | 24 June 2021 | JP 2023508891 A | 06 March 2023 |
| | | AU 2020408333 A1 | 07 July 2022 |
| | | CA 3162266 A1 | 24 June 2021 |
| | | US 2023099031 A1 | 30 March 2023 |
| | | EP 4076530 A1 | 26 October 2022 |
| WO 2021231927 A1 | 18 November 2021 | None | |
| WO 2021041664 A1 | 04 March 2021 | CA 3151824 A1 | 04 March 2021 |
| | | AU 2020336381 A1 | 03 March 2022 |
| | | EP 4021580 A1 | 06 July 2022 |
| | | US 2022388978 A1 | 08 December 2022 |
| | | JP 2022545735 A | 28 October 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **BEIJING INOCHEM** ; **JIANGSU AIKON** ; **SINOPHARM GROUP** ; **BEIJING J&K SCIENTIFIC** ; **YUNNAN XINLAN JING**. *Energy Chemical, Bide Pharmatech*